# EUROPEAN PATENT APPLICATION

(11) **EP 4 696 679 A1**
(43) Date of publication of application: **18.02.2026**
(21) Application number: 24831069.0
(22) Date of filing: 30.06.2024
(51) Int. Cl.: C07D 213/65, B01J 31/02, C07C 213/00

(54) **CHIRAL PYRIDINIUM SALT CARBONYL CATALYST, AND PREPARATION METHOD THEREFOR AND USE THEREOF**

(30) Priority: 30.06.2023 CN 202310794646
(71) Applicant: Shanghai Normal University, Shanghai 200234 (CN)
(72) Inventor: ZHAO, Baoguo, Shanghai 200234 (CN); XIAO, Xiao, Shanghai 200234 (CN); LIANG, Hanyu, Shanghai 200234 (CN)
(74) Representative: Sun, Yiming
(86) International application number: PCT/CN2024/102778
(87) International publication number: WO 2025/002445

(57) **Abstract**

The present invention relates to a chiral pyridinium salt carbonyl catalyst, a preparation method therefor and applications thereof. The catalyst has a general structural formula of: 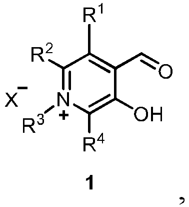 wherein R¹ has a structural formula of: 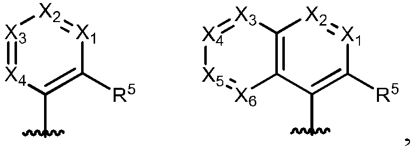 X₁₋₆ are each independently N or C-R⁶⁻¹¹;
R², R⁴, and R⁶⁻¹¹ each comprise hydrogen, C₁₋₂₄ alkyl, alkyloxy, (CH₂)ₘ, 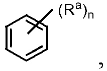 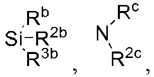 CO₂R^{d}, halogen, nitro, cyano or trifluoromethyl;
R³ comprises substituted or unsubstituted C₁₋₂₄ alkyl, the substituent comprises halogen, C₁₋₁₀ alkyl, C₃₋₁₀ cycloalkyl or aryl, C₁₋₈ carbonyl, C₁₋₈ sulfonyl or phosphoryl, or C₁₋₁₀ alkoxy or amino, and the carbonyl comprises aldehyde group, ketone carbonyl, ester carbonyl, carboxyl or amido;
R⁵ has a structural formula of: 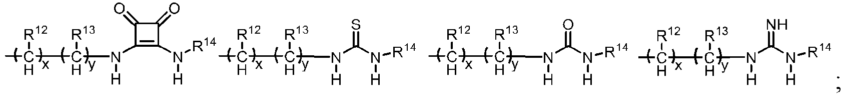 X⁻ is an anion. Compared with the prior art, the catalyst of the present invention is applied to the biomimetic aldol reaction of amino acid derivatives to synthesize a series of β-hydroxy-α-amino acid compounds having extremely high enantioselectivity.

## Description

### CROSS REFERENCE TO RELATED APPLICATIONS

This application claims partial priorities to Chinese Patent Application No. 202310794646.0, filed on June 30, 2023 and entitled "CHIRAL PYRIDINIUM SALT CARBONYL CATALYST, PREPARATION METHOD THEREFOR AND APPLICATIONS THEREOF", and Chinese Patent Application No. 202410863976.5, filed on June 30, 2024 and entitled "CHIRAL PYRIDINIUM SALT CARBONYL CATALYST, PREPARATION METHOD THEREFOR AND APPLICATIONS THEREOF", the related contents of which are herein incorporated by reference.

### Technical Field

The present invention belongs to the field of chemical synthesis, and relates to a chiral pyridinium salt carbonyl catalyst, a preparation method therefor and applications thereof.

### Description of Related Art

In living organisms, the direct addition of glycine with acetaldehyde is catalyzed by threonine aldolase to produce β-hydroxy-α-aminobutyric acid, i.e., threonine. In this reaction, vitamin B6, a general term for pyridoxal, pyridoxine, pyridoxamine and their phosphate derivatives, is the active center of enzyme. It is a water-soluble vitamin and acts mainly as a coenzyme factor involved in a variety of enzyme-catalyzed reactions. Since the 1980s, chemists have been mimicking the chemical structure of vitamin B6 to develop a biomimetic catalyst/artificial enzyme. For example, Kuzuhara and Breslow in their research group carried out the asymmetric aldol reaction of glycine with aldehyde by using the equivalent of chiral carbonyl catalyst to lay a solid foundation for the biomimetic mimicry studies of threonine aldolase (H. Kuzuhara, N. Watanabe, M. Ando, J. Chem. Soc., Chem. Commun. 1987, 95-96; M. Ando, H. Kuzuhara, Bull. Chem. Soc. Jpn. 1990, 63, 1925-1928; J. T. Koh, L. Delaude, R. Breslow, J. Am. Chem. Soc. 1994, 116, 11234-11240). These studies generally require the equivalent of chiral pyridoxal, but with moderate reaction activity, stereoselectivity and narrow application scope of aldehyde substrates. As a result, the early chiral pyridoxal carbonyl catalysts show lower applicability in organic synthesis. In recent years, Zhao's research group synthesized and developed a series of chiral pyridoxal carbonyl catalysts, which were successfully applied to the asymmetric Mannich reaction of glycinate, the asymmetric aldol reaction of trifluoromethyl ketones, asymmetric Michael addition reaction and asymmetric α-allylation reaction, and the asymmetric addition reaction of inert aryl and alkynyl methylamine compounds, achieving excellent results (Chen, J. et al, Science 2018, 360, 1438-1442; Ma, J. et al, Angew. Chem. Int. Ed. 2021, 60, 10588-10592; Cheng, A. et al, Angew. Chem. Int. Ed. 2021, 60, 20166-20172; Ma, J. et al, Angew. Chem. Int. Ed. 2022, 61, e202200850; Hou, K. et al, Nat. Catal. 2022, 5, 1061-1068; Ji, P. et al, Angew. Chem. Int. Ed. 2022, 61, e202206111). However, the chiral carbonyl catalysts that can efficiently catalyze the aldol reaction of glycine compounds with aldehydes have not been successfully developed, mainly due to numerous challenges in this reaction as follows: (1) aldehydes are prone to forming imines with glycine compounds, leading to a failed aldol reaction; (2) aldol products resulting from carbonyl catalysis are prone to an inverse aldol reaction, leading to the decomposition and racemization of the aldol products; and (3) the aldol products are prone to epimerization under alkaline conditions due to its highly acidic amino α-H, leading to difficulty in controlling the enantioselectivity and diastereoselectivity of the products. Because of these challenges, the catalysts that can efficiently catalyze this transformation have not been developed to date.

Many β-hydroxy-α-amino acid compounds are important pharmaceutical intermediates, and their synthesis have received widespread attention. There have been reports on the enzymatic aldol reaction of glycine with aldehydes, but with moderate conversion rate and low diastereoselectivity, which limits the synthetic applications of biological methods for preparing β-hydroxy-α-amino acids (Dückers, N. et al, Appl. Microbiol. Biotechnol. 2010, 88, 409-424; Fesko, K. et al, Appl. Microbiol. Biotechnol. 2016, 100, 2579-2590; Wang, S. et al, Appl. Microbiol. Biotechnol. 2021, 105, 3507-3520). Also, it is difficult to implement the asymmetric aldol reaction of glycine and its derivatives with aldehydes by chemical catalysis, since there is a lack of chiral catalysts that can efficiently catalyze this reaction, making it difficult to achieve the rapid chemical synthesis of chiral β-hydroxy-α-amino acid compounds.

### BRIEF SUMMARY OF THE INVENTION

An object of the present invention is to overcome at least one of the defects existing in the prior art described above, and provide a chiral pyridinium salt carbonyl catalyst, a preparation method therefor and applications thereof. The chiral pyridinium salt carbonyl catalyst of the present invention is applied to the biomimetic aldol reaction of amino acid derivatives to synthesize a series of β-hydroxy-α-amino acid compounds having extremely high enantioselectivity.

The object of the present invention can be achieved by means of the following technical solutions.

One of the technical solutions of the present invention consists in providing a chiral pyridinium salt carbonyl catalyst, and the pyridinium salt carbonyl catalyst 1 has a general structural formula of: 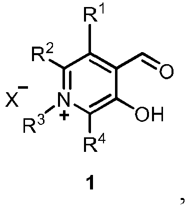 wherein R¹ has a structural formula of: 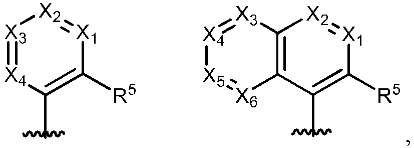
X₁ comprises N or C-R⁶, X₂ comprises N or C-R⁷, X₃ comprises N or C-R⁸, X₄ comprises N or C-R⁹, X₅ comprises N or C-R¹⁰, and X₆ comprises N or C-R¹¹;
R², R⁴, R⁶, R⁷, R⁸, R⁹, R¹⁰ and R¹¹ each comprise hydrogen, C₁₋₂₄ alkyl, alkyloxy, (CH₂)ₘ 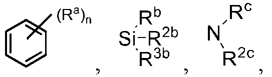 CO₂R^{d}, halogen, nitro, cyano or trifluoromethyl;
R^{a} comprises hydrogen, C₁₋₁₀ alkyl, aryl, halogen, nitro, cyano or trifluoromethyl, R^{b}, R^{2b}, R^{3b}, R^{c}, R^{2c} and R^{d} each comprise hydrogen, C₁₋₁₀ alkyl or aryl, m is 1, 2 or 3, and n is 0, 1, 2, 3, 4 or 5;
R³ comprises substituted or unsubstituted C₁₋₂₄ alkyl, the substituent comprises halogen, C₁₋₁₀ alkyl, C₃₋₁₀ cycloalkyl or aryl, C₁₋₈ carbonyl, C₁₋₈ sulfonyl or phosphoryl, or C₁₋₁₀ alkoxy or amino, and the carbonyl comprises aldehyde group, ketone carbonyl, ester carbonyl, carboxyl or amido;
R⁵ has a structural formula of: 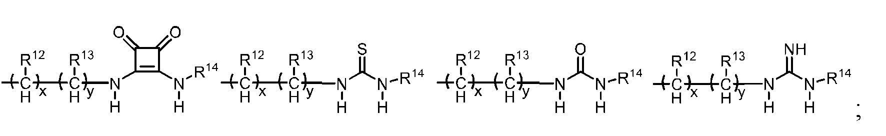
R¹² and R¹³ each comprise hydrogen, C₁₋₂₄ alkyl or 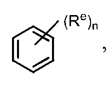 R¹⁴ comprises hydrogen, C₁₋₂₄ alkyl, 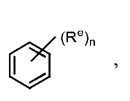 arylmethyl, diarylmethyl, triarylmethyl, halogen or trifluoromethyl;
R^{e} comprises hydrogen, C₁₋₁₀ alkyl, alkyloxy, aryl, halogen, nitro, cyano or trifluoromethyl, and x and y are 0, 1, 2 or 3;
the alkyl comprises methyl, ethyl, n-propyl, isopropyl, n-butyl, tert-butyl, isobutyl, cyclopentyl, n-pentyl, n-hexyl, cyclohexyl, n-heptyl, cycloheptyl, n-octyl, cyclooctyl, n-nonyl, cyclononyl, n-decyl or cyclodecyl, and the aryl comprises benzene, 2-biphenyl, 3-biphenyl, 4-biphenyl, 2,6-dibiphenyl, 3,5-dibiphenyl, 1-naphthyl, 2-naphthyl, 3,5-di-tert-butylbenzene, 4-tert-butylbenzene, 3,5-difluorobenzene, 4-fluorobenzene, 3,5-dichlorobenzene, 4-chlorobenzene, 3,5-bis(trifluoromethyl)benzene, 4-(trifluoromethyl)benzene, 3,5-dimethylbenzene, 4-methylbenzene or 4-methoxybenzene;
X⁻ is an anion, which comprises a fluoride ion, a chloride ion, a bromide ion, an iodide ion, a trifluoromethanesulfonate ion, a hexafluorophosphate ion, a tetrafluoroborate ion, a sulfate ion, a sulfite ion, a hydroxide ion, a nitrate ion, a phosphate ion, a carbonate ion, a silicate ion, a bicarbonate ion, a hydrogen phosphate ion, a dihydrogen phosphate ion, an iodate ion, a cyanide ion, or a thiocyanide ion.

Further, an axially chiral configuration is formed between R¹ and a pyridine ring, and the axially chiral configuration is an R or S configuration.

Further, the catalyst has a structure of formula Iₐ, formula I_{b}, formula IIₐ or formula II_{b}, the formula Iₐ and the formula I_{b} are enantiomers of each other, and the formula IIₐ and the formula II_{b} are enantiomers of each other:

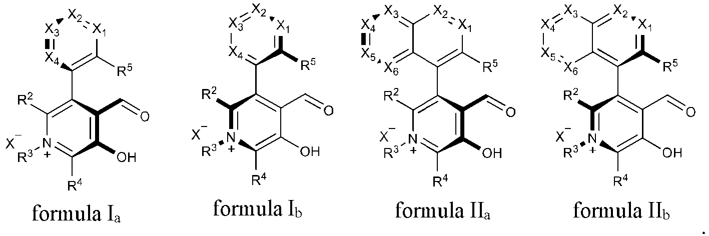

One of the technical solutions of the present invention consists in providing a preparation method for the chiral pyridinium salt carbonyl catalyst, wherein in the preparation method, by taking a split single-configuration compound 5, i.e., a chiral amine compound, as a starting material, the chiral amine compound 5 is condensed with dimethyl squarate in a solvent to produce a compound 6, the compound 6 is then condensed with primary amine to obtain an amide intermediate, the amide intermediate subsequently reacts with halogenated hydrocarbon to obtain a quaternary ammonium salt intermediate, which is finally hydrolyzed in the presence of an acid to obtain a final pyridinium salt carbonyl catalyst **1a**, with a reaction flow as follows:

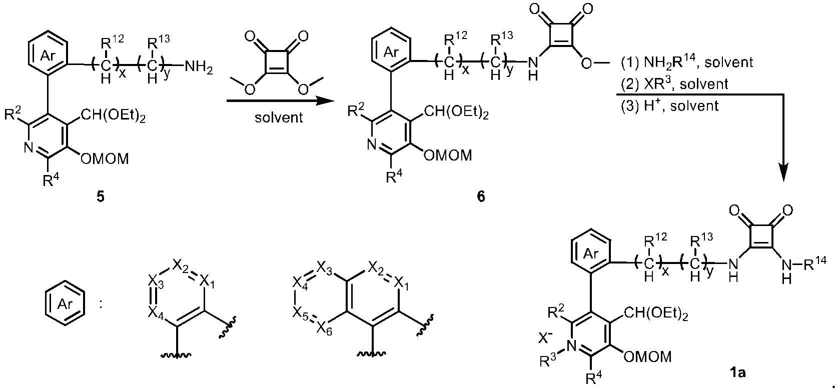

Further, a molar ratio of the compound **5** to the dimethyl squarate is 1:(1 to 10), with a reaction temperature of -20°C to 120°C and a reaction time of 1 h to 72 h;
a molar ratio of the compound **6** to the primary amine to the halogenated hydrocarbon is 1:(1 to 50):(1 to 50), with a reaction temperature of 0°C to 100°C and a reaction time of 1 h to 48 h.

One of the technical solutions of the present invention consists in providing a preparation method for the chiral pyridinium salt carbonyl catalyst, wherein in the preparation method, by taking a split single-configuration compound **5,** i.e., a chiral amine compound, as a starting material, the chiral amine compound **5** is condensed with unilateral squaramide to obtain an amide intermediate, and the amide intermediate subsequently reacts with halogenated hydrocarbon to obtain a quaternary ammonium salt intermediate, which is finally hydrolyzed to obtain a final pyridinium salt carbonyl catalyst **1a**, with a reaction flow as follows:

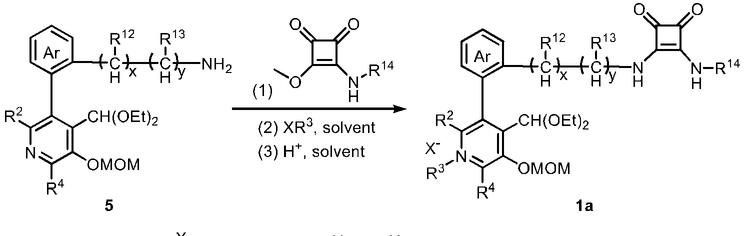

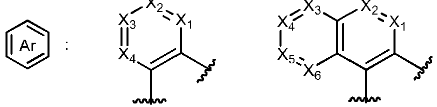

The difference between the above-mentioned methods lies in that the condensed unilateral squaramide is used in the second method to substitute the dimethyl squarate and primary amine in the two-step reaction in the first method.

Further, a molar ratio of the compound **5** to the unilateral squaramide to the halogenated hydrocarbon is 1:(1 to 50):(1 to 50), with a reaction temperature of -20°C to 120°C and a reaction time of 1 h to 72 h.

One of the technical solutions of the present invention consists in providing a preparation method for the chiral pyridinium salt carbonyl catalyst, wherein in the preparation method, by taking a split single-configuration compound **5,** i.e., a chiral amine compound, as a starting material, the chiral amine compound 5 is condensed with thioisocyanate or isocyanate in a solvent to produce a compound 7 or **8,** and the compound 7 or 8 subsequently reacts with halogenated hydrocarbon to obtain a quaternary ammonium salt intermediate, which is finally hydrolyzed in the presence of an acid to obtain a final pyridinium salt carbonyl catalyst **1b** or **1c**, with a reaction flow as follows:

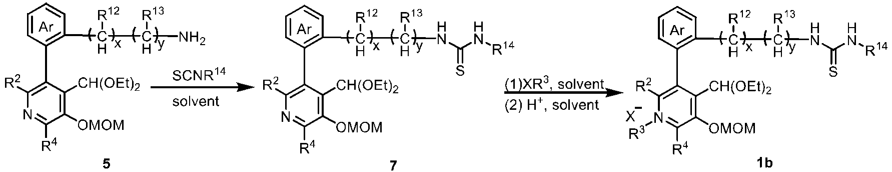

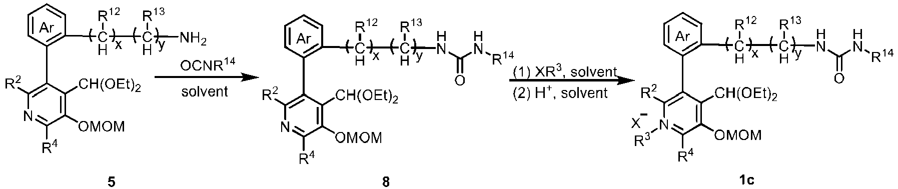

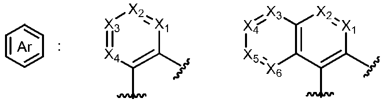

Further, a molar ratio of the compound 5 to the thioisocyanate or isocyanate is 1:(1 to 10), with a reaction temperature of -20°C to 120°C and a reaction time of 1 h to 72 h; a molar ratio of the compound 7 or **8** to the halogenated hydrocarbon is 1:(1 to 50), with a reaction temperature of 0°C to 100°C, and a reaction time of 1 h to 48 h.

One of the technical solutions of the present invention consists in providing a preparation method for the chiral pyridinium salt carbonyl catalyst, wherein in the preparation method, by taking a split single-configuration compound **5,** i.e., a chiral amine compound, as a starting material, the chiral amine compound 5 is condensed with thioisocyanate in a solvent to produce a compound 9, the compound 9 is coupled with primary amine in the presence of carbodiimide hydrochloride (EDCl) to produce a compound 10, the compound 10 is stripped of an ethyl carbonate protecting group in the presence of trimethyl bromosilane to produce a compound 11, and the compound 11 subsequently reacts with halogenated hydrocarbon to obtain a quaternary ammonium salt intermediate, which is finally hydrolyzed in the presence of an acid to obtain a final pyridinium salt carbonyl catalyst **1d**, with a reaction flow as follows:

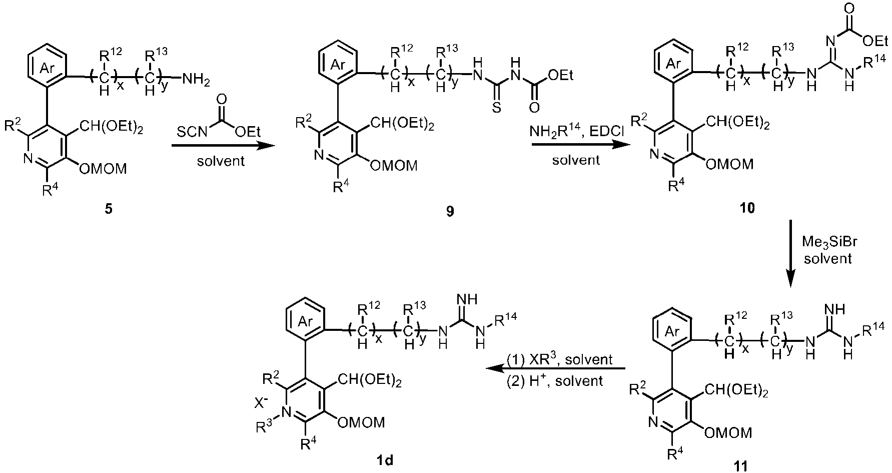

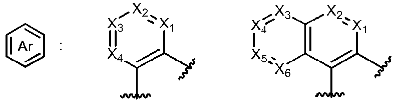

Further, a molar ratio of the compound 5 to the thioisocyanate is 1:(1 to 10), with a reaction temperature of -20°C to 120°C and a reaction time of 1 h to 72 h;
a molar ratio of the compound 9 to the carbodiimide hydrochloride to the primary amine is 1:(1 to 10):(1 to 10), with a reaction temperature of 0°C to 100°C and a reaction time of 1 h to 72 h;
a molar ratio of the compound 10 to the trimethyl bromosilane is 1:(1 to 10), with a reaction temperature of 0°C to 100°C, and a reaction time of 1 h to 72 h;
a molar ratio of the compound 11 to the halogenated hydrocarbon is 1 :(1 to 50), with a reaction temperature of 0°C to 100°C, and a reaction time of 1 h to 48 h.

Further, the solvent used in the preparation method is selected from one or more of water, benzene, toluene, xylene, trimethylbenzene, acetonitrile, ethyl ether, tetrahydrofuran, ethylene glycol dimethyl ether, chloroform, dichloromethane, methanol, ethanol, isopropanol, tert-butanol, 1,4-dioxane, N,N-dimethylformamide, *N,N-*dimethylacetamide, dimethyl sulfoxide, and N-methylpyrrolidone;
the acid used is selected from one or more of sulfuric acid, hydrochloric acid, hydrochloric acid in dichloromethane, hydrochloric acid in methanol, hydrochloric acid in tetrahydrofuran, hydrochloric acid in dioxane, phosphoric acid, hydrobromic acid, hydroiodic acid, acetic acid, trifluoroacetic acid, trichloroacetic acid, benzenesulphonic acid, p-toluenesulfonic acid, methanesulfonic acid and trifluoromethanesulfonic acid.

Further, in the preparation method, a silver salt is added to a chiral pyridinium salt carbonyl catalyst with an iodide ion for replacement to obtain a chiral pyridinium salt carbonyl catalyst with a corresponding anion of a silver salt;
the silver salt comprises silver fluoride, silver chloride, silver bromide, silver trifluoromethanesulfonate, silver hexafluorophosphate, silver tetrafluoroborate, silver sulfate, silver sulfite, silver hydroxide, silver nitrate, silver phosphate, silver carbonate, silver silicate, silver bicarbonate, silver hydrogen phosphate, silver dihydrogen phosphate, silver iodate, silver cyanide or silver thiocyanate.

As a preferred technical solution, the silver salt may also be selected from one of silver bisulfate, silver bisulfite, silver arsenate, silver arsenite, silver borate, silver bromate, silver hypobromite, silver chlorate, silver perchlorate, silver hypochlorite, silver chromate, silver aluminosilicate, silver acetate, silver formate, silver hydrogen oxalate, and silver hydrogen sulfide, and X⁻ in the catalyst may also be selected from one of a bisulfate ion, a bisulfite ion, an arsenate ion, an arsenite ion, a borate ion, a bromate ion, a hypobromite ion, a chlorate ion, a perchlorate ion, a hypochlorite ion, a chromate ion, an aluminosilicate ion, an acetate ion, a formate ion, a hydrogen oxalate ion or a hydrosulfide ion.

As a preferred technical solution, a molar ratio of the chiral pyridinium salt carbonyl catalyst with an iodide ion to the silver salt is 1:1, with a reaction temperature of room temperature and a reaction time of 2 h.

One of the technical solutions of the present invention consists in providing application of the chiral pyridinium salt carbonyl catalyst, wherein the pyridinium salt carbonyl catalyst 1 is applied to an asymmetric biomimetic aldol reaction of an amino acid derivative and a carbonyl compound to synthesize chiral β-hydroxy-α-amino acid compounds (chiral β-hydroxy-α-amino acid and derivatives thereof), and the reaction comprises the steps of:
carrying out an aldol reaction on an amino acid derivative **2** and a carbonyl compound **3** in a solvent under catalysis of the pyridinium salt carbonyl catalyst **1** in the presence of an base and an additive to produce a chiral β-hydroxy-α-amino acid compound **4,** wherein different configurations such as *syn-4A, anti*-**4A** and *syn-**4B,** anti*-**4B**, are comprised, and an absolute configuration of the pyridinium salt carbonyl catalyst **1** determines an absolute configuration of the chiral β-hydroxy-α-amino acid compound 4, with reaction equations as follows: 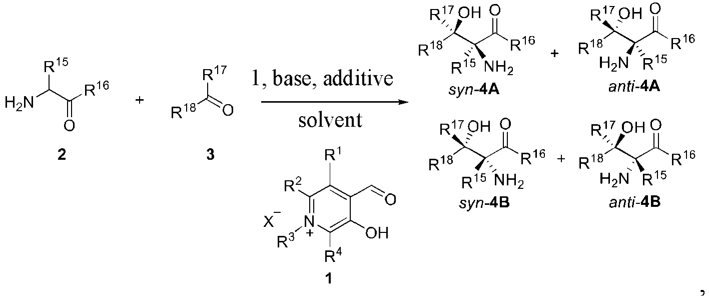
wherein R¹⁵ comprises hydrogen, substituted or unsubstituted C₁₋₂₄ alkyl, substituted or unsubstituted C₃-C₁₈ aryl, substituted or unsubstituted C₂₋₁₈ alkenyl, substituted or unsubstituted C₂₋₂₄ alkynyl, or C₁₋₁₈ carbonyl, the substituent comprises halogen, cyano, hydroxyl, nitro, C₁₋₁₈ alkyl, C₃₋₁₈ aryl, C₁₋₁₈ carbonyl, C₁₋₁₈ sulfonyl or phosphoryl, C₁₋₁₈ alkoxy, C₃₋₁₈ aryloxy or C₁₋₁₈ amino, the carbonyl comprises aldehyde group, ketone carbonyl, ester carbonyl, carboxyl or amido;
R¹⁶ is OR¹⁹ or NR²⁰R²¹, wherein R¹⁹ comprises hydrogen, substituted or unsubstituted C₁₋₂₄ alkyl, substituted or unsubstituted C₃₋₁₈ aryl, or C₁₋₁₈ carbonyl, R²⁰ and R²¹ each comprise hydrogen, substituted or unsubstituted C₁₋₂₄ alkyl, substituted or unsubstituted C₃₋₁₈ aryl, or C₁₋₁₈ carbonyl, or R²⁰ and R²¹ are linked together to form a ring B or NR²⁰R²¹ comprises amino acid amino, amino acid ester amino, amino acid amide amino or polypeptide amino, the substituent comprises halogen, cyano, hydroxyl, nitro, C₁₋₁₈ alkyl, C₃₋₁₈ aryl, C₁₋₁₈ carbonyl, C₁₋₁₈ sulfonyl or phosphoryl, C₁₋₁₈ alkoxy, C₃₋₁₈ aryloxy or C₁₋₁₈ amino, and the carbonyl comprises aldehyde group, ketone carbonyl, ester carbonyl, carboxyl or amido, the ring B is a C₁₋₁₈ ring with 1-4 heteroatoms, which comprise O, N or S atoms;
R¹⁷ and R¹⁸ each comprise hydrogen, substituted or unsubstituted C₁₋₂₄ alkyl, substituted or unsubstituted C₃₋₁₈ aryl, substituted or unsubstituted C₂₋₁₈ alkenyl, substituted or unsubstituted C₂₋₂₄ alkynyl, the substituent comprises halogen, cyano, hydroxyl, nitro, trifluoromethyl, C₁₋₁₈ alkyl, C₃₋₁₈ aryl, C₁₋₁₈ carbonyl, C₁₋₁₈ sulfonyl or phosphoryl, C₁₋₁₈ alkoxy, C₃₋₁₈ aryloxy or C₁₋₁₈ amino, 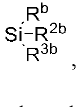 and the carbonyl comprises aldehyde group, ketone carbonyl, ester carbonyl, carboxyl or amido;
R^{b}, R^{2b} and R^{3b} each comprise hydrogen, C₁₋₁₀ alkyl or aryl, m is 1, 2 or 3, and n is 0, 1, 2, 3, 4 or 5.

Further, a molar ratio of the amino acid derivative 2 to the carbonyl compound 3 is (0.5 to 5):1, and the molar ratio of the pyridinium salt carbonyl catalyst 1 to the carbonyl compound 3 is (0.00001 to 0.5):1, with a reaction temperature of -60°C to 100°C and a reaction time of 1 h to 72 h;
a solvent used in the reaction is selected from one or more of water, methanol, ethanol, isopropanol, n-propanol, n-butanol, tert-butanol, 1,4-dioxane, trifluoroethanol, benzene, toluene, xylene, trimethylbenzene, acetonitrile, ethyl ether, tetrahydrofuran, ethylene glycol dimethyl ether, chloroform, dichloromethane, N,N-dimethylformamide, *N,N-*dimethylacetamide, dimethyl sulfoxide, and N-methylpyrrolidone;
a base used is selected from one or more lithium hydroxide, sodium hydroxide, potassium hydroxide, sodium carbonate, potassium carbonate, cesium carbonate, sodium bicarbonate, potassium bicarbonate, sodium phosphate, potassium phosphate, disodium hydrogen phosphate, dipotassium hydrogen phosphate, sodium hydride, potassium hydride, calcium hydride, trimethylamine, triethylamine, diisopropylamine, diisopropylethylamine, tetramethylethylenediamine, *N,N-diethylmethylamine, N,N-*dimethylethylamine, *N,N*-dimethylpropylamine, *N,N*-dimethylbutylamine, *N,N-*dimethylaniline, *N,N*-diethylaniline, 1,4-diazabicyclooctane (DABCO), 1,8-diazabicyclo[5.4.0]undec-7-ene (DBU), 1,5-diazabicyclo[4.3.0]-5-nonene (DBN), n-butyl lithium, 1,4-dimethylpiperazine, 1-methylpiperidine, 1-methylpyrrole, n-butylamine, tert-butylamine, diethylamine, ethylenediamine, quinoline and pyridine;
an additive used is selected from one or more of ammonium phosphate, ammonium dihydrogen phosphate, diammonium hydrogen phosphate, sodium phosphate, sodium dihydrogen phosphate, disodium hydrogen phosphate, potassium phosphate, potassium dihydrogen phosphate, dipotassium hydrogen phosphate, lithium phosphate, lithium dihydrogen phosphate, dilithium hydrogen phosphate, sodium carbonate, sodium bicarbonate, potassium carbonate, potassium bicarbonate, sodium acetate, potassium acetate, potassium fluoride and sodium fluoride.

In any one of the above technical solutions, the C₁₋₂₄ alkyl is selected from one or more of methyl, ethyl, n-propyl, isopropyl, cyclopropyl, n-butyl, tert-butyl, isobutyl, cyclobutyl, n-pentyl, isopentyl, 3-pentyl, cyclopentyl, n-hexyl, 2-hexyl, 3-hexyl, cyclohexyl, n-heptyl, 2-heptyl, 3-heptyl, 4-heptyl, cycloheptyl, n-octyl, 2-octyl, 3-octyl, 4-octyl, cyclooctyl, n-nonyl, 2-nonyl, 3-nonyl, 4-nonyl, 5-nonyl, cyclononyl, n-decyl, 2-decyl, 3-decyl, 4-decyl, 5-decyl, cyclodecyl, n-undecyl, cycloundecyl, n-dodecyl, cyclododecyl, n-tridecyl, cyclotridecyl, n-tetradecyl, cyclotetradecyl, n-pentadecyl, cyclopentadecyl, n-hexadecyl, cyclohexadecyl, n-heptadecyl, cycloheptadecyl, **n-**octadecyl, cyclooctadecyl, n-nonadecyl, cyclononadecyl, n-eicosyl, cycloeicosyl, **n-**heneicosyl , cycloheneicosyl , n-docosyl, cyclodocosyl, n-tricosyl, cyclotricosyl, **n-**tetracosyl, and cyclotetracosyl.

**In** any one of the above technical solutions, the C₁₋₁₈ alkyl is selected from one or more of methyl, ethyl, n-propyl, isopropyl, cyclopropyl, n-butyl, tert-butyl, isobutyl, cyclobutyl, n-pentyl, isopentyl, 3-pentyl, cyclopentyl, n-hexyl, 2-hexyl, 3-hexyl, cyclohexyl, n-heptyl, 2-heptyl, 3-heptyl, 4-heptyl, cycloheptyl, n-octyl, 2-octyl, 3-octyl, 4-octyl, cyclooctyl, n-nonyl, 2-nonyl, 3-nonyl, 4-nonyl, 5-nonyl, cyclononyl, n-decyl, 2-decyl, 3-decyl, 4-decyl, 5-decyl, cyclodecyl, n-undecyl, cycloundecyl, n-dodecyl, cyclododecyl, n-tridecyl, cyclotridecyl, n-tetradecyl, cyclotetradecyl, n-pentadecyl, cyclopentadecyl, n-hexadecyl, cyclohexadecyl, n-heptadecyl, cycloheptadecyl, **n-**octadecyl, and cyclooctadecyl.

**In** any one of the above technical solutions, the C₁₋₁₀ alkyl is selected from one or more of methyl, ethyl, n-propyl, isopropyl, cyclopropyl, n-butyl, tert-butyl, isobutyl, cyclobutyl, n-pentyl, isopentyl, 3-pentyl, cyclopentyl, n-hexyl, 2-hexyl, 3-hexyl, cyclohexyl, n-heptyl, 2-heptyl, 3-heptyl, 4-heptyl, cycloheptyl, n-octyl, 2-octyl, 3-octyl, 4-octyl, cyclooctyl, n-nonyl, 2-nonyl, 3-nonyl, 4-nonyl, 5-nonyl, cyclononyl, n-decyl, 2-decyl, 3-decyl, 4-decyl, 5-decyl, and cyclodecyl.

**In** any one of the above technical solutions, the C₃₋₁₈ aryl is selected from one or more of 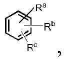 1-naphthyl, 2-naphthyl, 9-anthryl, 10-anthryl, 9-phenanthryl, 10-phenanthryl, 1-pyrenyl, 2-pyrenyl, 5-tetracenyl, 2-perylene, 2-oxazole, 4-oxazole, 5-oxazole, 2-thiazole, 4-thiazole, 5-thiazole, 2-furyl, 3-furyl, 2-pyrryl, 3-pyrryl, 2-thienyl, 3-thienyl, 2-imidazolyl, 2-oxazolyl, 2-thiazolyl, 2-pyridyl, 3-pyridyl, 4-pyridyl, 2-pyrimidinyl, 2-pyrazinyl, 3-pyrazinyl, 2-indolyl, 3-indolyl, 2-quinolyl, 3-quinolyl, 4-quinolyl, and benzo-fused heteroaryl of the above heteroaryl cyclic groups, wherein R^{a}, R^{b} and R^{c} are each independently selected from one or more of hydrogen, methyl, ethyl, n-propyl, isopropyl, cyclopropyl, n-butyl, tert-butyl, cyclobutyl, n-pentyl, cyclopentyl, n-hexyl, cyclohexyl, trifluoromethyl, phenyl, halogen, methoxy, ethoxy, dimethylamino, diethylamino, and nitro.

In any one of the above technical solutions, the C₂₋₁₈ alkenyl is selected from one or more of prop-1-en-1-yl, prop-2-en-1-yl, but-1-en-1-yl, but-2-en-1-yl, but-3-en-1-yl, pent-1-en-1-yl, pent-2-en-1-yl, pent-3-en-1-yl, pent-4-en-1-yl, hex-1-en-1-yl, hex-2-en-1-yl, hex-3-en-1-yl, hex-4-en-1-yl, hex-5-en-1-yl, hept-1-en-1-yl, hept-2-en-1-yl, hept-3-en-1-yl, hept-4-en-1-yl, hept-5-en-1-yl, hept-6-en-1-yl, oct-1-en-1-yl, oct-2-en-1-yl, oct-3-en-1-yl, oct-4-en-1-yl, oct-5-en-1-yl, oct-6-en-1-yl, oct-7-en-1-yl, non-1-en-1-yl, non-2-en-1-yl, non-3-en-1-yl, non-4-en-1-yl, non-5-en-1-yl, dec-1-en-1-yl, dec-2-en-1-yl, dec-3-en-1-yl, dec-4-en-1-yl, dec-5-en-1-yl, undec-1-en-1-yl, dodec-1-en-1-yl, tridec-1-en-1-yl, tetradec-1-en-1-yl, pentadec-1-en-1-yl, hexadec-1-en-1-yl, heptadec-1-en-1-yl, and octadec-1-en-1-yl.

In any one of the above technical solutions, the C₂₋₂₄ alkynyl is selected from one or more of ethynyl, prop-1-yn-1-yl, prop-2-yn-1-yl, but-1-yn-1-yl, but-2-yn-1-yl, but-3-yn-1-yl, pent-1-yn-1-yl, pent-2-yn-1-yl, pent-3-yn-1-yl, pent-4-yn-1-yl, hex-1-yn-1-yl, hex-2-yn-1-yl, hex-3-yn-1-yl, hex-4-yn-1-yl, hex-5-yn-1-yl, hept-1-yn-1-yl, hept-2-yn-1-yl, hept-3-yn-1-yl, hept-4-yn-1-yl, hept-5-yn-1-yl, hept-6-yn-1-yl, oct-1-yn-1-yl, oct-2-yn-1-yl, oct-3-yn-1-yl, oct-4-yn-1-yl, oct-5-yn-1-yl, oct-6-yn-1-yl, oct-7-yn-1-yl, non-1-yn-1-yl, non-2-yn-1-yl, non-3-yn-1-yl, non-4-yn-1-yl, non-5-yn-1-yl, dec-1-yn-1-yl, dec-2-yn-1-yl, dec-3-yn-1-yl, dec-4-yn-1-yl, dec-5-yn-1-yl, undec-1-yn-1-yl, dodec-1-yn-1-yl, tridec-1-yn-1-yl, tetradec-1-yn-1-yl, pentadec-1-yn-1-yl, hexadec-1-yn-1-yl, heptadec-1-yn-1-yl, and octadec-1-yn-1-yl.

In any one of the above technical solutions, the C₁₋₁₀ alkoxy is selected from one or more of methoxy, methylene 1,1-dioxy 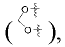 ethyl 1,2-dioxy 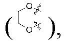 ethoxy, n-propoxy, isopropoxy, cyclopropyloxy, n-butoxy, tert-butoxy, isobutoxy, cyclobutoxy, cyclopentyloxy, n-pentyloxy, isopentyloxy, n-hexyloxy, cyclohexyloxy, n-heptyloxy, cycloheptyloxy, n-octyloxy, cyclooctyloxy, n-nonyloxy, cyclononyloxy, n-decyloxy, cyclodecyloxy, n-undecyloxy, cycloundecyloxy, n-undecyloxy, and cyclododecyloxy.

**In** any one of the above technical solutions, the halogen is selected from one or more of fluorine, chlorine, bromine and iodine.

The present invention relates to a new class of chiral pyridinium salt carbonyl catalysts with a biaryl structure, synthesis methods for this class of catalysts and uses thereof. This class of catalysts can be used in the biomimetic aldol reaction to synthesize a series of chiral β-hydroxy-α- amino acid compounds, providing a new method for asymmetric synthesis of β-hydroxy-α-amino acid compounds. The biomimetic aldol reaction has mild conditions, and is easy to operate, good in reproducibility and characterized by extremely high dr and ee values as well as high to extremely high yields.

Compared with the prior art, the present invention has the following advantageous effects.
(1) Pyridoxal, a member of the vitamin B6 family, is a coenzyme for a variety of biological enzymes in living organisms, and acts as a reaction center to catalyze numerous biotransformations. Pyridoxal-catalyzed amino acid derivatives are of great synthetic significance for the reaction to prepare β-hydroxy-α-amino acid compounds by the addition of carbonyl compounds. However, the biomimetic mimicry of this reaction has not been achieved successfully for a long time. In the present invention, a new class of chiral pyridinium salt carbonyl catalysts are designed and synthesized to catalyze the biomimetic aldol reaction of the amino acid derivatives with carbonyl compounds, with high activity and high stereoselectivity, which achieves the efficient synthesis of the chiral β-hydroxy-α-amino acid compounds.
(2) The chiral pyridinium salt carbonyl catalysts of the present invention can be prepared from readily available starting materials at low cost by multi-step reactions, which have mild conditions and are mostly easy to scale up and applicable to large-scale preparation.
(3) The chiral pyridinium salt carbonyl catalysts of the present invention catalyze the biomimetic aldol reaction under very mild conditions, with stability, operator friendliness, excellent product stereoselectivity and yield, making it one of the most efficient preparation methods for optically active β-hydroxy-α-amino acid compounds.
(4) The chiral β-hydroxy-α-amino acid compounds prepared through the biomimetic aldol reaction catalyzed by the chiral pyridinium salt carbonyl catalysts of the present invention can be applied as important intermediates to the synthesis of medicaments such as florfenicol, droxidopa and eliglustat, providing a new green synthetic process for the synthesis of these pharmaceutical molecules.

### DETAILED DESCRIPTION OF THE INVENTION

The present invention will be explained in detail below in combination with specific embodiments. These embodiments are implemented on the premise of the technical solutions of the present invention and provide the detailed implementations and specific operational processes, but the protection scope of the present invention is not limited to the embodiments as follows.

In the following embodiments, the devices used indicate conventional devices in the art, unless otherwise specified; the reagents used indicate commercially available products or are prepared using the conventional methods in the art, unless otherwise specified; and the embodiments below that are not explained in detail can be implemented using the conventional experimental means in the art.

In these embodiments, the structural formula of the chiral pyridinium salt carbonyl catalyst 1 and the reaction equations for preparing the β-hydroxy-α-amino acid compound 4 from the chiral pyridinium salt carbonyl catalyst 1 are as follows:

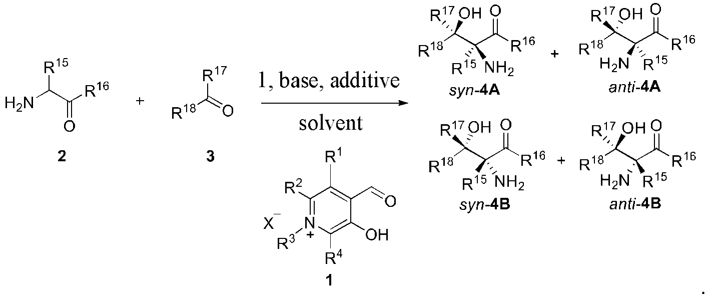

The method in these embodiments can be further exemplified by the process of preparing a representative compound as follows:

### Embodiments 1 to 9:

Catalyst (*S,S*)-**1a** and preparation method therefor, with the reaction equation as follows:

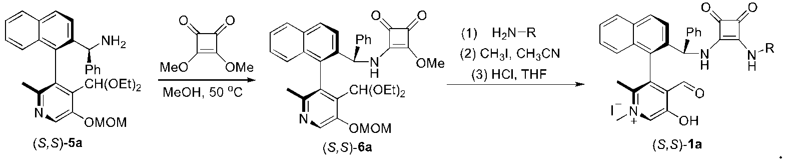

The general steps were as follows:
A compound (*S,S*)-**5a** (1.0 g, 2.06 mmol) and dimethyl squarate (1.46 g, 10.3 mmol) were dissolved in anhydrous methanol (10 mL) and stirred at 50 °C overnight. The reaction mixture was concentrated, and the residue was purified by column chromatography to afford a compound (*S*,*S*)-**6a** as a colorless oil (0.75 g, 61% yield).

A compound (*S,S*)-**6a** (0.15 g, 0.25 mmol) and a primary amine compound (1.38 mmol) were dissolved in anhydrous ethanol (1.5 mL) and stirred at 42 °C for 48 h, which was then concentrated and purified by column chromatography to afford the squaramide intermediate. To the squaramide intermediate in CH₃CN (1.5 mL) was added CH₃I (20 eq. of the squaramide intermediate). Upon stirring at 30 °C for 8 h, the reaction mixture was concentrated and purified by column chromatography to afford the quaternary ammonium salt intermediate. To a solution of the quaternary ammonium salt in tetrahydrofuran (1.5 mL) was added hydrochloric acid (1.0 M, 2.0 mL). Upon stirring at 55 °C for 6 h, the mixture was concentrated to remove THF and dried in the presence of phosphorus pentoxide under vacuum. Then the solid was washed with a mixed solution of Et₂O and THF, and then dried under vacuum to afford a catalyst (*S,S*)-**1a**.

### Embodiment 1:

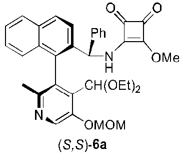

This compound had the following basic parameters: (*S,S*)-**6a**: colorless oil; [α]_{D}²⁵ = 4.1 (c = 0.10, CHCl₃); ¹H NMR (400 MHz, CDCl₃) δ 8.54 (s, 1H), 7.86-7.82 (m, 2H), 7.56 (s, 1H), 7.46 (t, *J* = 7.6 Hz, 1H), 7.36-7.31 (m, 3H), 7.29-7.28 (m, 2H), 7.20 (d, J= 8.0 Hz, 1H), 7.14 (d, *J* = 6.4 Hz, 2H), 5.75 (s, 1H), 5.43 (s, 1H), 5.29 (s, 2H), 4.10 (s, 3H), 3.59 (s, 3H), 3.45-3.29 (m, 3H), 3.12-3.04 (m, 1H), 1.86 (s, 3H), 0.91 (t, *J=* 6.8 Hz, 3H), 0.65-0.48 (m, 3H); ¹³C NMR (100 MHz, CDCl₃) δ 189.0, 183.8, 177.8, 171.7, 151.7, 150.0, 140.2, 138.1, 135.8, 135.3, 134.1, 132.8, 132.4, 131.0, 128.9, 128.7, 128.1, 128.0, 127.6, 126.7, 126.5, 126.4, 124.5, 99.4, 96.2, 64.7, 64.2, 60.3, 56.6, 22.5, 15.1, 14.5.

### Embodiment 2:

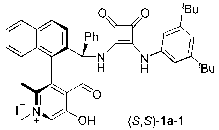

This catalyst had the following basic parameters: (*S*,*S*)-**1a-1**: brownish-yellow solid, 71% yield; [α]_{D}²⁵ = -16.3 (c = 0.050, MeOH); ¹H NMR (400 MHz, DMSO-*d*₆) δ 10.32 (s, 1H), 9.89 (s, 1H), 9.33 (d, *J* = 10.0 Hz, 1H), 8.92 (s, 1H), 8.21 (d, *J=* 8.8 Hz, 1H), 8.07 (d, *J* = 8.4 Hz, 1H), 7.65 (d, *J=* 8.4 Hz, 1H), 7.61 (t, *J=* 8.0 Hz, 1H), 7.49 (t, *J* = 8.4 Hz, 1H), 7.45-7.38 (m, 2H), 7.38-7.31 (m, 4H), 7.20 (d, *J* = 6.8 Hz, 2H), 7.06 (t, *J* = 1.6 Hz, 1H), 6.23 (d, *J=* 9.6 Hz, 1H), 4.28 (s, 3H), 1.81 (s, 3H), 1.26 (s, 18H); ¹³C NMR (100 MHz, DMSO-*d*₆) δ 188.9, 182.9, 179.9, 167.0, 164.5, 155.2, 151.6, 145.6, 140.0, 138.2, 137.6, 136.9, 136.1, 133.4, 132.3, 131.3, 130.4, 129.1, 128.4, 128.1, 127.8, 127.1, 127.0, 125.2, 124.3, 116.7, 112.6, 58.2, 47.4, 34.7, 31.2, 15.9.

### Embodiment 3:

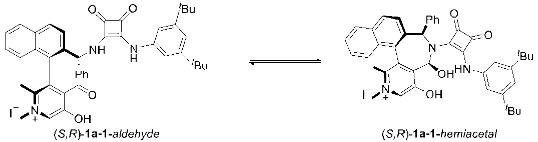

This catalyst had the following basic parameters: (*S*,*R*)-**1a-1**, brownish-yellow solid, 50% yield; [α]_{D}²⁵ = 61.5 (c = 0.050, MeOH); ¹H NMR (400 MHz, DMSO-*d*₆) δ 10.64 (s, 0.54H for **1a-1***-aldehyde),* 9.78 (brs, 0.46H for **1a-1***-hemiacetal),* 9.58 (d, *J = 9.2* Hz, 0.54H for **1a-1***-aldehyde),* 9.48 (s, 0.54H for **1a-1***-aldehyde*), 8.97 (s, 0.54H for **1a-1-***aldehyde),* 8.31 (s, 0.46H for **1a-1***-hemiacetal*), 8.25 (d, *J* = 8.4 Hz, 0.46H for **1a-1-***hemiacetal),* 8.21 (d, *J* = 8.8 Hz, 0.54H for **1a-1***-aldehyde),* 8.14 (d, *J=* 8.0 Hz, 0.46H for **1a-1***-hemiacetal*), 8.08 (d, *J=* 8.4 Hz, 0.54H for **1a-1***-aldehyde*), 7.70 (d, *J=* 8.8 Hz, 0.54H for **1a-1***-aldehyde*), 7.68-7.58 (m, 1H), 7.58-7.52 (m, 0.92H for **1a-1***-hemiacetal*), 7.52-7.46 (m, 0.54H for **1a-1*****-**aldehyde*), 7.46-7.27 (m, 4H), 7.21-7.12 (m, 2H), 7.12-7.02 (m, 3H), 6.81 (d, *J=* 6.4 Hz, 0.92H for **1a-1***-hemiacetal*), 6.14 (d, *J=* 9.4 Hz, 0.54H for **1a-1***-aldehyde*), 4.31 (s, 1.62H for **1a-1***-aldehyde*), 3.95 (s, 1.38H for **1a-1-***hemiacetal),* 2.11 (s, 1.62H for **1a-1***-aldehyde*), 1.83 (s, 1.38H for **1a-1***-hemiacetal*), 1.27 (s, 8.28H for **1a-1***-hemiacetal*), 1.26 (s, 9.72H for **1a-1***-aldehyde*); ¹³C NMR (100 MHz, DMSO-*d*₆) δ 188.7, 183.8, 182.9, 182.0, 179.8, 166.9, 164.7, 155.2, 151.6, 150.5, 146.0, 145.5, 140.4, 139.9, 138.3, 137.5, 137.1, 136.8, 136.0, 133.7, 132.9, 132.5, 132.2, 131.2, 130.9, 130.3, 129.0, 128.8, 128.7, 128.4, 127.9, 127.7, 127.5, 127.0, 126.7, 126.5, 125.4, 125.3, 124.5, 124.2, 116.6, 116.2, 112.6, 72.6, 58.3, 54.9, 47.5, 46.3, 34.7, 34.6, 31.2, 17.7, 16.7.

### Embodiment 4:

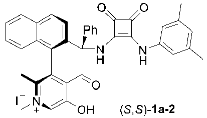

This catalyst had the following basic parameters: (*S*,*S*)-**1a-2**: brownish-yellow solid, 64% yield; [α]_{D}²⁵ = 17.3 (c = 0.050, MeOH); ¹H NMR (400 MHz, DMSO-*d*₆) δ 10.61 (s, 1H), 9.87 (s, 1H), 9.73 (d, *J* = 9.6 Hz, 1H), 8.92 (s, 1H), 8.20 (d, *J* = 8.4 Hz, 1H), 8.06 (d, *J=* 8.0 Hz, 1H), 7.73 (d, *J =* 8.8 Hz, 1H), 7.60 (t, *J=* 7.6 Hz, 1H), 7.48 (dd, *J* = 8.4, 6.8 Hz, 1H), 7.43-7.38 (m, 2H), 7.35 (d, *J* = 7.2 Hz, 1H), 7.31 (d, *J* = 8.4 Hz, 1H), 7.20 (d, *J* = 7.2 Hz, 2H), 7.12 (s, 2H), 6.66 (s, 1H), 6.25 (d, *J* = 9.6 Hz, 1H), 4.27 (s, 3H), 2.22 (s, 6H), 1.76 (s, 3H); ¹³C NMR (100 MHz, DMSO-*d*₆) δ 188.9, 183.0, 179.7, 167.0, 164.3, 155.0, 145.6, 139.9, 138.8, 138.4, 137.5, 136.8, 136.1, 133.4, 132.2, 131.3, 130.4, 129.0, 128.8, 128.4, 128.0, 127.8, 127.2, 127.0, 125.1, 124.5, 124.4, 115.8, 58.4, 47.4, 21.1, 15.9.

### Embodiment 5:

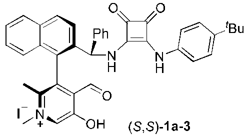

This catalyst had the following basic parameters: (*S*,*S*)-**1a-3**, brownish-yellow solid, 58% yield; [α]_{D}²⁵ = 21.8 (c = 0.050, MeOH); ¹H NMR (400 MHz, DMSO-*d*₆) δ 10.30 (s, 1H), 9.88 (s, 1H), 9.33 (d, *J* = 9.6 Hz, 1H), 8.90 (s, 1H), 8.20 (d, *J* = 8.8 Hz, 1H), 8.06 (d, *J* = 8.0 Hz, 1H), 7.65-7.58 (m, 2H), 7.52-7.45 (m, 1H), 7.45-7.39 (m, 3H), 7.38-7.32 (m, 5H), 7.20 (d, *J=* 7.2 Hz, 2H), 6.24 (d, *J=* 9.6 Hz, 1H), 4.28 (s, 3H), 1.81 (s, 3H), 1.25 (s, 9H); ¹³C NMR (100 MHz, DMSO-*d*₆) δ 188.9, 182.9, 179.8, 166.8, 164.2, 155.1, 145.5, 145.4, 139.8, 137.6, 136.8, 136.3, 136.0, 133.4, 132.3, 131.3, 130.4, 129.1, 129.0, 128.4, 128.0, 127.8, 127.1, 127.0, 126.0, 125.2, 124.3, 117.9, 58.2, 47.4, 34.0, 31.2, 15.9.

### Embodiment 6:

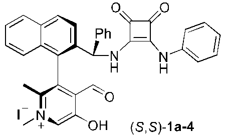

This catalyst had the following basic parameters: (*S*,*S*)-**1a-4**, brownish-yellow solid, 60% yield; [α]_{D}²⁵ = 31.5 (c = 0.050, MeOH); ¹H NMR (400 MHz, DMSO-*d*₆) δ 10.54 (s, 1H), 9.88 (s, 1H), 9.54 (d, *J* = 9.6 Hz, 1H), 8.91 (s, 1H), 8.21 (d, *J* = 8.8 Hz, 1H), 8.06 (d, *J=* 8.4 Hz, 1H), 7.69 (d, *J=* 8.4 Hz, 1H), 7.61 (t, *J=* 7.6 Hz, 1H), 7.52-7.45 (m, 3H), 7.45-7.38 (m, 2H), 7.38-7.30 (m, 4H), 7.20 (d, *J= 7.2* Hz, 2H), 7.02 (t, *J= 7.2* Hz, 1H), 6.24 (d, *J=* 9.6 Hz, 1H), 4.27 (s, 3H), 1.79 (s, 3H); ¹³C NMR (100 MHz, DMSO-*d*₆) δ 188.9, 183.1, 179.7, 167.1, 164.2, 155.1, 145.6, 139.8, 138.9, 137.6, 136.8, 136.0, 133.4, 132.3, 131.3, 130.4, 129.3, 129.0, 128.9, 128.4, 128.0, 127.8, 127.1, 127.0, 125.2, 124.3, 122.9, 118.0, 58.3, 47.4, 15.9.

### Embodiment 7:

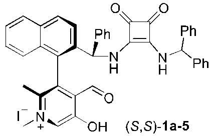

This catalyst had the following basic parameters: (*S*,*S*)-**1a-5**, brownish-yellow solid, 54% yield; [α]_{D}²⁵ = -28.0 (c = 0.050, MeOH); ¹H NMR (400 MHz, DMSO-*d*₆) δ 9.85 (s, 1H), 9.21 (d, *J=* 9.6 Hz, 1H), 9.07 *(d, J=* 9.6 Hz, 1H), 8.87 (s, 1H), 8.17 (d, *J=* 8.8 Hz, 1H), 8.05 (d, *J* = 8.0 Hz, 1H), 7.64-7.57 (m, 2H), 7.47 (ddd, *J* = 8.4, 6.8, 1.2 Hz, 1H), 7.41-7.26 (m, 14H), 7.14 (d, *J* = 7.6 Hz, 2H), 6.36 (d, *J* = 9.6 Hz, 1H), 6.17 (d,*J* = 9.6 Hz, 1H), 4.25 (s, 3H), 1.76 (s, 3H); ¹³C NMR (100 MHz, DMSO-*d*₆) δ 188.9, 183.0, 179.7, 167.0, 164.3, 155.0, 145.6, 139.9, 138.8, 138.4, 137.5, 136.8, 136.1, 133.4, 132.2, 131.3, 130.4, 129.0, 128.8, 128.4, 128.0, 127.8, 127.2, 127.0, 125.1, 124.5, 124.4, 115.8, 58.4, 47.4, 21.1, 15.9.

### Embodiment 8:

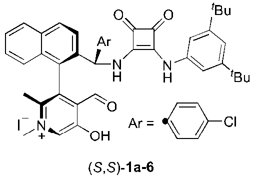

This catalyst had the following basic parameters: (*S*,*S*)-**1a-6**, brownish-yellow solid, 51% yield; [α]_{D}²⁵ = 105.8 (c = 0.050, MeOH); ¹H NMR (400 MHz, DMSO-*d*₆) δ 10.35 (s, 1H), 9.86 (s, 1H), 9.38 (d, *J=* 9.6 Hz, 1H), 8.92 (s, 1H), 8.20 (d, *J* = 8.8 Hz, 1H), 8.07 (d, *J* = 8.0 Hz, 1H), 7.65-7.57 (m, 2H), 7.52-7.47 (m, 3H), 7.37-7.31 (m, 3H), 7.26 (d, *J=* 8.4 Hz, 2H), 7.06 (t, *J=* 1.6 Hz, 1H), 6.23 (d, *J= 9.6* Hz, 1H), 4.33 (s, 3H), 1.96 (s, 3H), 1.26 (s, 18H); ¹³C NMR (100 MHz, DMSO-*d*₆) δ 189.2, 183.3, 180.4, 167.3, 165.0, 155.7, 152.1, 145.8, 139.5, 138.7, 138.3, 136.9, 136.4, 133.8, 133.1, 132.8, 131.7, 131.0, 129.8, 129.4, 128.9, 128.3, 127.6, 125.8, 124.9, 117.1, 113.1, 58.0, 48.0, 35.2, 31.6, 16.6.

### Embodiment 9:

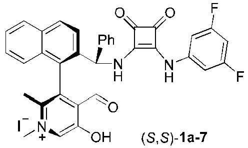

This catalyst had the following basic parameters: (S,5)-1a-7, brownish-yellow solid, 13% yield; ¹H NMR (400 MHz, DMSO-d6) δ 12.65 (s, 1H), δ 11.17 (s, 1H), 9.88 (s, 1H), 9.83 (d, *J* = 9.6 Hz, 1H), 8.92 (s, 1H), 8.22 (d, *J* = 8.6 Hz, 1H), 8.07 (d, *J = 8.0* Hz, 1H), 7.76 (d, *J* = 8.6 Hz, 1H), 7.61 (t, *J* = 7.6 Hz, 1H), 7.48 (t, *J* = 7.6 Hz, 1H), 7.45-7.15 (m, 10H), 6.85 (t, *J* = 9.4 Hz, 1H), 6.22 (d, *J* = 9.6 Hz, 1H), 4.26 (s, 3H), 1.72 (s, 3H); ¹³C NMR (100 MHz, DMSO-d6) δ 189.5, 184.1, 180.1, 168.0, 164.7, 164.6, 164.0, 162.3, 162.2, 155.5, 146.1, 142.3, 142.1, 140.1, 138.1, 137.2, 136.4, 133.9, 132.7, 131.7, 130.9, 129.5, 129.5, 129.2, 128.9, 128.6, 128.3, 127.9, 127.6, 127.5, 125.6, 124.7, 101.7, 101.6, 101.4, 98.3, 98.1, 97.8, 59.0, 47.9, 16.3; ¹⁹F NMR (376 MHz, DMSO-d6) δ - 108.53.

### Embodiments 10 to 14:

Catalyst (*S,S*)-**1a** and preparation method therefor, with the reaction equation as follows:

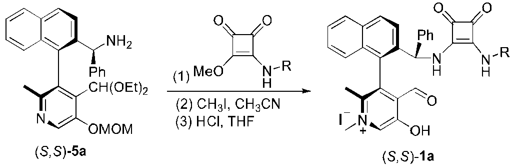

The general steps were as follows:
A compound (*S*,*S*}-**5a** (0.195 g, 0.40 mmol) and a squaramide compound (2.21 mmol) were dissolved in anhydrous ethanol (2.0 mL) and stirred at 42 °C for 72 h, which was then concentrated and purified by column chromatography to afford the squaramide intermediate. To the squaramide intermediate in CH₃CN (1.5 mL) was added CH₃I (20 eq. of the squaramide intermediate). Upon stirring at 30 °C for 8 h, the reaction mixture was concentrated and purified by column chromatography to afford the quaternary ammonium salt intermediate. To a solution of the quaternary ammonium salt in tetrahydrofuran (1.5 mL) was added hydrochloric acid (1.0 M, 2.0 mL). Upon stirring at 55 °C for 6 h, the mixture was concentrated to remove THF and dried in the presence of phosphorus pentoxide under vacuum. Then the solid was washed with a mixed solution of Et₂O and THF, and then dried under vacuum to afford a catalyst (*S*,*S*)-**1a**.

### Embodiment 10:

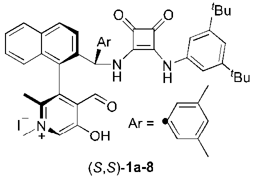

This catalyst had the following basic parameters: (*S,S*)-**1a-8**, brownish-yellow solid, 29% yield; [α]_{D}²⁵ = -6.8 (c = 0.050, MeOH); ¹H NMR (400 MHz, DMSO-*d*₆) δ 10.30 (s, 1H), 9.87 (s, 1H), 9.26 (d, *J* = 9.6 Hz, 1H), 8.93 (s, 1H), 8.20 (d, *J* = 8.8 Hz, 1H), 8.07 (d, *J=* 8.4 Hz, 1H), 7.67 (d, *J=* 8.8 Hz, 1H), 7.61 (t, *J=* 7.2 Hz, 1H), 7.51-7.45 (m, 1H), 7.38-7.33 (m, 2H), 7.32 (d, *J=* 8.8 Hz, 1H), 7.06 (s, 1H), 6.98 (s, 1H), 6.79 (s, 2H), 6.16 (d, *J=* 9.2 Hz, 1H), 4.28 (s, 3H), 2.25 (s, 6H), 1.83 (s, 3H), 1.27 (s, 18H); ¹³C NMR (100 MHz, DMSO-*d*₆) δ 188.9, 182.9, 179.9, 166.9, 164.4, 155.1, 151.6, 145.6, 139.9, 138.24, 138.19, 137.5, 137.1, 136.2, 133.4, 132.2, 131.3, 130.4, 129.4, 128.8, 128.4, 127.8, 127.0, 125.1, 124.7, 124.3, 116.6, 112.6, 58.2, 47.3, 34.7, 31.1, 20.9, 16.1.

### Embodiment 11:

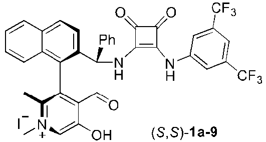

This catalyst had the following basic parameters: (*S,S*)-**1a-9,** brownish-yellow solid, 53% yield; [α]_{D}²⁵ = 30.8 (c = 0.050, MeOH); ¹H NMR (400 MHz, DMSO-*d*₆) δ 11.31 (s, 1H), 9.89 (s, 1H), 9.71 (d, *J* = 9.6 Hz, 1H), 8.91 (s, 1H), 8.22 (d, *J* = 8.4 Hz, 1H), 8.12 (s, 2H), 8.07 (d, *J=* 8.0 Hz, 1H), 7.74 (d, *J=* 8.8 Hz, 1H), 7.67 (s, 1H), 7.62 (t, *J* = 7.2 Hz, 1H), 7.49 (ddd, *J* = 8.4, 6.8, 1.2 Hz, 1H), 7.41 (t, *J=* 7.2 Hz, 2H), 7.36 (d, *J* = 7.2 Hz, 1H), 7.33 (d, *J= 8.4* Hz, 1H), 7.19 (d, *J=* 7.2 Hz, 2H), 6.23 (d, *J=* 9.6 Hz, 1H), 4.27 (s, 3H), 1.74 (s, 3H); ¹³C NMR (100 MHz, DMSO-*d*₆) δ 189.6, 184.4, 180.4, 168.2, 163.8, 155.6, 146.0, 141.6, 140.1, 138.2, 137.1, 136.4, 133.9, 132.8, 131.9 (q, *J*_{C}-_{F} *=* 32.8 Hz), 131.7, 130.9, 129.5, 129.3, 128.9, 128.6, 128.3, 127.6, 127.5, 125.7, 124.7, 123.6 (q, *J*_{C}-_{F} = 271.2 Hz), 118.3, 115.5, 59.0, 47.9, 16.4; ¹⁹F NMR (376 MHz, DMSO-*d*₆) δ -61.7.

### Embodiment 12:

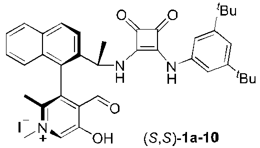

This catalyst had the following basic parameters: (*S*,*S*)-**1a-10**, brownish-yellow solid, 41% yield; [α]_{D}²⁵ = 3.0 (c = 0.050, MeOH); ¹H NMR (400 MHz, DMSO-*d*₆) δ 10.16 (s, 1H), 9.77 (s, 1H), 8.90 (s, 1H), 8.81 (d, *J= 9.6* Hz, 1H), 8.24 (d, *J= 8.8* Hz, 1H), 8.07 (d, *J=* 8.0 Hz, 1H), 7.96 (d, *J=* 8.8 Hz, 1H), 7.59 (t, *J= 7.6* Hz, 1H), 7.46 (d, *J* = 8.4 Hz, 1H), 7.38-7.29 (m, 2H), 7.25 (d, *J* = 8.4 Hz, 1H), 7.06-7.01 (m, 1H), 5.10 (dq, *J* = 9.6, 6.8 Hz, 1H), 4.41 (s, 3H), 2.18 (s, 3H), 1.56 (d, *J=* 6.8 Hz, 3H), 1.26 (s, 18H); ¹³C NMR (100 MHz, DMSO-*d*₆) δ 188.6, 182.9, 179.5, 167.0, 164.0, 155.1, 151.5, 145.7, 138.3, 137.9, 137.2, 135.9, 133.6, 132.3, 131.0, 130.5, 128.3, 128.1, 127.6, 126.7, 125.2, 123.3, 116.5, 112.5, 50.6, 47.5, 34.7, 31.1, 22.8, 16.3.

### Embodiment 13:

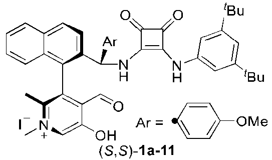

This catalyst had the following basic parameters: (*S*,*S*)-**1a-11**, brownish-yellow solid, 32% yield; [α]_{D}²⁵ = 16.5 (c = 0.050, MeOH); ¹H NMR (400 MHz, DMSO-*d*₆) δ 10.32 (s, 1H), 9.89 (s, 1H), 9.30 (d, *J* = 9.6 Hz, 1H), 8.91 (s, 1H), 8.21 (d, *J* = 8.8 Hz, 1H), 8.07 *(d, J=* 8.4 Hz, 1H), 7.71 (d, *J* = 8.4 Hz, 1H), 7.61 (t, *J= 7.6* Hz, 1H), 7.48 (t, *J* = 8.0 Hz, 1H), 7.34 (s, 2H), 7.31 *(d, J=* 8.4 Hz, 1H), 7.10 (d, *J=* 8.8 Hz, 2H), 7.06 (s, 1H), 6.96 (d, *J* = 8.8 Hz, 2H), 6.17 (d, *J* = 9.6 Hz, 1H), 4.27 (s, 3H), 3.75 (s, 3H), 1.76 (s, 3H), 1.26 (s, 18H); ¹³C NMR (100 MHz, DMSO-*d*₆) δ 189.0, 183.0, 180.0, 167.0, 164.4, 158.9, 155.1, 151.6, 145.6, 138.3, 137.5, 137.2, 136.1, 133.4, 132.2, 131.9, 131.3, 130.3, 128.7, 128.4, 128.4, 127.7, 126.9, 125.1, 124.2, 116.6, 114.3, 112.6, 57.8, 55.3, 47.4, 34.7, 31.1, 15.9.

### Embodiment 14:

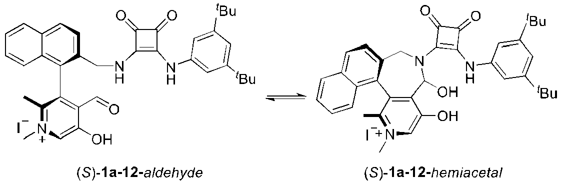

This catalyst had the following basic parameters: (*S*)-**1a-12**, brownish-yellow solid, 58% yield; [α]_{D}²⁵ = -71.3 (c = 0.050, MeOH); ¹H NMR (400 MHz, DMSO-*d*₆) δ 10.44 (s, 0.30H for **1a-12***-aldehyde*), 9.88 (s, 0.30H for **1a-12***-aldehyde*), 9.80 (s, 0.70H for **1a-12***-hemiacetal),* 8.89 (s, 0.30H for **1a-12***-aldehyde),* 8.77-8.65 (m, 1H), 8.17 (d, *J= 8.4* Hz, 0.30H for **1a-12***-aldehyde*), 8.11 (d, *J=* 8.4 Hz, 0.70H for **1a-12***-hemiacetal*), *8.07* (d, *J=* 7.6 Hz, 1H), 7.80 (d, *J=* 8.8 Hz, 0.30H for **1a-12***-aldehyde*), 7.69-7.53 (m, 0.60H for **1a-12***-aldehyde* and 3.50H for **1a-12***-hemiacetal*), 7.51-7.44 (m, 0.30H for **1a-12-***aldehyde),* 7.35 (s, 0.70H for **1a-12** *-hemiacetal),* 7.29 (d, *J* = 8.8 Hz, 0.30H for **1a-12-***aldehyde),* 7.23-7.10 (m, 2H), 7.05 (s, 0.30H for **1a-12***-aldehyde*), δ 4.67 (m, 0.60H for **1a-12***-aldehyde*), 4.52-4.13 (m, 1.40H for **1a-12***-hemiacetal*), 4.33 (s, 3H), 3.64 (brs, 0.7H for **1a-12***-hemiacetal*), 2.25 (s, 2.10H for **1a-12***-hemiacetal*), 2.14 (s, 0.90H for **1a-12***-aldehyde*), 1.28 (s, 12.60H for **1a-12***-hemiacetal*), 1.26 (s, 5.40H for **1a-12-***aldehyde);* ¹³C NMR (100 MHz, DMSO-*d*₆) δ 189.5, 183.1, 181.9, 179.8, 168.0, 167.6, 165.0, 164.2, 154.7, 151.5, 150.6, 150.1, 146.1, 145.9, 139.8, 138.4, 137.4, 137.1, 136.3, 136.1, 134.3, 133.9, 133.6, 132.9, 132.4, 132.3, 131.2, 130.3, 130.1, 128.9, 128.7, 128.4, 127.5, 127.3, 126.8, 126.7, 126.4, 125.7, 125.0, 117.5, 116.5, 115.7, 112.6, 73.1, 47.5, 46.9, 45.6, 34. 7, 34.6, 31.2, 18.3, 16.4.

### Embodiment 15:

Catalyst (*S,S*)-**1a-**13 and preparation method therefor, with the reaction equation as follows:

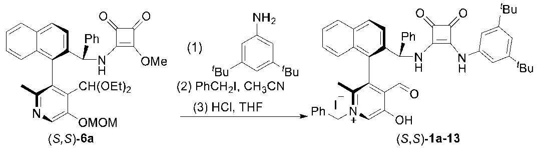

The steps are as follows: a compound (*S*,*S*)-**6a** (0.15 g, 0.25 mmol) and 3,5-ditert-butylaniline (0.28 g, 1.38 mmol) were dissolved in anhydrous ethanol (1.5 mL) and stirred at 42 °C for 48 h, which was then concentrated and purified by column chromatography to afford the squaramide intermediate. To the squaramide intermediate in CH₃CN (1.5 mL) was added an iodide (20 eq. of the squaramide intermediate). Upon stirring at 30 °C for 8 h, the reaction mixture was concentrated and purified by column chromatography to afford the quaternary ammonium salt intermediate. To a solution of the quaternary ammonium salt in tetrahydrofuran (1.5 mL) was added hydrochloric acid (1.0 M, 2.0 mL). Upon stirring at 55 °C for 6 h, the mixture was concentrated to remove THF and dried in the presence of phosphorus pentoxide under vacuum; then the solid was washed with a mixed solution of Et₂O and THF, and then dried under vacuum to afford a catalyst (*S,S*)-**1a-13** (brownish-yellow solid, 0.1501 g, 69% yield).

This compound had the following basic parameters: ¹H NMR (400 MHz, DMSO-*d*₆) δ 10.33 (s, 1H), 9.90 (s, 1H), 9.32 (d, *J=* 10.0 Hz, 1H), 8.93 (s, 1H), 8.22 (d, *J=* 8.8 Hz, 1H), 8.09 (d, *J=* 8.4 Hz, 1H), 7.63 (d, *J* = 8.4 Hz, 1H), 7.60 (t, *J* = 8.0 Hz, 1H), 7.50 (t, *J=* 8.4 Hz, 1H), 7.46-7.39 (m, 2H), 7.36-7.26 (m, 9H), 7.21 (d, *J=* 6.8 Hz, 2H), 7.05 (t, *J=* 1.6 Hz, 1H), 6.22 (d, *J=* 9.6 Hz, 1H), 5.88 (s, 2H), 1.82 (s, 3H), 1.25 (s, 18H).

### Embodiment 16:

Catalyst (*R,R*)-**1a-14** and preparation method therefor, with the reaction equation as follows:

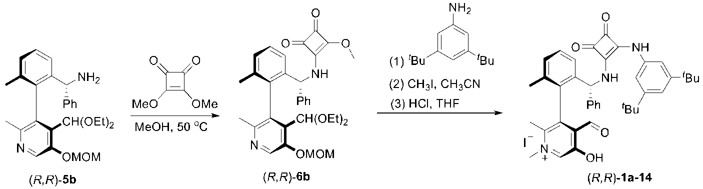

The specific steps were as follows: a compound (*R,R*)**-5b** (0.247 g, 0.55 mmol) and dimethyl squarate (0.560 g, 3.3 mmol) were dissolved in anhydrous methanol (2.5 mL) and stirred at 50 °C overnight. The reaction mixture was concentrated, and the residue was purified by column chromatography to afford a compound (*R,R*)**-6b** (colorless oil, 0.1417 g, 53% yield).

This compound had the following basic parameters: ¹H NMR (400 MHz, CDCl₃) δ 8.54 (s, 1H), 7.86-7.82 (m, 1H), 7.56 (s, 1H), 7.46 (t, *J* = 7.6 Hz, 1H), 7.36-7.31 (m, 1H), 7.29-7.28 (m, 2H), 7.20 (d, *J=* 8.0 Hz, 1H), 7.14 (d, *J* = 6.4 Hz, 2H), 5.75 (s, 1H), 5.43 (s, 1H), 5.29 (s, 2H), 4.10 (s, 3H), 3.59 (s, 3H), 3.45-3.29 (m, 3H), 3.12-3.04 (m, 1H), 2.0 (s, 3H), 1.86 (s, 3H), 0.91 (t, *J* = 6.8 Hz, 3H), 0.65-0.48 (m, 3H).

The steps further included: a compound (R,R)-6b (0.1417 g, 0.25 mmol), 3,5-ditert-butylaniline (0.4152 g, 2.02 mmol) and zinc trifluoromethanesulfonate (0.014 g, 0.038 mmol) were dissolved in anhydrous ethanol (1.5 mL) and stirred at 42 °C for 48 h, which was then concentrated and purified by column chromatography to afford the squaramide intermediate. To the squaramide intermediate (0.148 g, 0.21 mmol) in CH₃CN (1.5 mL) was added CH₃I (20 eq. of the squaramide intermediate). Upon stirring at 30 °C for 8 h, the reaction mixture was concentrated and purified by column chromatography to afford the quaternary ammonium salt intermediate. To a solution of the quaternary ammonium salt in tetrahydrofuran (1.5 mL) was added hydrochloric acid (1.0 M, 2.0 mL). Upon stirring at 55 °C for 6 h, the mixture was concentrated to remove THF and dried in the presence of phosphorus pentoxide under vacuum. Then the solid was washed with a mixed solution of Et₂O and THF, and then dried under vacuum to afford a catalyst (R,R)-**1a-14** (brownish-yellow solid, 0.1235 g, 65% yield).

This compound had the following basic parameters: ¹H NMR (400 MHz, DMSO-*d*₆) δ 10.19 (s, 1H), 10.02 (s, 1H), 9.05 (d, *J* = 9.6 Hz, 1H), 8.79 (s, 1H), 7.49 (t, *J=* 7.6 Hz, 1H), 7.44-7.33 (m, 4H), 7.32 *(d, J=* 1.6 Hz, 2H), 7.22 *(d, J=* 7.6 Hz, 1H), 7.14 (dd,*J* = 7.0, 1.6 Hz, 2H), 7.06 (t, *J* = 1.6 Hz, 1H), 5.97 (d, *J* = 9.6 Hz, 1H), 4.23 (s, 3H), 1.94 (s, 3H), 1.90 (s, 3H), 1.27 (s, 18H).

### Embodiment 17:

Catalyst (*S,S*)-**1a-15** and preparation method therefor, with the reaction equation as follows:

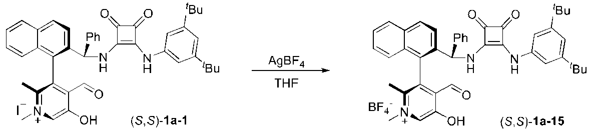

The specific steps were as follows: a compound (*S,S*)-**1a-1** (0.0476 g, 0.06 mmol) was dissolved in tetrahydrofuran (0.5 mL), and silver tetrafluoroborate (0.0117 g, 0.06 mmol) was dissolved in 0.5 mL of water and added to the reaction mixture. Upon stirring at room temperature for 2 h under nitrogen atmosphere, the mixture was concentrated to remove THF and dried in the presence of phosphorus pentoxide under vacuum. Then the solid was washed with a mixed solution of Et₂O and THF, and then dried under vacuum to afford a catalyst(*S*,*S*)-**1a-15** (brownish-yellow solid, 0.0407 g, yield 90%). This compound had the following basic parameters: ¹H NMR (400 MHz, DMSO-*d*₆) δ 10.32 (s, 1H), 9.89 (s, 1H), 9.33 (d, *J=* 10.0 Hz, 1H), 8.92 (s, 1H), 8.21 (d, *J=* 8.8 Hz, 1H), 8.07 (d, *J=* 8.4 Hz, 1H), 7.65 (d, *J=* 8.4 Hz, 1H), 7.61 (t, *J=* 8.0 Hz, 1H), 7.49 (t, *J= 8.4* Hz, 1H), 7.45-7.38 (m, 2H), 7.38-7.31 (m, 4H), 7.20 (d, *J* = 6.8 Hz, 2H), 7.06 (t, *J=* 1.6 Hz, 1H), 6.23 (d, *J=* 9.6 Hz, 1H), 4.28 (s, 3H), 1.81 (s, 3H), 1.26 (s, 18H). Embodiment 18:

Catalyst (*S*,*S*)-**1c** and preparation method therefor, with the reaction equation as follows:

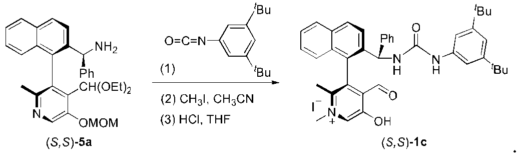

The specific steps were as follows: a compound (*S*,*S*)-**5a** (0.1463 g, 0.3 mmol) and 1,3-ditert-butyl-5-isocyanobenzene (2.0 mmol) were dissolved in DMF (1.5 mL). After stirring at room temperature for 24 h under nitrogen atmosphere, ethyl acetate (3 mL) was added, and the mixture was washed with water (2.5 mL × 3) and brine (2.5 mL). The combined organic layers were dried over anhydrous sodium sulfate. The mixture was concentrated and purified by column chromatography to afford the urea intermediate. To the urea intermediate in CH₃CN (1.5 mL) was added CH₃I (20 eq. of the squaramide intermediate). Upon stirring at 30 °C for 8 h, the reaction mixture was concentrated and purified by column chromatography to afford the quaternary ammonium salt intermediate. To a solution of the quaternary ammonium salt in tetrahydrofuran (1.5 mL) was added hydrochloric acid (1.0 M, 2.0 mL). Upon stirring at 55 °C for 6 h, the mixture was concentrated to remove THF and dried in the presence of phosphorus pentoxide under vacuum. Then the solid was washed with a mixed solution of Et₂O and THF, and then dried under vacuum to afford a catalyst (*S,S*)-**1c** (brownish-yellow solid, 0.0905 g, yield 49%).

This compound had the following basic parameters: ¹H NMR (400 MHz, DMSO-*d*₆) δ 10.31 (s, 1H), 9.90 (s, 1H), 9.32 (d, *J=* 10.0 Hz, 1H), 8.91 (s, 1H), 8.22 (d, *J=* 8.8 Hz, 1H), 8.07 (d, *J=* 8.4 Hz, 1H), 7.66 (d, *J* = 8.4 Hz, 1H), 7.62 (t, *J=* 8.0 Hz, 1H), 7.49 (t, *J= 8.4* Hz, 1H), 7.46-7.38 (m, 2H), 7.38-7.30 (m, 4H), 7.20 (d, *J* = 6.8 Hz, 2H), 7.07 (t, *J= 1.6* Hz, 1H), 6.24 (d, *J=* 9.6 Hz, 1H), 4.29 (s, 3H), 1.82 (s, 3H), 1.25 (s, 18H); ¹³C NMR (100 MHz, DMSO-*d*₆) δ 188.8, 182.8, 179.8, 167.1, 164.6, 155.3, 151.6, 145.5, 140.1, 138.3, 137.7, 136.8, 136.3, 133.4, 132.2, 131.4, 130.3, 129.2, 128.4, 128.2, 127.7, 127.1, 127.0, 125.1, 124.3, 116.6, 112.7, 58.2, 47.3, 34.6, 31.1, 15.9.

### Embodiments 19 to 68:

In these embodiments, the reaction conditions were screened for the preparation method for (2*R*,3*S*) or (2*S*,3*R*)-β-hydroxy-β-(p-methylsulfonylphenyl) -α-tert-butyl alaninate.

The general steps in this series of embodiments were as follows:
According to the specific requirements in Table 1, to a 50 mL Schlenk tube were added p-methylsulfonyl benzaldehyde **3a** (0.2321 g, 1.26 mmol), additive (1.512 mmol) and chiral pyridinium salt carbonyl catalyst (0.00126 mmol) in a glovebox. The sealed Schlenk tube was taken out of the glovebox and added dry solvent (7.0 mL) via a syringe at room temperature. After the mixture was stirred at required reaction temperature for 5 min, glycine tert-butyl ester **2a** (0.4127 g, 3.15 mmol) was added and the reaction mixture was stirred at required reaction temperature for required reaction time. Then anhydrous methanol (1.0 mL) and hydroxylamine aqueous solution (1.0 mL, 50 wt.% aqueous solution) were added to quench the reaction. The resulting mixture was stirred at reaction temperature for 1 h and at room temperature for 30 min. The mixture was filtered via a pad of Celite, concentrated and dried in the presence of phosphorus pentoxide under vacuum to afford a crude compound **4a.** The yield and dr value of the compound **4a** were determined by ¹H NMR analysis and the ee value of compound **4a** was determined by HPLC analysis after being converted to the corresponding oxazolidin-2-thioketone by treatment with 1,1'-thiocarbonyldiimidazole.

The reaction equation was as follows:

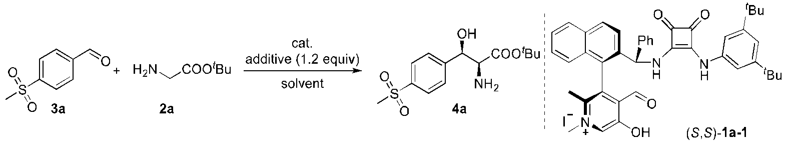

This compound had the following basic parameters:
**4a:** white solid; ¹H NMR (400 MHz, CDCl₃) δ 7.93 (d, *J=* 8.0 Hz, 2H), 7.60 (d, *J=* 8.0 Hz, 2H), 4.87 (d, *J=* 5.0 Hz, 1H), 3.53 (d, *J=* 5.0 Hz, 1H), 3.05 (s, 3H), 1.40 (s, 9H); ¹³C NMR (100 MHz, CDCl₃) δ 171.9, 147.8, 139.9, 127.5, 127.4, 82.4, 73.6, 60.6, 44.6, 27.9.

**Table 1. Reaction conditions, yields, dr values and ee values in Embodiments 19 to 68**

| Embodiment | Catalyst | Additive | Solvent | Temperature/°C | Reaction time/h | Yield (%) | dr value/ee value (%) |
|---|---|---|---|---|---|---|---|
| 19 | (*S,S*)-**1a-1**/0.1 mol% | Na₂HPO₄·12H₂O | Dichloromethane | -20 | 8 | 84 | 12:1/98 |
| 20 | (*S,R*)*-***1a-1** /0.1 mol% | Na₂HPO₄·12H₂O | Dichloromethane | -20 | 10 | 33 | 0.8:1/13 |
| 21 | (S,*S*)-**1a-2** /0.1 mol% | Na₂HPO₄·12H₂O | Dichloromethane | -20 | 8 | 82 | 12:1/97 |
| 22 | (S,*S*)**-1a-3** /0.1 mol% | Na₂HPO₄·12H₂O | Dichloromethane | -20 | 8 | 86 | 12:1/95 |
| 23 | (*S,S*)**-1a-4** / 0.1 mol% | Na₂HPO₄·12H₂O | Dichloromethane | -20 | 8 | 89 | 10:1/86 |
| 24 | (*S,S*)**-1a-5** / 0.1 mol% | Na₂HPO₄·12H₂O | Dichloromethane | -20 | 8 | 81 | 8:1196 |
| 25 | (*S,S*)**-1a-6** / 0.1 mol% | Na₂HPO₄·12H₂O | Dichloromethane | -20 | 8 | 77 | 11:1197 |
| 26 | (*S,S*)**-1a-7** / 0.1 mol% | Na₂HPO₄·12H₂O | Dichloromethane | -20 | 8 | 76 | 11:1/95 |
| 27 | (*S,S*)**-1a-8** / 0.1 mol% | Na₂HPO₄·12H₂O | Dichloromethane | -20 | 8 | 86 | 11:1/96 |
| 28 | (*S,S*)**-1a-9** / 0.1 mol% | Na₂HPO₄·12H₂O | Dichloromethane | -20 | 8 | 80 | 8:1/94 |
| 29 | (*S*,*S*)-**1a-10** / 0.1 mol% | Na₂HPO₄·12H₂O | Dichloromethane | -20 | 8 | 86 | 3:1/92 |
| 30 | (*S,S*)-**1a-11** / 0.1 mol% | Na₂HPO₄·12H₂O | Dichloromethane | -20 | 8 | 78 | 12:1/97 |
| 31 | (*S,S*)**-1a-12** / 0.1 mol% | Na₂HPO₄·12H₂O | Dichloromethane | -20 | 8 | 83 | 2:1/92 |
| 32 | (*S,S*)**-1a-13** / 0.1 mol% | Na₂HPO₄·12H₂O | Dichloromethane | -20 | 8 | 82 | 12:1197 |
| 33 | (*R,R*)-**1a-14** / 0.1 mol% | Na₂HPO₄·12H₂O | Dichloromethane | -20 | 8 | 65 | 9:1193 |
| 34 | (*S,S*)-**1a-15**/0.1 mol% | Na₂HPO₄·12H₂O | Dichloromethane | -20 | 8 | 83 | 11:1/98 |
| 35 | (*S,S*)-**1c** / 0.1 mol% | Na₂HPO₄·12H₂O | Dichloromethane | -20 | 8 | 71 | 4:1/90 |
| 36 | (*S,S*)-**1a-1** /0.1 mol% | Na₂HPO₄·12H₂O | Tetrahydrofuran | -20 | 8 | 92 | 7:1/97 |
| 37 | (*S,S*)-**1a-1**/0.1 mol% | Na₂HPO₄·12H₂O | Toluene | -20 | 8 | 25 | 8:1/83 |
| 38 | (*S,S*)-**1a-1** /0.1 mol% | Na₂HPO₄·12H₂O | Methanol | -20 | 8 | N.R. | |
| 39 | (*S,S*)-**1a-1** /0.1 mol% | Na₂HPO₄·12H₂O | Ethyl ether | -20 | 8 | 24 | 9: 1/90 |
| 40 | (*S,S*)-**1a-1** / 0.1 mol% | Na₂HPO₄·12H₂O | Tetrahydrofuran | -20 | 6 | 88 | 9: 1/97 |
| 41 | (*S,S*)-**1a-1** / 0.1 mol% | Na₂HPO₄ | Tetrahydrofuran | -20 | 6 | 68 | 9: 1/94 |
| 42 | (*S,S*)-**1a-1** / 0.1 mol% | None | Tetrahydrofuran | -20 | 6 | 91 | 10:1/90 |
| 43 | (*S,S*)-**1a-1** / 0.1 mol% | None | Tetrahydrofuran | -30 | 8 | 54 | 13/90 |
| 44 | (*S,S*)-**1a-1** / 0.1 mol% | MgSO₄ | Tetrahydrofuran | -20 | 6 | 43 | 7:1/54 |
| 45 | (*S,S*)-**1a-1** / 0.1 mol% | NaH₂PO₄ | Tetrahydrofuran | -20 | 6 | 98 | 10:1/97 |
| 46 | (*S,S*)-**1a-1** / 0.1 mol% | 0.9 eq. NaH₂PO₄ | Tetrahydrofuran | -20 | 6 | 86 | 11/93 |
| 47 | (*S,S*)-**1a-1** / 0.1 mol% | 0.9 eq. NaH₂PO₄ | Tetrahydrofuran | -20 | 6 | 78 | 11/94 |
| 48 | (*S,S*)-**1a-1** / 0.1 mol% | KH₂PO₄ | Tetrahydrofuran | -20 | 6 | 98 | 9: 1/96 |
| 49 | (*S,S*)-**1a-1** / 0.1 mol% | LiH₂PO₄ | Tetrahydrofuran | -20 | 6 | 97 | 10:1/98 |
| 50 | (*S,S*)-**1a-1** / 0.1 mol% | NH₄H₂PO₄ | Tetrahydrofuran | -20 | 6 | 98 | 12:1/98 |
| 51 | (*S,S*)-**1a-1** / 0.1 mol% | NH₄H₂PO₄ | Tetrahydrofuran | -40 | 48 | 98 | 26:1/98 |
| 52 | (*S,S*)-**1a-1** / 0.1mol% | NH₄H₂PO₄ | Tetrahydrofuran | -30 | 10 | 98 | 21:1/99 |
| 53 | (*S,S*)-**1a-2** / 0.1 mol% | NH₄H₂PO₄ | Tetrahydrofuran | -30 | 17 | 98 | 28/98 |
| 54 | (*S,S*)-**1a-3** / 0.1 mol% | NH₄H₂PO₄ | Tetrahydrofuran | -30 | 12 | 98 | 28/98 |
| 55 | (*S,S*)-**1a-**4 / 0.1 mol% | NH₄H₂PO₄ | Tetrahydrofuran | -30 | 15 | 97 | 30/98 |
| 56 | (*S,S*)-**1a-5** / 0.1 mol% | NH₄H₂PO₄ | Tetrahydrofuran | -30 | 15 | 72 | 9/96 |
| 57 | (*S,S*)-**1a-6** / 0.1 mol% | NH₄H₂PO₄ | Tetrahydrofuran | -30 | 15 | 98 | 19/97 |
| 58 | (*S,S*)**-1a-7** / 0.1 mol% | NH₄H₂PO₄ | Tetrahydrofuran | -30 | 16 | 84 | 26/97 |
| 59 | (*S,S*)-**1a-8** / 0.1 mol% | NH₄H₂PO₄ | Tetrahydrofuran | -30 | 10 | 98 | 22/98 |
| 60 | (*S,S*)**-1a-9** / 0.1 mol% | NH₄H₂PO₄ | Tetrahydrofuran | -30 | 15 | 93 | 27/97 |
| 61 | (*S,S*)-**1a-**10 / 0.1 mol% | NH₄H₂PO₄ | Tetrahydrofuran | -30 | 12 | 98 | 6/94 |
| 62 | (*S,S*)-**1a-11** / 0.1 mol% | NH₄H₂PO₄ | Tetrahydrofuran | -30 | 17 | 98 | 21/98 |
| 63 | (*S,S*)**-1a-12** / 0.1 mol% | NH₄H₂PO₄ | Tetrahydrofuran | -30 | 15 | 88 | 2/93 |
| 64 | (*S,S*)-**1a-1** / 0.05 mol% | NaH₂PO₄ | Tetrahydrofuran | -20 | 12 | 97 | 9: 1/98 |
| 65 | (*S,S*)**-1a-1** / 0.01 mol% | NH₄H₂PO₄ | Tetrahydrofuran | -15 | 48 | 77 | 10:1/97 |
| 66 | (*S,S*)-**1a-1** / 0.01 mol% | NH₄H₂PO₄ | Tetrahydrofuran | -10 | 24 | 77 | 9: 1/97 |
| 67 | (*S,S*)**-1a-1** / 0.01 mol% | NH₄H₂PO₄ | Tetrahydrofuran | -30 | 168 | 64 | 14:1/98 |
| 68 | (*S,S*)**-1a-1** / 0.01 mol% | NH₄H₂PO₄ | Tetrahydrofuran | -20 | 72 | 80(3.22 g) | 15:1/98 |

The reaction conditions were screened in Table 1, with the screening carried out in terms of catalyst at first. From Embodiments 19 to 35, it can be seen all the chiral pyridinium salt carbonyl catalysts (*S,S*)**-1a-1 to 15** and (*S,S*)**-1c** show a catalytic effect on the identified reaction. Based on comparison, it was found that (*S,S*)**-1a-1 to 3, 8, 13** and **15** as catalysts showed better and comparable yields, dr values and ee values for the reaction, and could act as preferred catalysts to catalyze the reaction (Embodiments 19, 21, 22, 27, 32 and 34), among which (*S,S*)-**1a-3 and** (*S,S*)**-1a-8** showed the best yield (86%), (*S,S*)**-1a-1 to 3** and **13** showed the best dr value (12:1), and (*S,S*)**-1a-1 and 15** showed the best ee value (98%). In contrast, (*S,S*)**-1a-4 to 7, 9 to 12 and 14** and (*S,S*)**-1c** behaved less well in at least one of the three results, but could still catalyze the identified reaction to achieve a better reaction result. In comprehensive consideration of the catalytic reaction results and the difficulty in catalyst preparation, (*S,S*)**-1a-1** was finally selected as the major catalyst for subsequent screening (Embodiment 19).

Then, the screening was carried out in terms of solvent, additive, catalyst equivalent, and temperature. In terms of solvent, five different solvents were screened, including dichloromethane, tetrahydrofuran, toluene, methanol and ethyl ether (Embodiments 36 to 40). Except for methanol that reacted with the catalyst to produce acetal to lead to a failed reaction, other solvents were available for carrying out the reaction, among which tetrahydrofuran showed the highest yield, with the dr value increasing even after the reaction time was reduced to 6 h (Embodiment 40). In terms of additive, different additives were screened, including Na₂HPO₄·12H₂O, Na₂HPO₄ (with simultaneous screening in terms of equivalent), no additive (with simultaneous screening in terms of temperature), MgSO₄, NaH₂PO₄, KH₂PO₄, LiH₂PO₄ and NH₄H₂PO₄ (Embodiments 40 to 50). These additives were available for carrying out the reaction, among which NH₄H₂PO₄ showed the best reaction result, along with improvements in yield, dr value and ee value (Embodiment 50). In terms of temperature screening, the reaction proceeded with cooling (-40°C to -20°C, Embodiments 50 to 52). It was observed that the dr value for the reaction continuously increased with the decrease of the temperature, but at -40°C, the reaction time needed to be increased (6 h to 48 h) and the ee value slightly decreased, and at -30°C, the dr value was slightly lower but greater than 20:1. At this temperature, the screening was then carried out again in terms of catalyst (Embodiments 52 to 63), and the catalyst (*S,S*)**-1a-1** was stilled observed with the best effect, with a slightly lower dr value but the best ee value. Therefore, we finally determined Embodiment 52 as the reaction conditions for substrate scope. In addition, we attempted to reduce the equivalent of catalyst (0.01 mol% to 0.1 mol%) and screen the temperature (-30°C to 10°C, Embodiments 64 to 68). The equivalent of catalyst could be reduced to as low as 0.01 mol%. 3.22 g of products were achieved from 1 mg of catalyst at -20°C for an extended reaction time (from 24 h to 72 h, with the final time set at 72 h). The dr and ee values maintained good results (15:1/98%).

### Embodiments 69 to 87:

In these embodiments, the reaction conditions are screened for the preparation method for (2R,3,S) or (2*S*,3*R*)-β-hydroxy-β-(p-phenylphenyl)-α-tert-butyl alaninate. The general steps in these embodiments are as follows:
According to the specific requirements in Table 2, to a 50 mL Schlenk tube were added p-phenyl benzaldehyde **3af** (0.2293 g, 1.26 mmol), additive (1.512 mmol), solid base (1.89 mmol) and chiral pyridinium salt carbonyl catalyst (0.00126 mmol) in a glovebox.

The sealed Schlenk tube was taken out of the glovebox and added dry solvent (6.0 mL) and liquid base (1.89 mmol) via a syringe at room temperature. After the mixture was stirred at required reaction temperature for 5 min, tert-butyl glycinate **2a** (0.4127 g, 3.15 mmol) was added and the reaction mixture was stirred at required reaction temperature for required reaction time. Then anhydrous methanol (1.0 mL) and hydroxylamine aqueous solution (1.0 mL, 50 wt.% aqueous solution) were added to quench the reaction.

The resulting mixture was stirred at reaction temperature for 1 h and at room temperature for 30 min. The mixture was filtered via a pad of Celite, concentrated and dried in the presence of phosphorus pentoxide under vacuum to afford a crude compound **4af.** The yield and dr value of the compound **4af** were determined by ¹H NMR analysis and the ee value of compound **4af** was determined by HPLC analysis after being converted to the corresponding oxazolidin-2-thioketone by treatment with 1,1'-thiocarbonyldiimidazole.

The reaction equation was as follows:

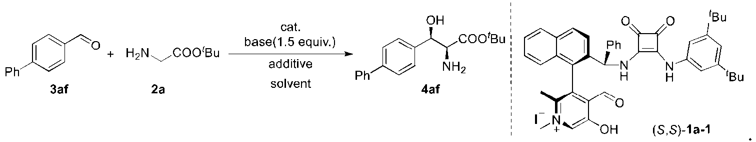

This compound had the following basic parameters:
**4af:** white solid; ¹H NMR (400 MHz, CDCl₃) δ 7.65-7.57 (m, 4H), δ 7.51-7.42 (m, 4H), 7.37 (t, *J=* 7.2 Hz, 1H), 4.82 (d, *J* = 5.4 Hz, 1H), 3.59 (d, *J* = 5.4 Hz, 1H), 1.39 (s, 9H). ¹³C NMR (100 MHz, CDCl₃) δ 172.5, 140.9, 140.8, 140.1, 128.8, 127.3, 127.1, 127.1, 127.0, 81.9, 74.4, 61.0, 27.9.

**Table 2. Bases, yields, dr values and ee values screened by reactions in Embodiments 69 to 87**

| Embodiment | Catalyst | Additive | Solvent | Base | Temperature/°C | Reaction time/h | Yield (%) | dr value/ee value (%) |
|---|---|---|---|---|---|---|---|---|
| 69 | (*S,S*)**-1a-1** /0.5 mol% | None | Dichloromethane | Na₃PO₄ | -5 | 48 | 35 | 1.6:1/52 |
| 70 | (*S,S*)**-1a-1** / 0.5 mol% | None | Dichloromethane | K₃PO₄ | -5 | 48 | 25 | 1.6:1/58 |
| 71 | (*S,S*)**-1a-1** / 0.5 mol% | None | Dichloromethane | Cs₂CO₃ | -5 | 48 | 32 | 1:0.8/55 |
| 72 | (*S,S*)**-1a-1** / 0.5 mol% | None | Dichloromethane | DIPEA | -5 | 48 | 60 | 1.6:1/90 |
| 73 | (*S,S*)**-1a-1** / 0.5 mol% | None | Dichloromethane | Et₃N | -5 | 48 | 70 | 1.9:1/49 |
| 74 | (*S,S*)**-1a-1** / 0.5 mol% | None | Dichloromethane | Et₃N | -5 | 20 | 71 | 3:1/68 |
| 75 | (*S,S*)**-1a-5** / 0.5 mol% | None | Dichloromethane | Et₃N | -5 | 20 | 67 | 1.9:1/69 |
| 76 | (*S,S*)**-1a-1** / 0.5 mol% | None | Dichloromethane | Et₃N | -20 | 24 | 86 | 5:1/93 |
| 77 | (*S,S*)**-1a-1** / 0.5 mol% | None | Chloroform | Et₃N | -20 | 24 | 52 | 5:1/90 |
| 78 | (*S,S*)**-1a-1** / 0.5 mol% | None | Toluene | Et₃N | -20 | 24 | 87 | 6:1/79 |
| 79 | (*S,S*)**-1a-1** / 0.5 mol% | None | Methanol | Et₃N | -20 | 24 | 5 | 1:1/9 |
| 80 | (*S,S*)-1**a-1** / 0.5 mol% | None | Acetonitrile | Et₃N | -20 | 24 | 55 | 5:1/87 |
| 81 | (*S,S*)**-1a-1** / 0.5 mol% | None | Tetrahydrofuran | Et₃N | -20 | 24 | 92 | 10:1/91 |
| 82 | (*S,S*)**-1a-1** /0.25 mol% | None | Tetrahydrofuran | Et₃N | -20 | 24 | 91 | 9: 1/93 |
| 83 | (*S,S*)**-1a-1** / 0.1 mol% | None | Tetrahydrofuran | Et₃N | -20 | 24 | 85 | 15:1/94 |
| 84 | (*S,S*)-**1a-1** / 0.1 mol% | None | Tetrahydrofuran | Et₃N | -30 | 24 | 94 | > **20:1/97** |
| 85 | (*S,S*)**-1a-1** / 0.1 mol% | None | Tetrahydrofuran | Et₃N | -30 | 48 | 94 | 15:1/98 |
| 86 | (*S,S*)**-1a-1** / 0.1 mol% | None | Tetrahydrofuran | Et₃N | -40 | 48 | 94 | > **20:1/98** |
| 87 | (*S,S*)**-1a-1** / 0.1 mol% | NaH₂PO₄ | Tetrahydrofuran | Et₃N | -40 | 48 | 94 | > **20:1/99** |

The reaction conditions were screened in Table 2. In Embodiments 69 to 75, five bases, including Na₃PO₄, K₃PO₄, Cs₂CO₃, DIPEA and Et₃N (Embodiments 69 to 73) were compared, with the best result observed in Et₃N. Also, the screening was carried out in terms of reaction time and catalyst type (Embodiments 73 to 75), it was observed that reduced time (20 h to 48 h) led to better reaction selectively, and (*S,S*)**-1a-1** still showed the best catalytic effect compared to (*S,S*)**-1a-5.** Then, by reducing the temperature to - 20°C (-20°C to -5°C), solvent screening was carried out on dichloromethane, chloroform, toluene, methanol, acetonitrile and tetrahydrofuran (Embodiments 76 to 81) respectively, and it was observed that, except for methanol that had the lowest reaction effect, other solvents still showed good effects, with the best effect observed in tetrahydrofuran (Embodiments 81). Afterwards, the screening was carried out in terms of catalyst equivalent (0.1 mol% to 0.5 mol%, Embodiments 81 to 83), and it was observed a better yield was maintained even when the equivalent was reduced to 0.1 mol%, and the dr value still increased with the decrease in equivalent. Finally, the screening was carried out in terms of temperature (-40°C to -20°C, Embodiments 83 to 86), and the dr and ee values increased with the decrease in temperature. A comparison of Embodiments 84 and 85 further confirms that the dr value decreased with the increase in time (24 h to 48 h). In Embodiment 87, after NaH₂PO₄ was added, the ee value of the reaction increased to 99%. Accordingly, the reaction conditions for substrate scope were finally determined.

### Embodiments 88 to 113:

In these embodiments, the reaction conditions are screened for the preparation method for (2R,3,S) or (2*S*,3*R*)-β-hydroxy-β-(n-nonyl)-α-tert-butyl alaninate. The general steps in these embodiments are as follows:
According to the specific requirements in Table 3, to a 50 mL Schlenk tube were added NH₄H₂PO₄ and chiral pyridinium salt carbonyl catalyst in a glovebox. The sealed Schlenk tube was taken out of the glovebox and added tetrahydrofuran, n-capric aldehyde **3bb** (0.1969 g, 1.26 mmol) and triethylamine via a syringe at -30°C. After the mixture was stirred at -30°C for 20 min, tert-butyl glycinate **2a** was added and the reaction mixture was stirred at required reaction temperature for required reaction time.

Then anhydrous methanol (1.0 mL) and hydroxylamine aqueous solution (1.0 mL, 50 wt.% aqueous solution) were added to quench the reaction. The resulting mixture was stirred at reaction temperature for 1 h and at room temperature for 30 min. The mixture was filtered via a pad of Celite, concentrated and dried in the presence of phosphorus pentoxide under vacuum to afford a crude compound **4bb.** The yield and dr value of compound **4bb** were determined by ¹H NMR analysis and the ee value of the compound **4bb** was determined by HPLC analysis after being converted to the corresponding oxazolidin-2-thioketone by treatment with 1,1'-thiocarbonyldiimidazole.

The reaction equation was as follows:

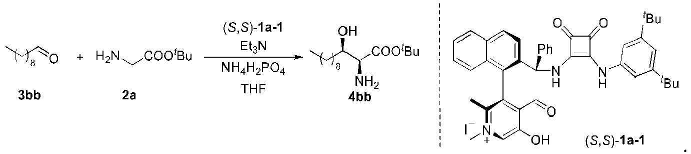

This compound had the following basic parameters:
**4bb:** yellow oil; ¹H NMR (400 MHz, CDCl₃) δ 3.70-3.62 (m, 1H), 3.22 (d, *J=* 5.2 Hz, 1H), 2.06 (brs, 3H), 1.52-1.49 (m, 1H), 1.48 (s, 9H), 1.46-1.44 (m, 1H), 1.34-1.22 (m, 14H), 0.87 (t, *J=* 6.8 Hz, 3H). 13C NMR (100 MHz, CDCl₃) δ 173.7, 81.8, 72.3, 59.0, 34.2, 32.0, 29.8, 29.7, 29.7, 29.4, 28.2, 25.8, 22.8, 14.2.

**Table 3. Bases, yields, dr values and ee values screened by reactions in Embodiments 88 to 113**

| Embodiment | Catalyst | **2a** /equiv. | NH₄H₂PO₄ /equiv. | THF /mL | Et₃N /equiv. | Temperature /°C | Reaction time /h | Yield (%) | dr value/ee value (%) |
|---|---|---|---|---|---|---|---|---|---|
| 88 | (*S,S*)-**1a-1** / 0.1 mol% | 2.0 | 0.8 | 6 | 2.0 | -10 | 48 | 46 | 11:1/99 |
| 89 | (*S,S*)**-1a-1** / 0.1 mol% | 2.0 | 0.8 | 6 | 2.0 | -20 | 48 | 57 | > 20:1/99 |
| 90 | (*S,S*)**-1a-1** / 0.1 mol% | 2.0 | 0.8 | 6 | 2.0 | -30 | 48 | 64 | > 20:1/99 |
| 91 | (*S,S*)**-1a-1** / 0.1 mol% | 2.0 | 0.8 | 6 | 2.0 | -40 | 48 | 61 | > 20:1/99 |
| 92 | (*S,S*)**-1a-1** / 0.1 mol% | 1.2 | 0.8 | 6 | 2.0 | -30 | 48 | 49 | > 20:1/99 |
| 93 | (*S,S*)**-1a-1** / 0.1 mol% | 3.0 | 0.8 | 6 | 2.0 | -30 | 48 | 50 | > 20:1/99 |
| 94 | (*S,S*)**-1a-1** / 0.1 mol% | 4.0 | 0.8 | 6 | 2.0 | -30 | 48 | 42 | > 20:1/99 |
| 95 | (*S,S*)**-1a-1** / 0.1 mol% | 2.0 | 0.8 | 6 | 1.5 | -30 | 24 | 55 | > 20:1/99 |
| 96 | (*S,S*)**-1a-1** / 0.1 mol% | 2.0 | 0.8 | 6 | 2.0 | -30 | 24 | 63 | > 20:1/99 |
| 97 | (*S,S*)**-1a-1** / 0.1 mol% | 2.0 | 0.8 | 6 | 2.5 | -30 | 24 | 62 | > 20:1/99 |
| 98 | (*S,S*)**-1a-1** / 0.1 mol% | 2.0 | 0.8 | 6 | 3.0 | -30 | 24 | 58 | > 20:1/99 |
| 99 | (*S,S*)**-1a-1** / 0.1 mol% | 2.0 | 0.6 | 6 | 2.0 | -30 | 24 | 59 | > 20:1/99 |
| 100 | (*S,S*)**-1a-1** / 0.1 mol% | 2.0 | 1.0 | 6 | 2.0 | -30 | 24 | 62 | > 20:1/99 |
| 101 | (*S,S*)**-1a-1** / 0.1 mol% | 2.0 | 1.2 | 6 | 2.0 | -30 | 24 | 64 | > 20:1/99 |
| 102 | (*S,S*)**-1a-1** / 0.1 mol% | 2.0 | 1.5 | 6 | 2.0 | -30 | 24 | 58 | > 20:1/99 |
| 103 | (*S,S*)**-1a-1** / 0.1 mol% | 2.0 | 2.0 | 6 | 2.0 | -30 | 24 | 59 | > 20:1/99 |
| 104 | (*S,S*)**-1a-1** / 0.2 mol% | 2.0 | 1.0 | 6 | 2.0 | -30 | 24 | 68 | > 20:1/99 |
| 105 | (*S,S*)**-1a-1** /0.5 mol% | 2.0 | 1.0 | 6 | 2.0 | -30 | 24 | 75 | > 20:1/99 |
| 106 | (*S,S*)**-1a-1** / 1.0 mol% | 2.0 | 1.0 | 6 | 2.0 | -30 | 24 | 78 | > 20:1/99 |
| 107 | (*S,S*)**-1a-1** / 0.5 mol% | 2.0 | 1.0 | 9 | 2.0 | -30 | 24 | 79 | > 20:1/99 |
| 108 | (*S,S*)**-1a-1** / 0.5 mol% | 2.0 | 1.0 | 12 | 2.0 | -30 | 24 | 81 | > 20:1/99 |
| 109 | (*S,S*)**-1a-**1 / 0.5 mol% | 2.0 | 1.0 | 15 | 2.0 | -30 | 24 | 82 | > 20:1/99 |
| 110 | (*S,S*)**-1a-1** /0.5 mol% | 2.0 | 1.0 | 20 | 2.0 | -30 | 24 | 83 | 16:1/99 |
| 111 | (*S,S*)**-1a-1** / 0.1 mol% | 2.0 | 1.0 | 12 | 2.0 | -30 | 24 | 71 | > 20:1/99 |
| 112 | (*S,S*)**-1a-1** / 0.2 mol% | 2.0 | 1.0 | 12 | 2.0 | -30 | 24 | 79 | > 20:1/99 |
| 113 | (*S,S*)**-1a-1** / 0.2 mol% | Add 11.5 mL of THF first, dissolve **2a** in 0.5 mL of THF, and freeze for 30 min at -30°C, followed by quick addition | | | | | 20 | 82 | > 20:1/99 |

The reaction conditions were screened in Table 3. In Embodiments 88 to 91, temperature screening was carried out, and it was observed that the reaction results gradually became better with the decrease in temperature (-40°C to -10°C), with the best effect observed at -30°C. In Embodiments 92 to 94, screening was carried out in terms of the equivalent of tert-Butyl glycinate (1.2 to 4.0), and it was observed that the equivalent of 2.0 still showed the best effect. In Embodiments 95 to 98, screening was carried out in terms of the equivalent of base (1.5 to 3.0) while reducing the reaction time (24 h to 48 h), and it was observed that the equivalent of 2.0 still showed the best effect. In Embodiments 99 to 103, screening was carried out in terms of the equivalent of NH₄H₂PO₄ (0.6 to 2.0), it was observed that the equivalent of 0.8-1.2 showed a comparable effect, and the equivalent of 1.0 was selected finally for subsequent screening. In Embodiments 104 to 106, screening was carried out in terms of the equivalent of catalyst (0.1 mol% to 1.0 mol%), and it was observed that the yield gradually increased with the increase in the equivalent of catalyst and with the selectivity unchanged, but without significant changes observed in result in case of 0.005 to 0.01. Afterwards, 0.005 was used for subsequent screening. In Embodiments 107 to 110, screening was carried out in terms of concentration (solvent volume: 6 mL to 20 mL), and it was observed that, with the increase in concentration, the yield gradually increased and the dr value gradually decreased, with the best reaction result observed when the solvent volume was 12 mL to 15 mL. Afterwards, 12 mL was used for subsequent screening. At this concentration, in Embodiments 111 to 112, screening was carried out again in terms of the equivalent of catalyst (0.1 mol% to 0.5 mol%), and it was observed that the reaction result was maintained when the equivalent was reduced to 0.002 and the yield decreased in case of 0.001. Finally, in Embodiment 113, the mode of adding the tert-butyl glycinate and solvent was changed under the condition of 0.002 eq. catalyst, and the reaction conditions for substrate scope were then determined.

### Embodiments 114 to 1905:

In these embodiments, the scope of aryl aldehyde and alkyl aldehyde substrates applicable to the preparation method for a chiral β-hydroxy-α-amino acid derivative catalyzed by a pyridoxal quaternary ammonium salt were expanded. The general steps in these embodiments were as follows (General Method 1 to be used in Embodiments 114 to 116, 130, 136 and 143, General Method 2 to be used in Embodiments 150 to 175, General Method 3 to be used in Embodiments 117 to 129, 131 to 135, 137 to 142, 144 to 149, and 176 to 182, and General Method 4 to be used in Embodiments 183 to 1905).

General Method 1 (aromatic aldehyde with strong electron-withdrawing substituents) included: to a 50 mL Schlenk tube were added aromatic aldehyde (1.26 mmol), NH₄H₂PO₄ (0.1739 g, 1.512 mmol) and chiral pyridinium salt carbonyl catalyst (R,R)-**1a-1** (0.001 g, 0.00126 mmol) in a glovebox. The sealed Schlenk tube was taken out of the glovebox and added tetrahydrofuran (7.0 mL) via a syringe at room temperature.

After the mixture was stirred at required reaction temperature for 5 min, a glycine derivative 2 (3.15 mmol) was added at -30°C and the mixture was stirred at required reaction temperature for 10 h. Then anhydrous methanol (1.0 mL) and hydroxylamine aqueous solution (1.0 mL, 50 wt.% aqueous solution) were added to quench the reaction.

The resulting mixture was stirred at -30°C for 10 min, at -20°C for 50 min, and at room temperature for 30 min. The mixture was filtered via a pad of Celite, concentrated and dried in the presence of phosphorus pentoxide under vacuum to afford a crude product of chiral β-hydroxy-α-amino acid derivative. The yield and dr value of the product were determined by ¹H NMR analysis and the ee value of the product was determined by HPLC analysis after being converted to the corresponding oxazolidin-2-thioketone by treatment with 1,1'-thiocarbonyldiimidazole.

General Method 2 (aromatic aldehyde with strong electron-donating, weak electron-withdrawing and weak electron-donating substituents) included: to a 50 mL Schlenk tube were added aromatic aldehyde (1.26 mmol) , chiral pyridinium salt carbonyl catalyst (*R,R*)-**1a-1** (0.001 g, 0.00126 mmol) and NaH₂PO₄ (0.1814 g, 1.512 mmol) in a glovebox. The sealed Schlenk tube was taken out of the glovebox and added tetrahydrofuran (6.0 mL) and triethylamine (0.263 mL, 1.89 mmol) via a syringe at room temperature. After the mixture was stirred at required reaction temperature for 5 min, a glycine derivative **2** (3.15 mmol) was added at -40°C and the mixture was stirred at required reaction temperature for 48 h. Then anhydrous methanol (1.0 mL) and hydroxylamine aqueous solution (1.0 mL, 50 wt.% aqueous solution) were added to quench the reaction. The resulting mixture was stirred at -40°C for 10 min, at -20°C for 50 min, and at room temperature for 30 min. The mixture was filtered via a pad of Celite, concentrated and dried in the presence of phosphorus pentoxide under vacuum to afford a crude product of chiral β-hydroxy-α-amino acid derivative. The yield and dr value of the product were determined by ¹H NMR analysis and the ee value of the product was determined by HPLC analysis after being converted to the corresponding oxazolidin-2-thioketone by treatment with 1,1'-thiocarbonyldiimidazole.

General Method 3 (alkyl aldehyde) included: to a 50 mL Schlenk tube were added alkyl aldehyde (1.26 mmol), NH₄H₂PO₄ (0.145 g, 1.26 mmol) and chiral pyridinium salt carbonyl catalyst (*R,R*)-**1a-1** (0.00252 mmol) in a glovebox. The sealed Schlenk tube was taken out of the glovebox and added a solvent (11.5 mL) and triethylamine (350 µL, 2.52 mmol) via a syringe at -30°C. After the mixture was stirred at -30°C for 20 min, a glycine derivative **2** (3.15 mmol, dissolved in 0.5 mL of tetrahydrofuran and then frozen at -30°C for 30 min) was added quickly via a syringe and the mixture was stirred at required reaction temperature for 20 min. Then anhydrous methanol (1.0 mL) and hydroxylamine aqueous solution (1.0 mL, 50 wt.% aqueous solution) were added to quench the reaction. The resulting mixture was stirred at -30°C for 10 min, at -20°C for 50 min, and at room temperature for 30 min. The mixture was filtered via a pad of Celite, concentrated and dried in the presence of phosphorus pentoxide under vacuum to afford a crude product of chiral β-hydroxy-α-amino acid derivative. The yield and dr value of the product were determined by ¹H NMR analysis and the ee value of the product was determined by HPLC analysis after being converted to the corresponding oxazolidin-2-thioketone by treatment with 1,1'-thiocarbonyldiimidazole.

General Method 4 (high-throughput synthesis) included: a module that can accommodate 20 threaded test tubes for simultaneous reaction was customized and placed in a low-temperature reactor for reaction. Aromatic aldehyde (0.315 mmol), NH₄H₂PO₄ (43.5 mg, 0.378 mmol) or NaH₂PO₄ (45.3 mg, 0.378 mmol), and the chiral pyridinium salt carbonyl catalyst (*S,S*)-**1a-1** or *(R,R)-***1a-1** (0.5 mg in 1.0 mL of THF, 0.00063 mmol) were added in a glovebox. The sealed tubes were taken out of the glovebox and added tert-butyl glycinate (82.5 mg, 0.63 mmol) or the mixture of tert-butyl glycinate and triethylamine (66 µL, 0.4725 mmol) via a syringe at -40°C, and the reation mixture was stirred at -40°C for 48 h. Then anhydrous methanol (0.2 mL) and hydroxylamine aqueous solution (0.2 mL, 50 wt.% aqueous solution) were added to quench the reaction. The resulting mixture was stirred at -40°C for 10 min, at -20°C for 40 min, and at room temperature for 30 min. Then the mixture was diluted with dichloromethane/anhydrous methanol (3 mL/2 mL), filtered via a syringe filter, concentrated under reduced pressure, and dried for 20 h in the presence of phosphorus pentoxide under vacuum. A dried crude product was dissolved with deuterated chloroform (2 mL) and water (0.1 mL) to remove the needless hydroxylamine from the crude product. The organic layer was directly transferred via a pipette to a syringe holding sodium sulfate, and the mixture was filtered via the syringe filter. The yield and dr value of the product were determined by ¹H NMR analysis and the ee value of the product was determined by HPLC analysis after being converted to the corresponding oxazolidin-2-thioketone by treatment with 1,1'-thiocarbonyldiimidazole.

The reaction equation was as follows:

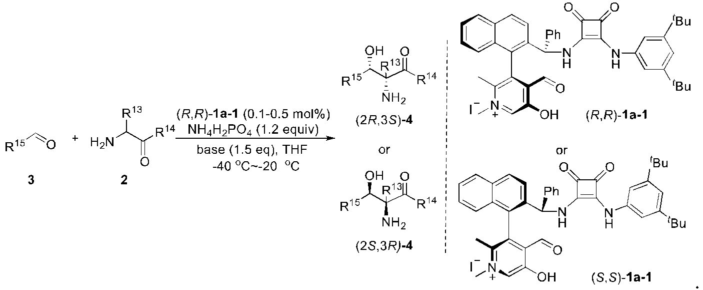

The basic parameters of the compounds can be found in Table 4.

Table 4. Product structures, yields, dr values, ee values and NMR characterization in Embodiments 114 to 1905

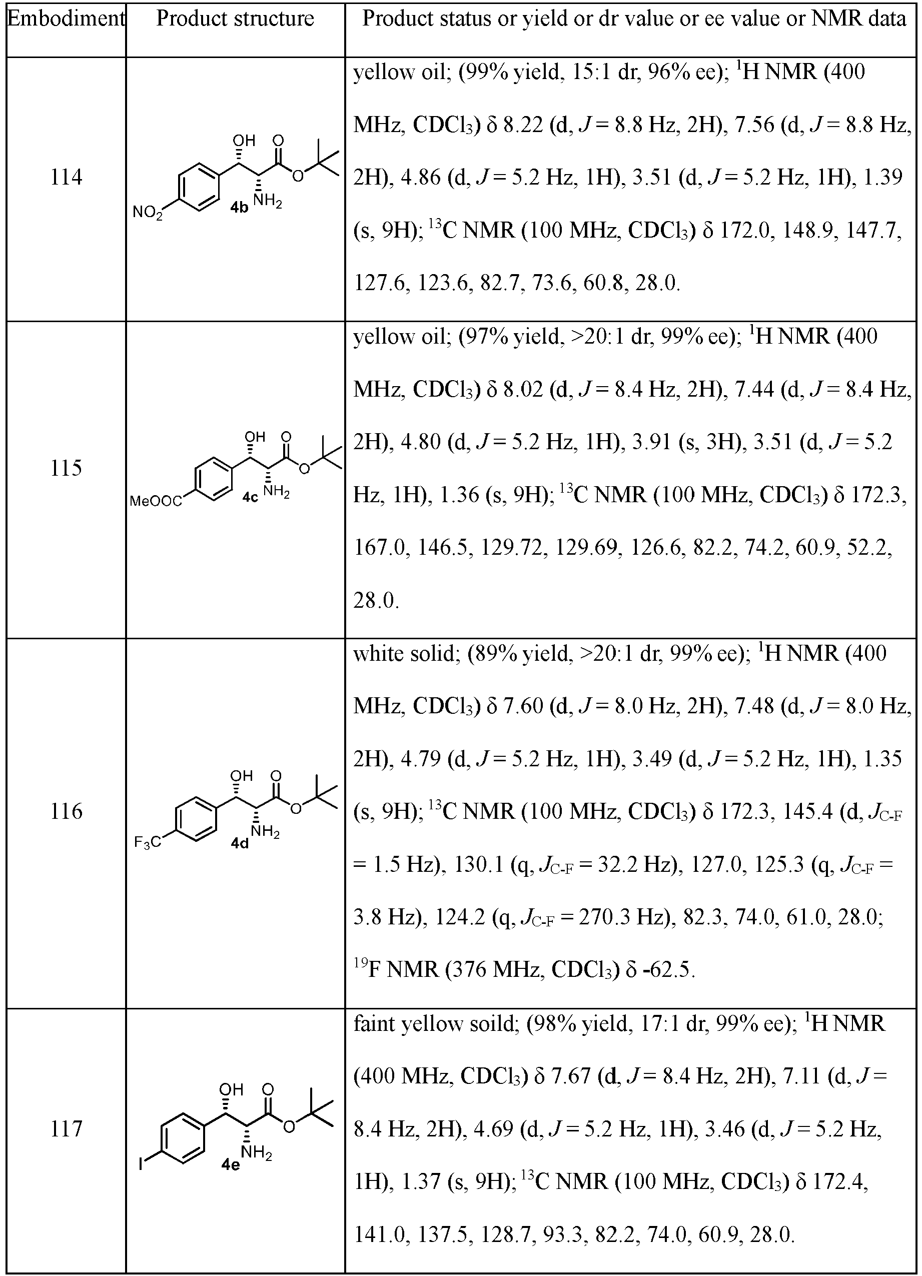

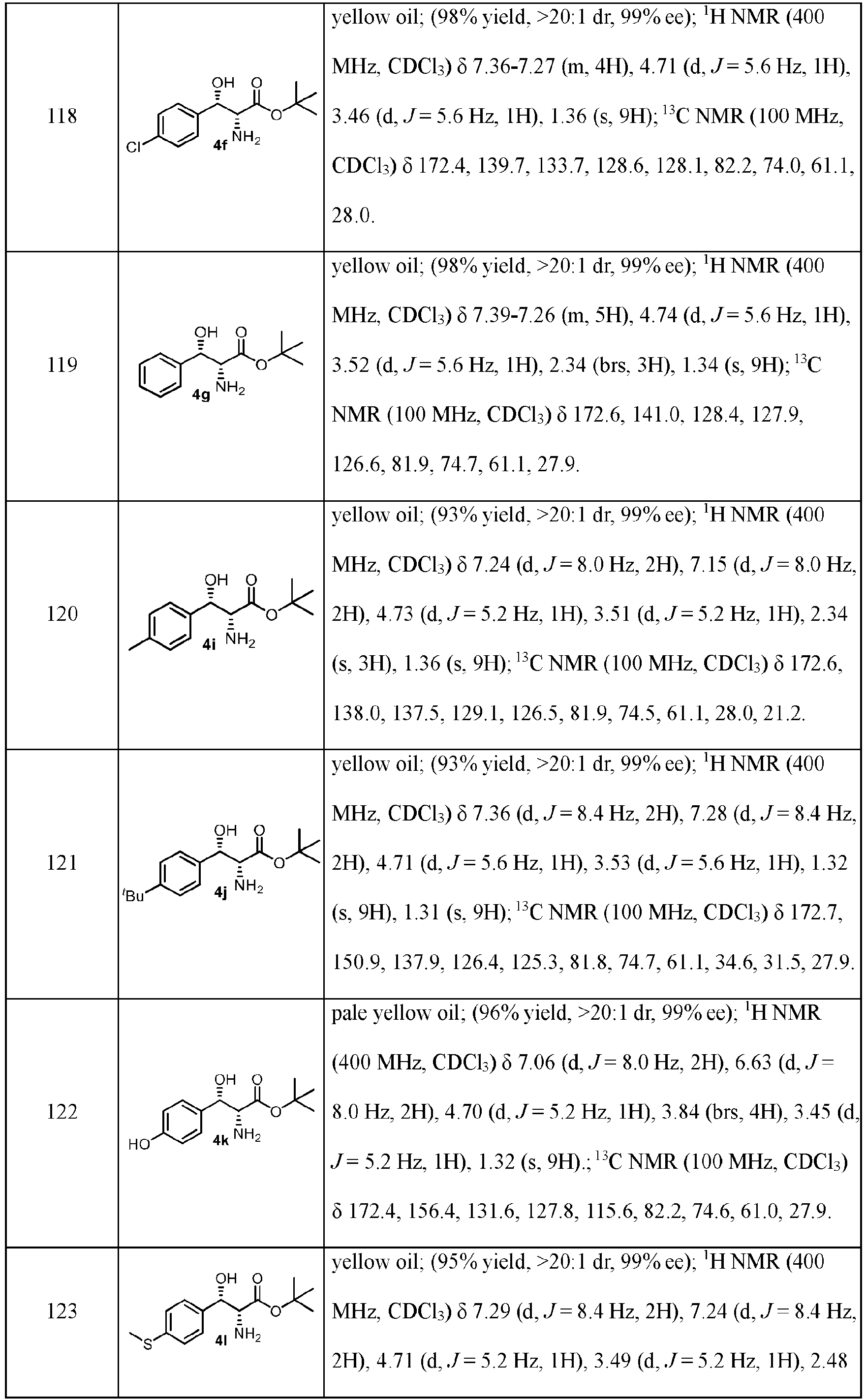

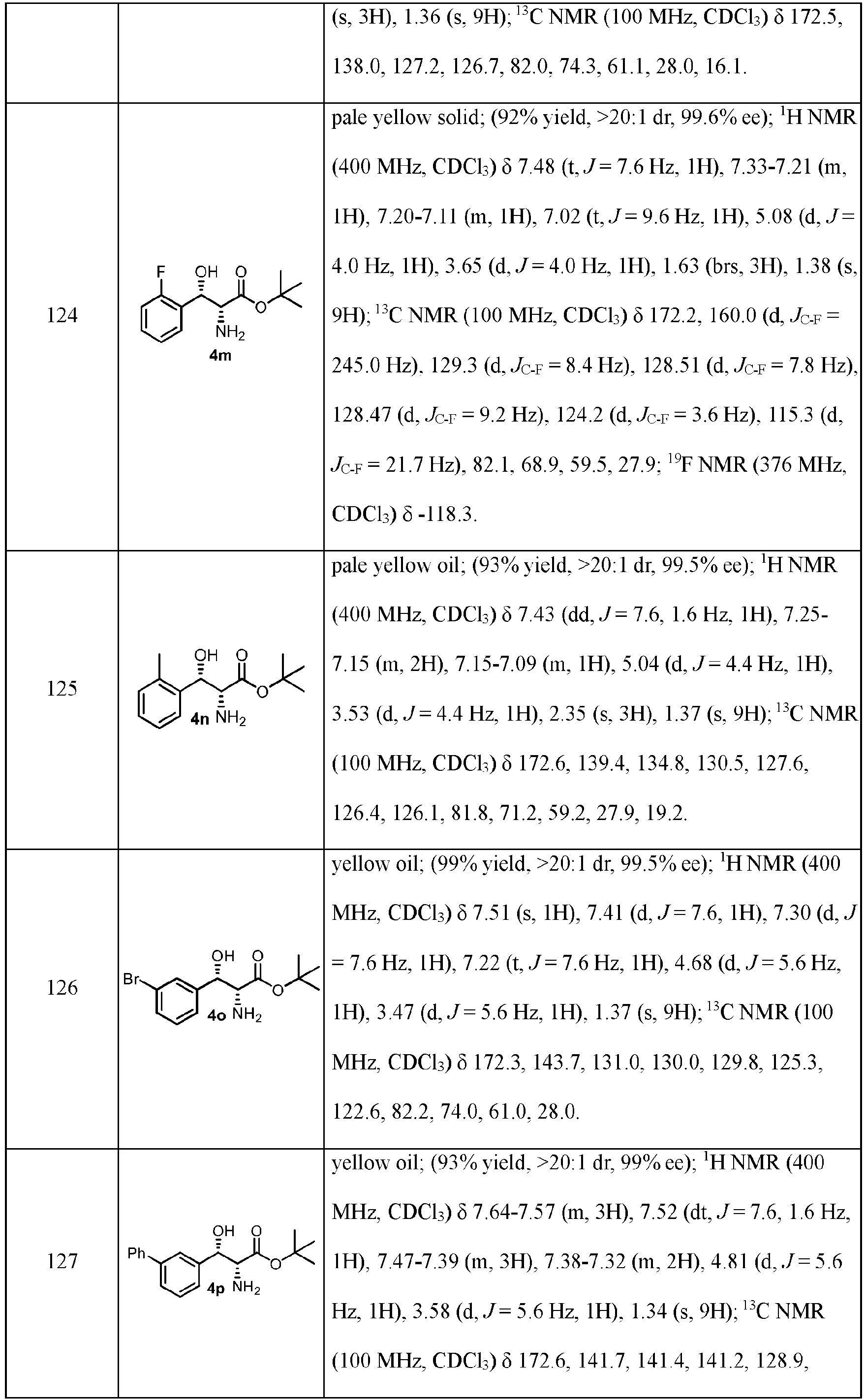

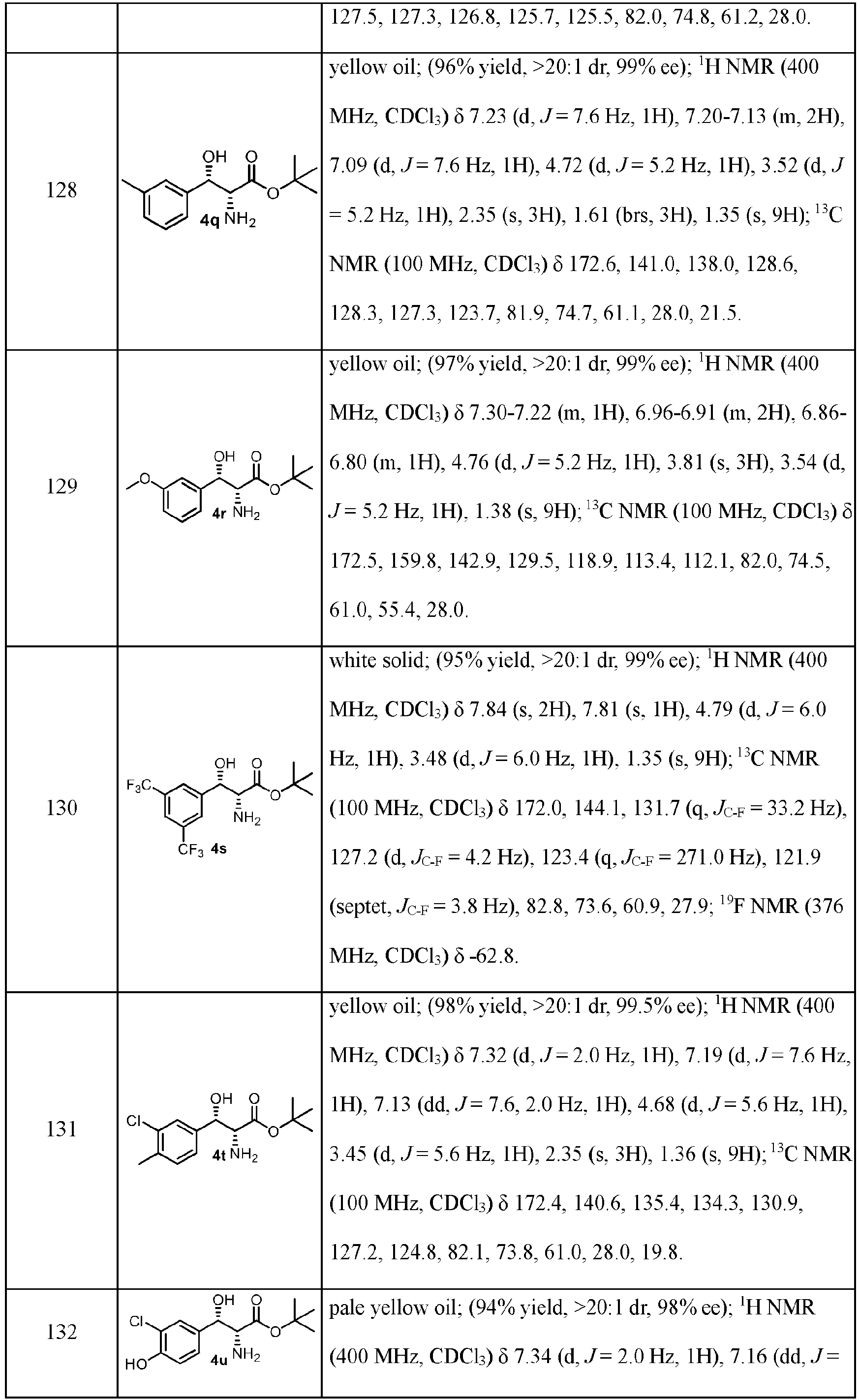

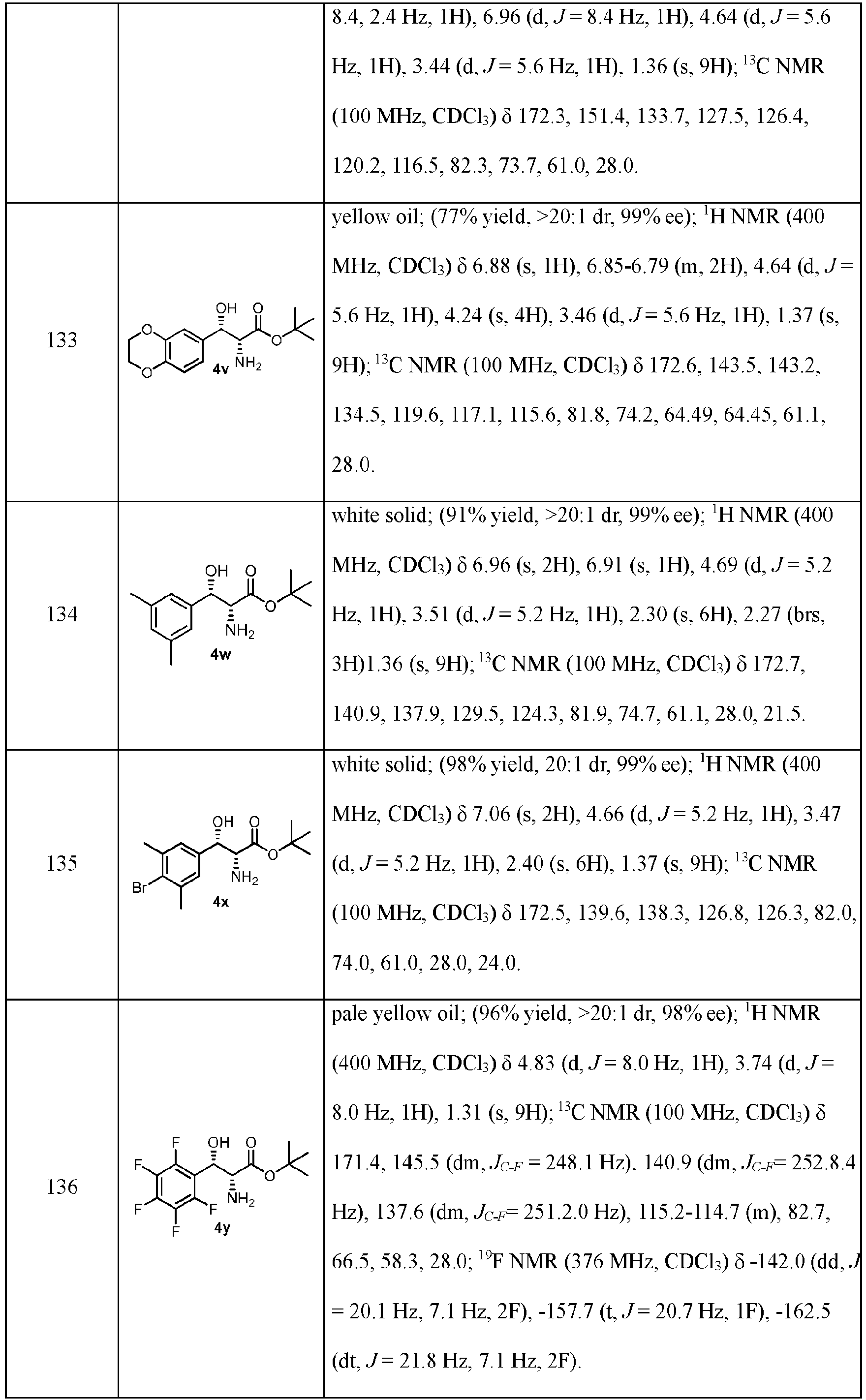

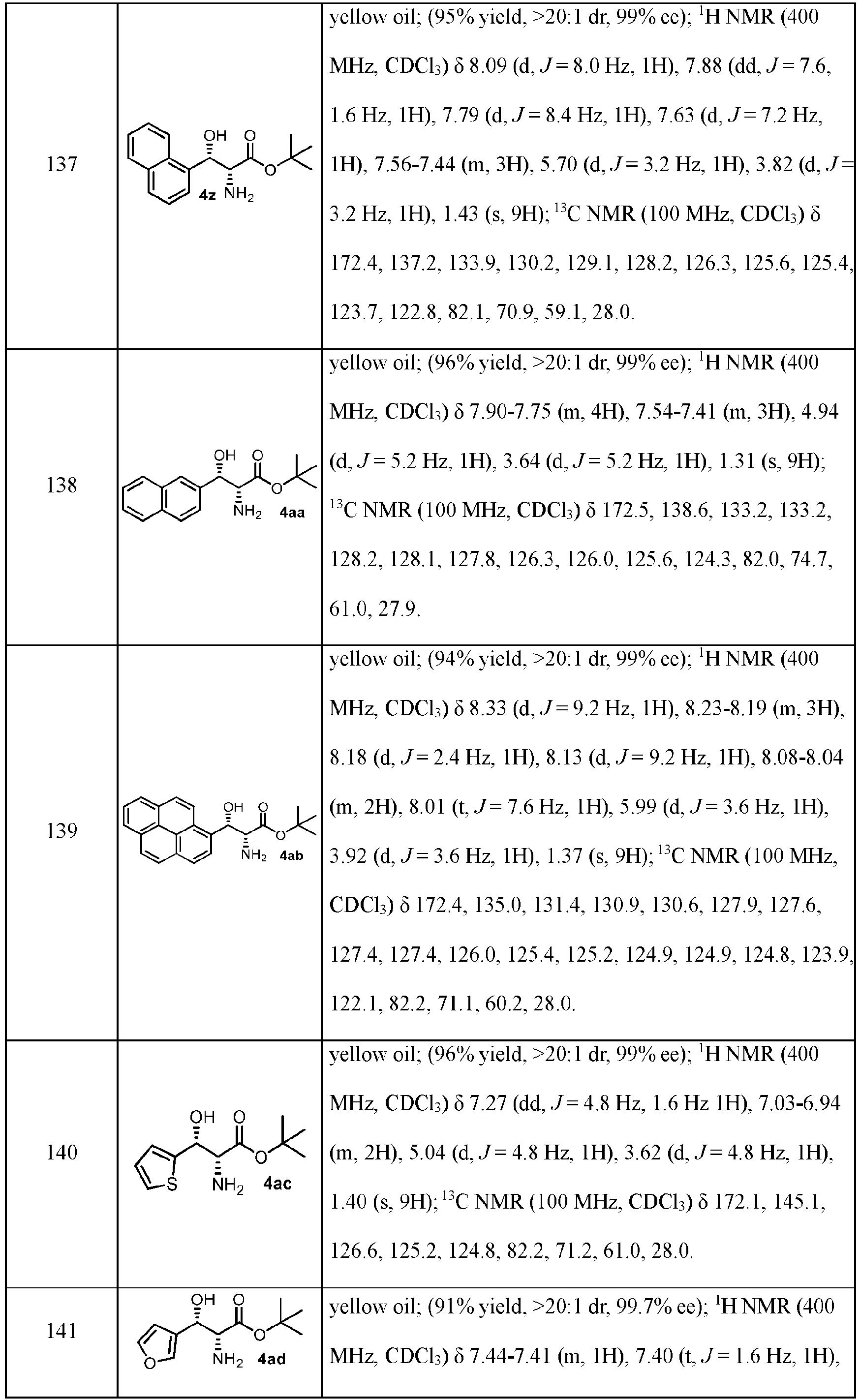

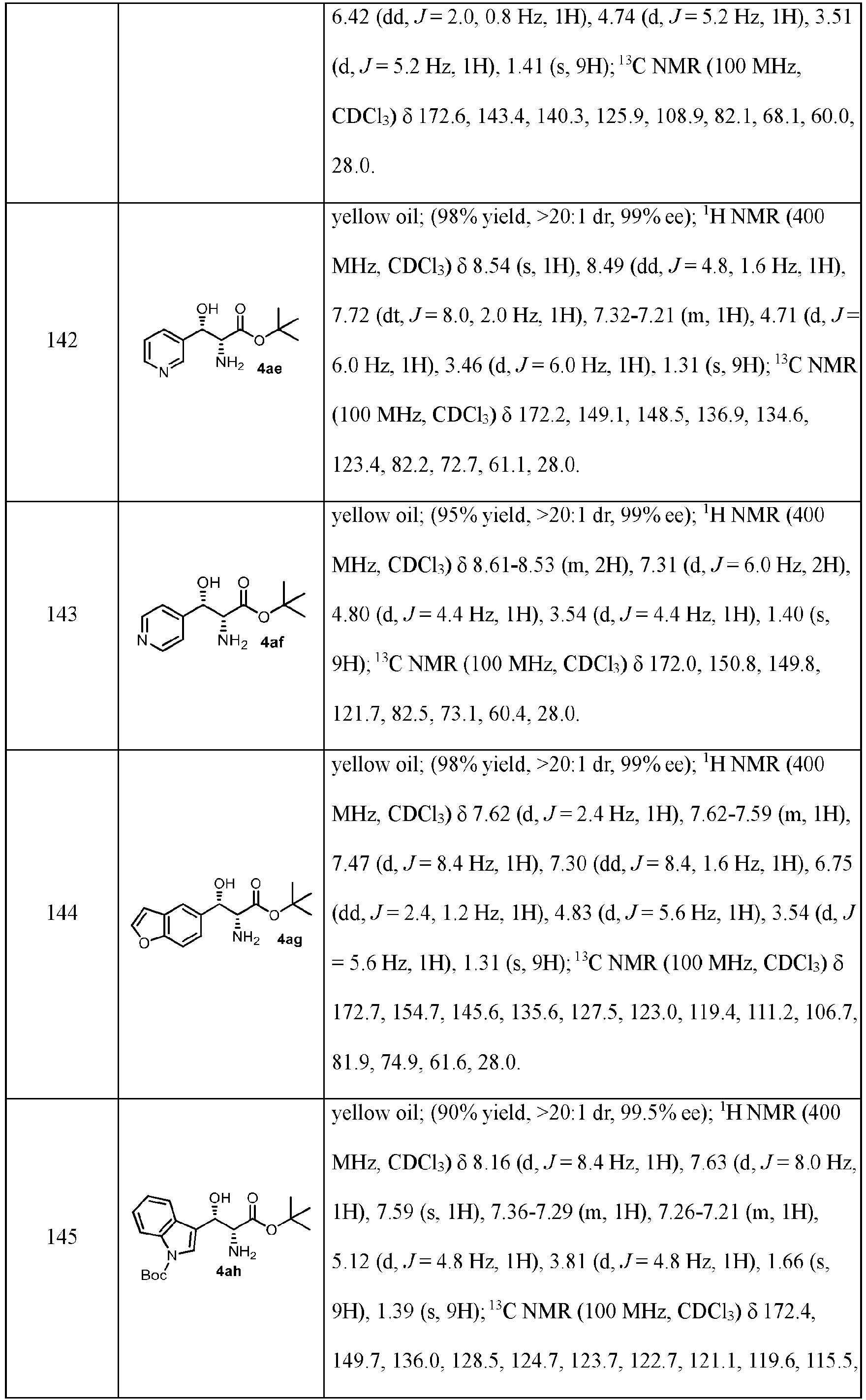

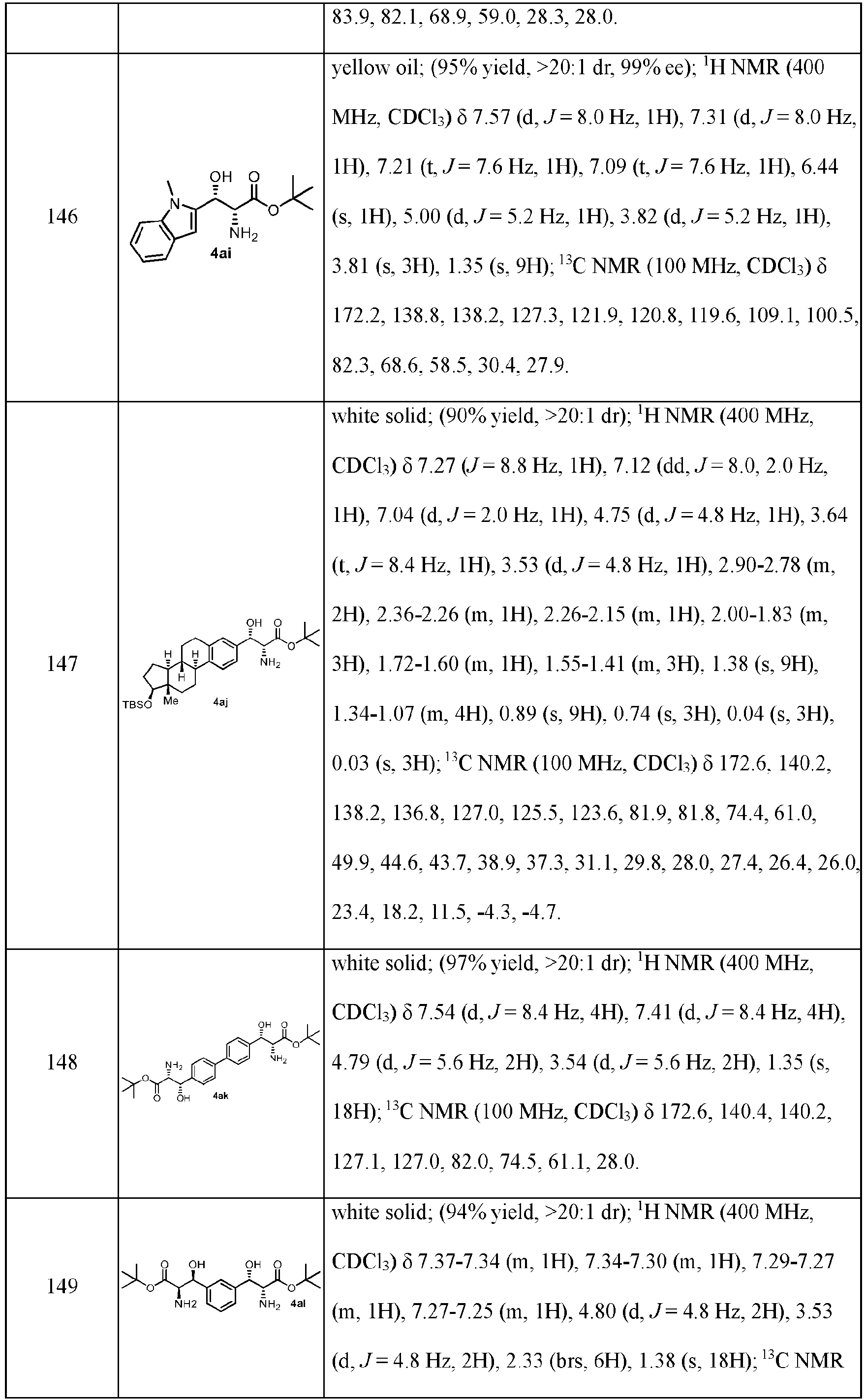

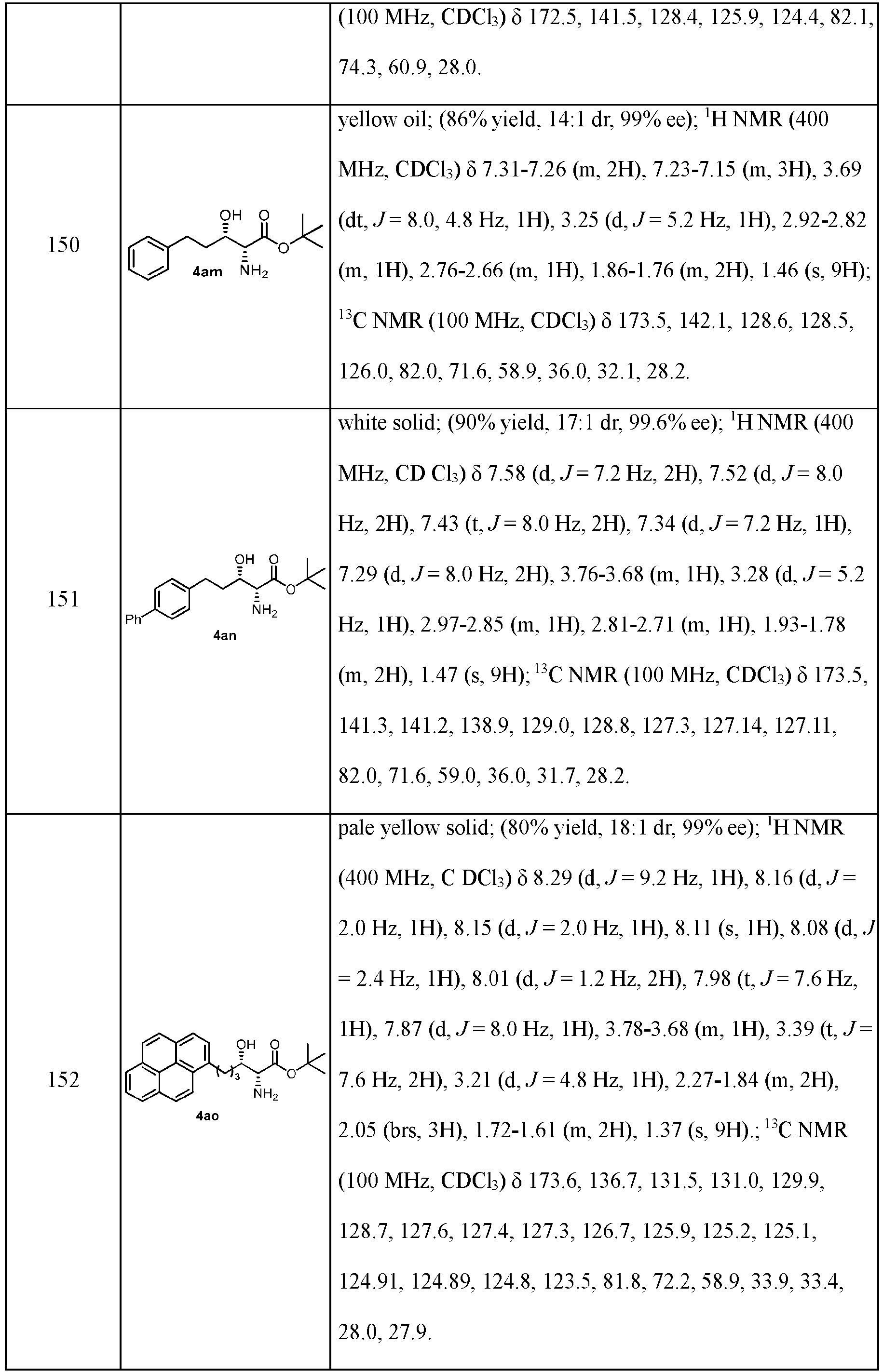

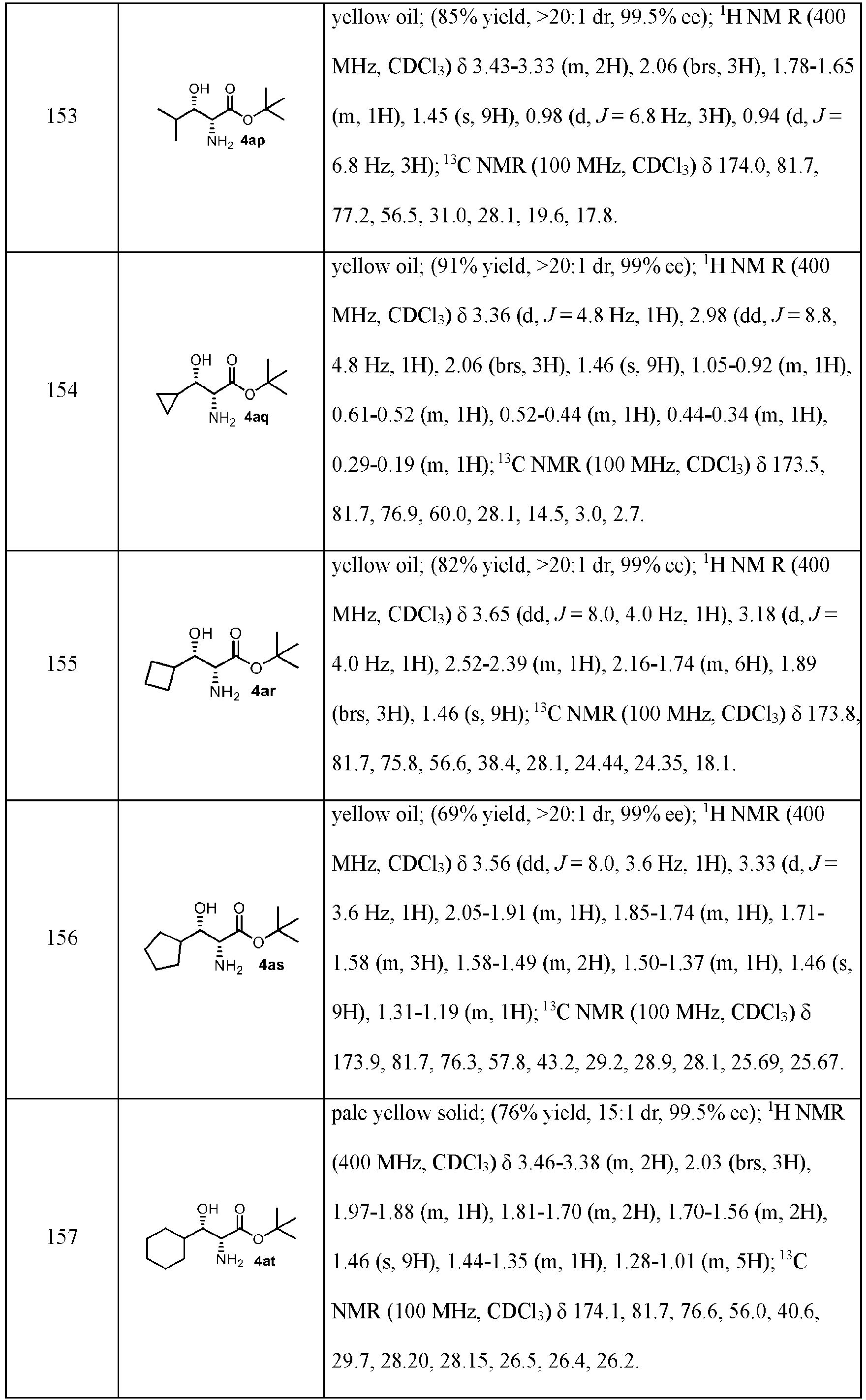

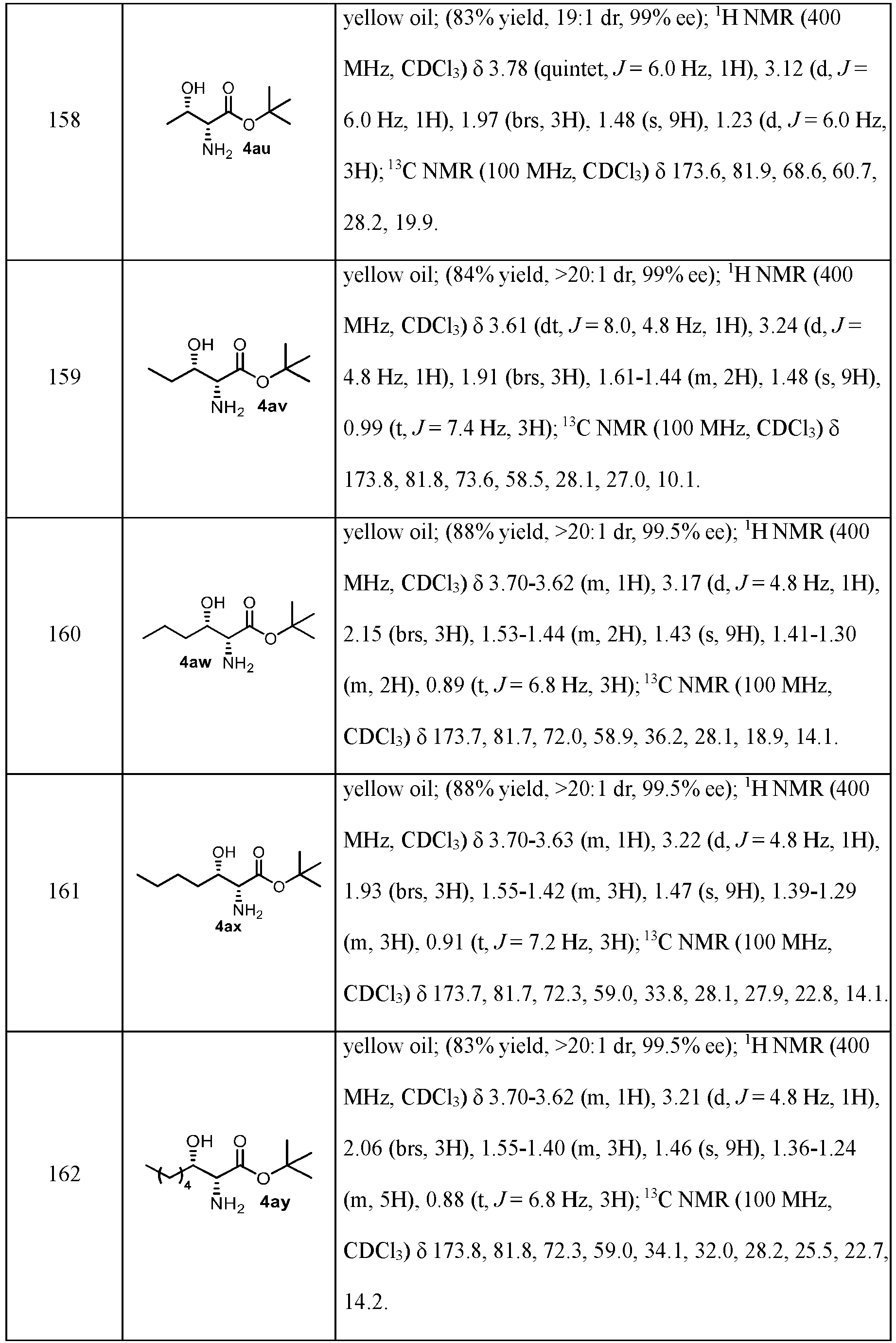

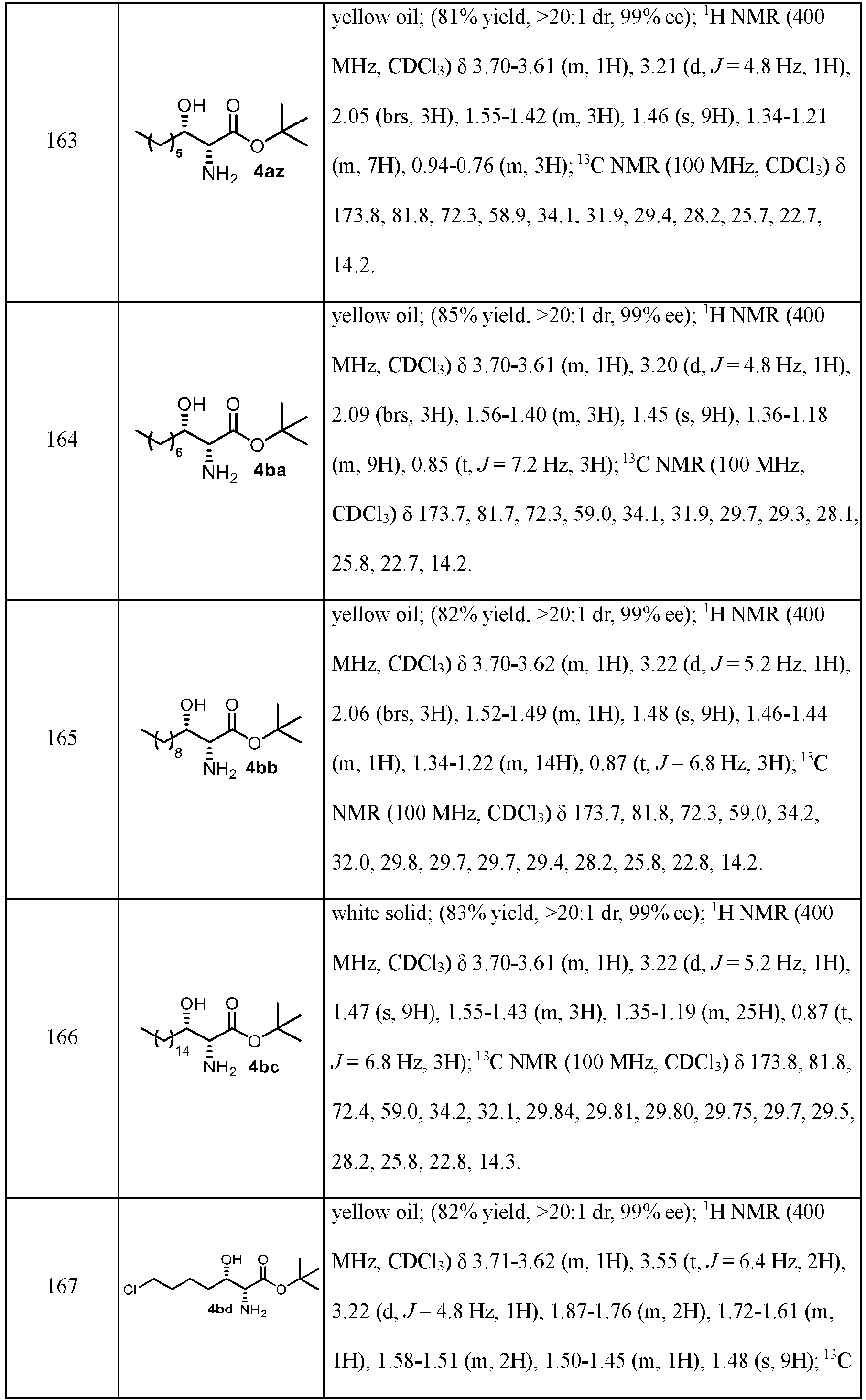

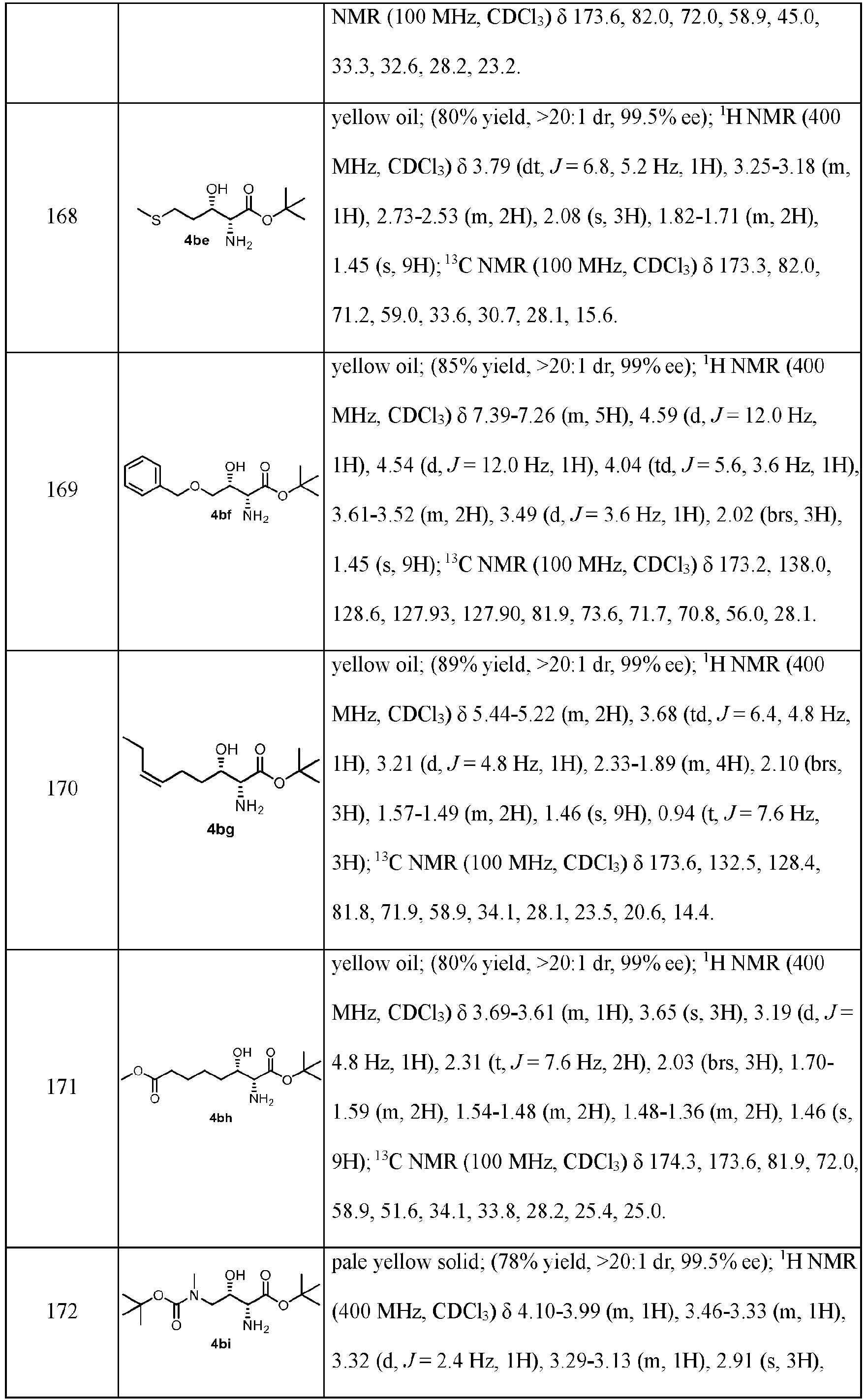

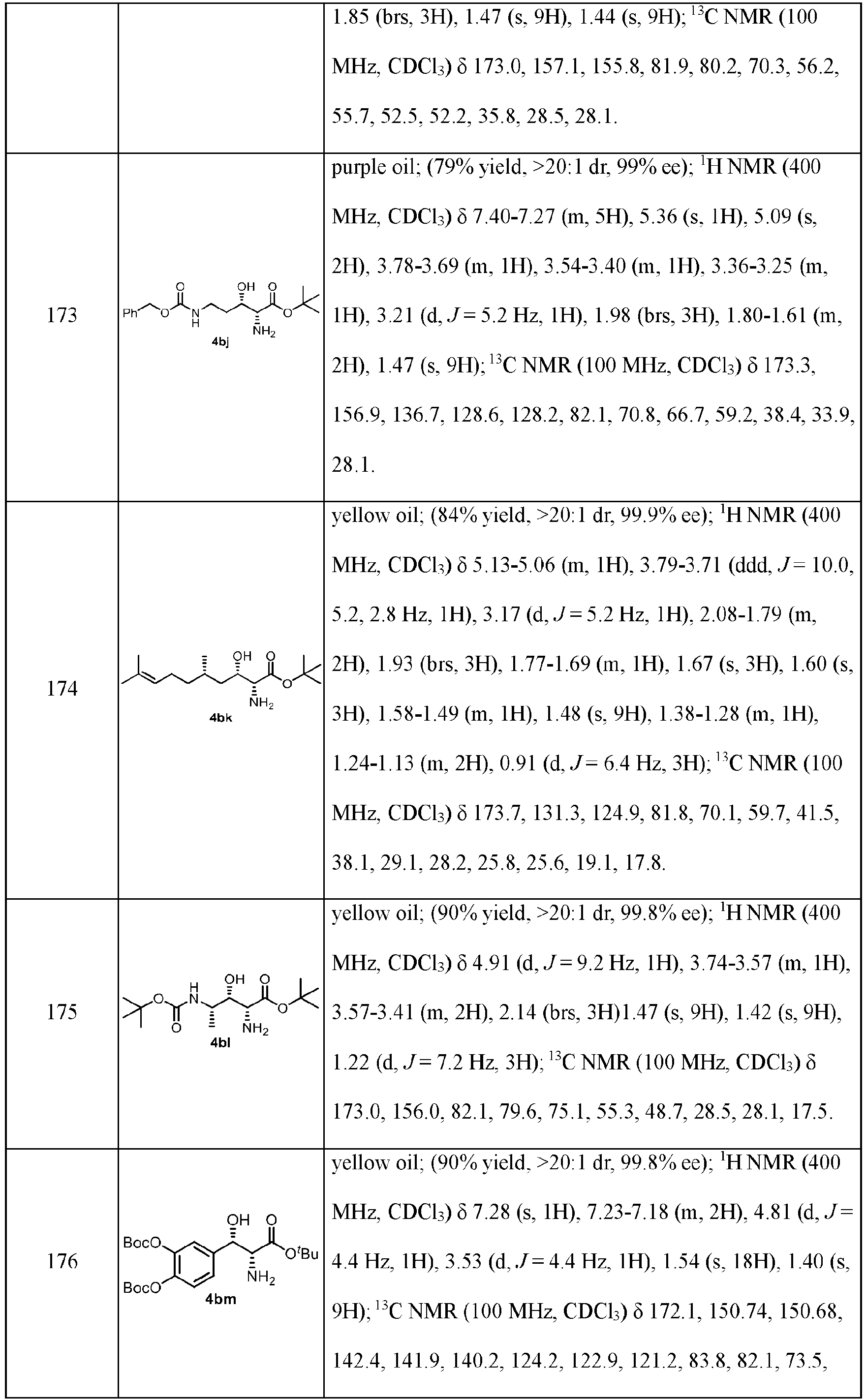

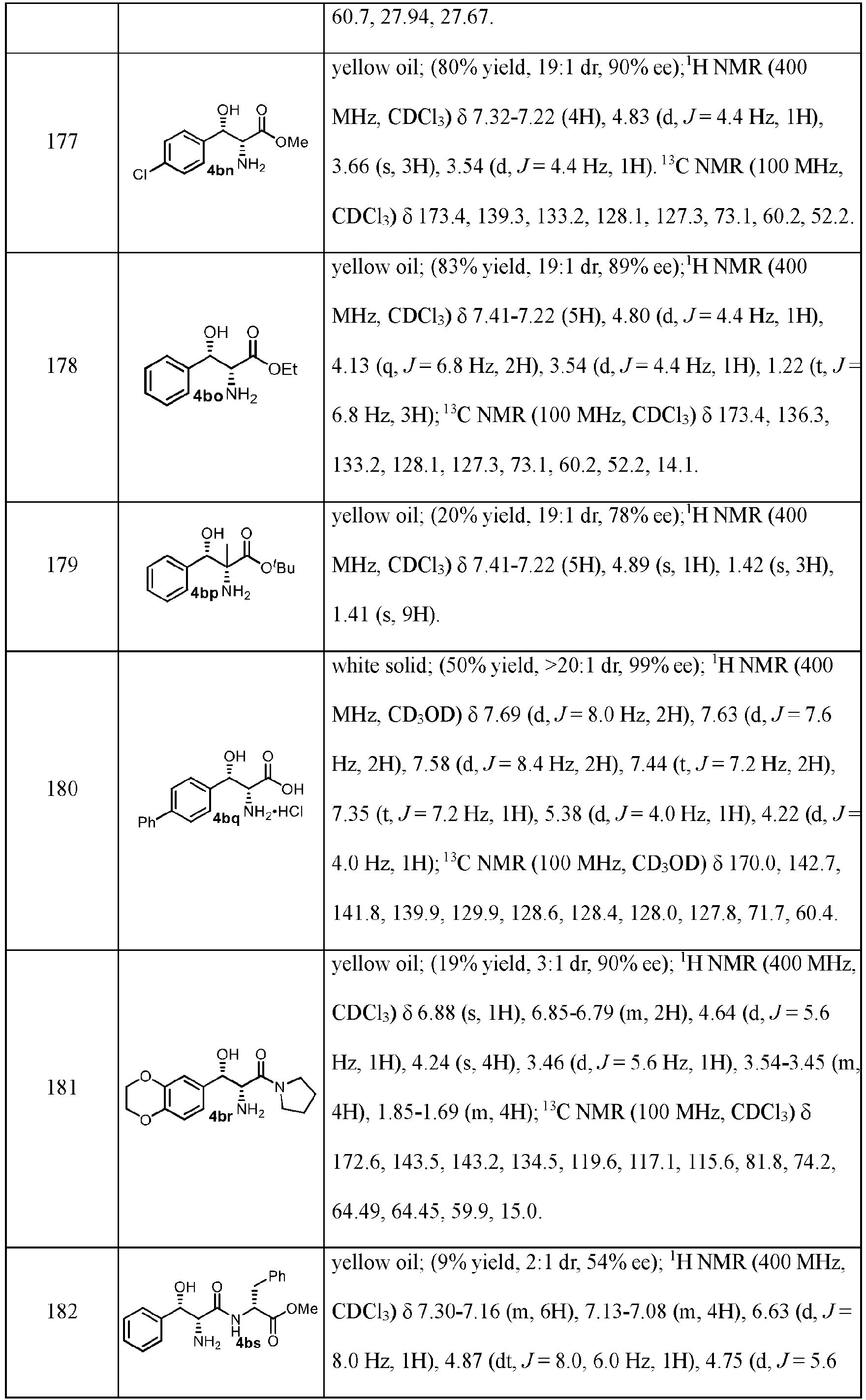

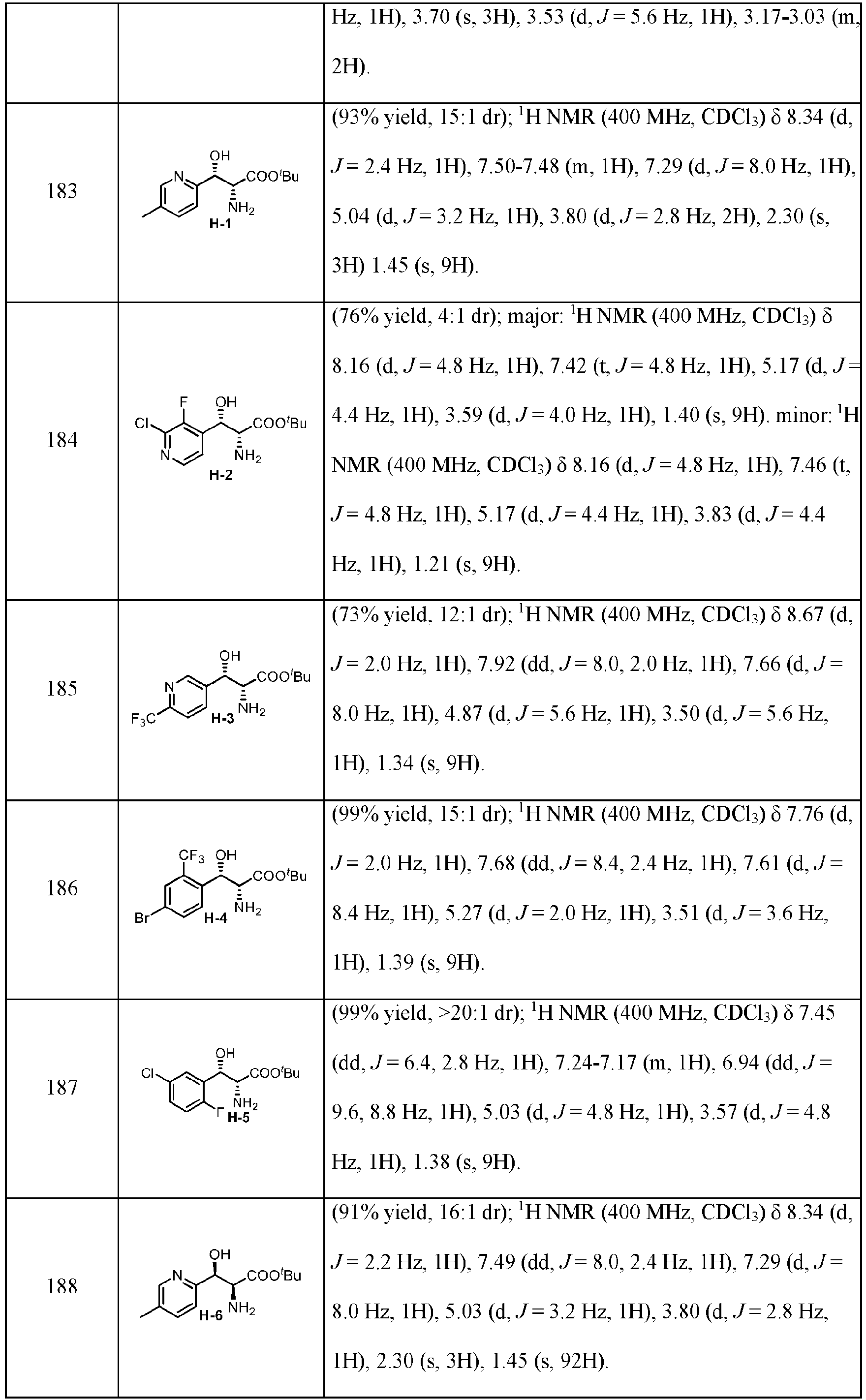

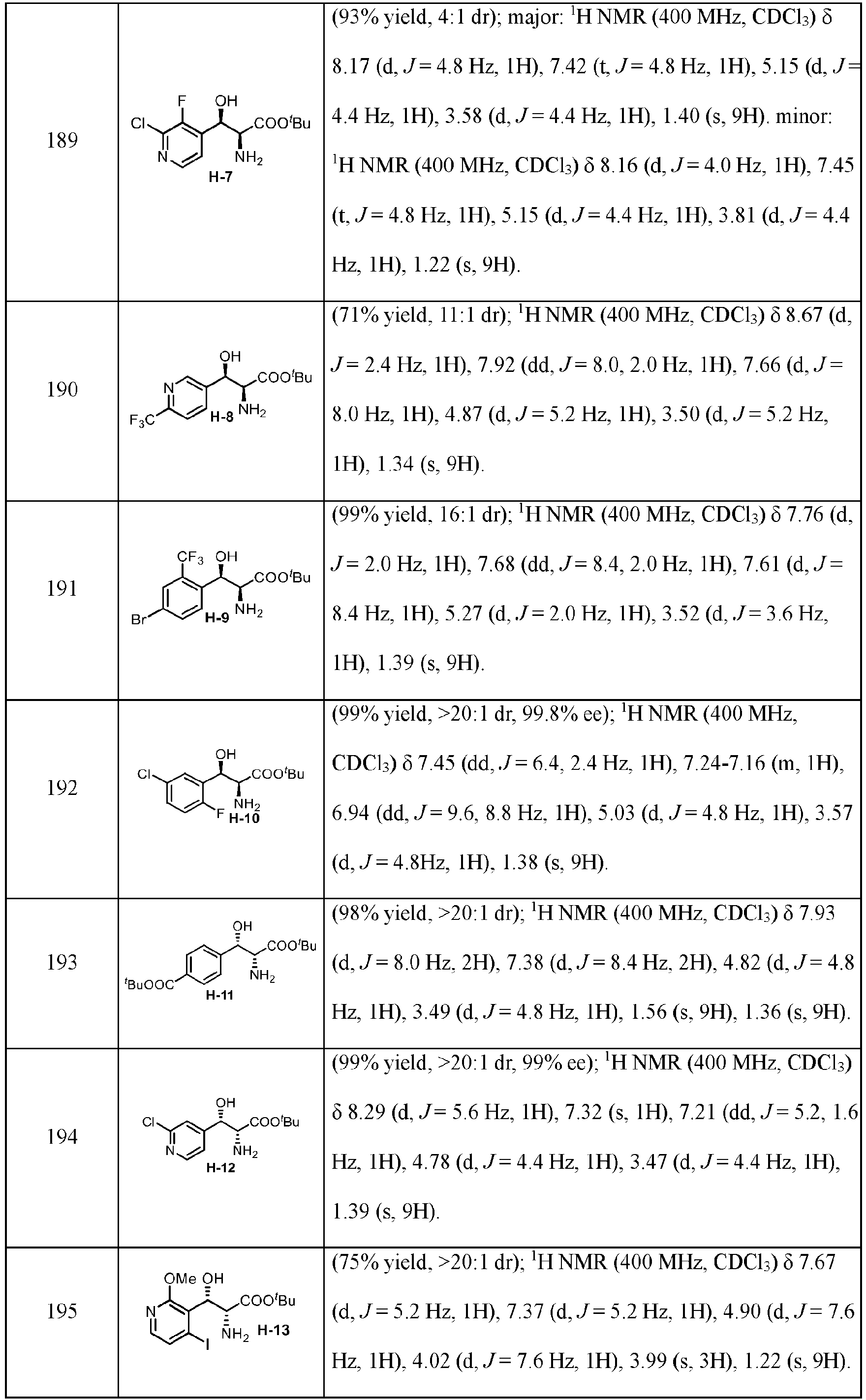

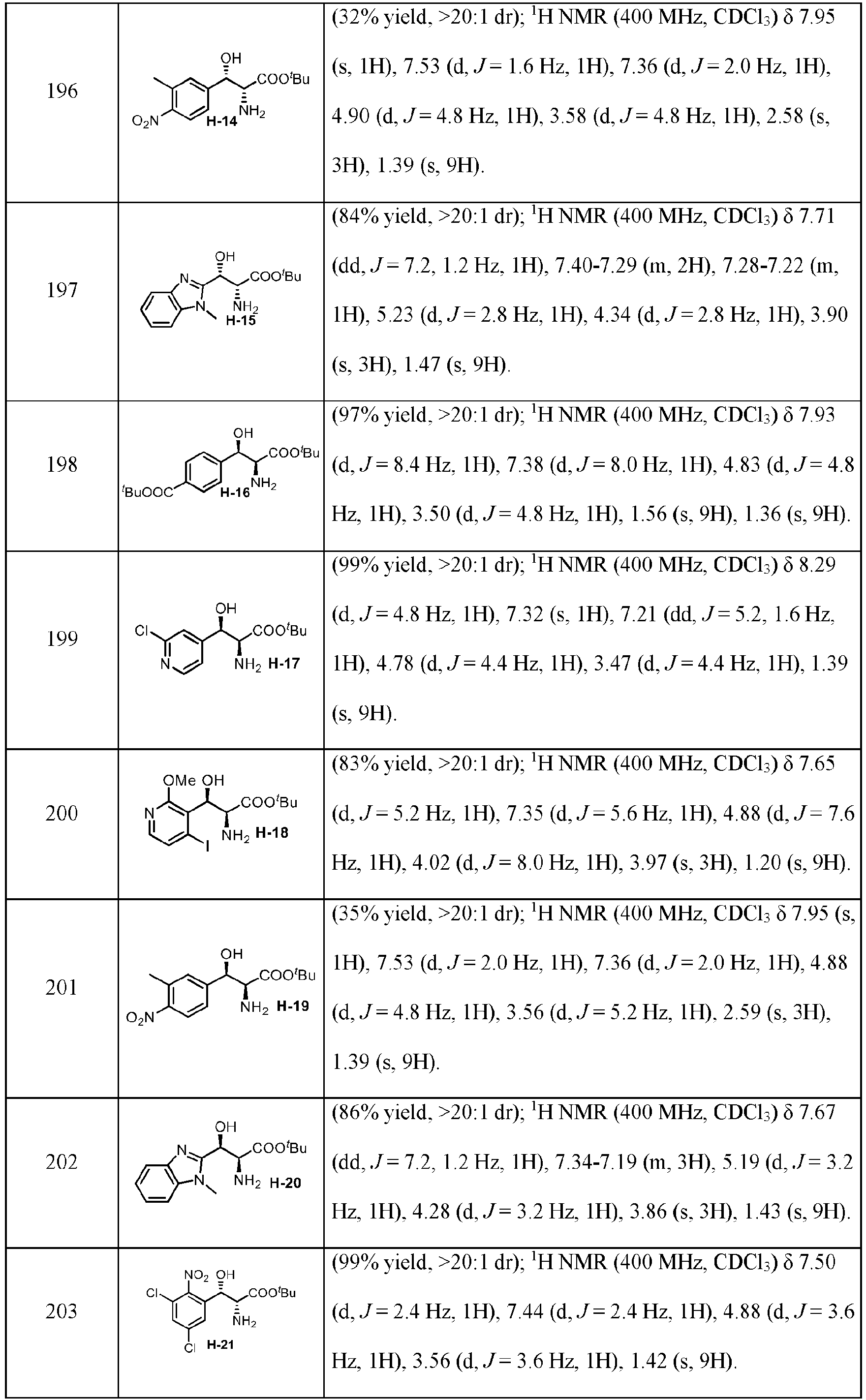

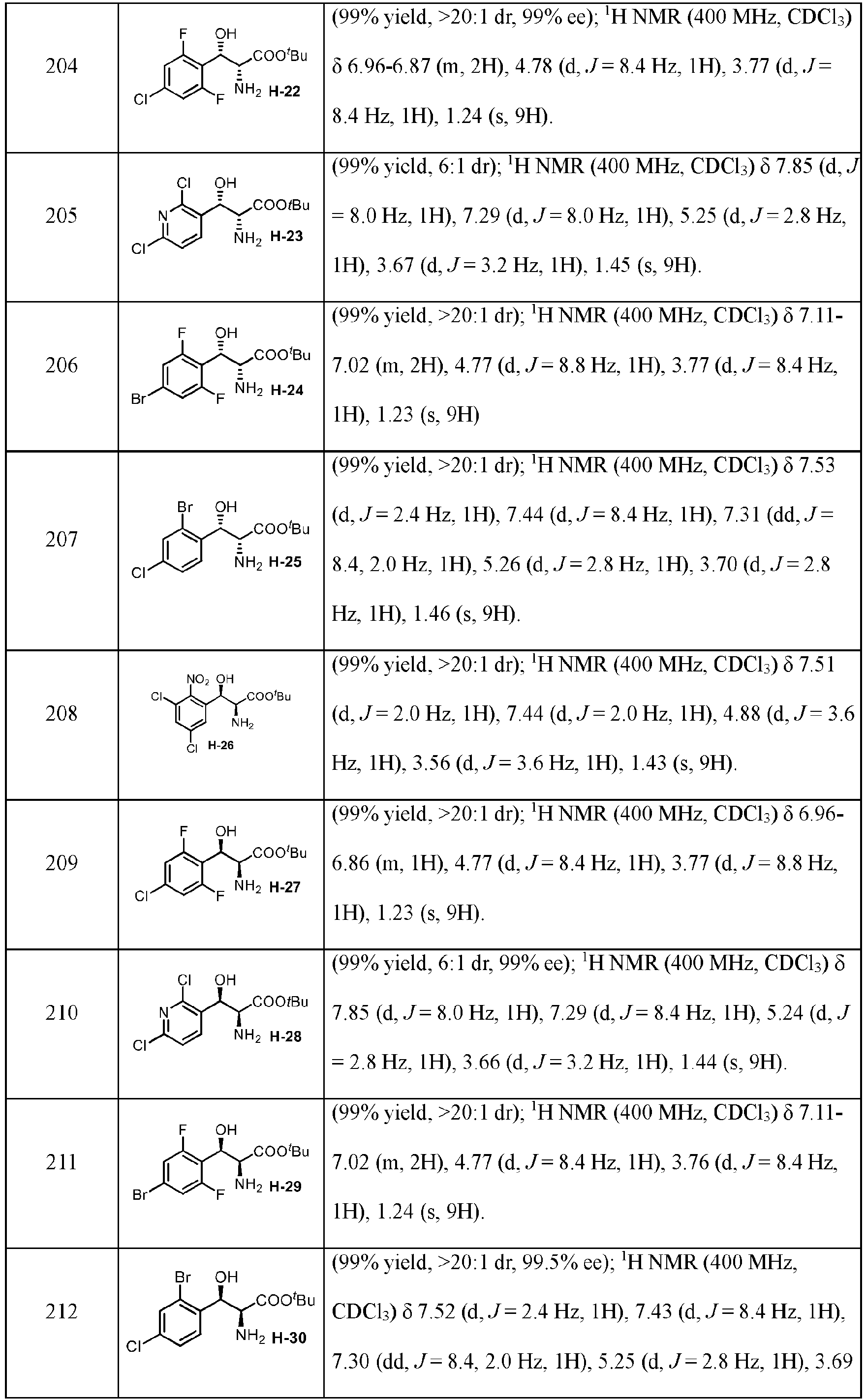

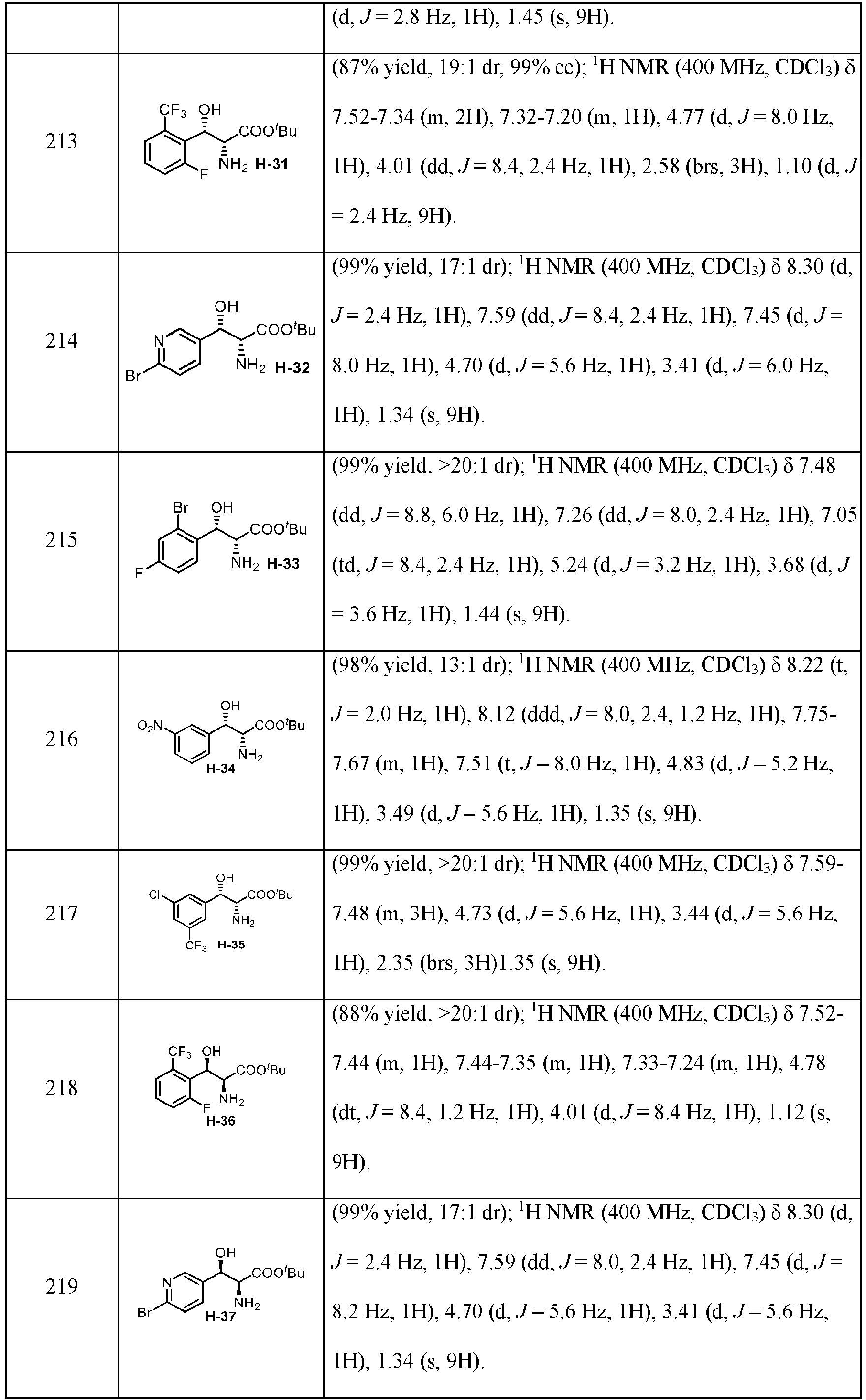

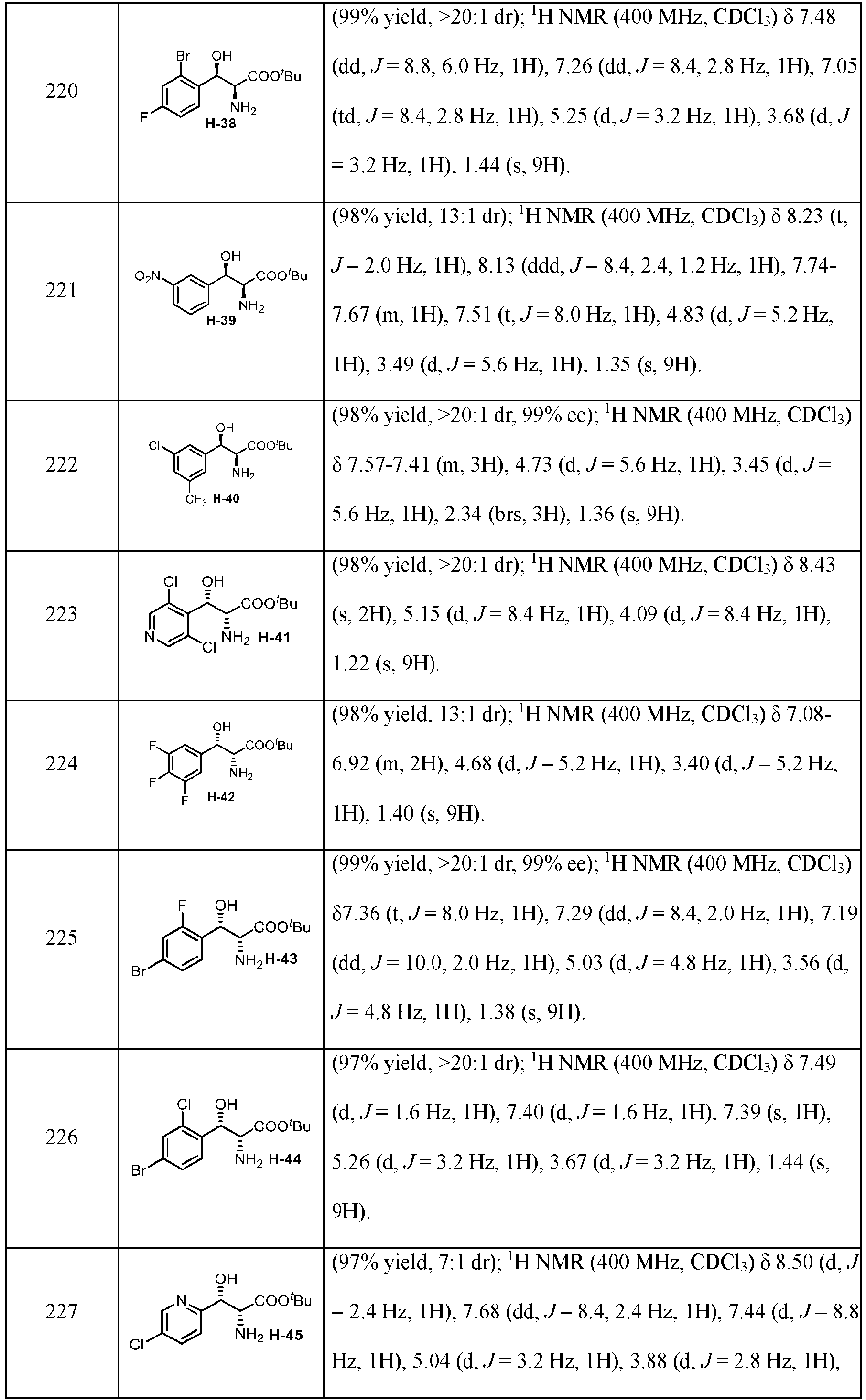

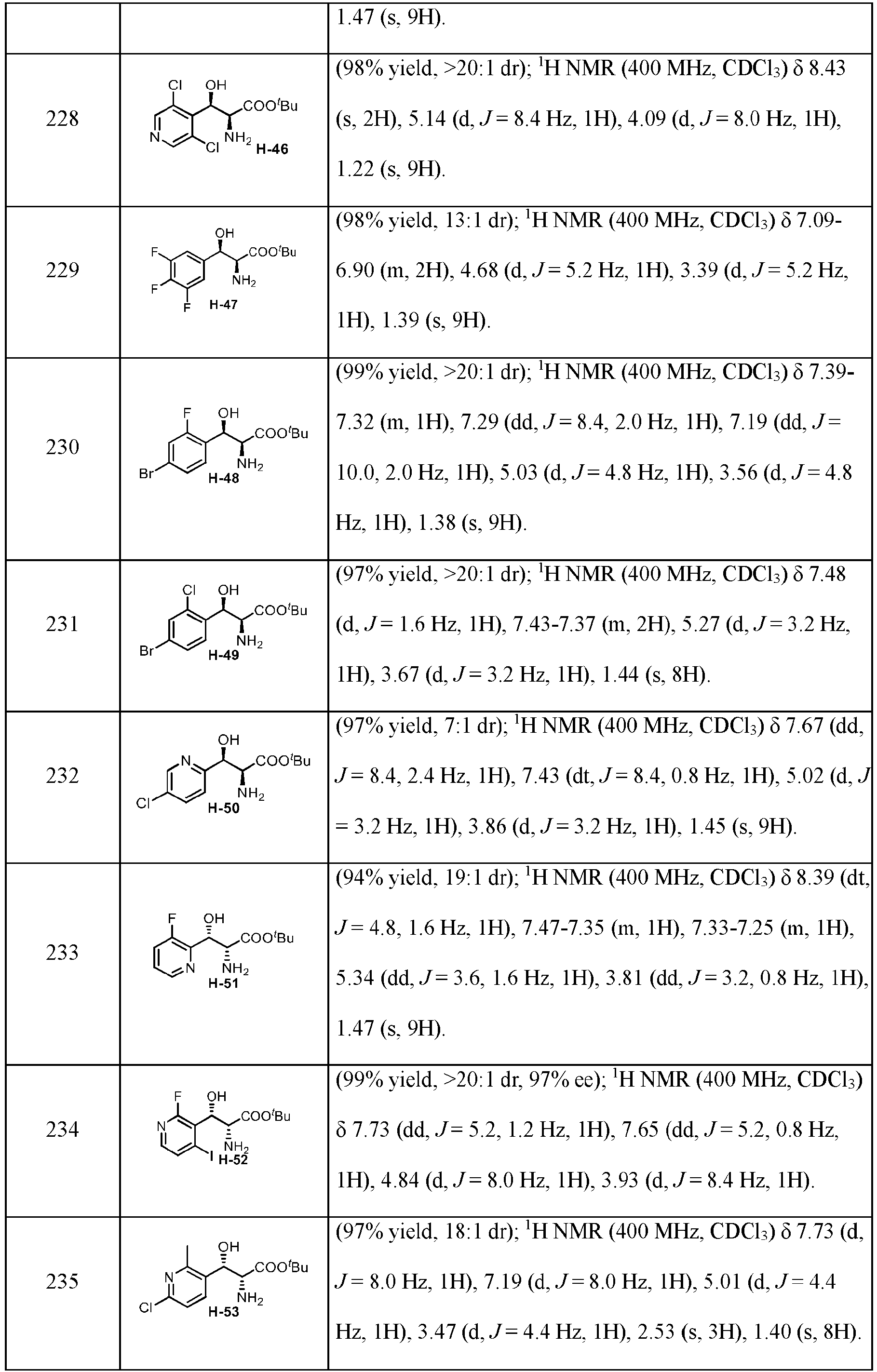

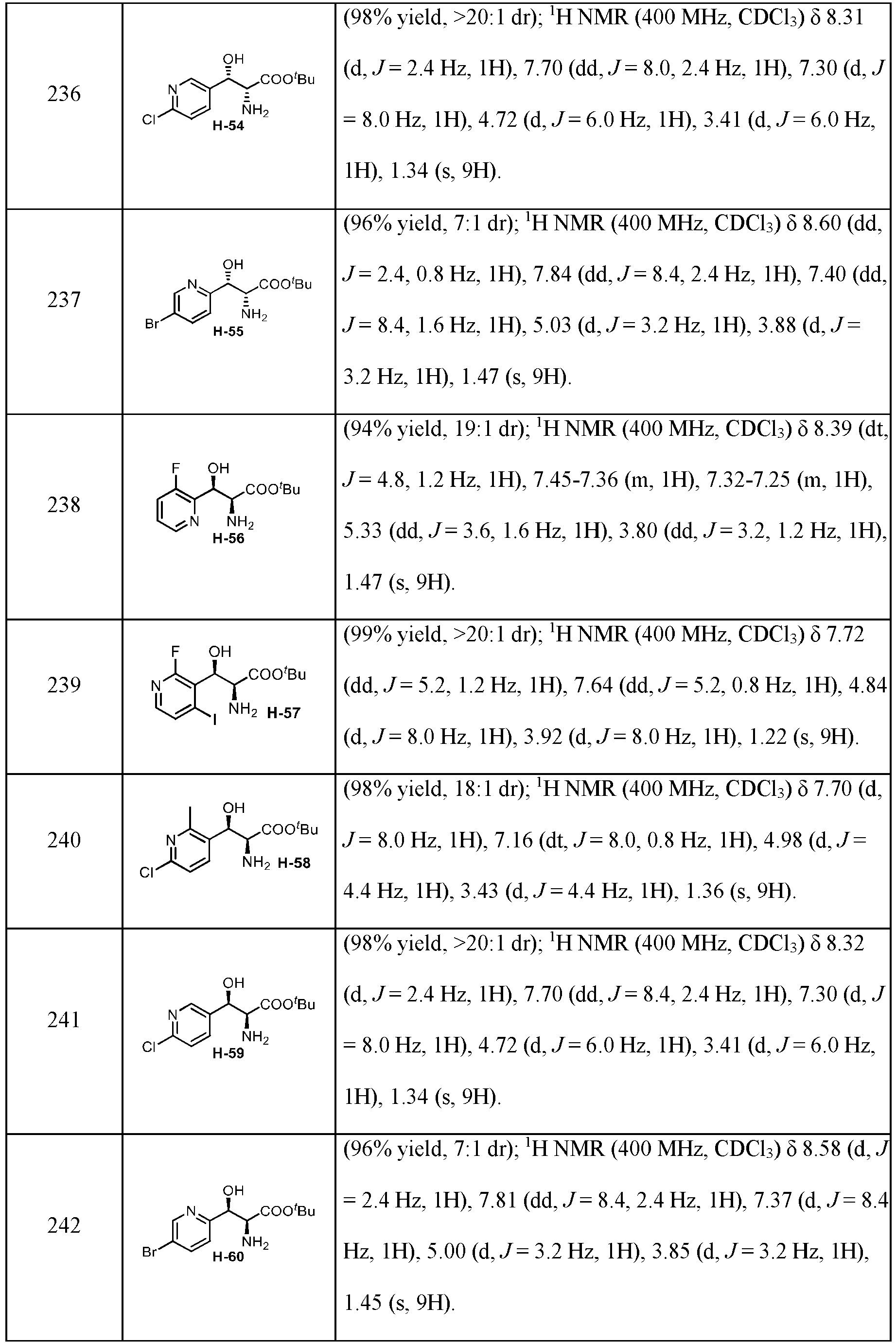

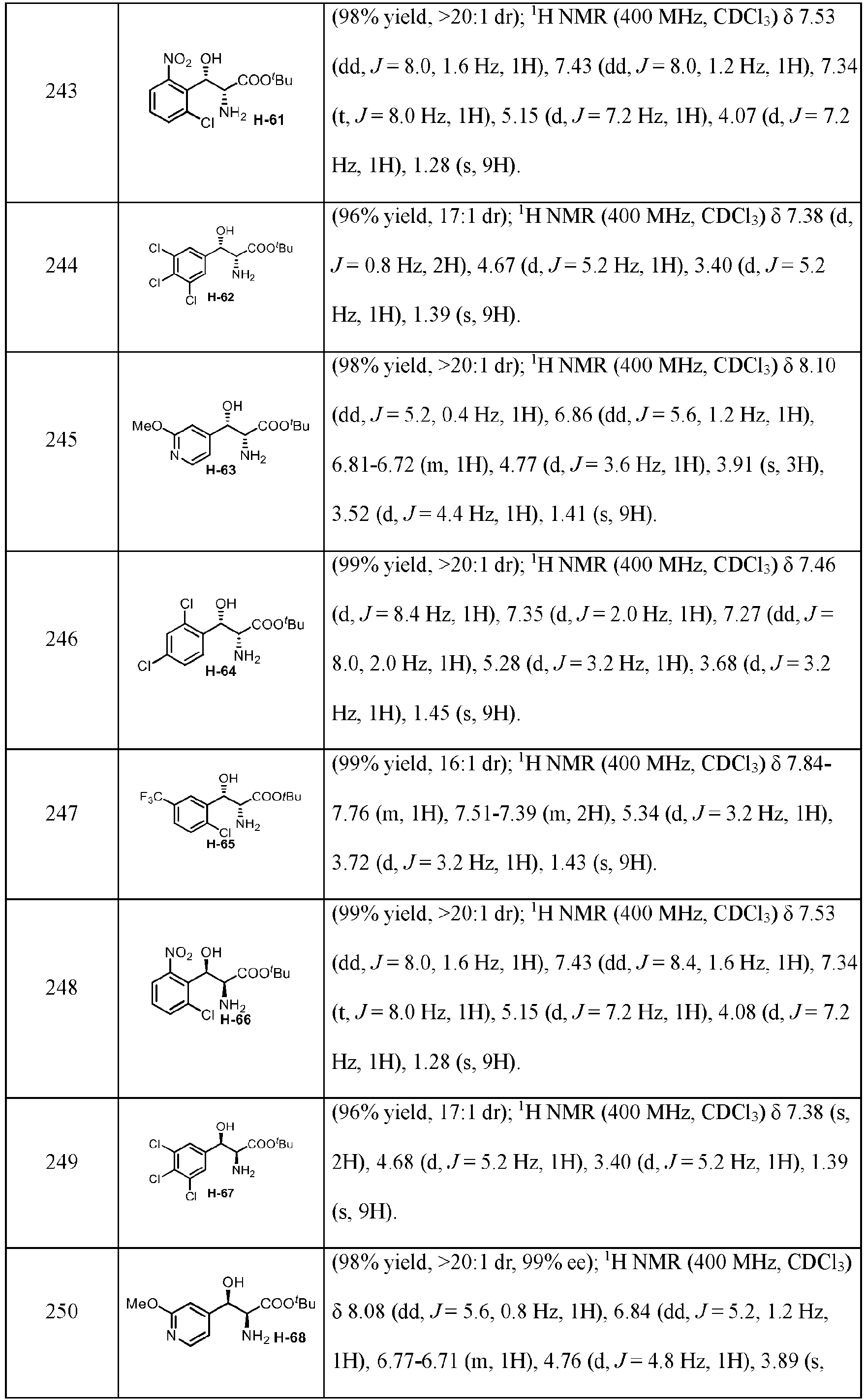

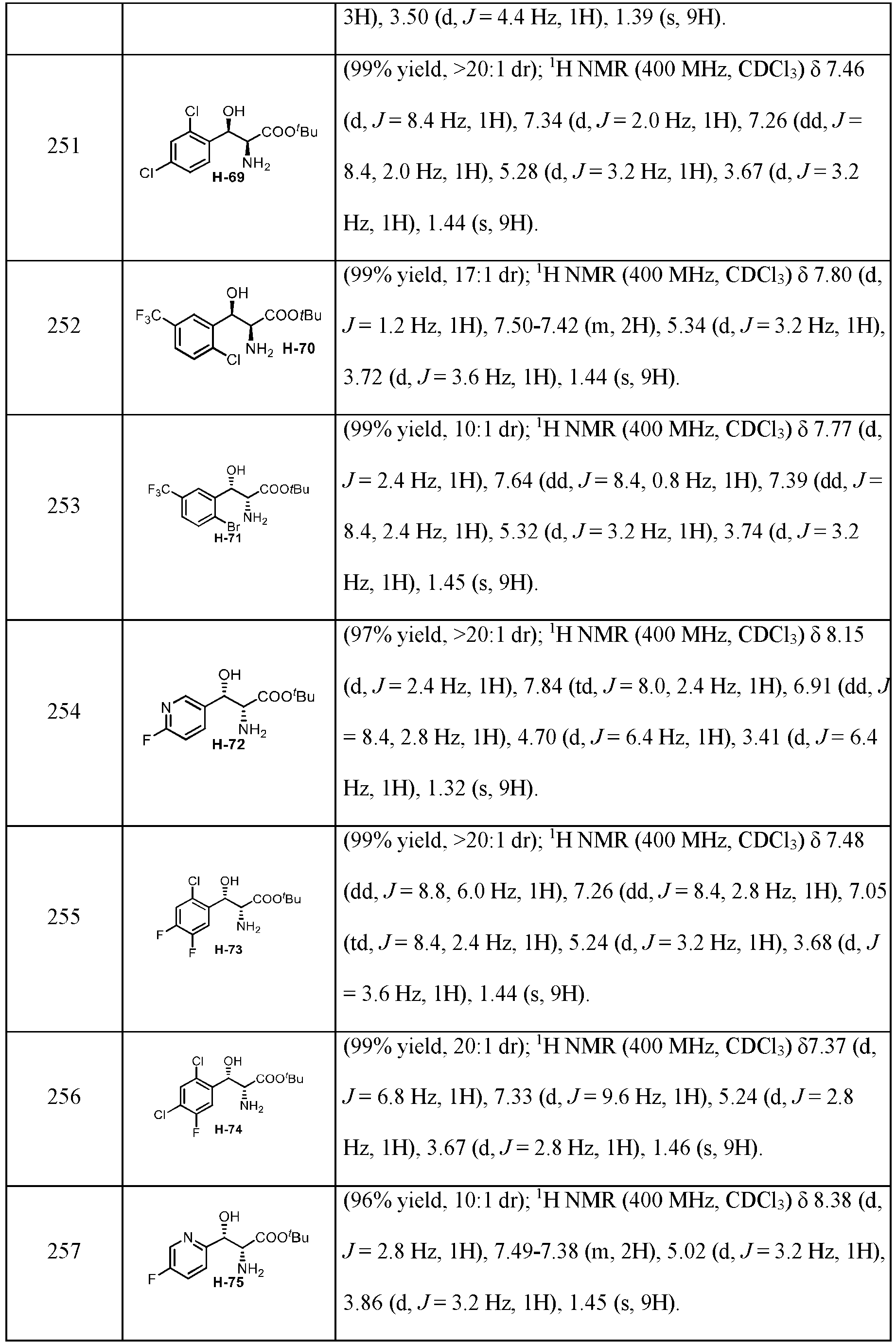

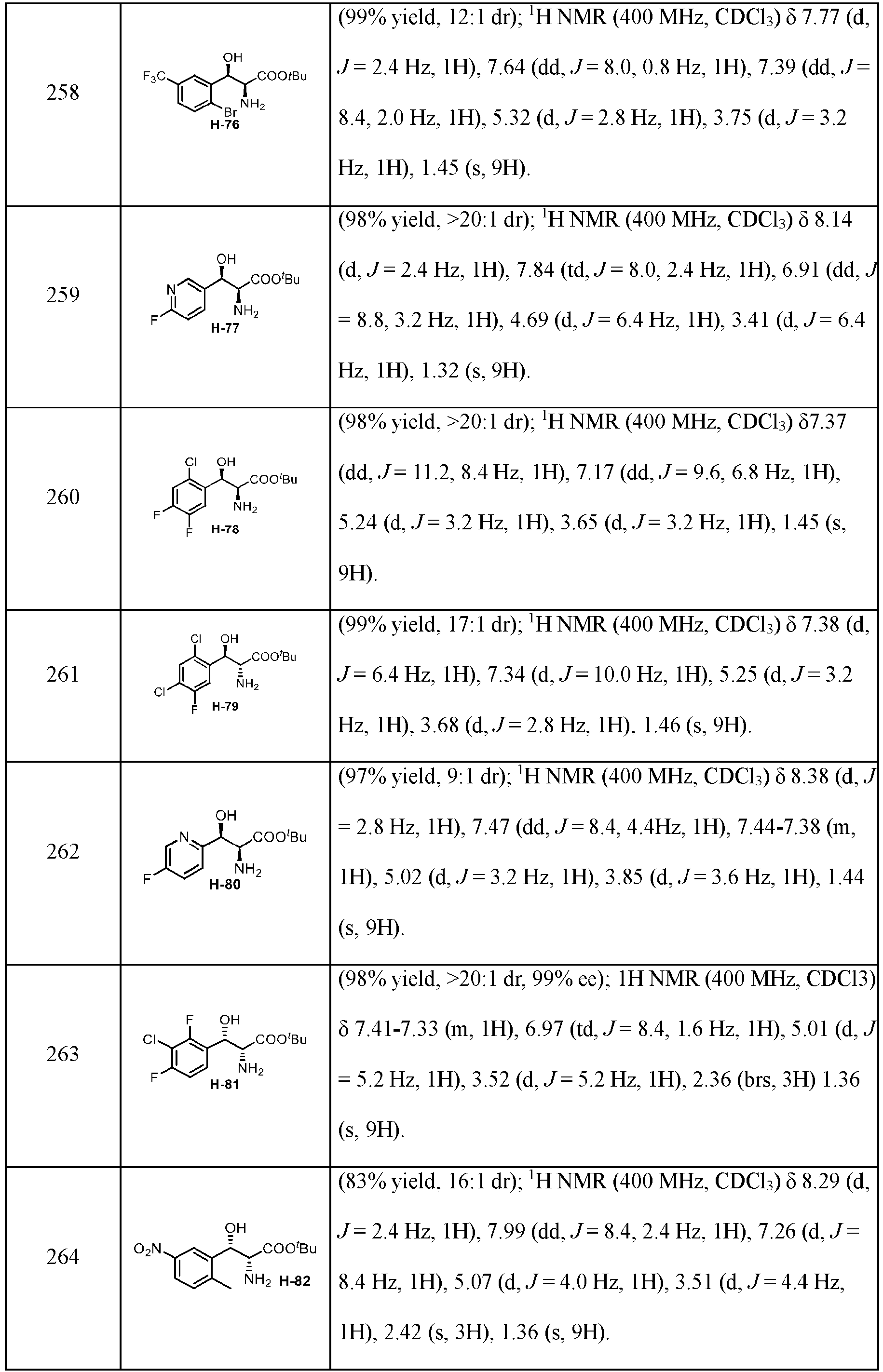

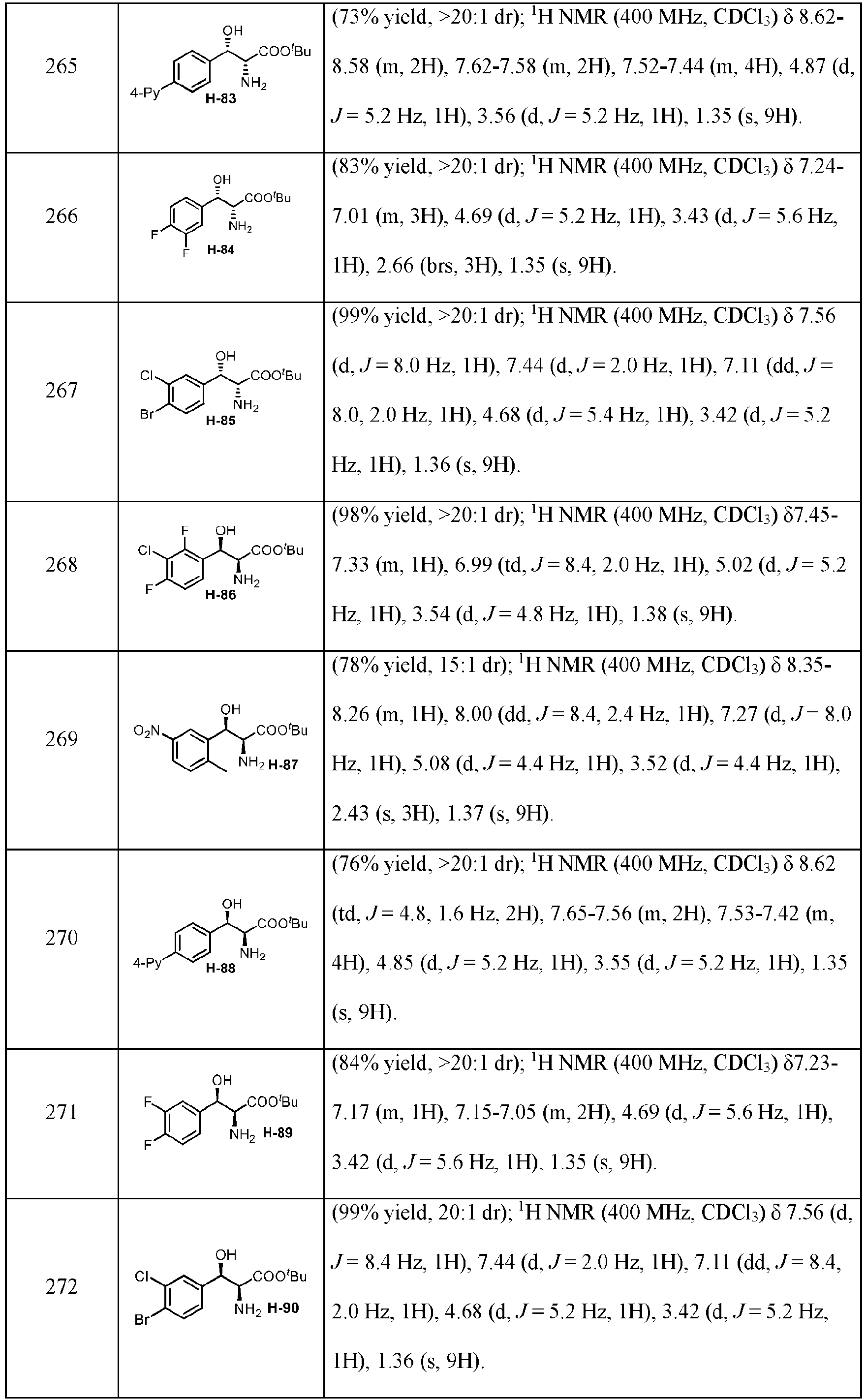

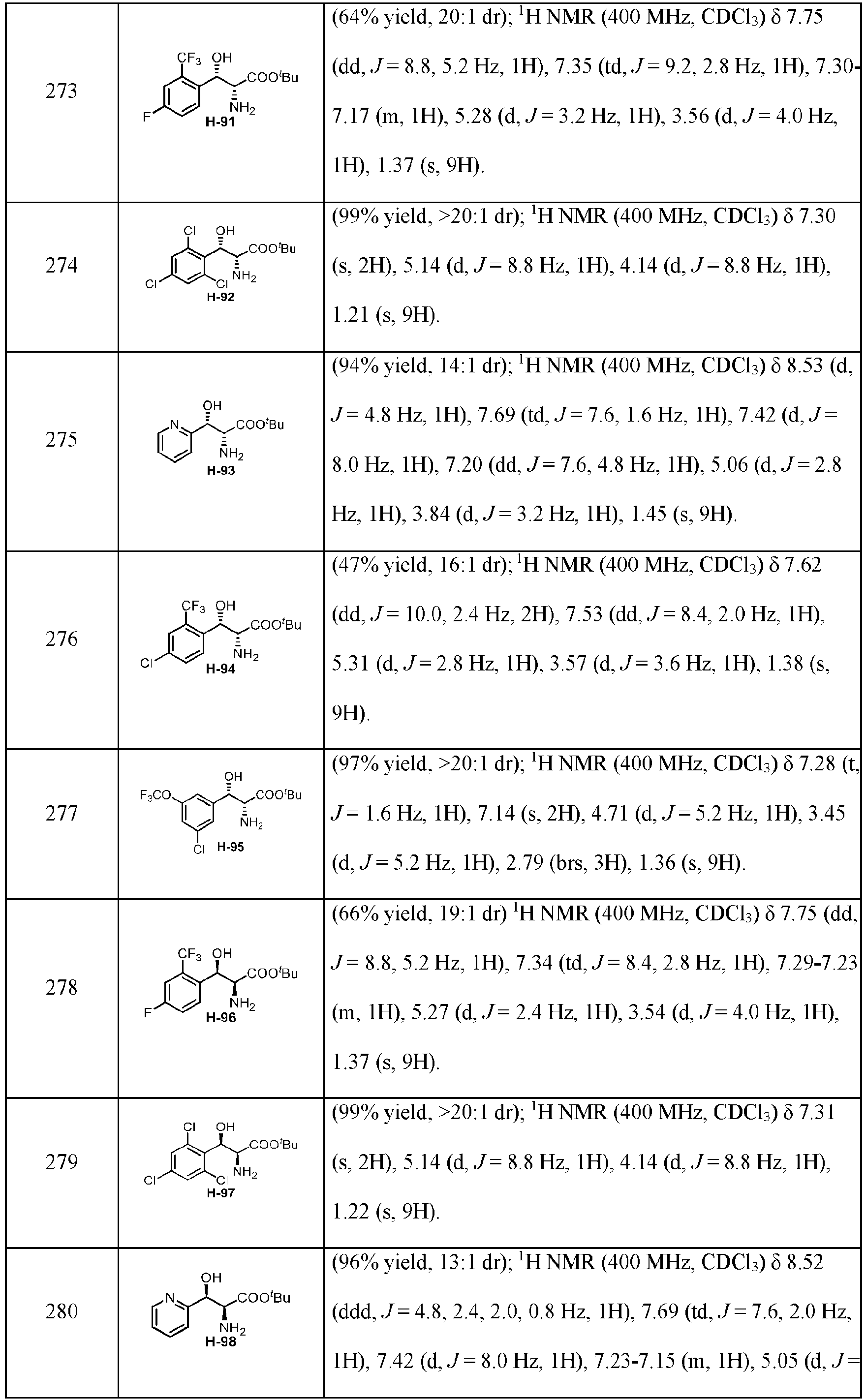

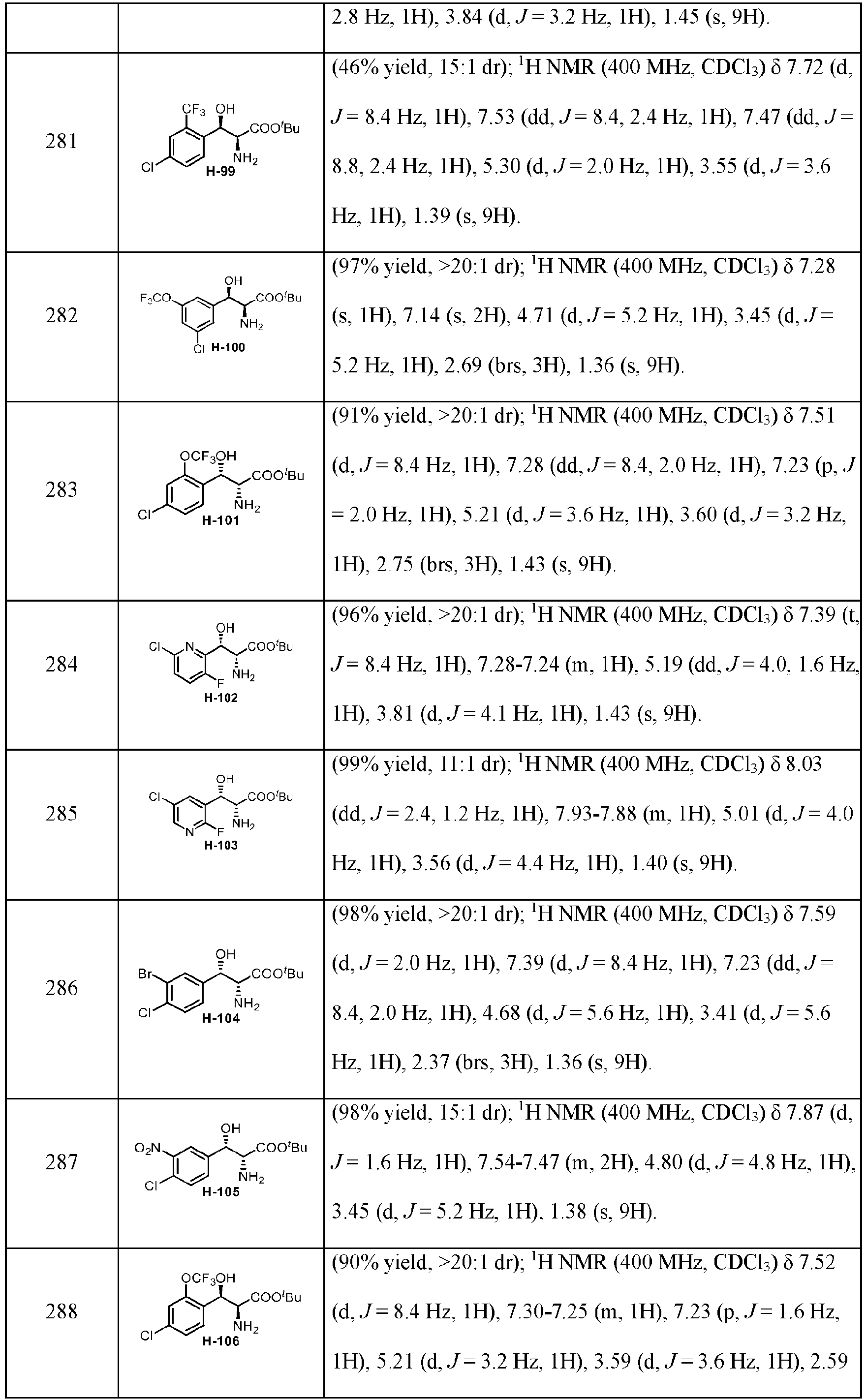

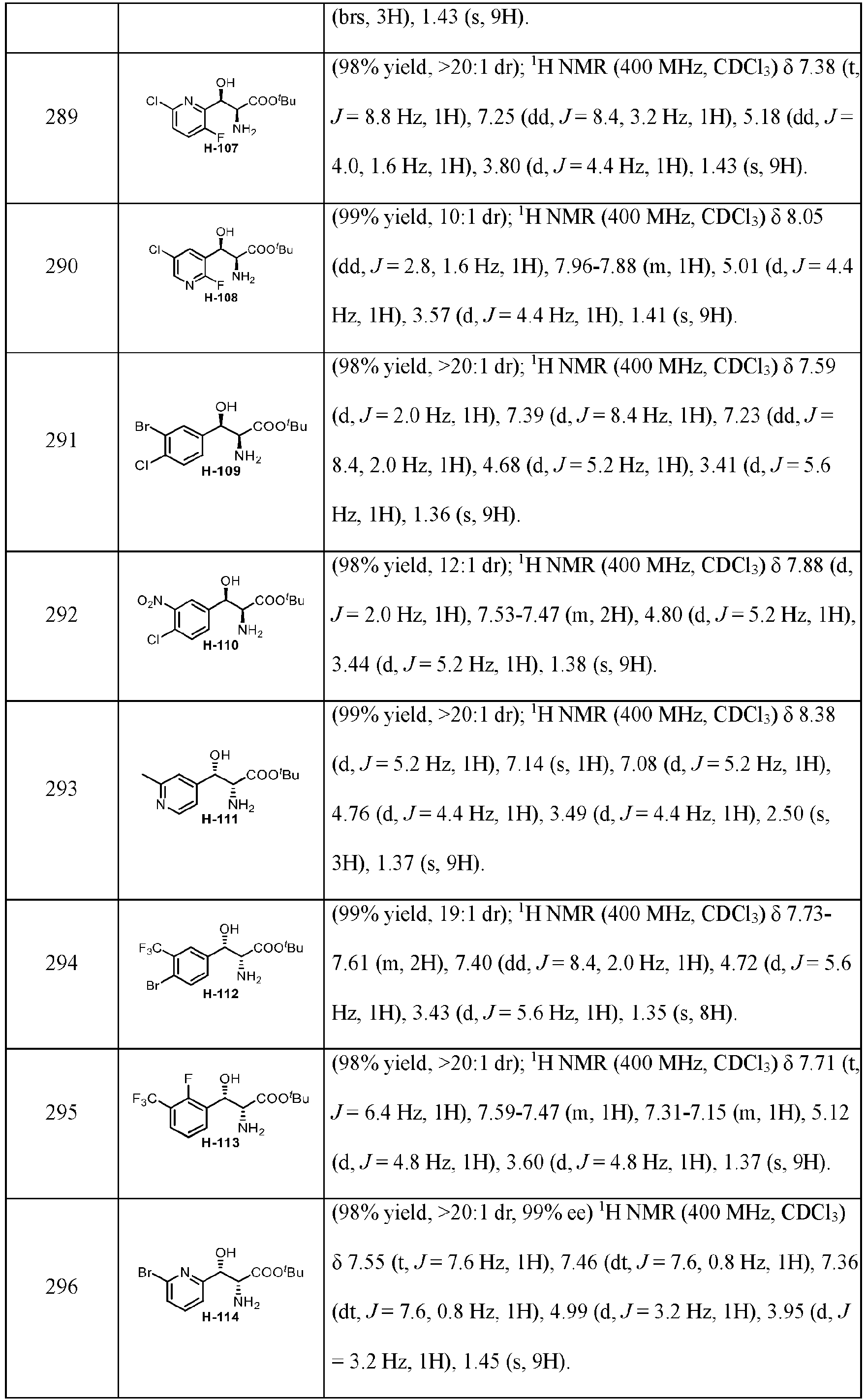

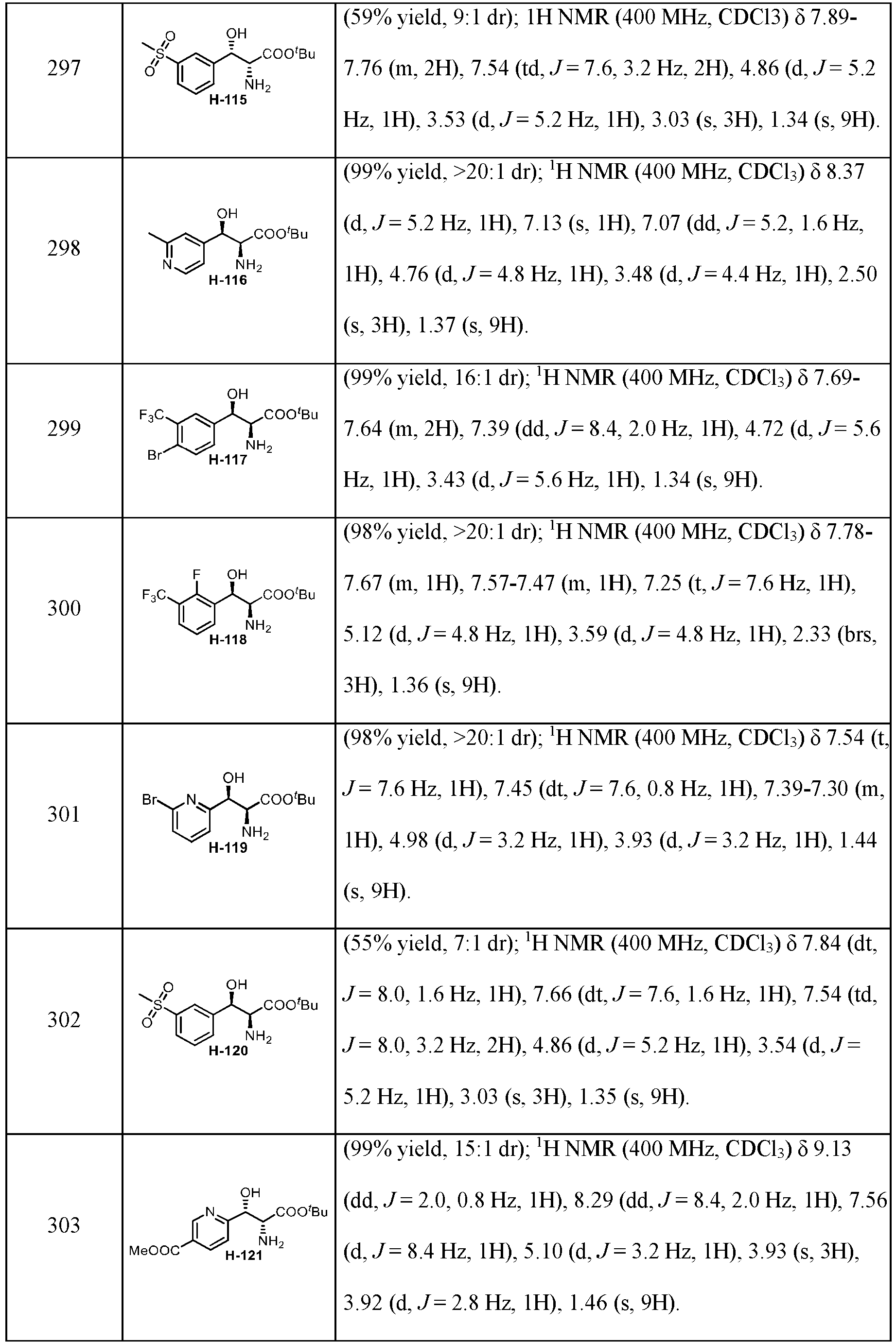

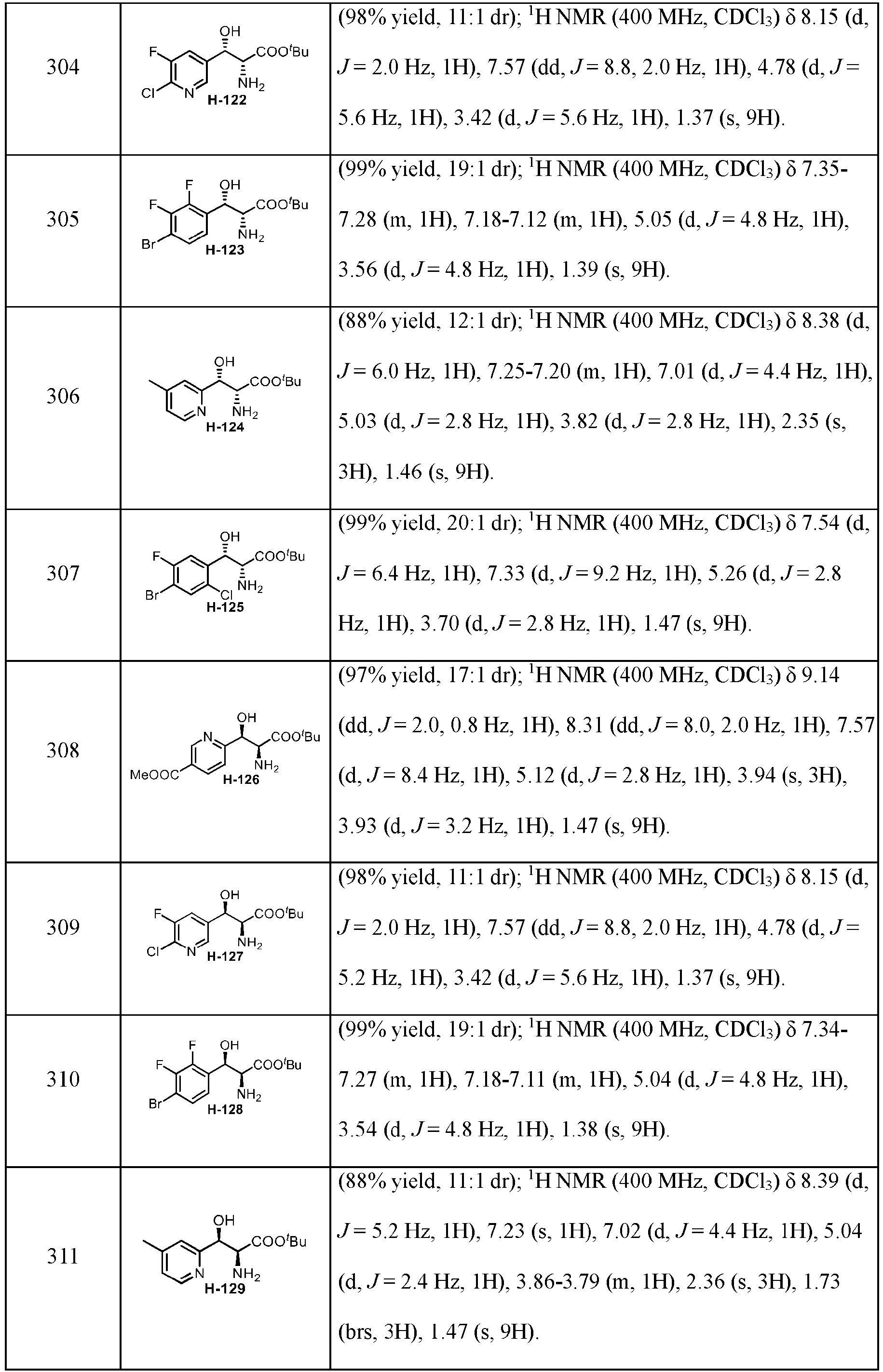

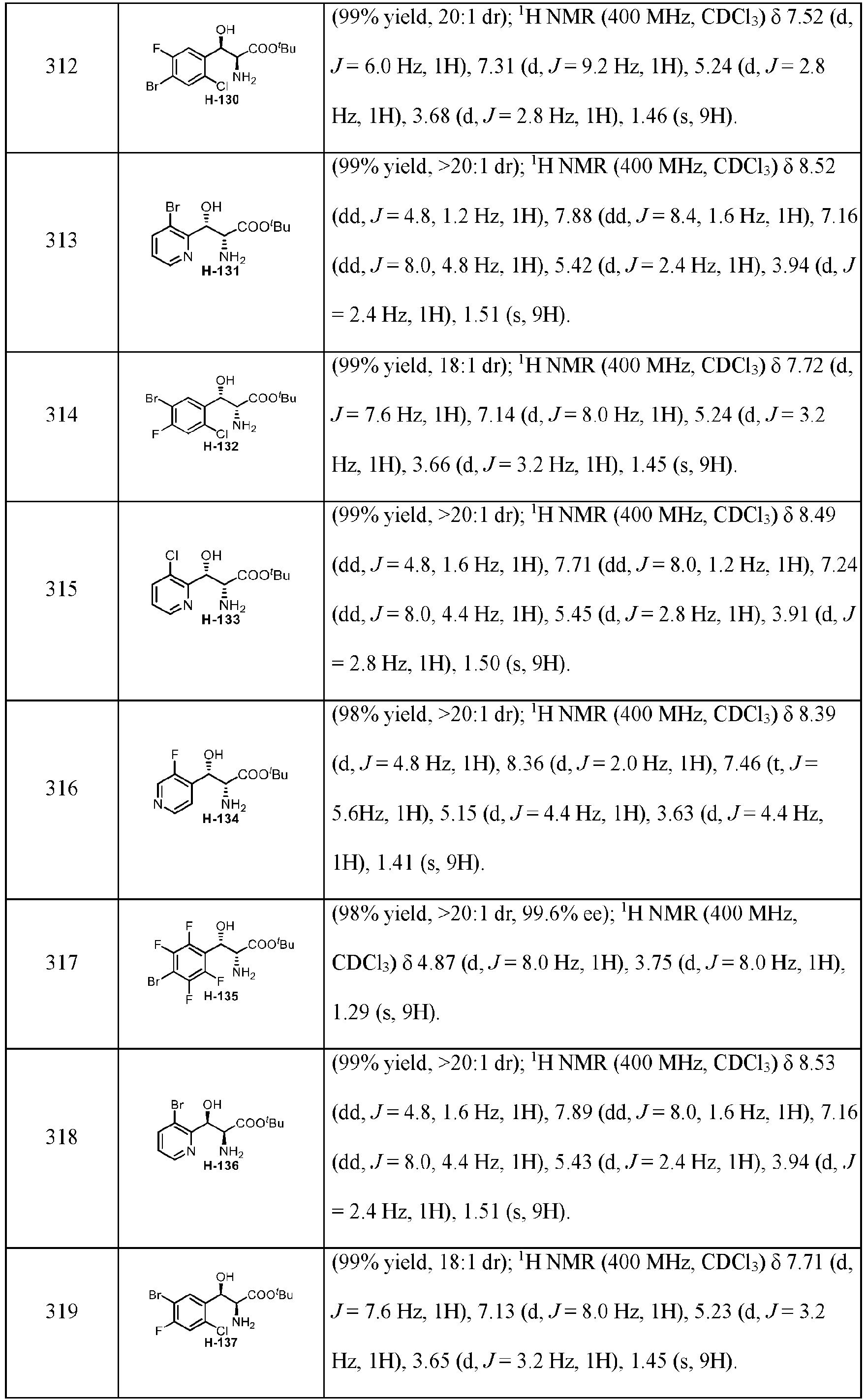

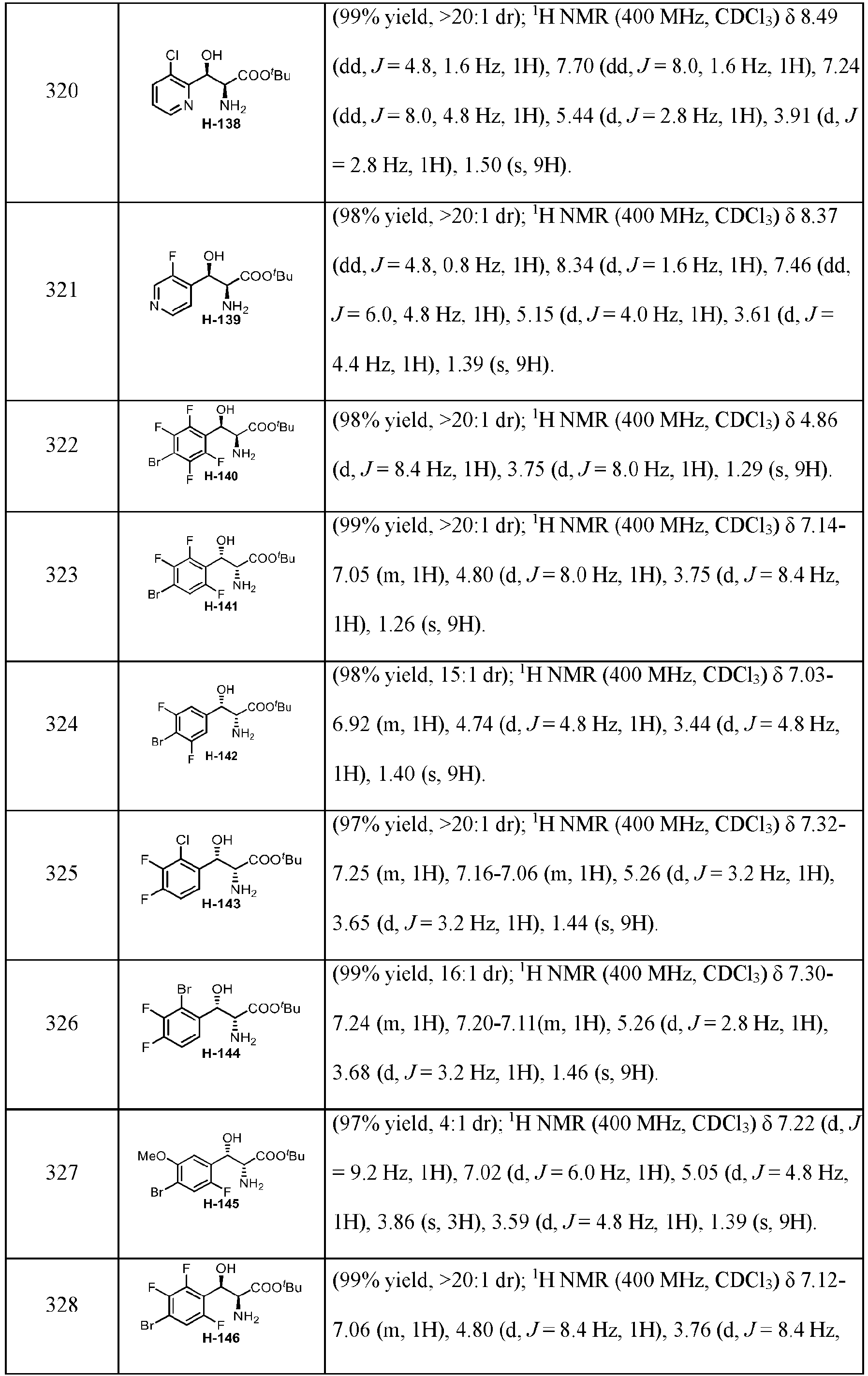

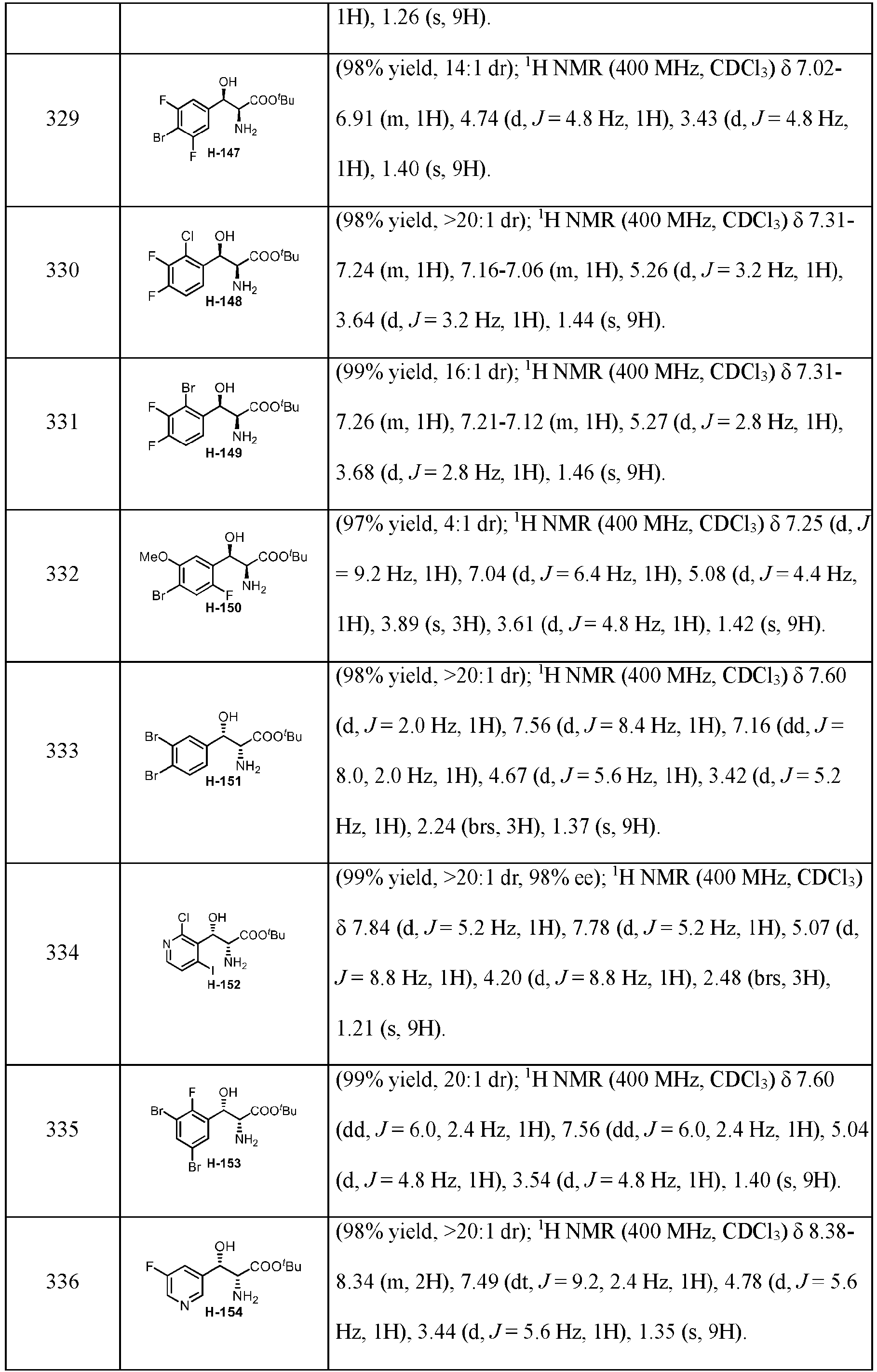

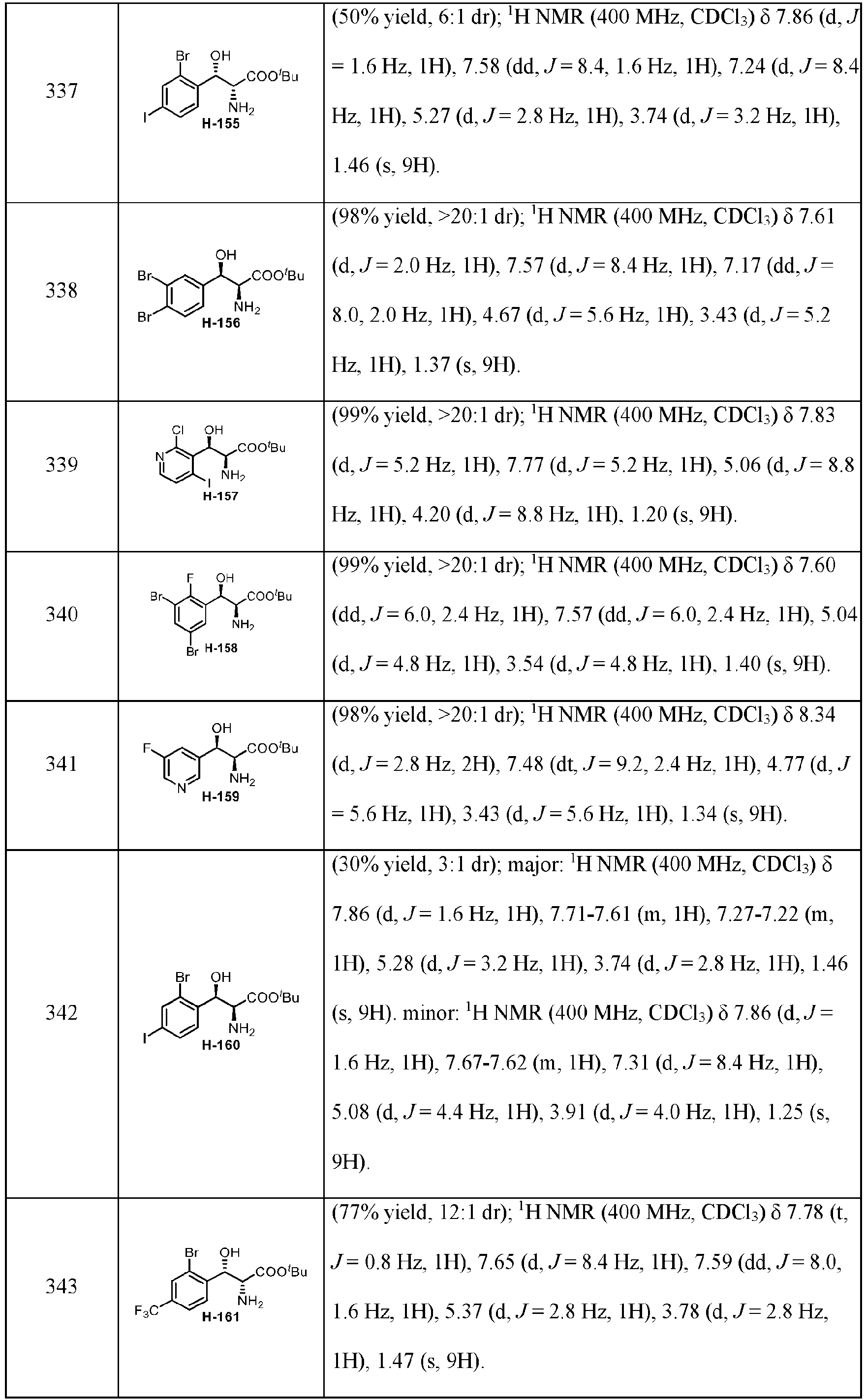

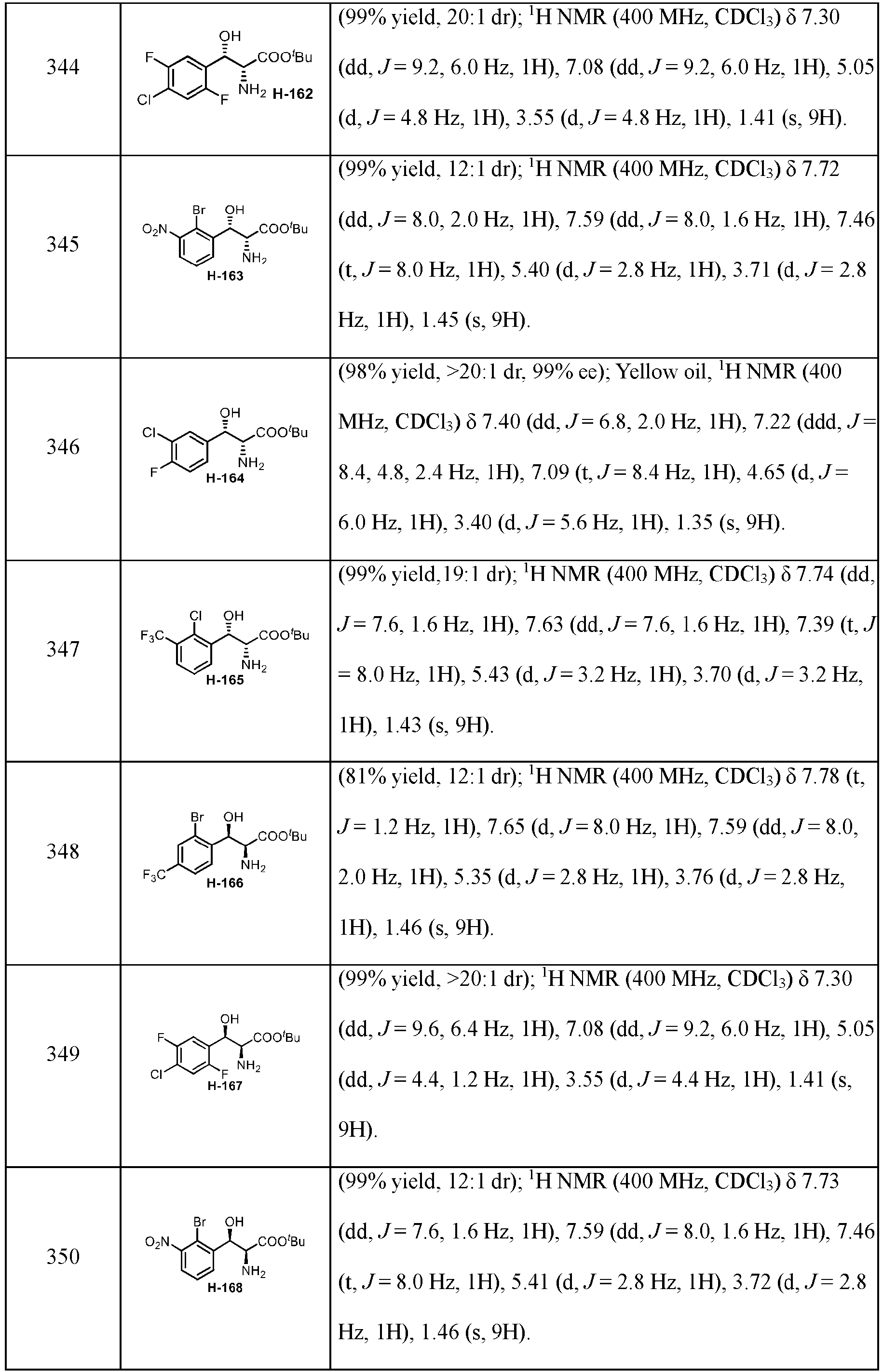

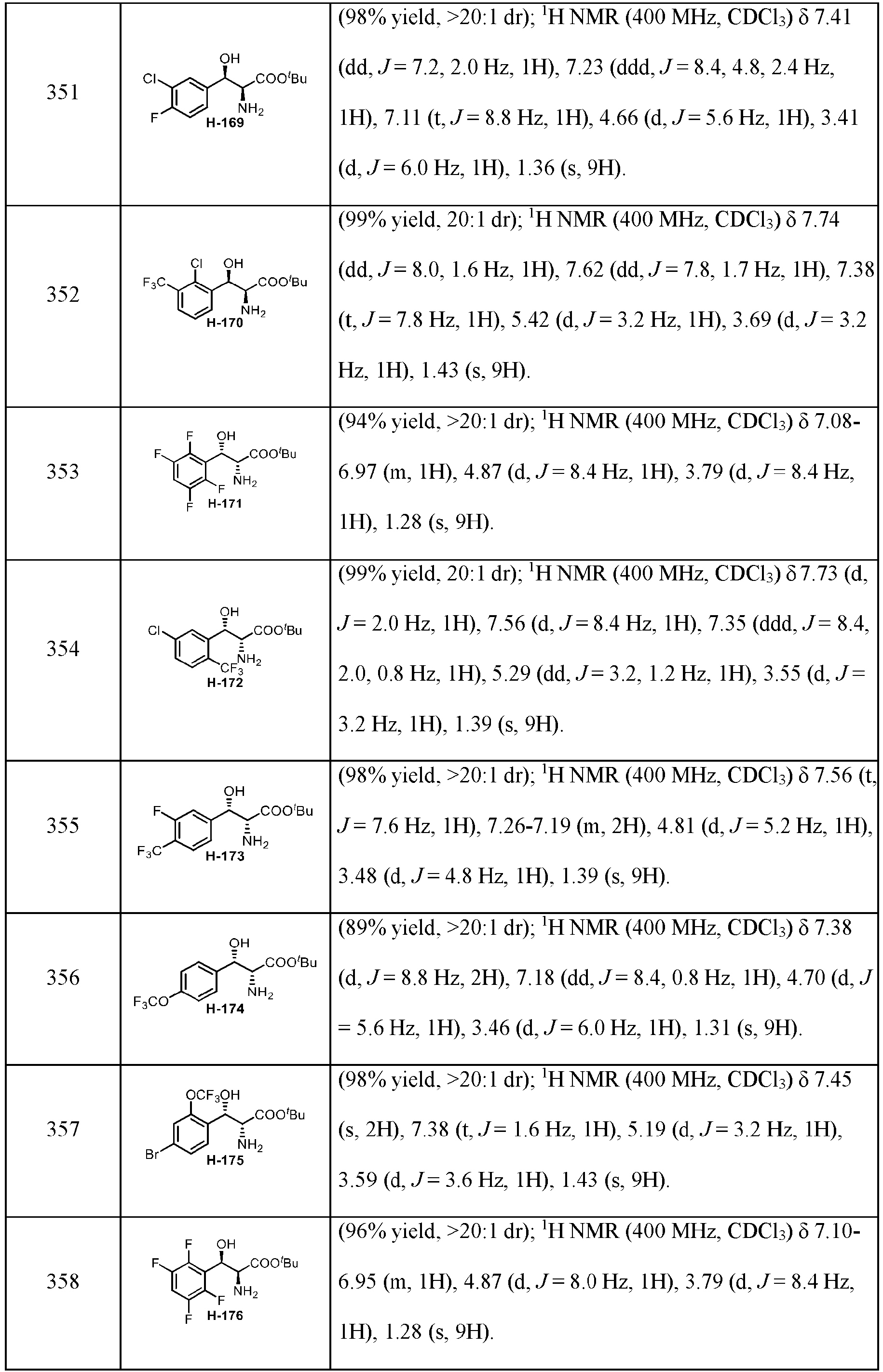

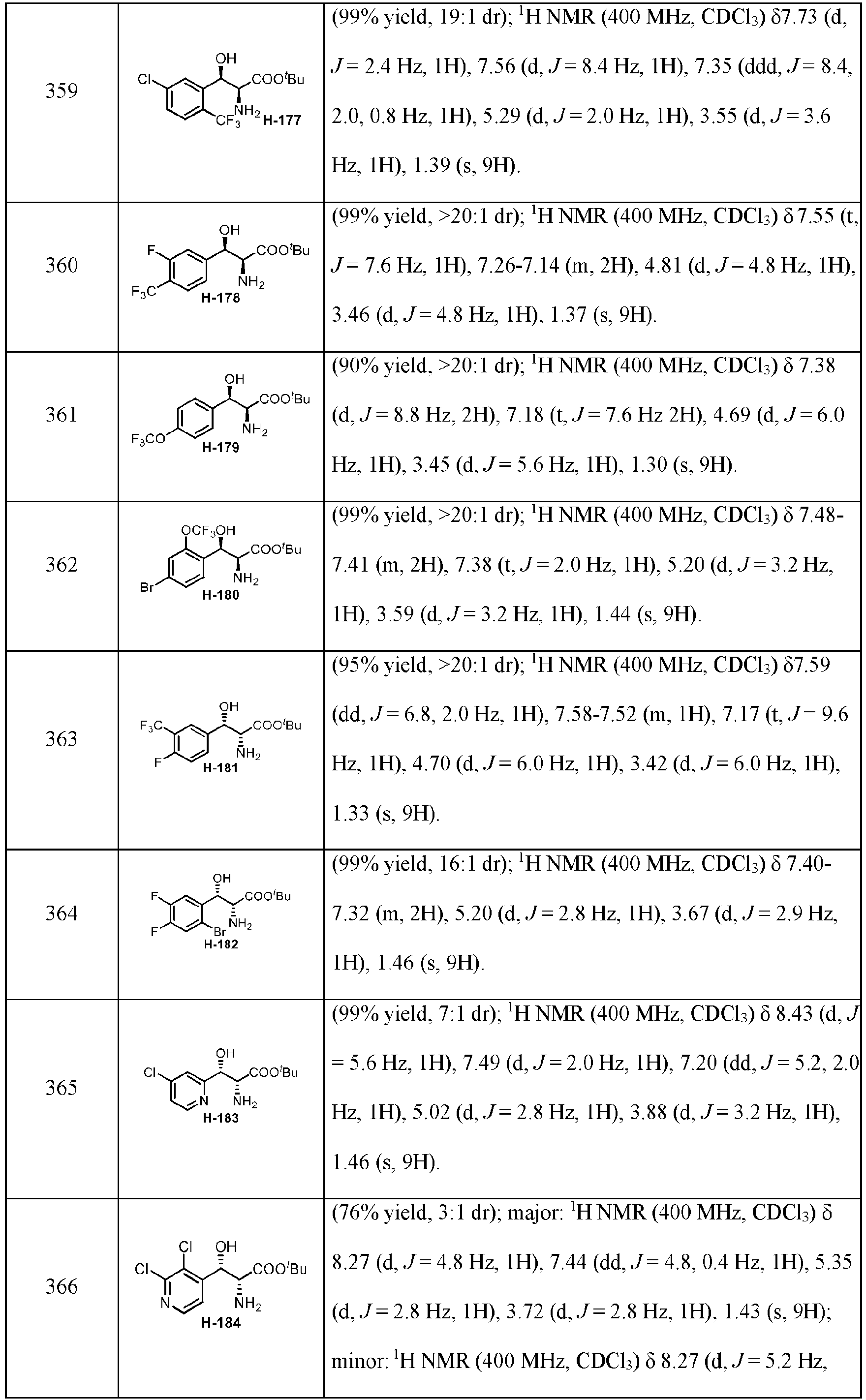

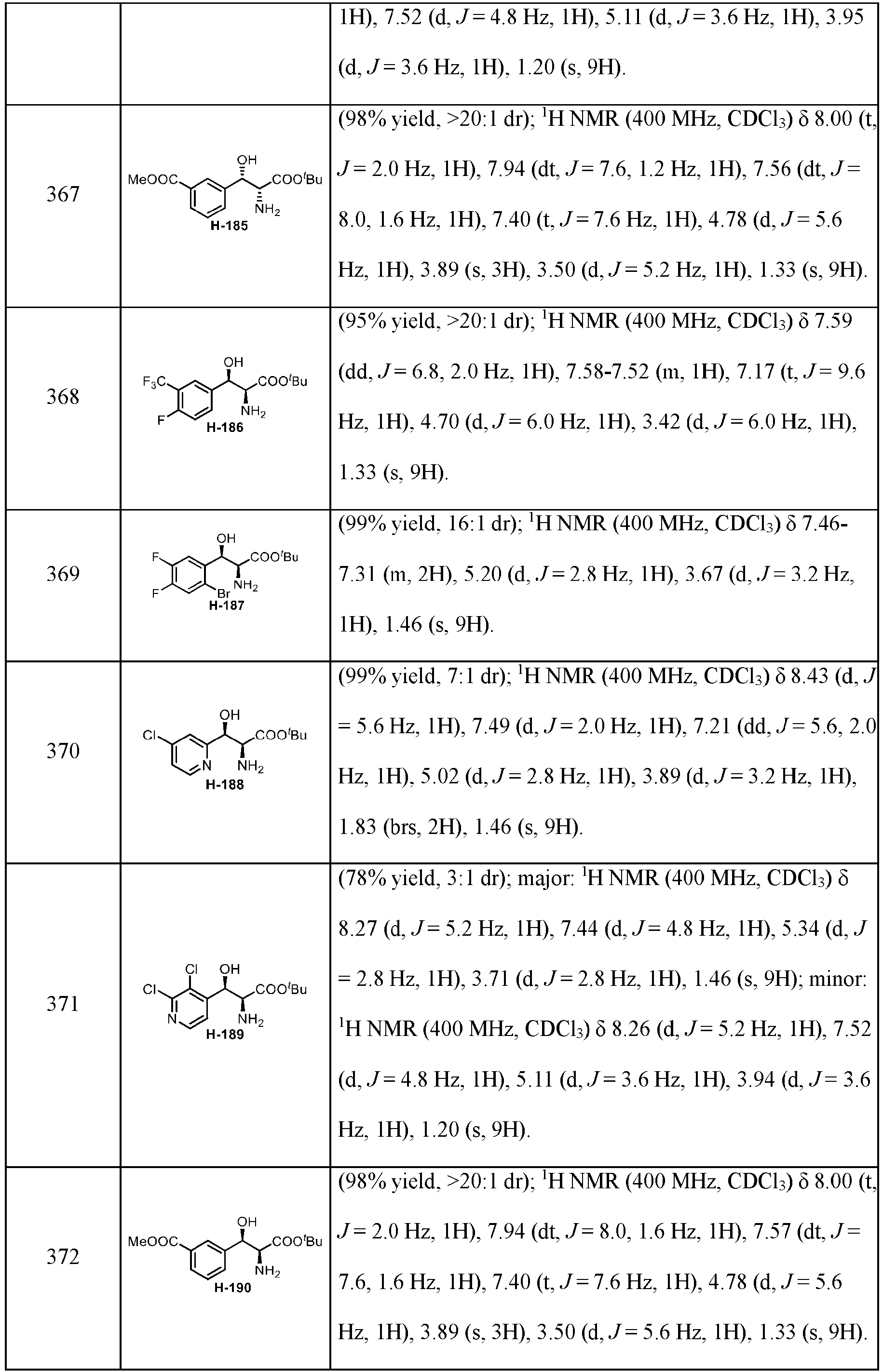

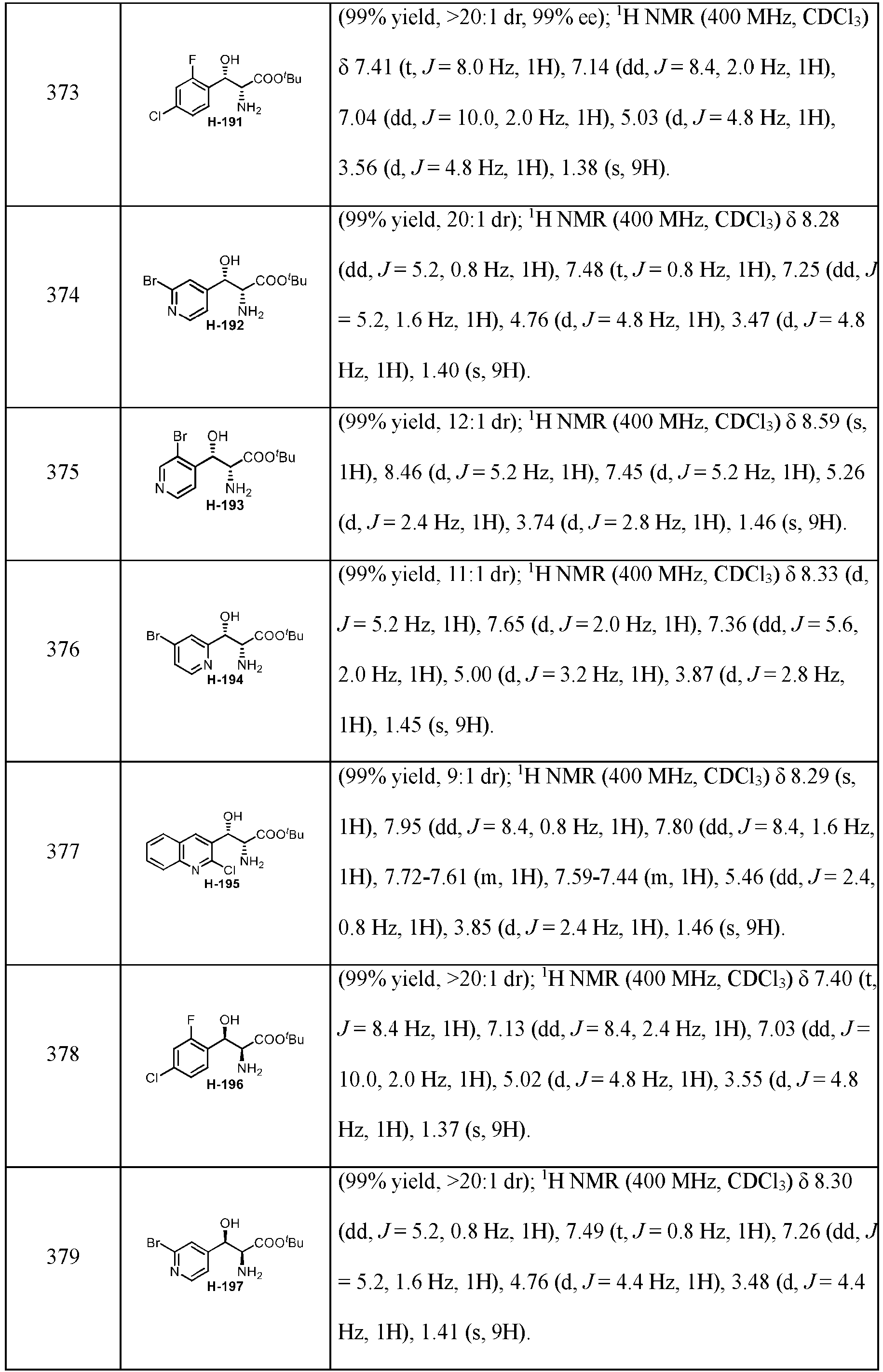

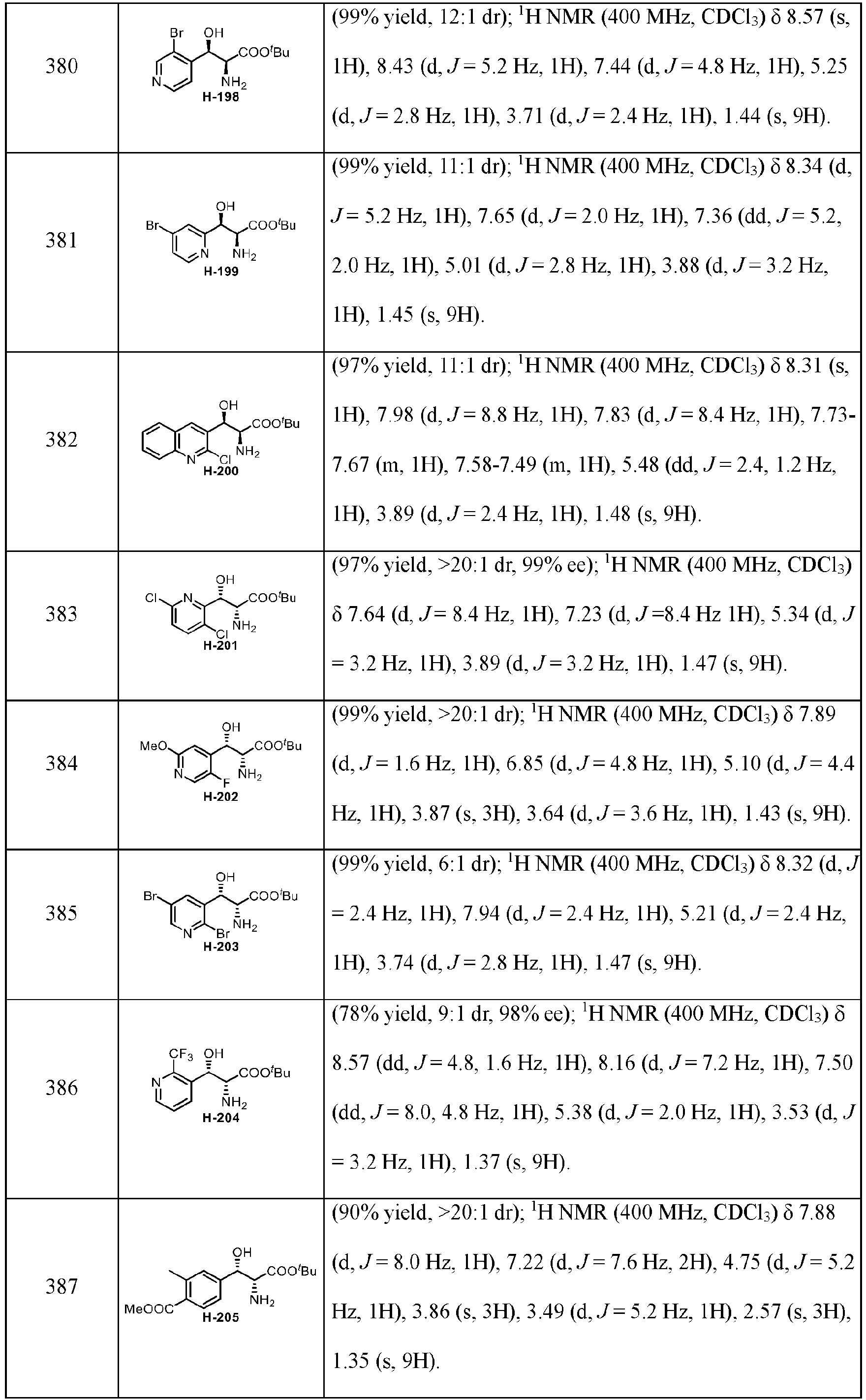

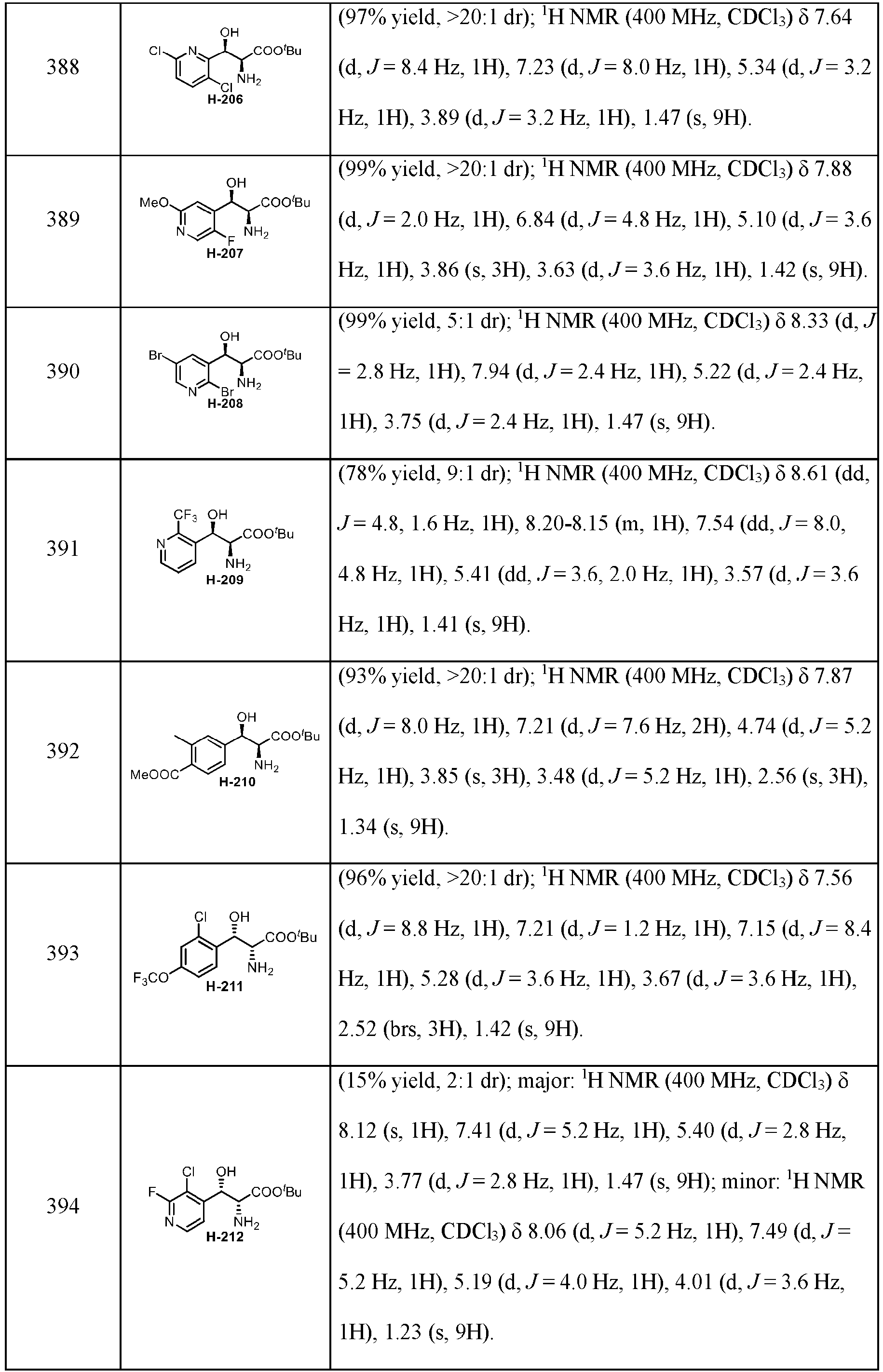

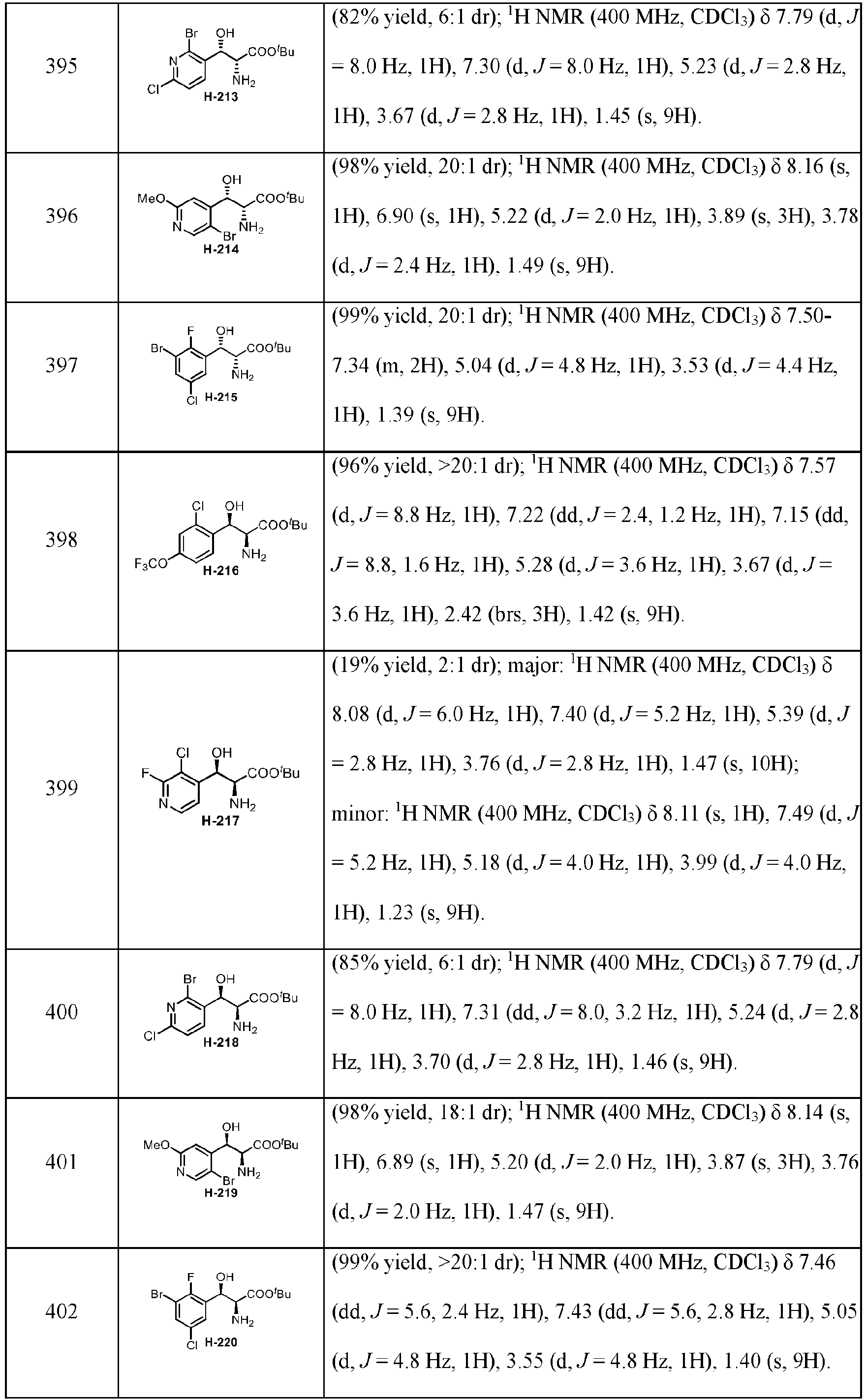

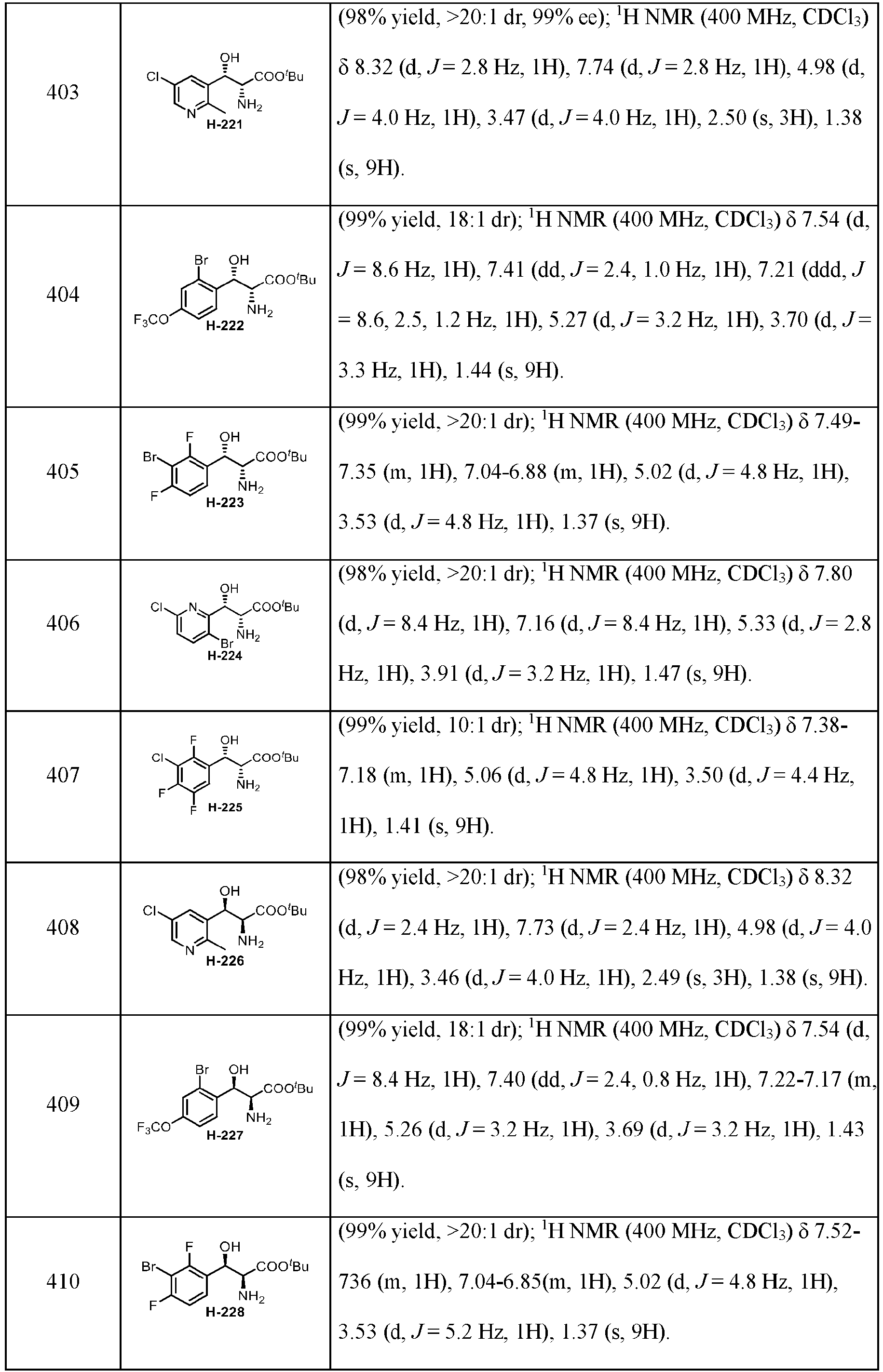

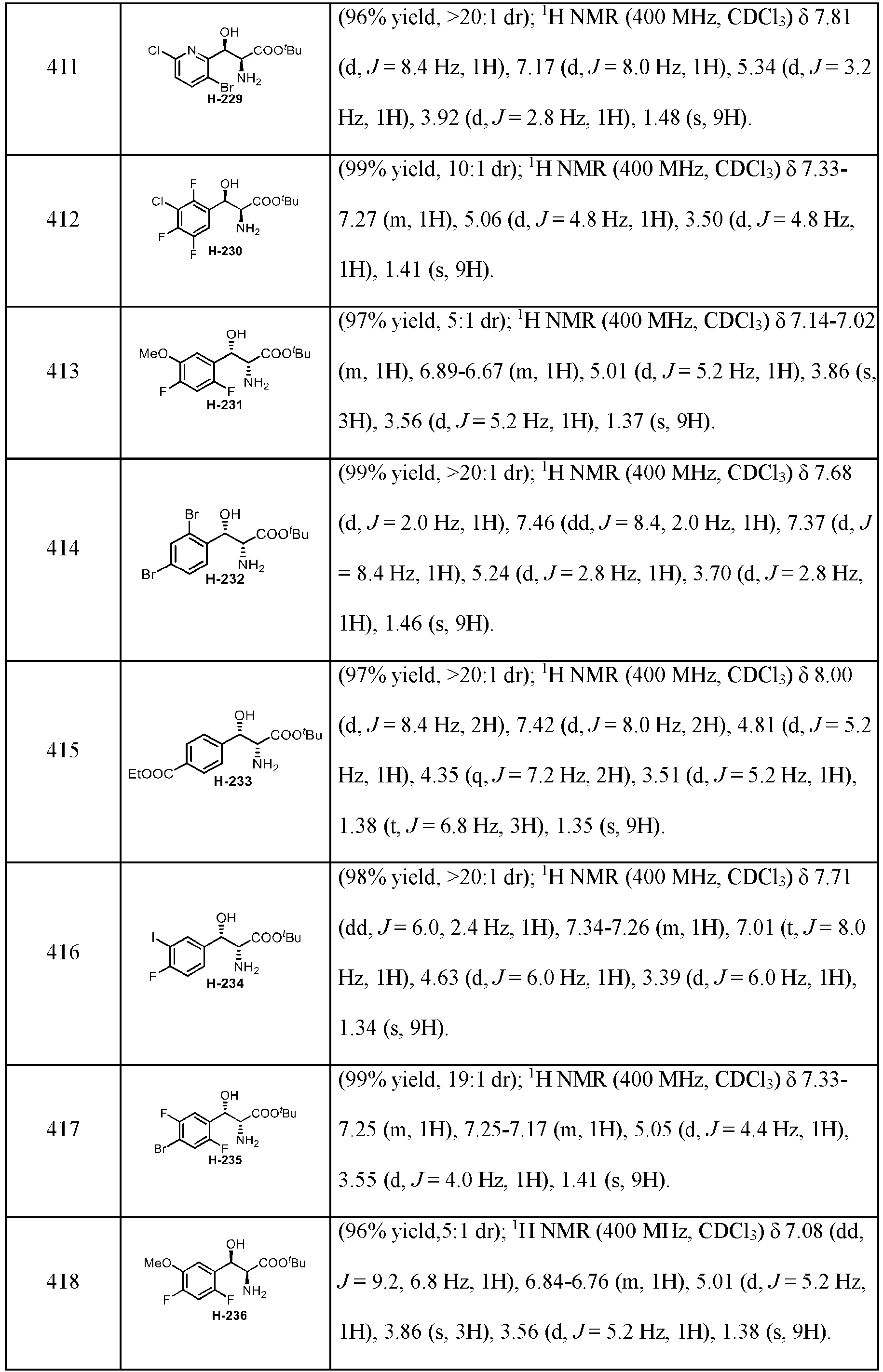

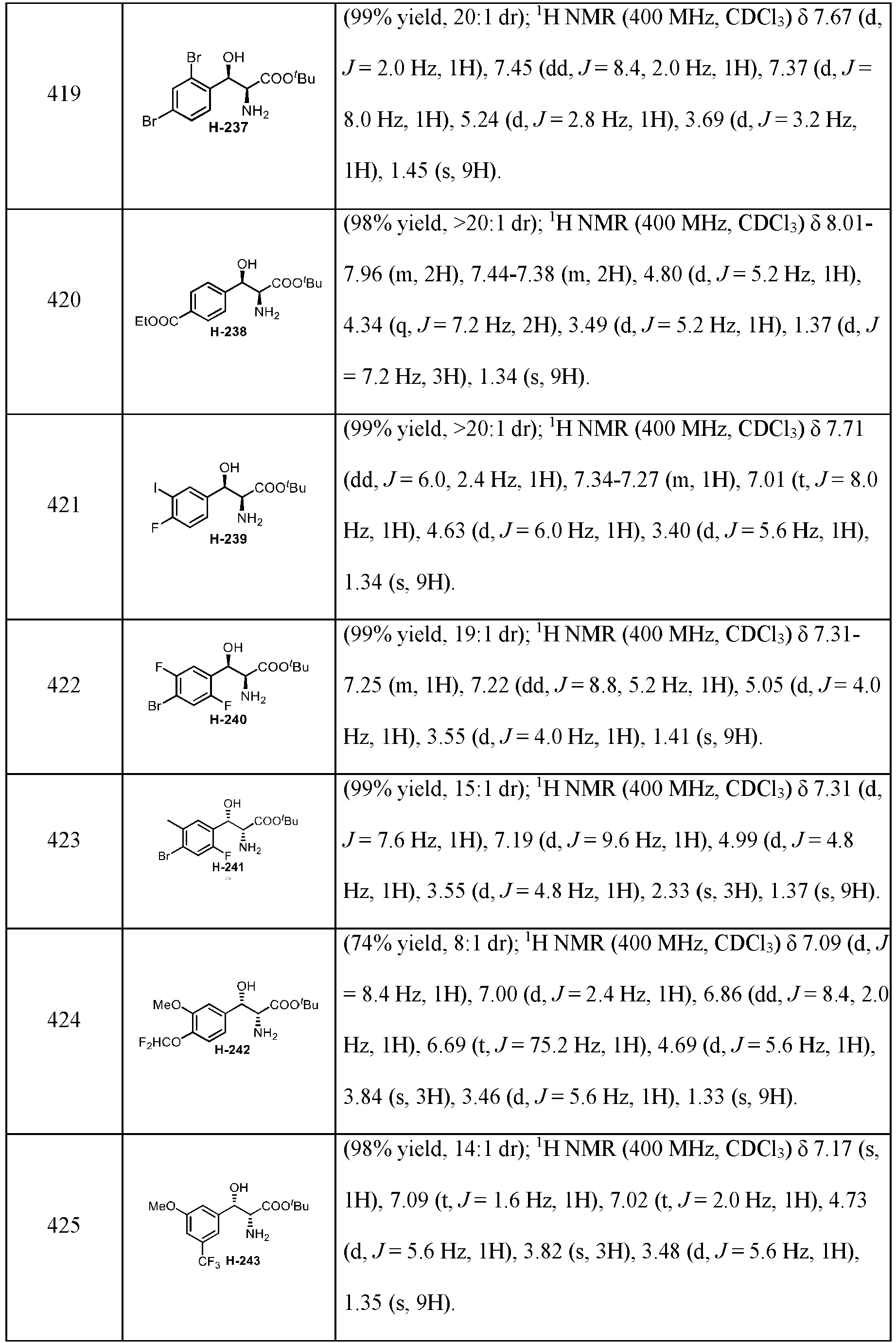

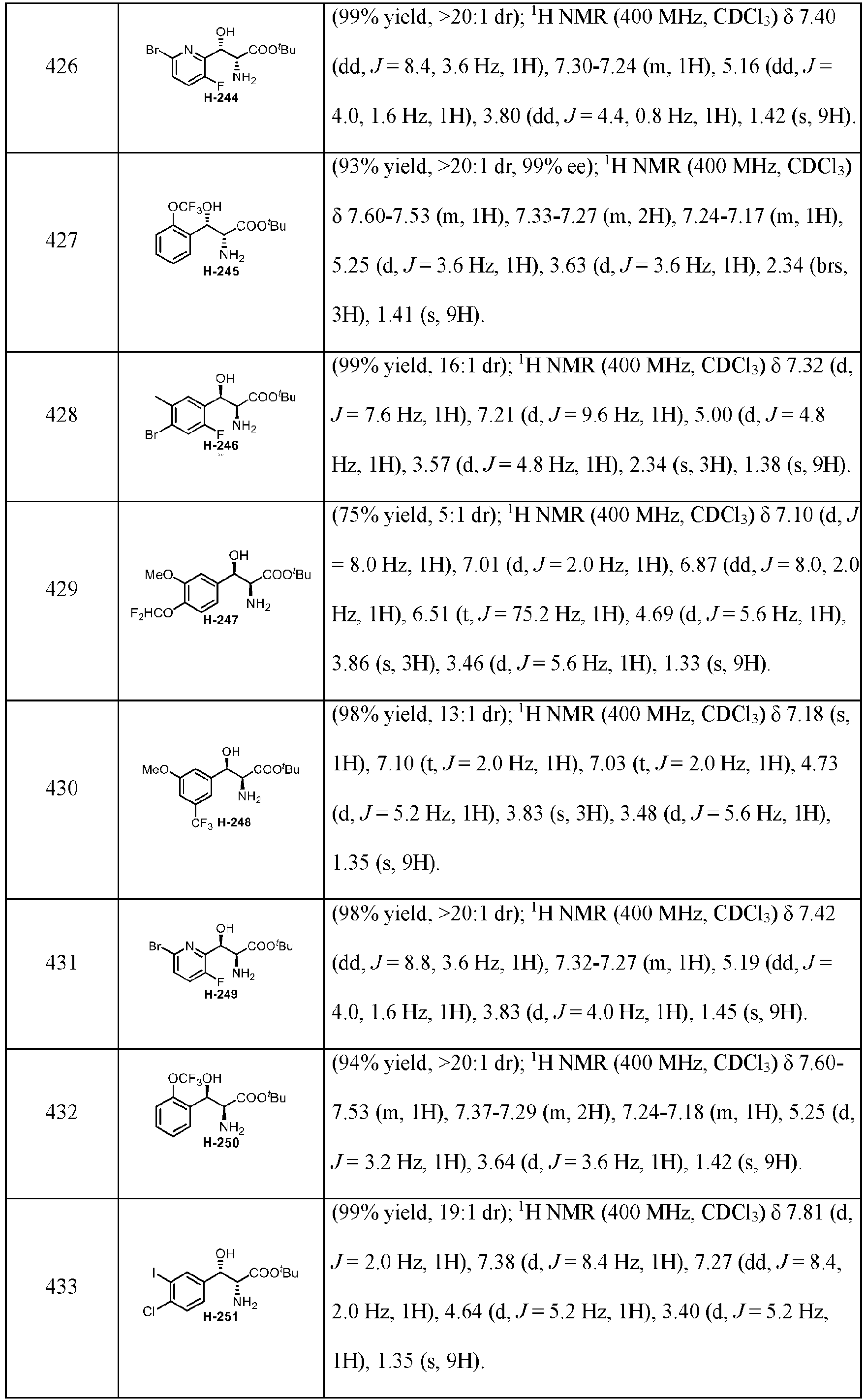

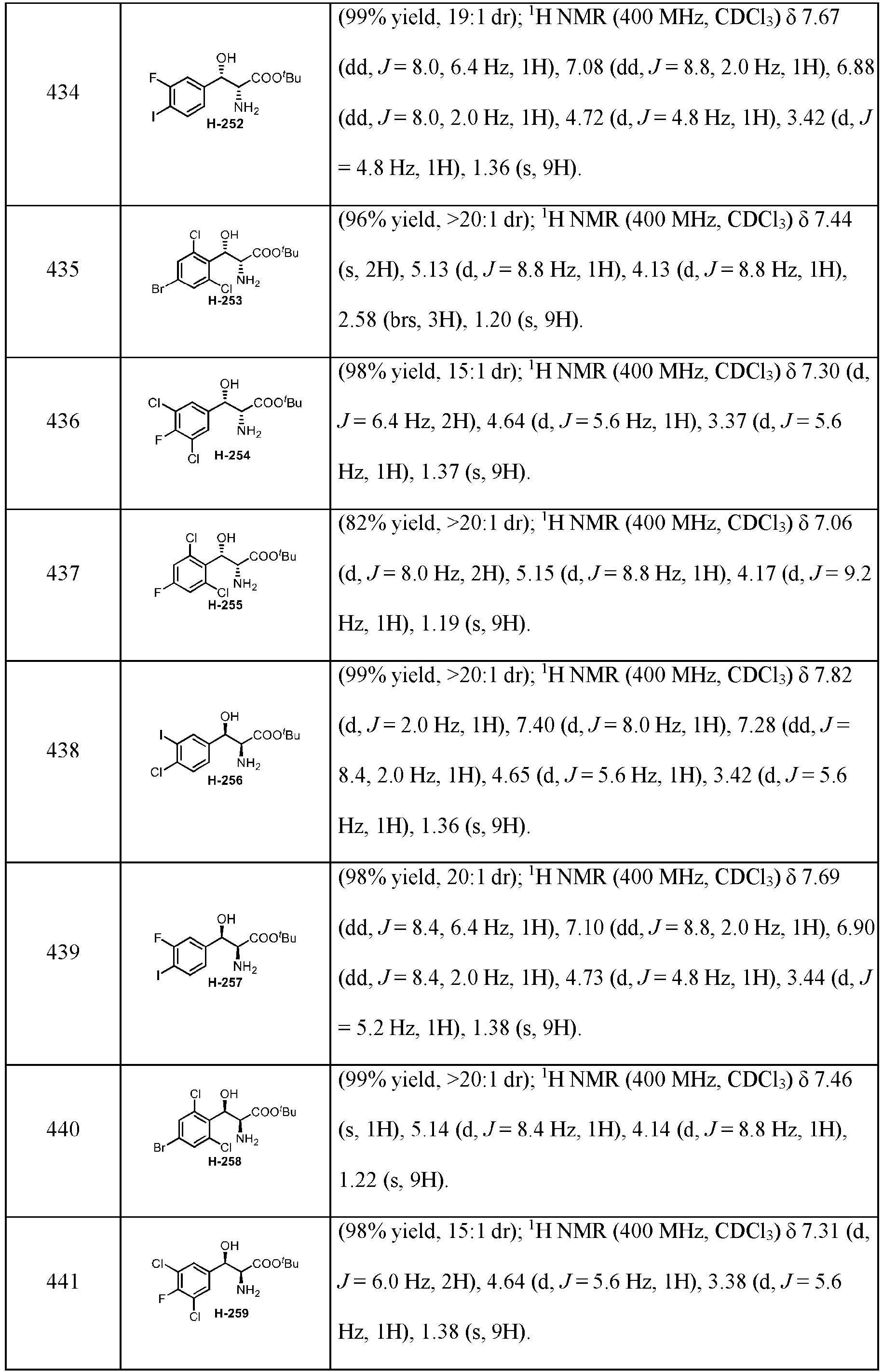

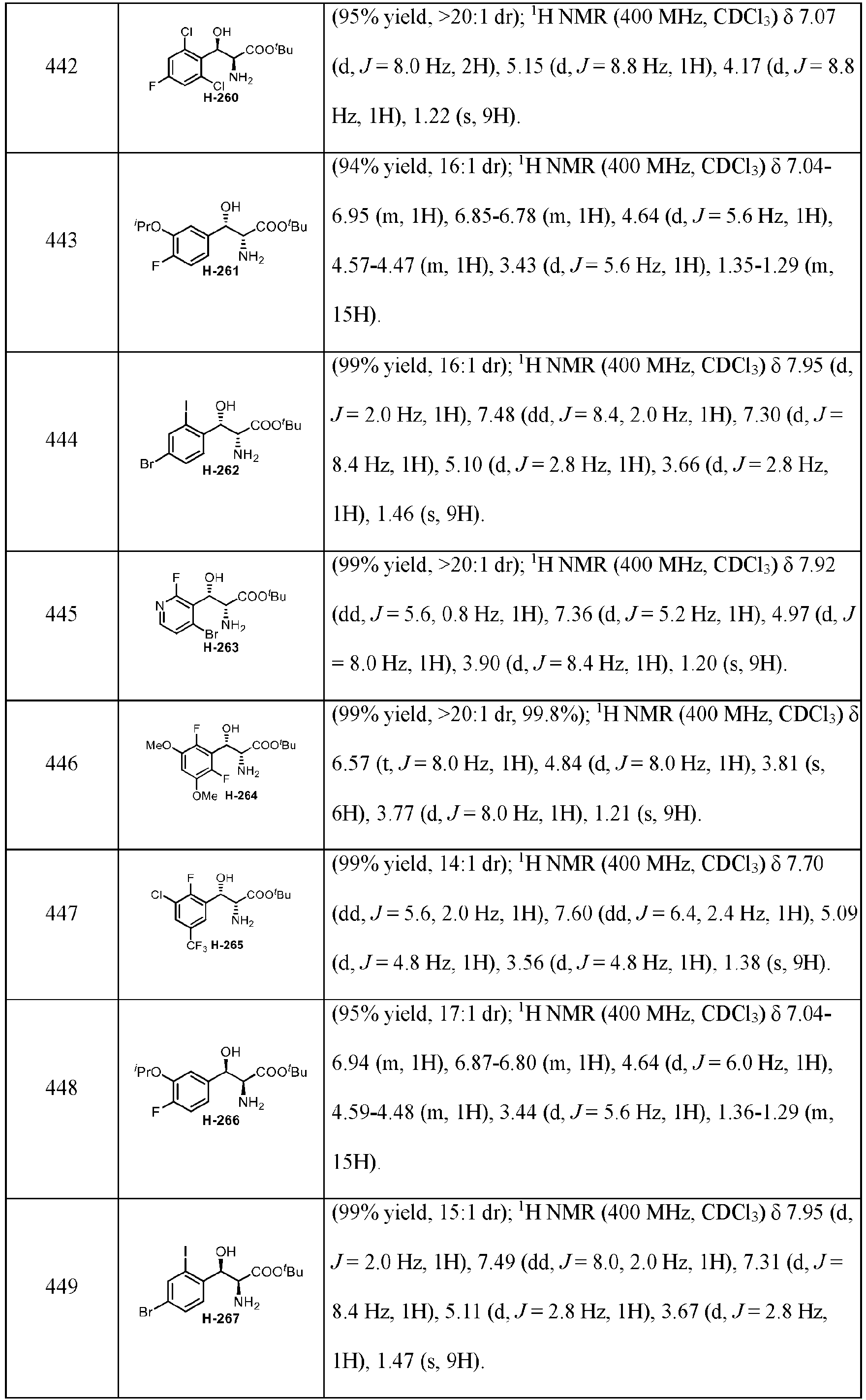

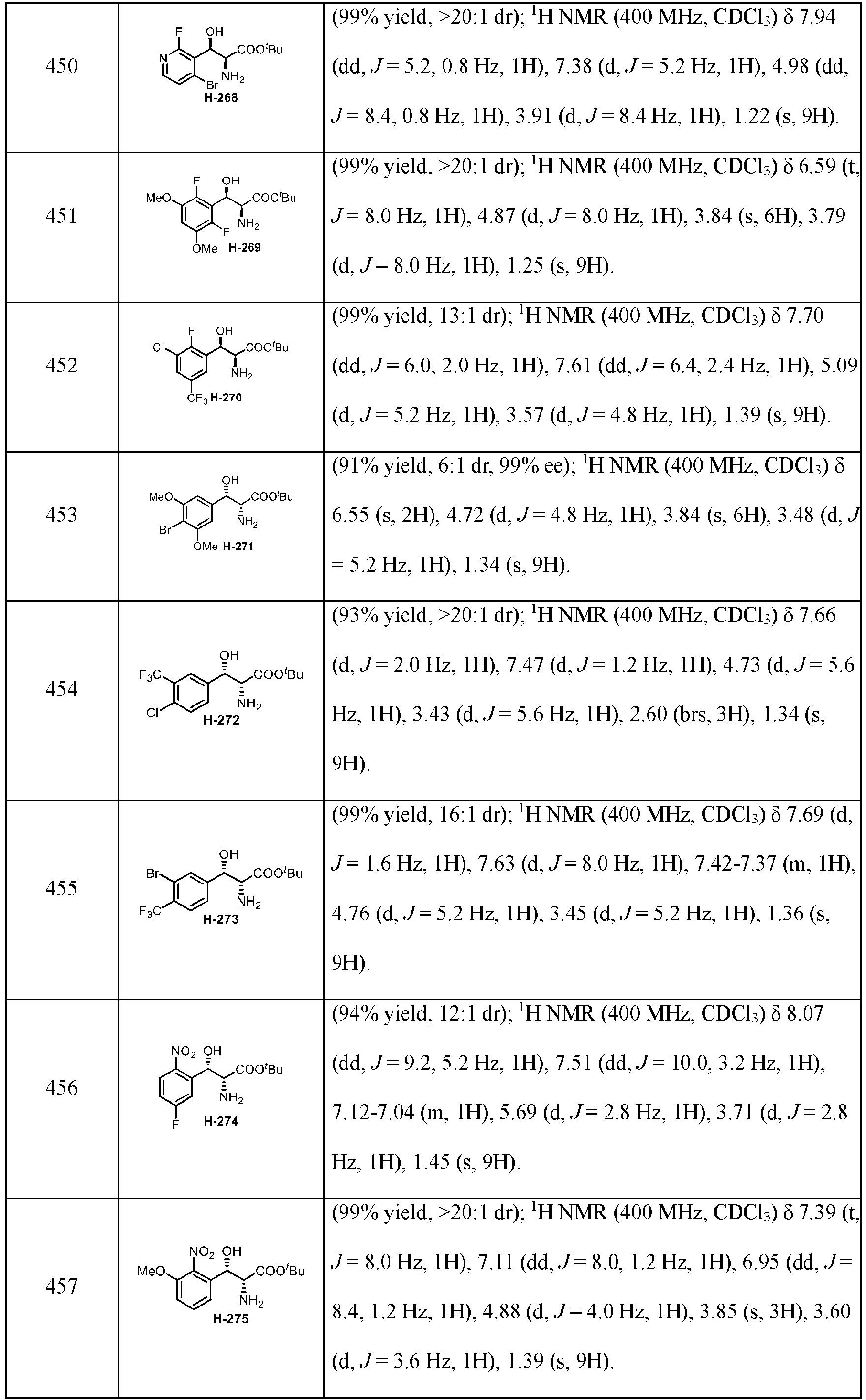

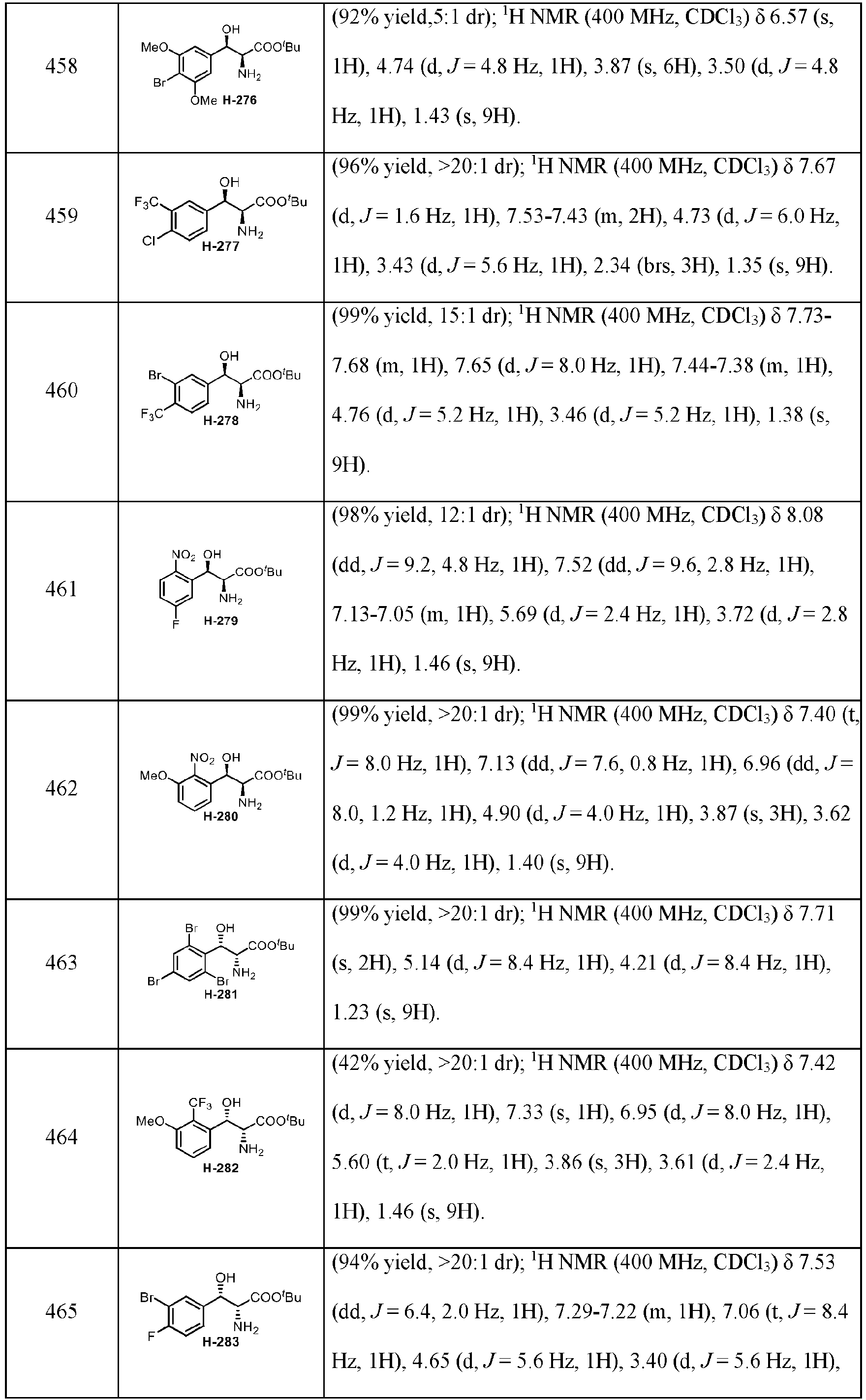

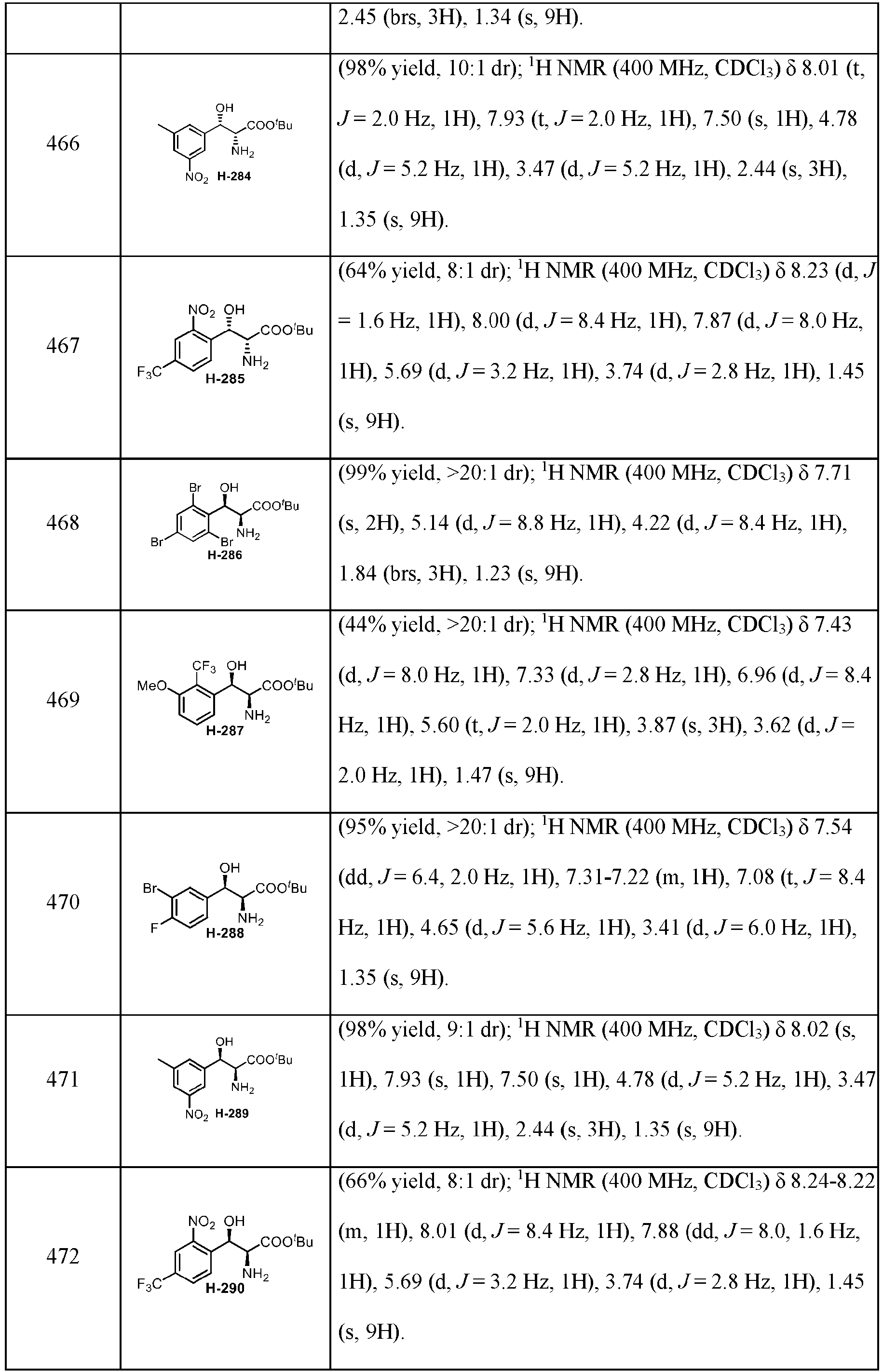

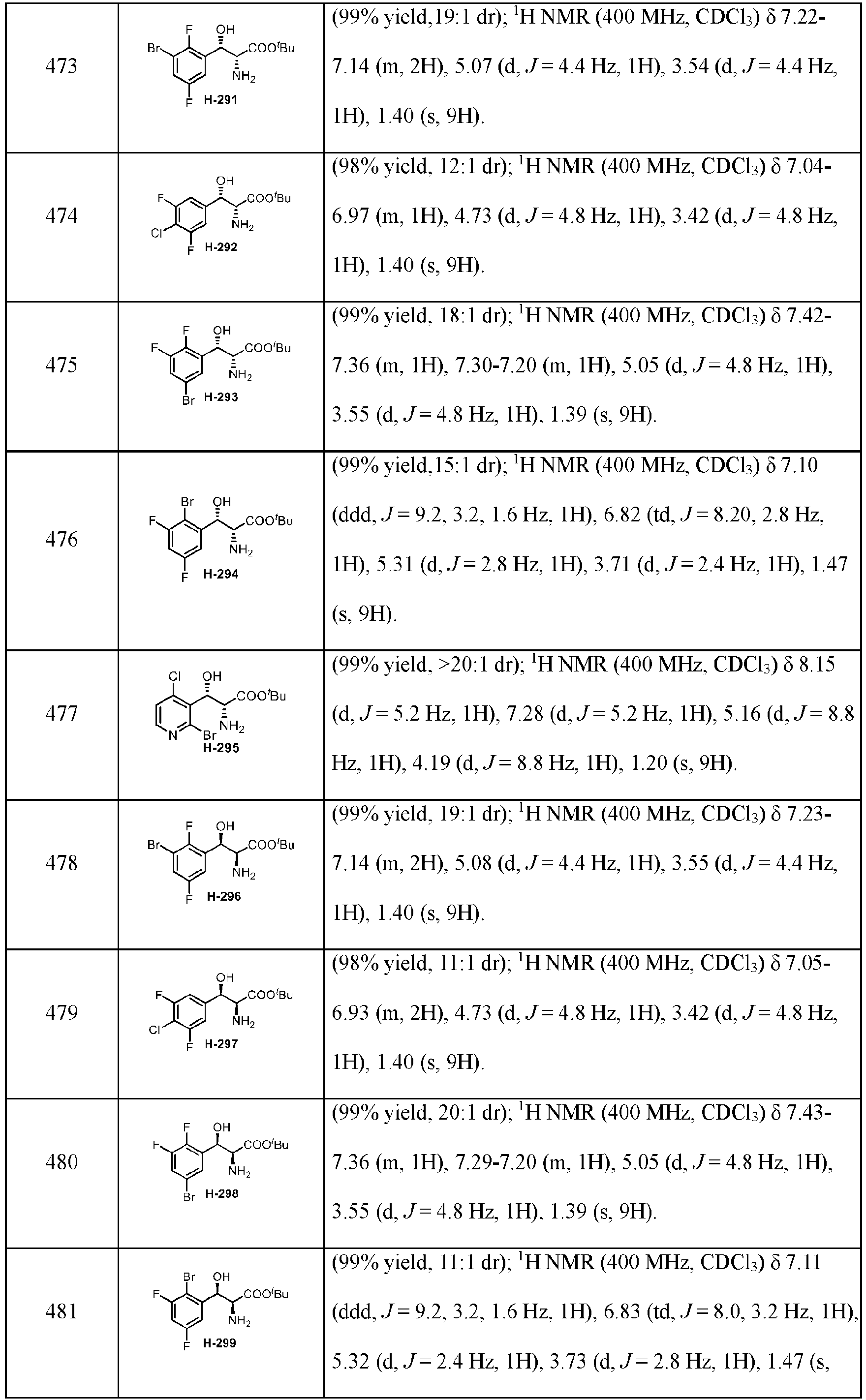

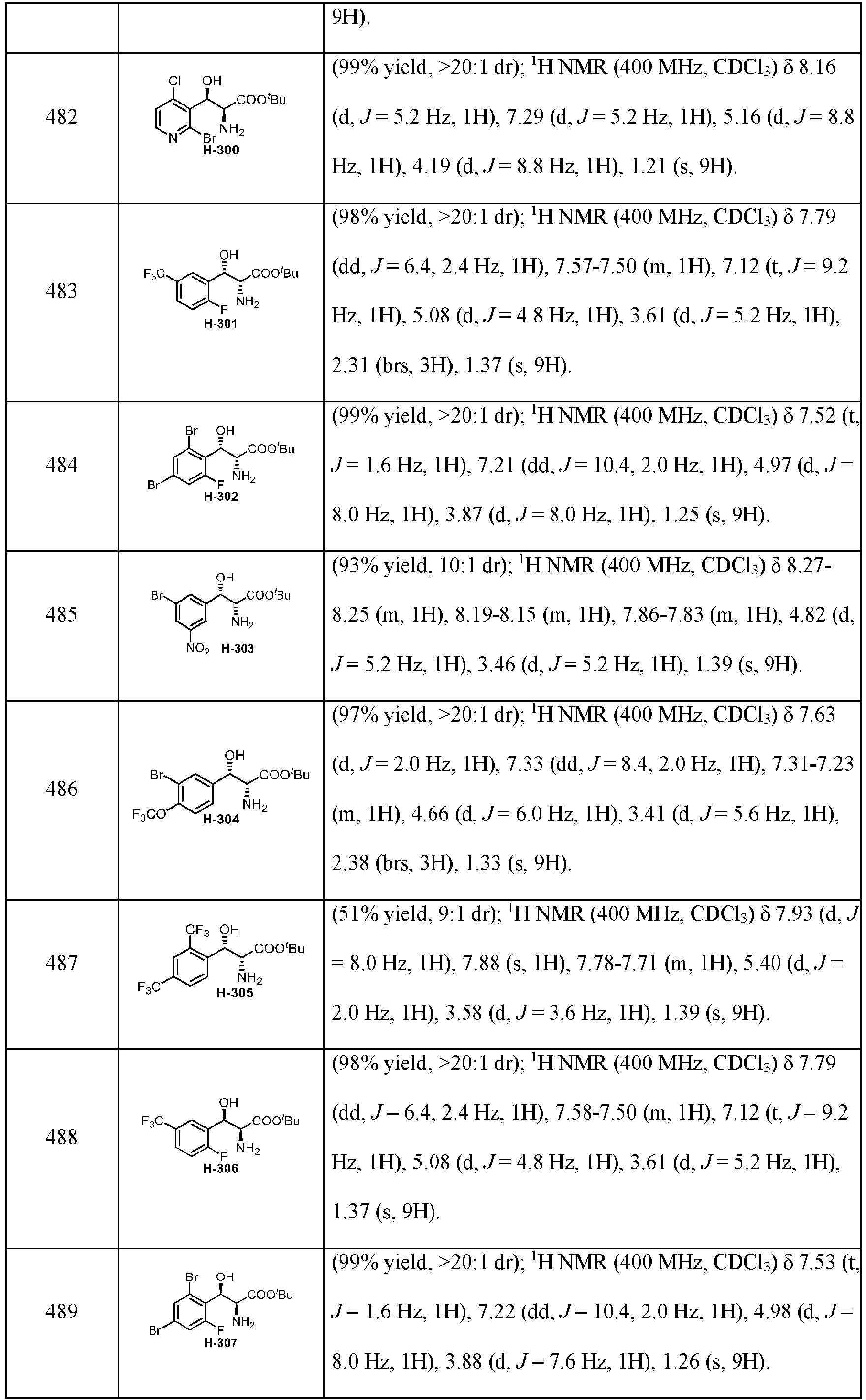

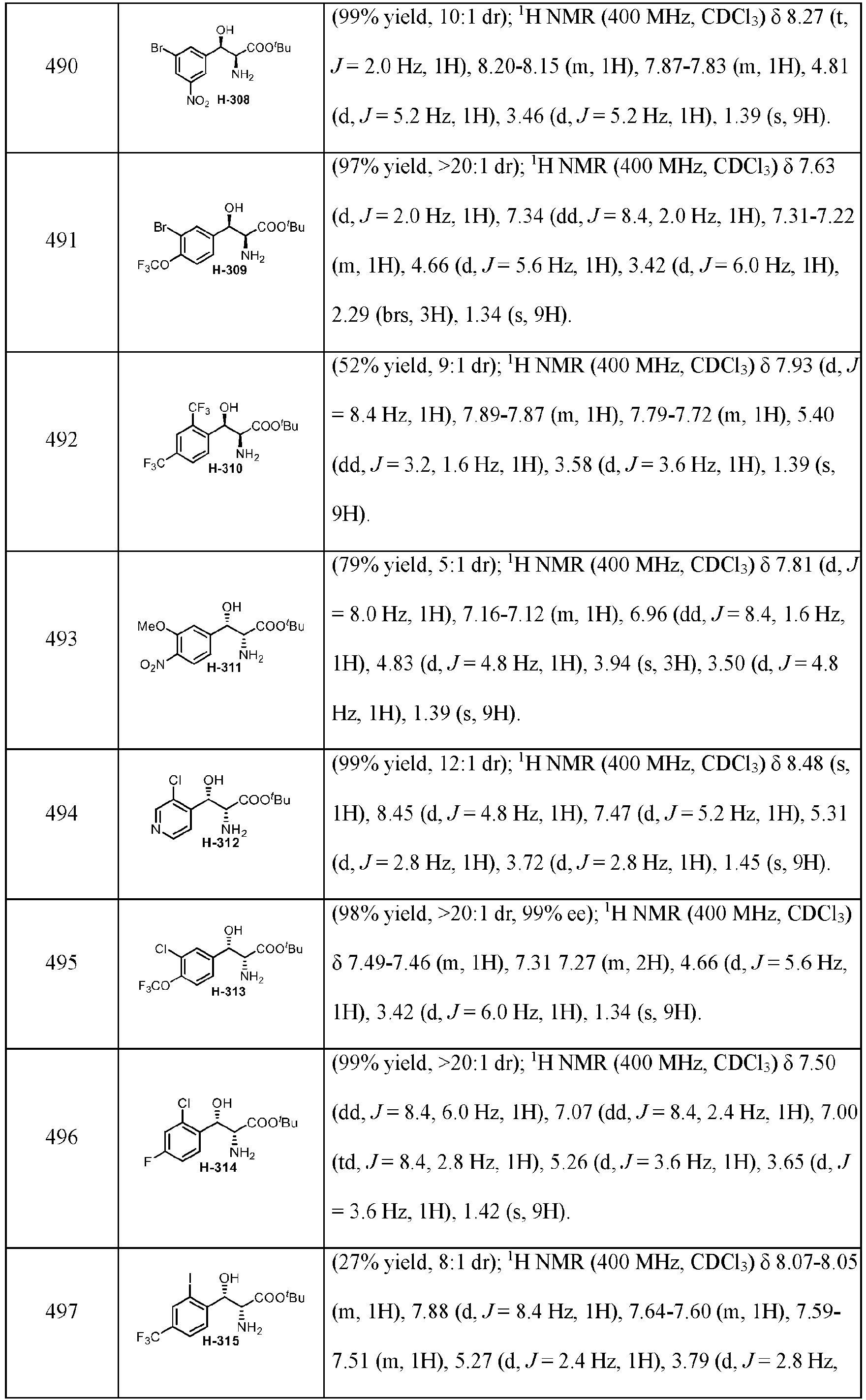

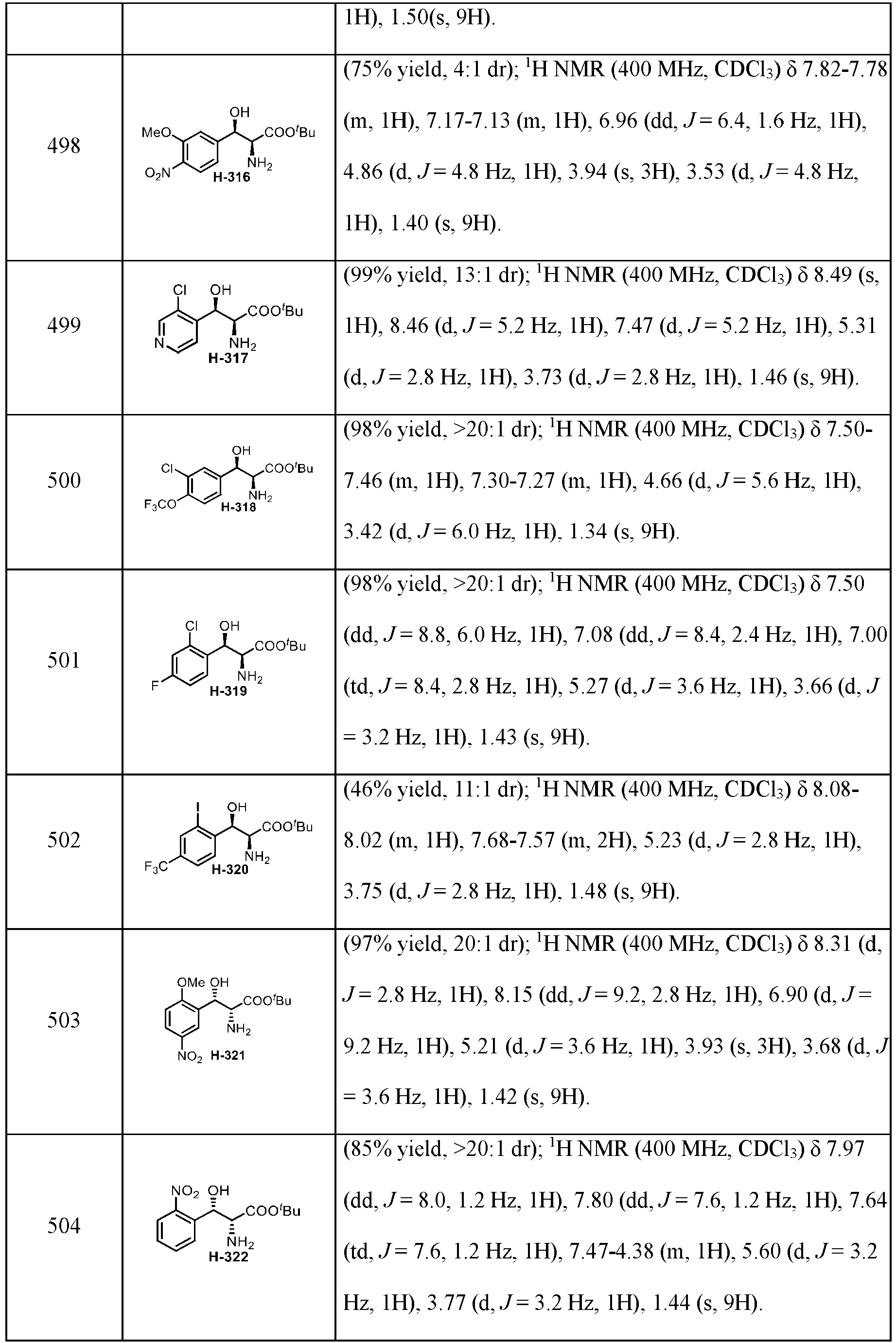

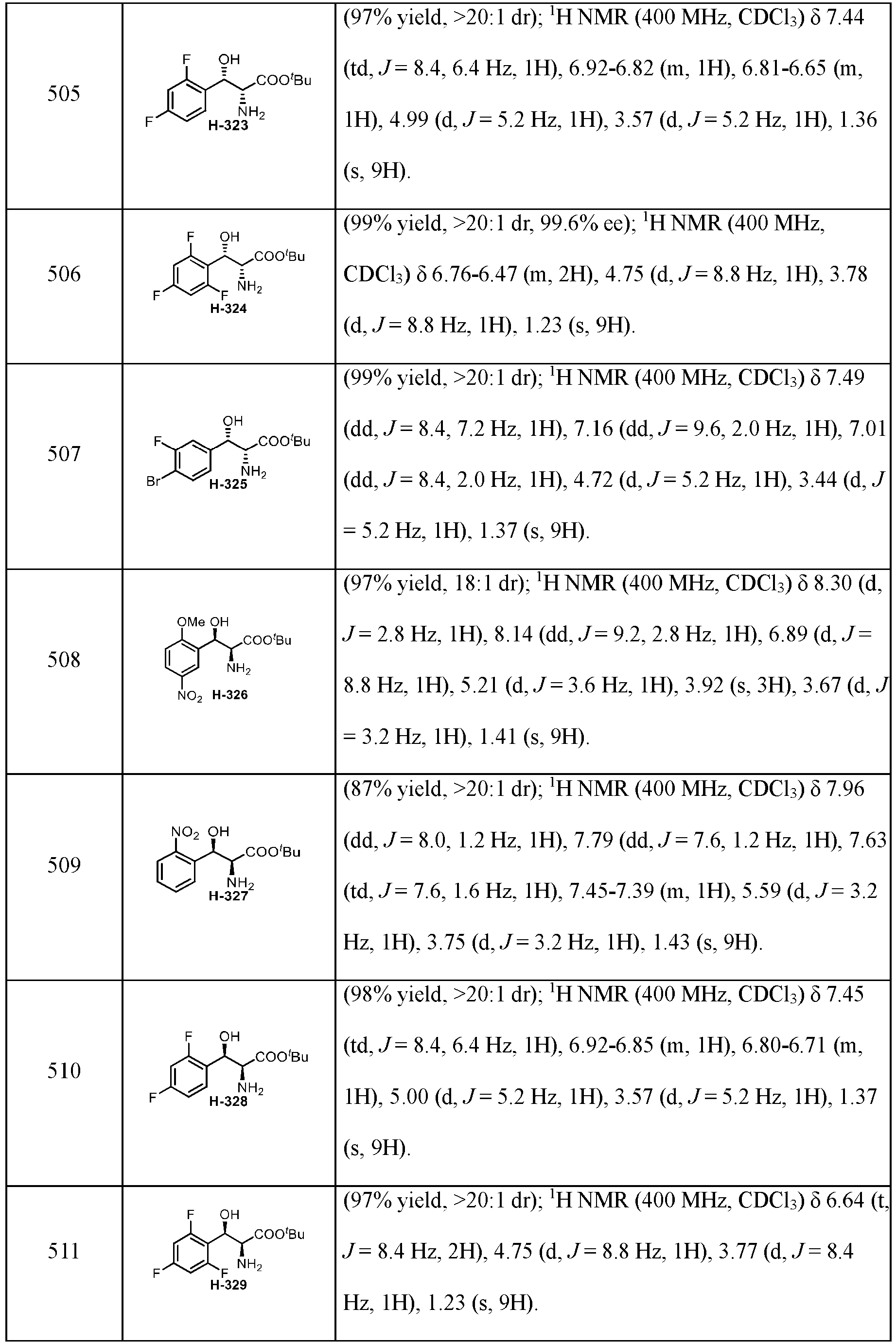

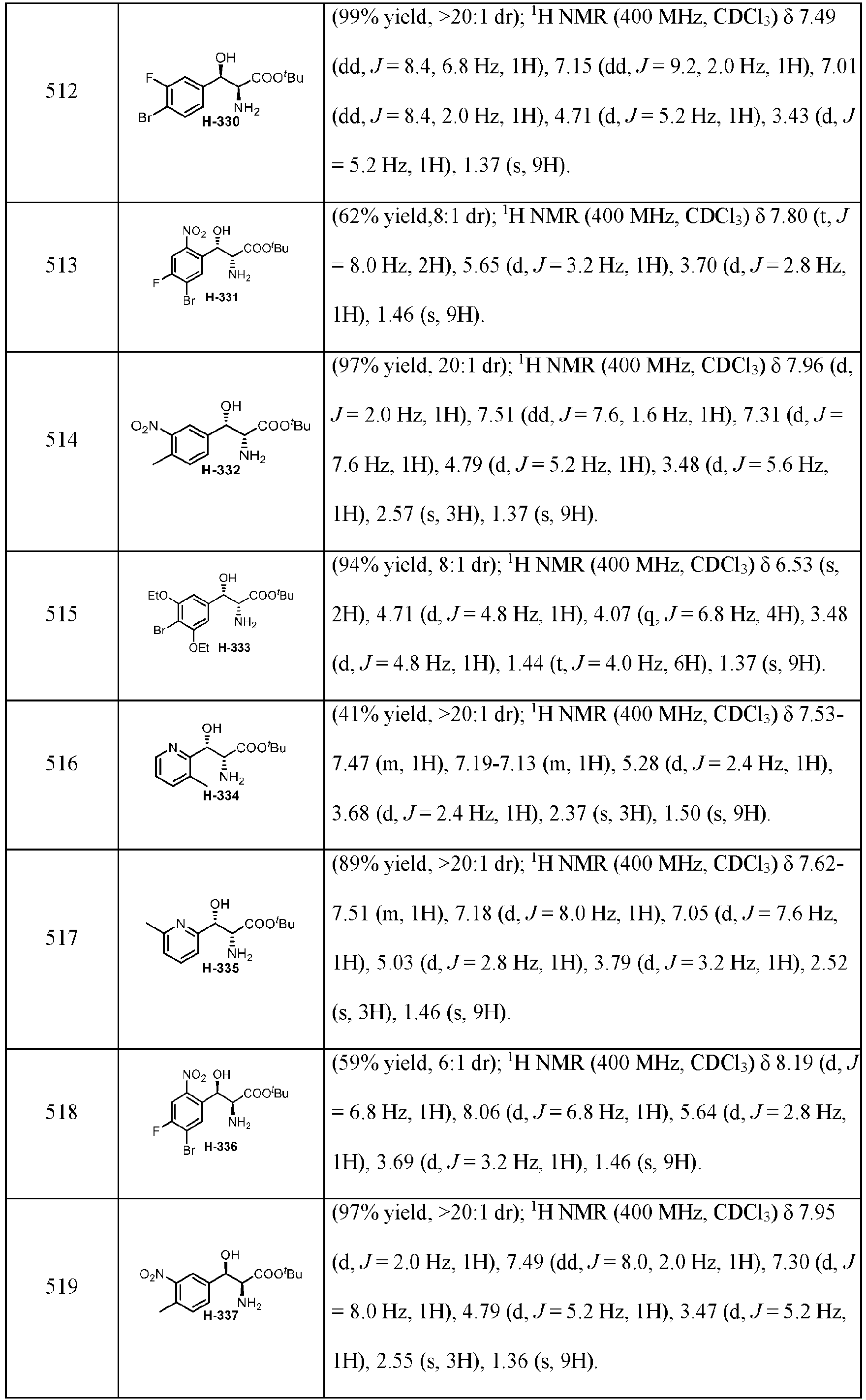

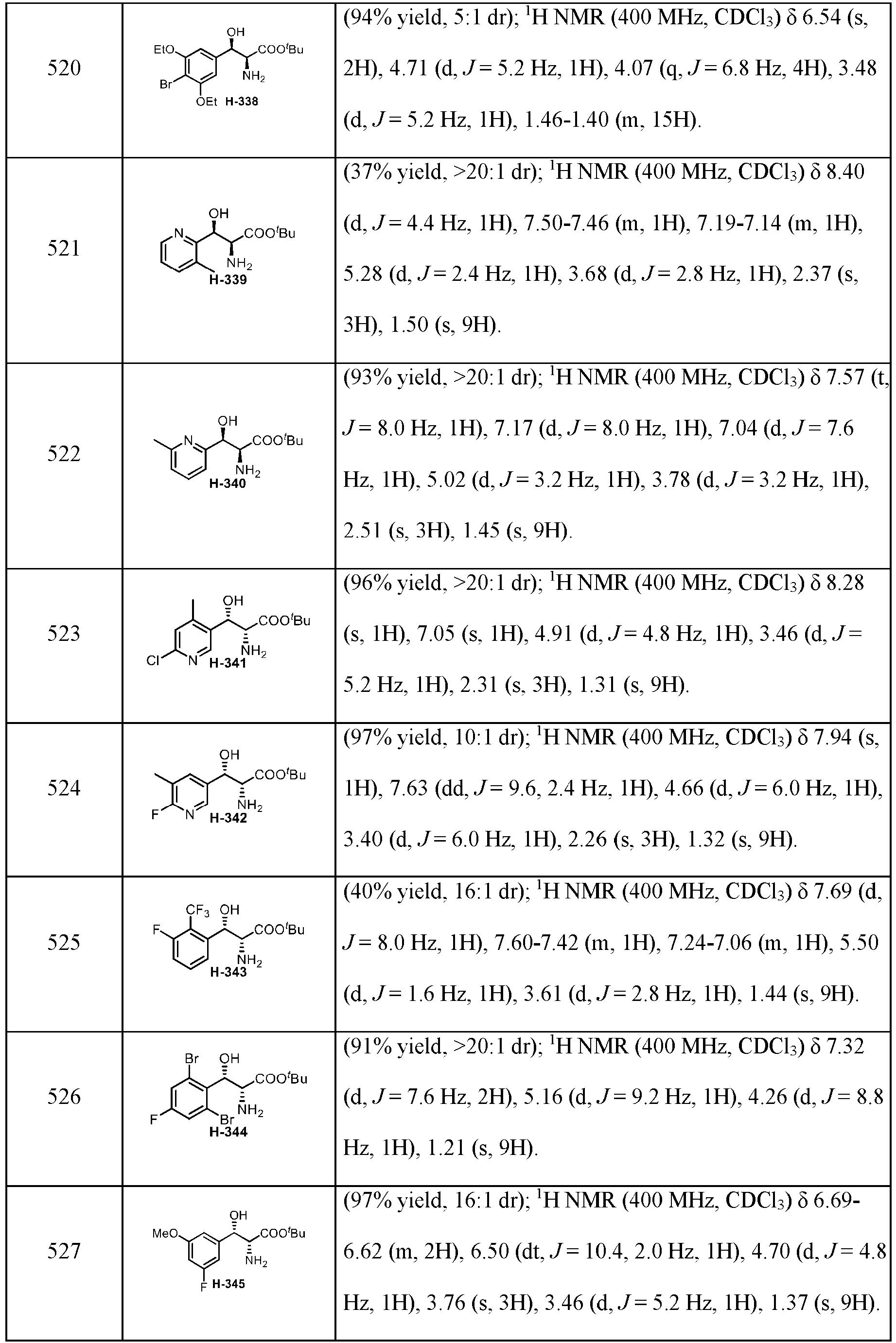

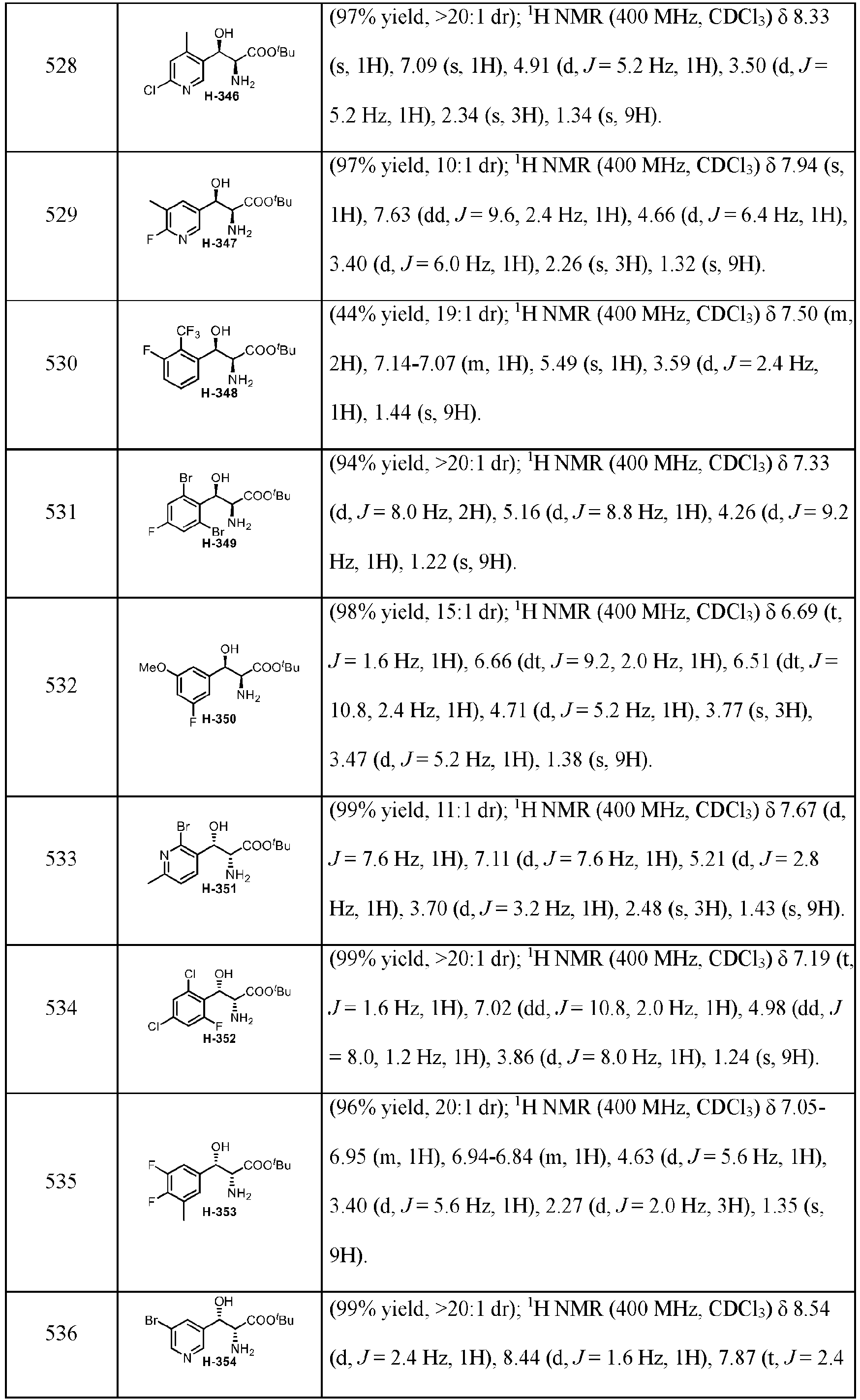

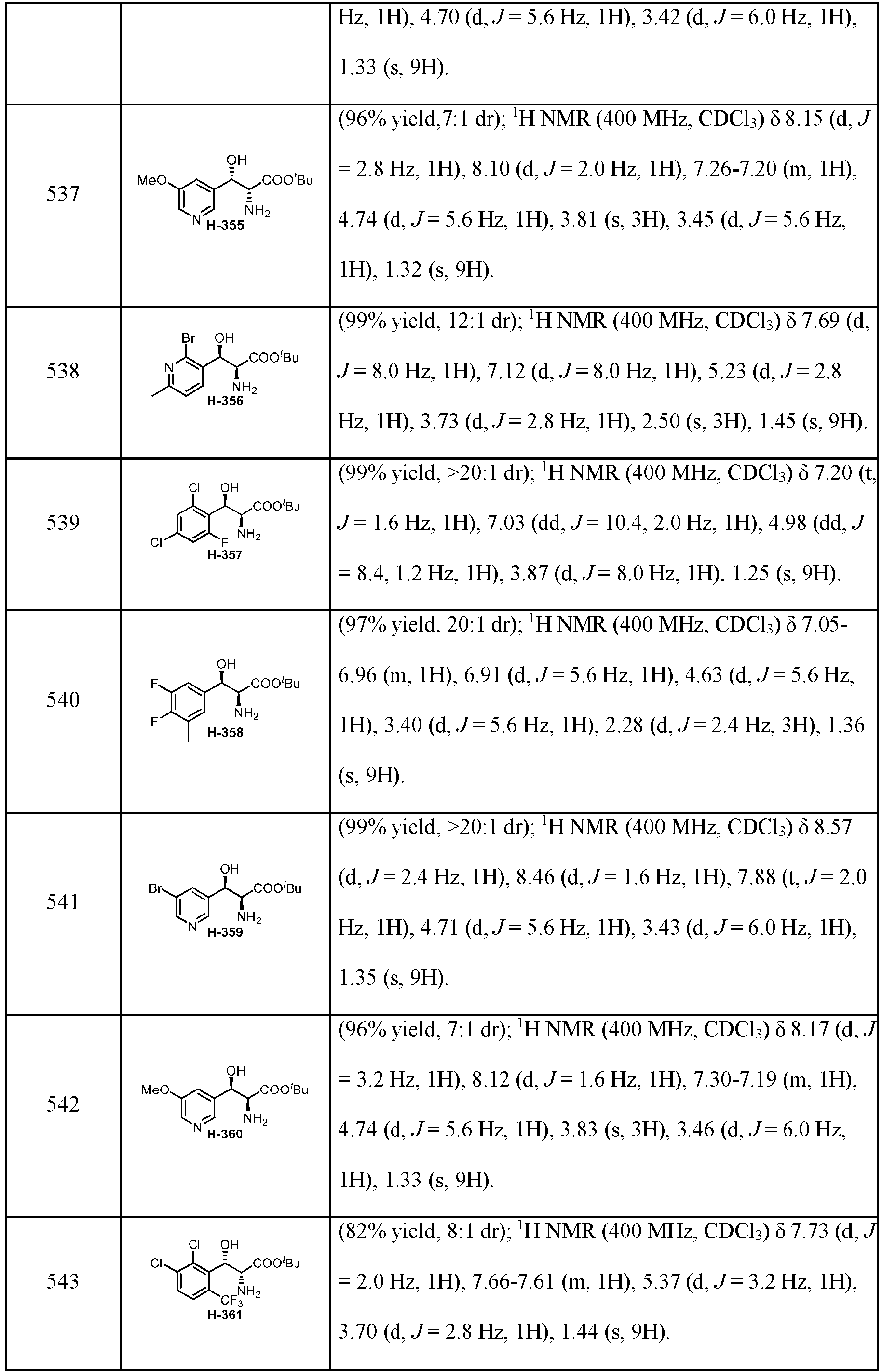

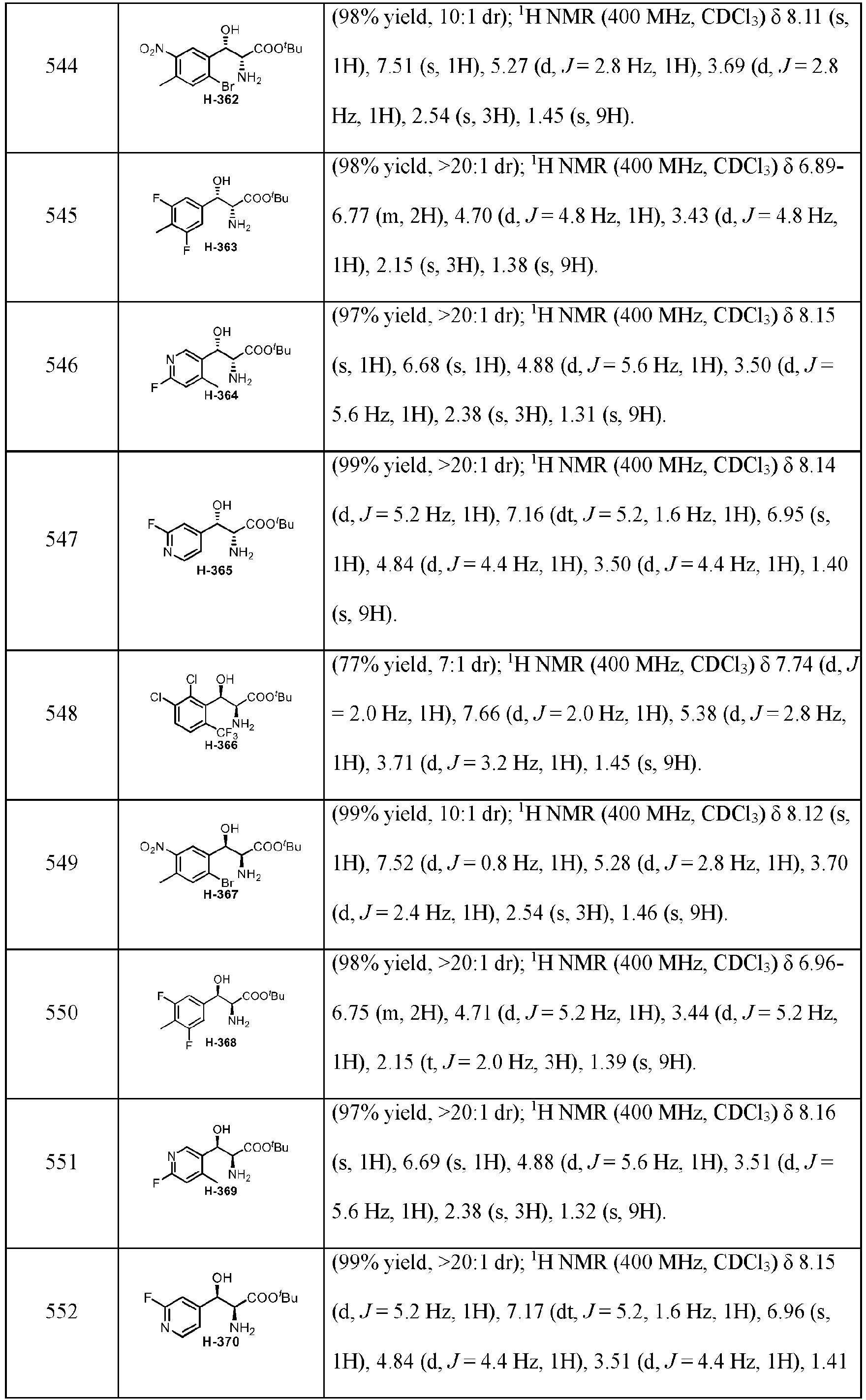

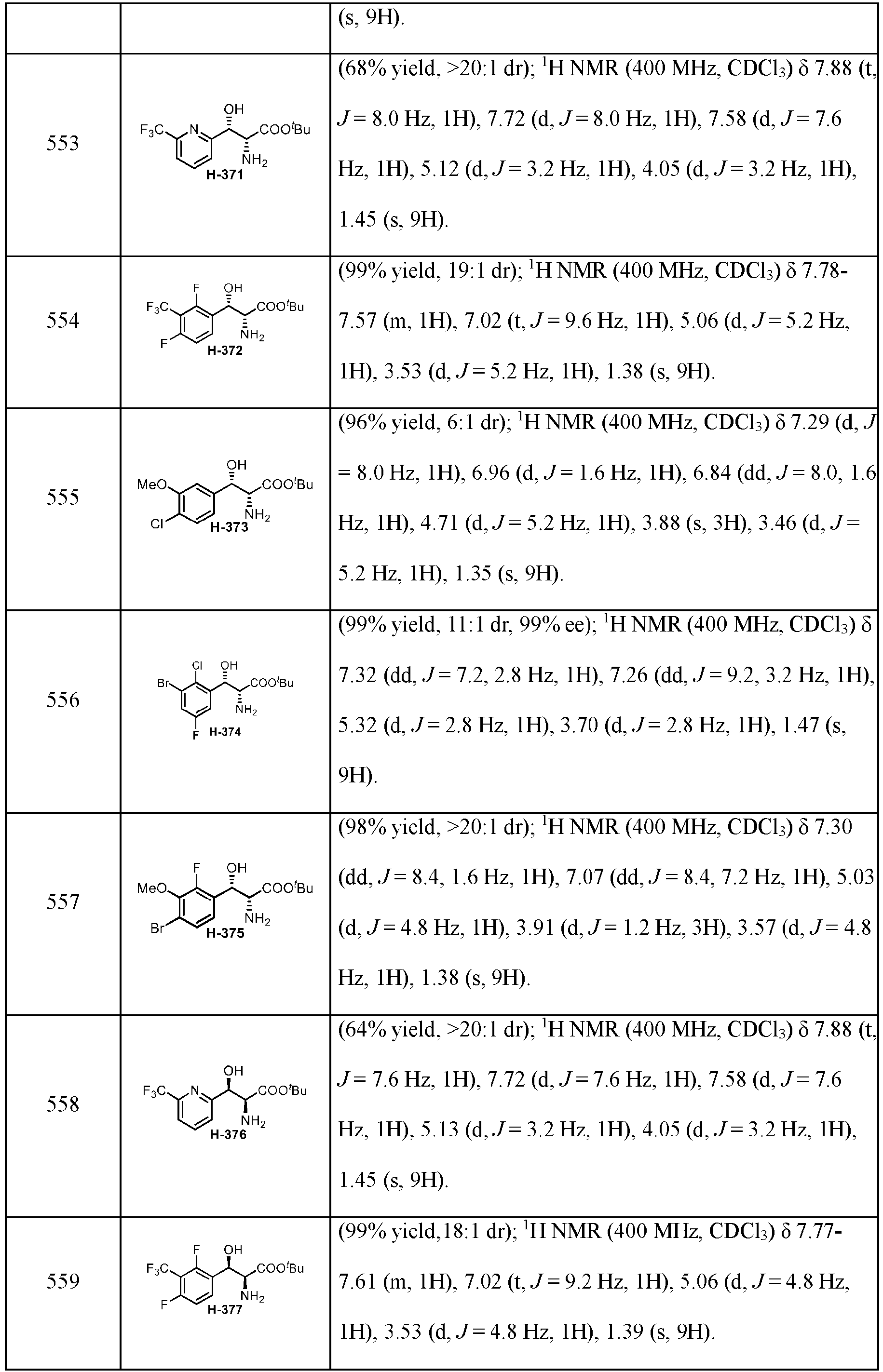

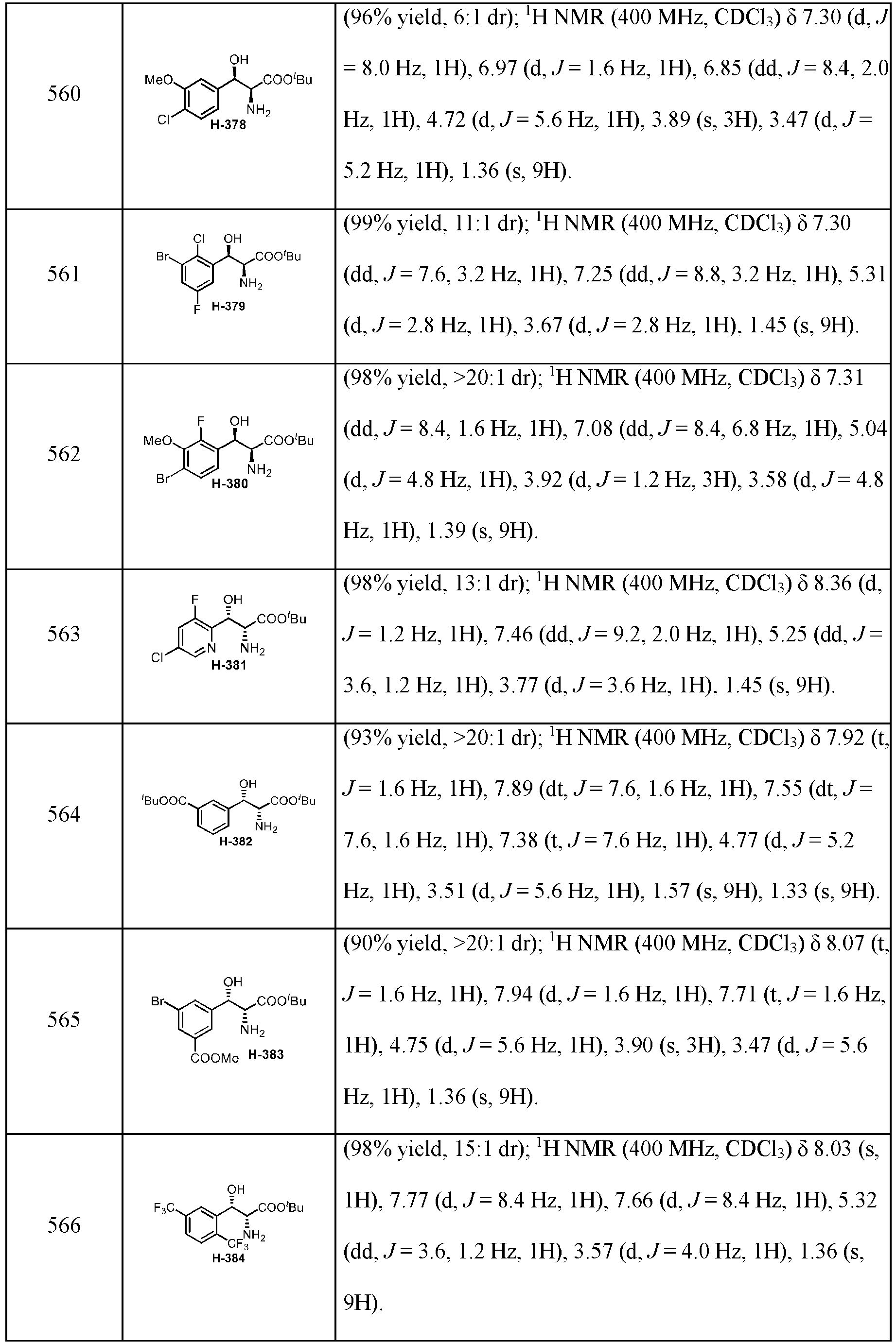

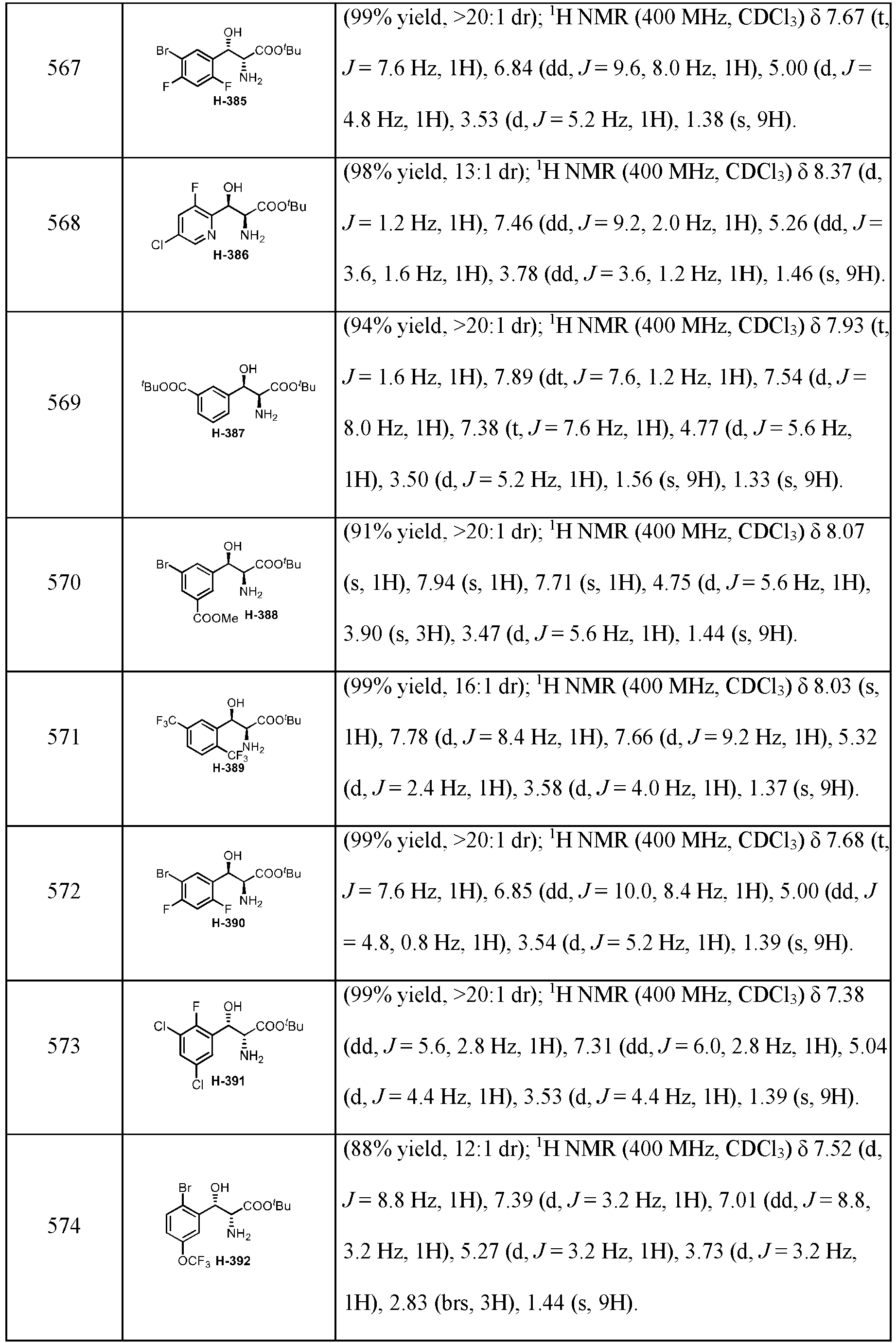

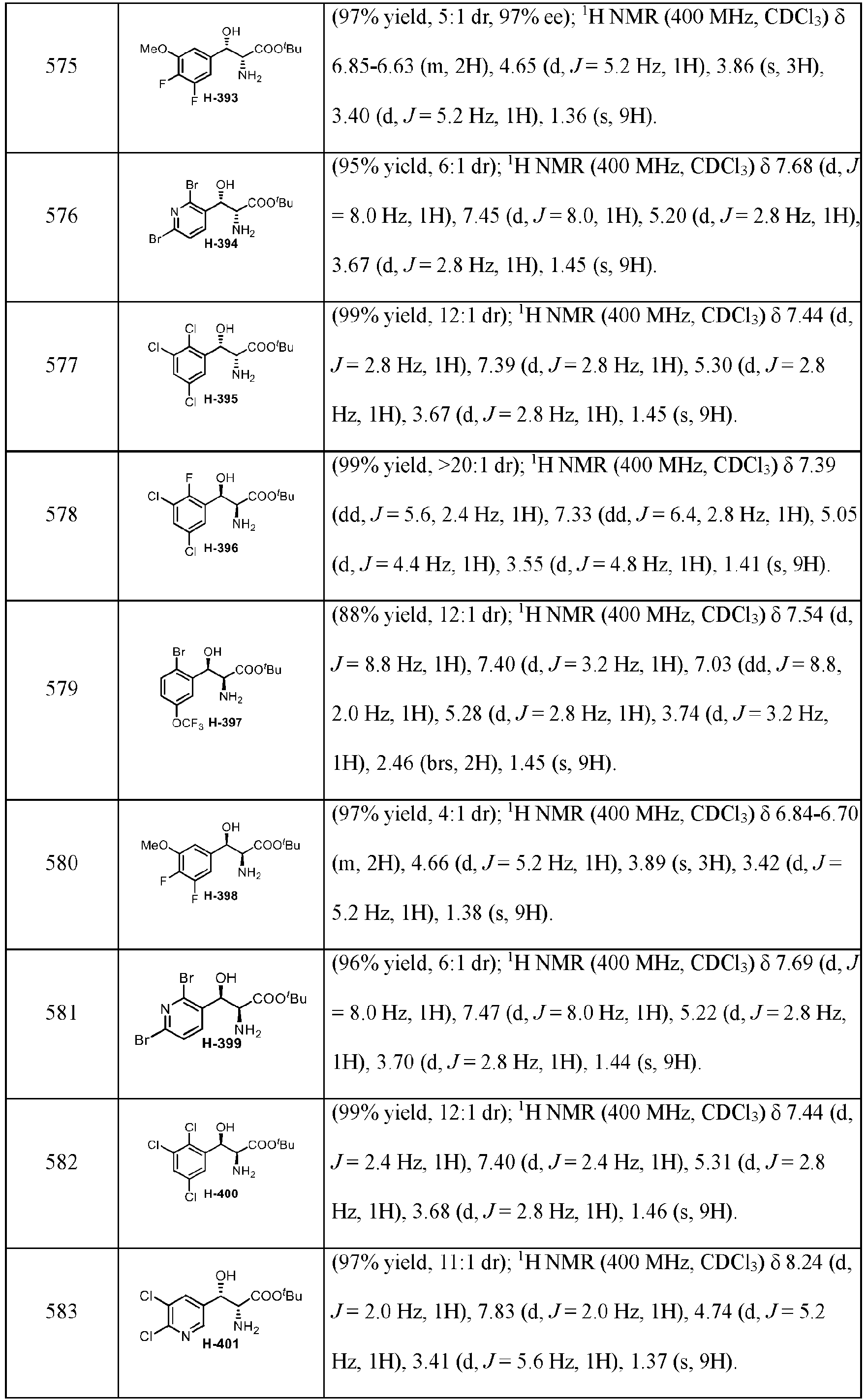

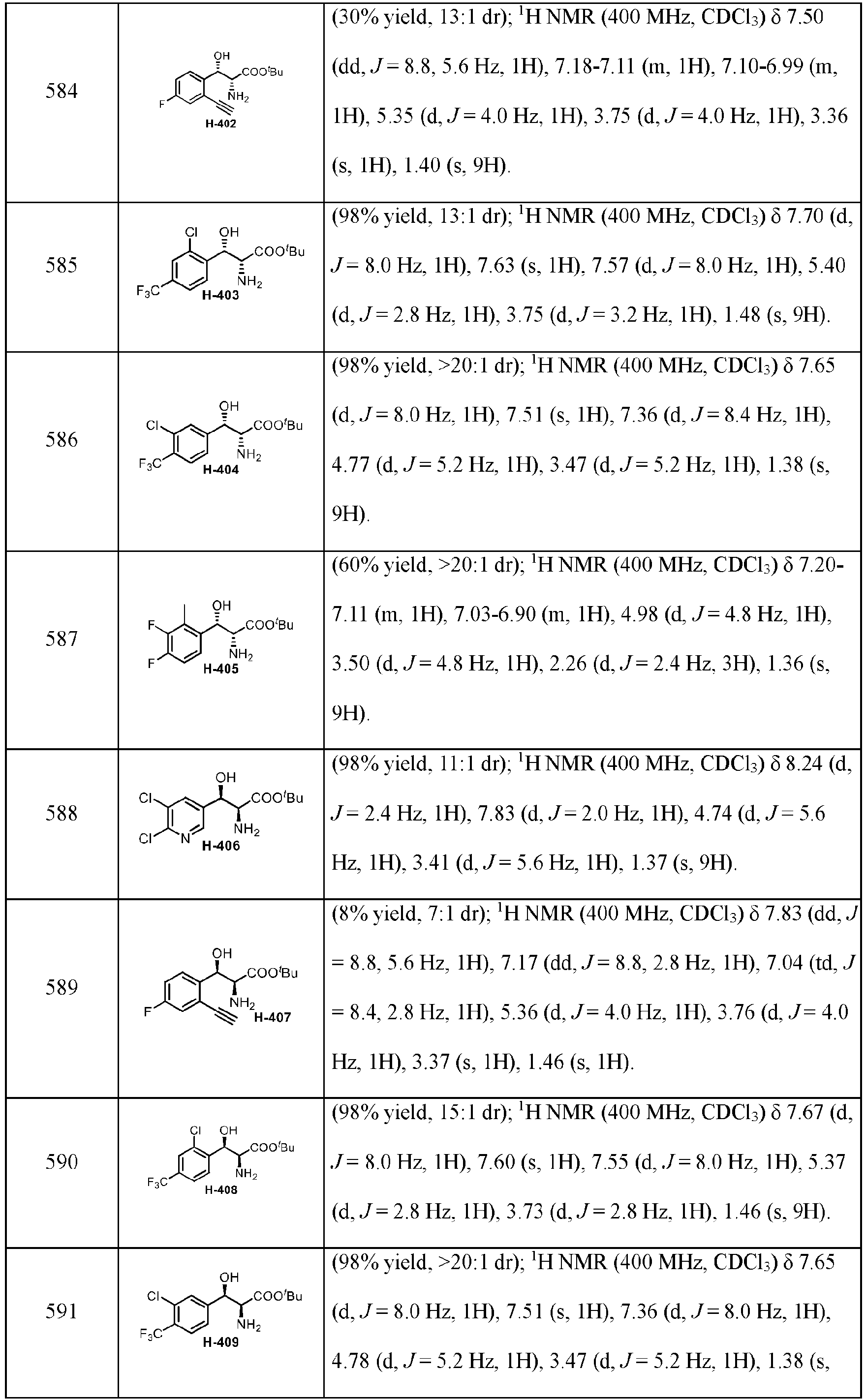

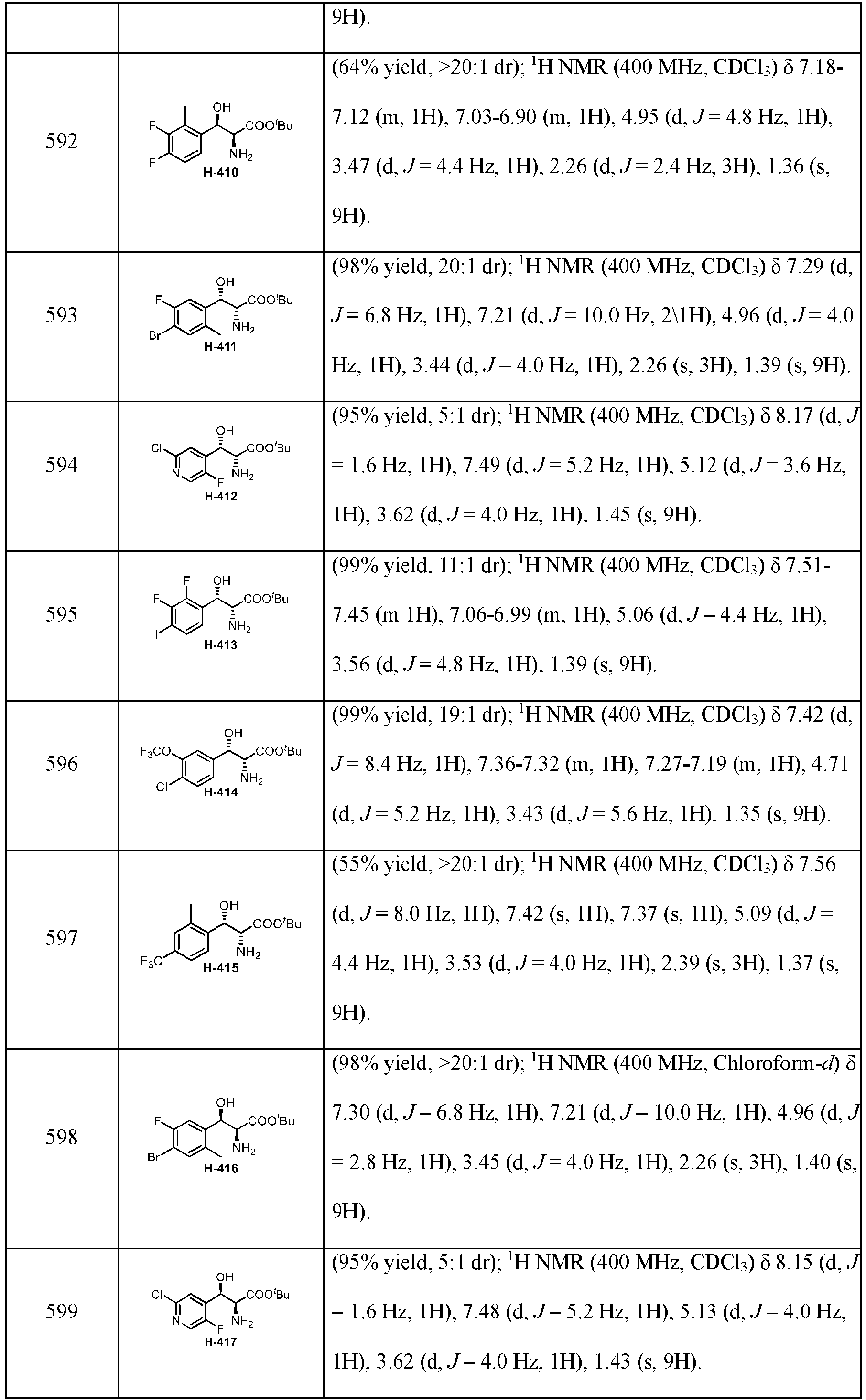

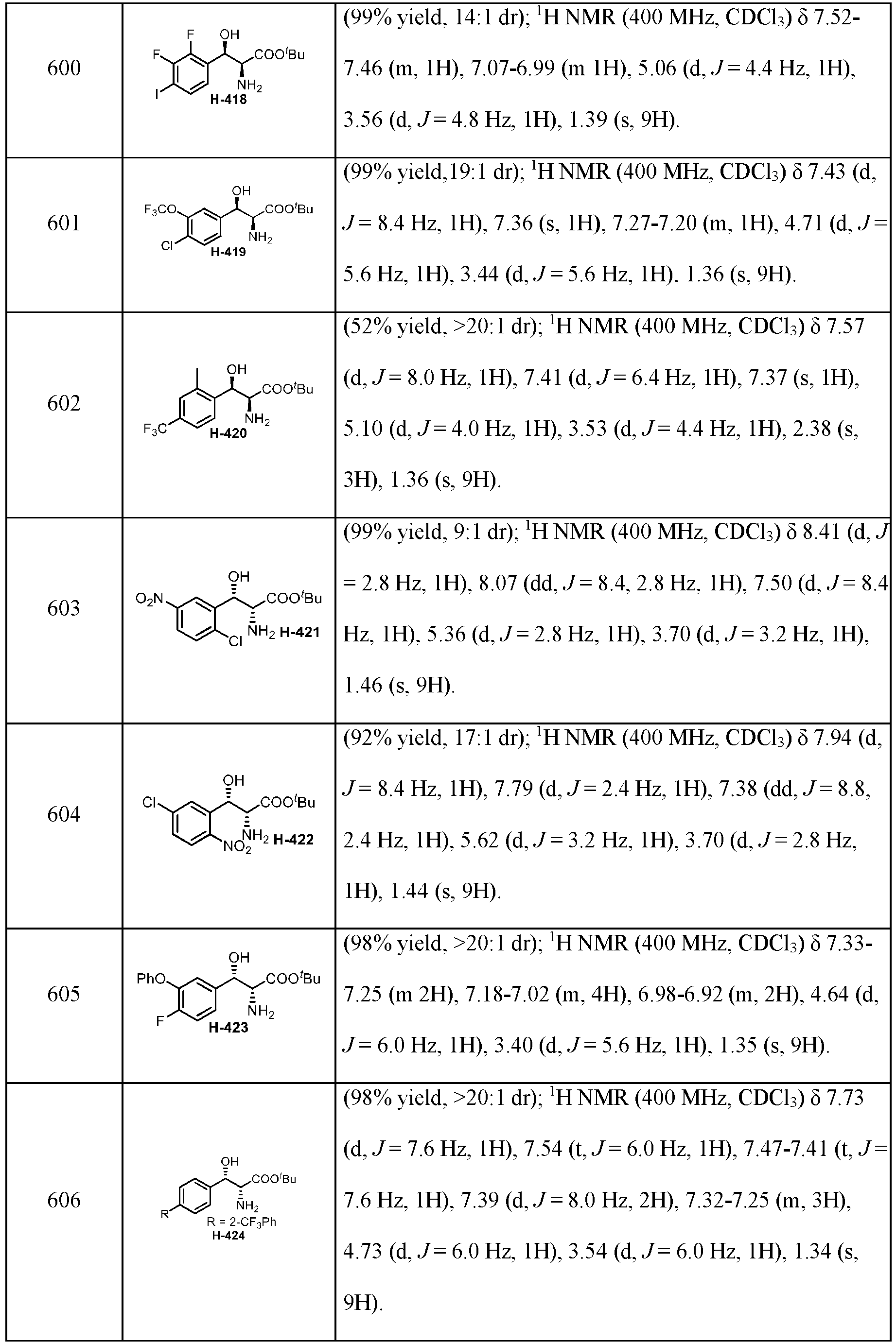

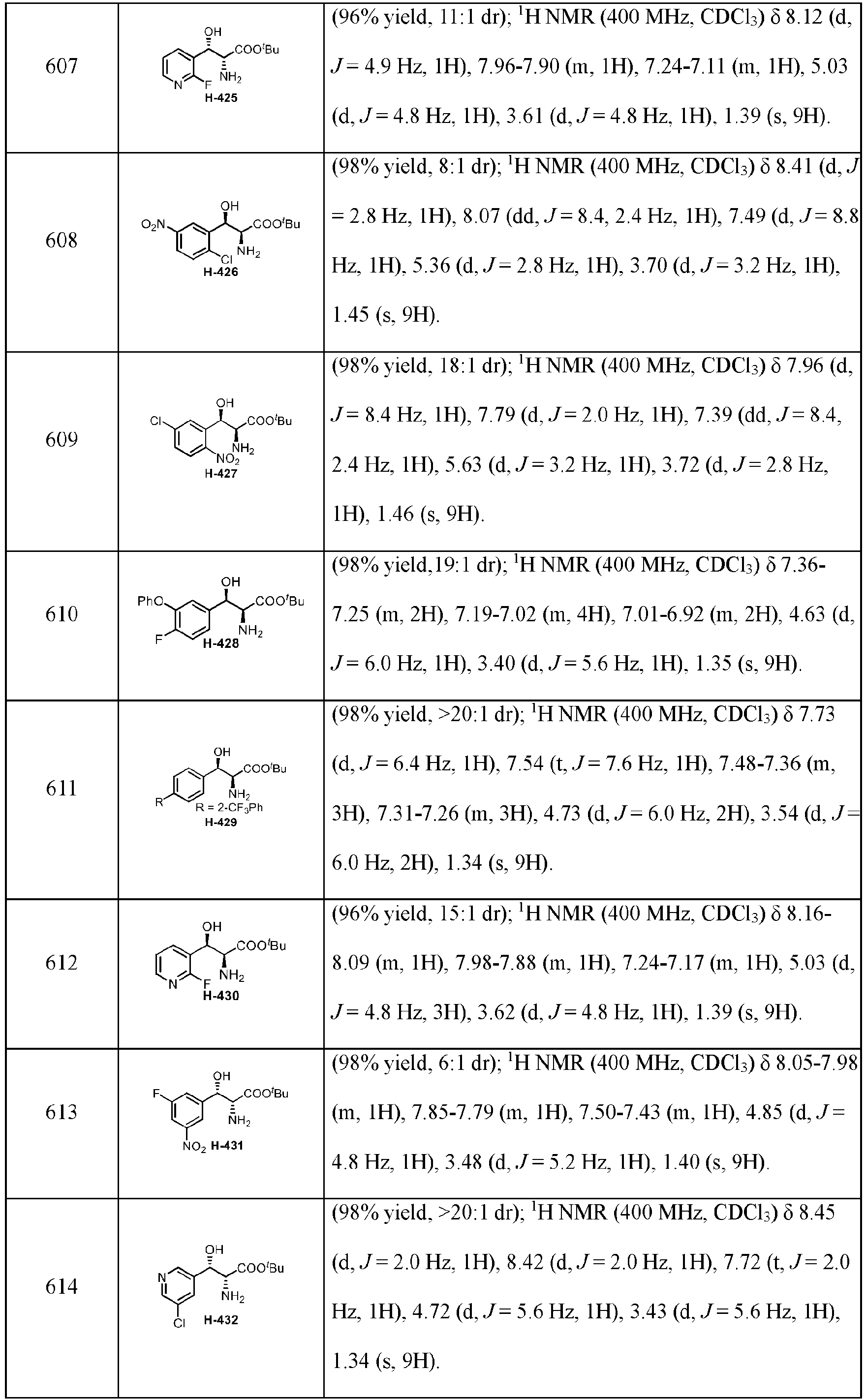

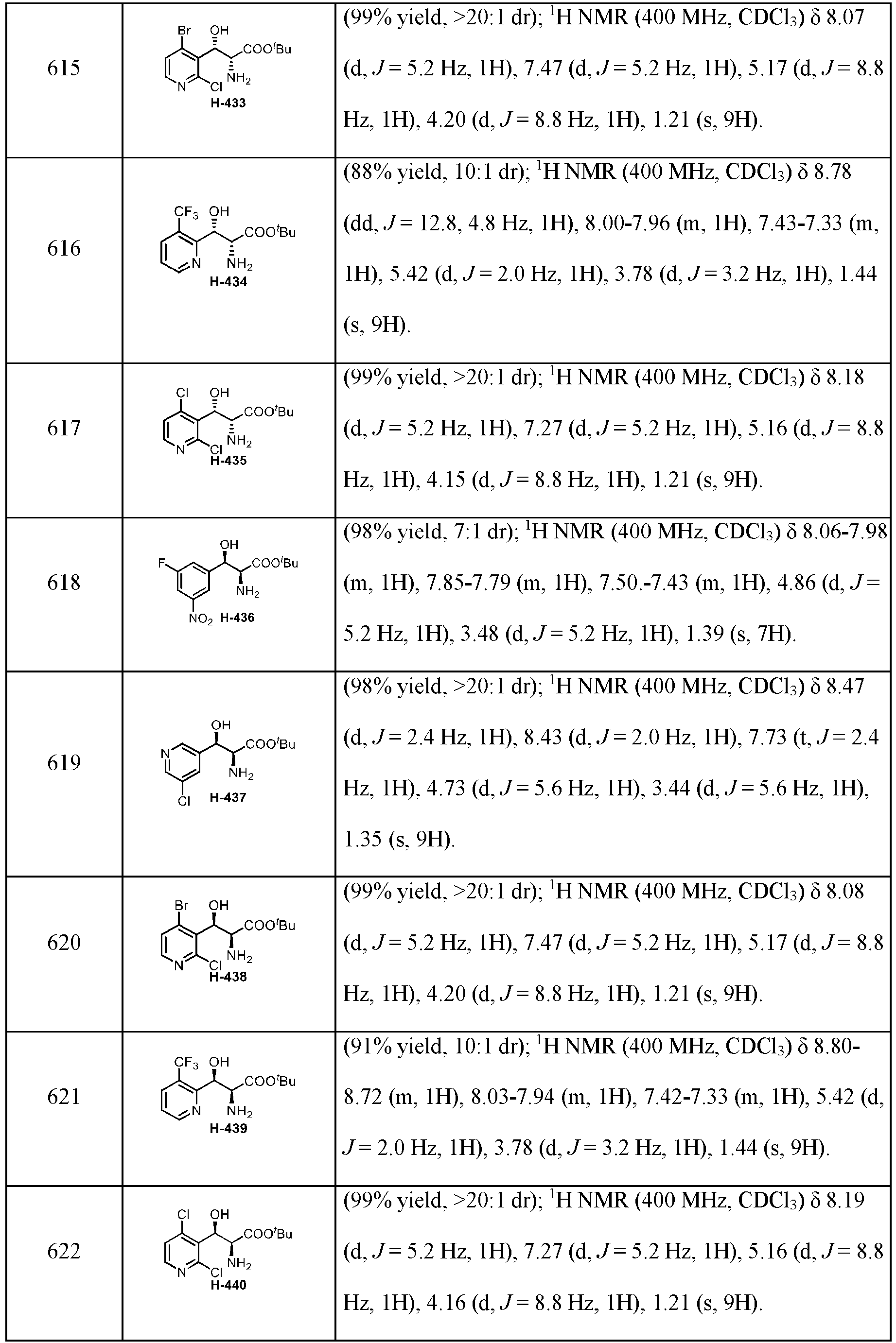

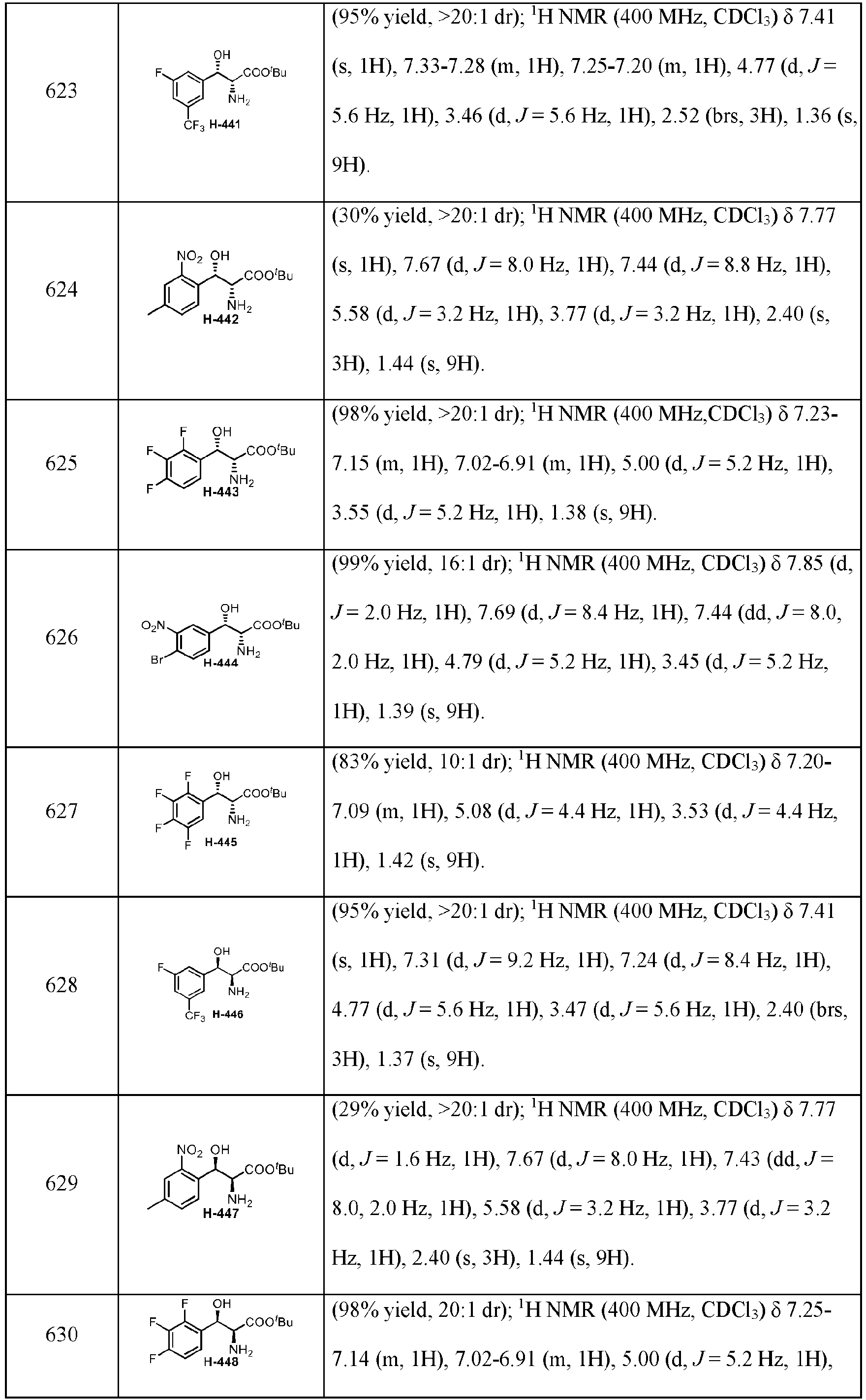

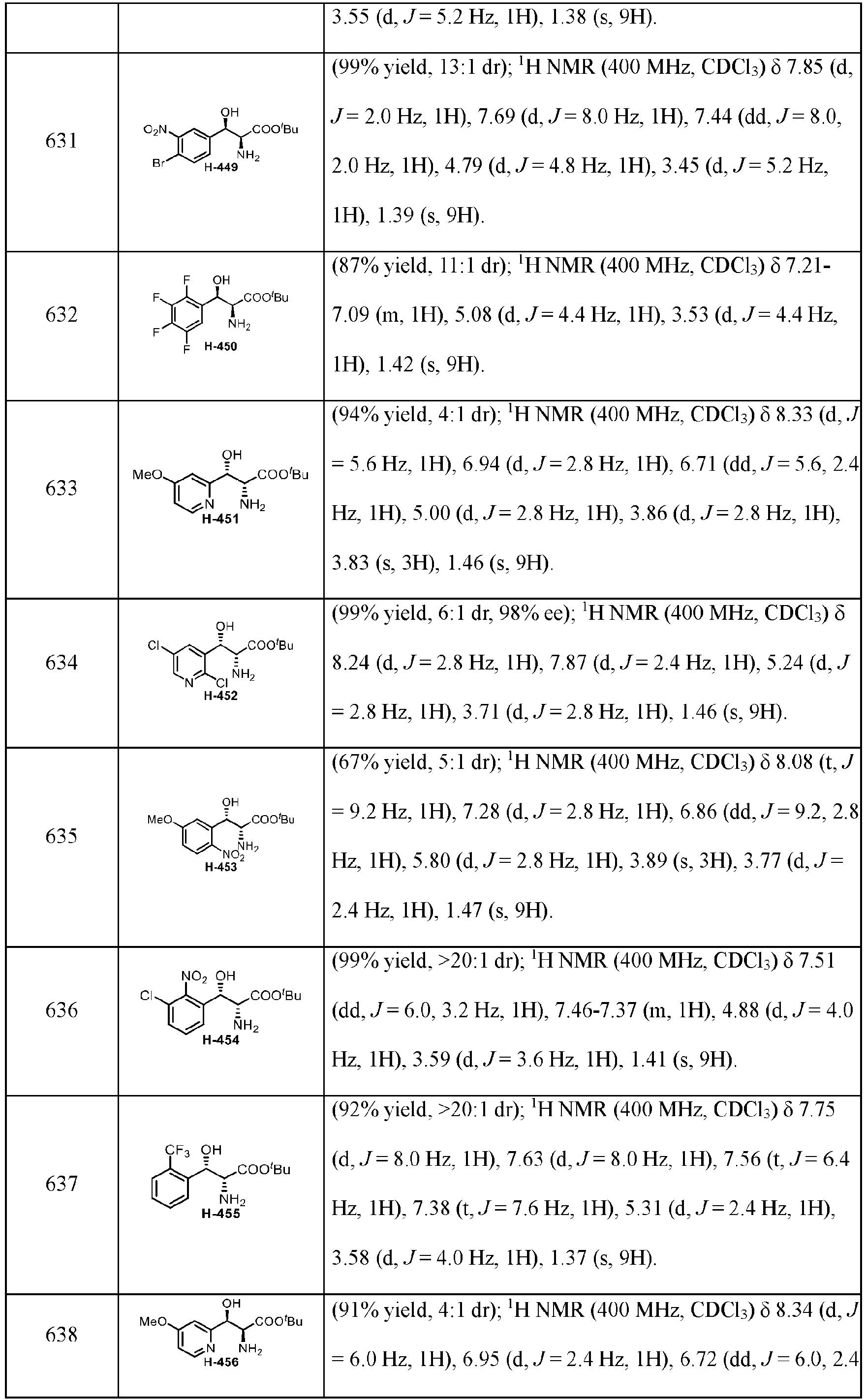

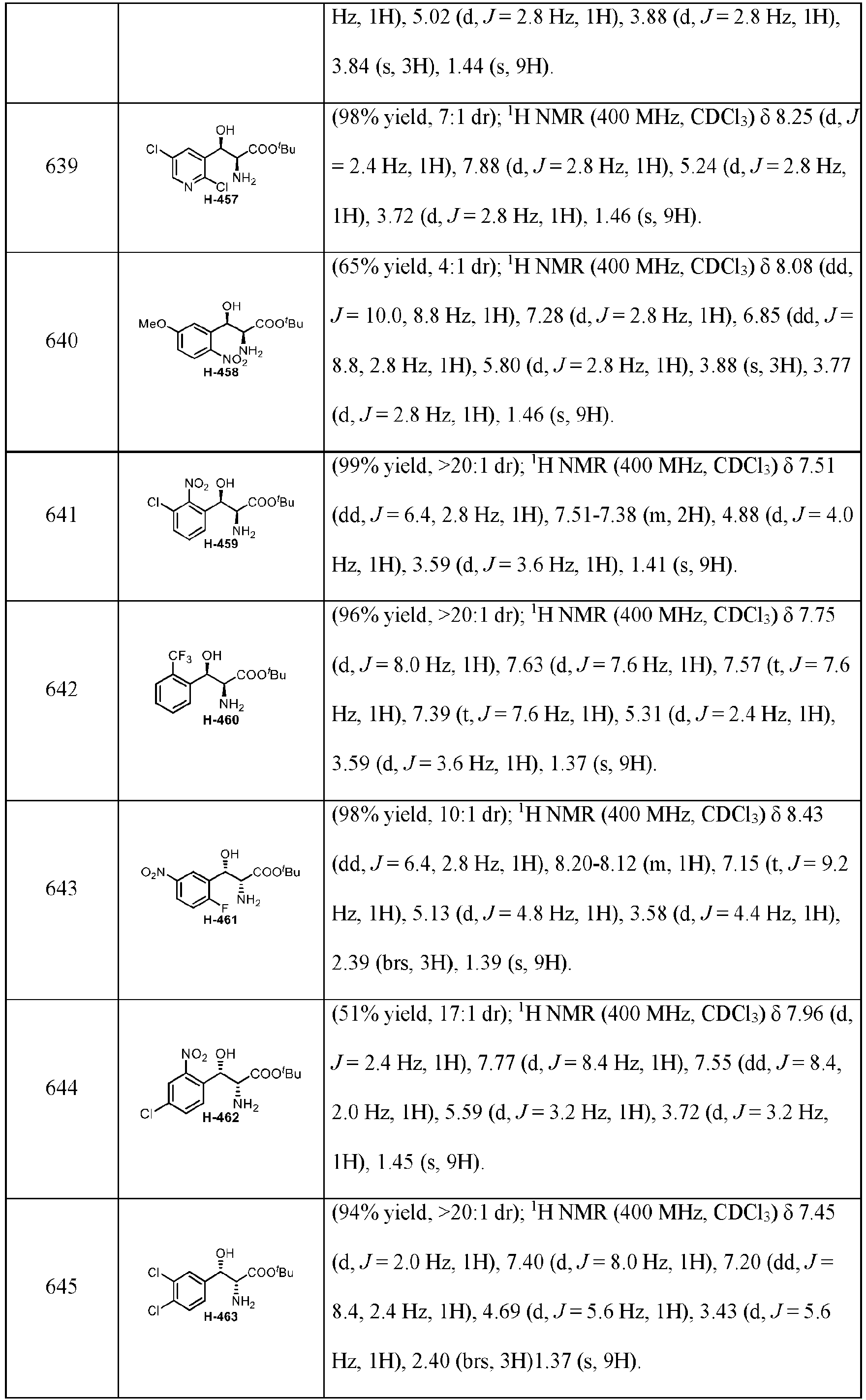

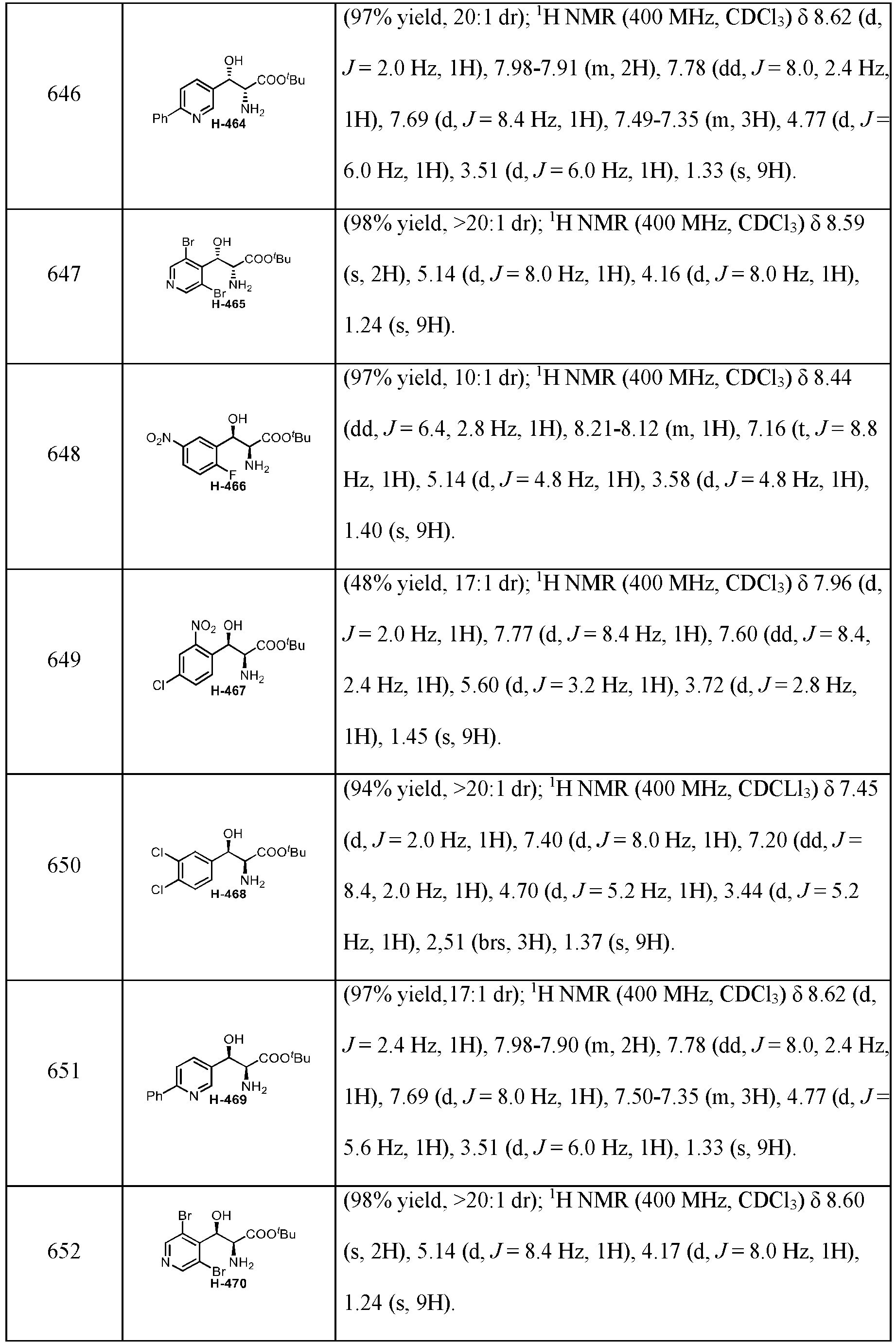

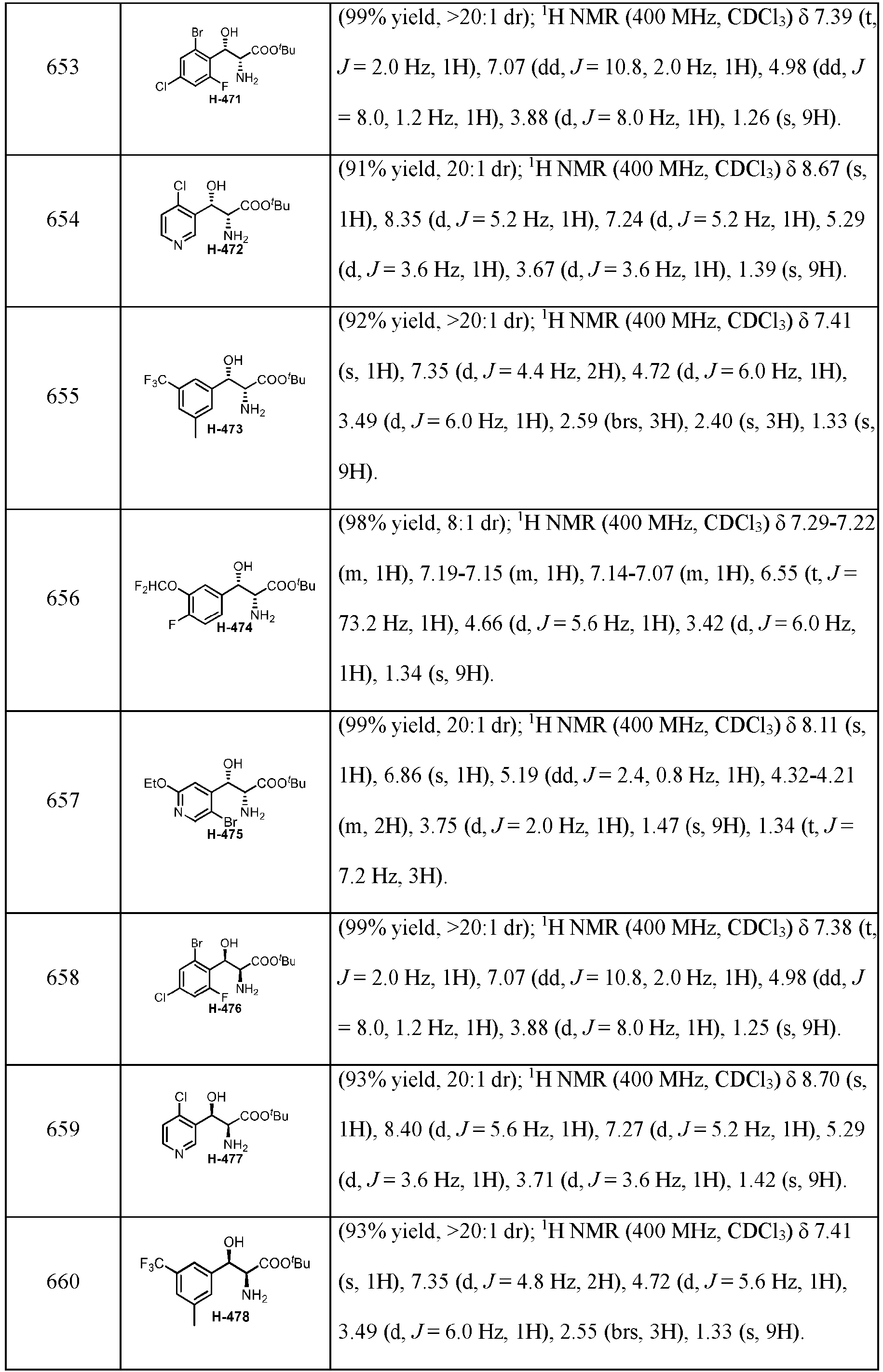

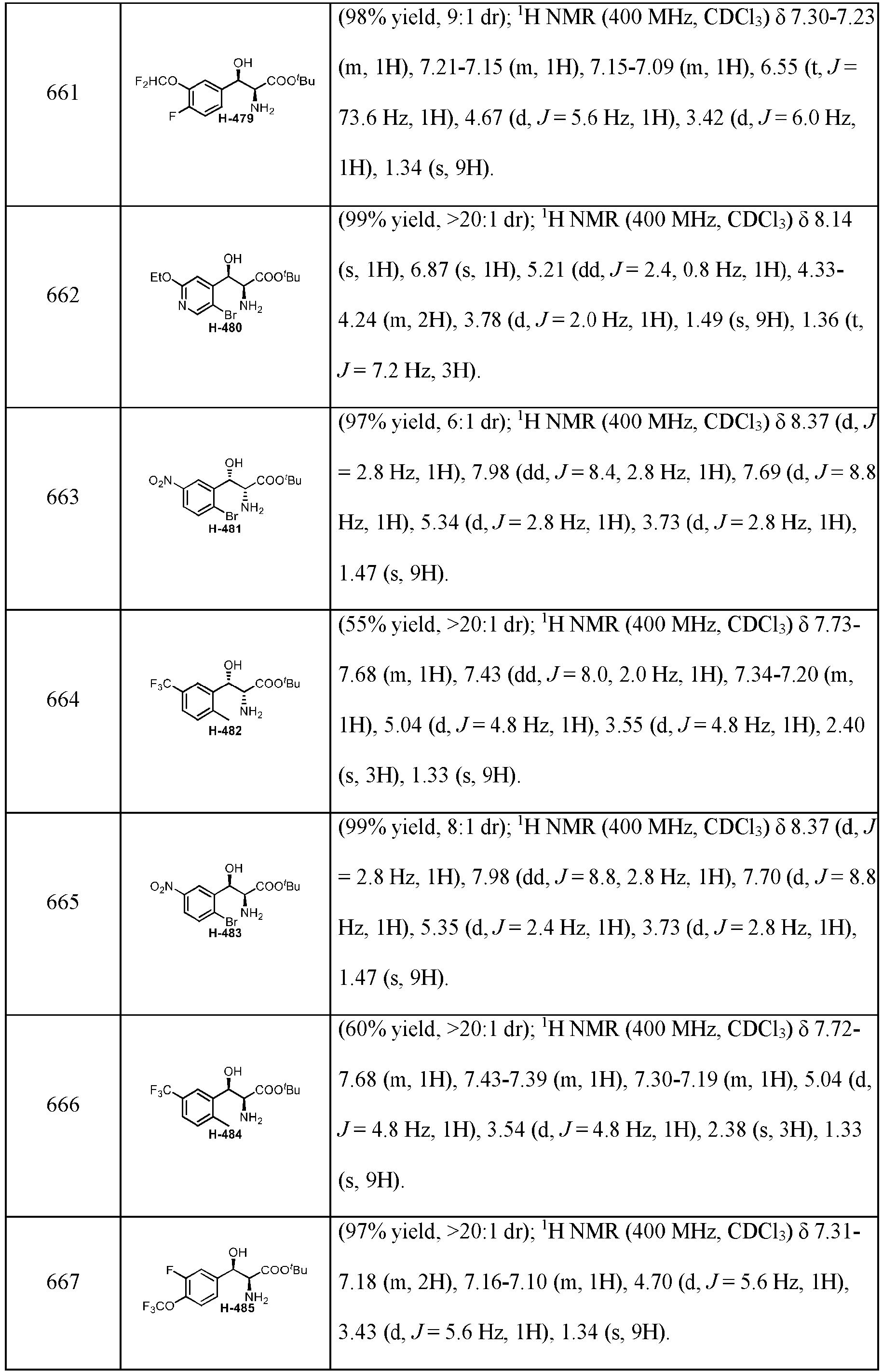

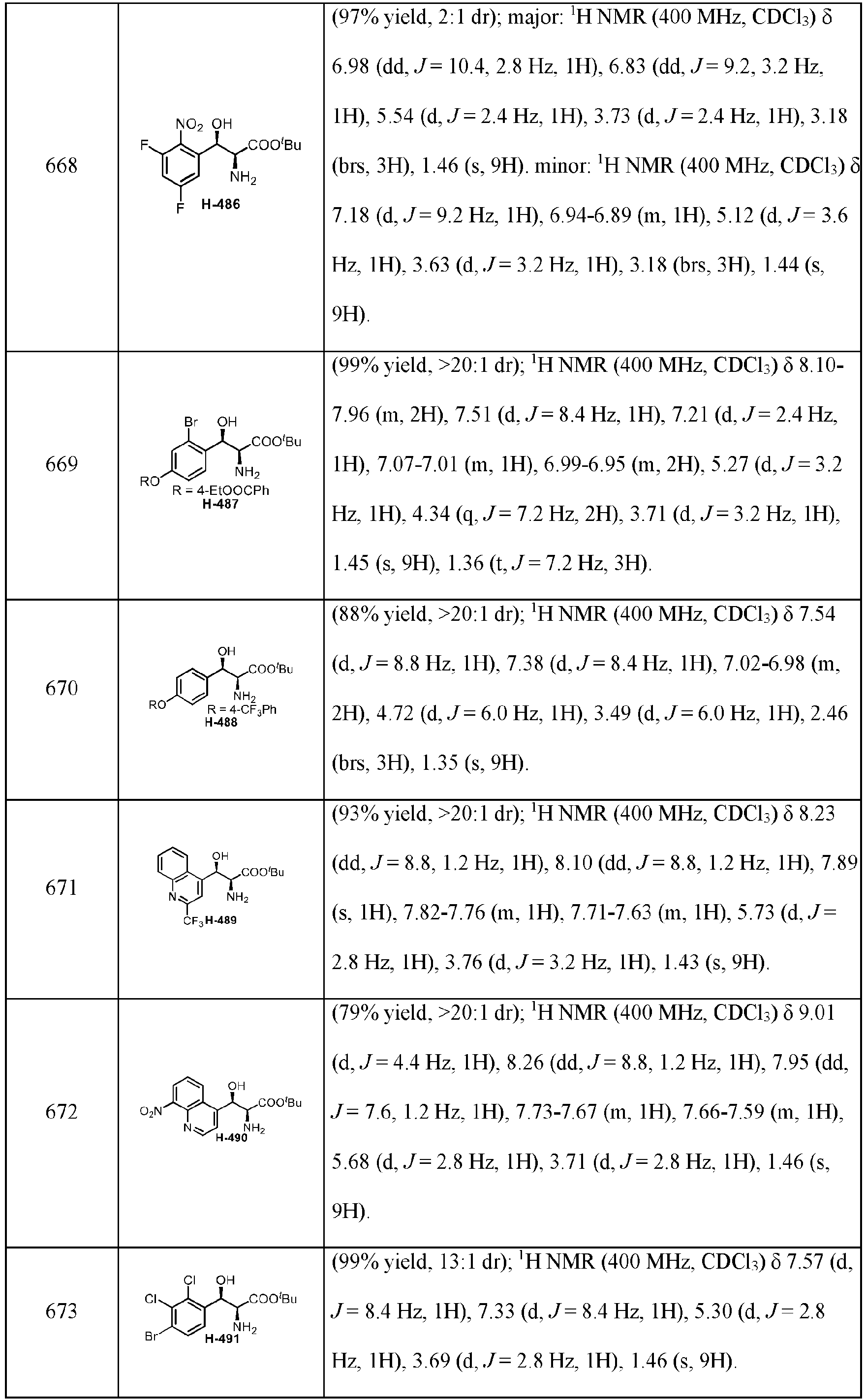

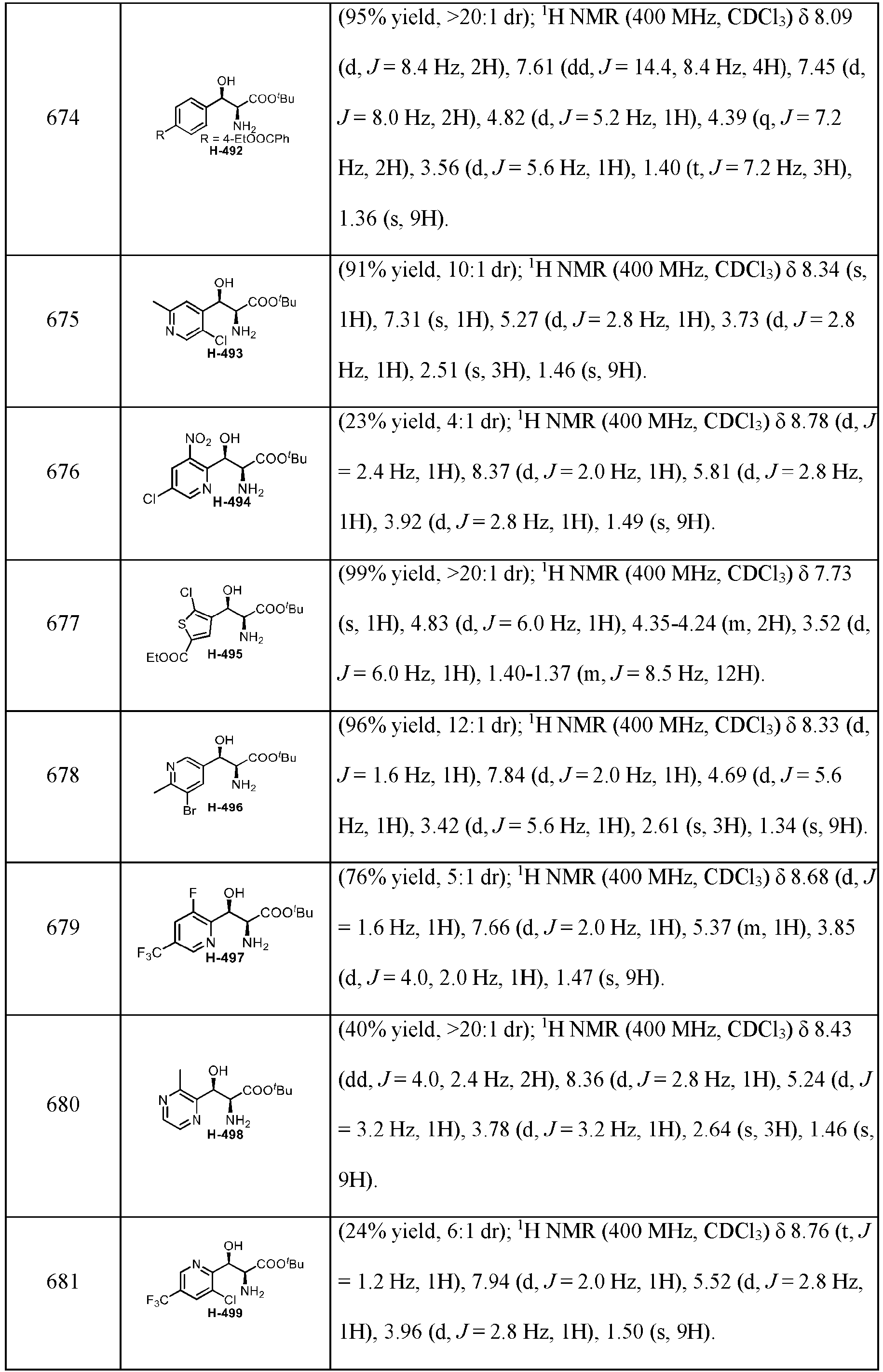

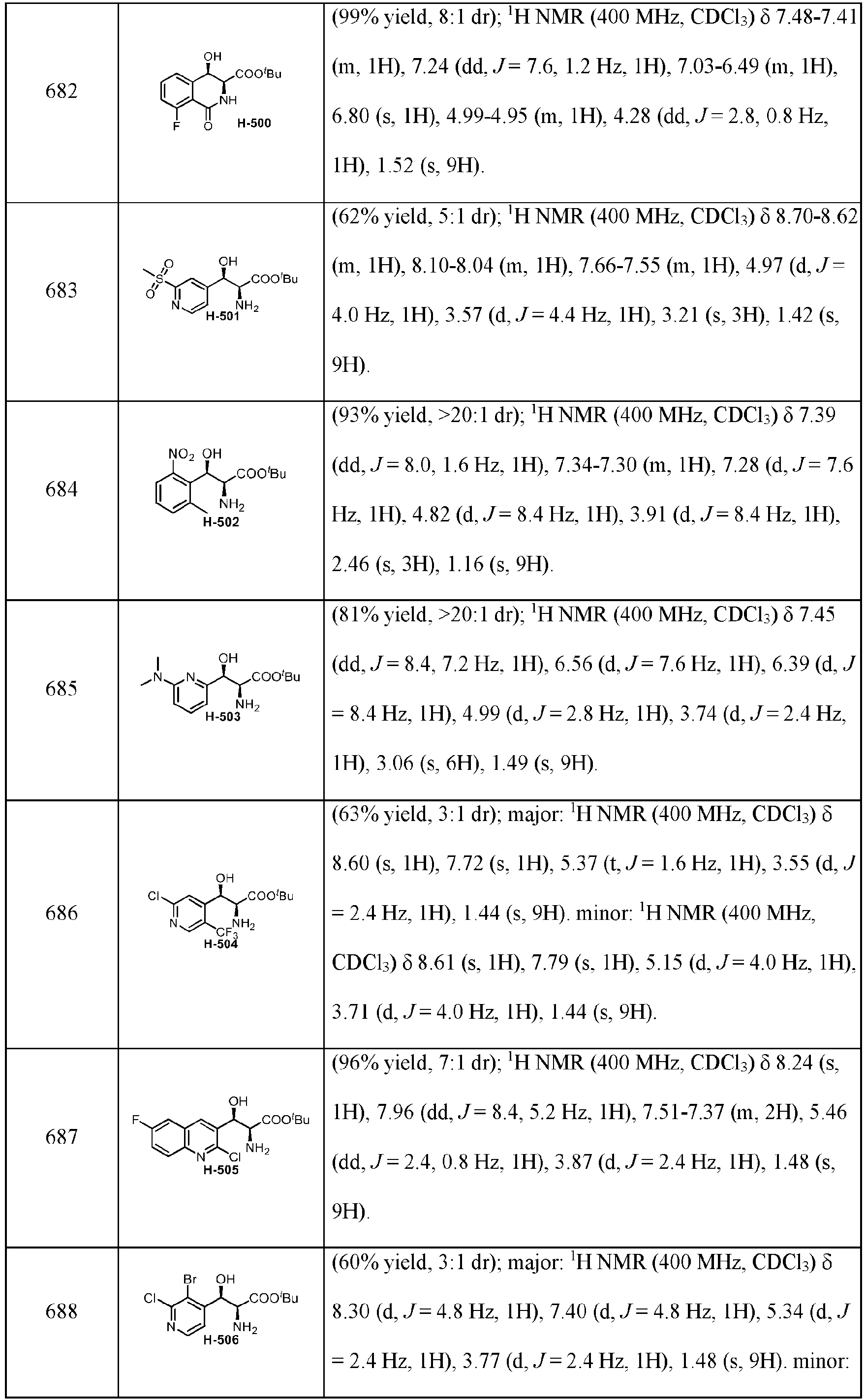

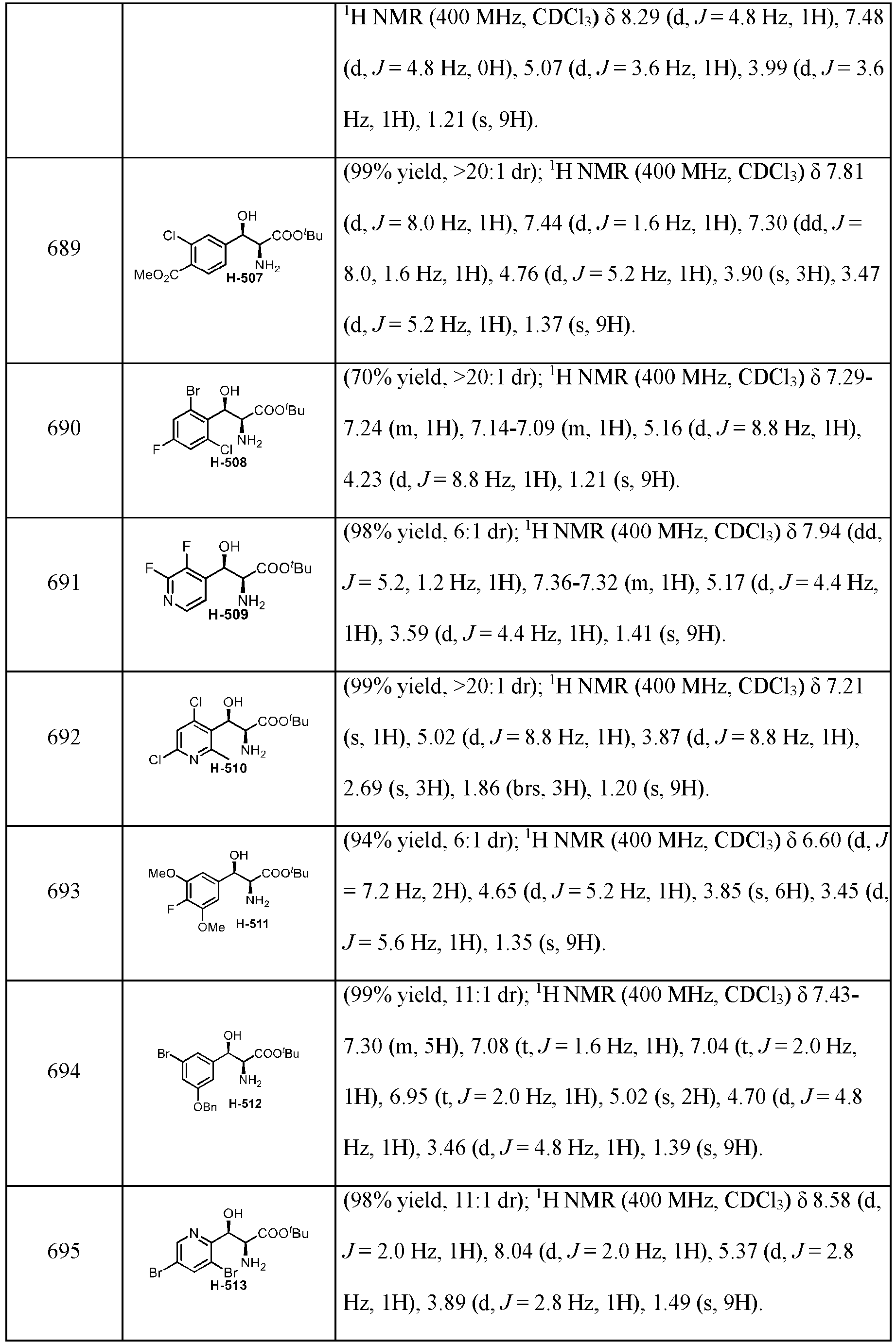

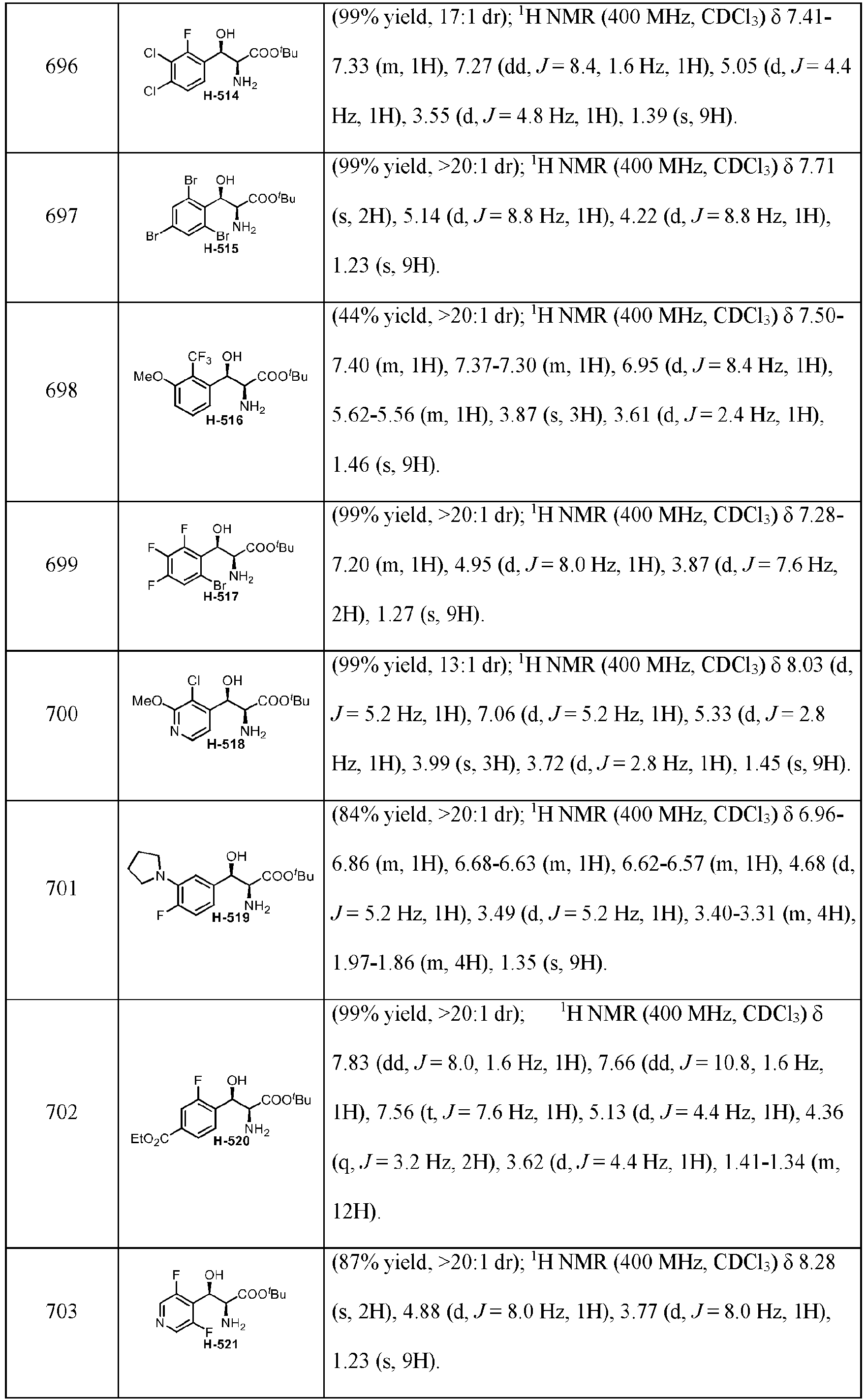

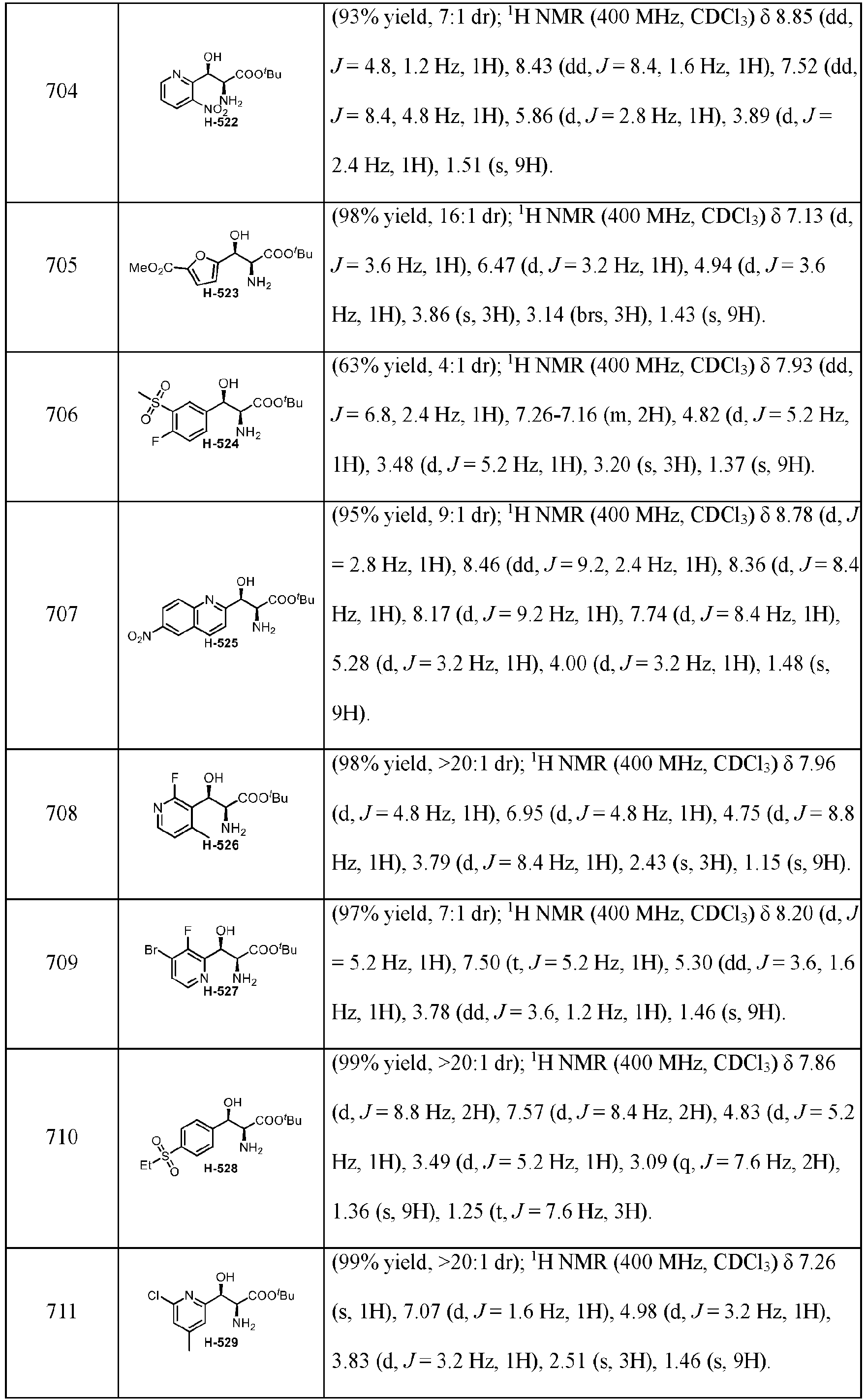

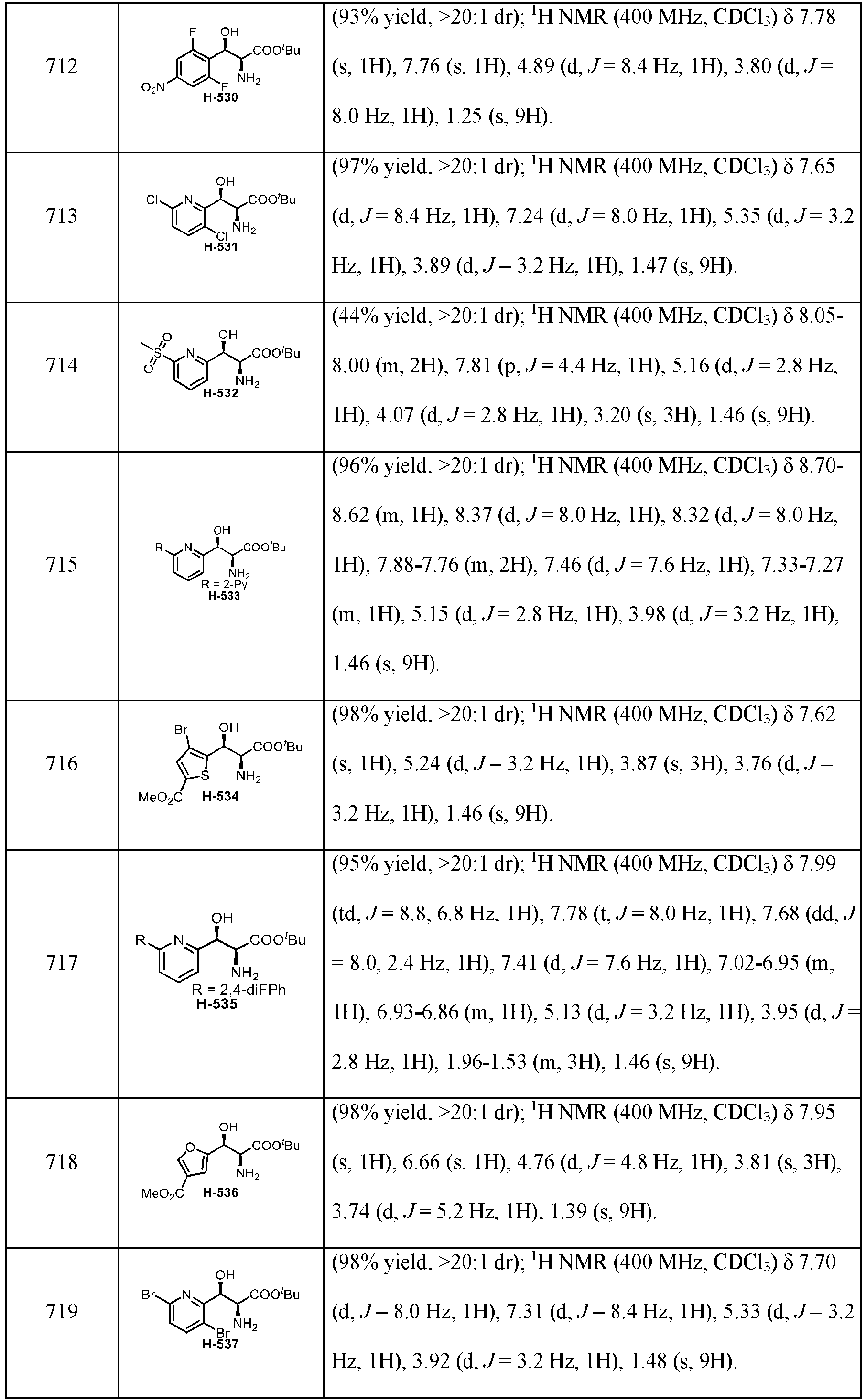

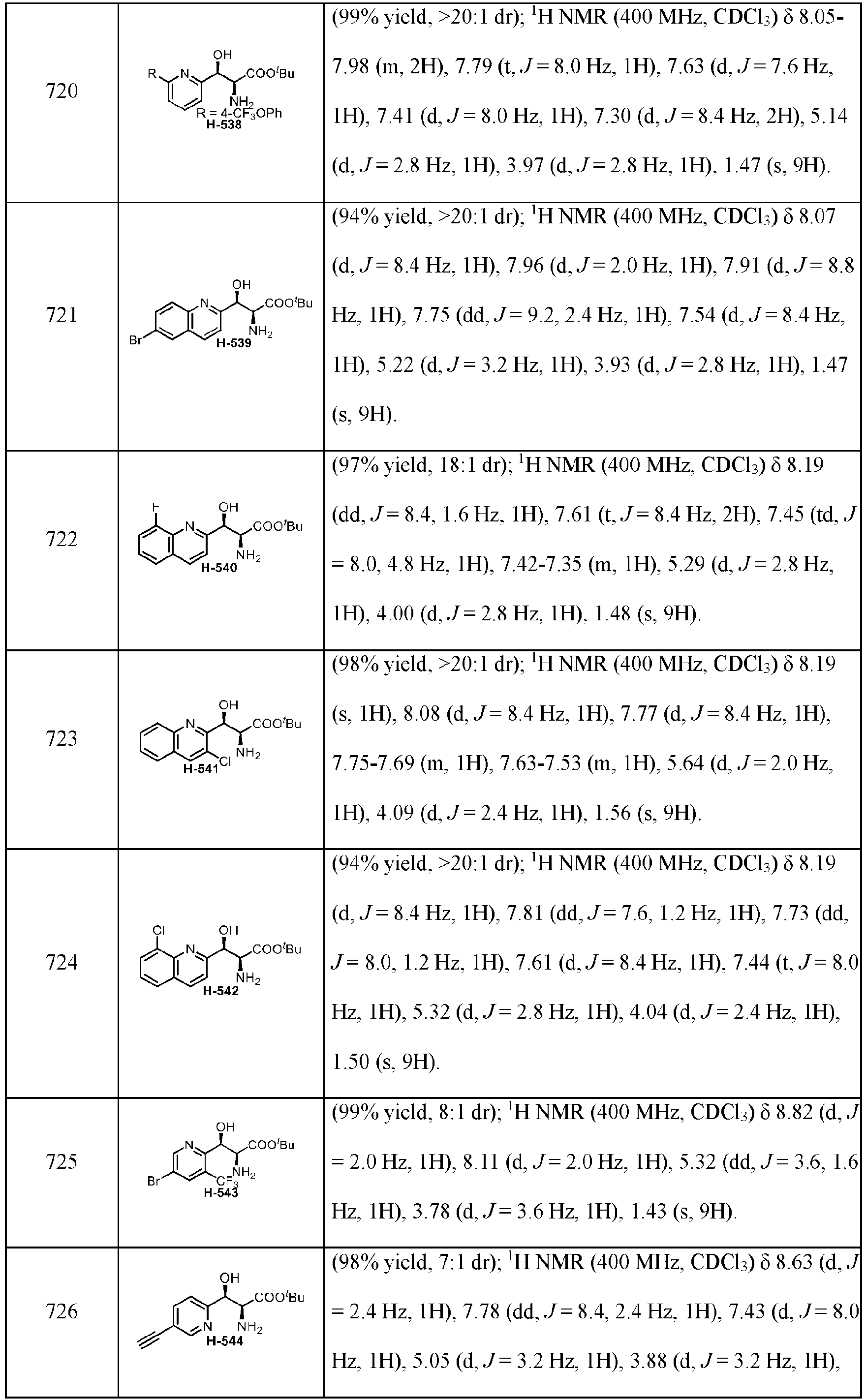

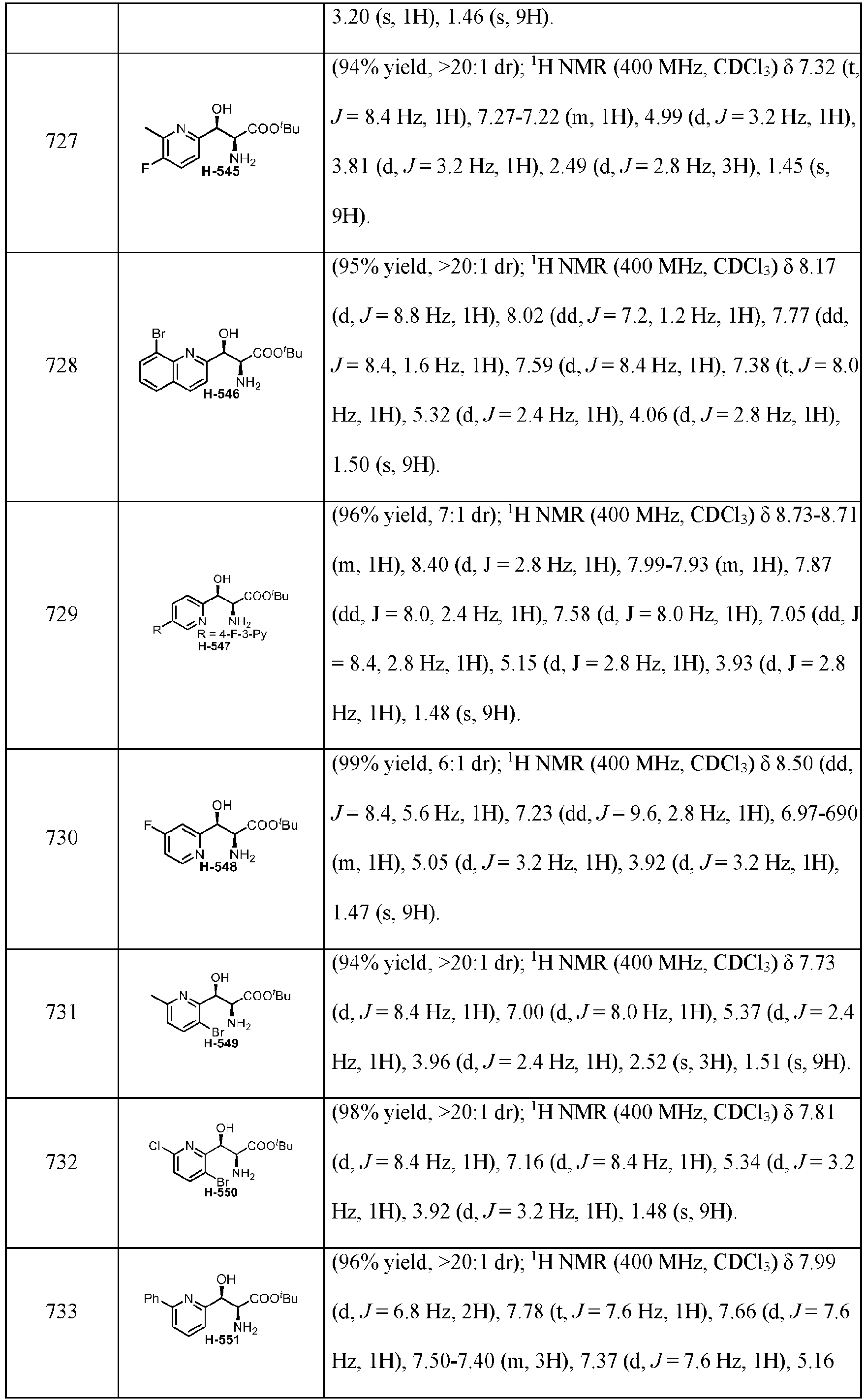

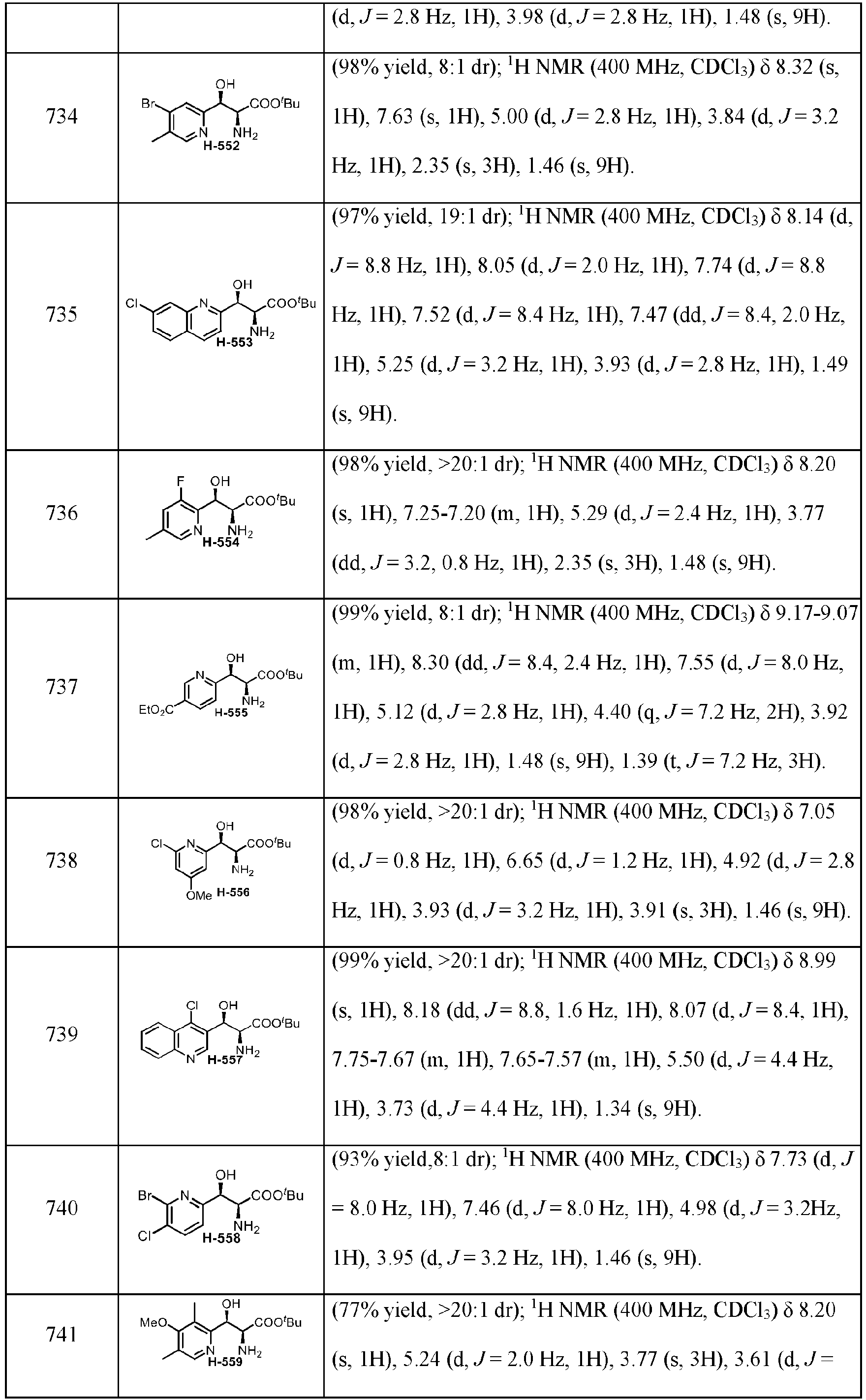

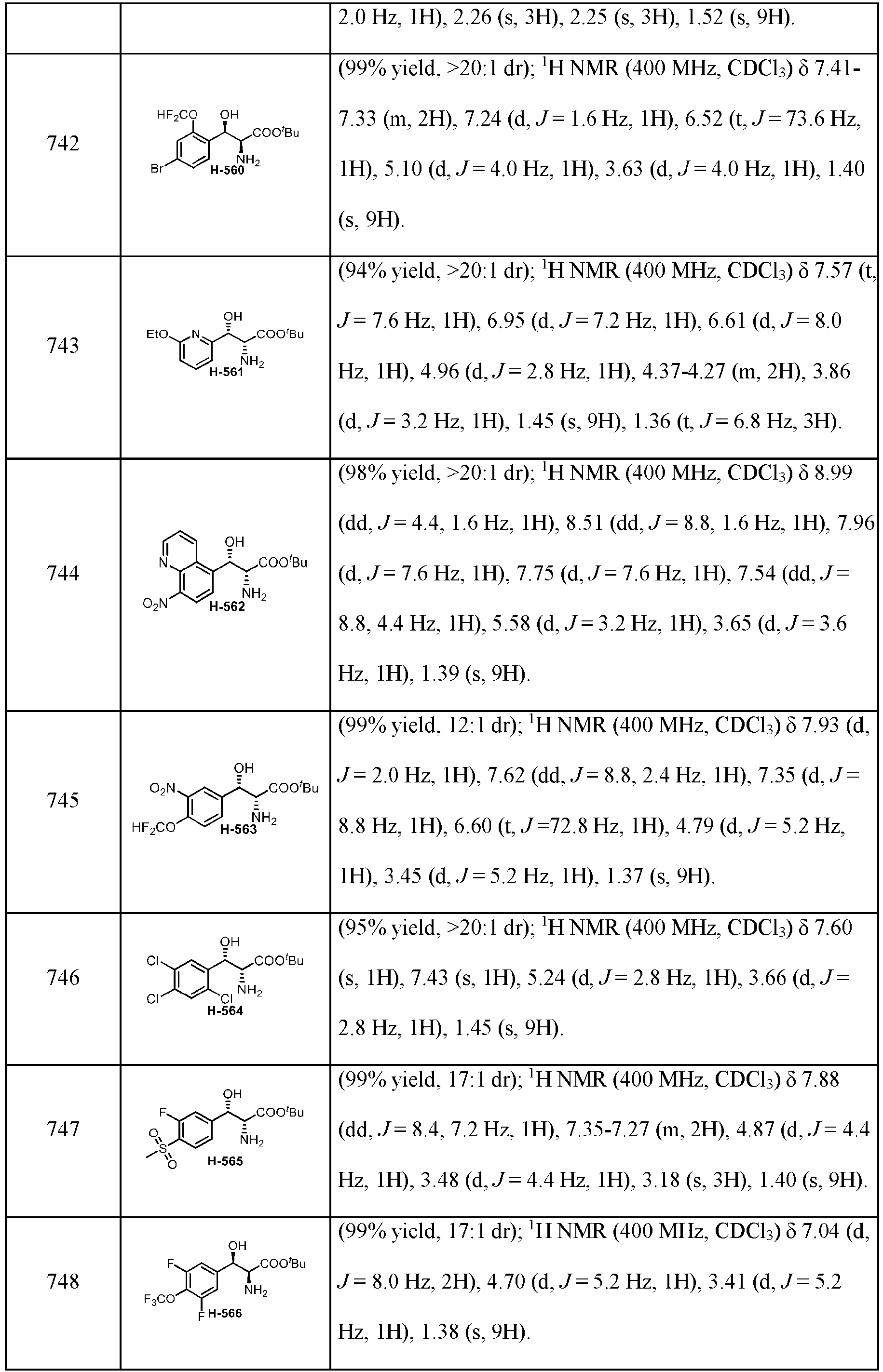

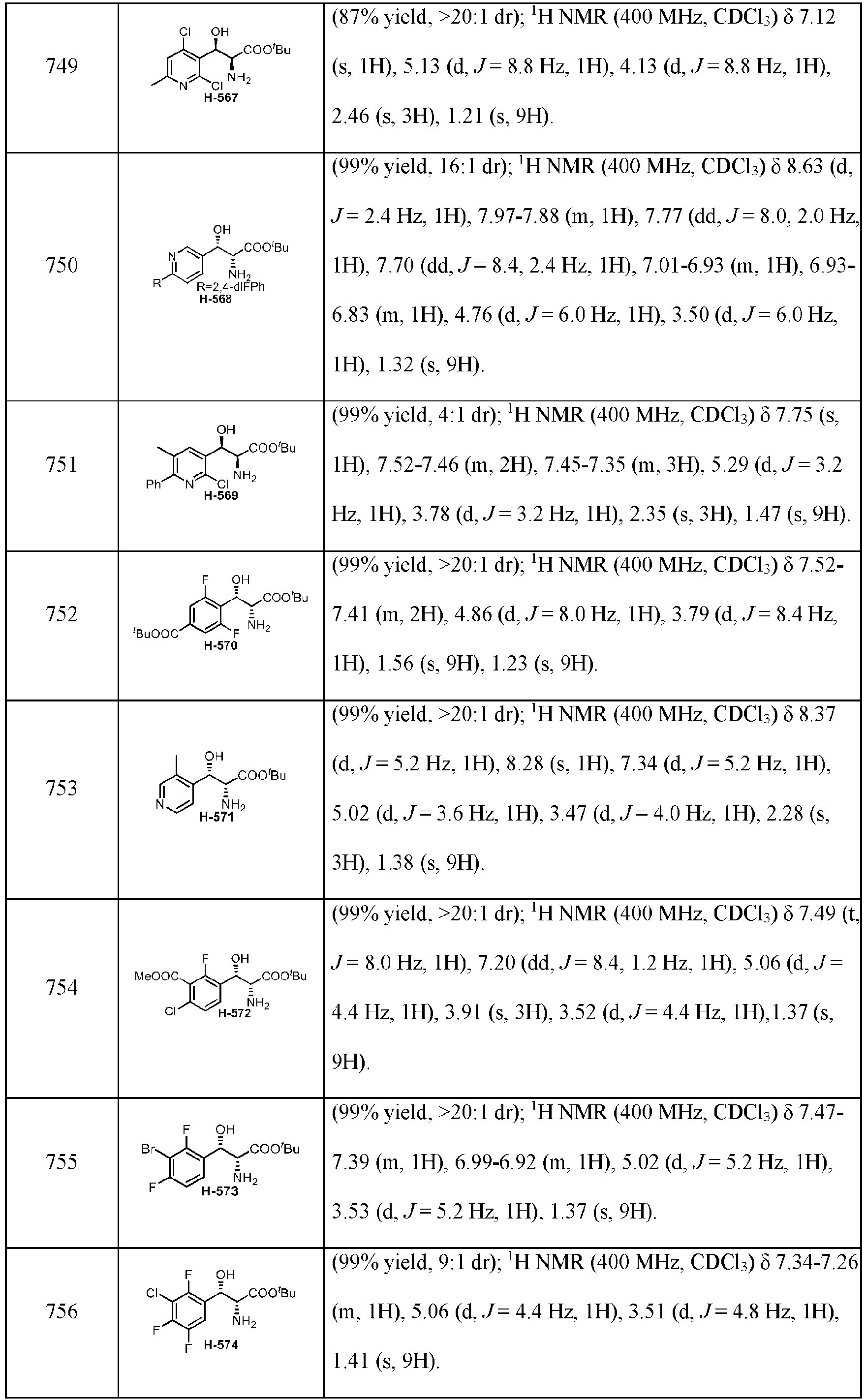

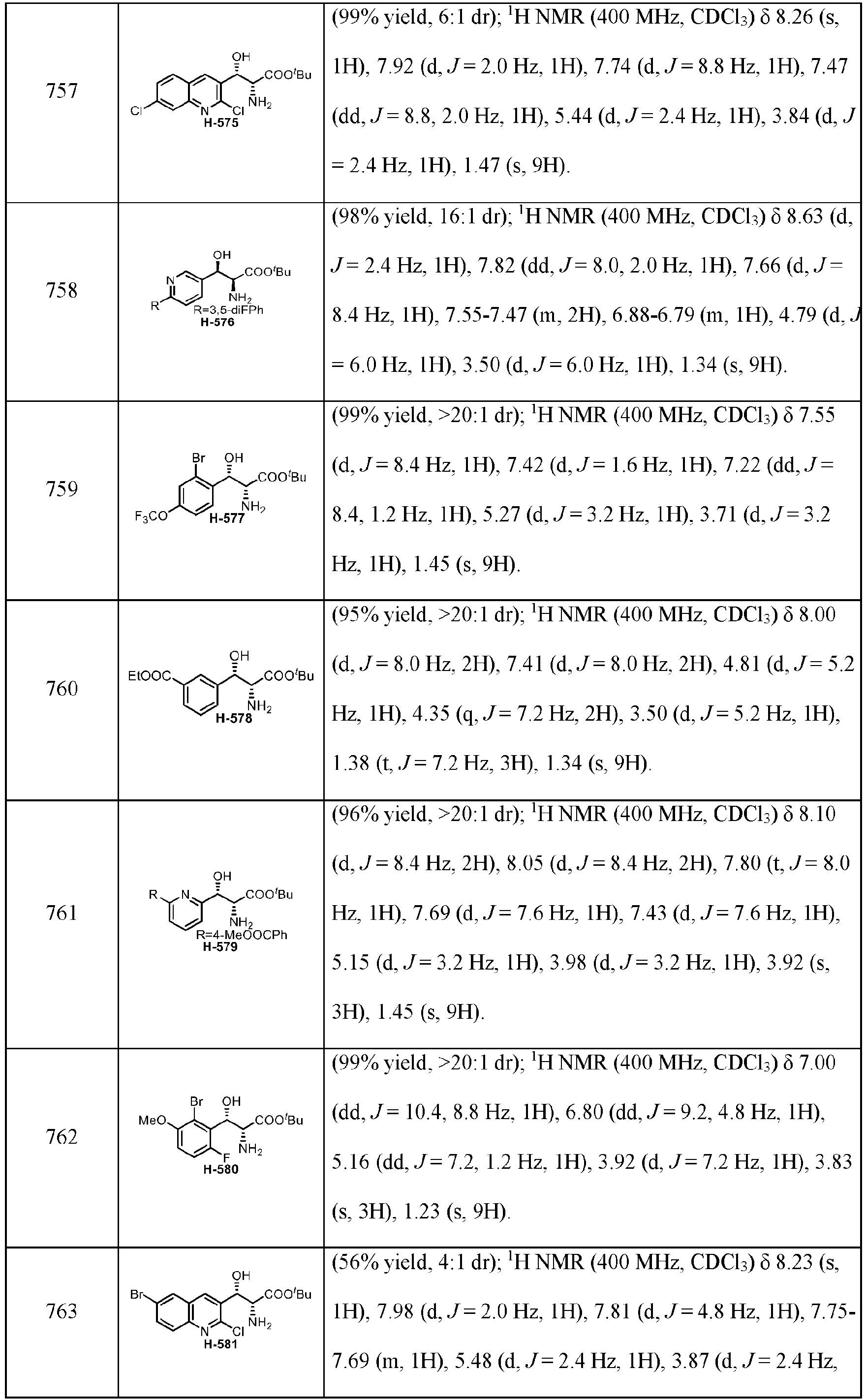

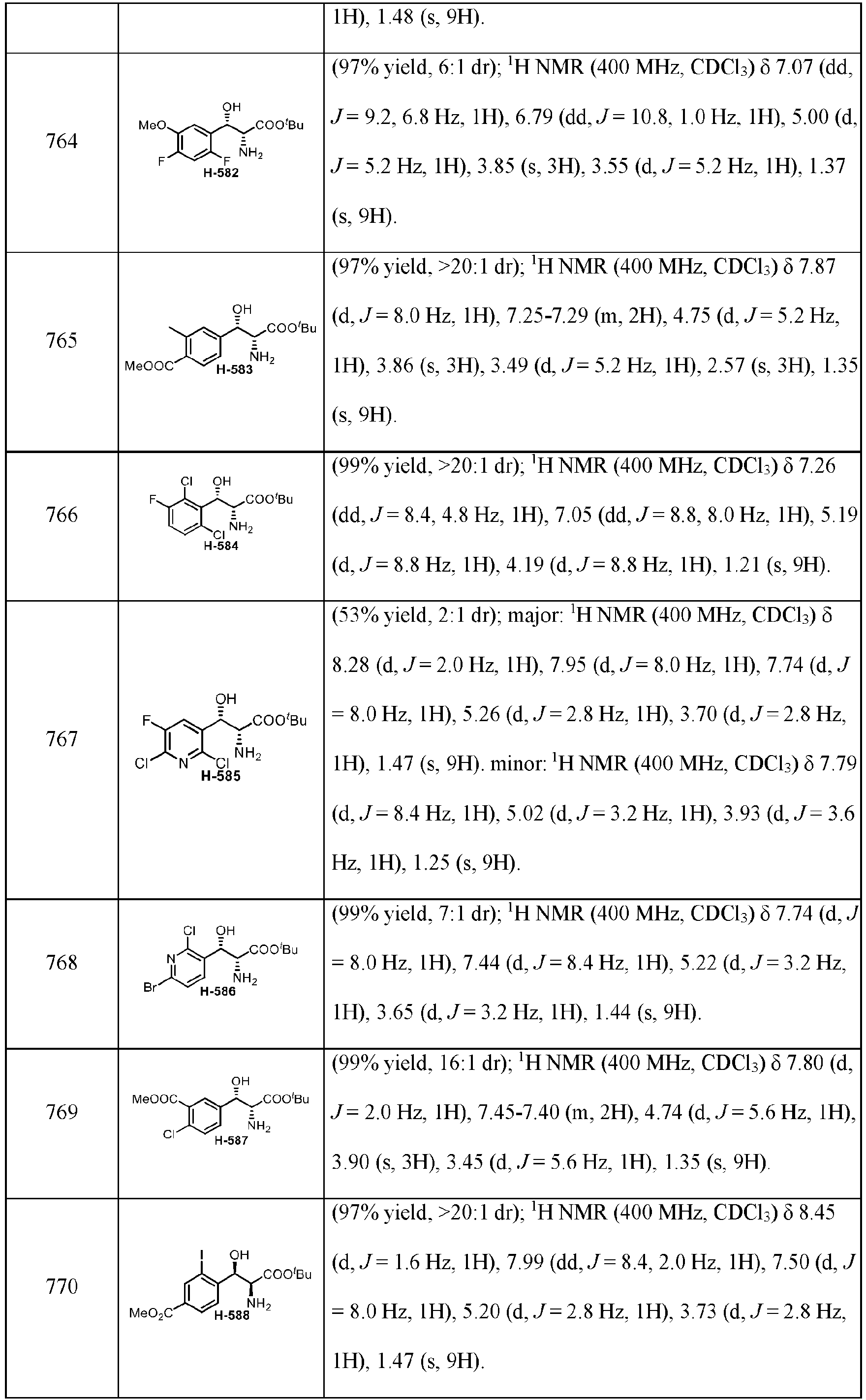

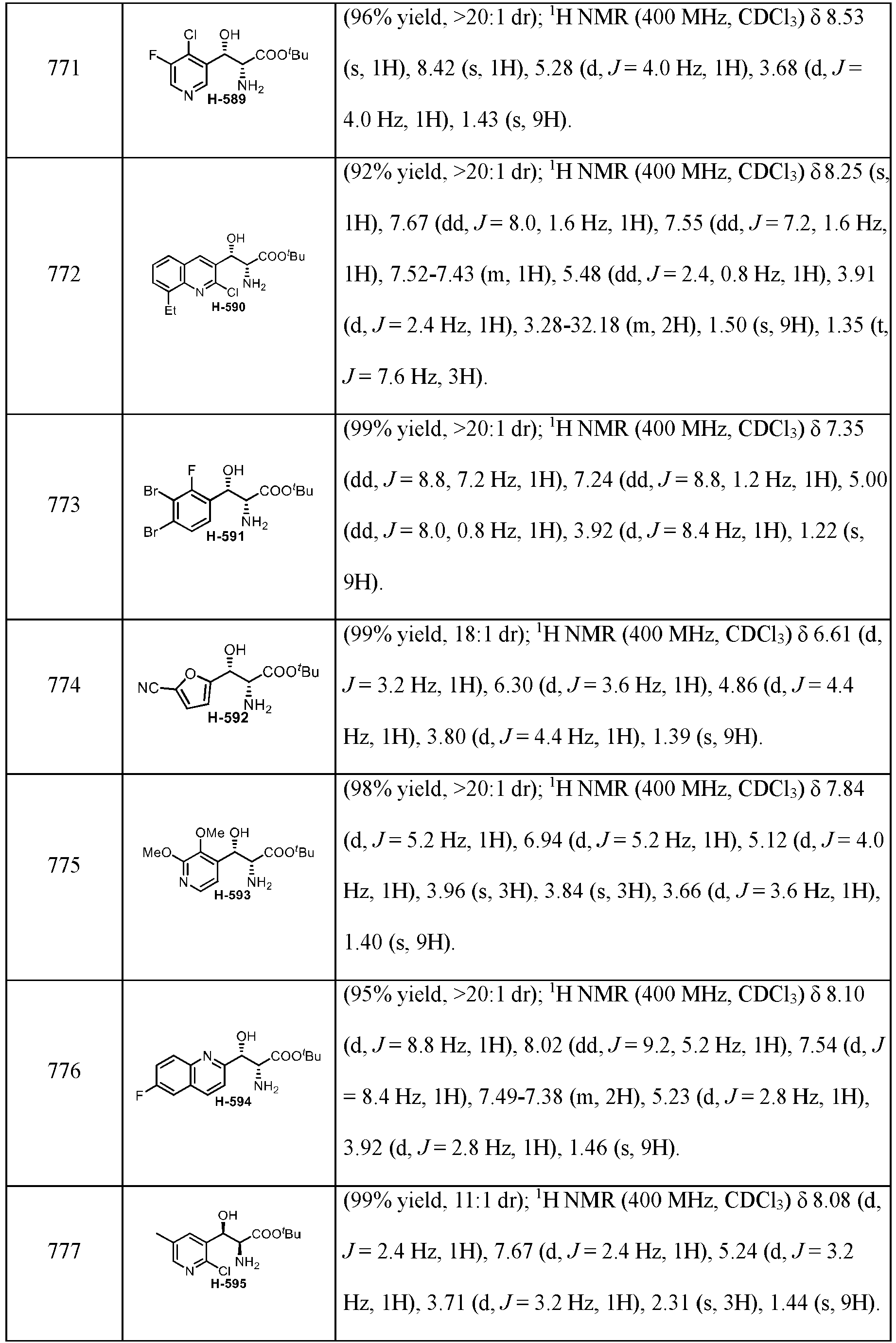

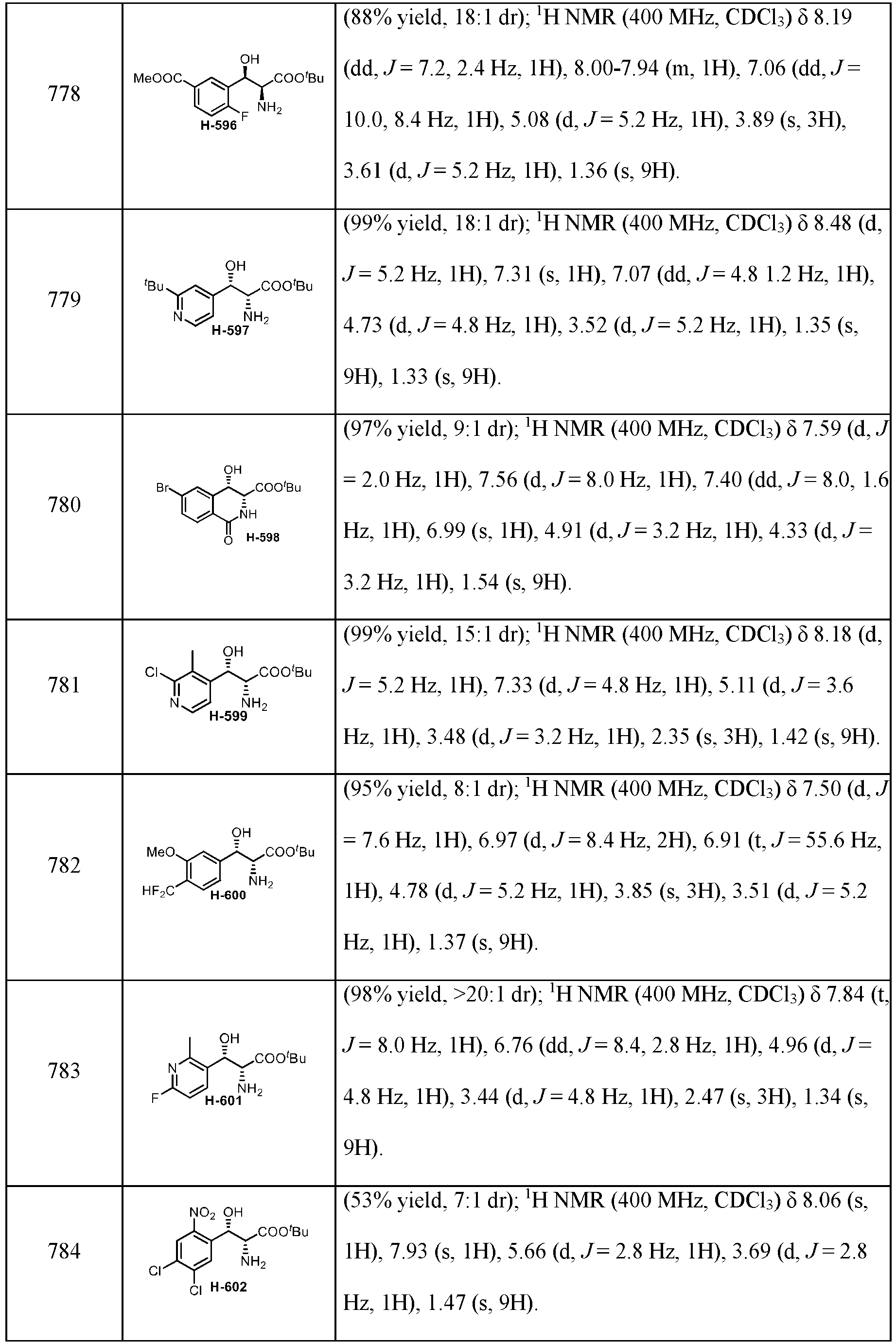

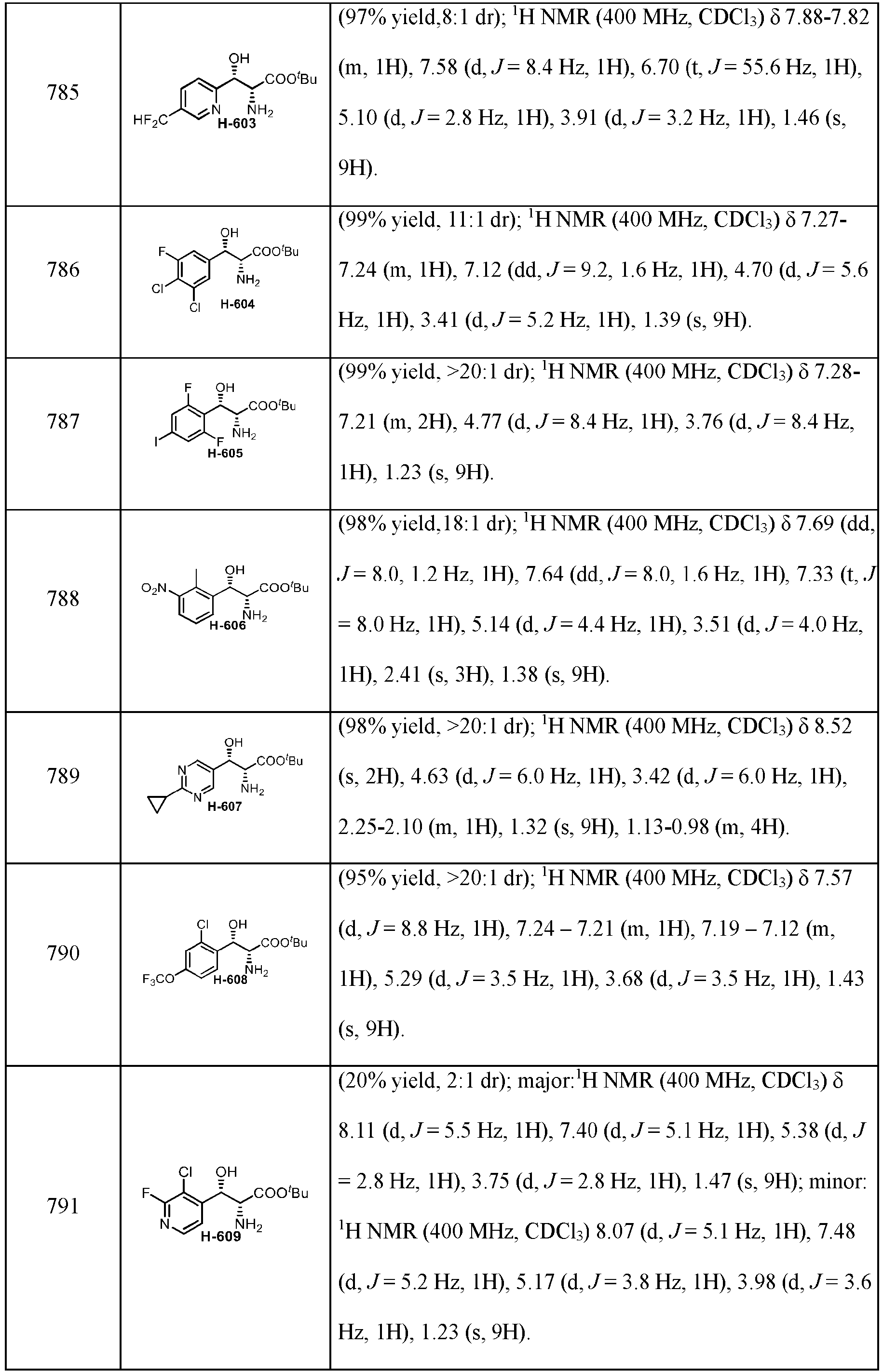

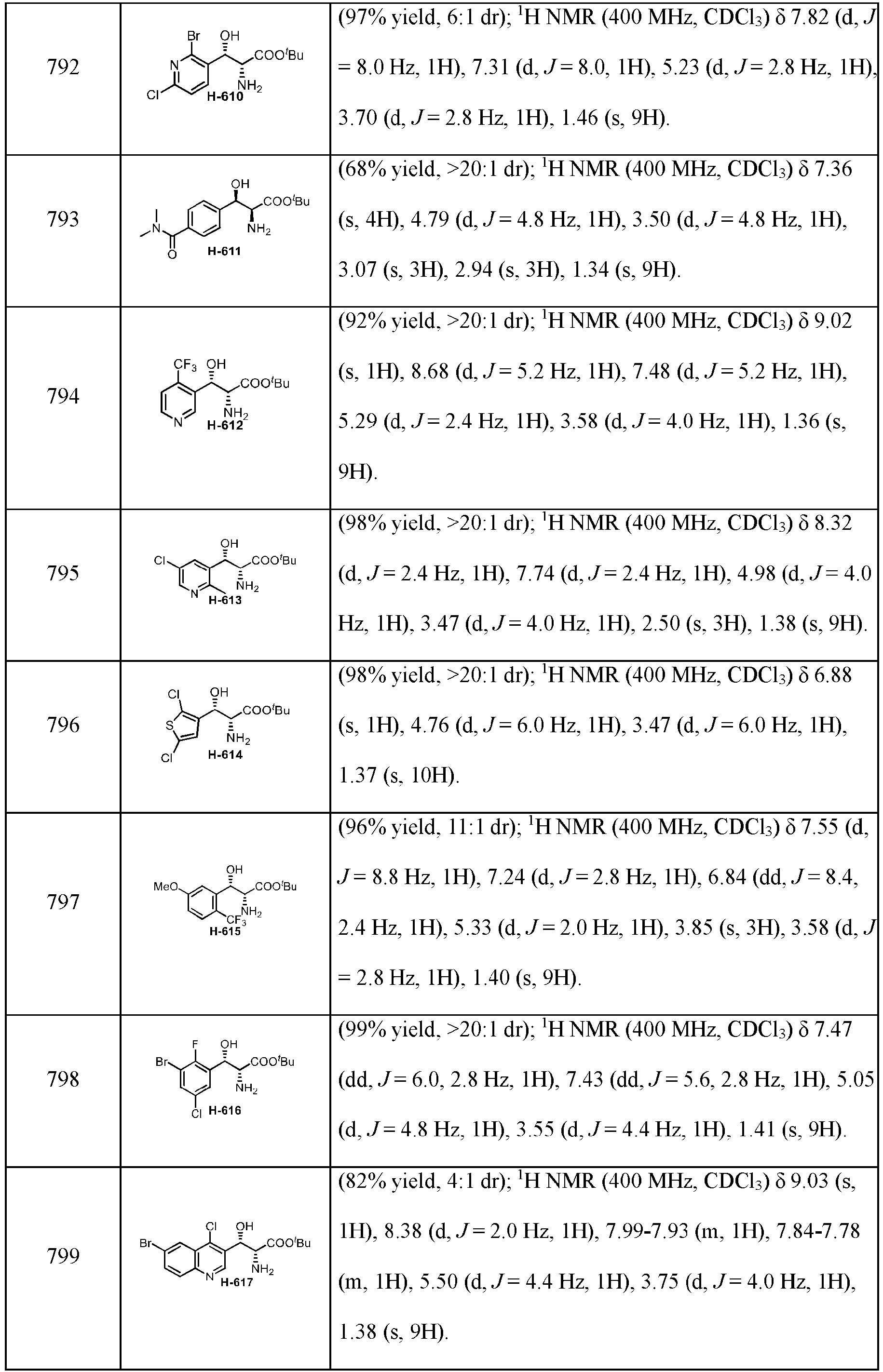

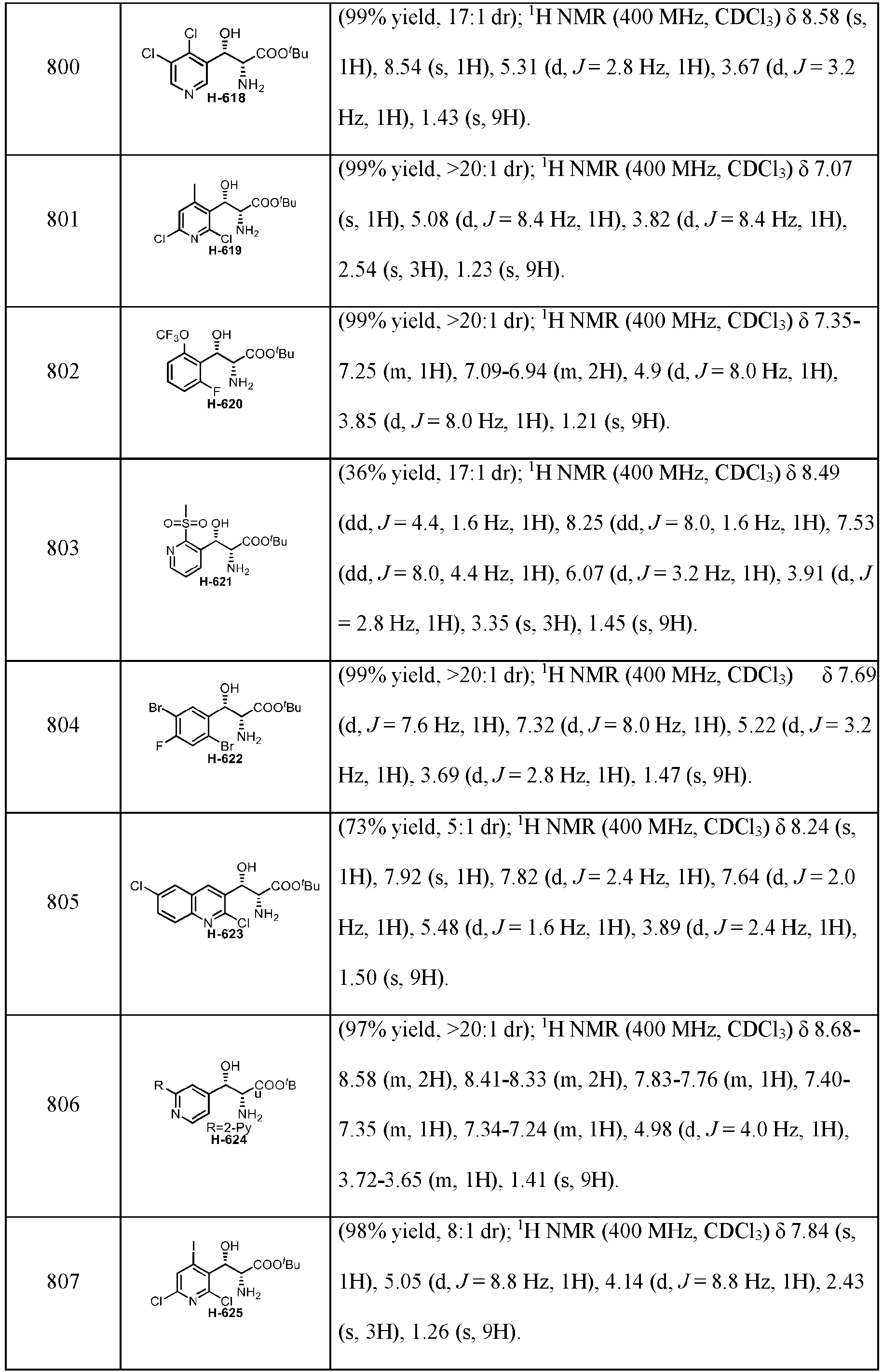

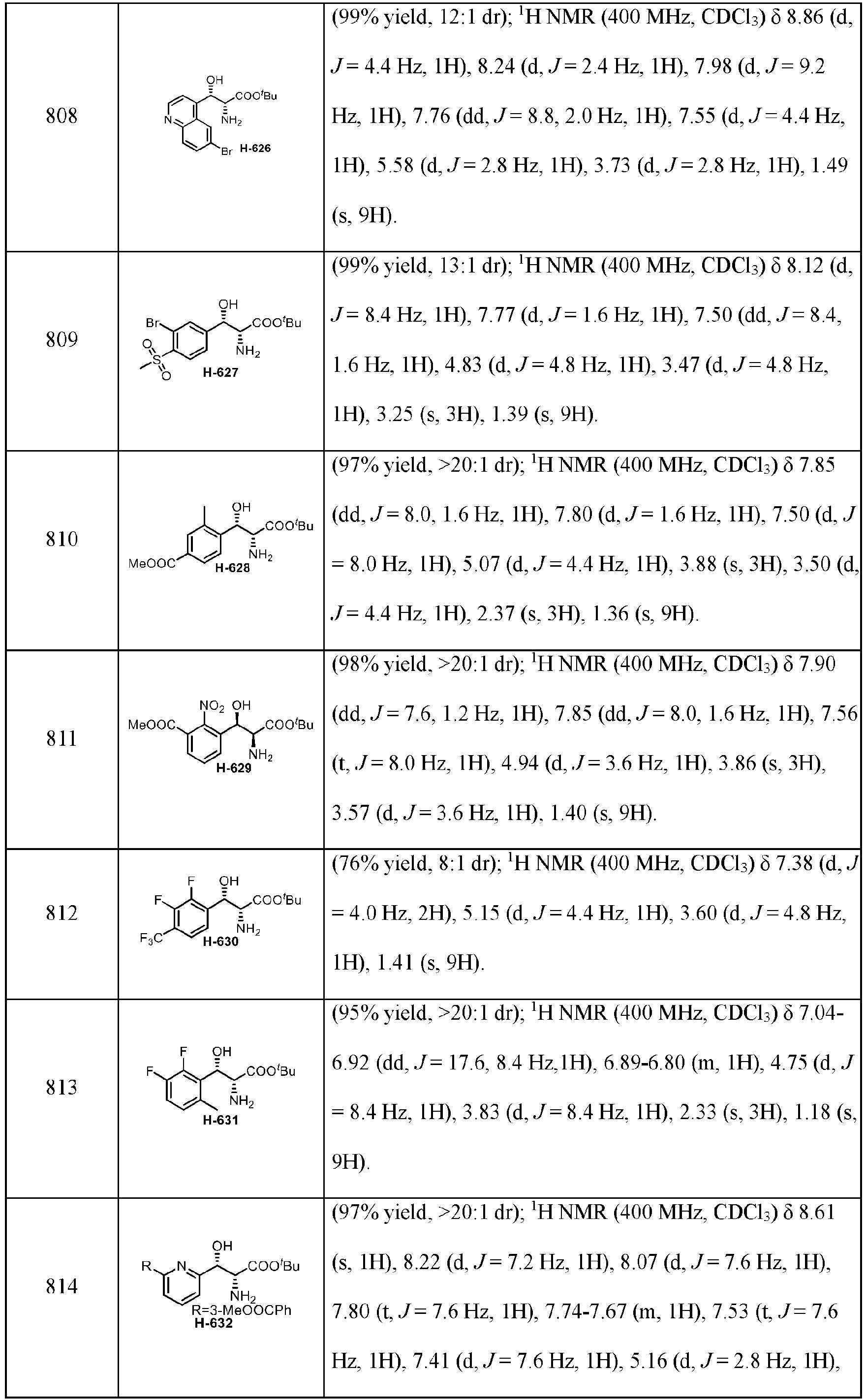

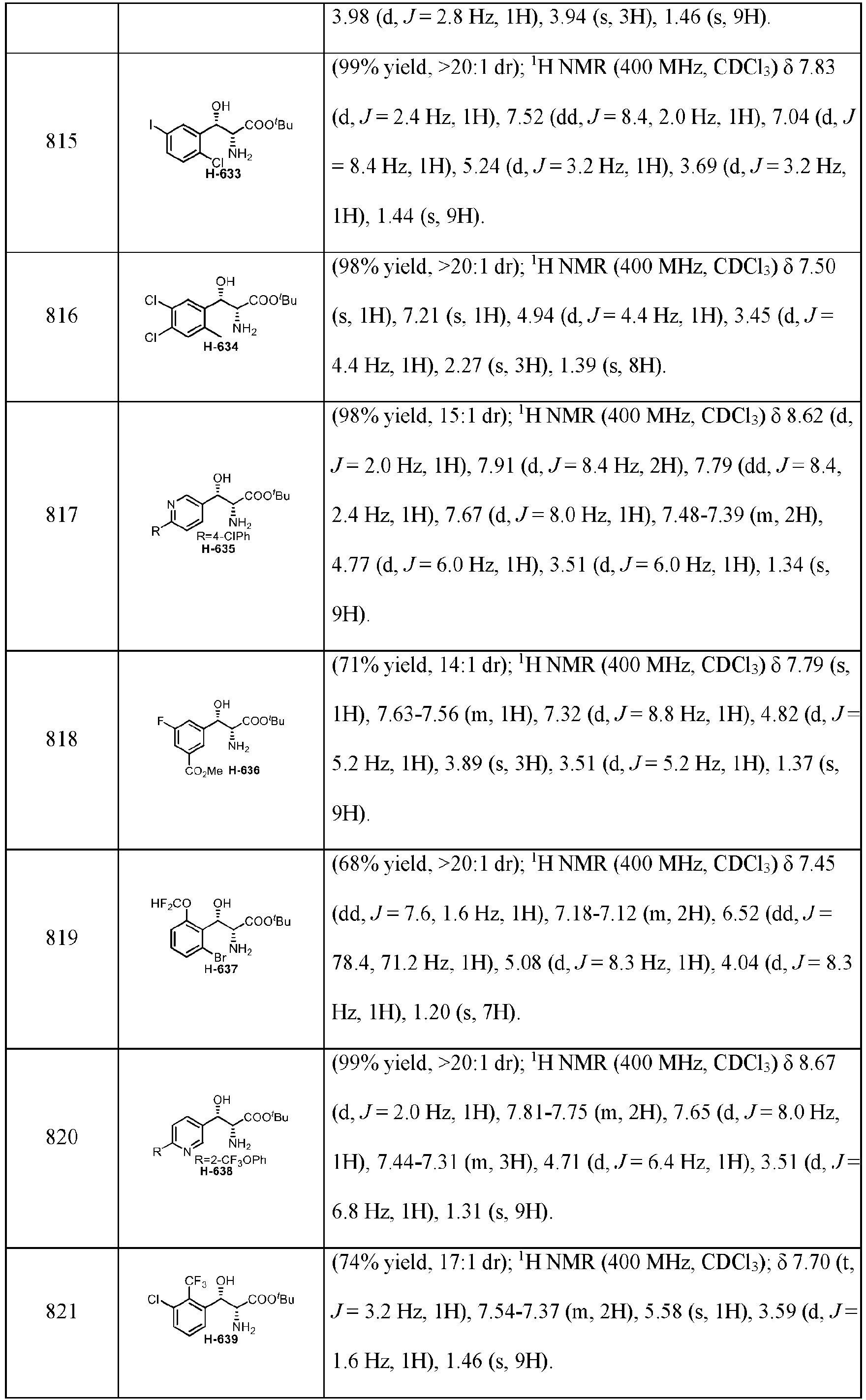

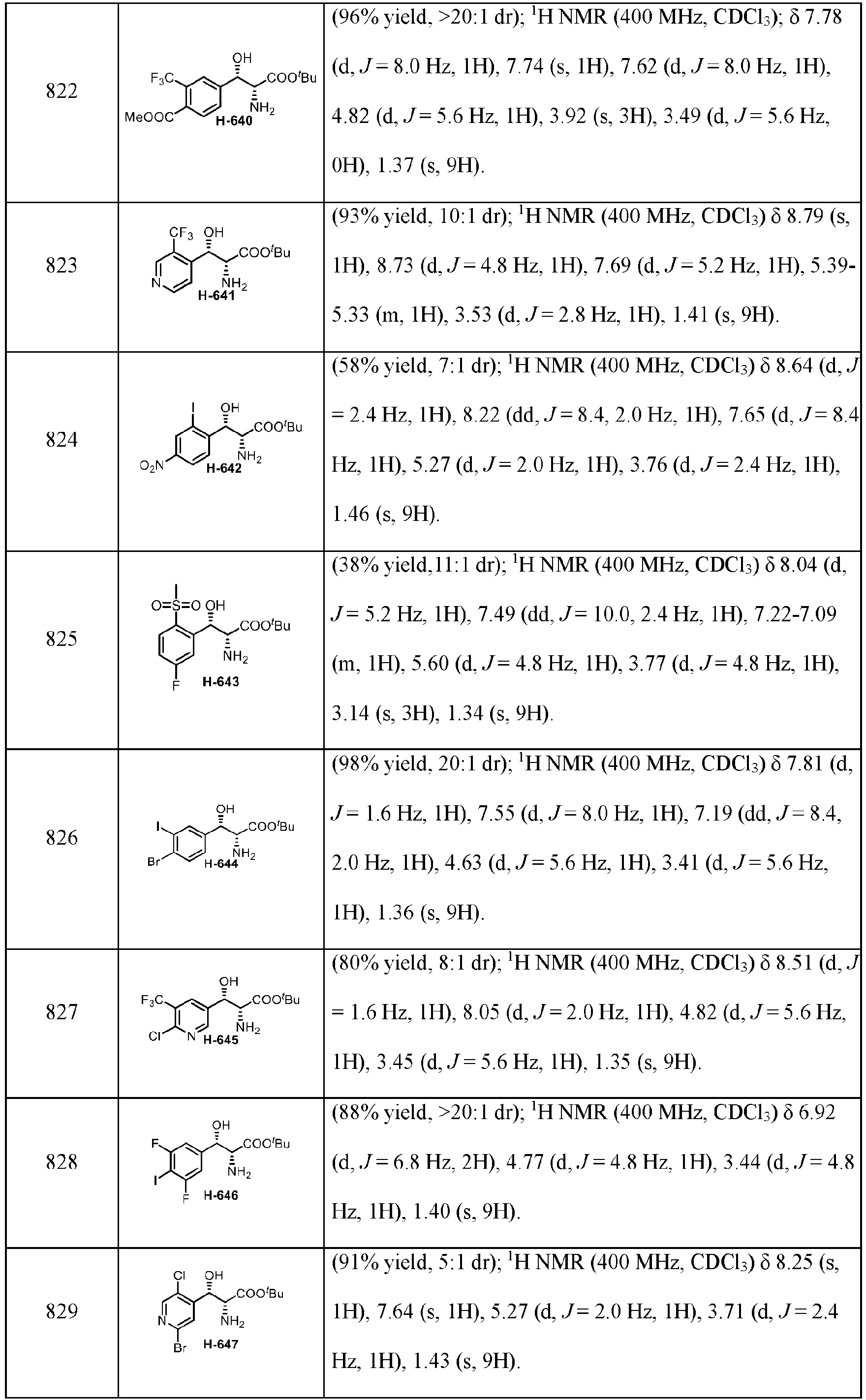

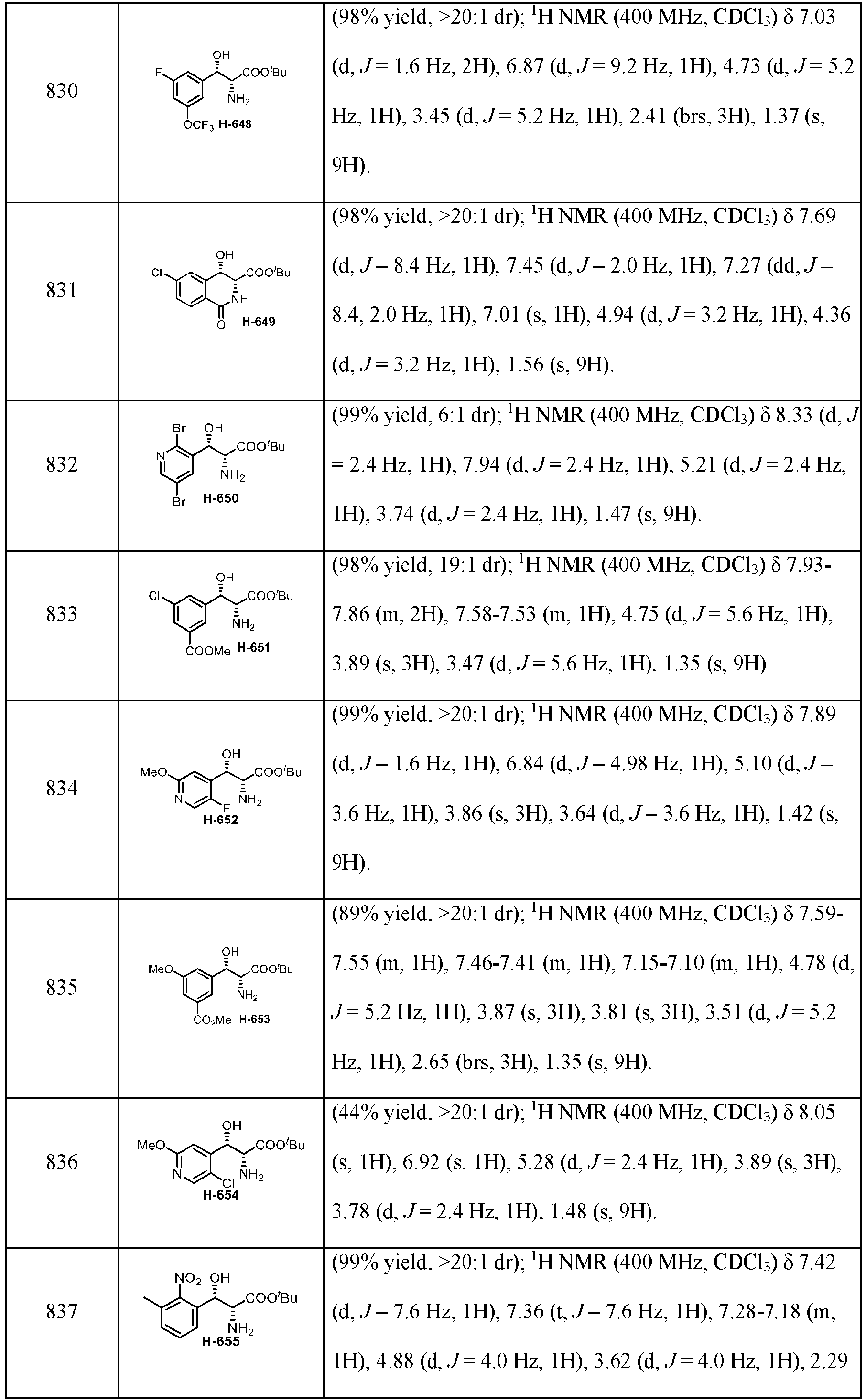

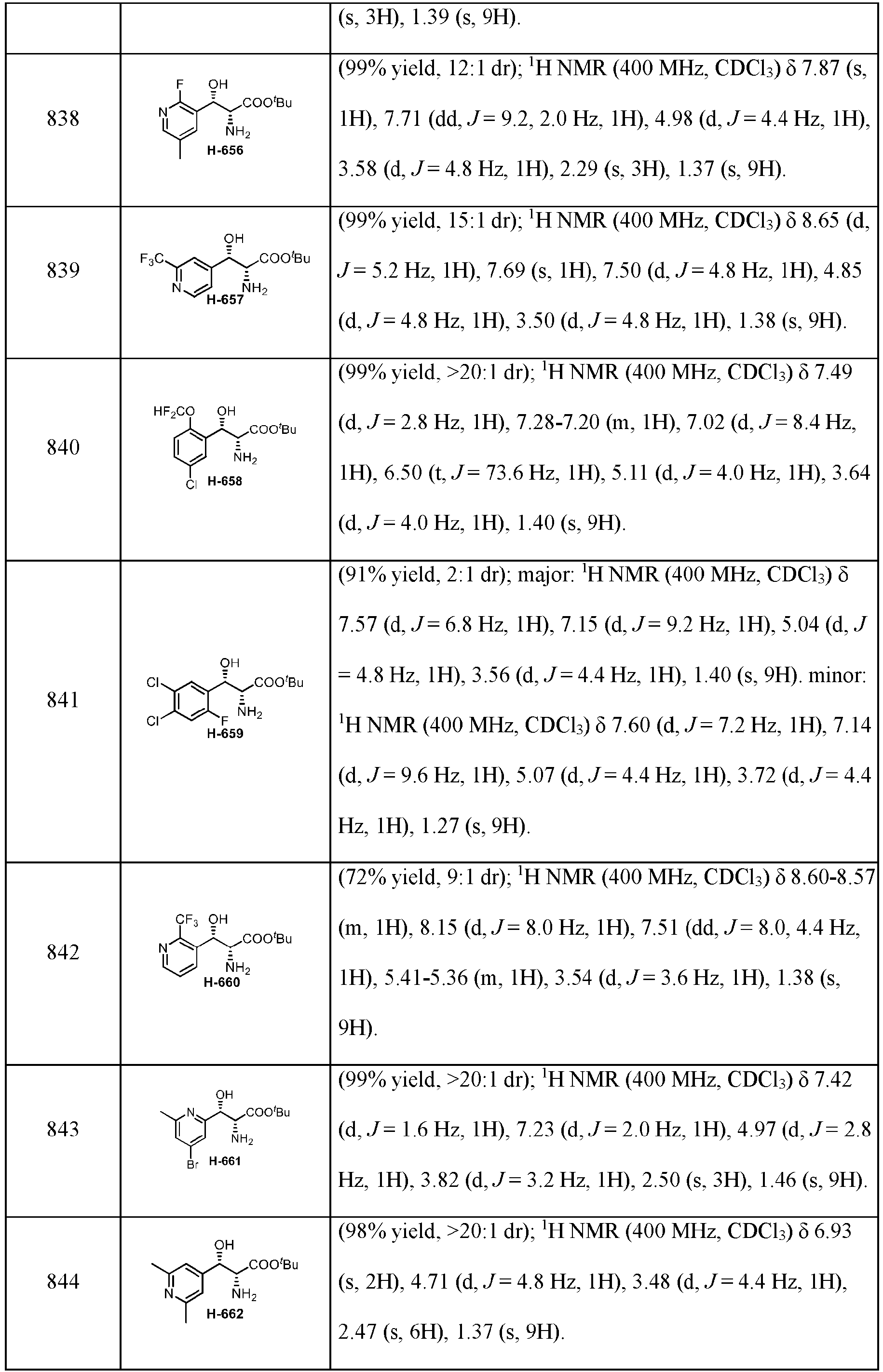

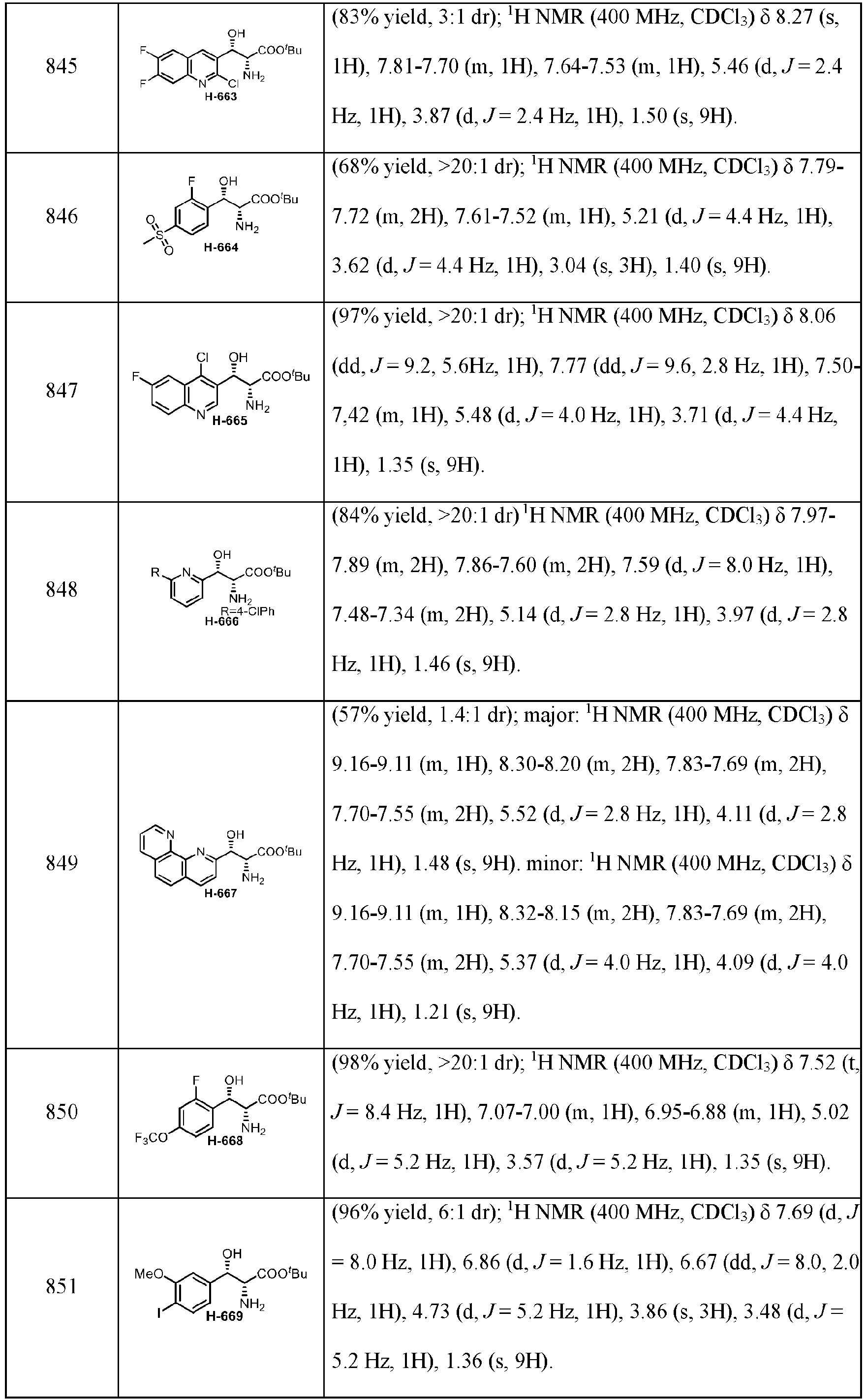

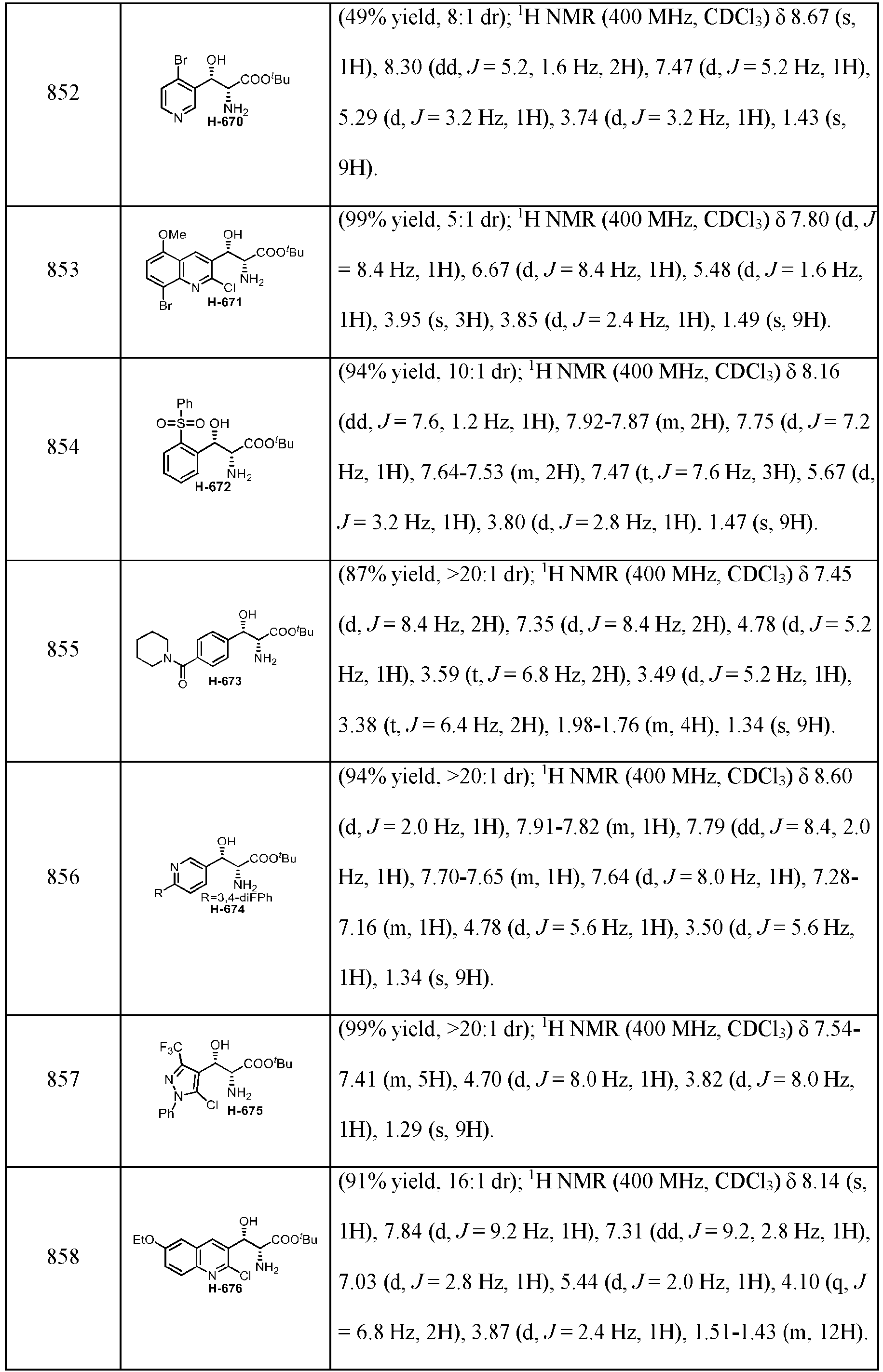

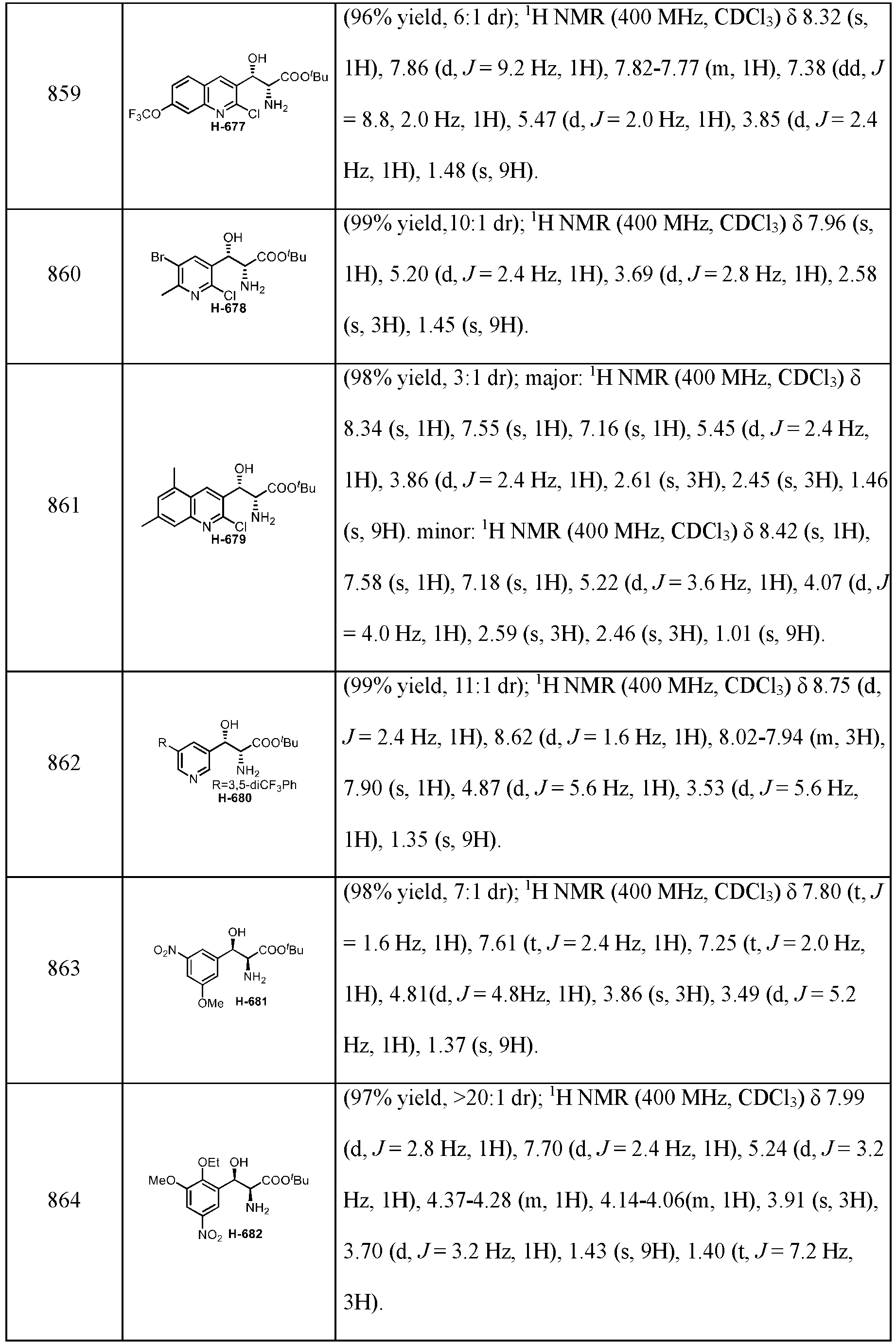

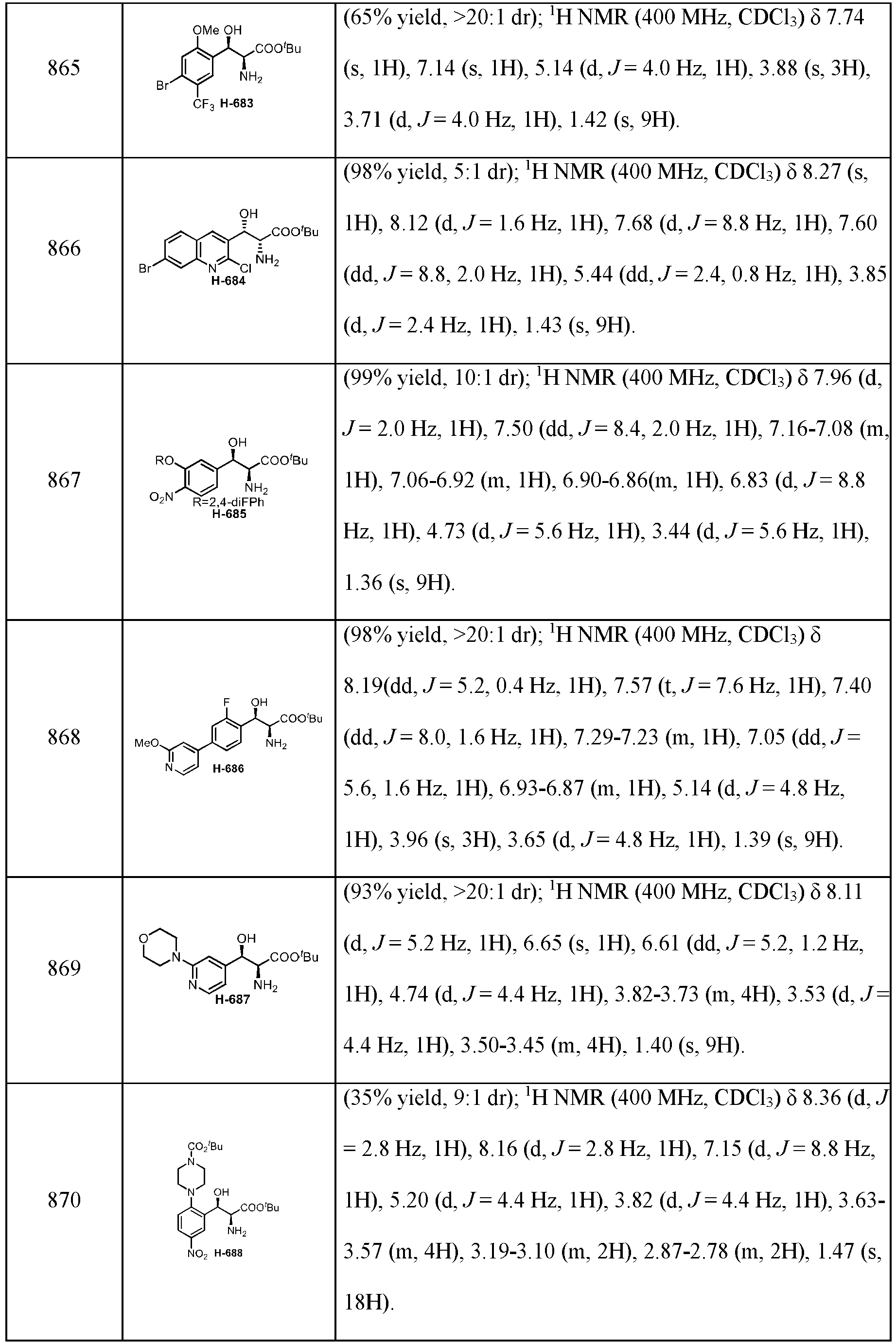

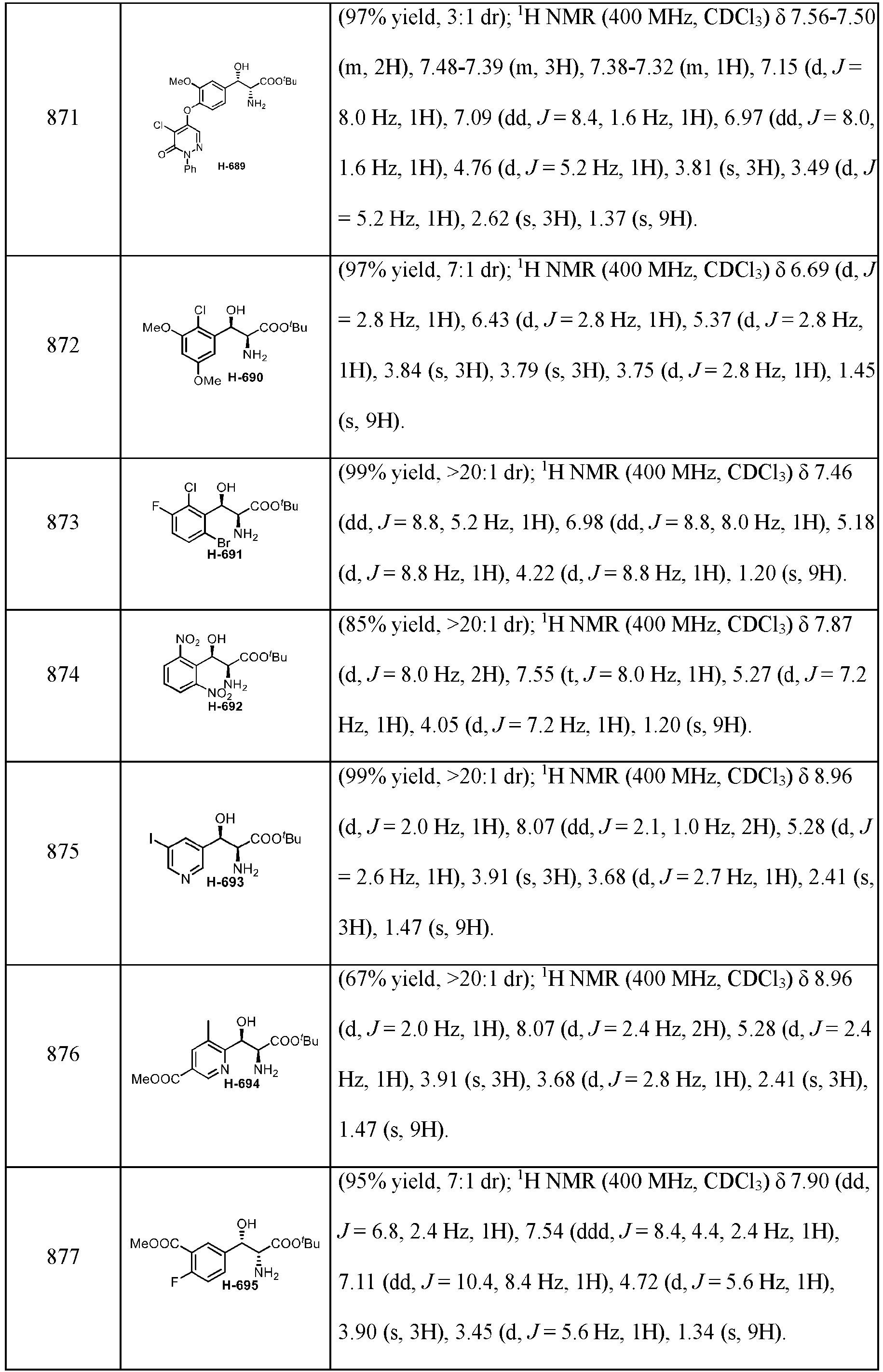

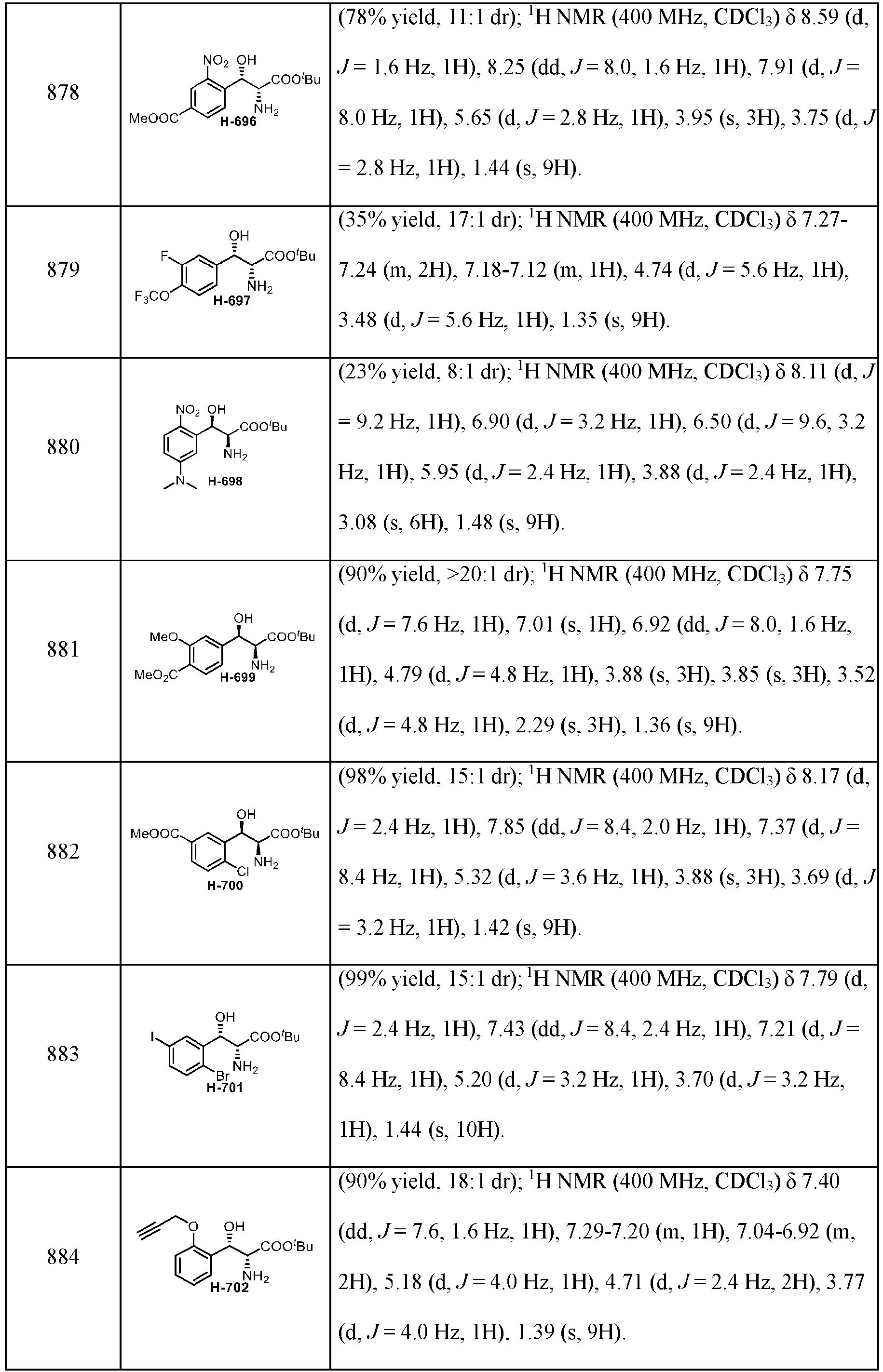

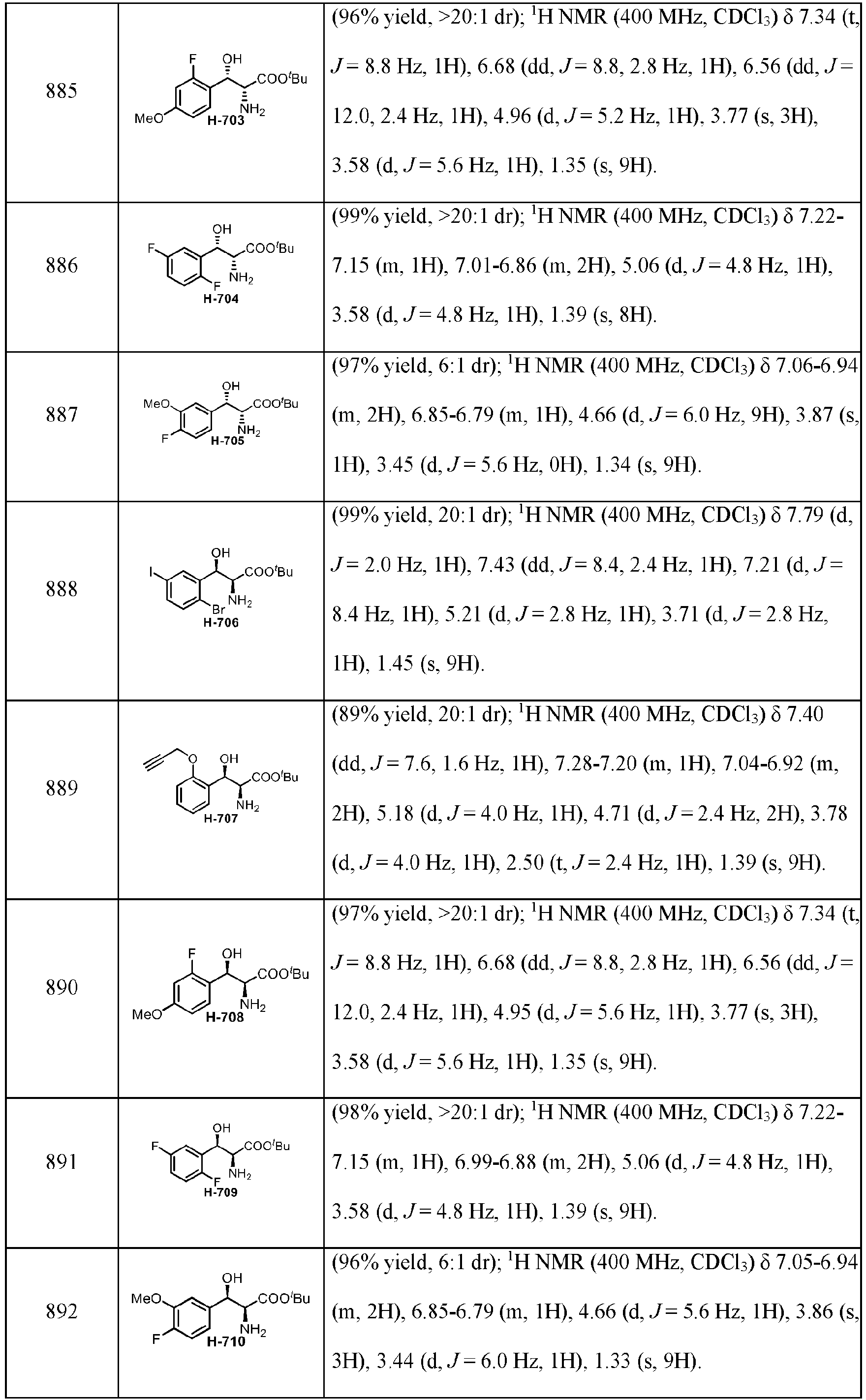

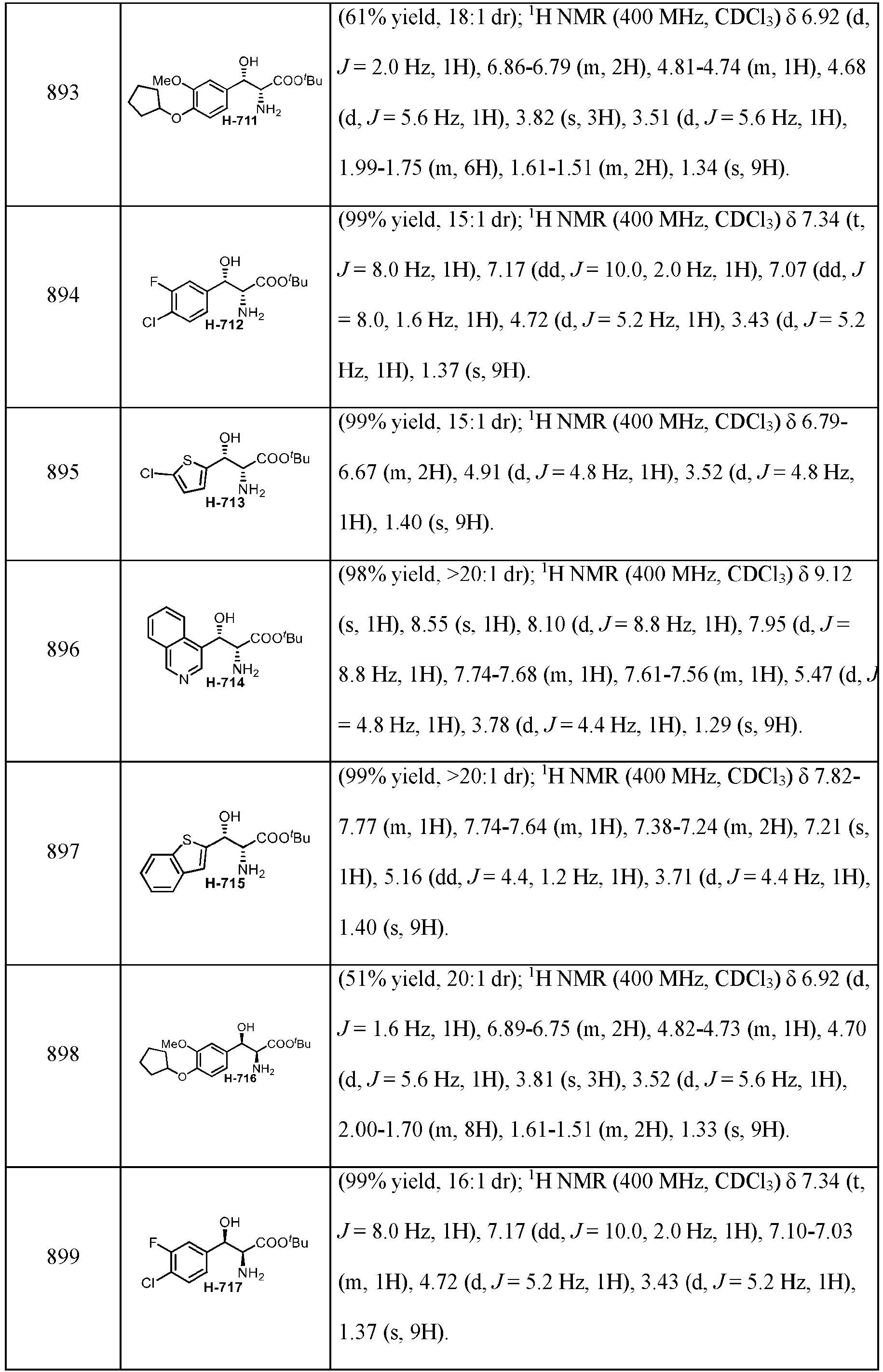

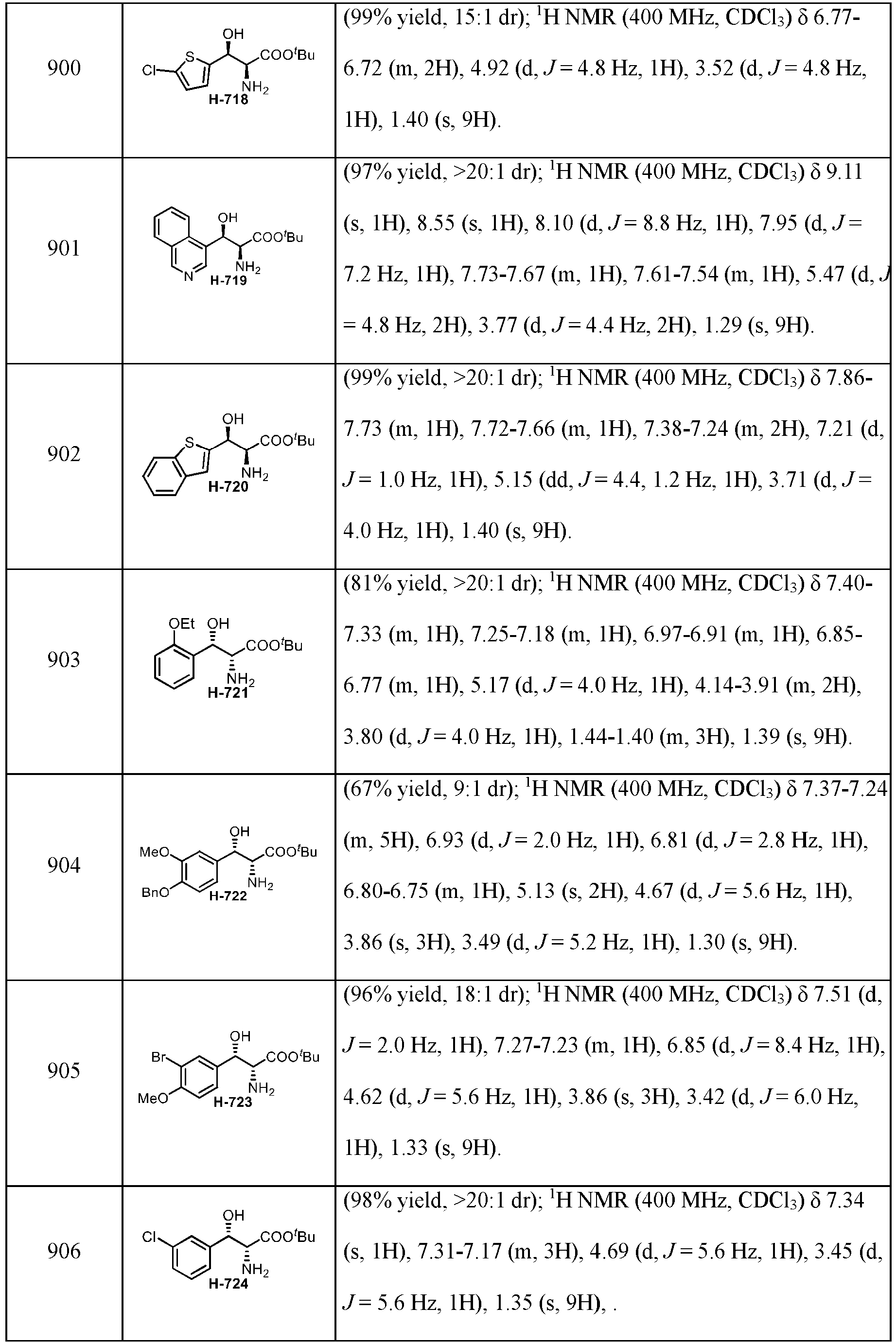

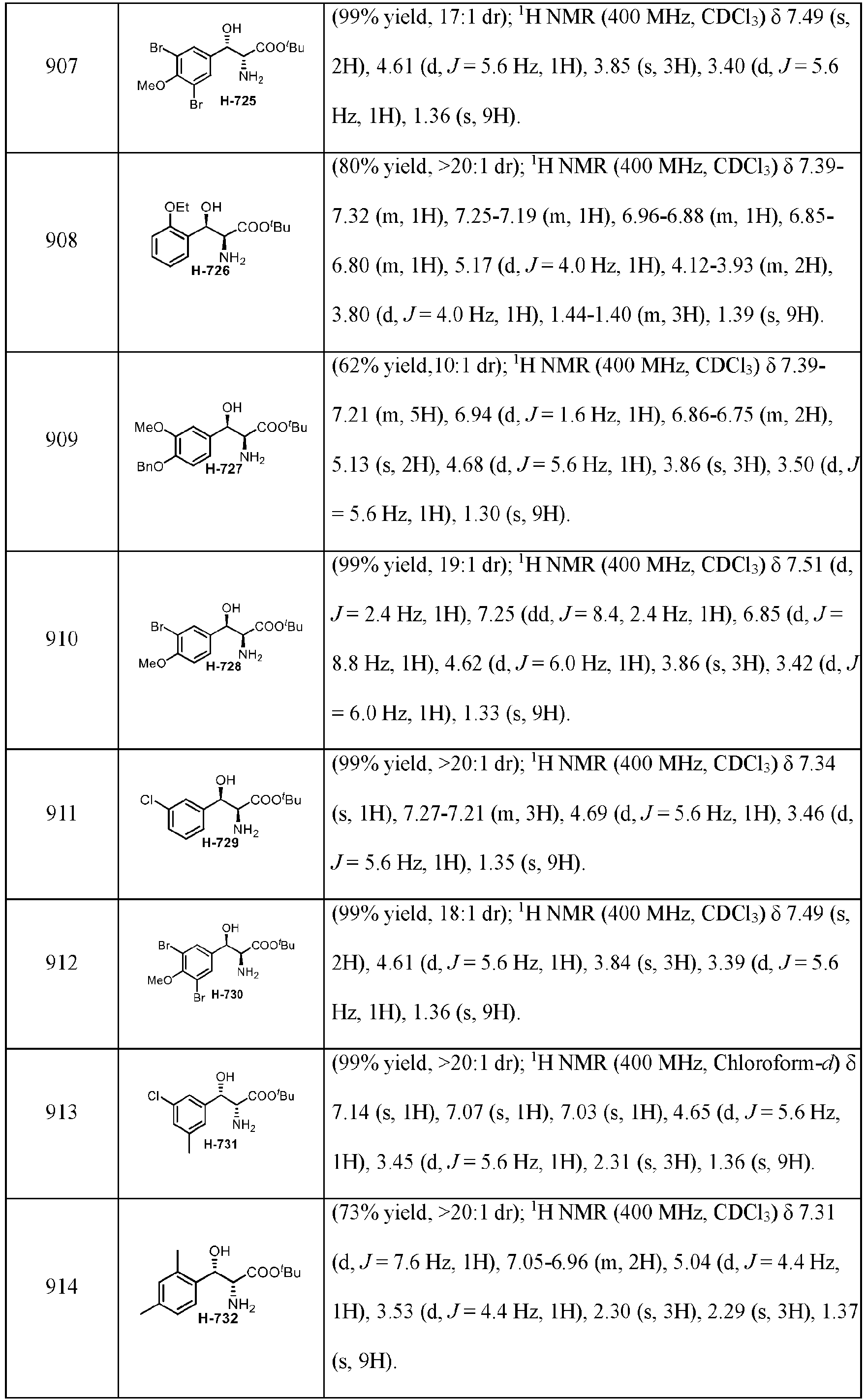

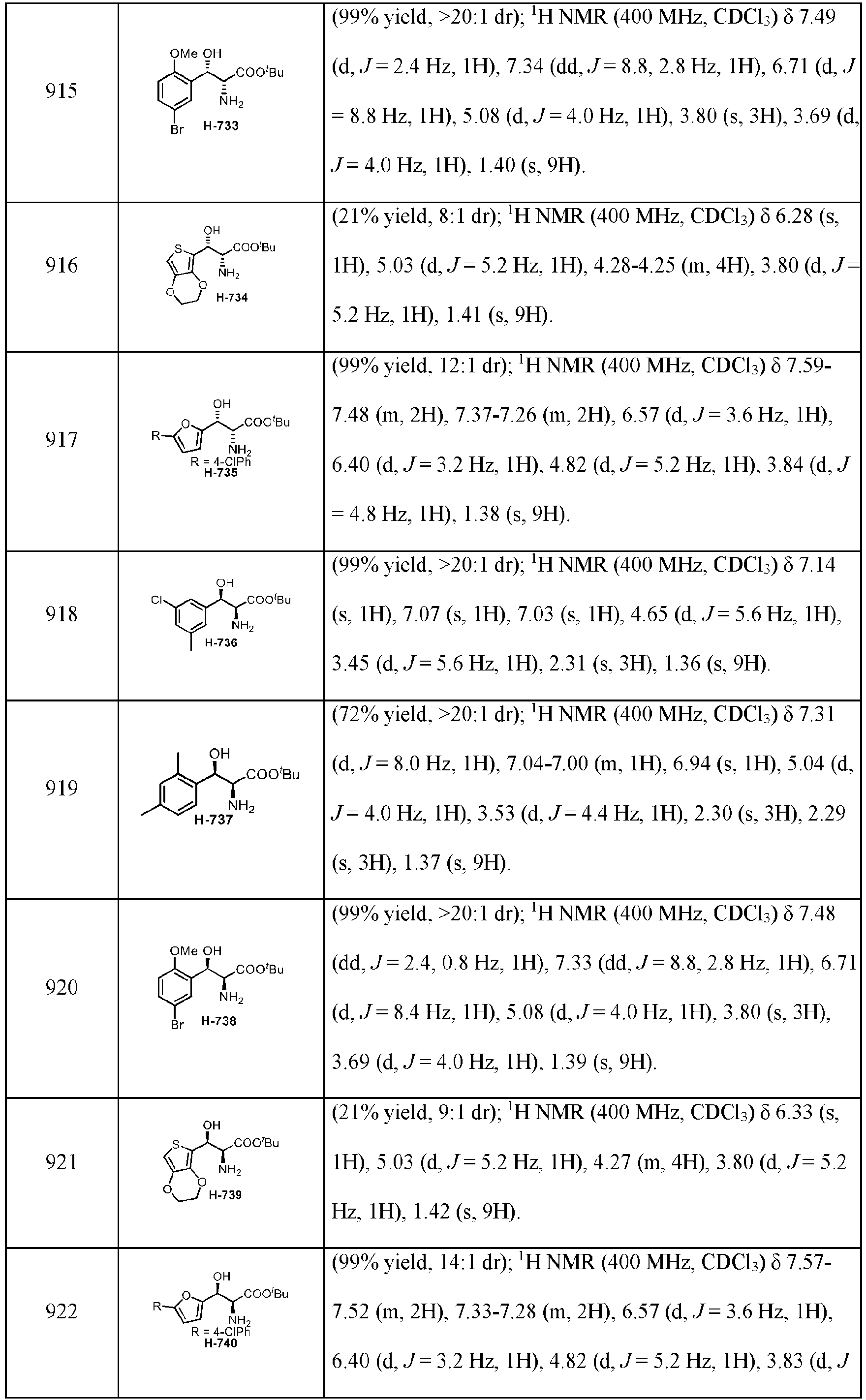

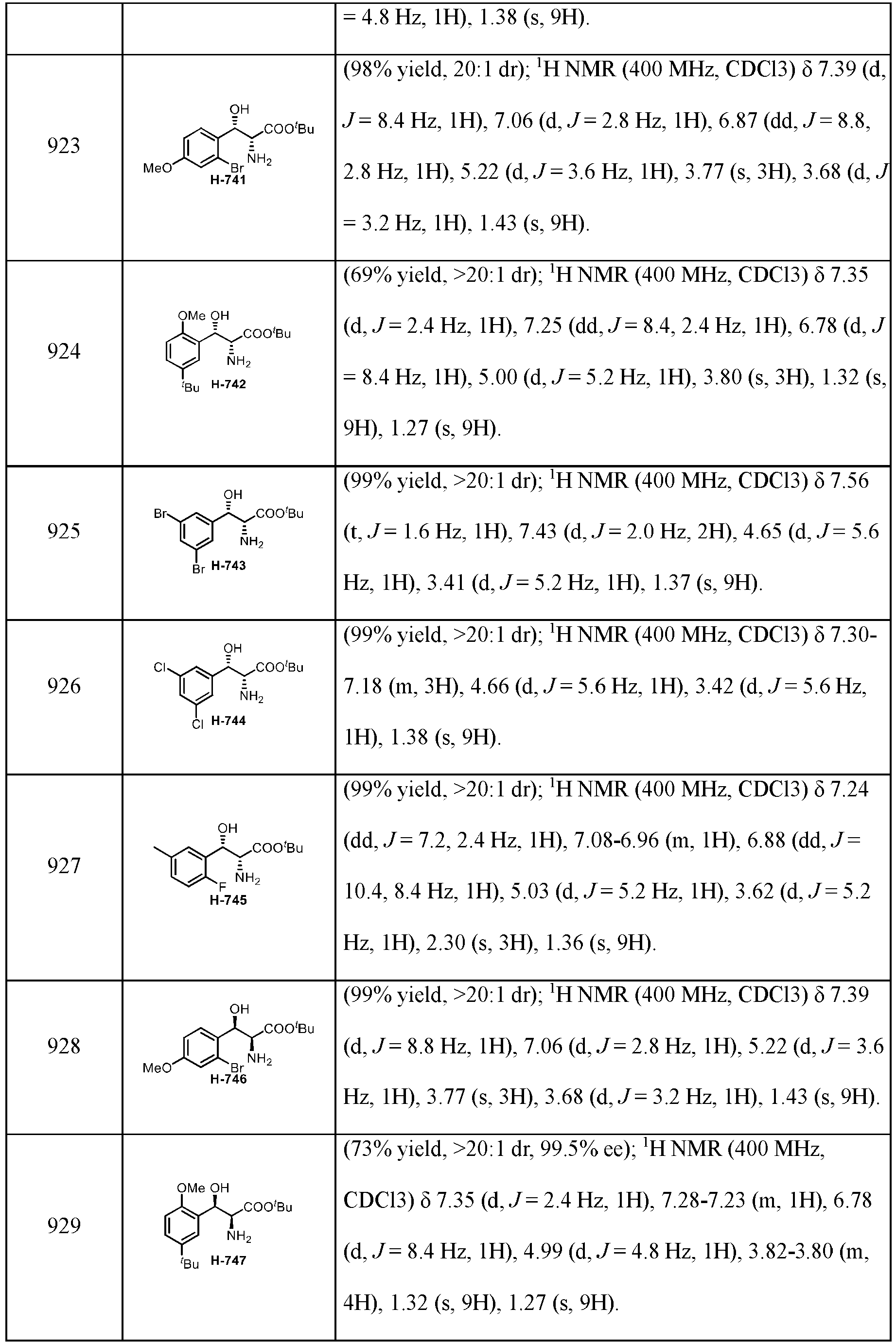

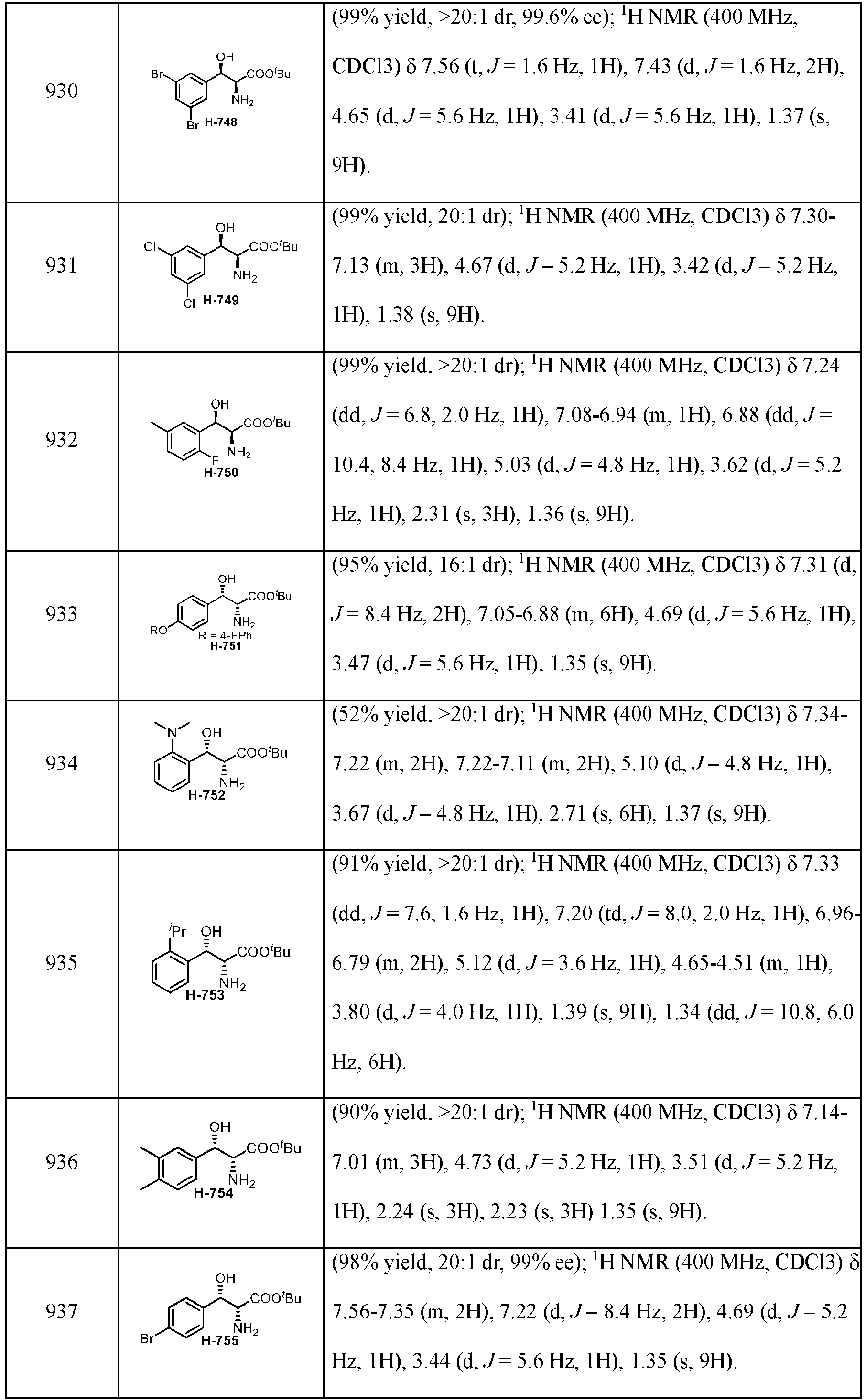

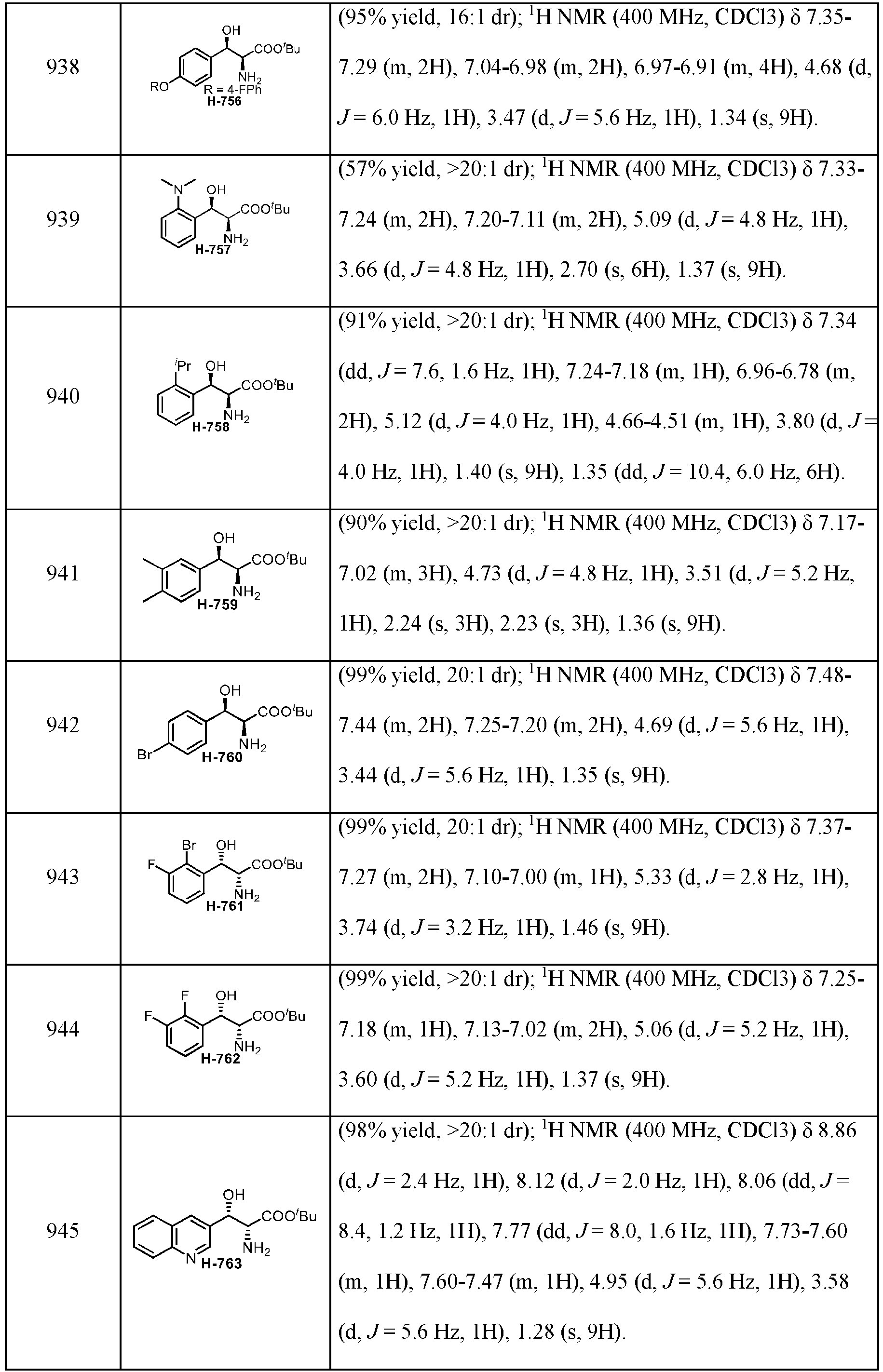

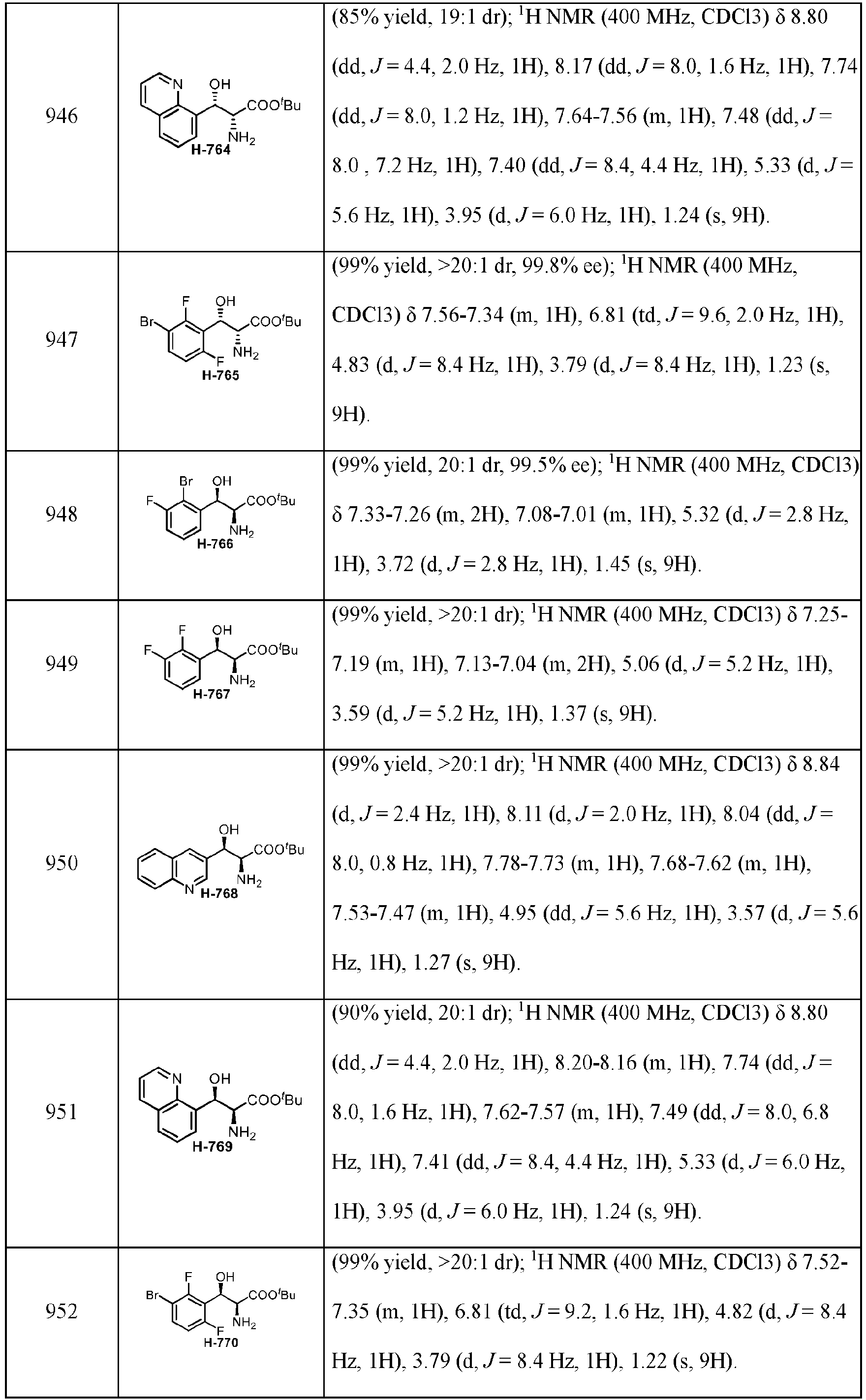

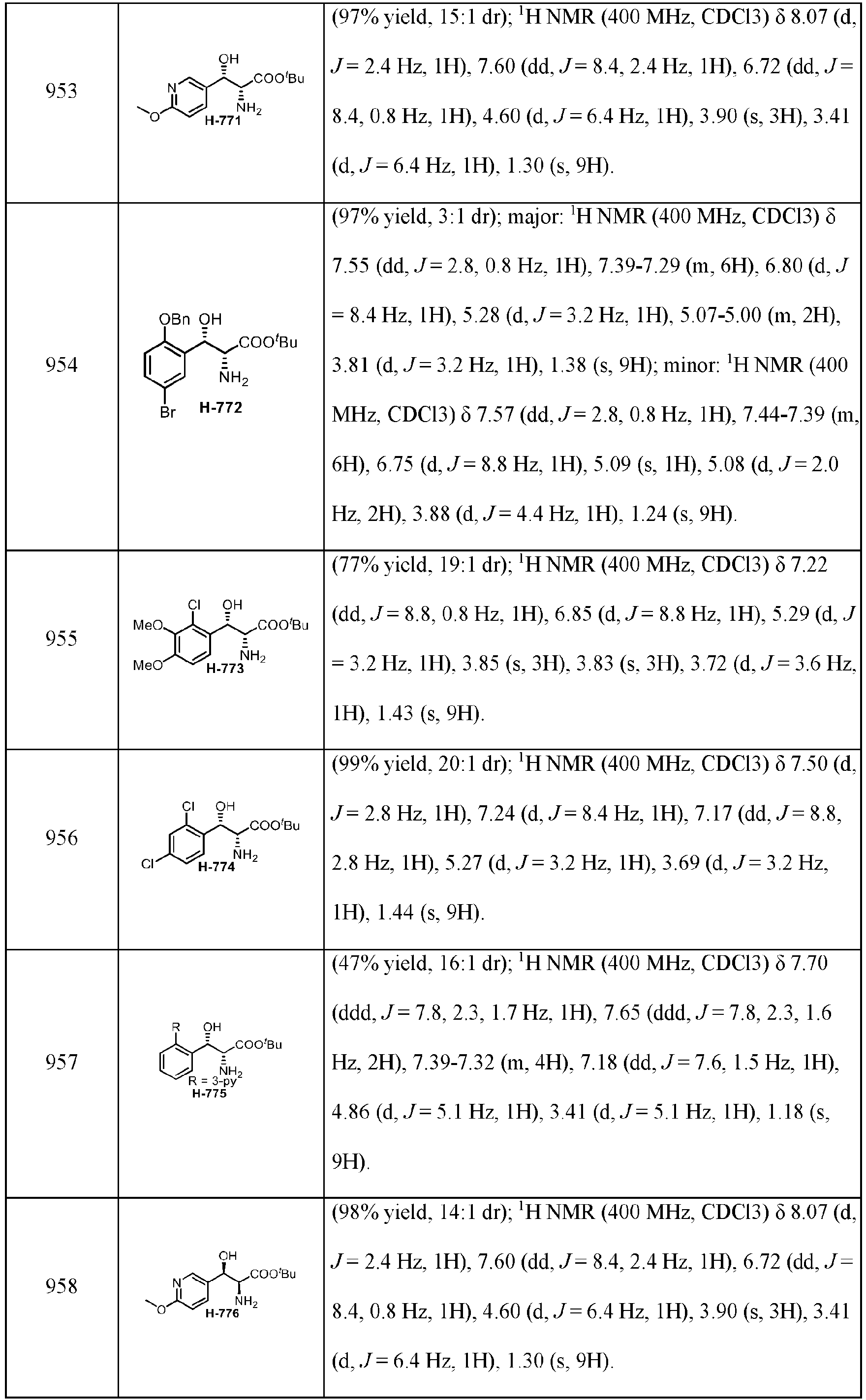

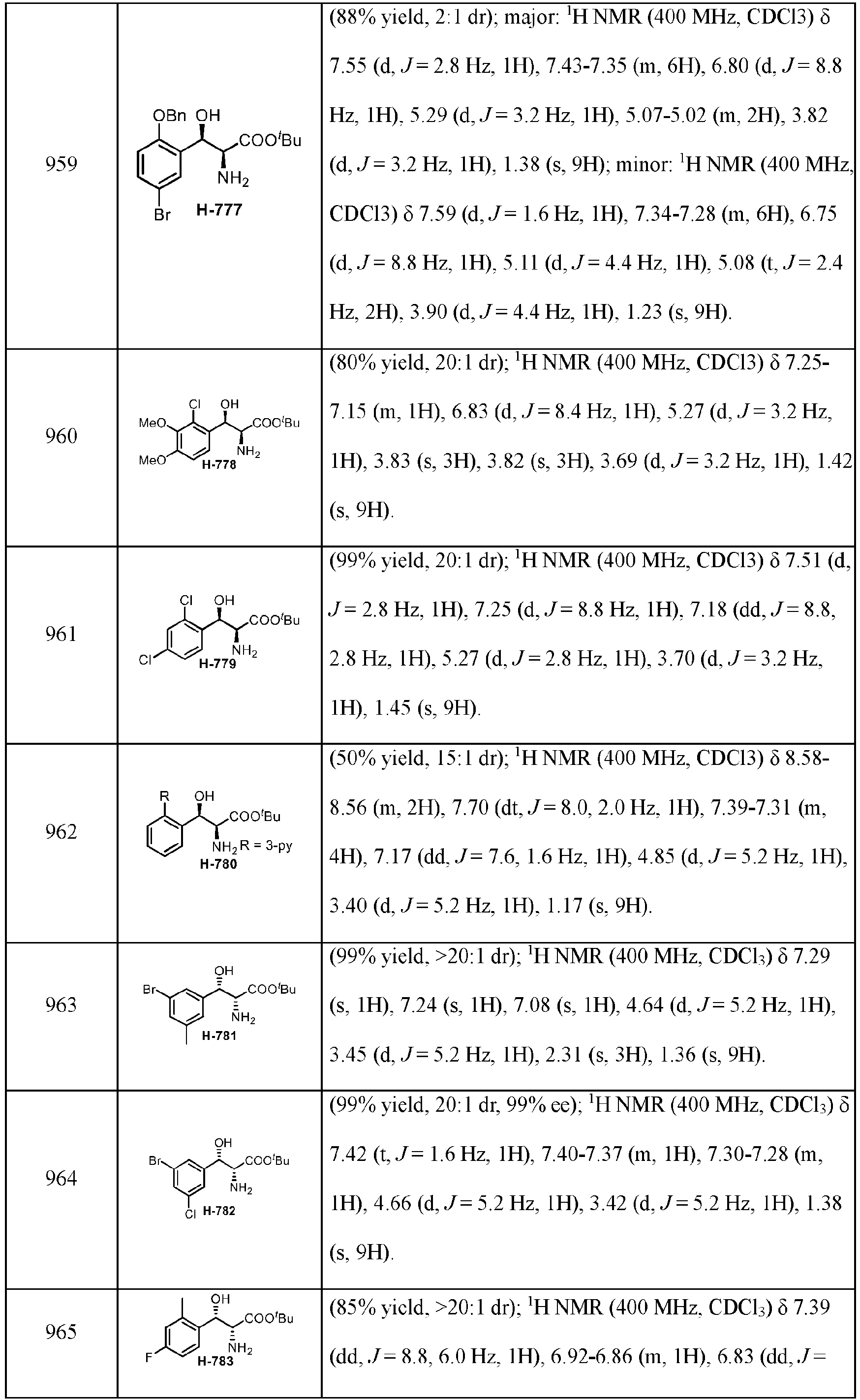

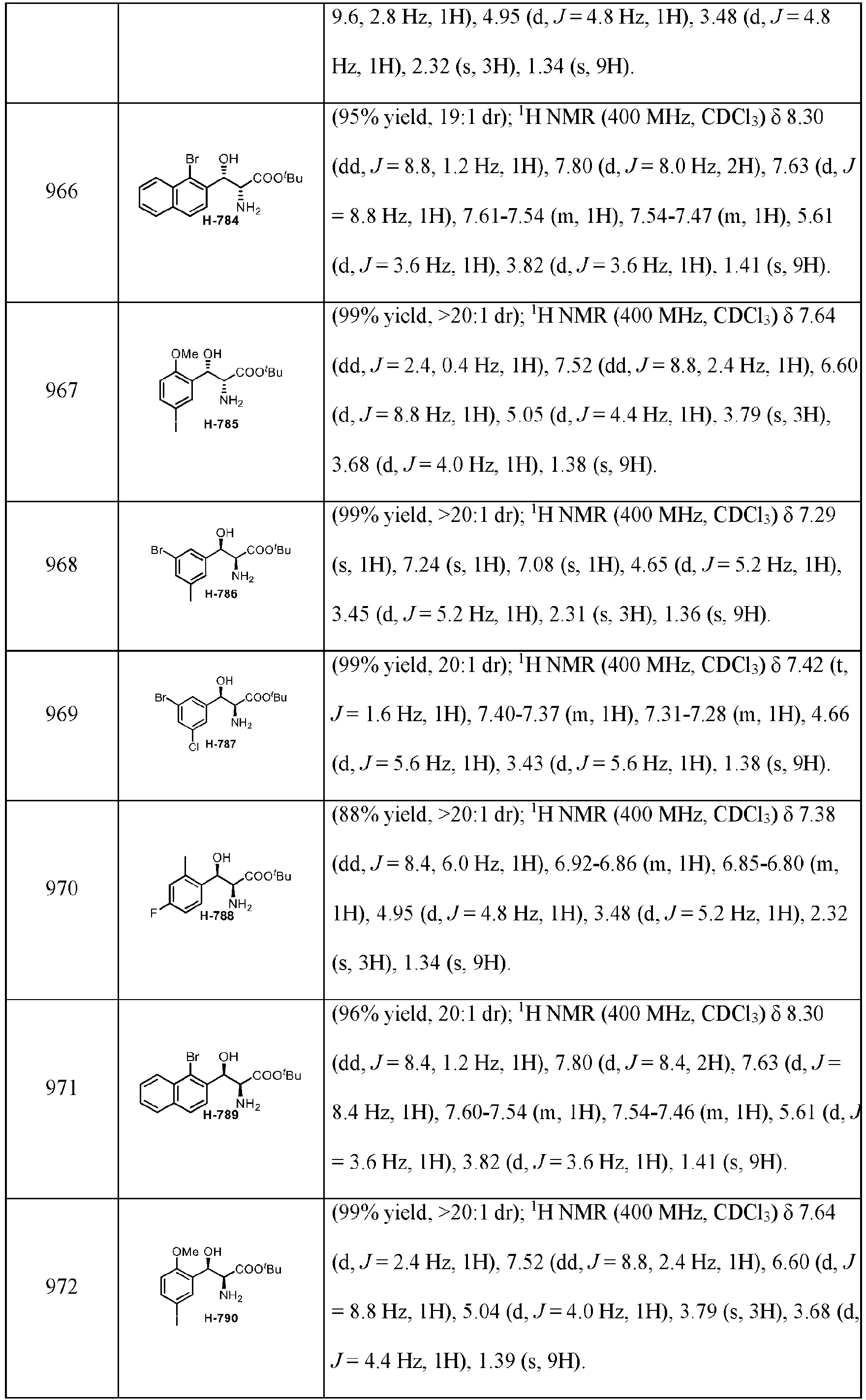

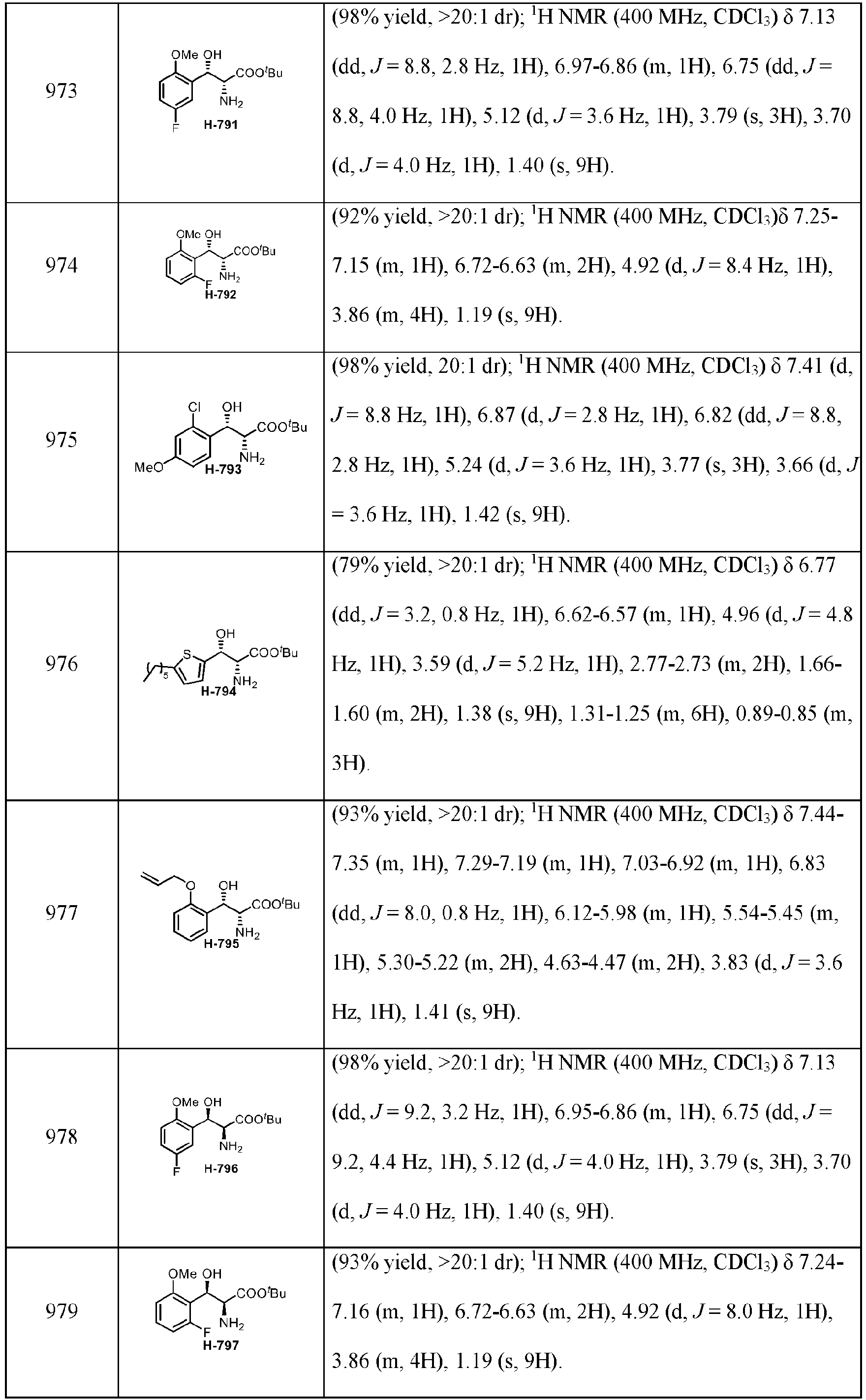

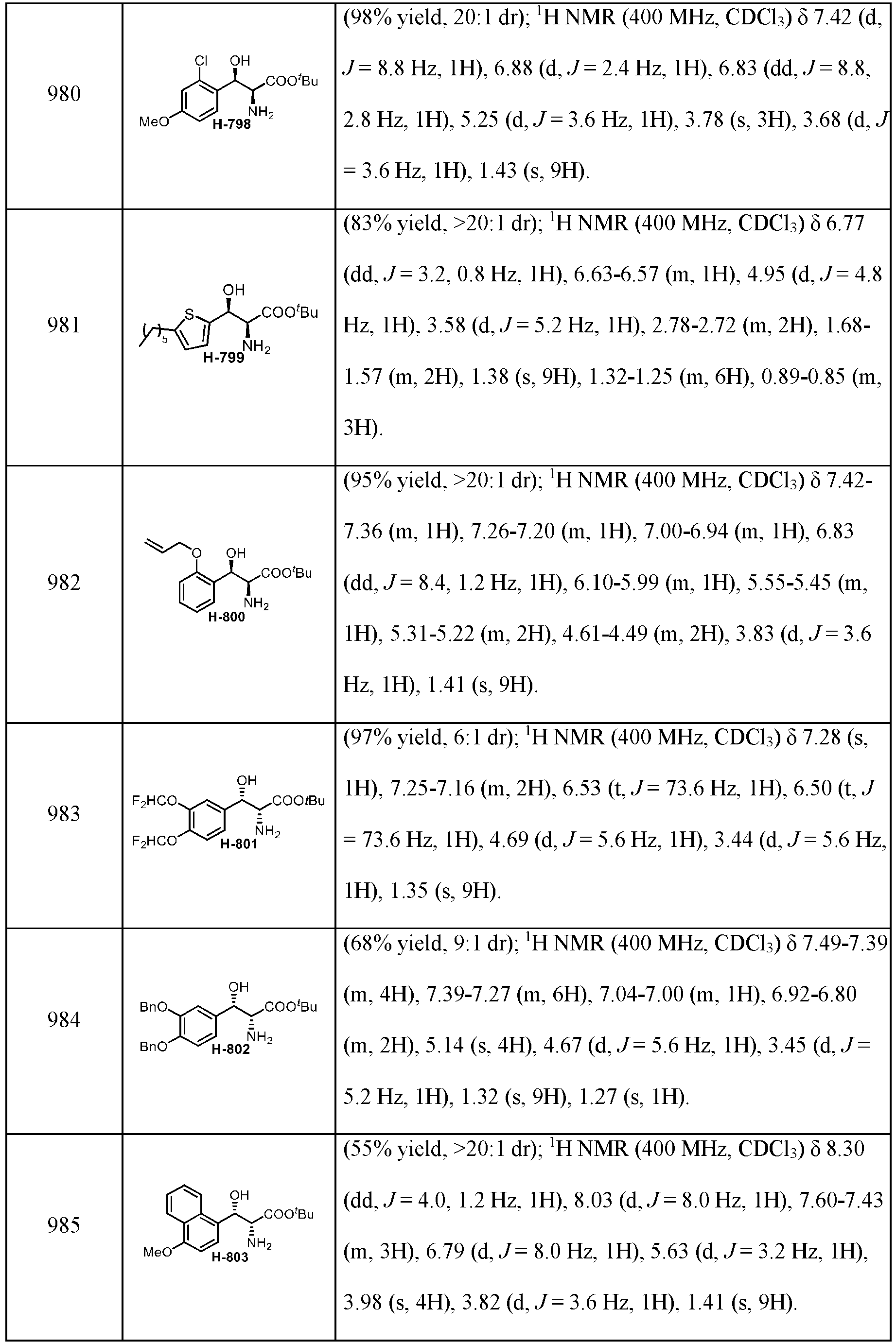

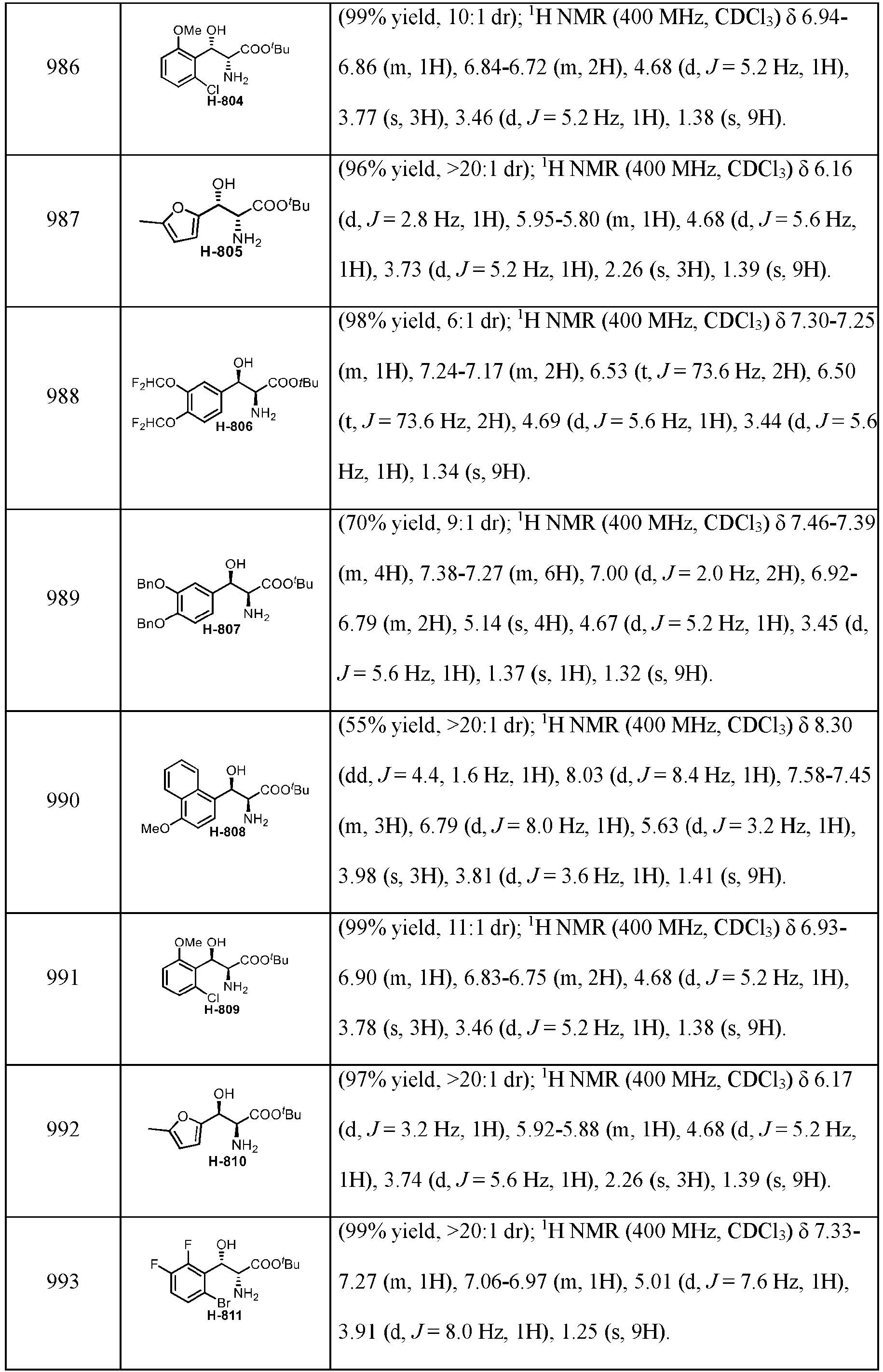

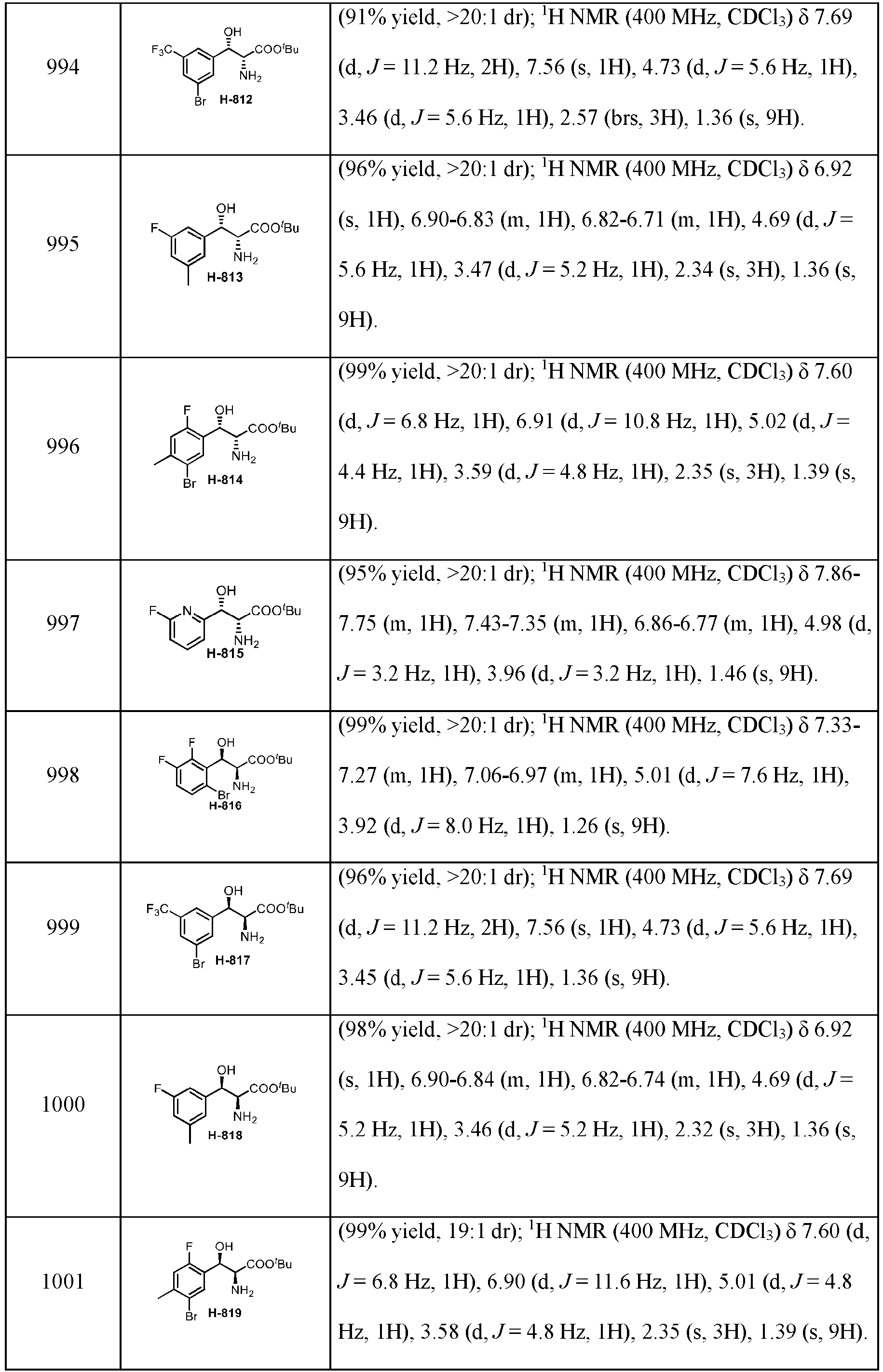

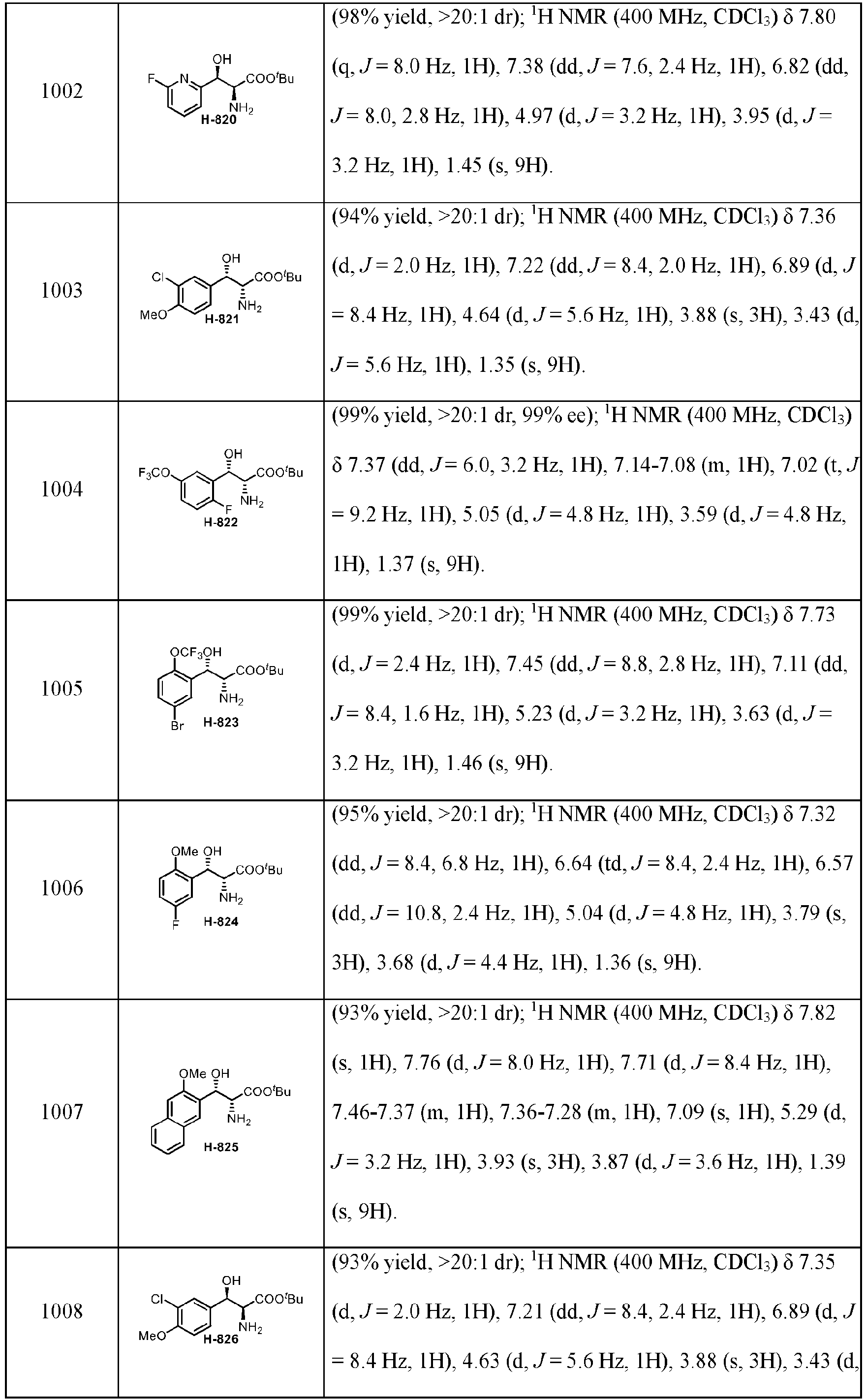

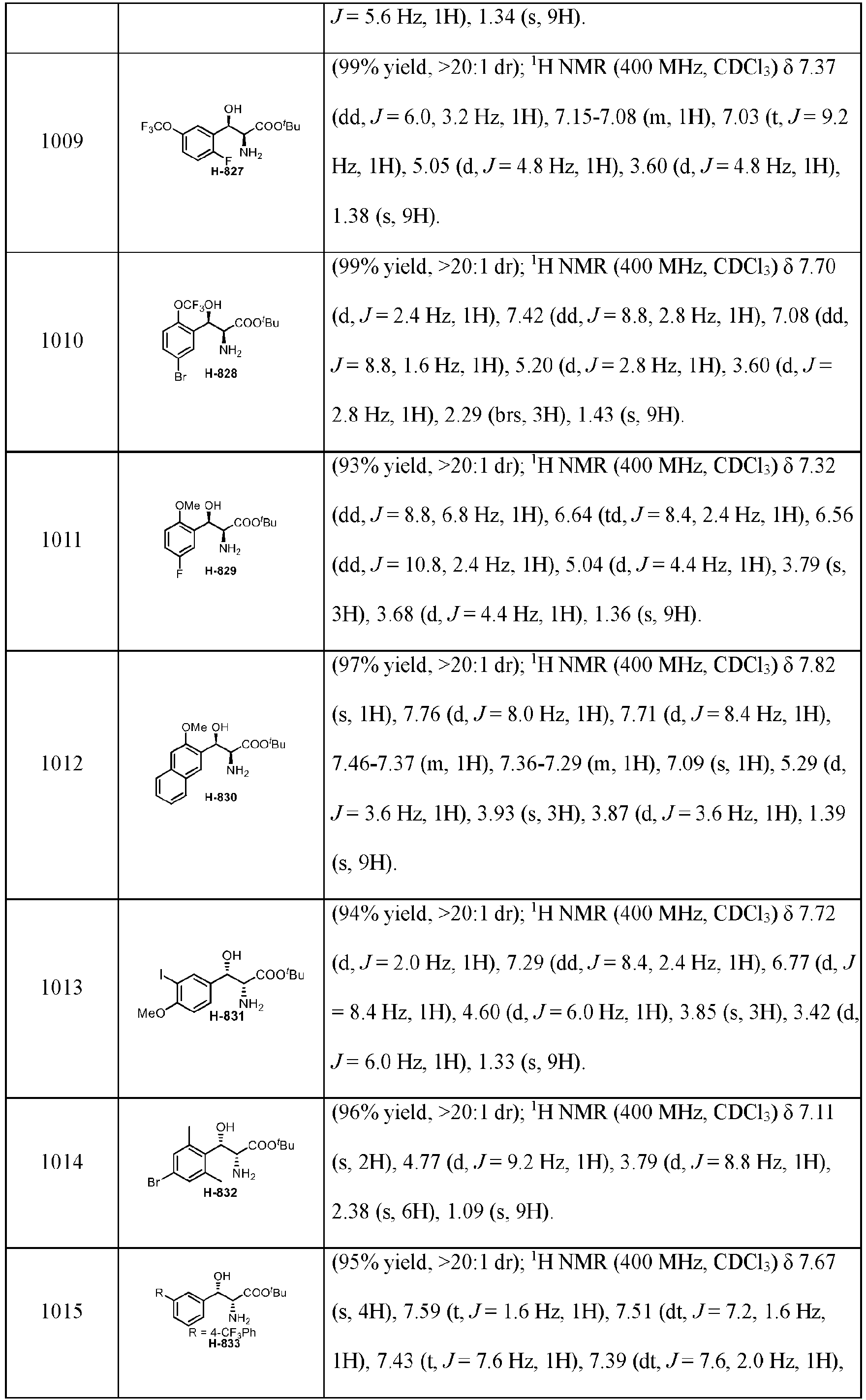

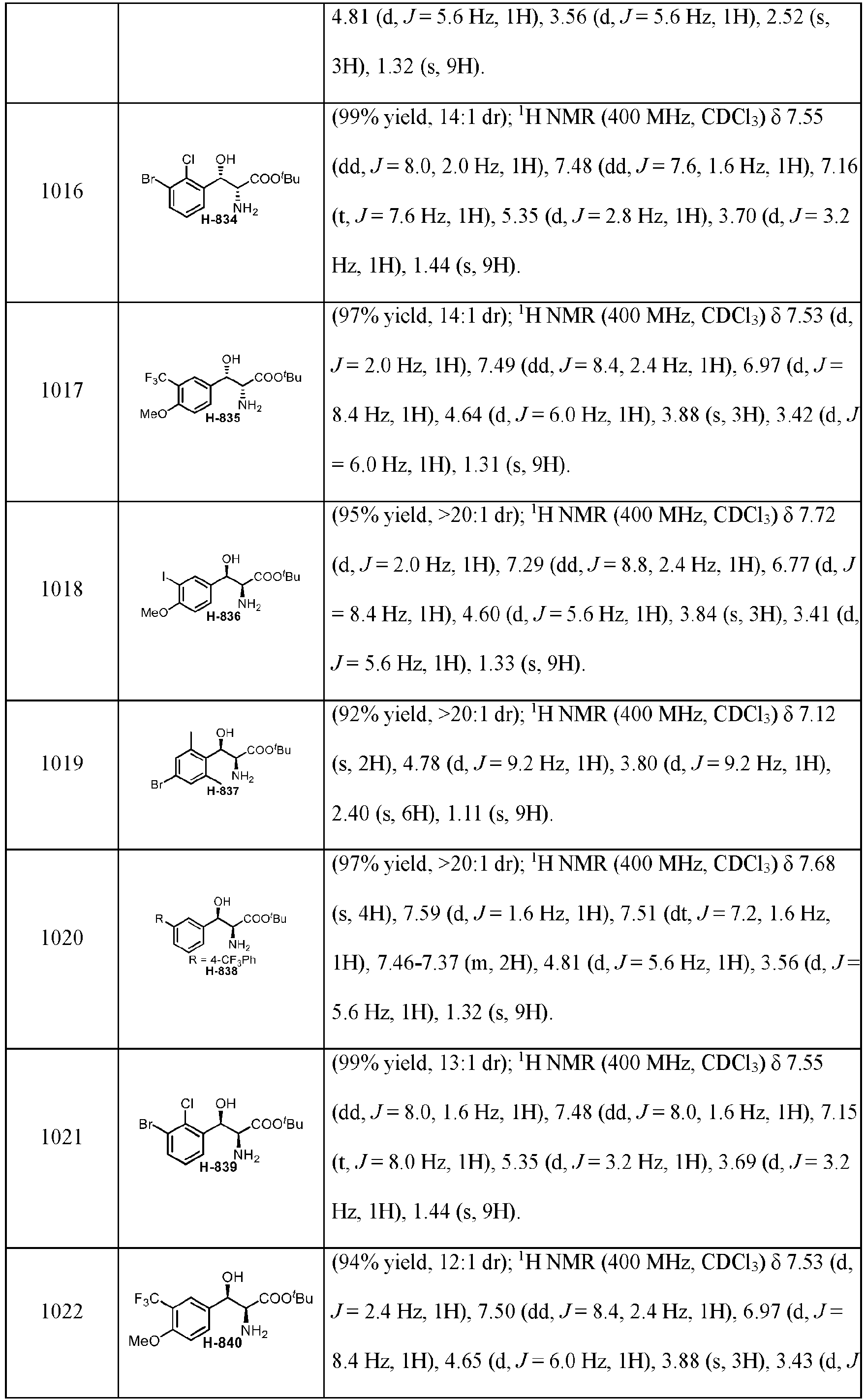

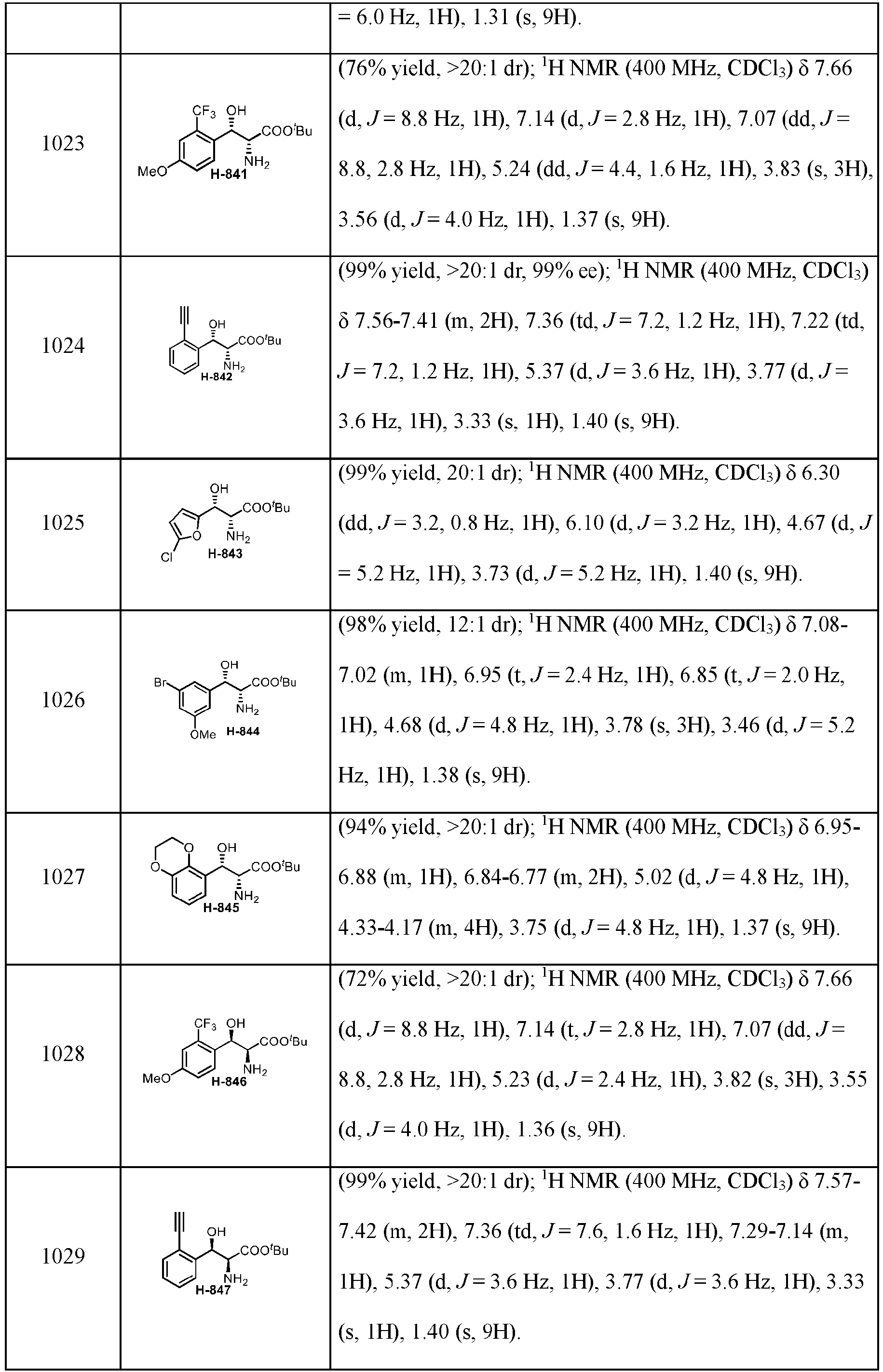

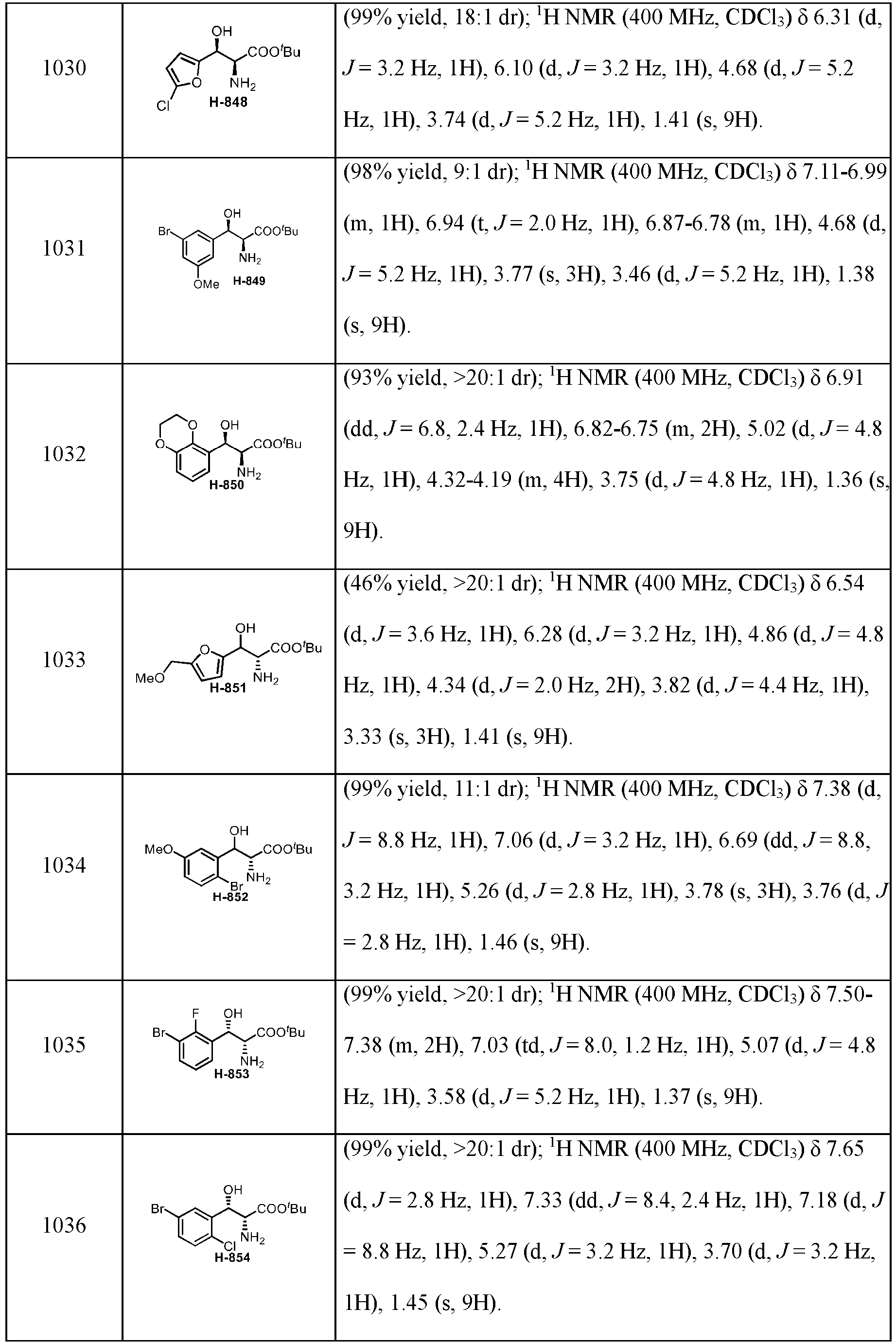

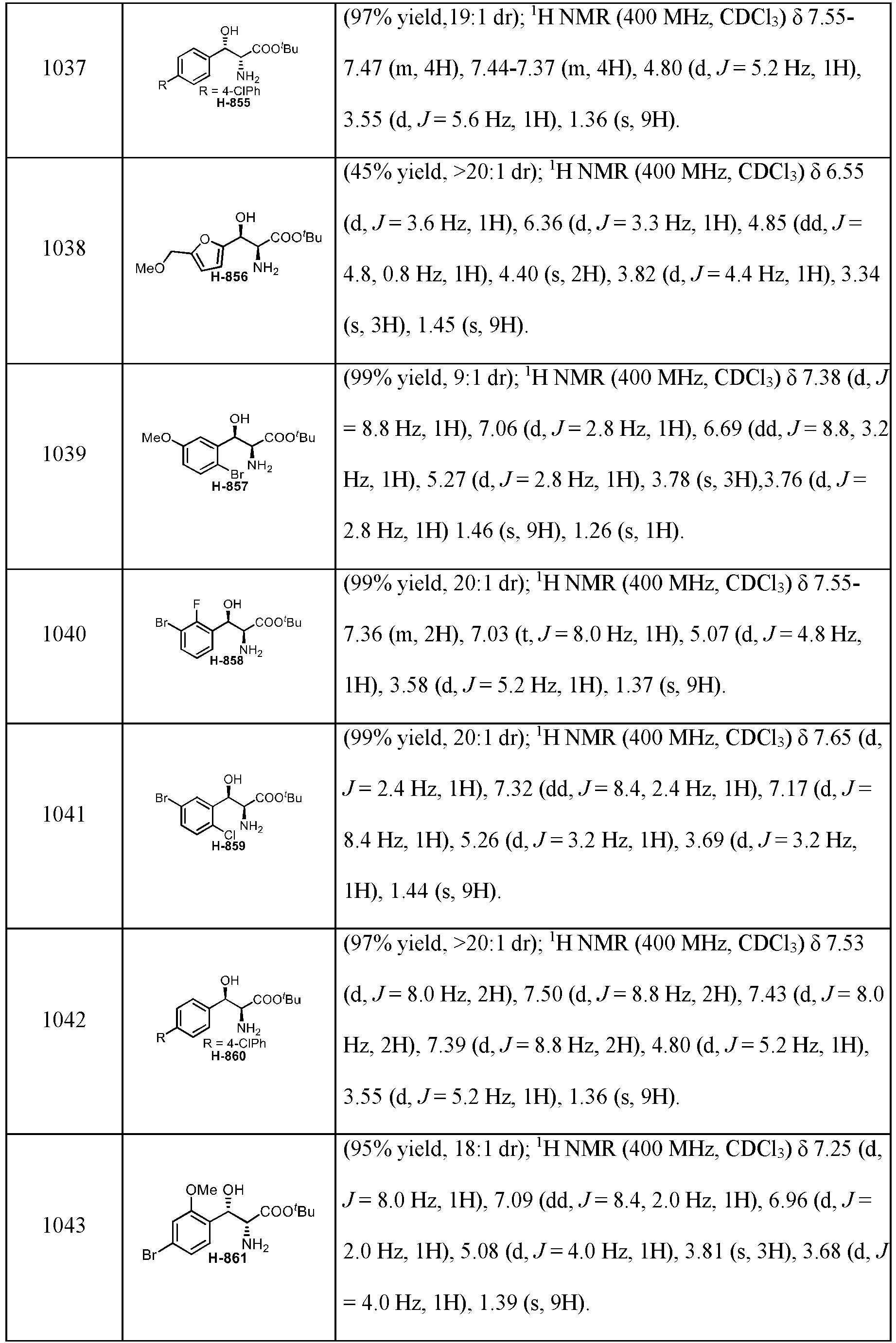

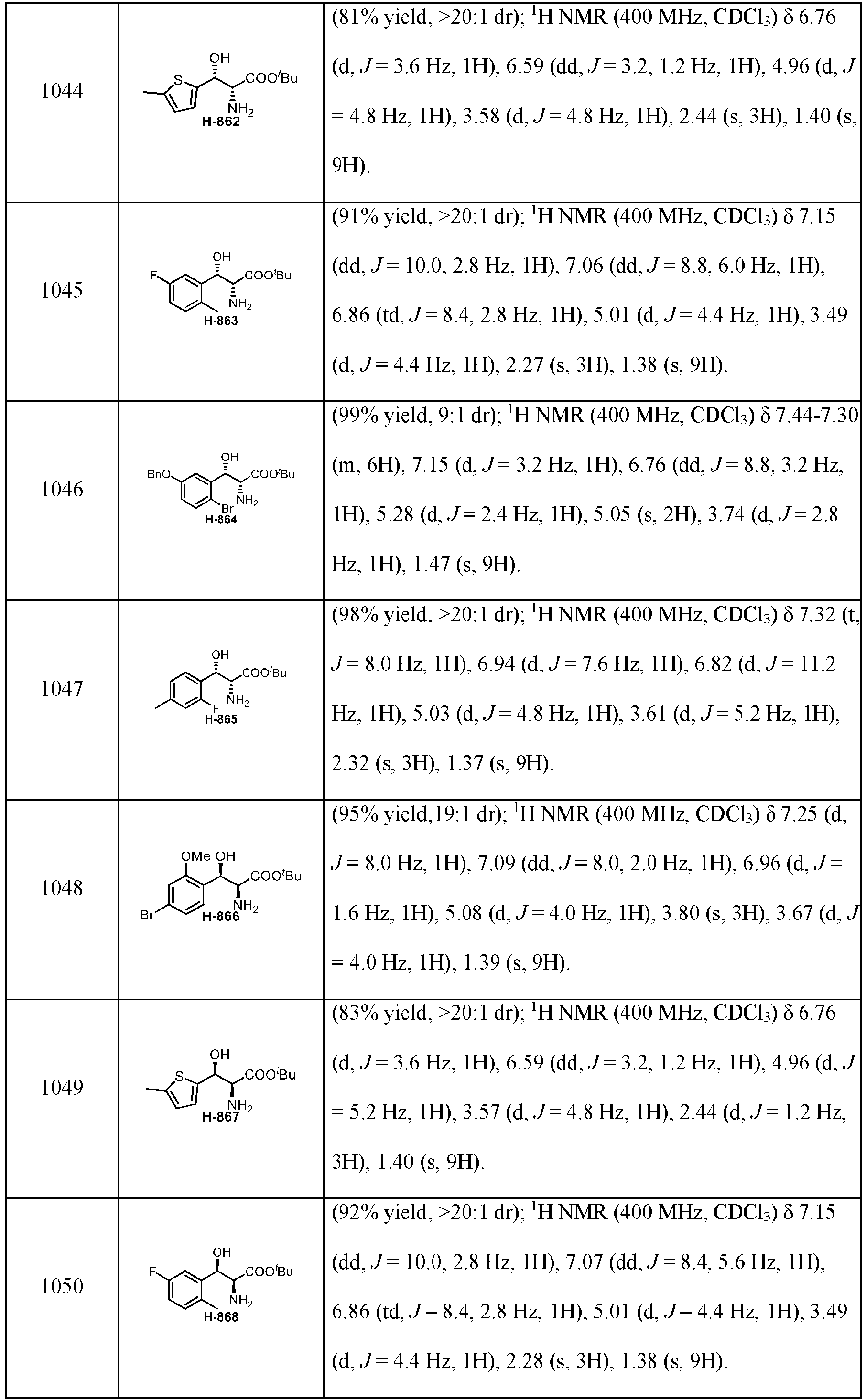

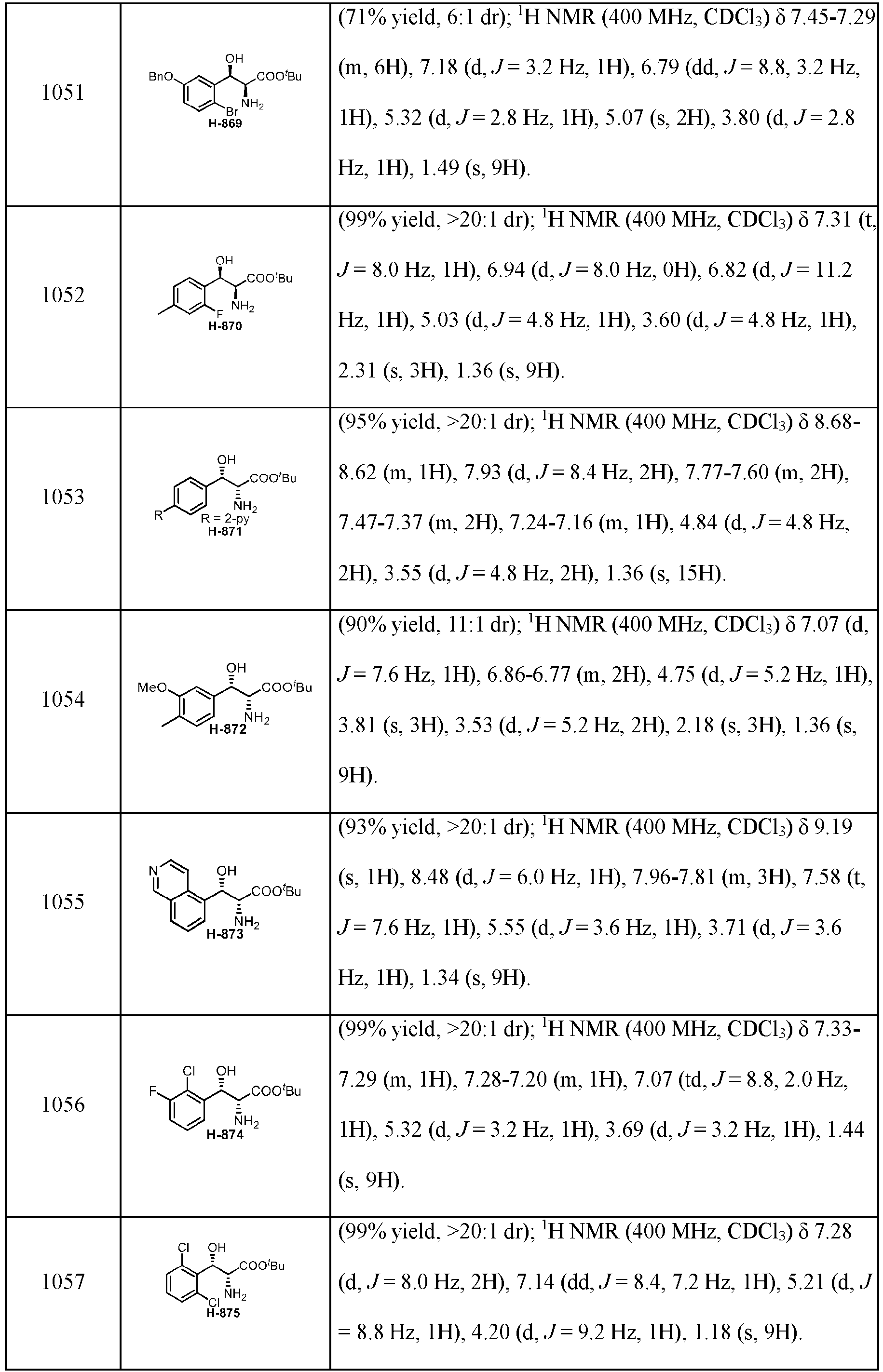

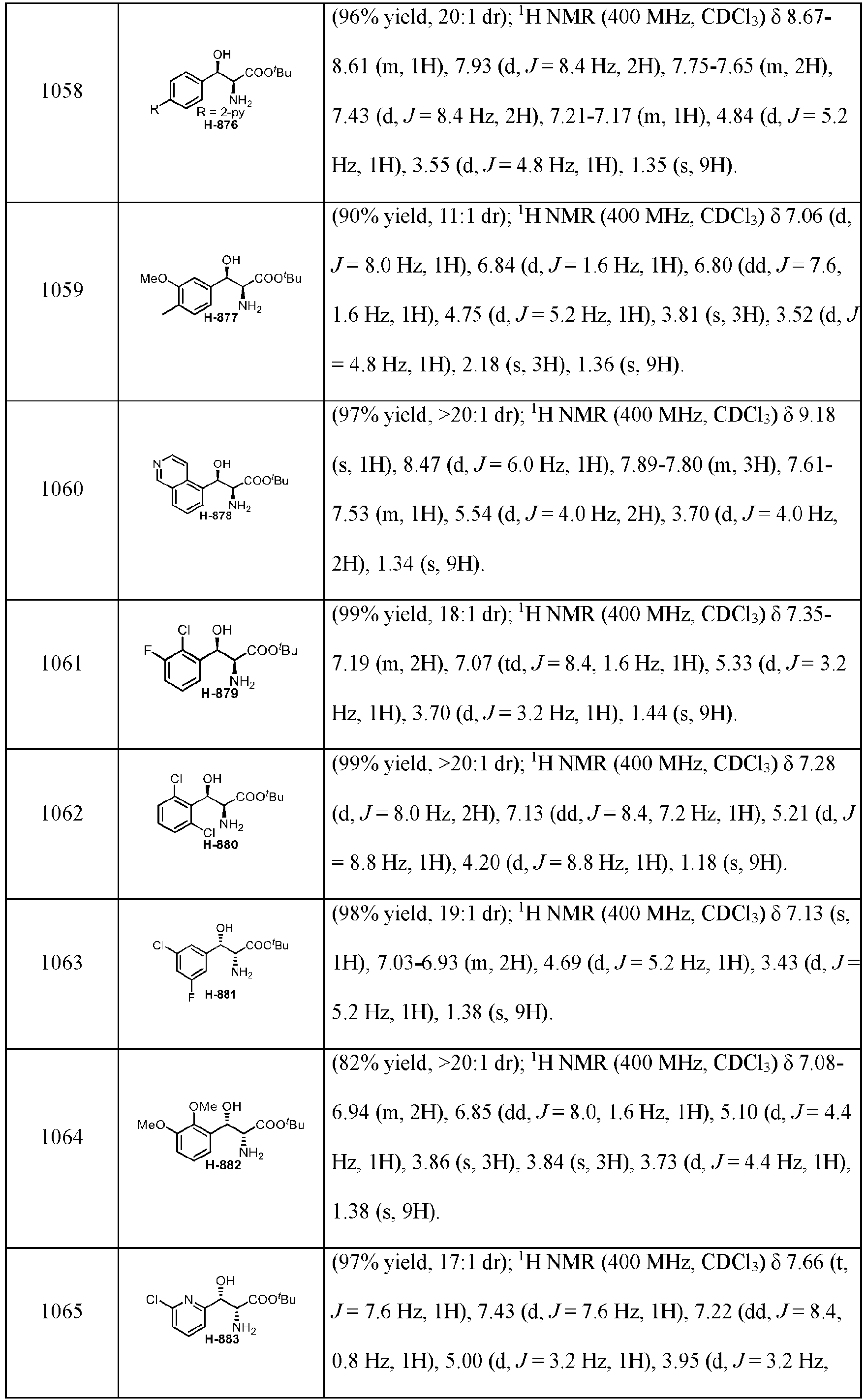

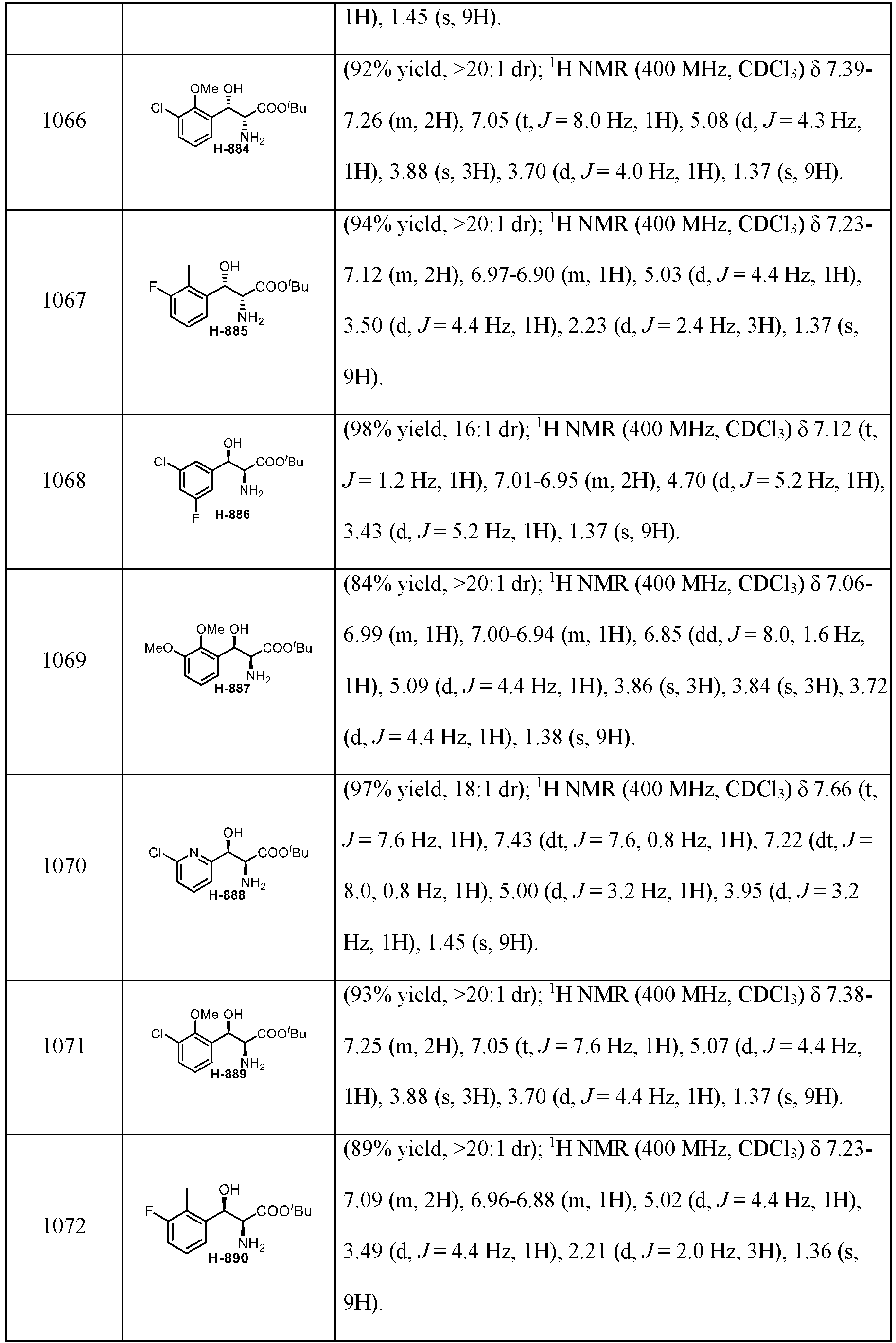

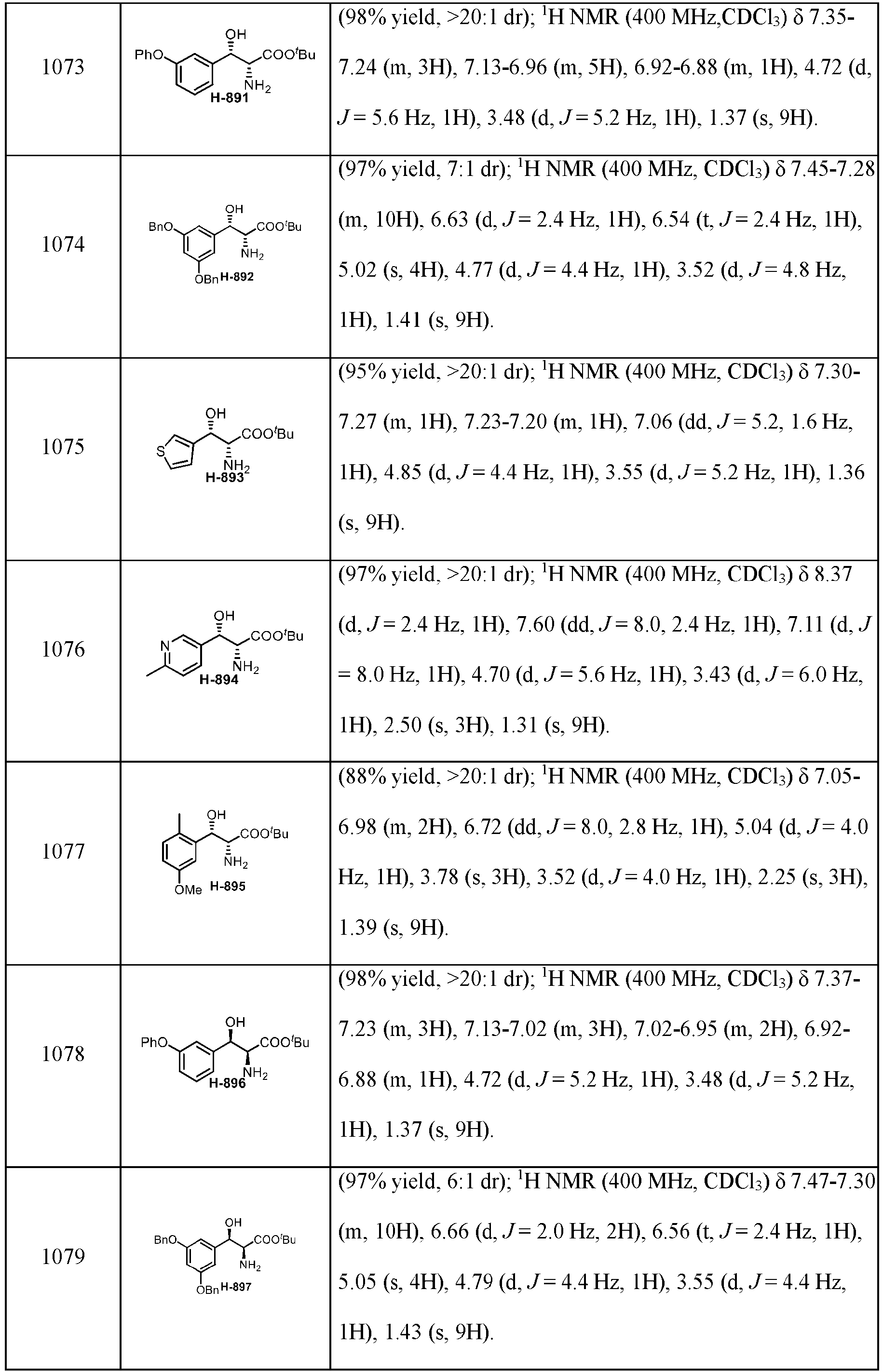

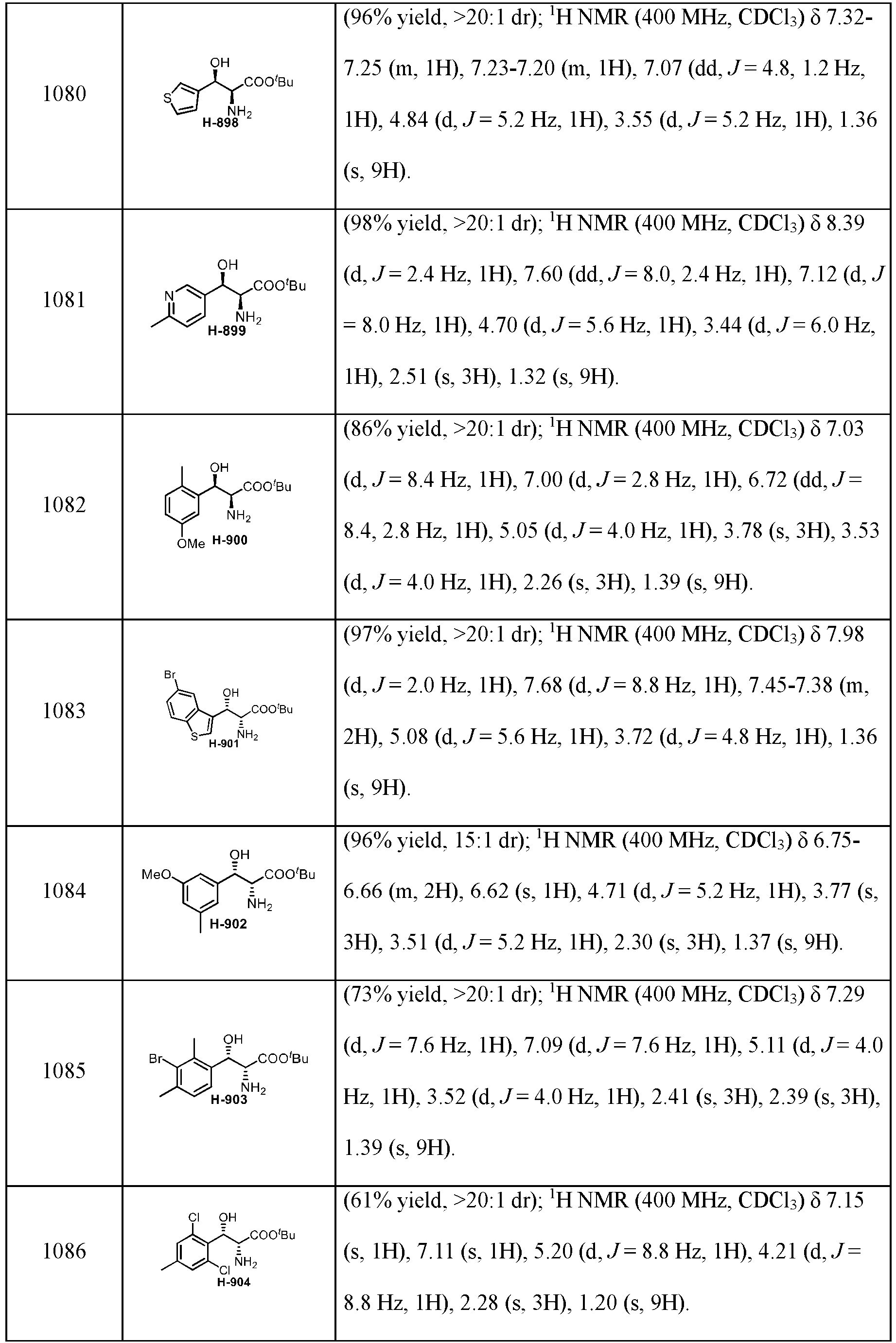

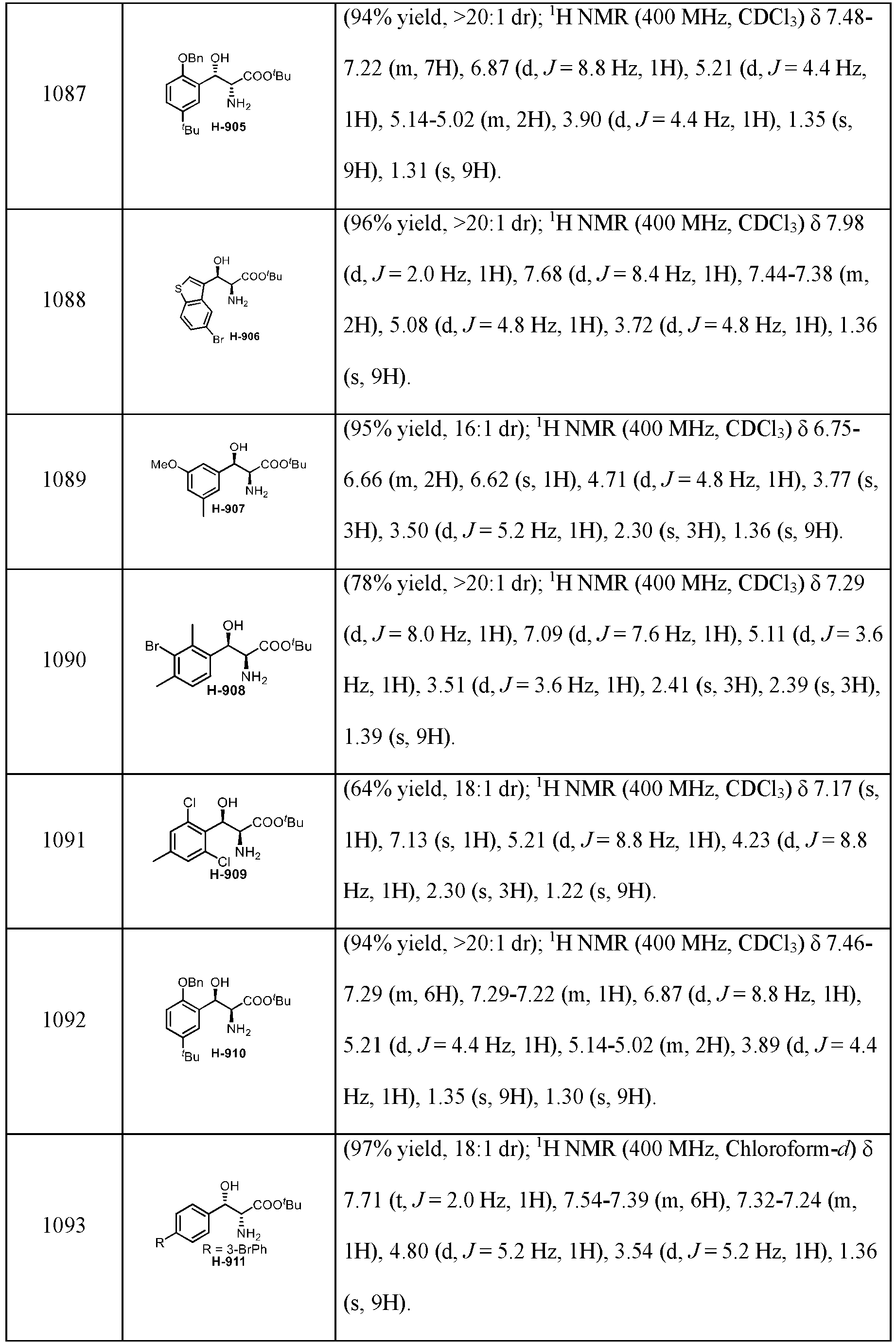

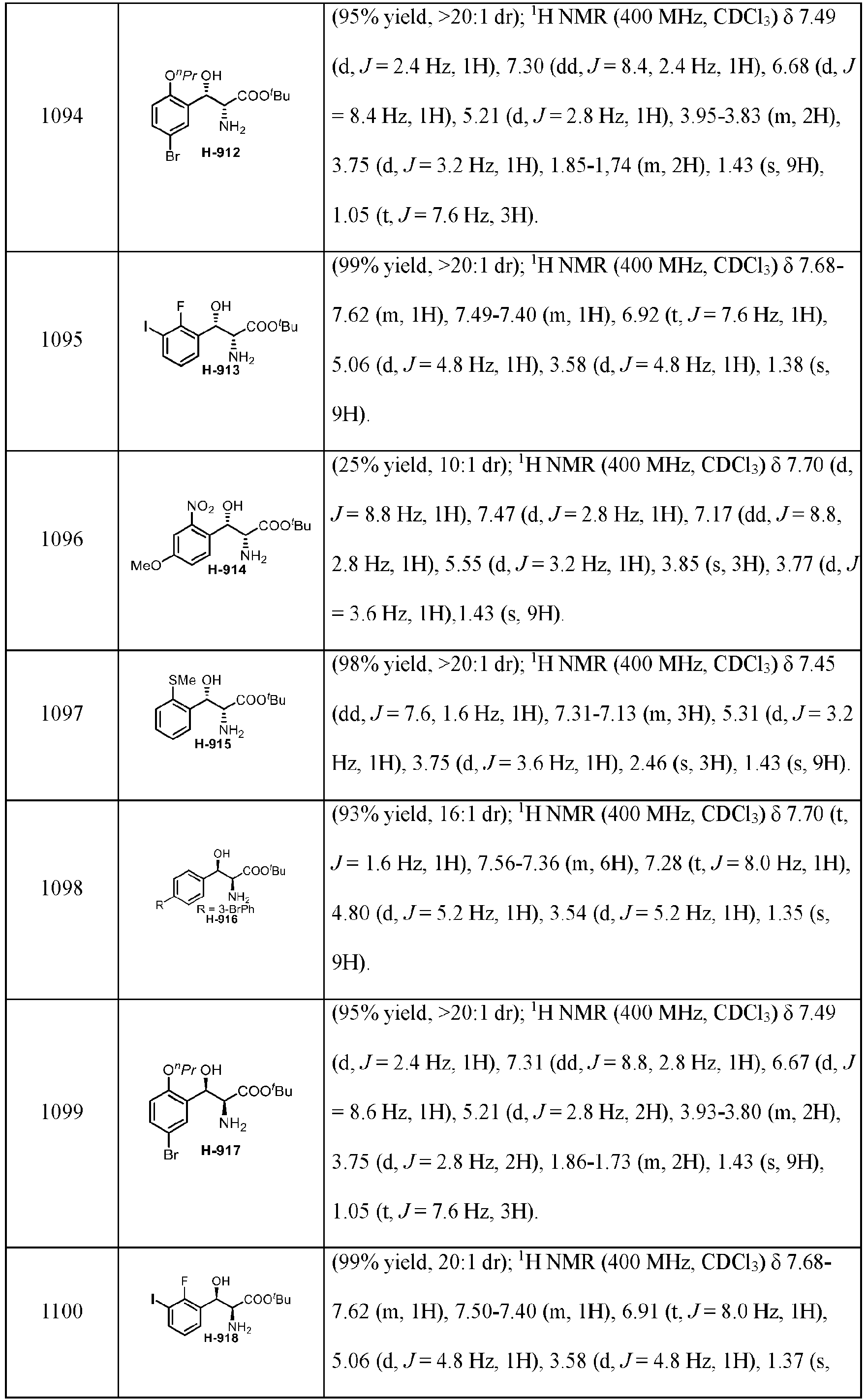

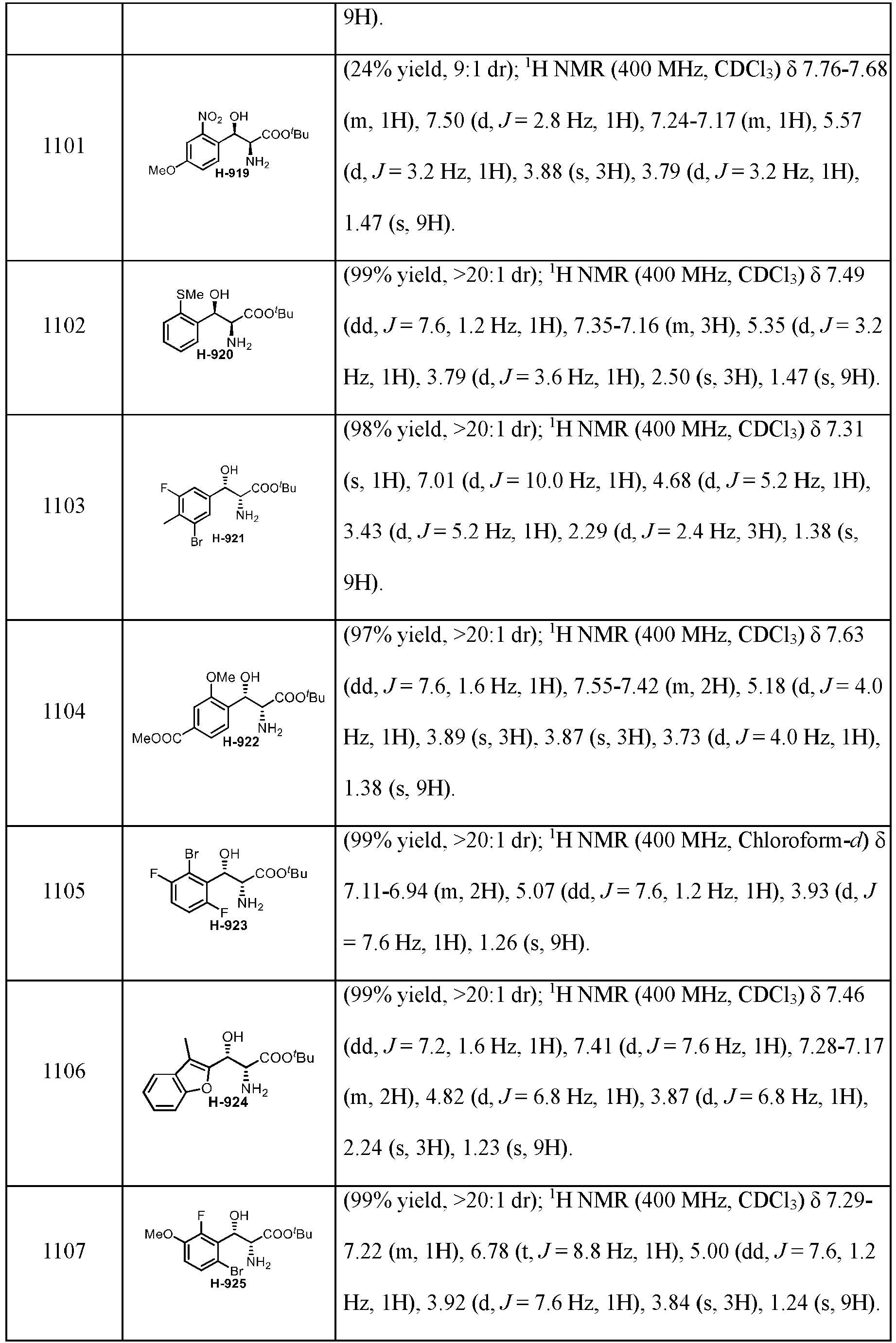

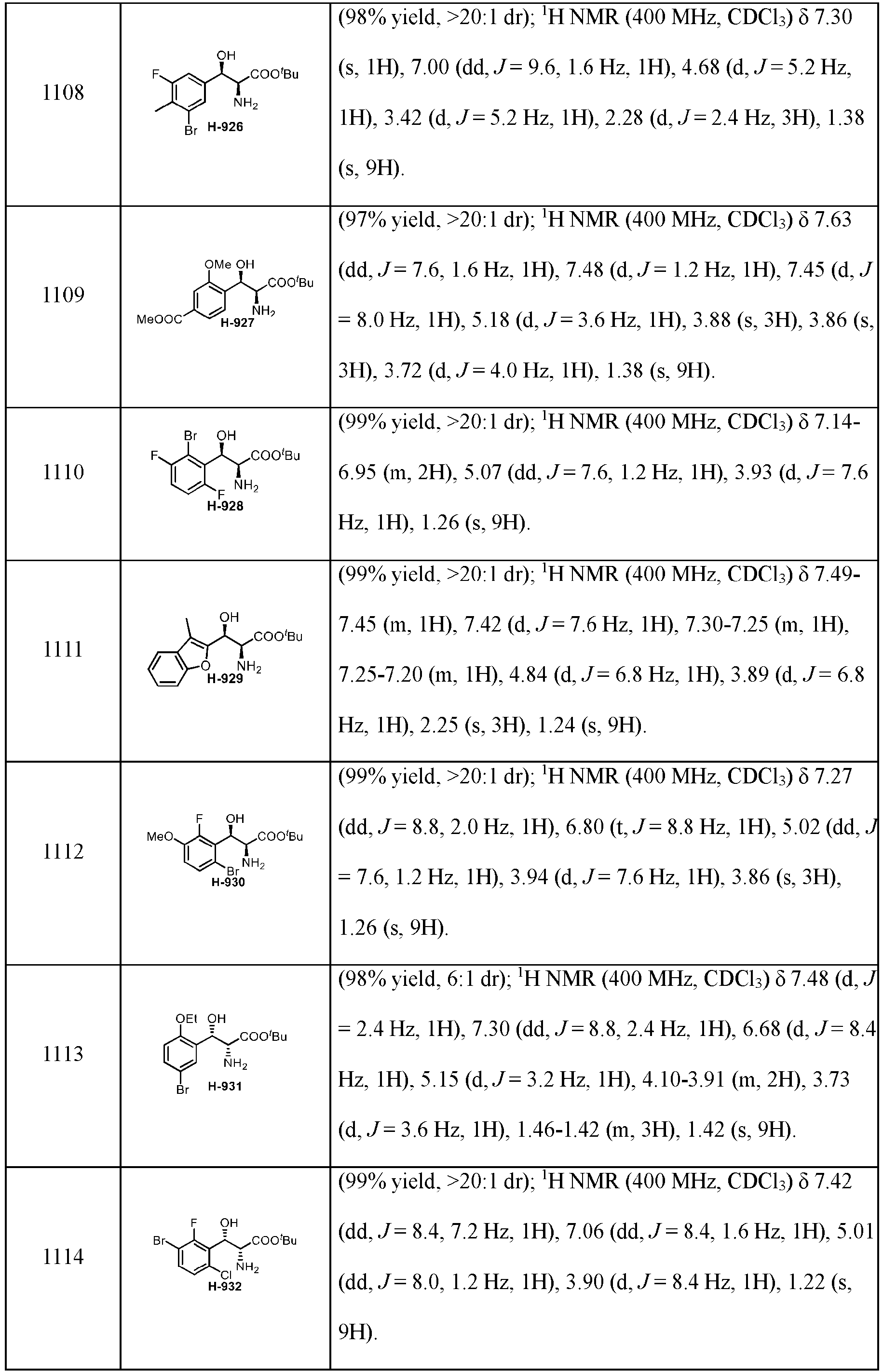

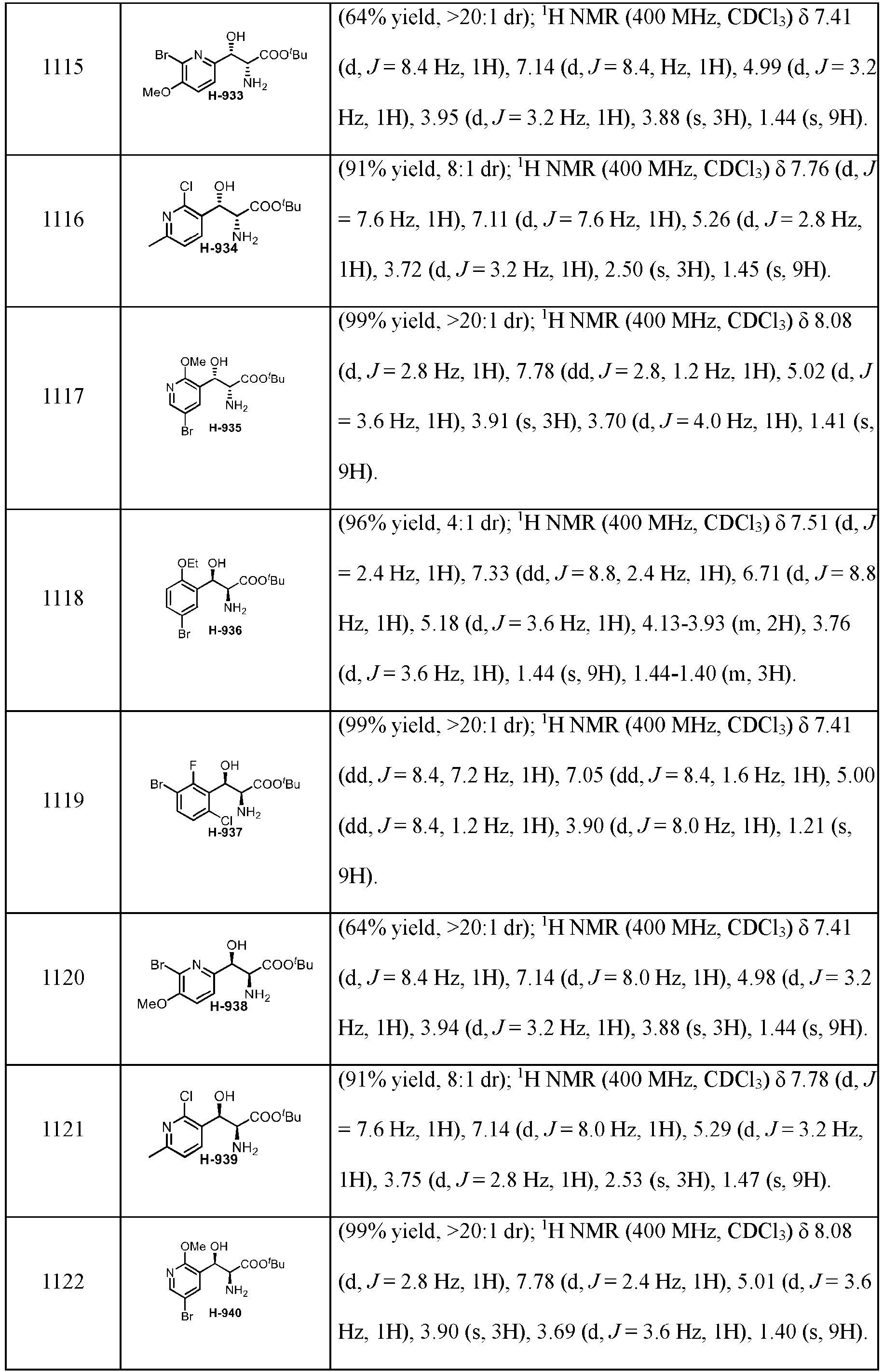

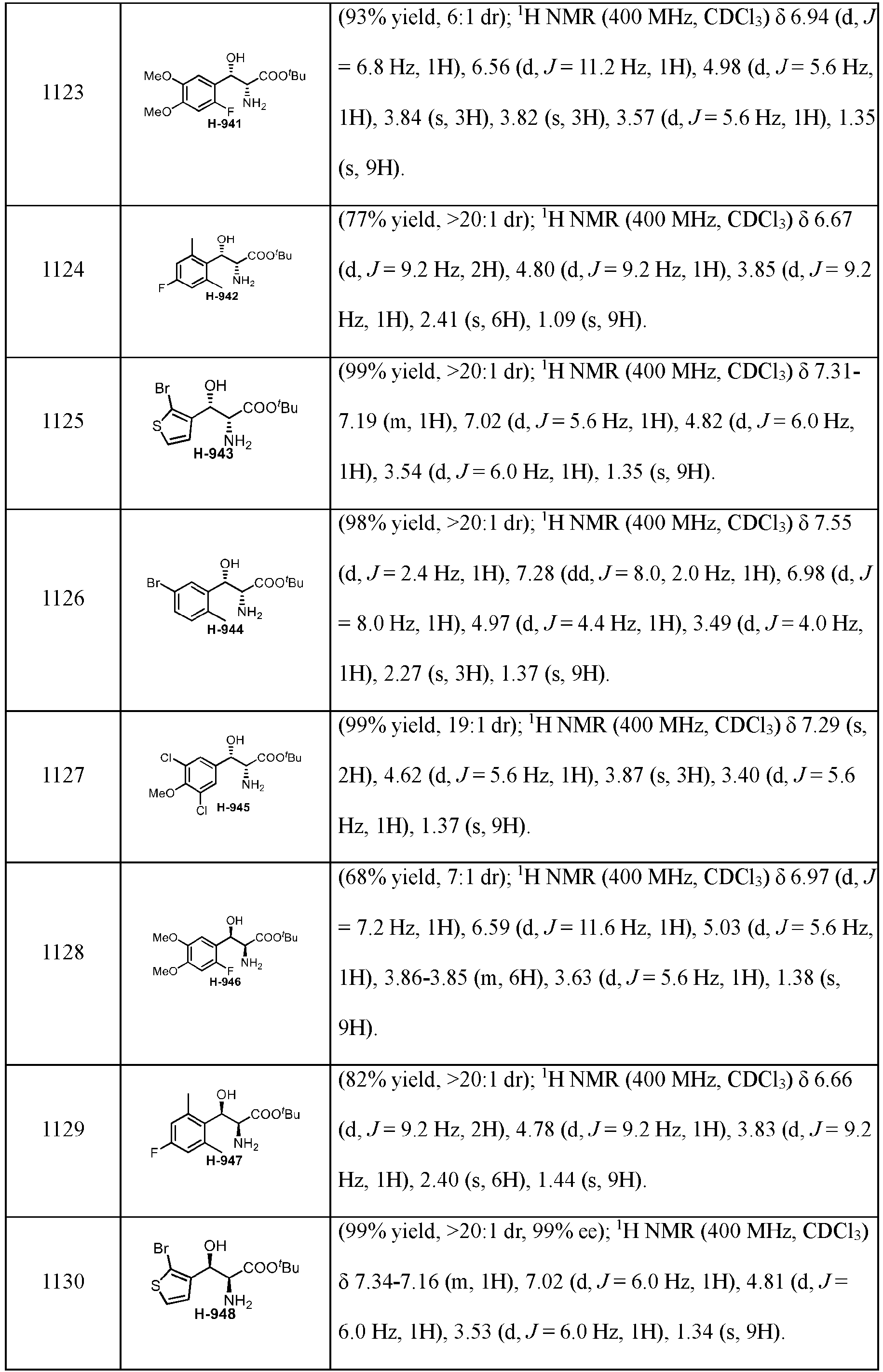

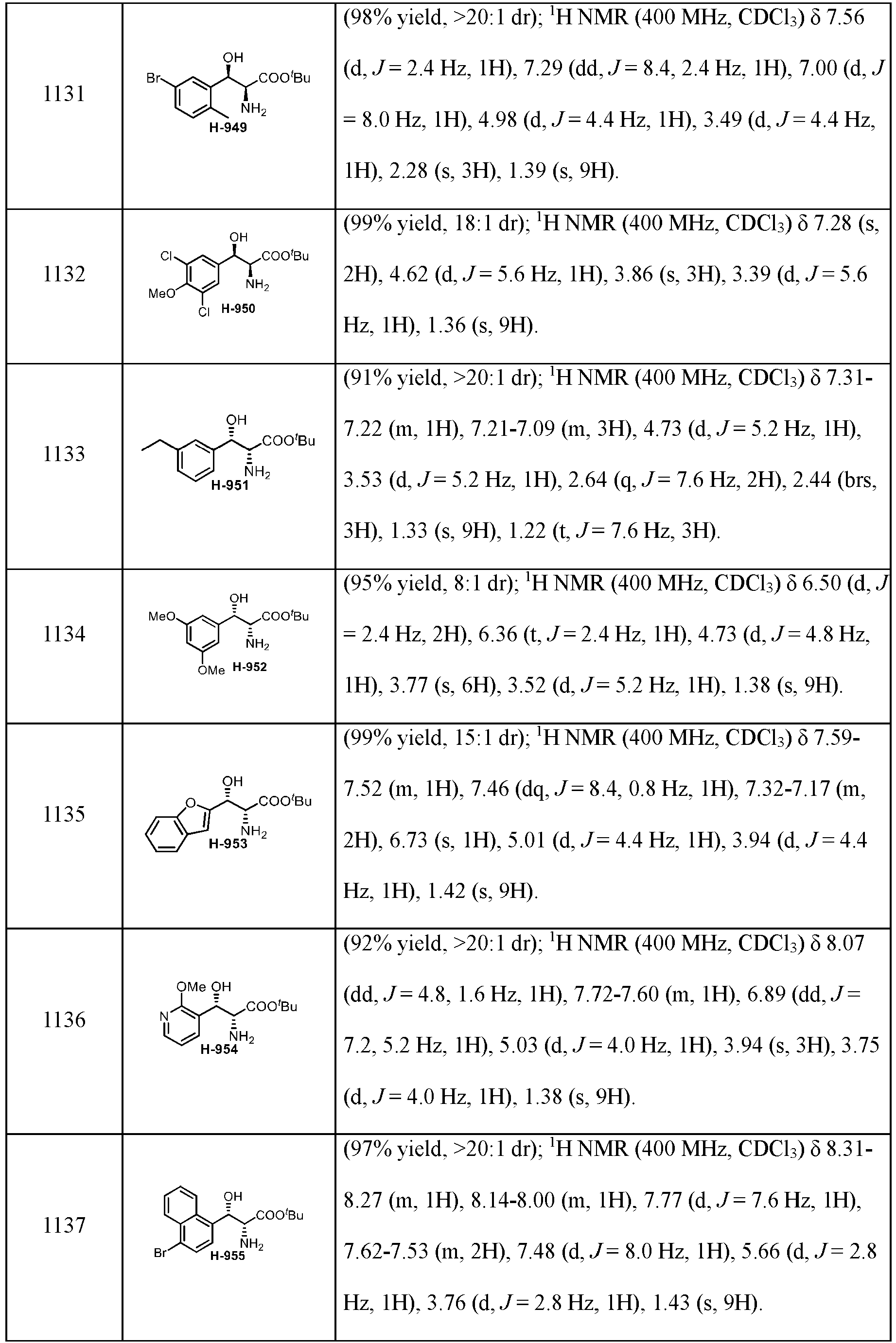

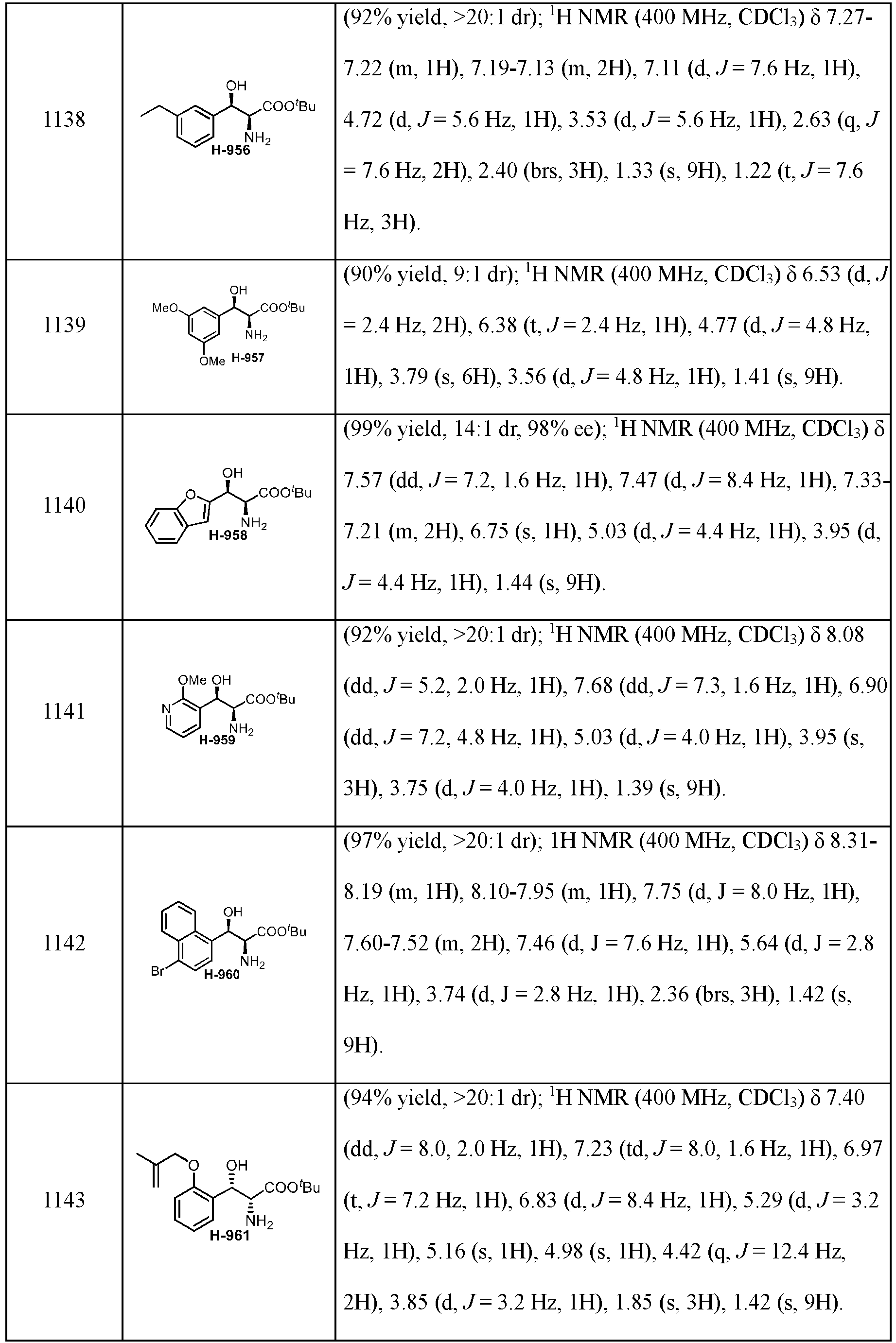

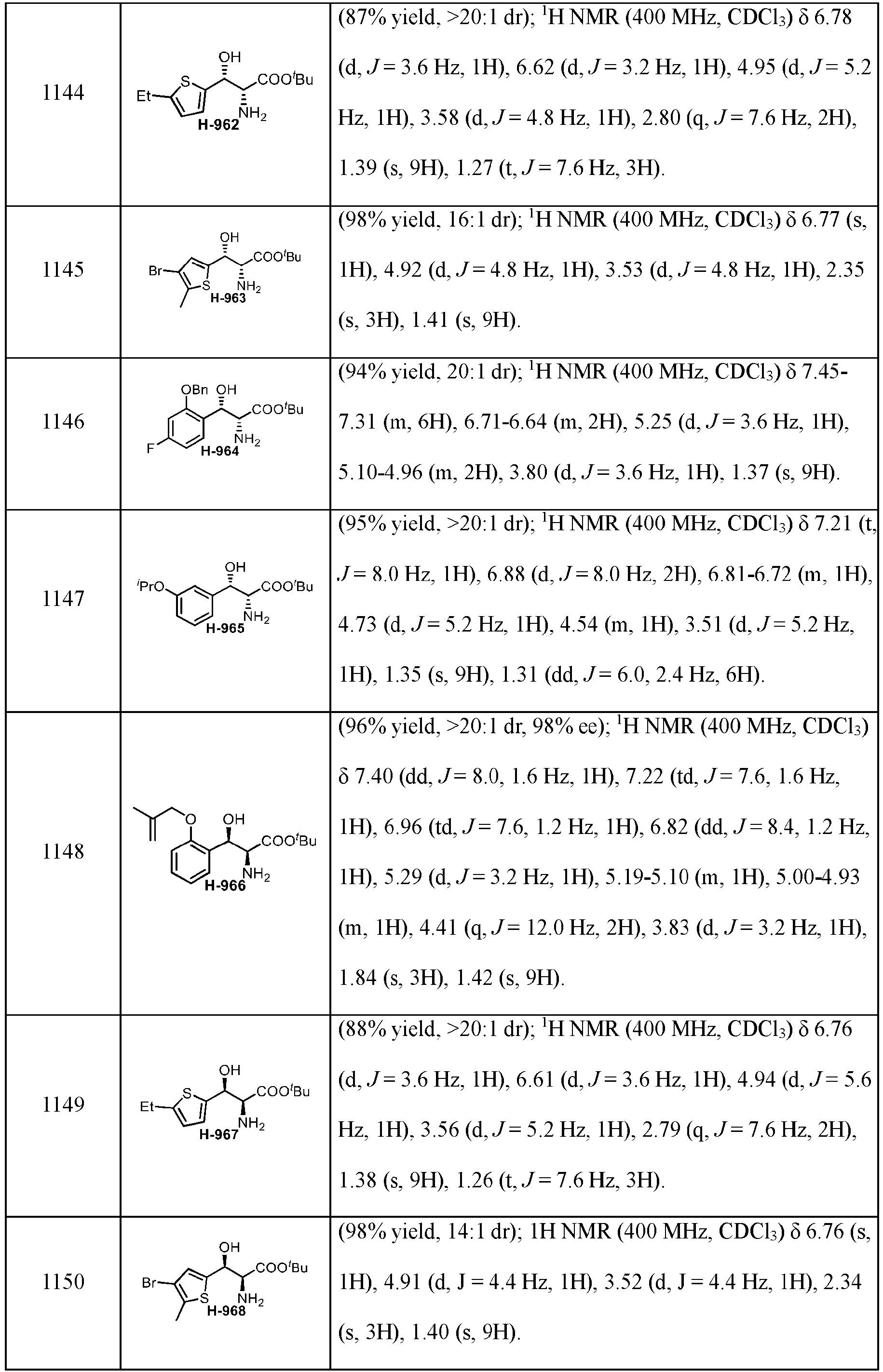

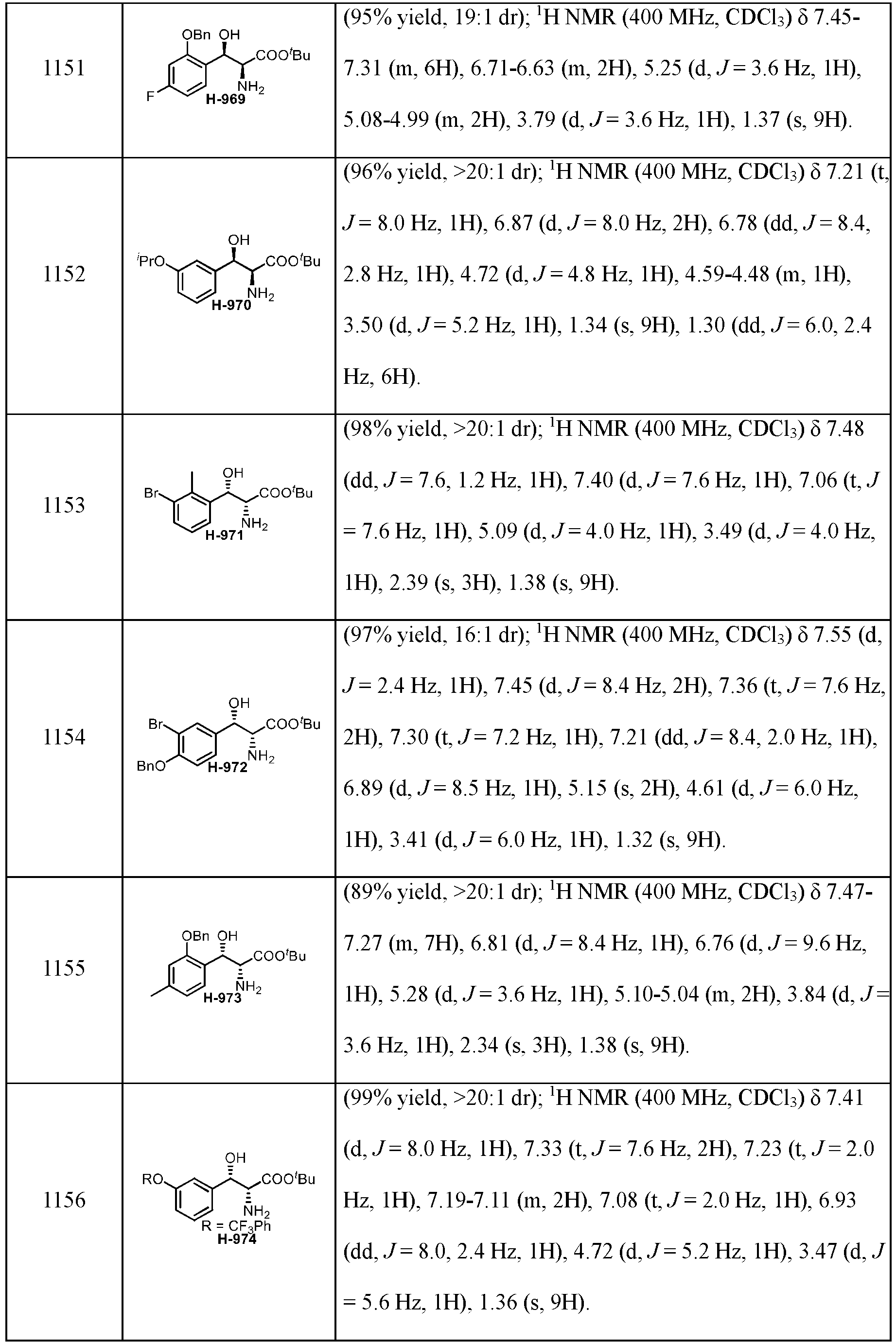

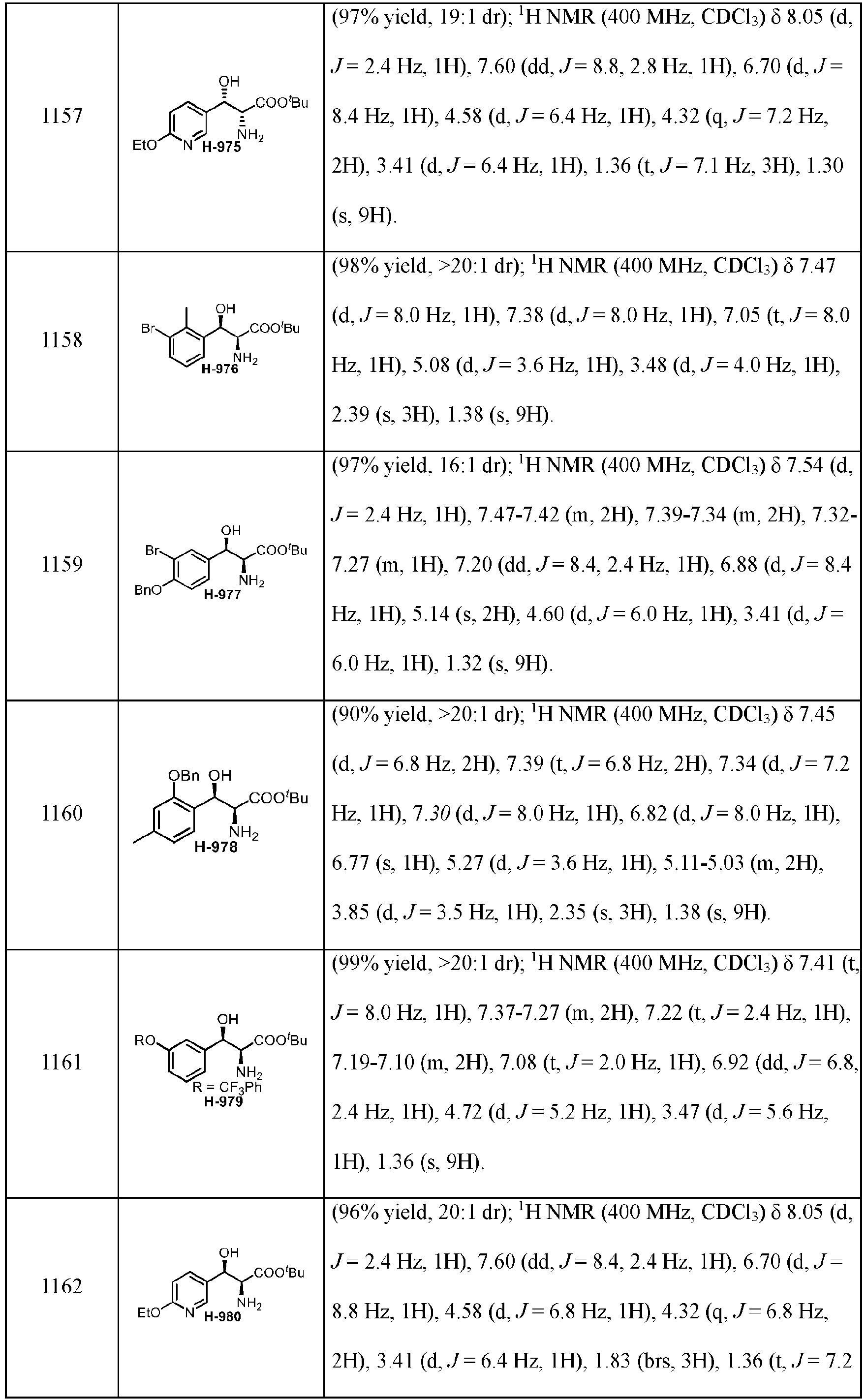

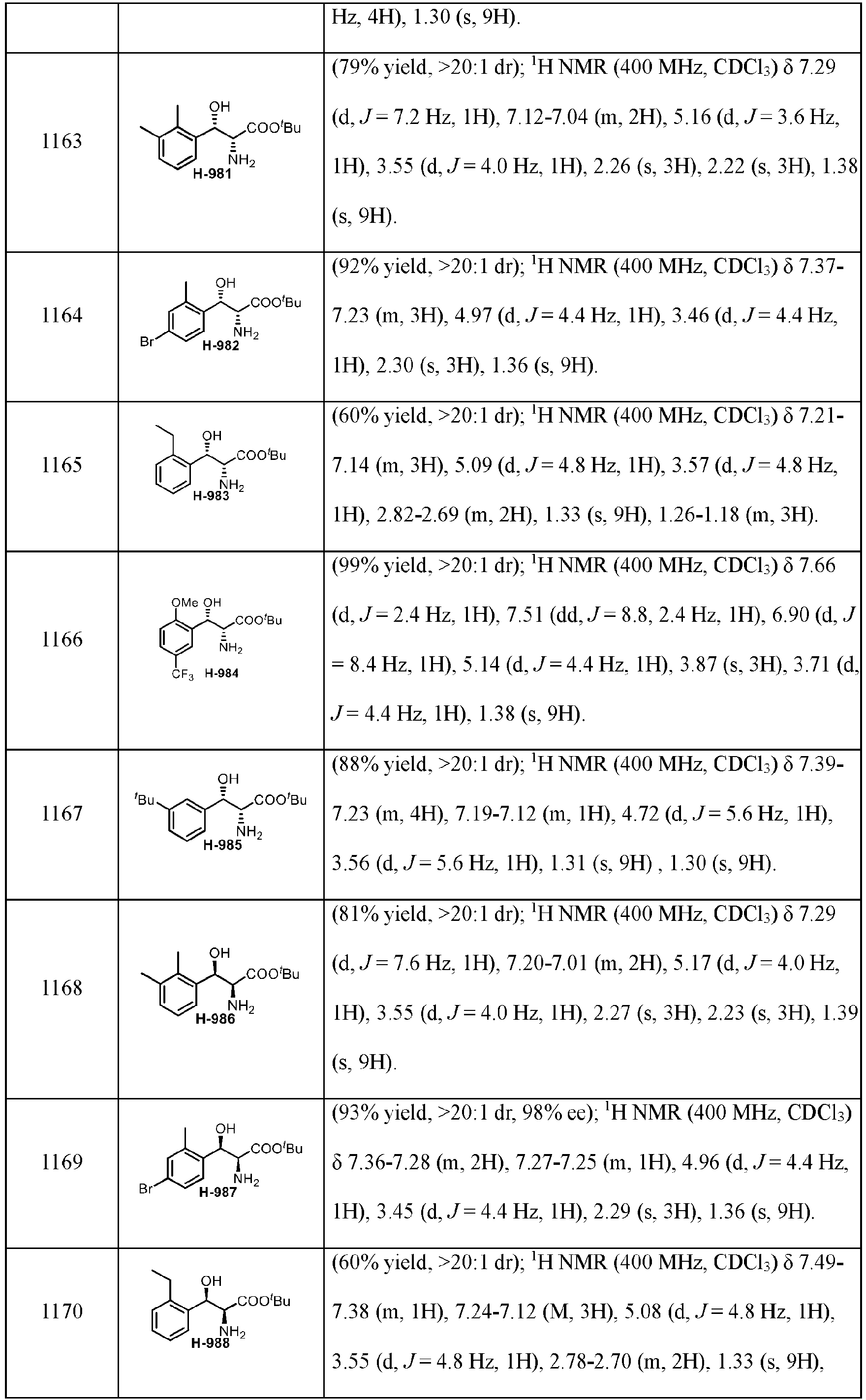

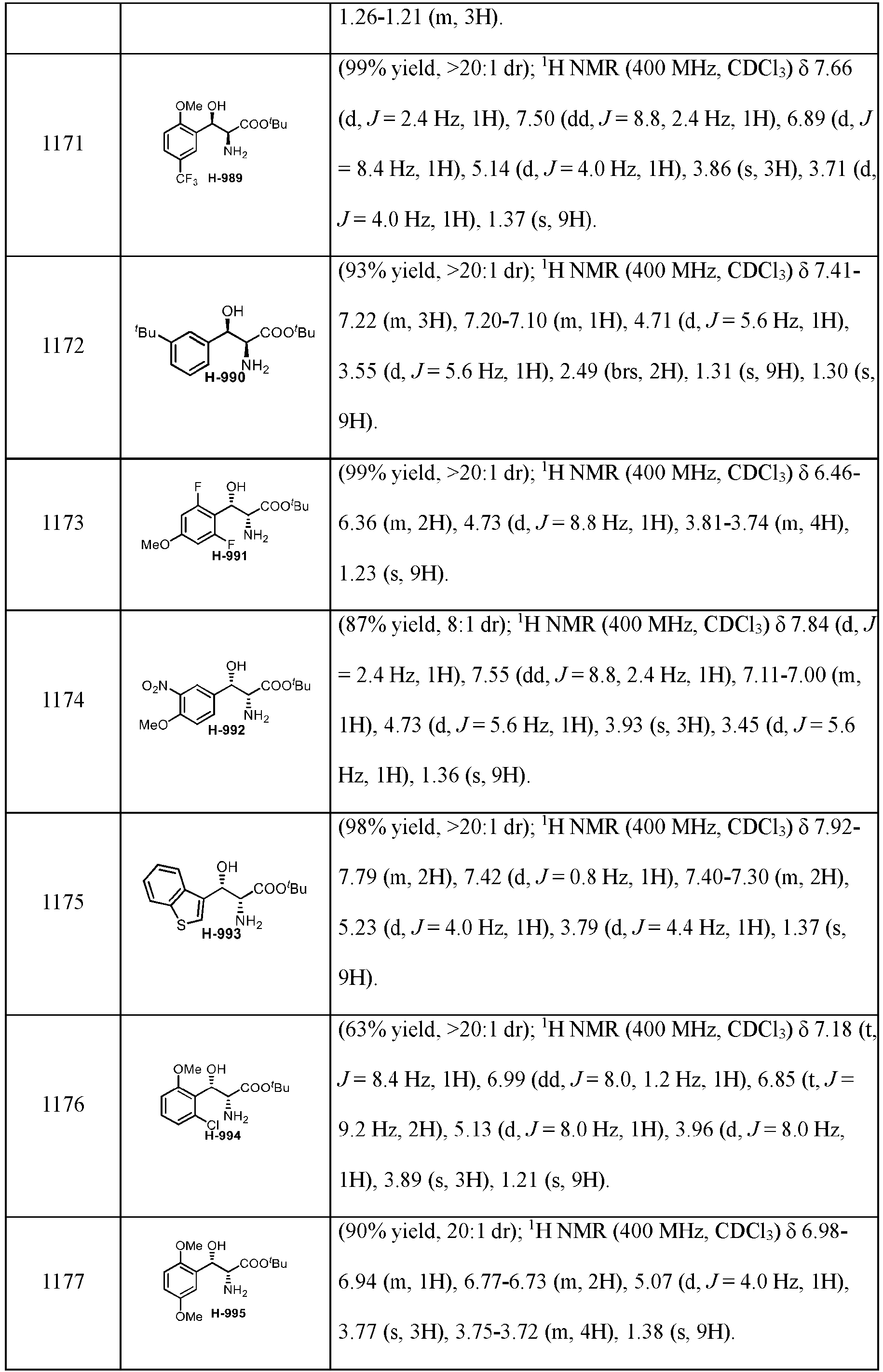

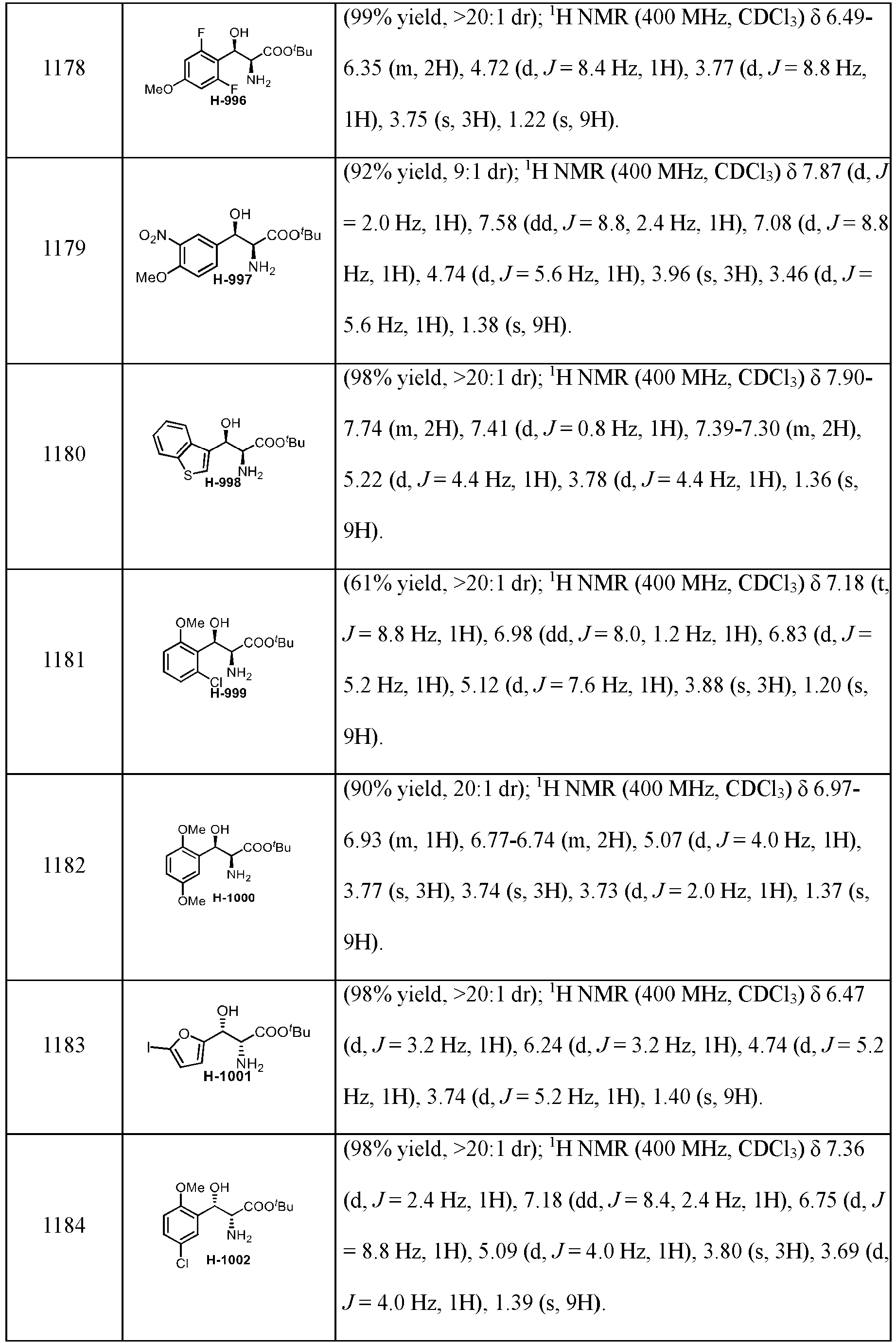

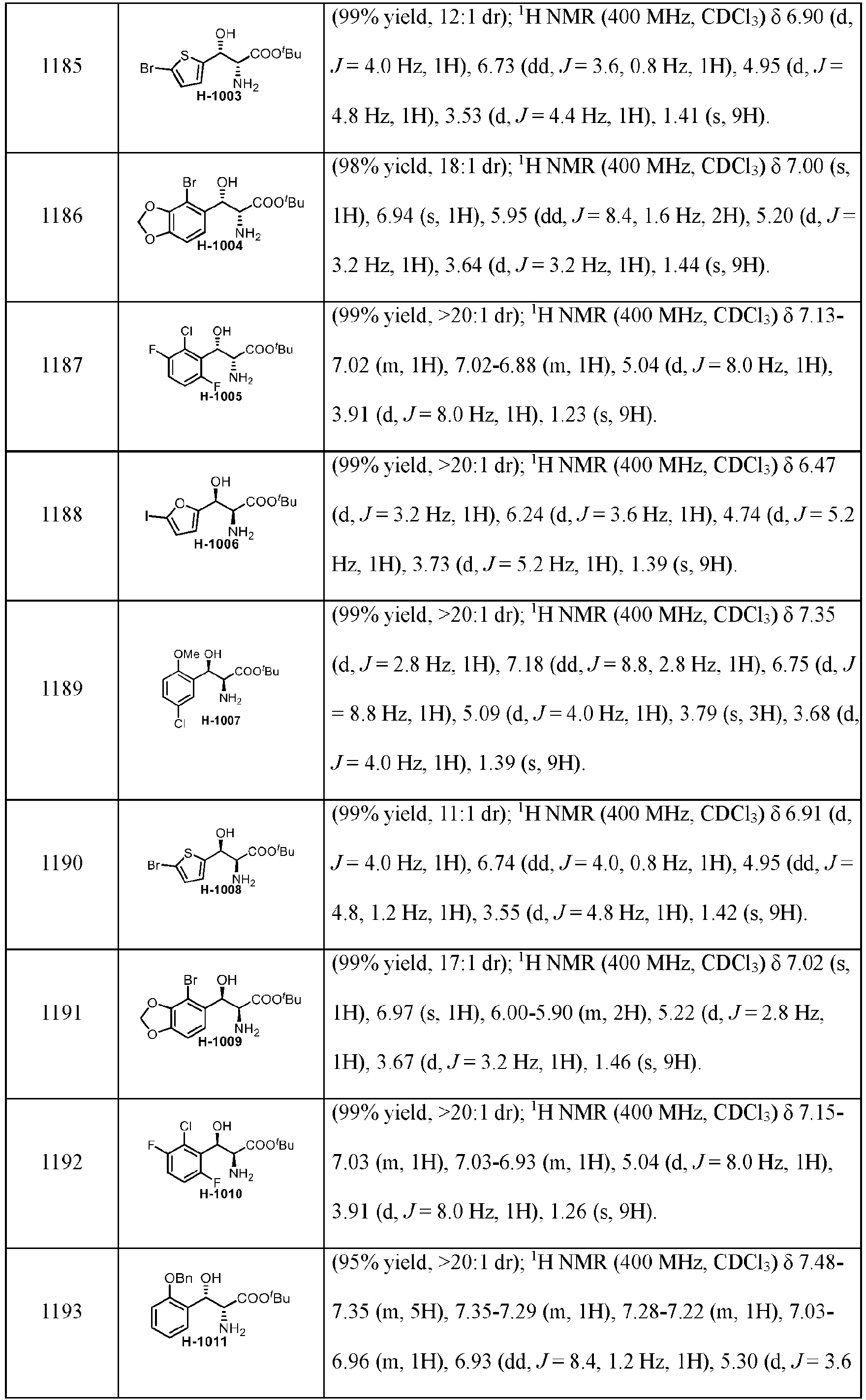

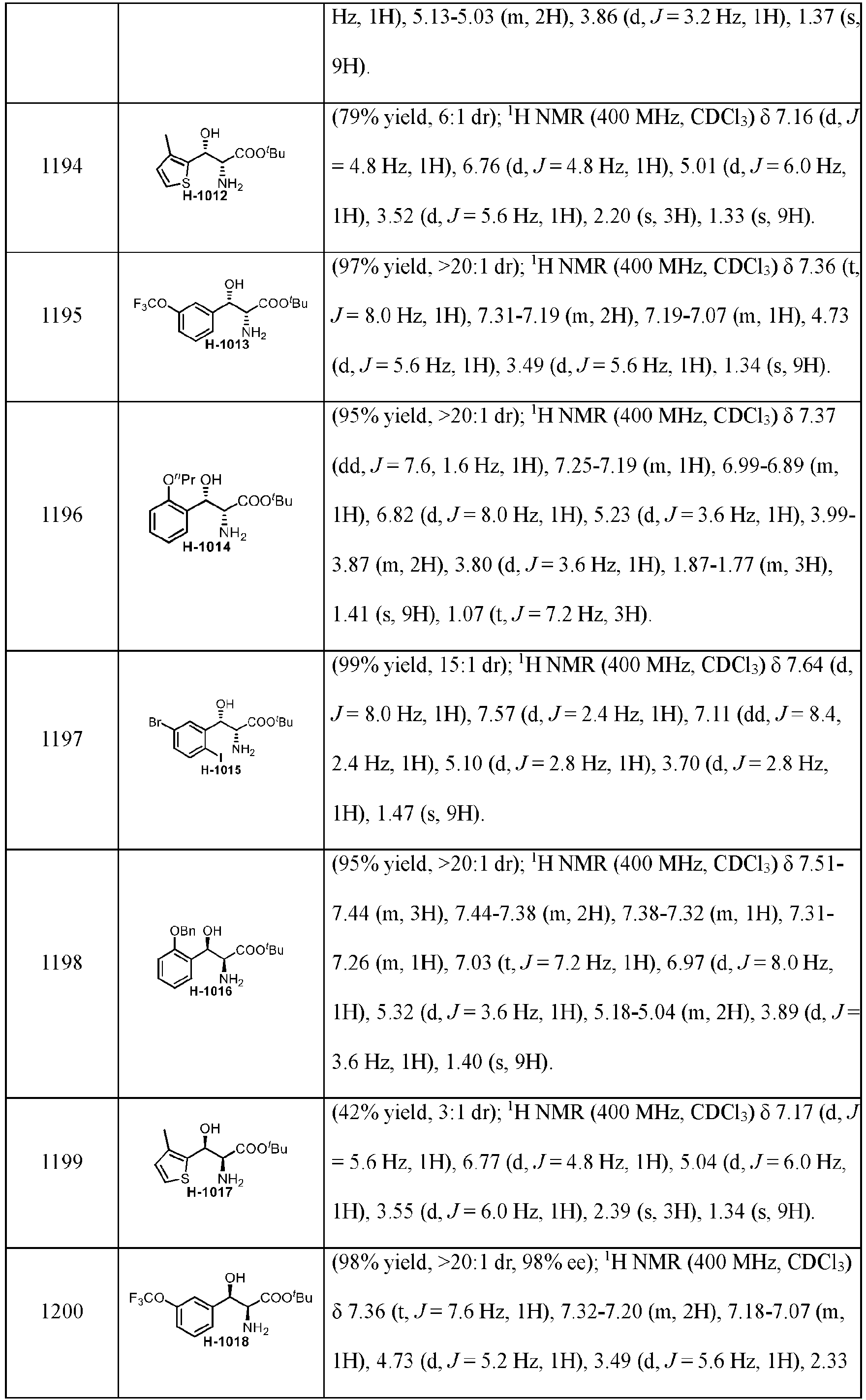

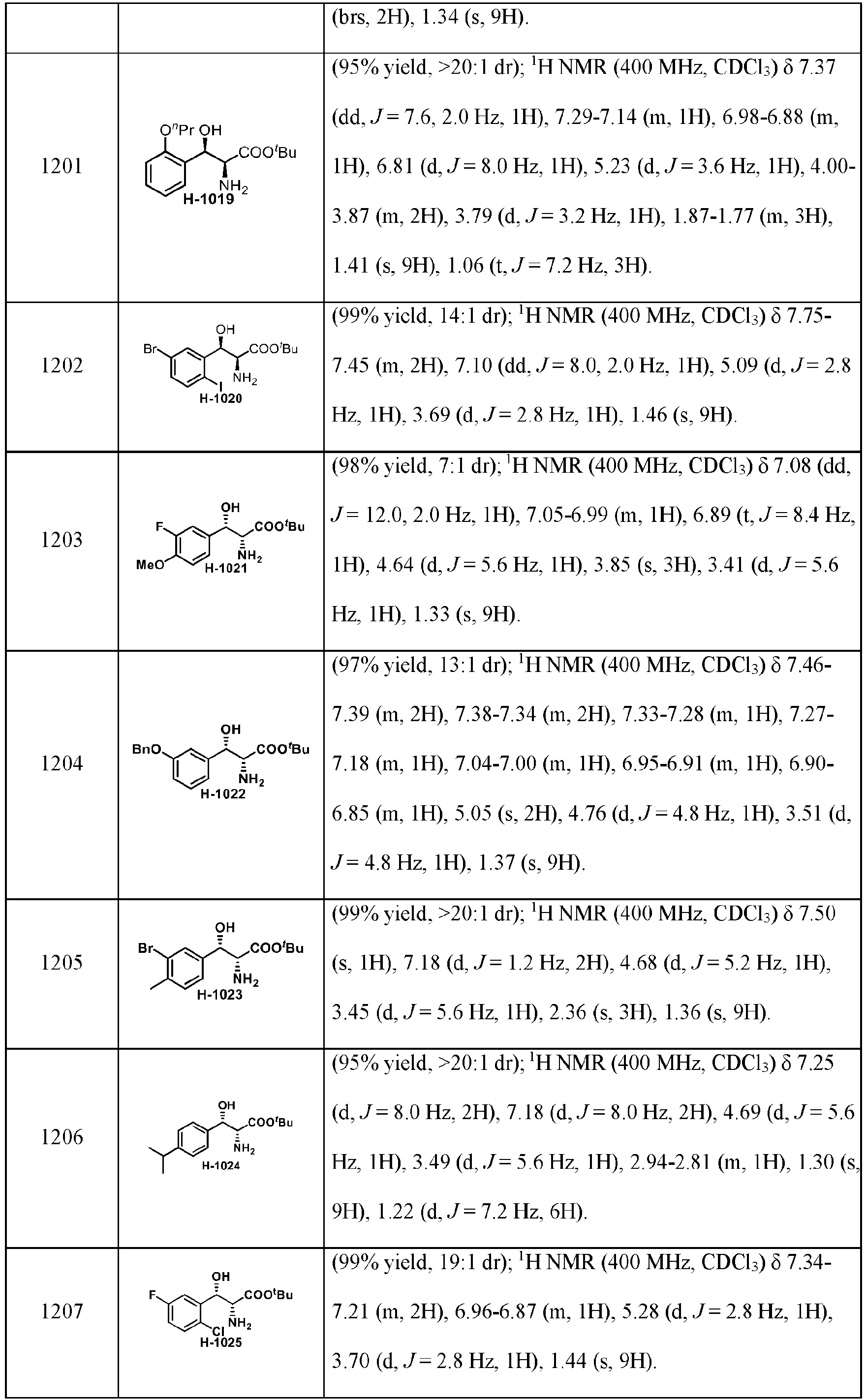

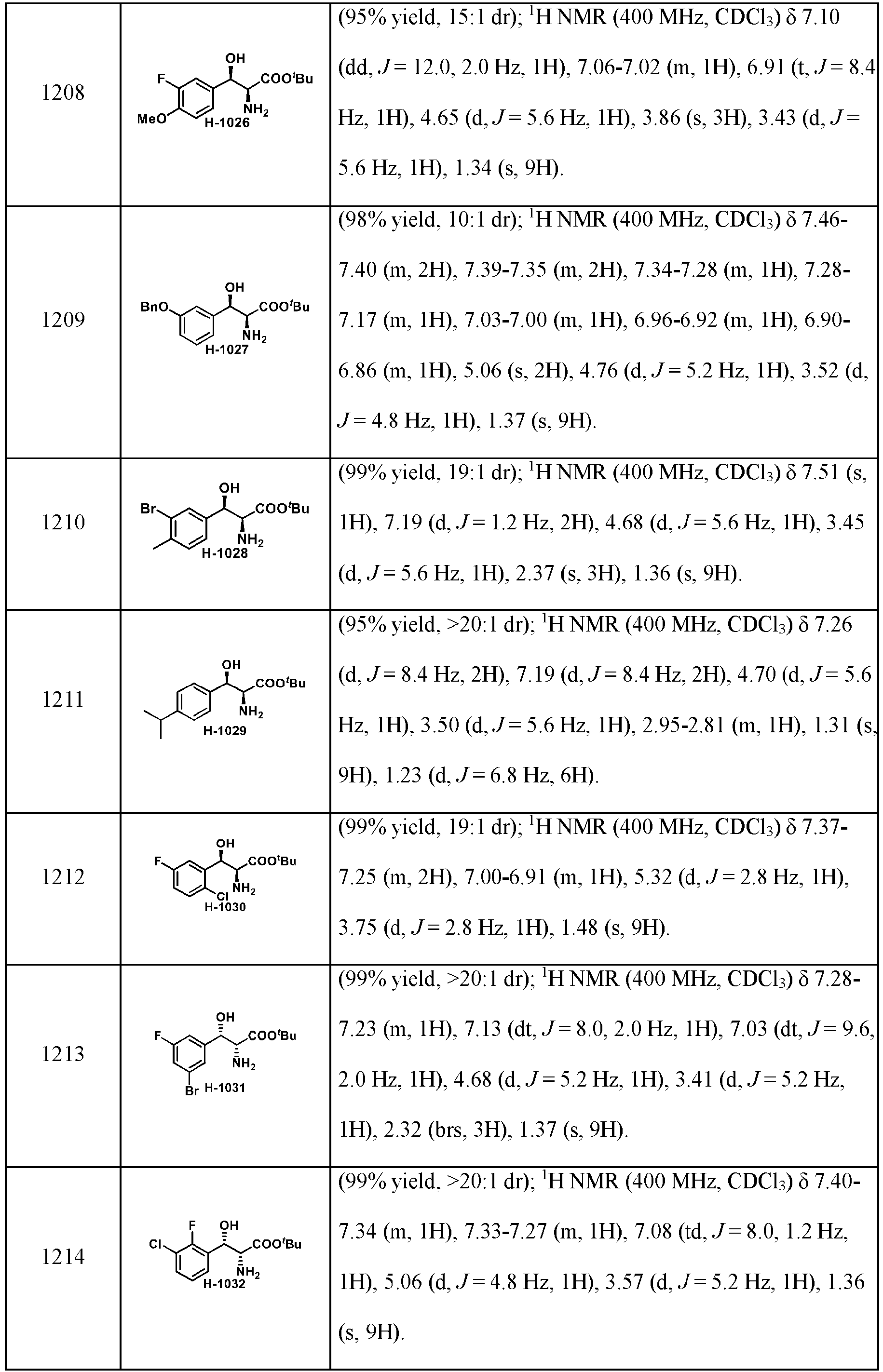

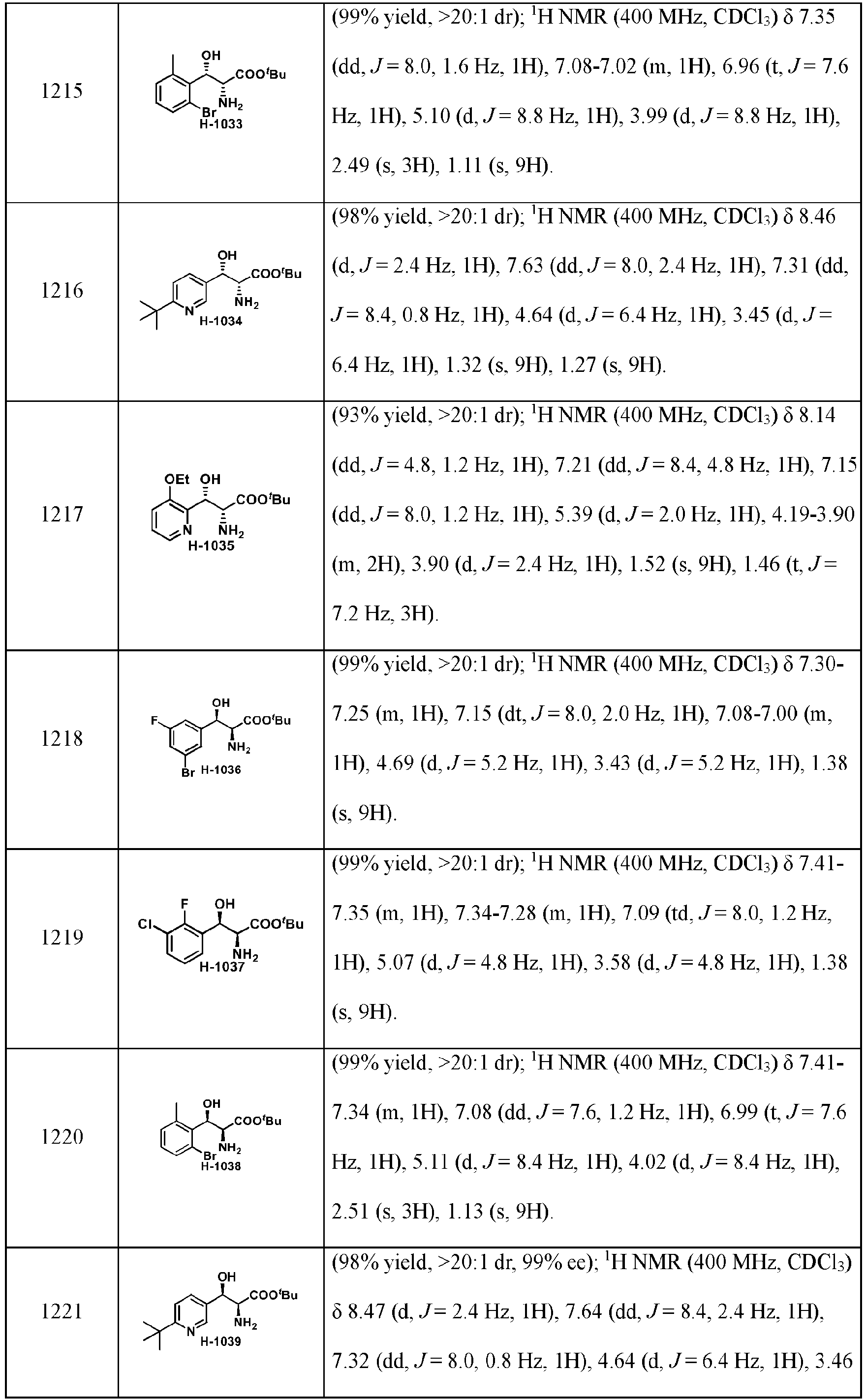

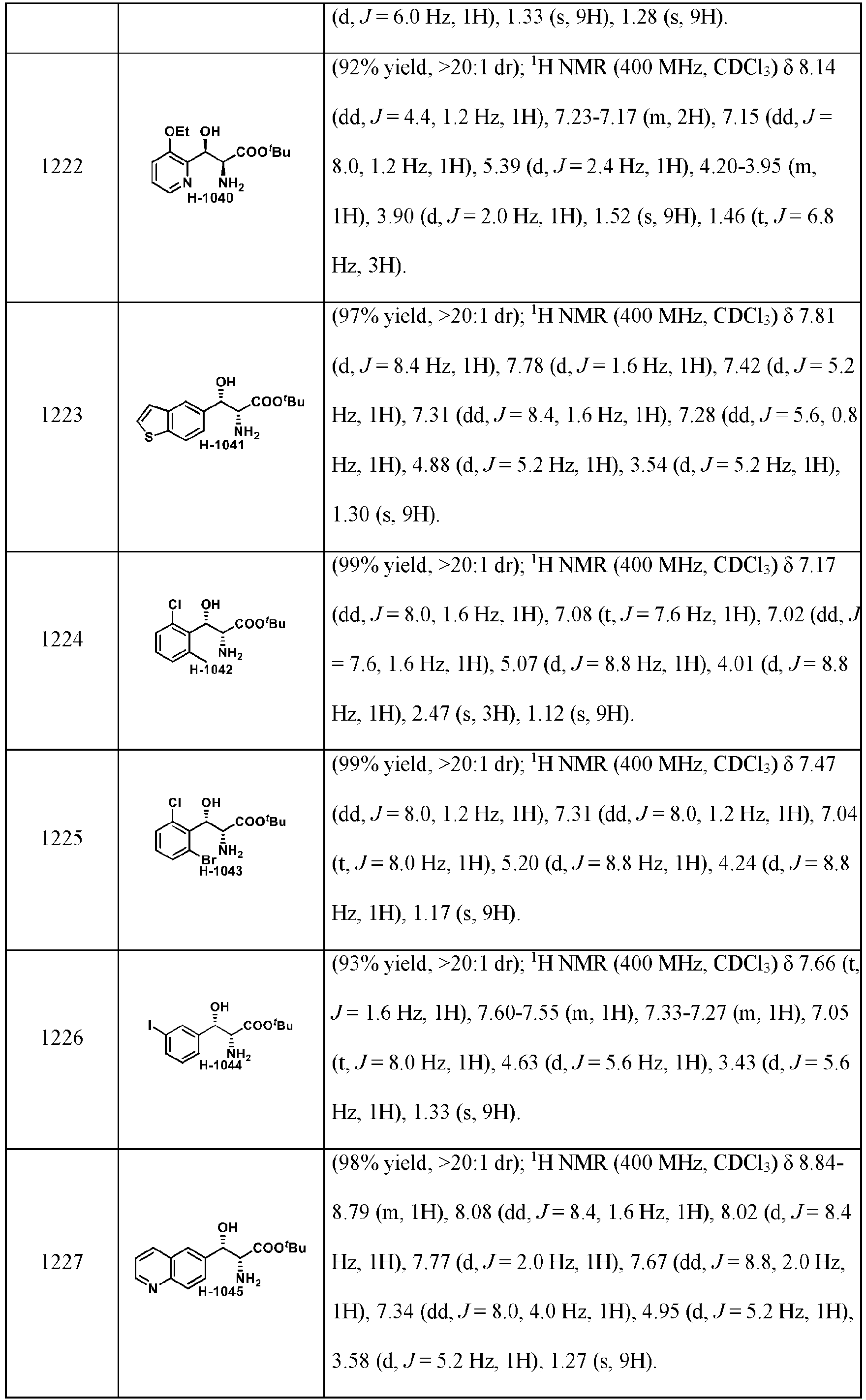

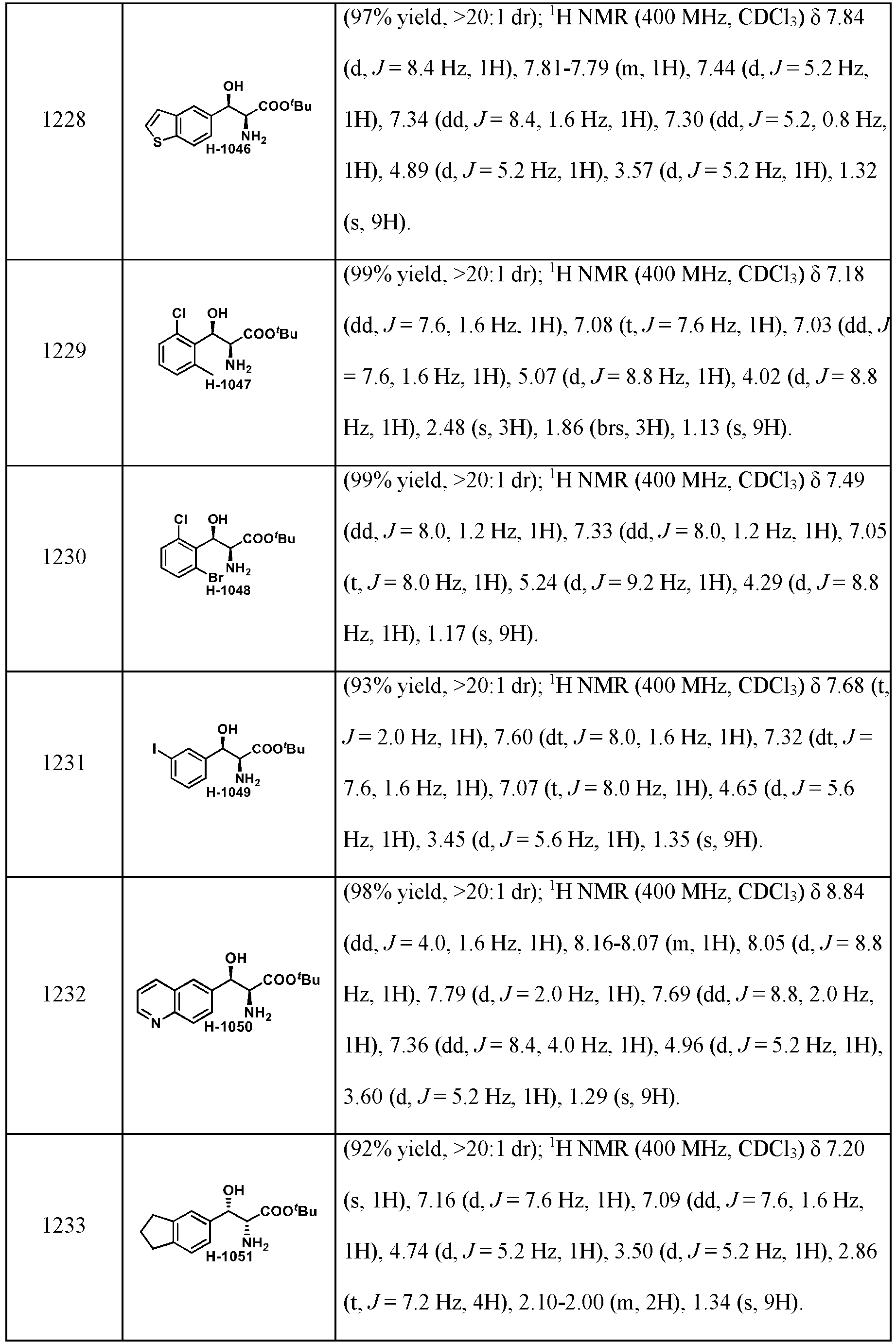

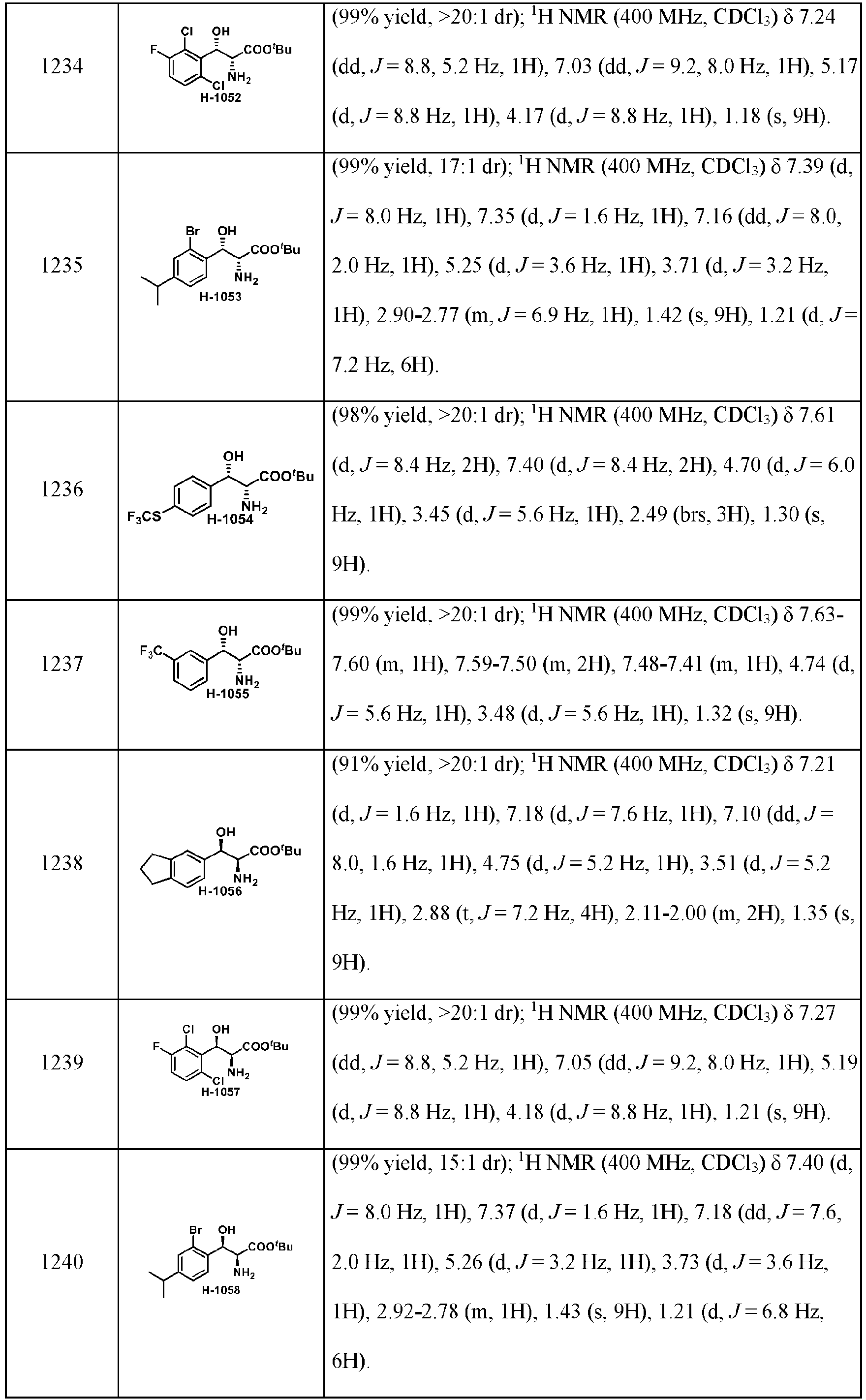

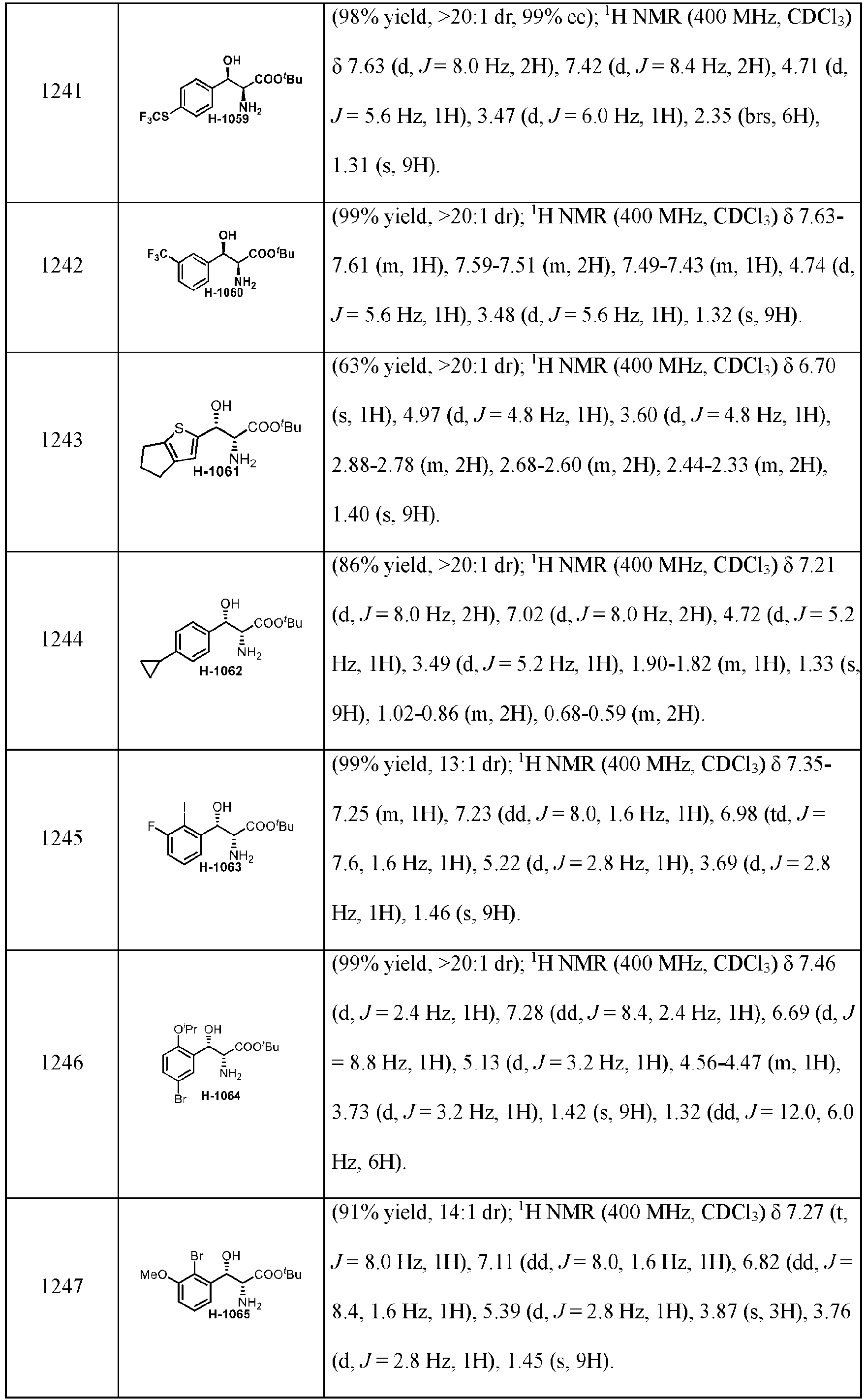

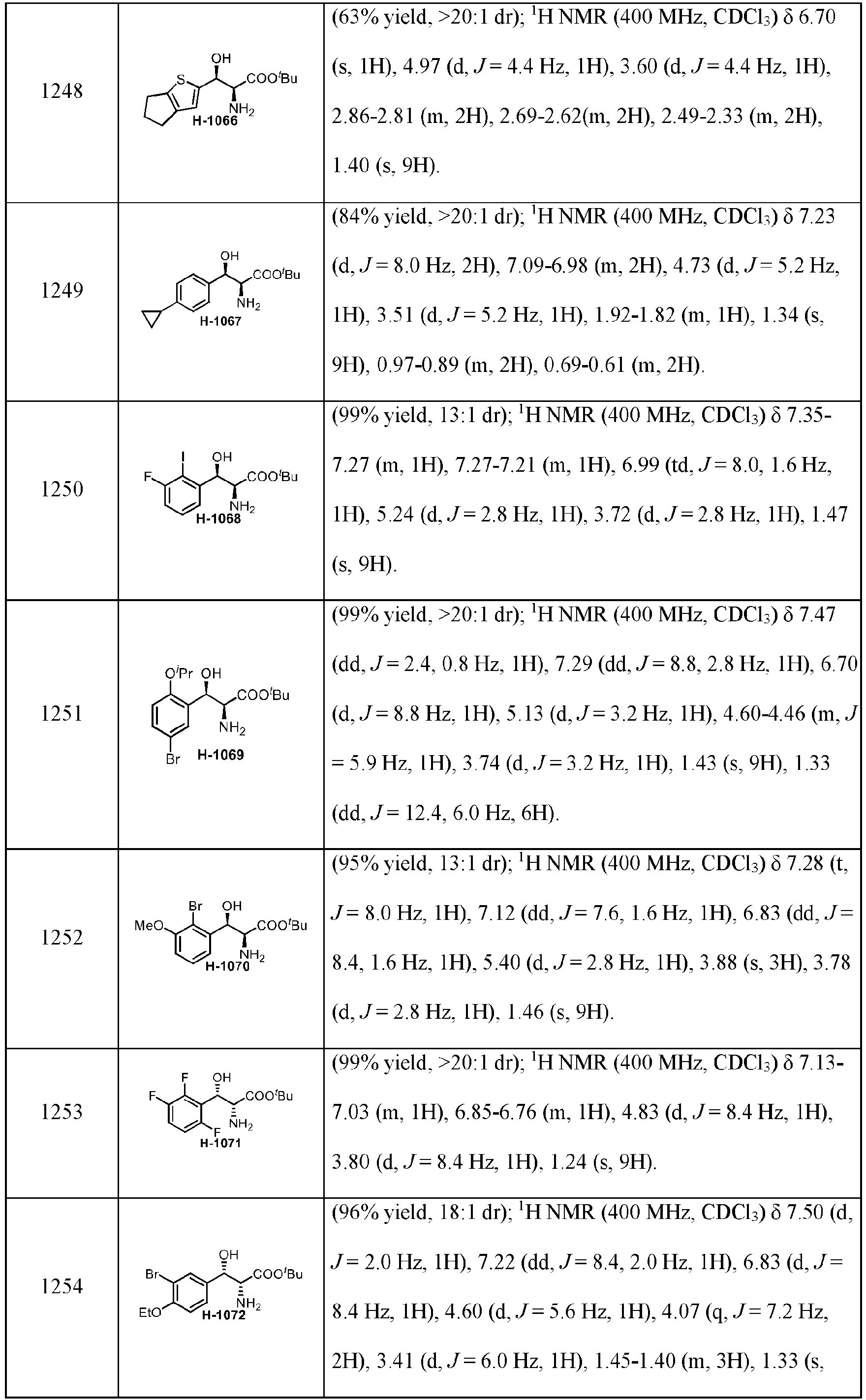

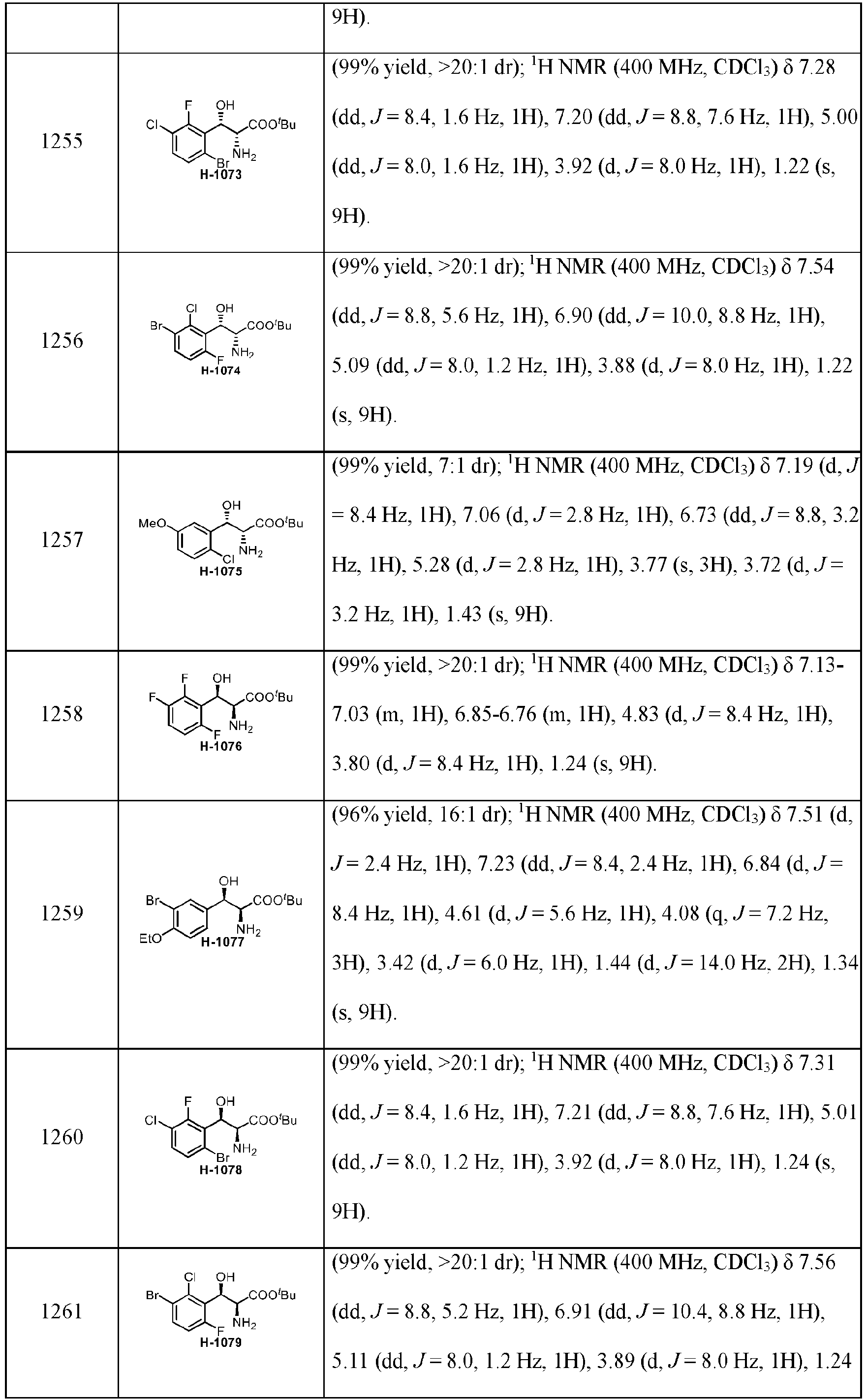

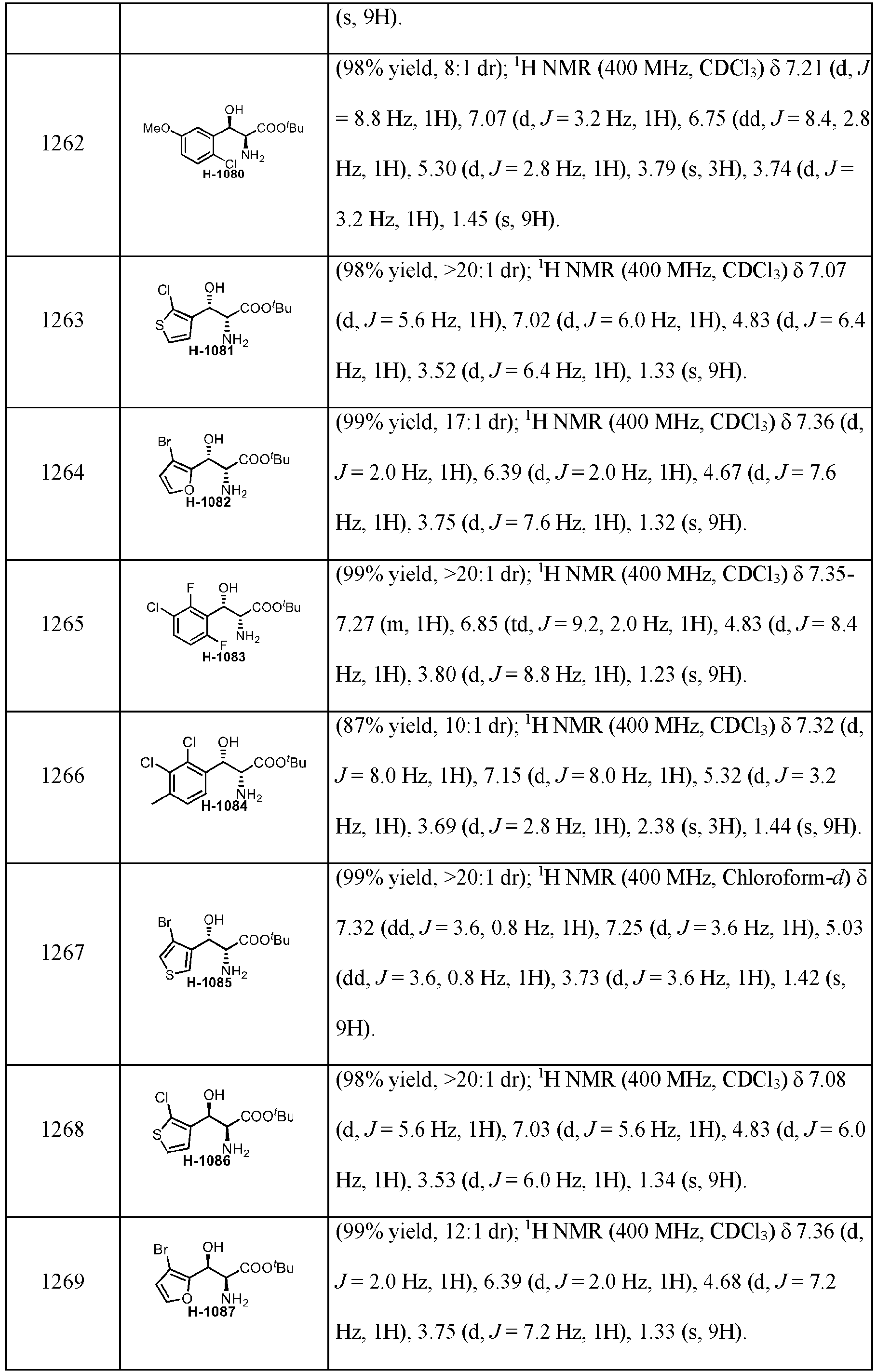

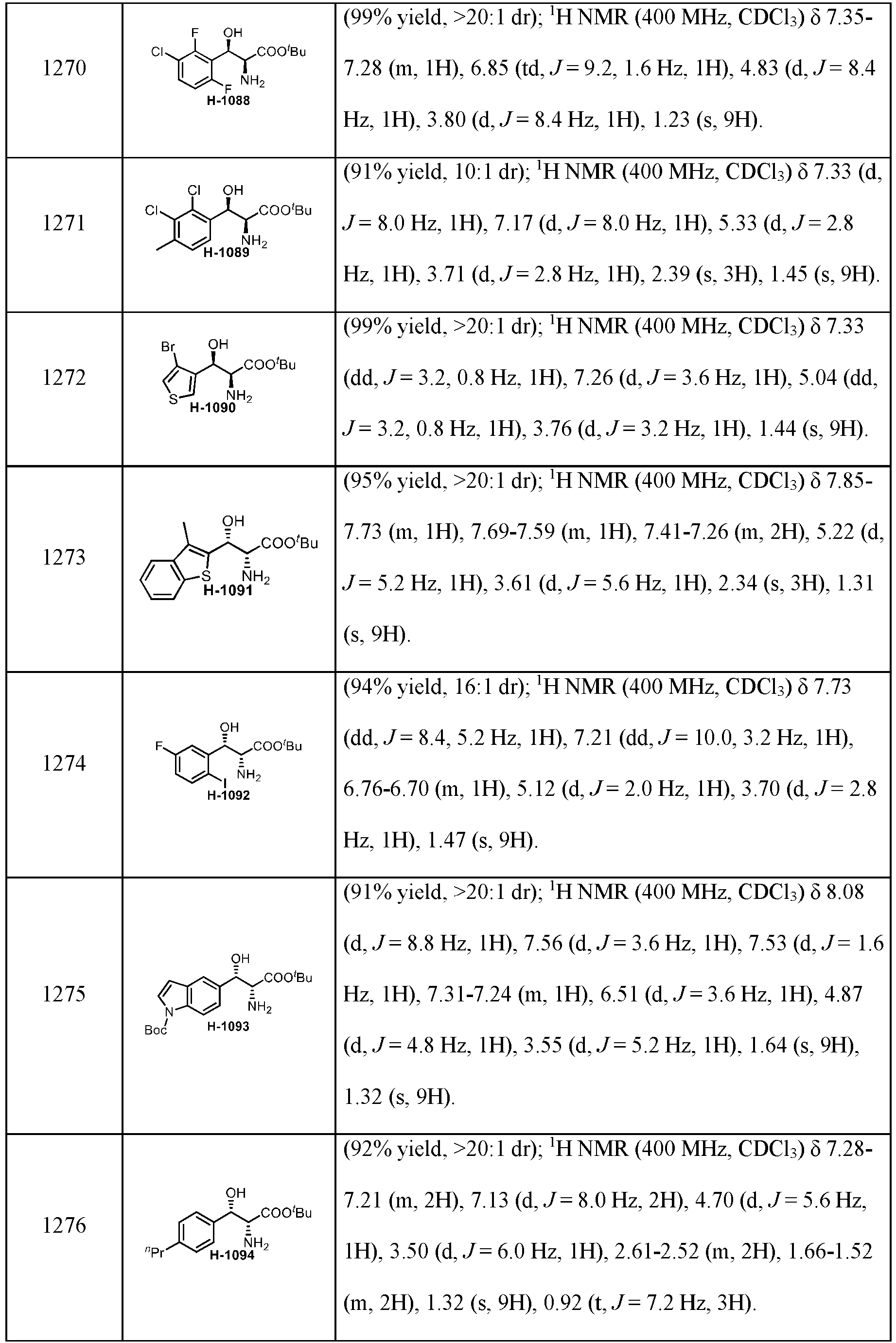

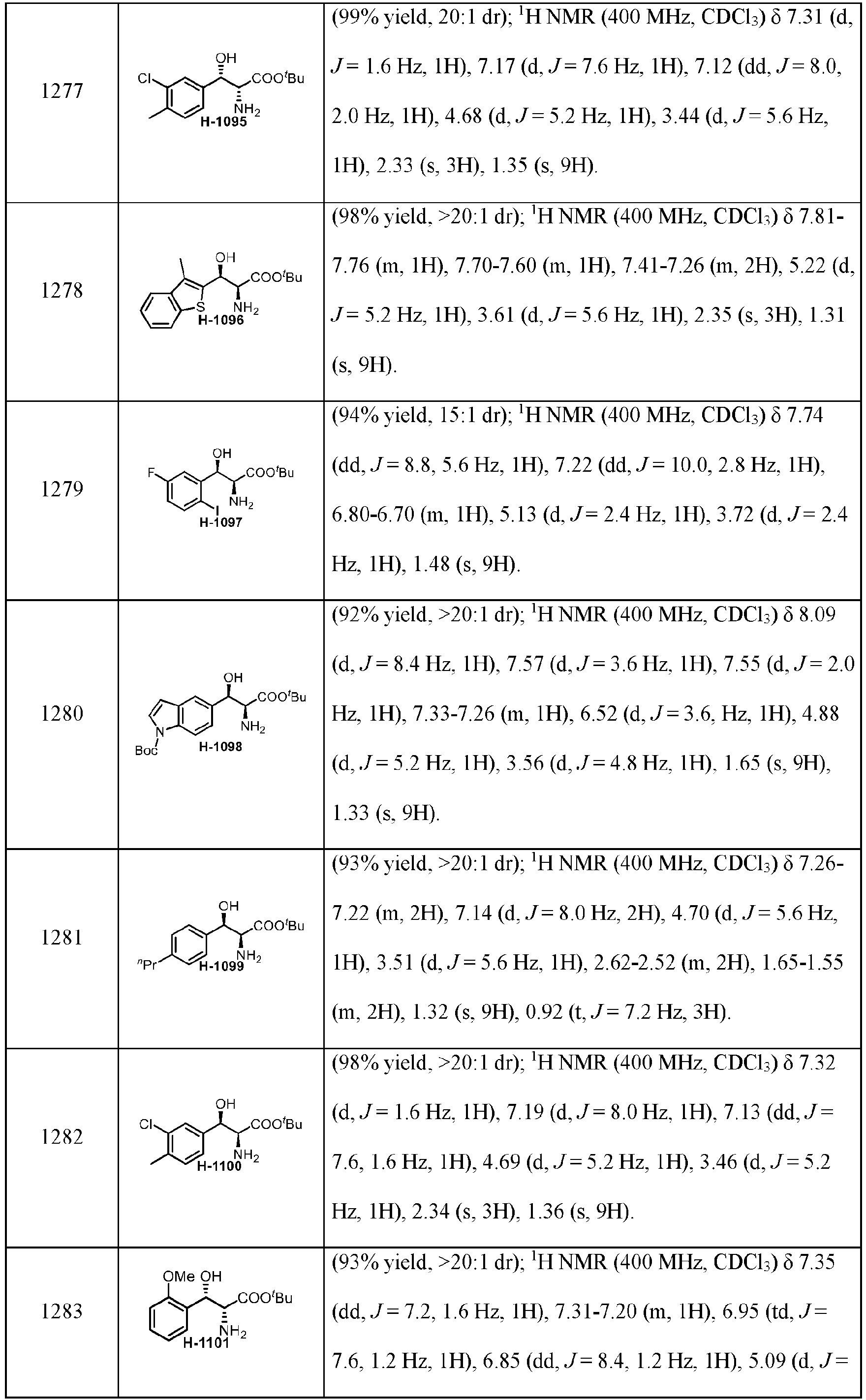

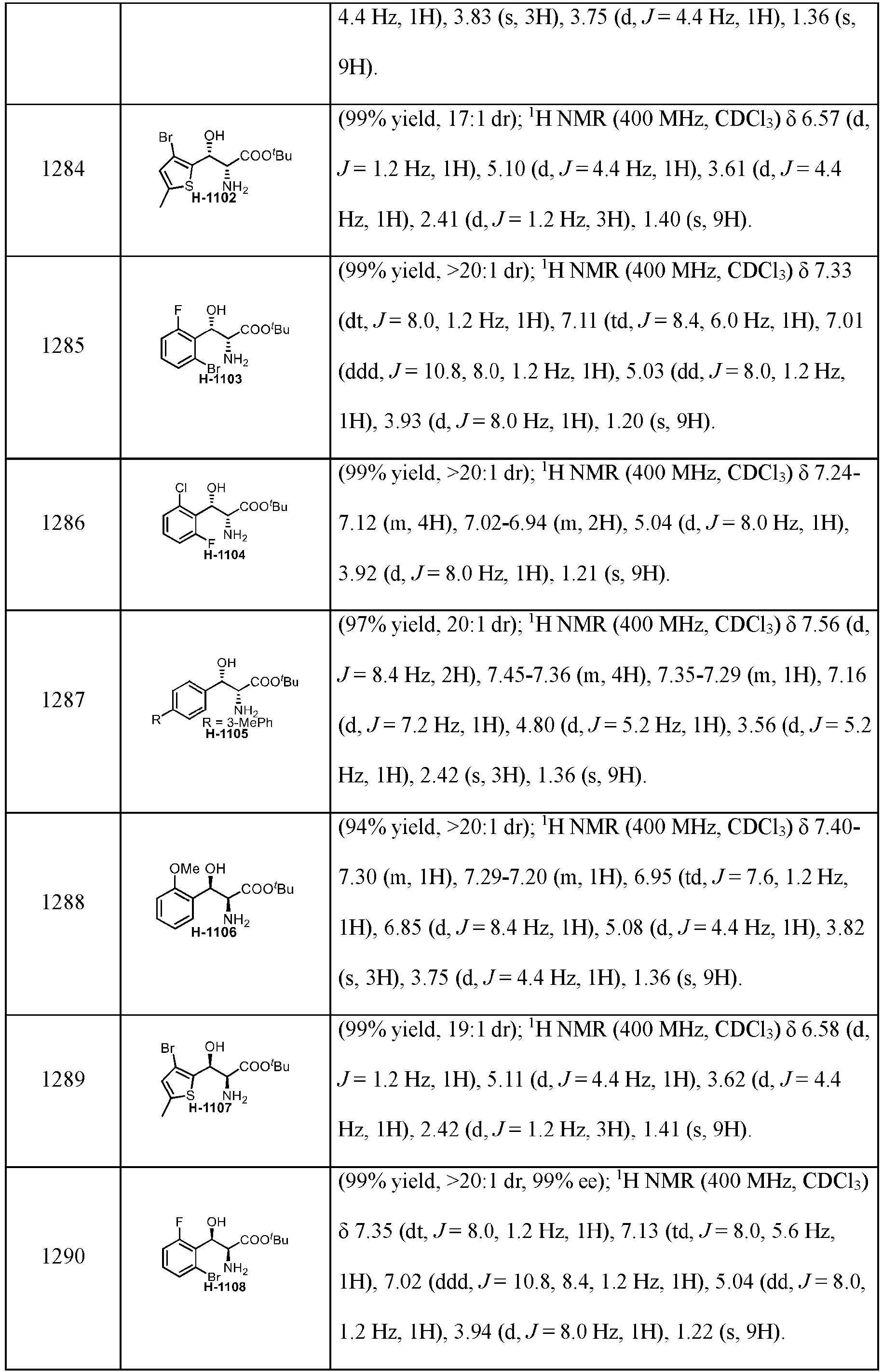

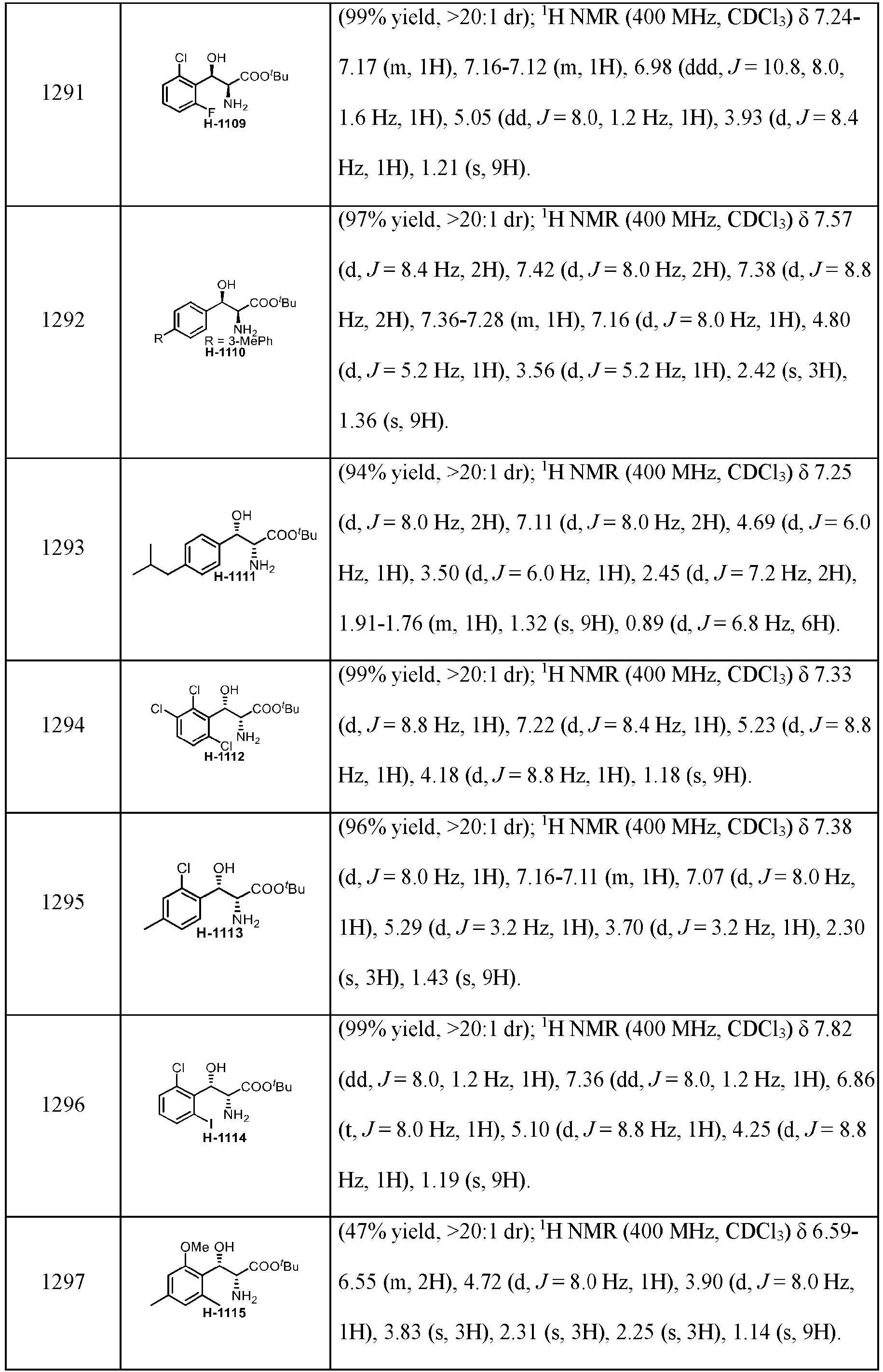

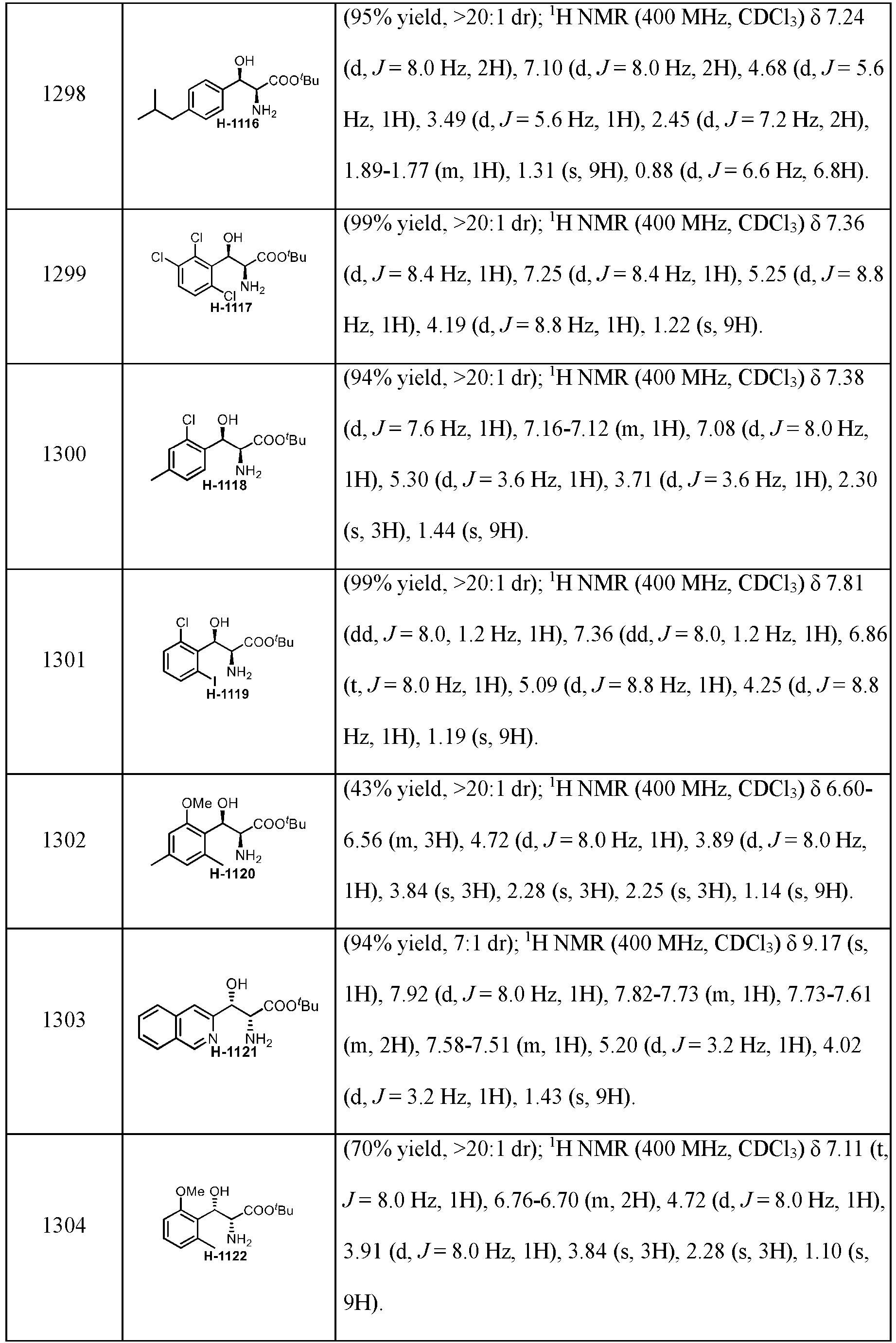

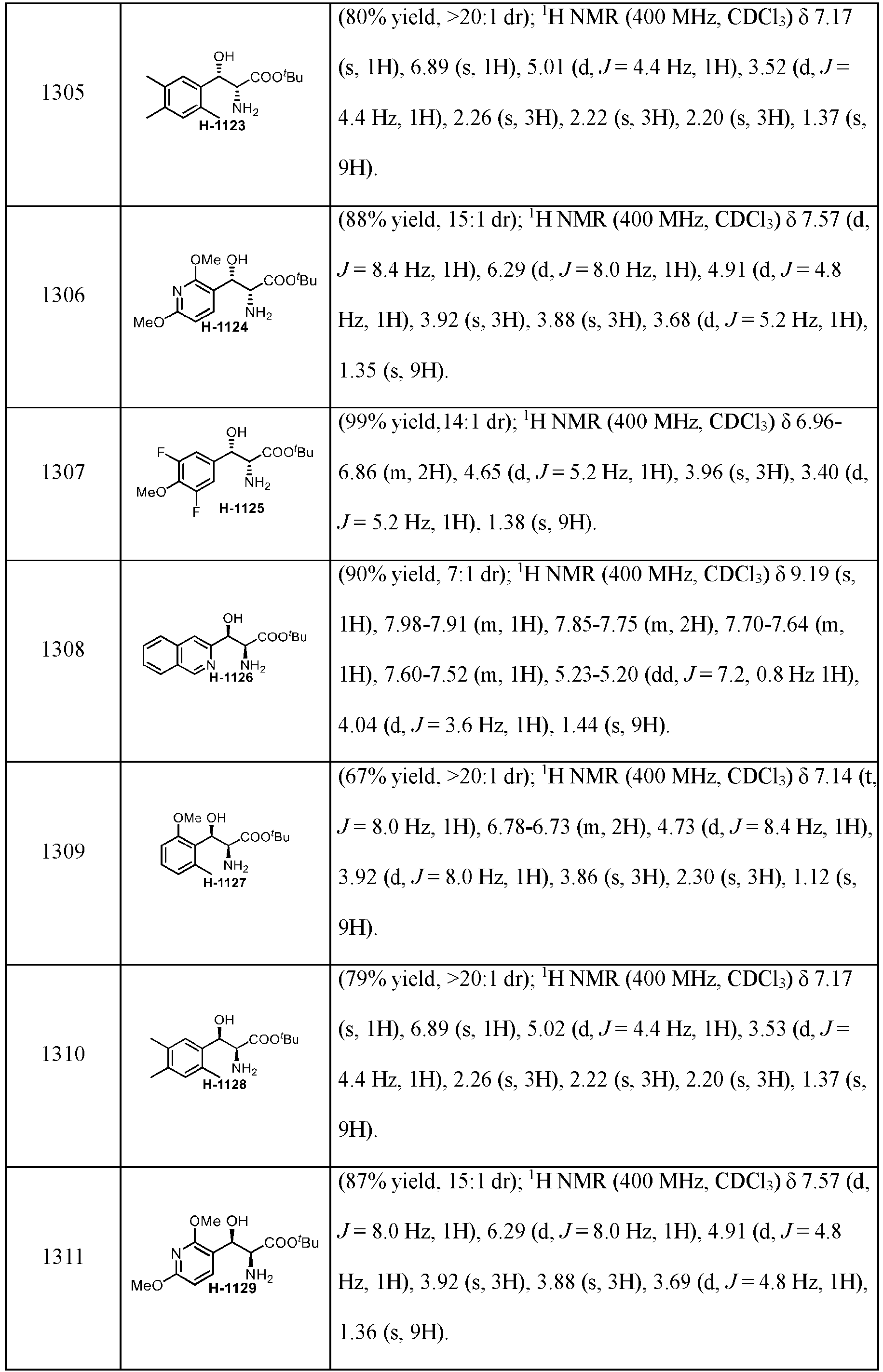

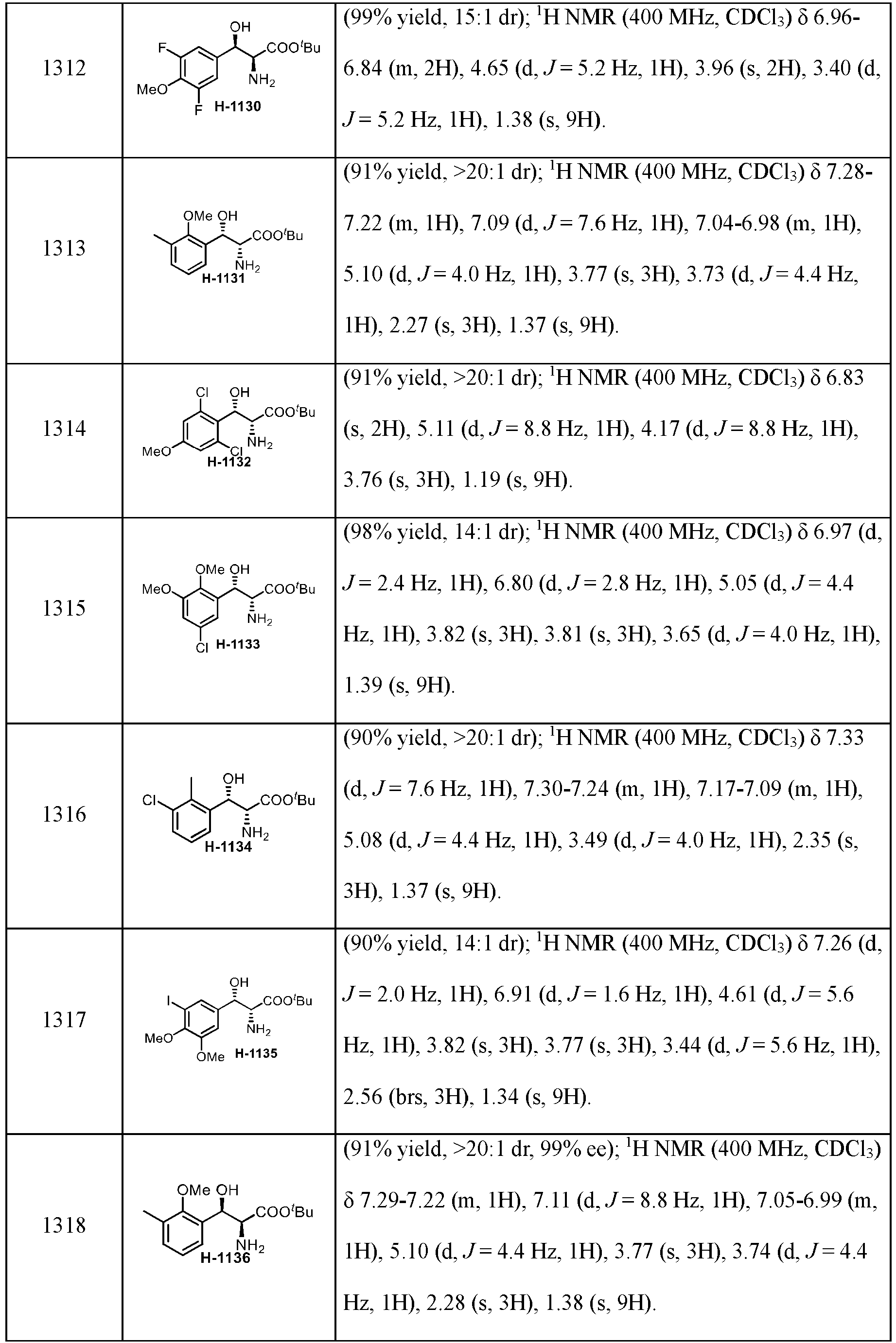

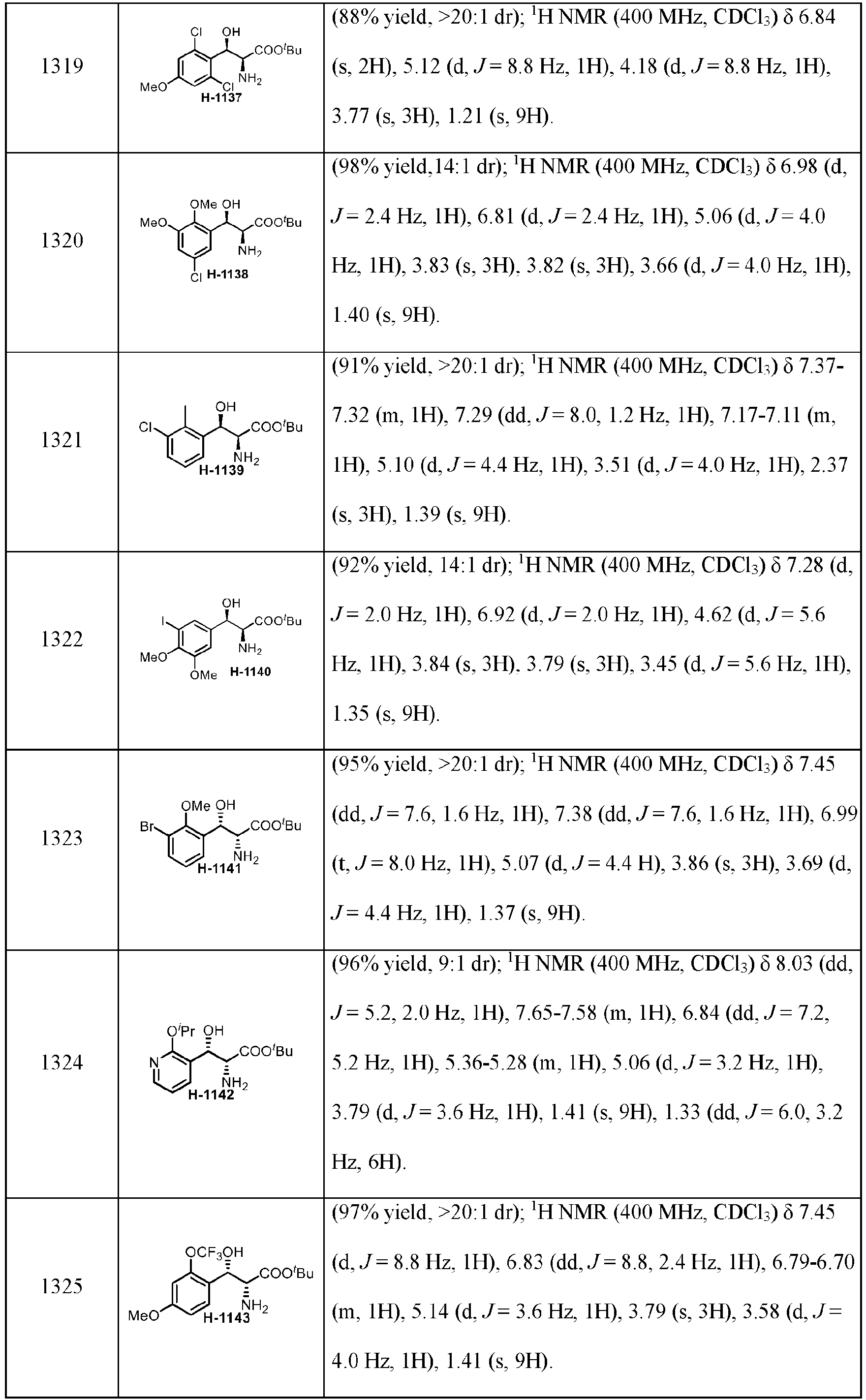

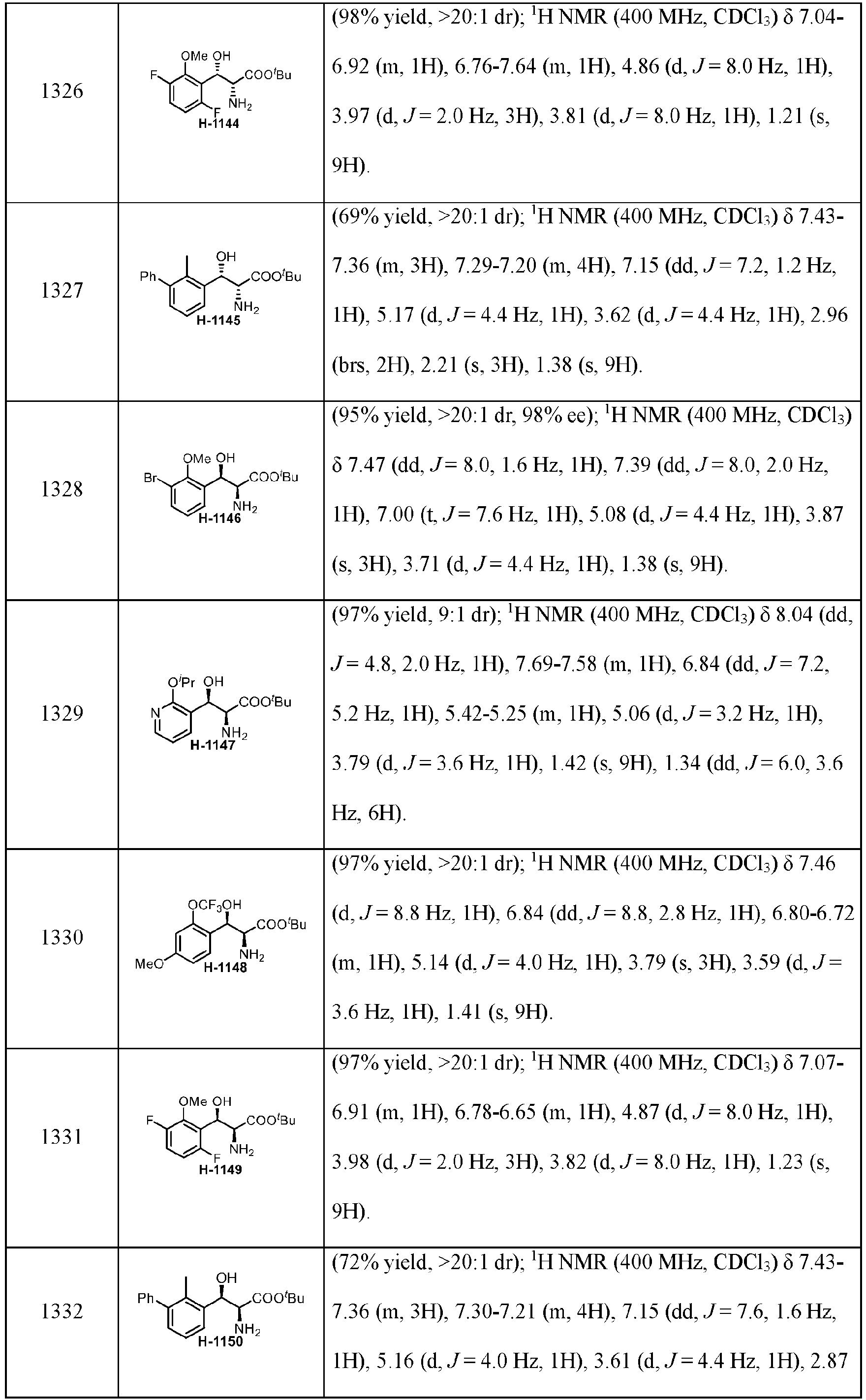

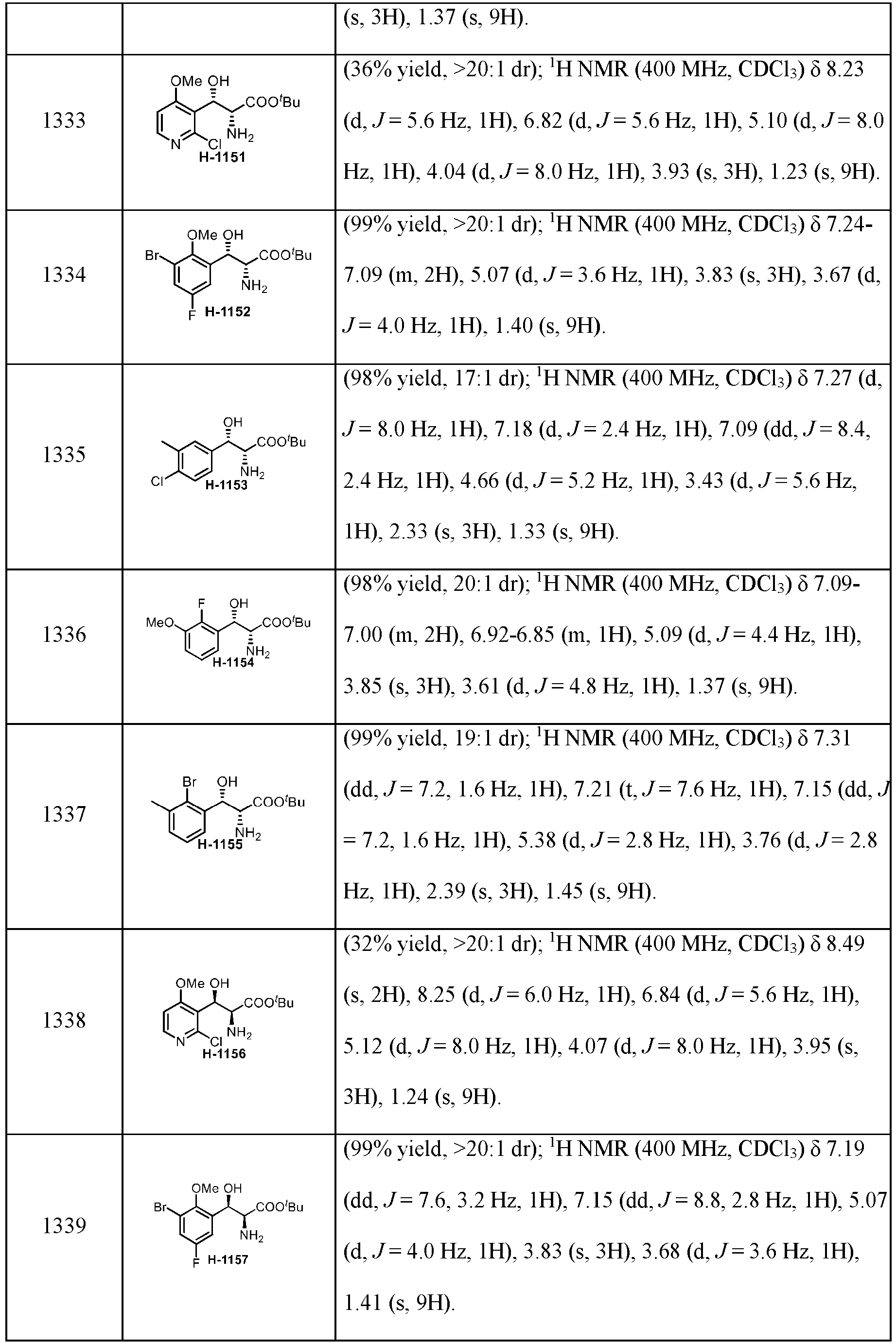

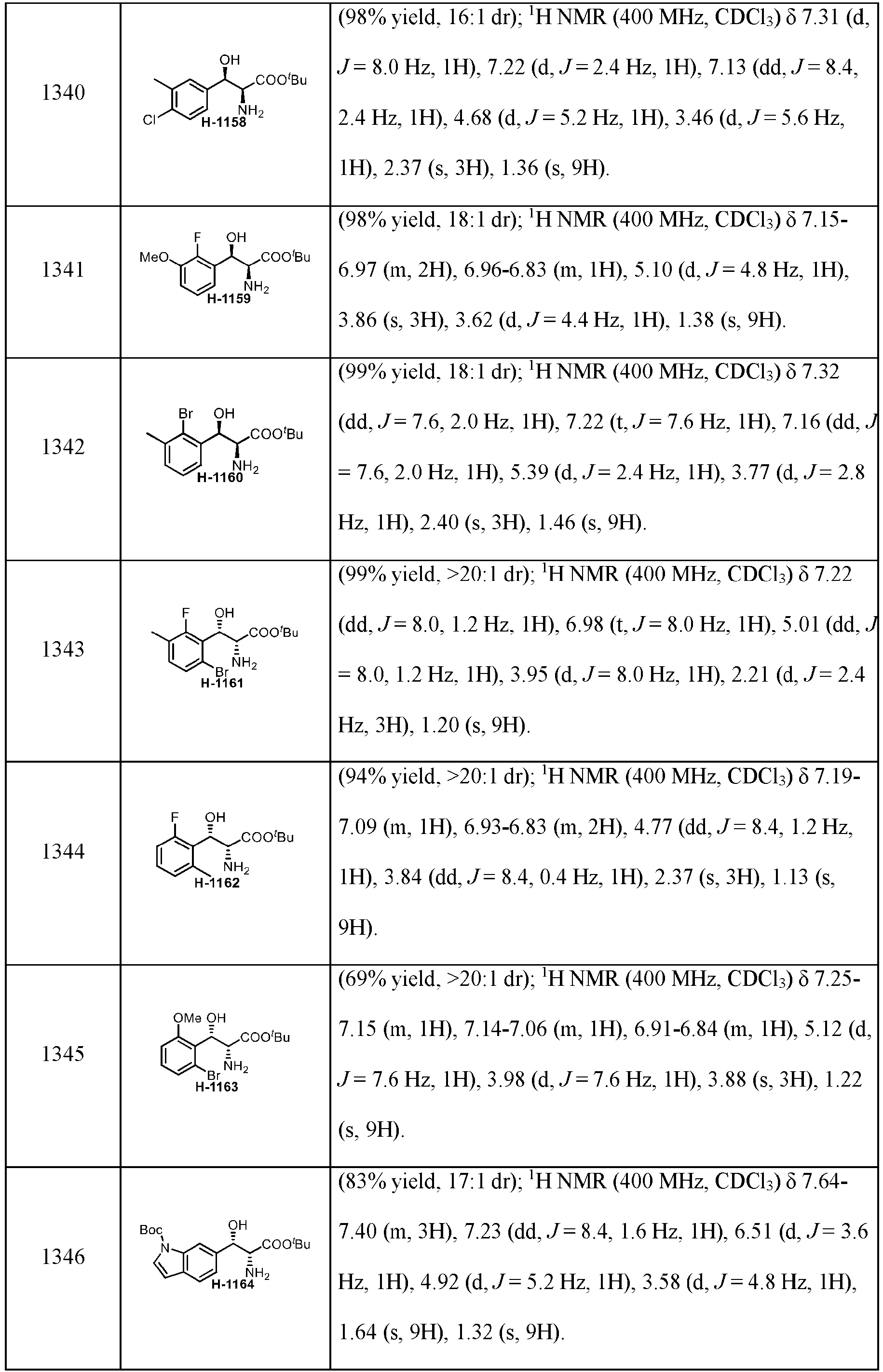

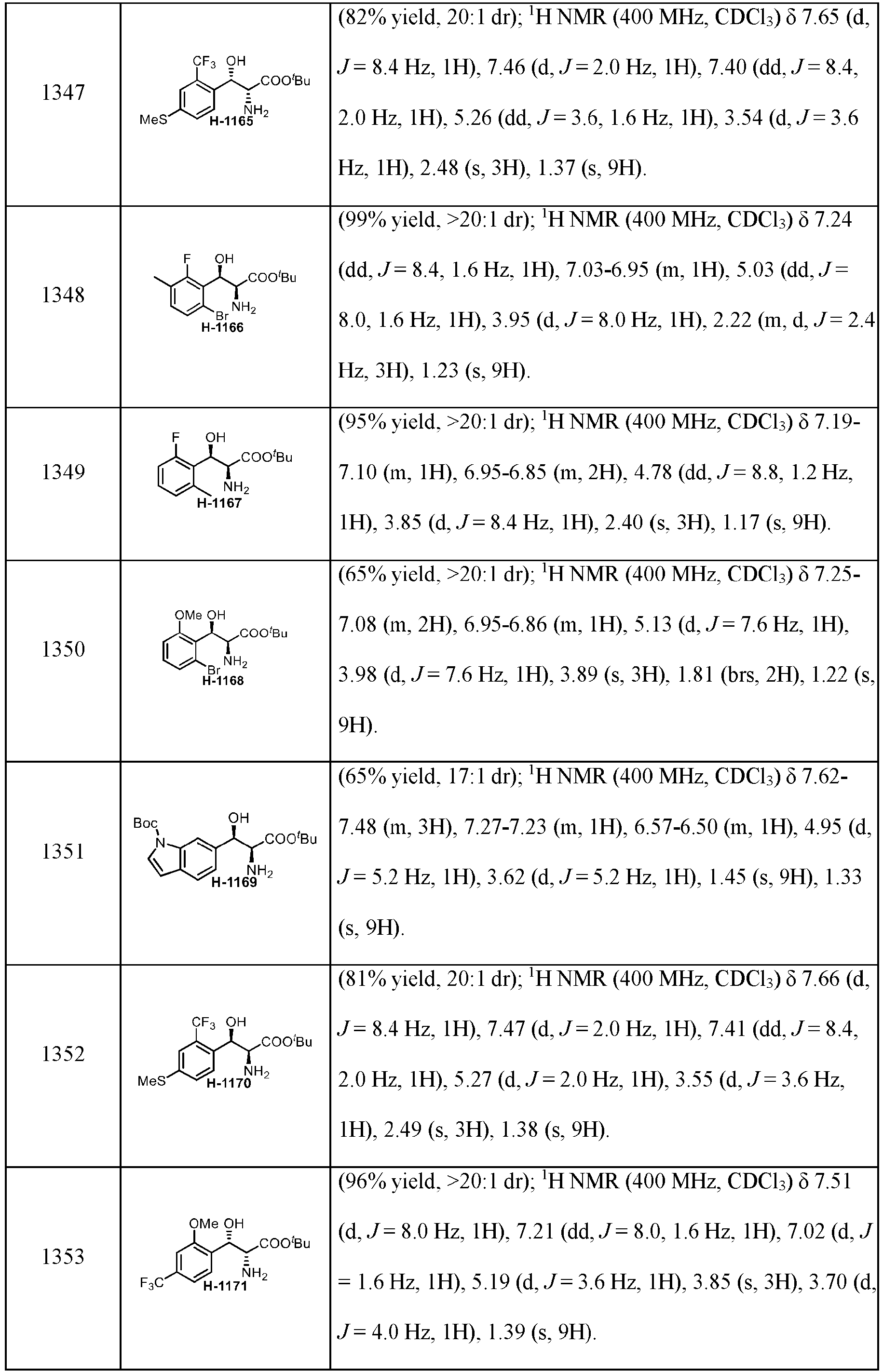

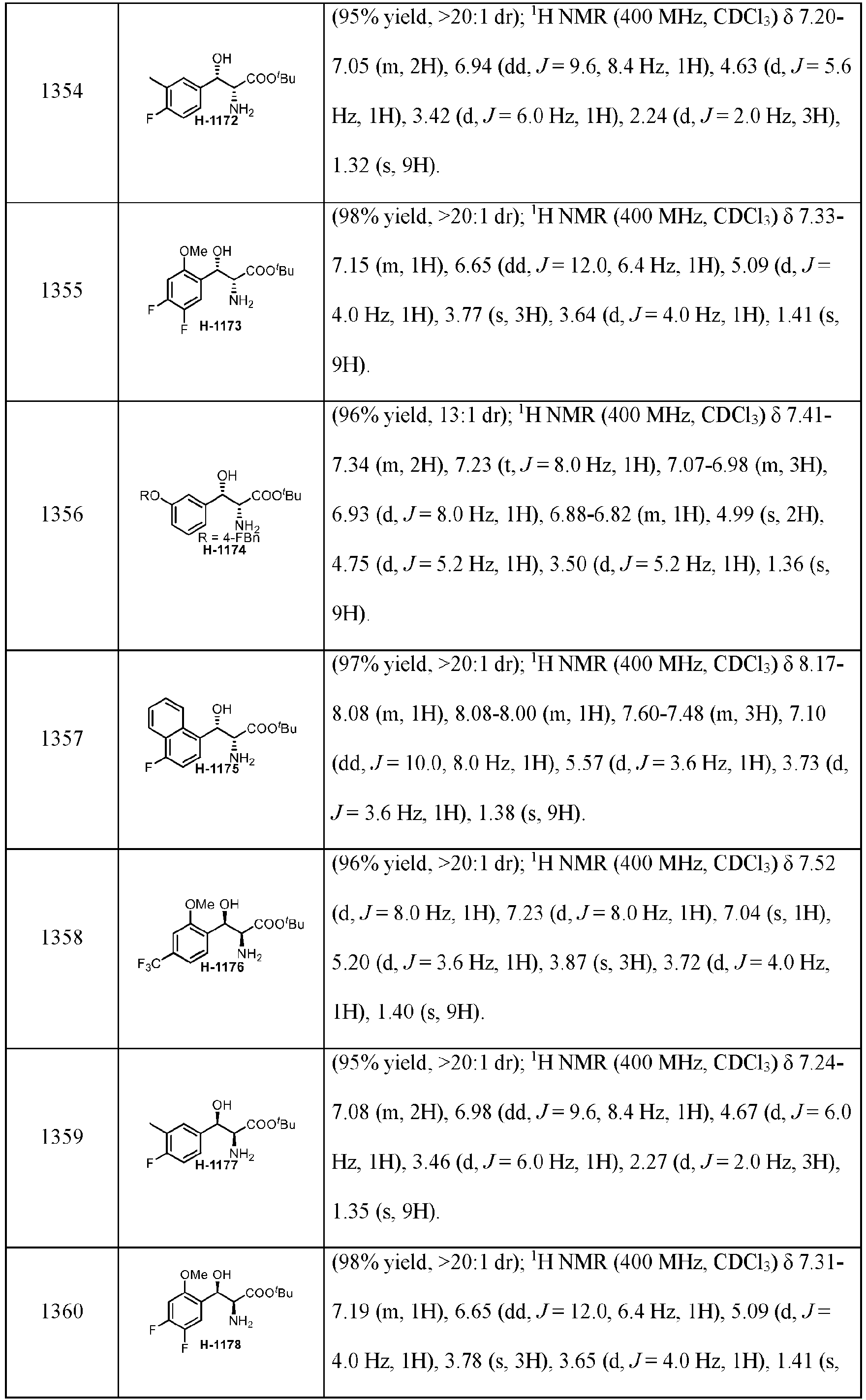

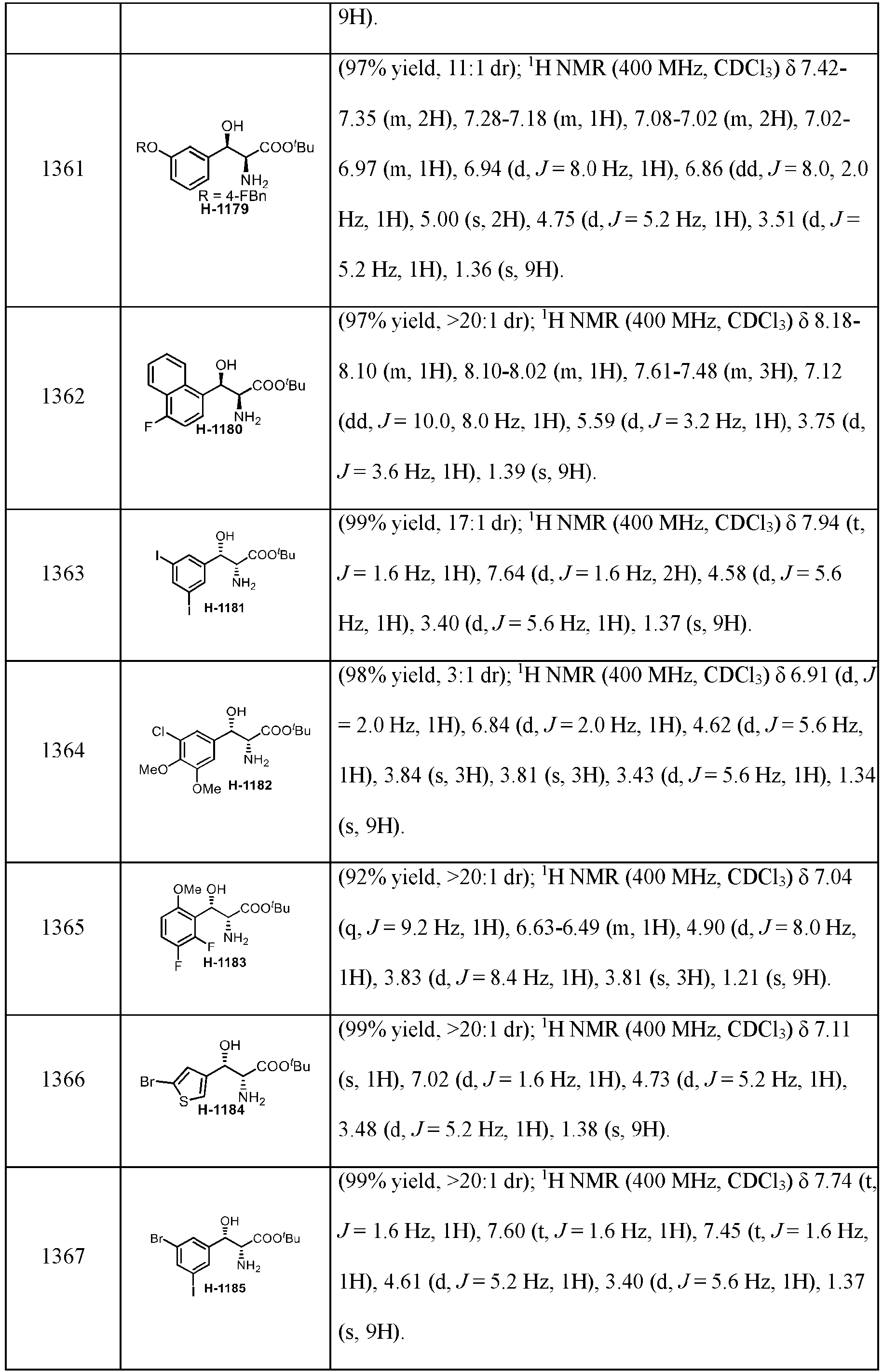

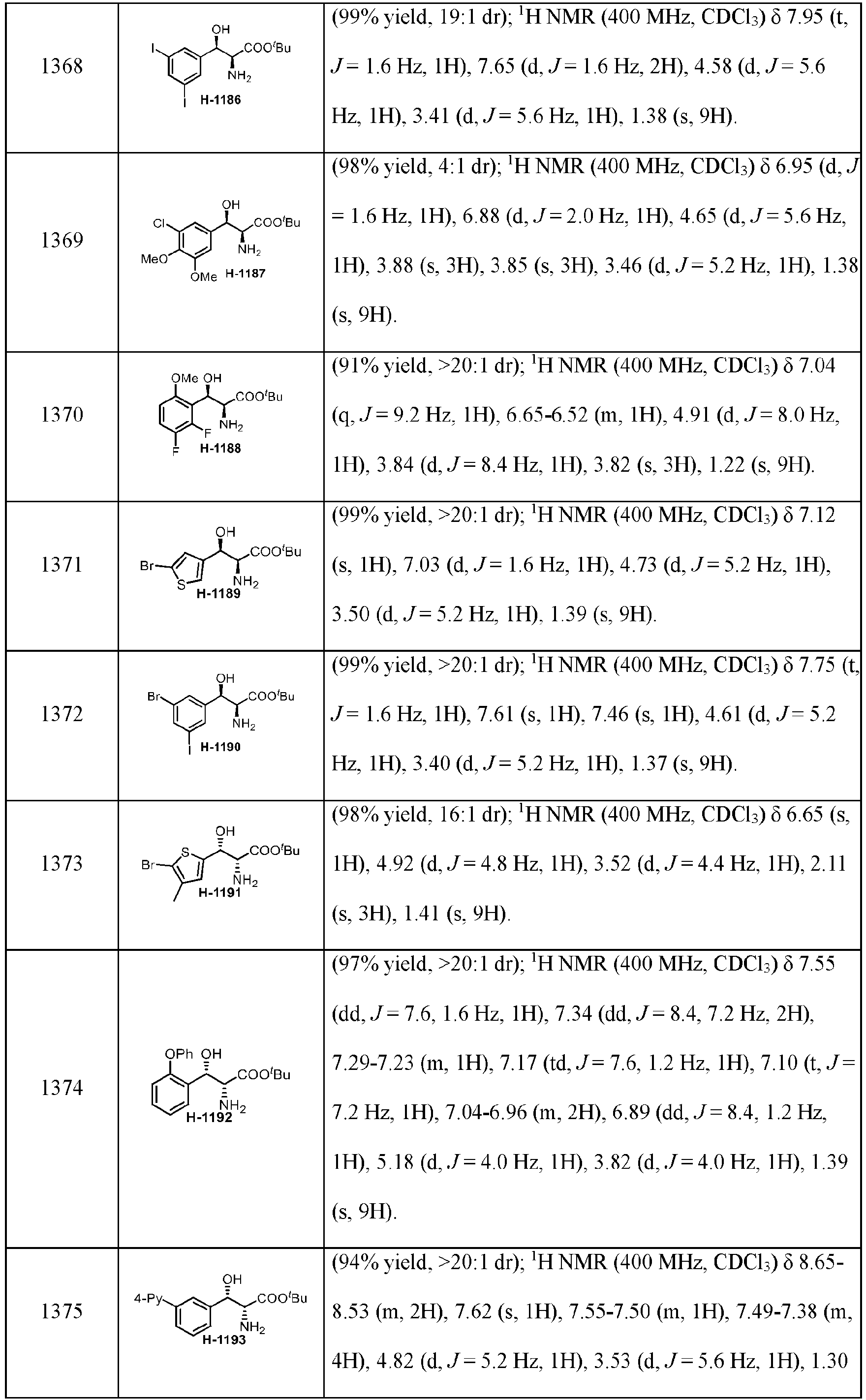

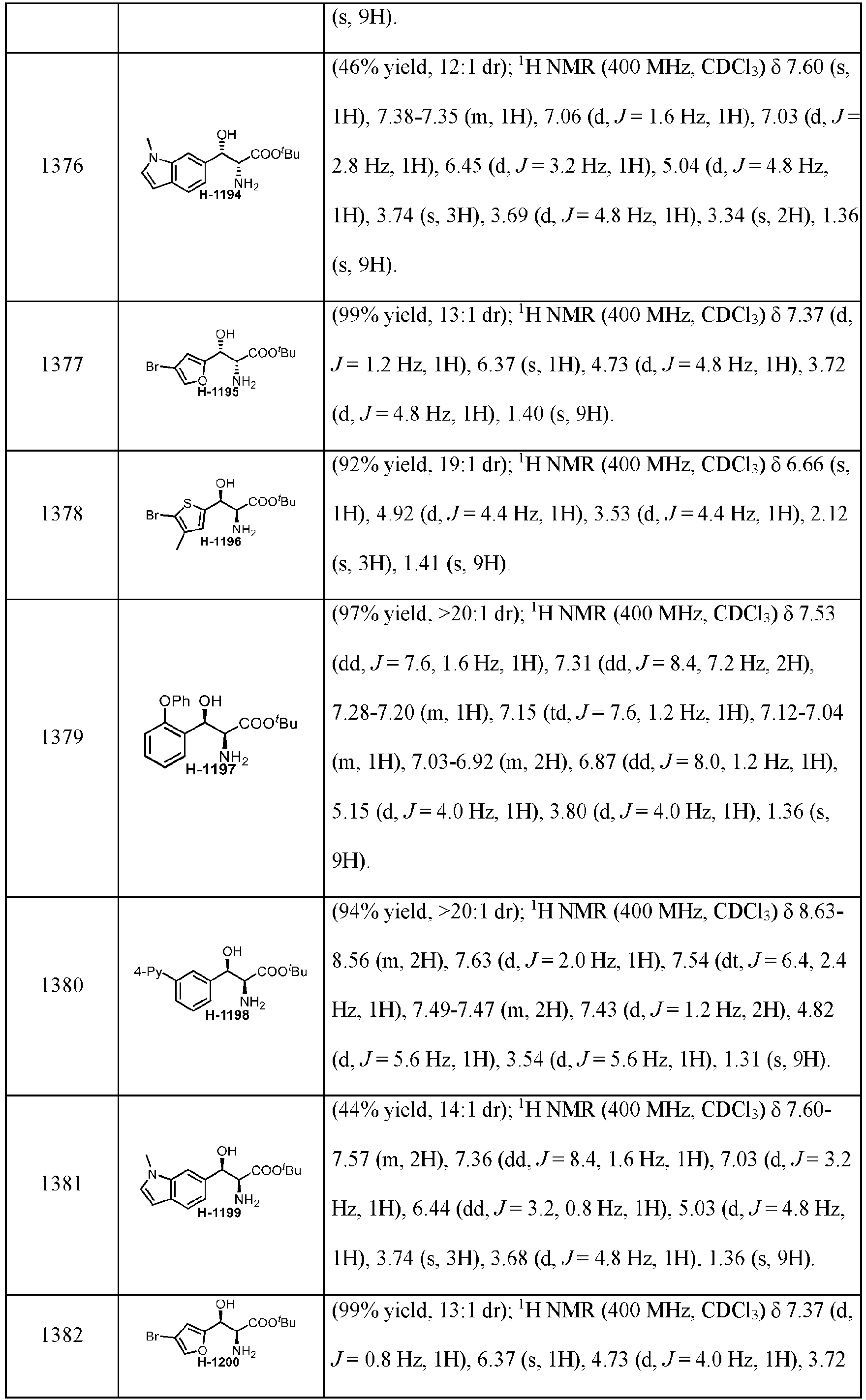

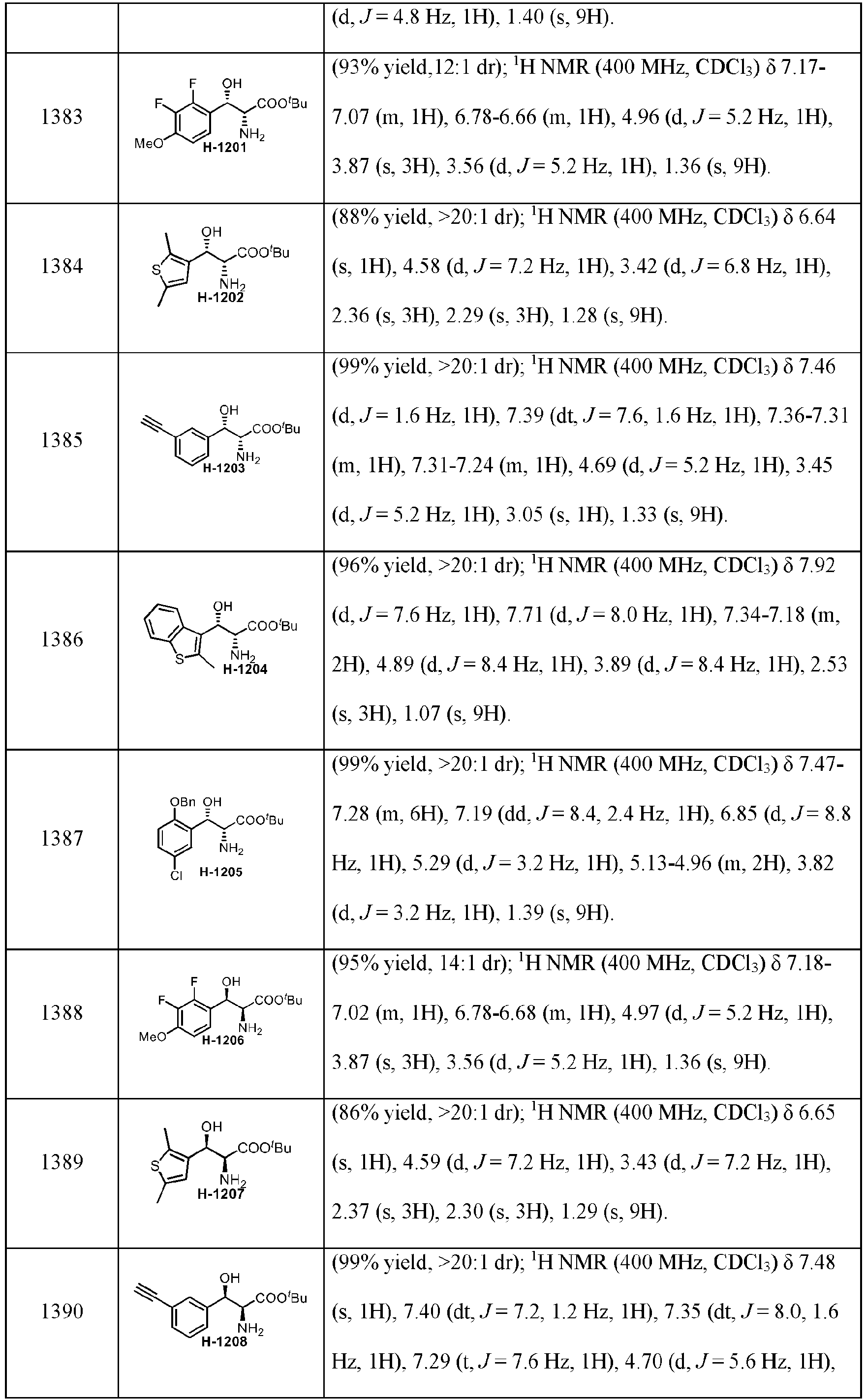

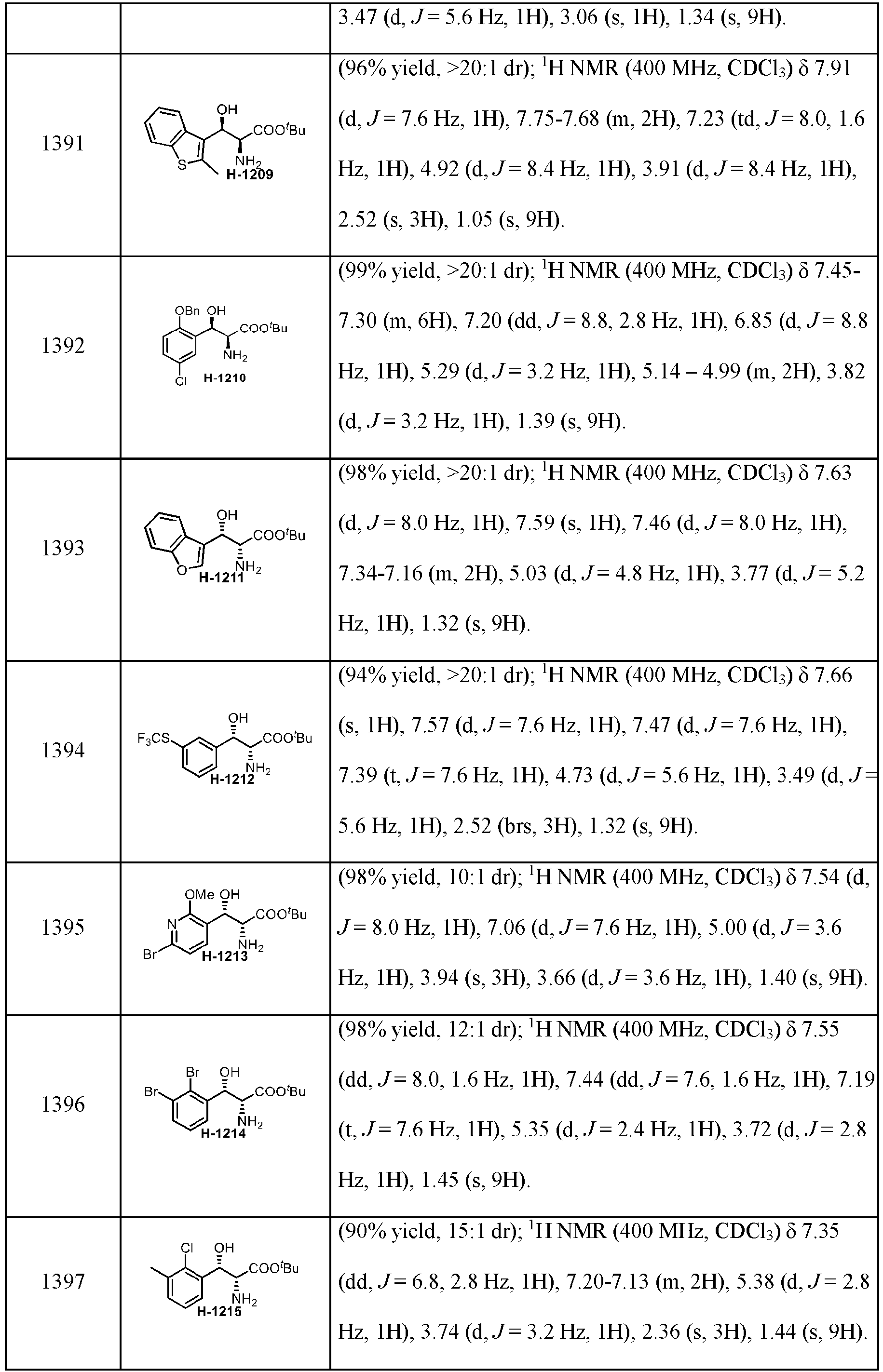

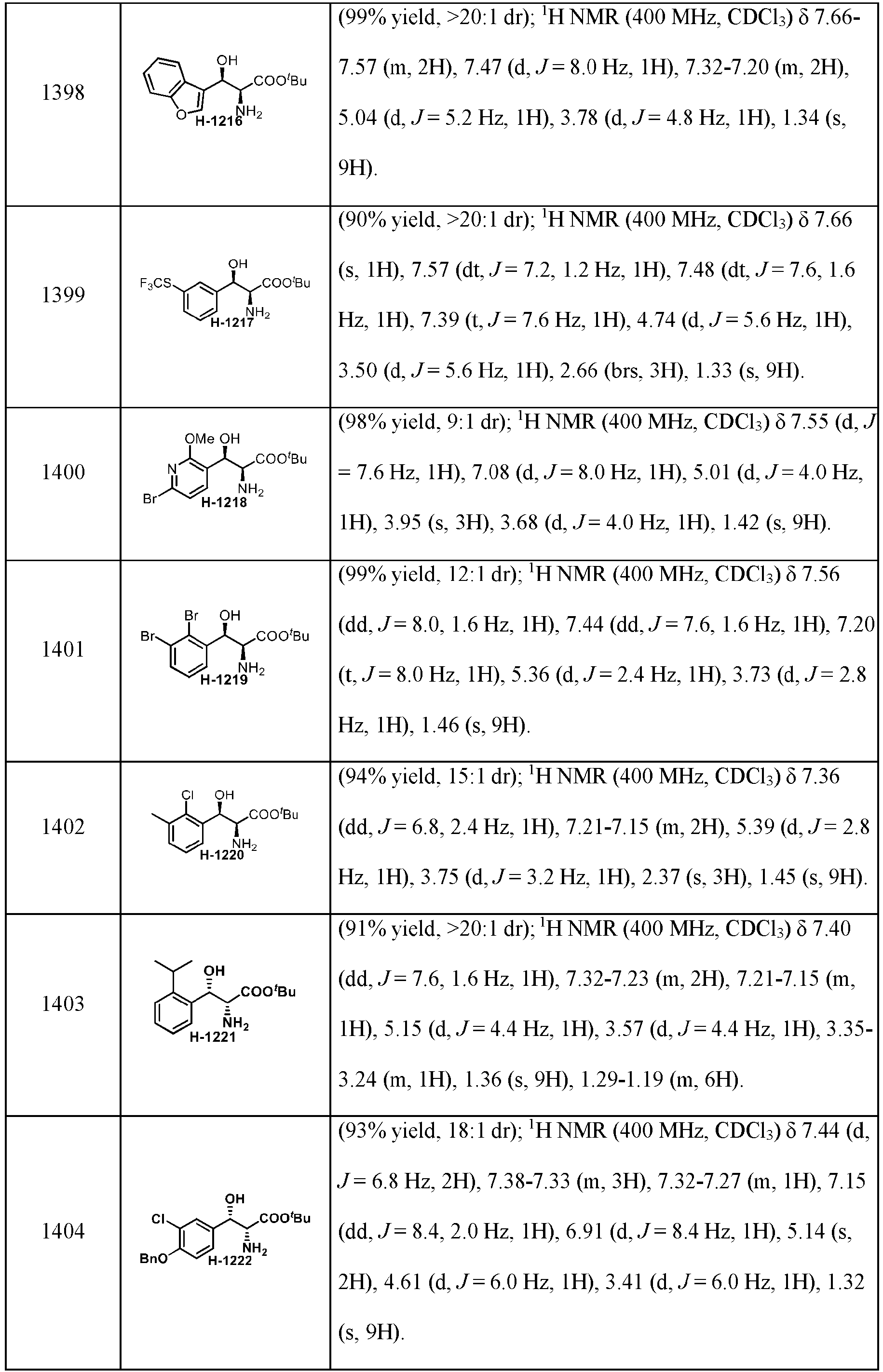

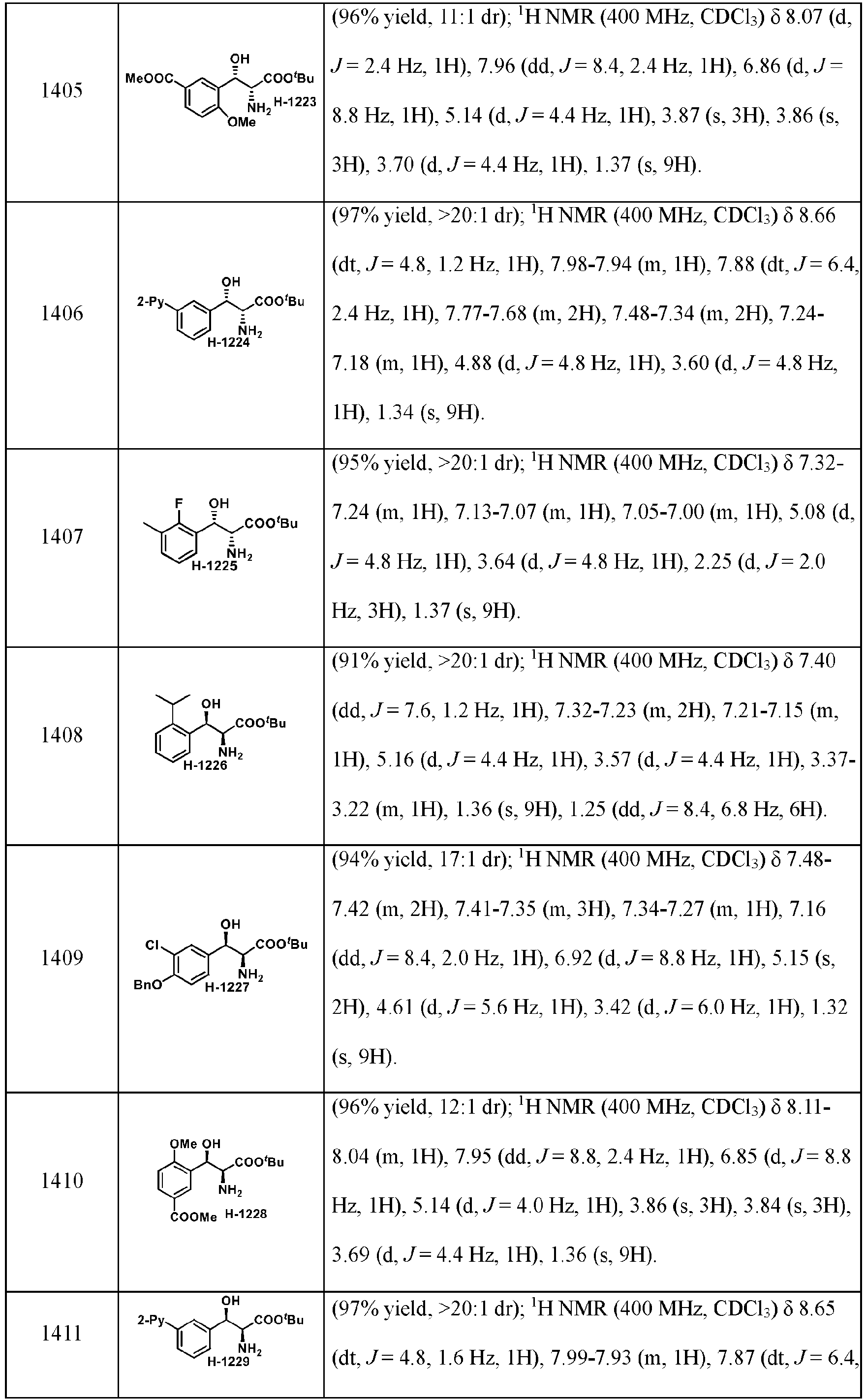

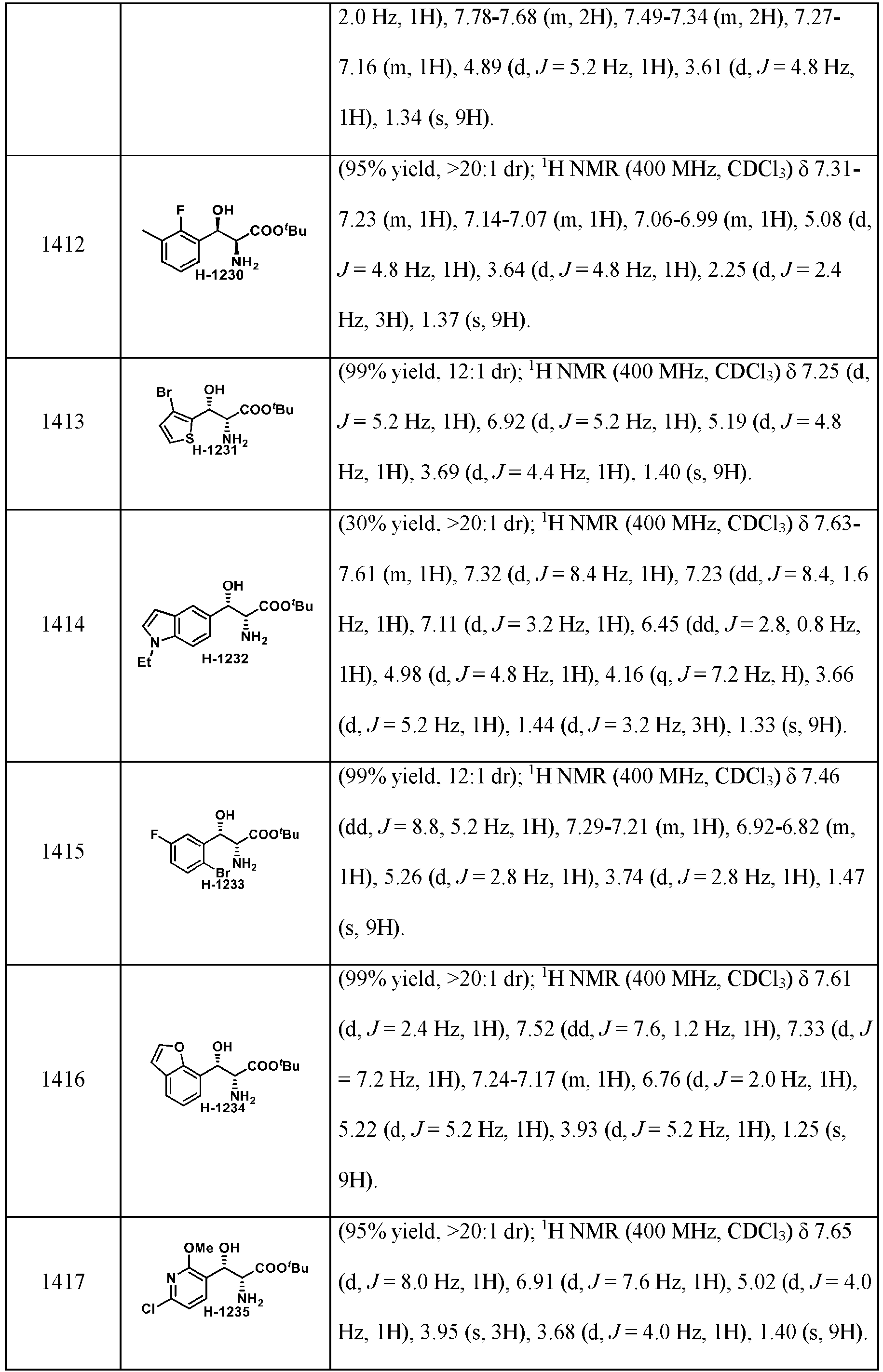

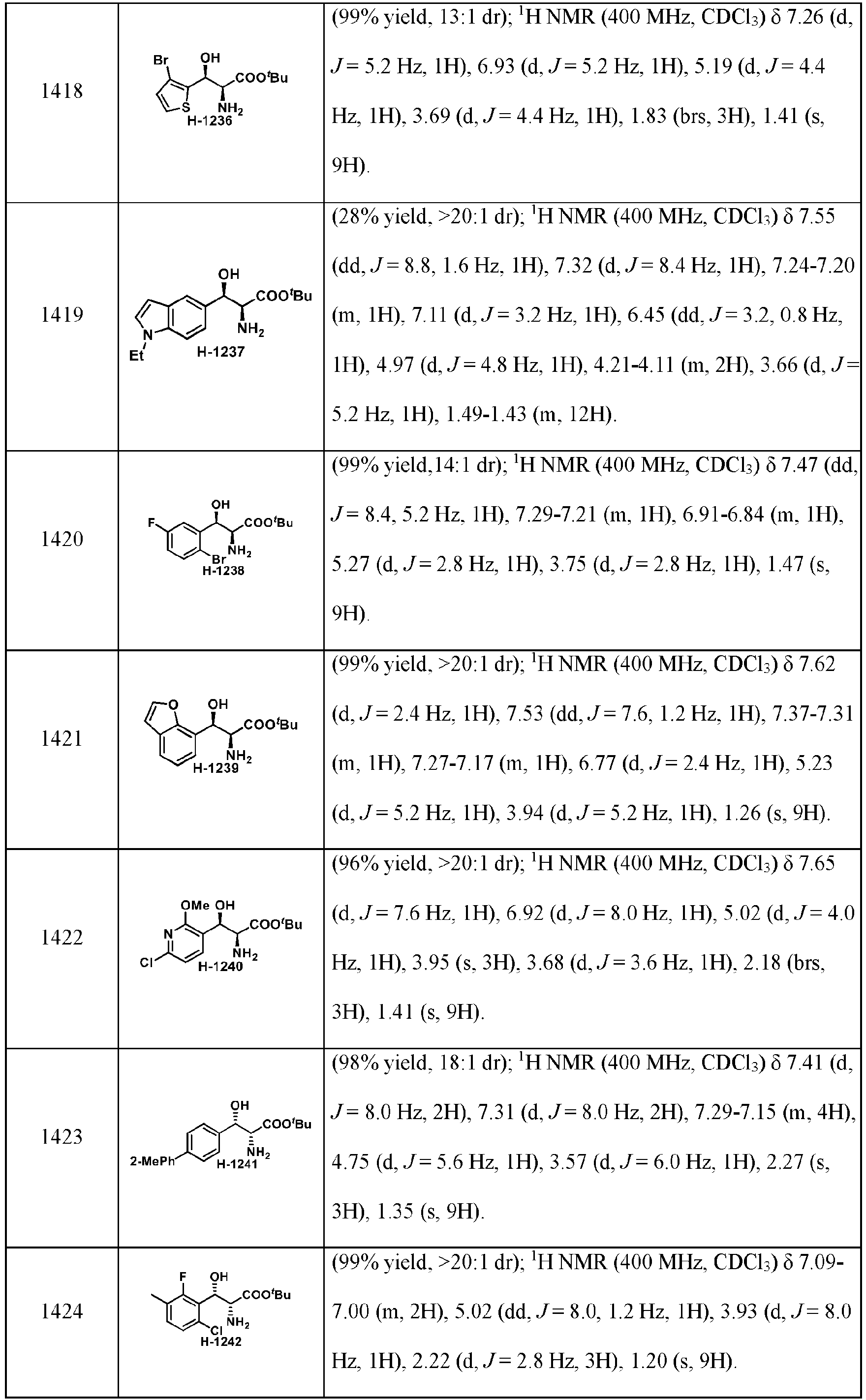

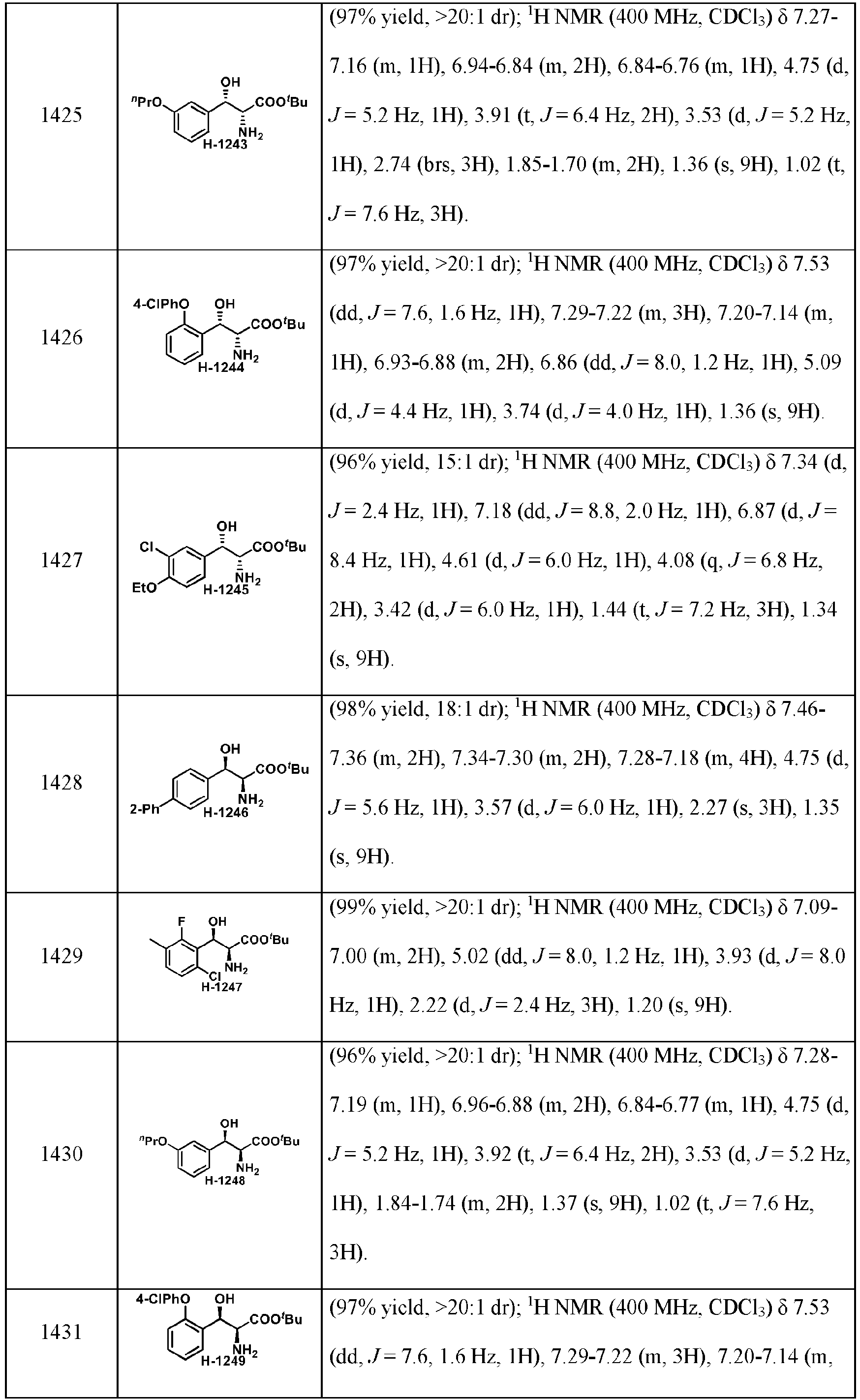

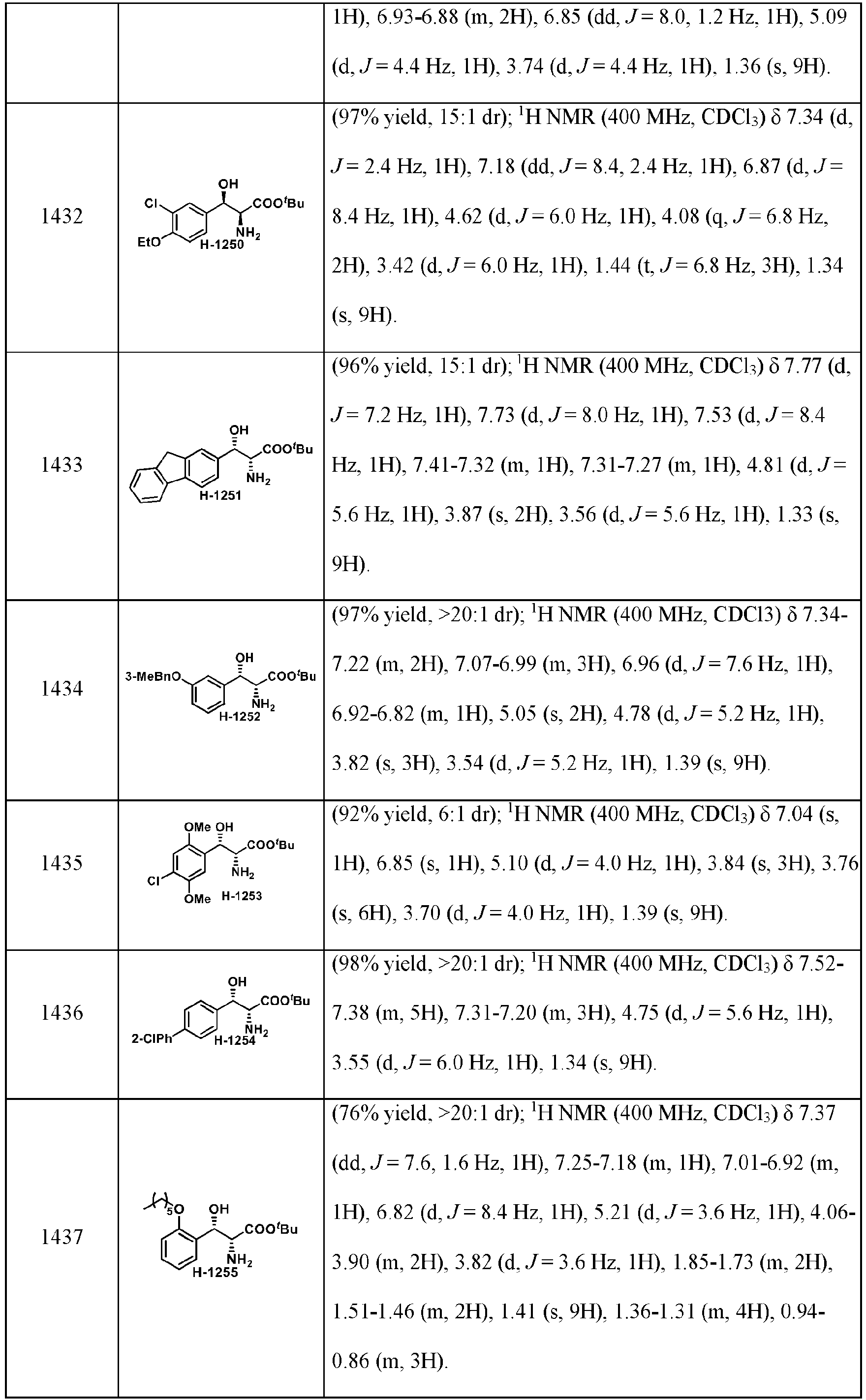

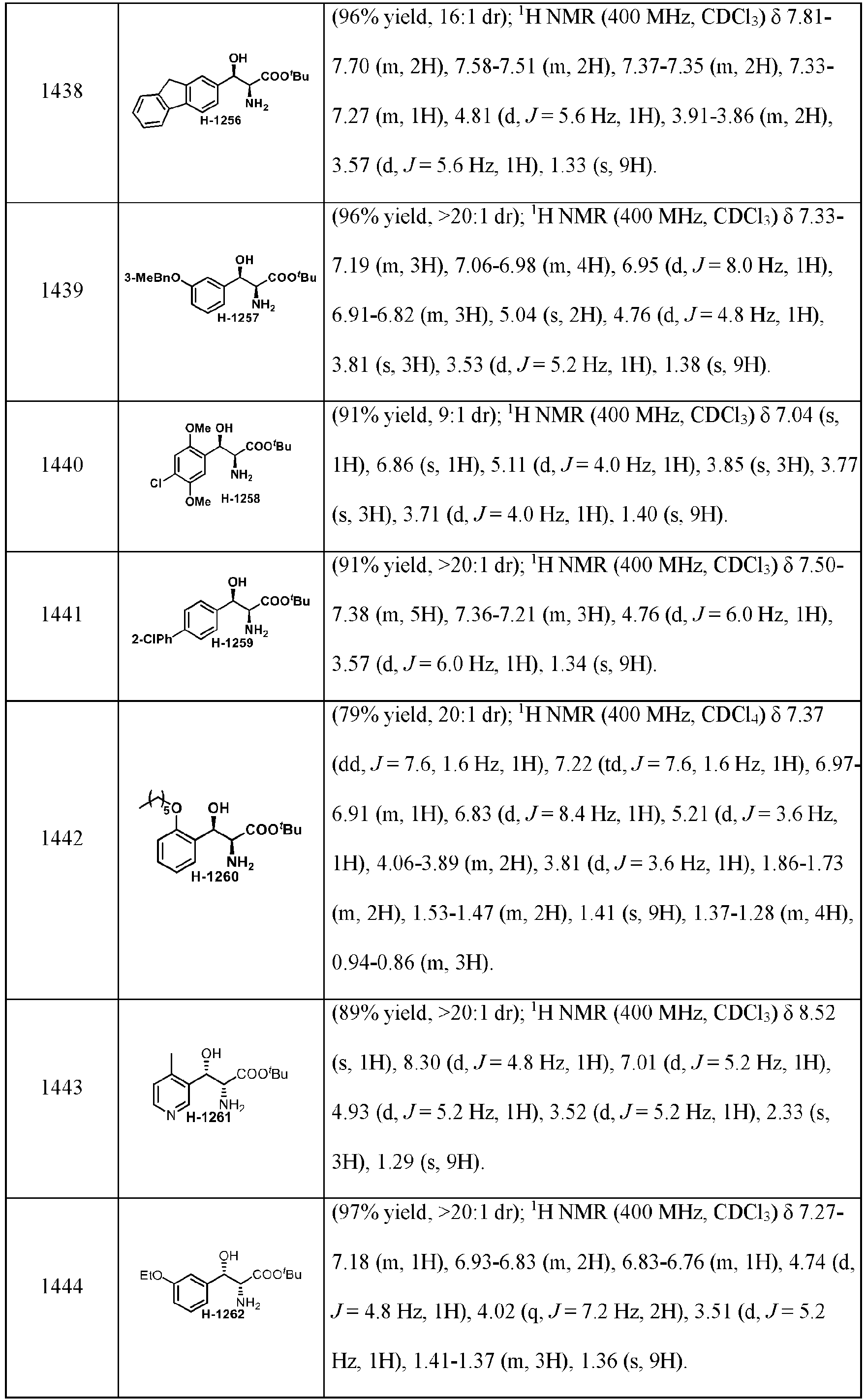

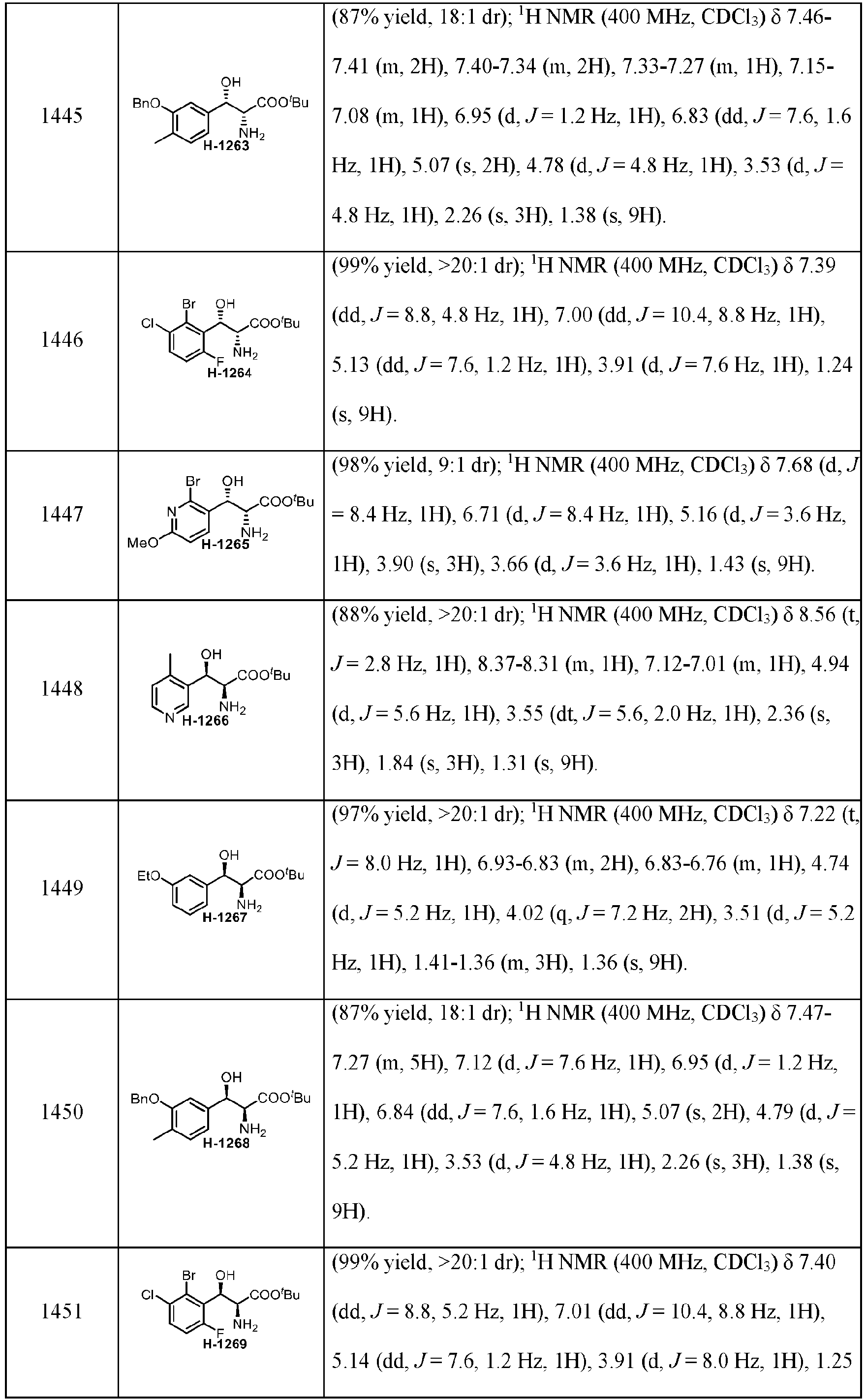

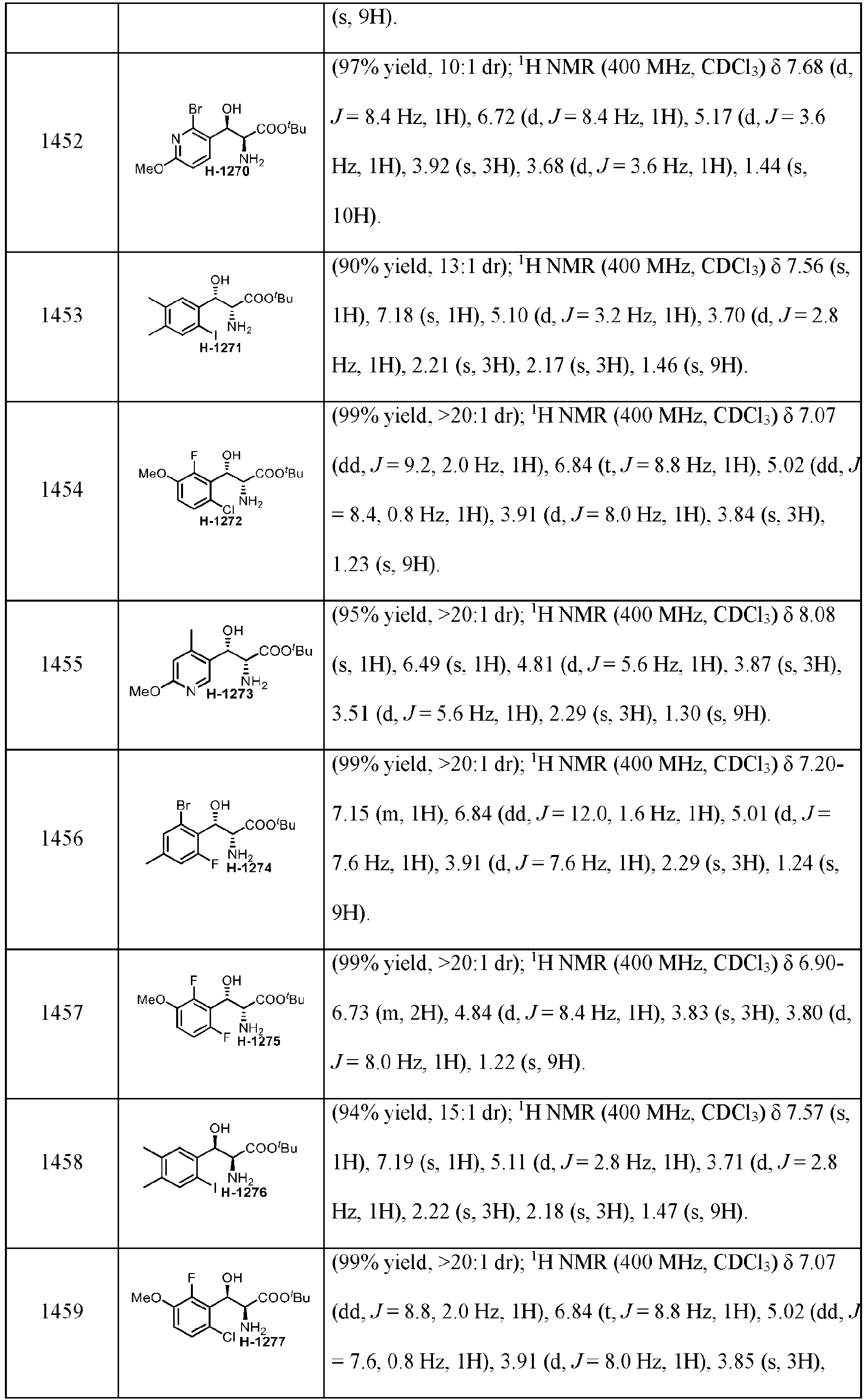

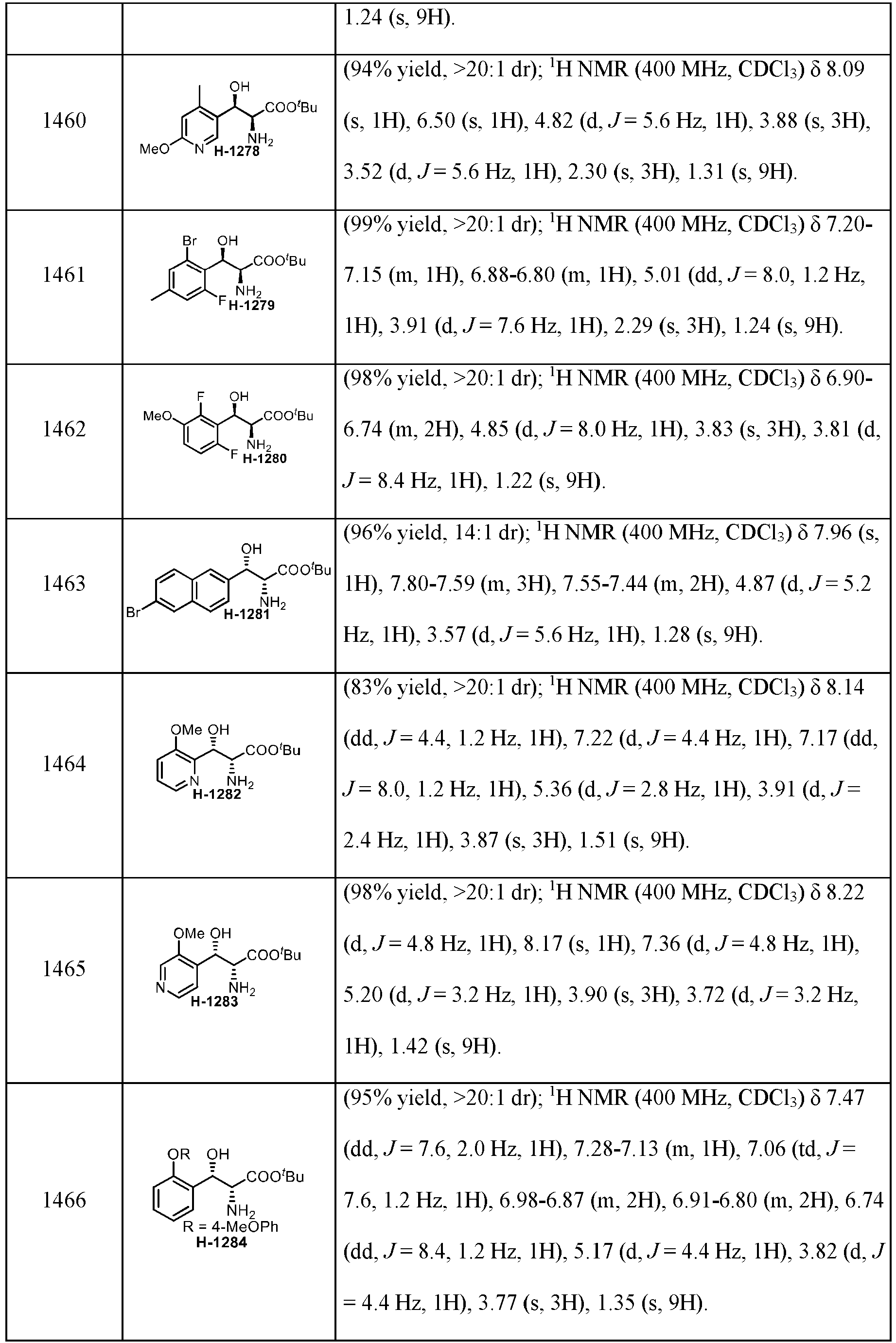

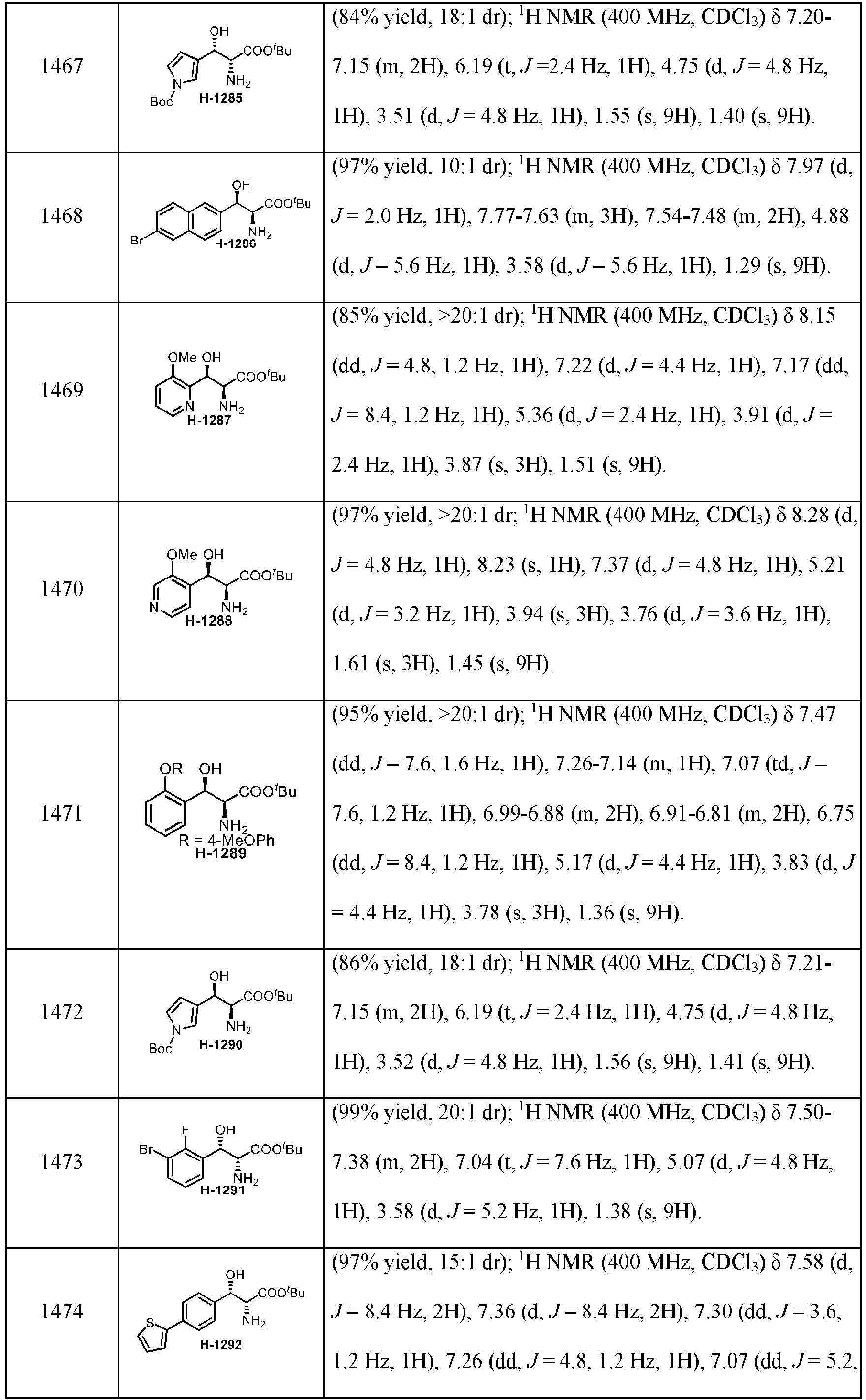

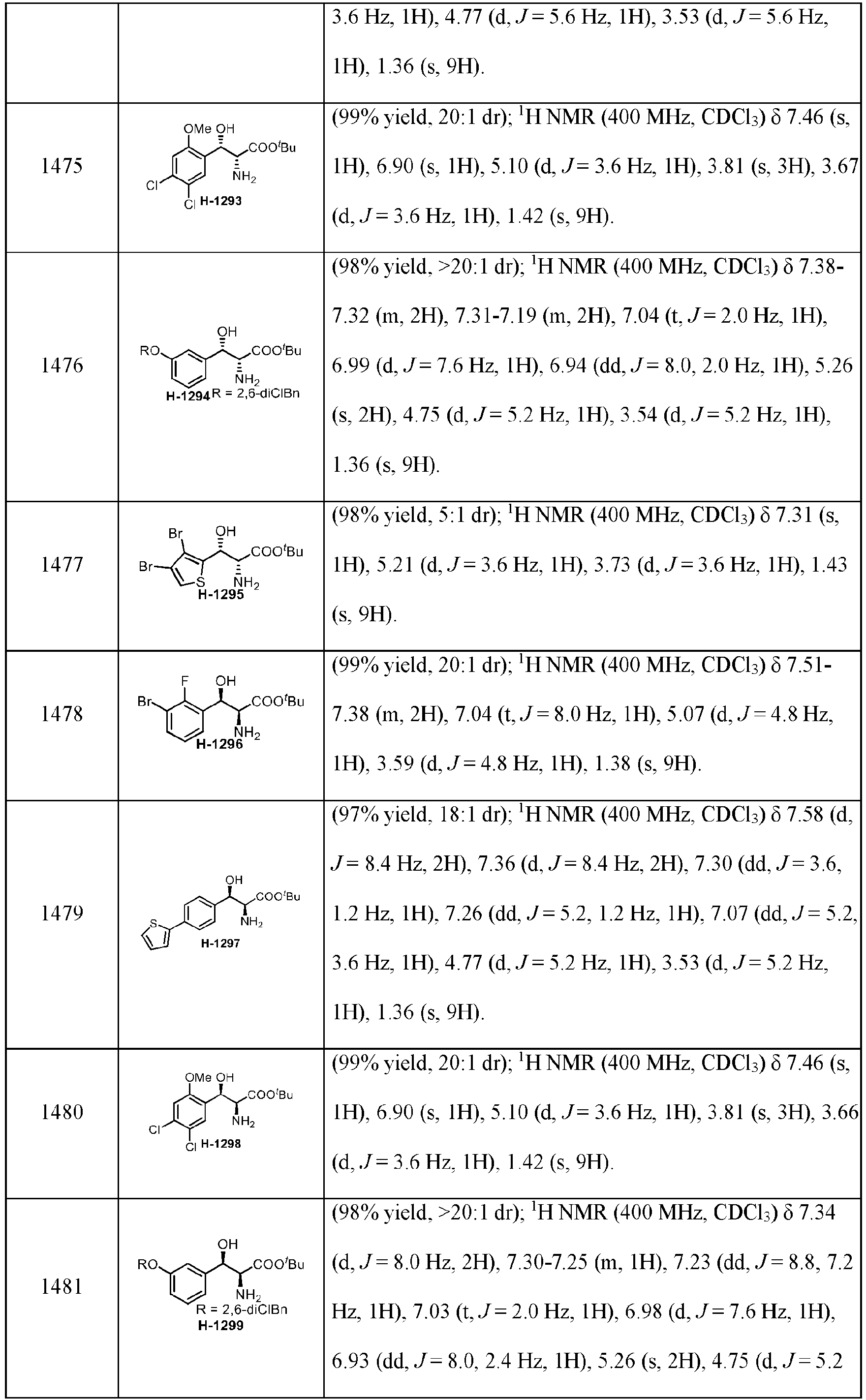

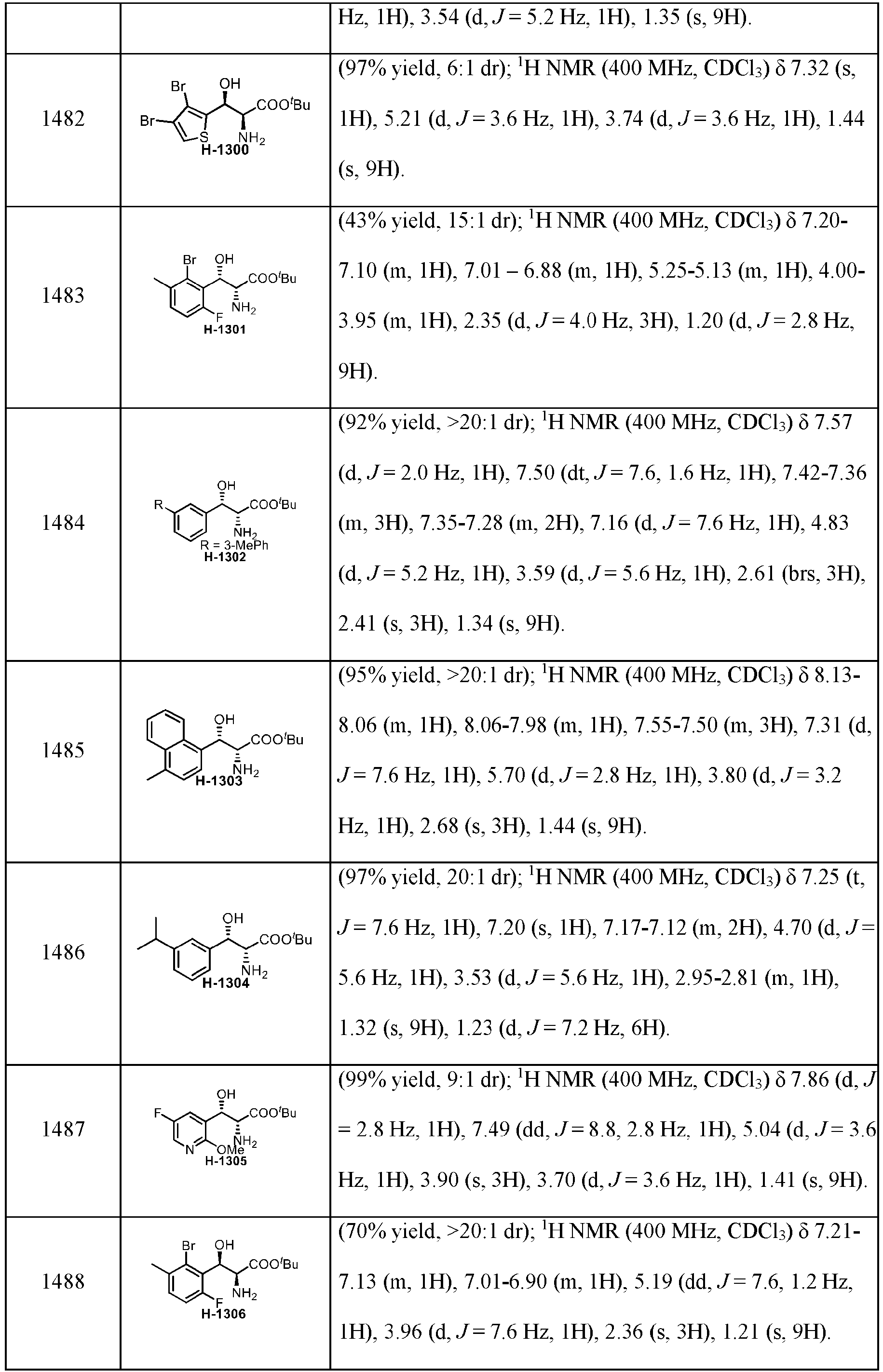

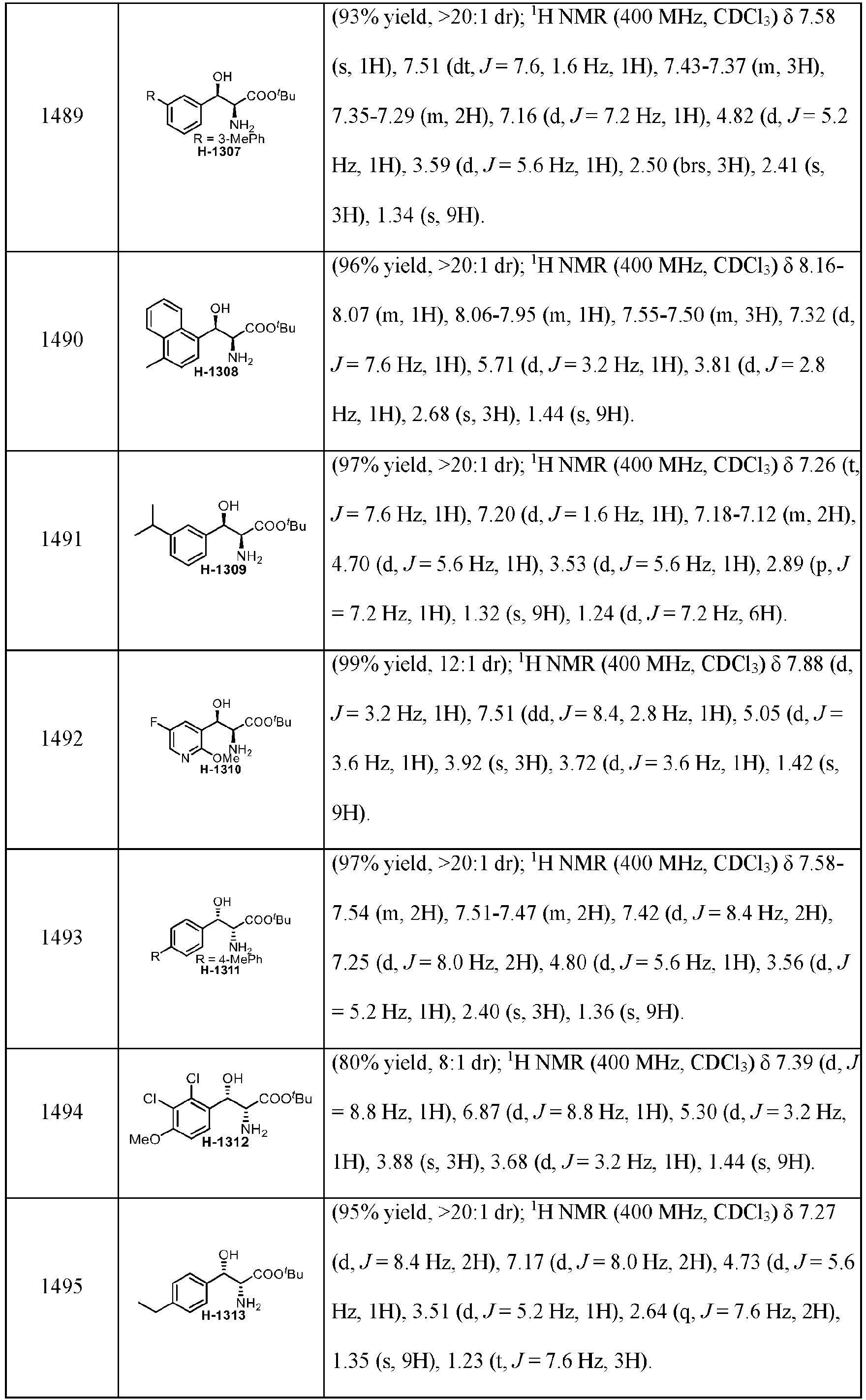

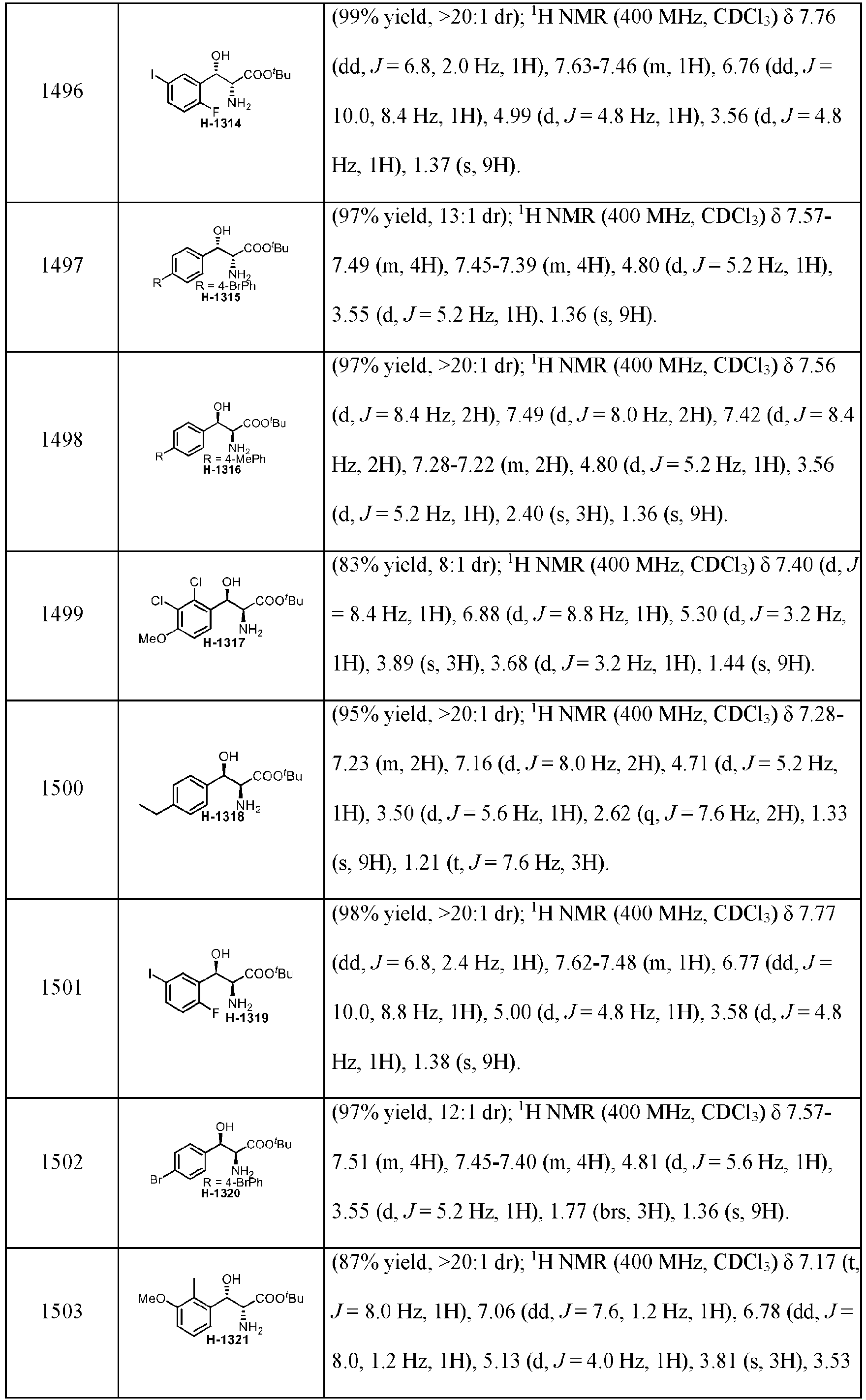

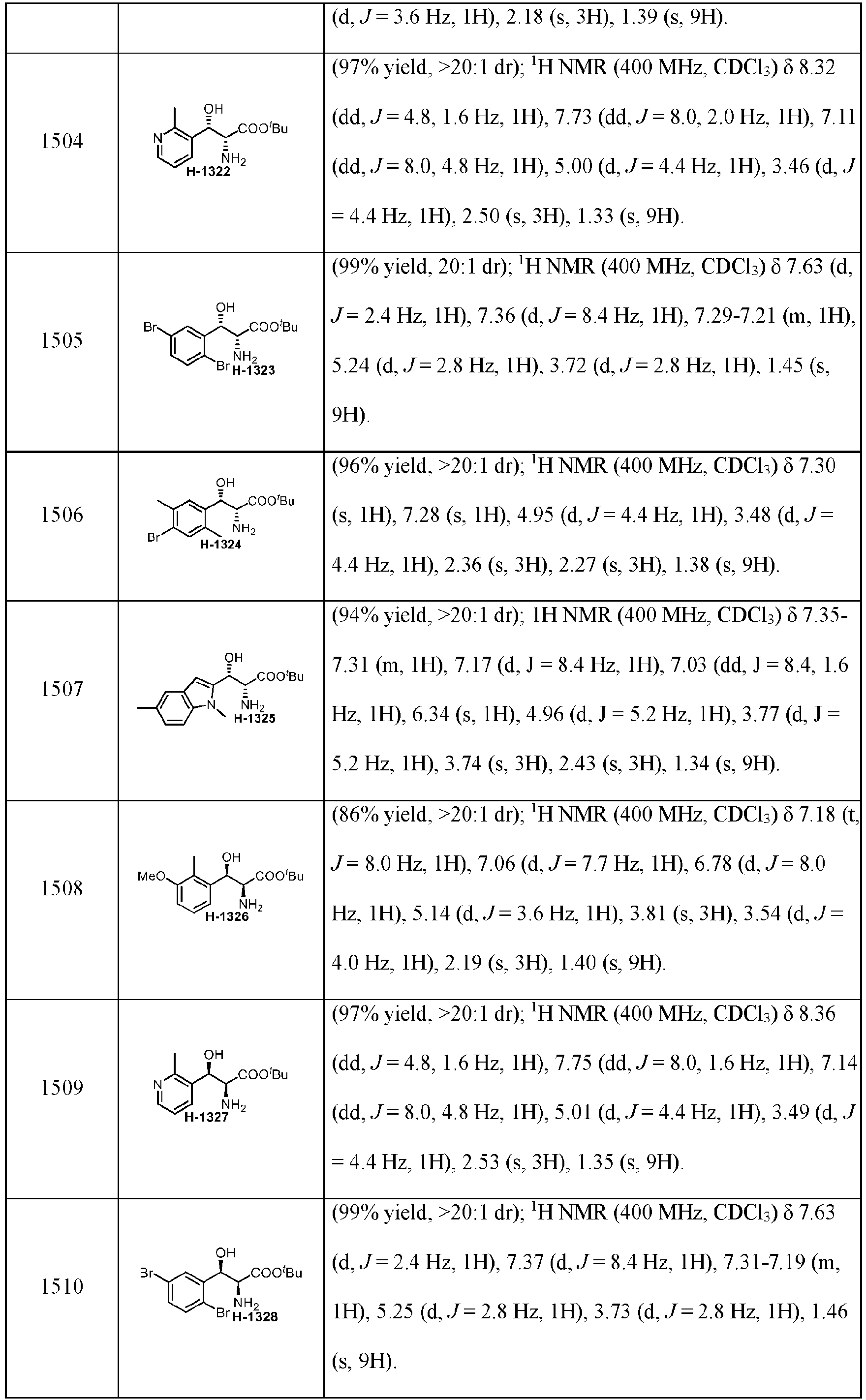

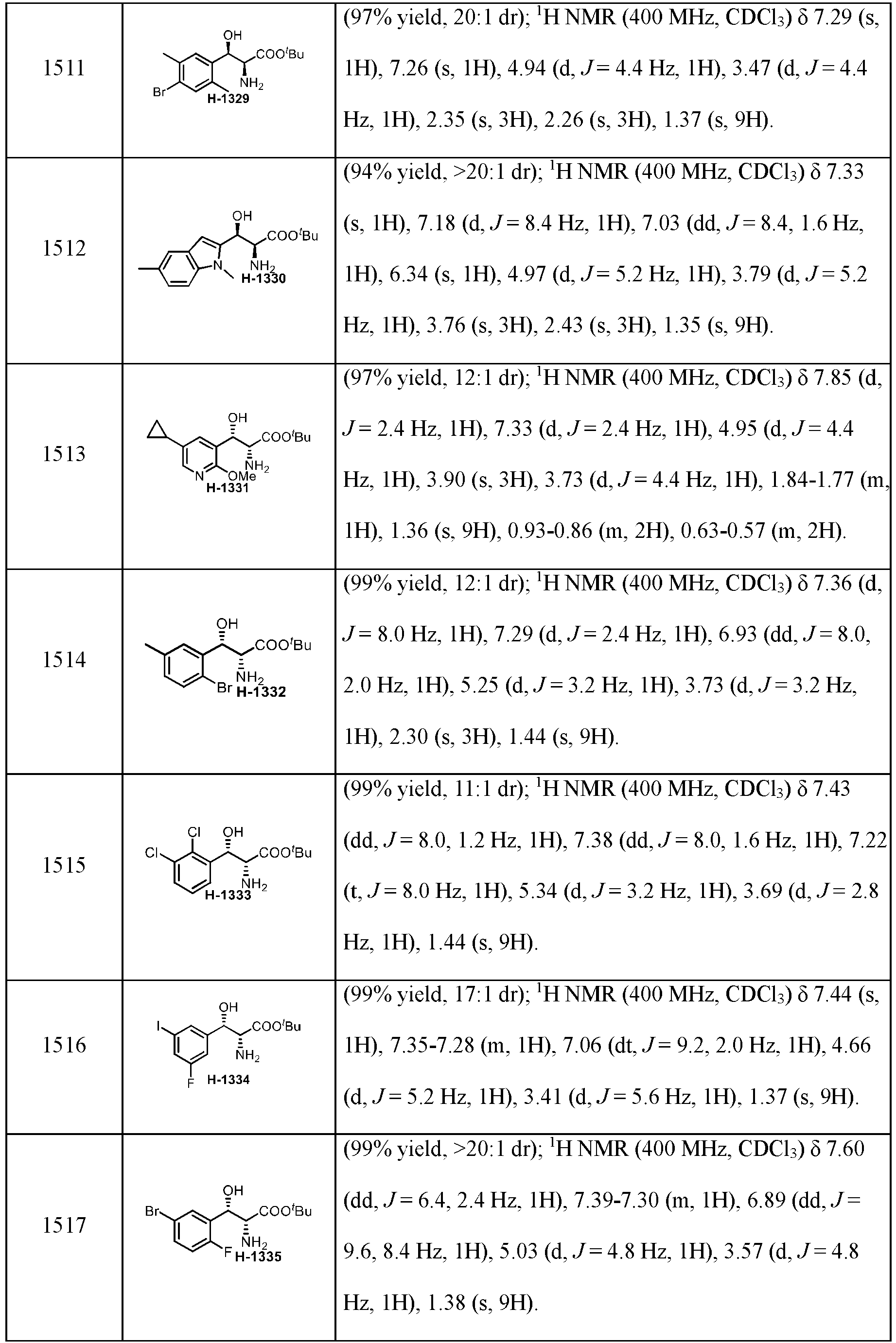

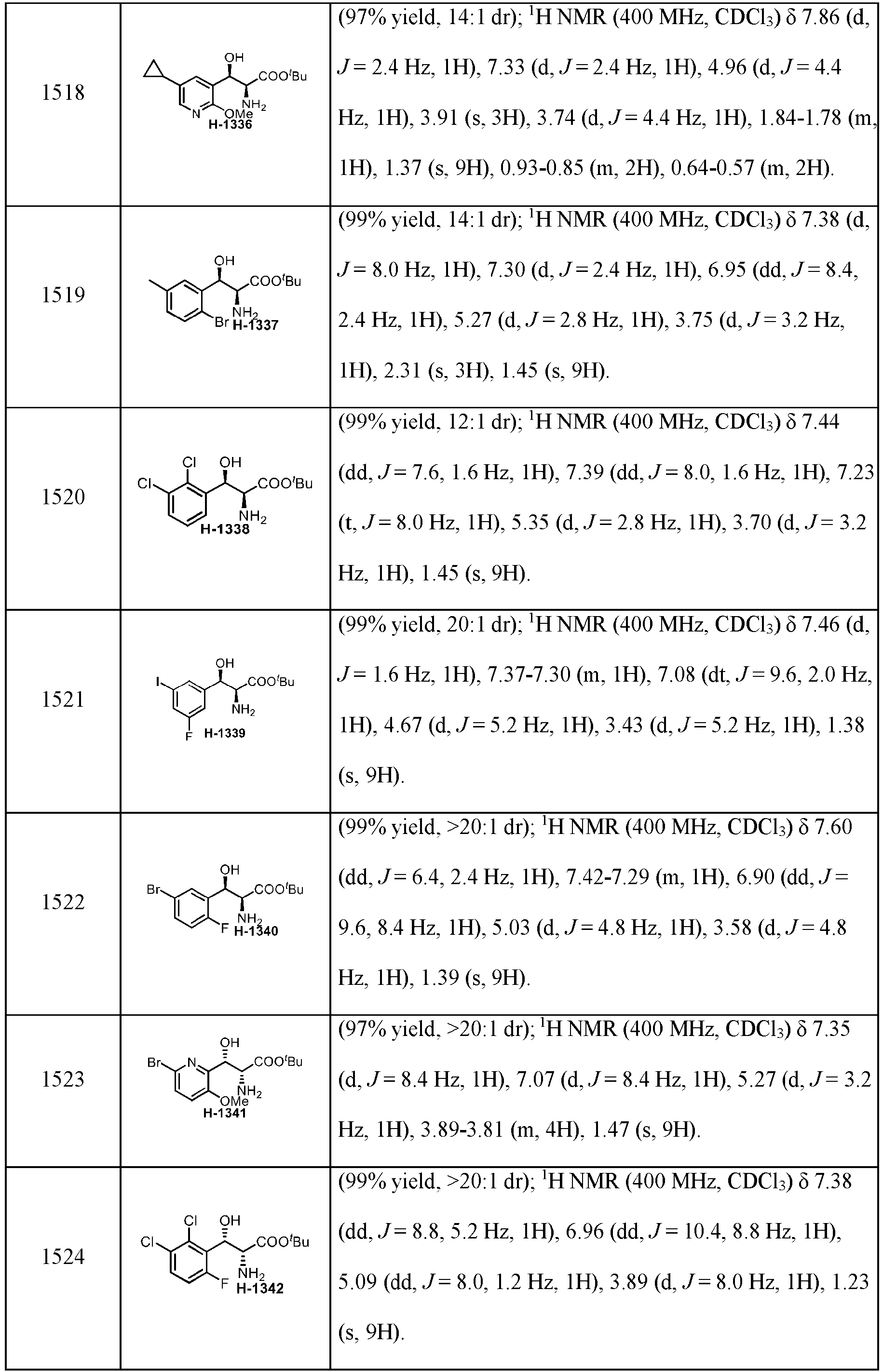

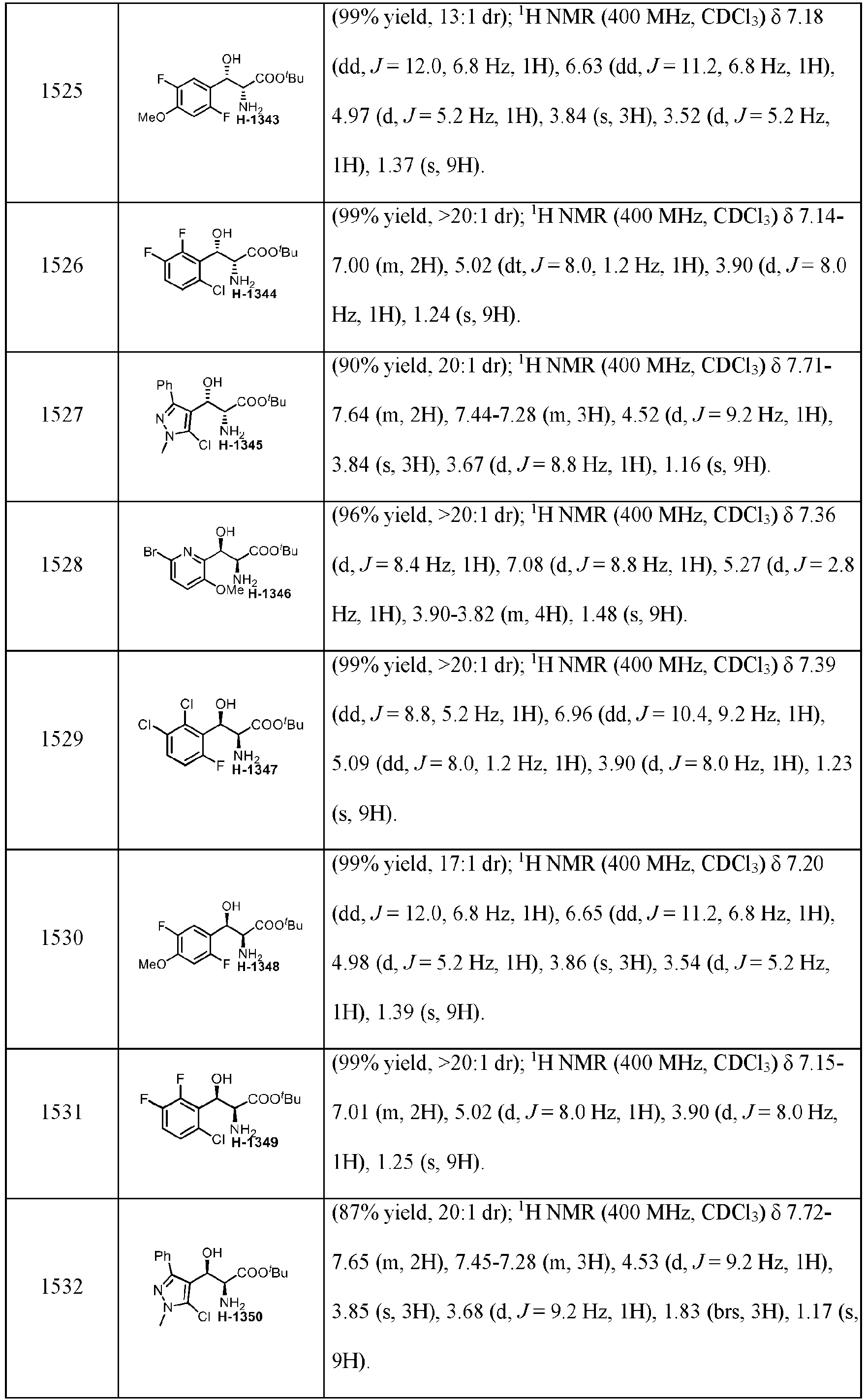

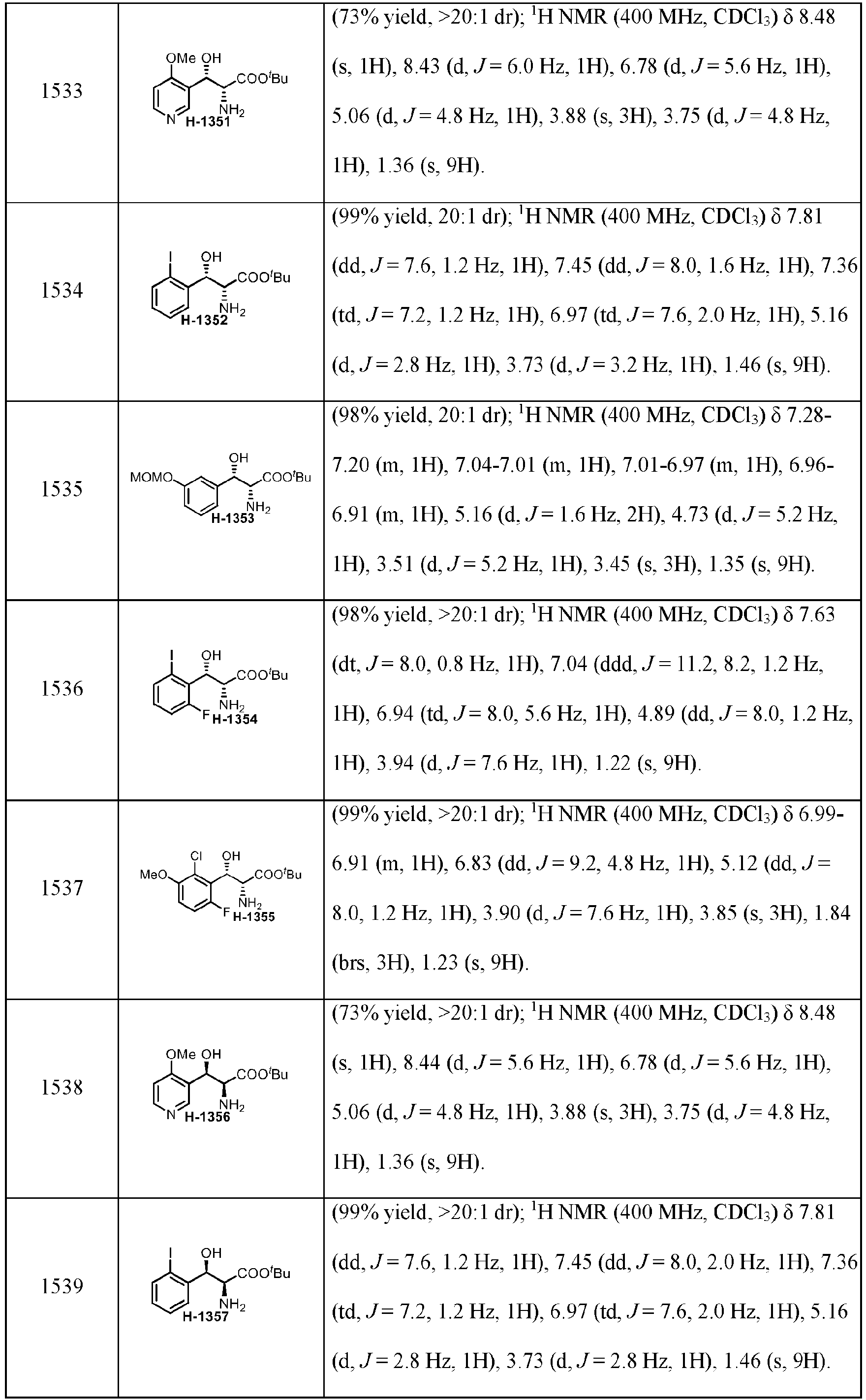

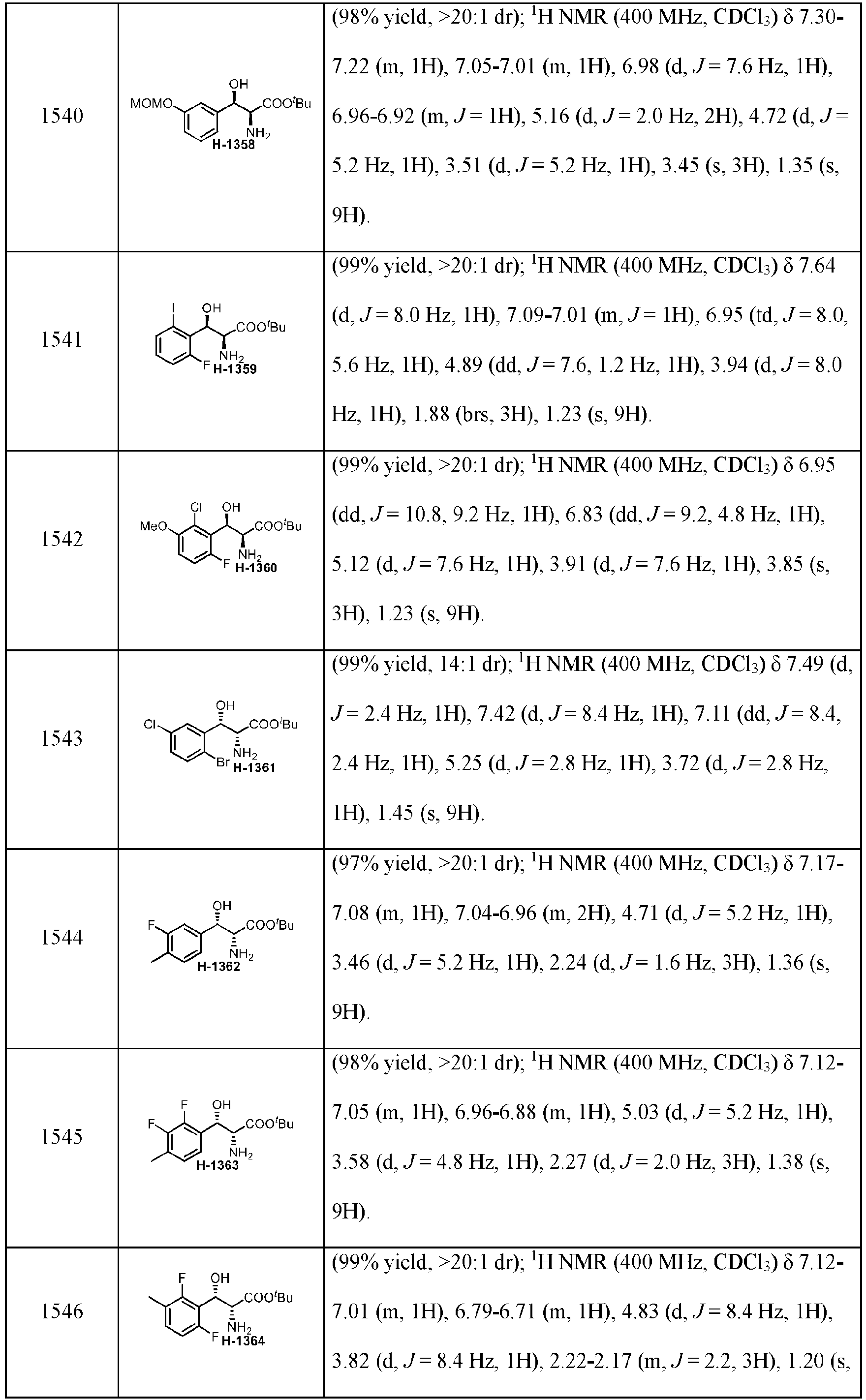

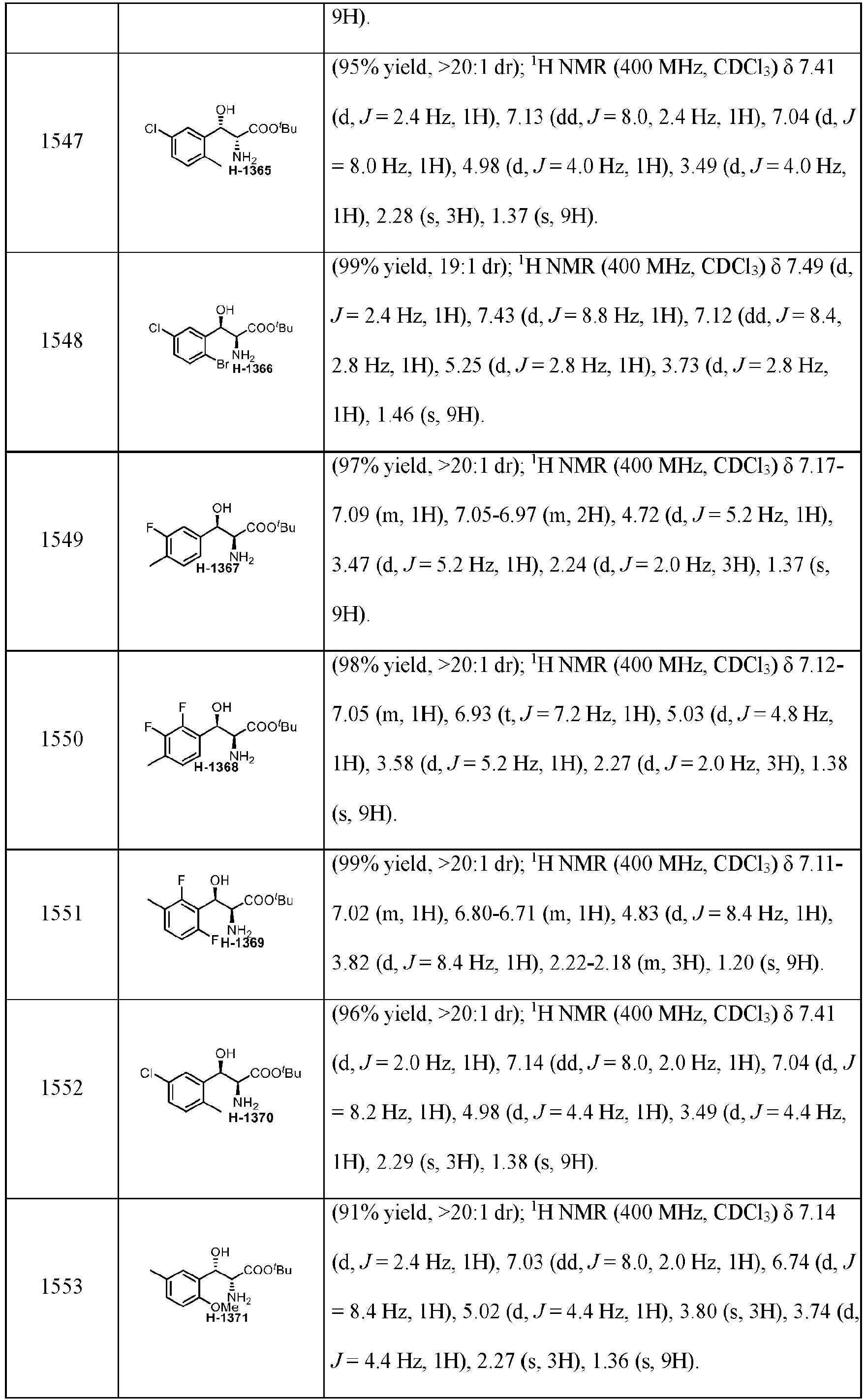

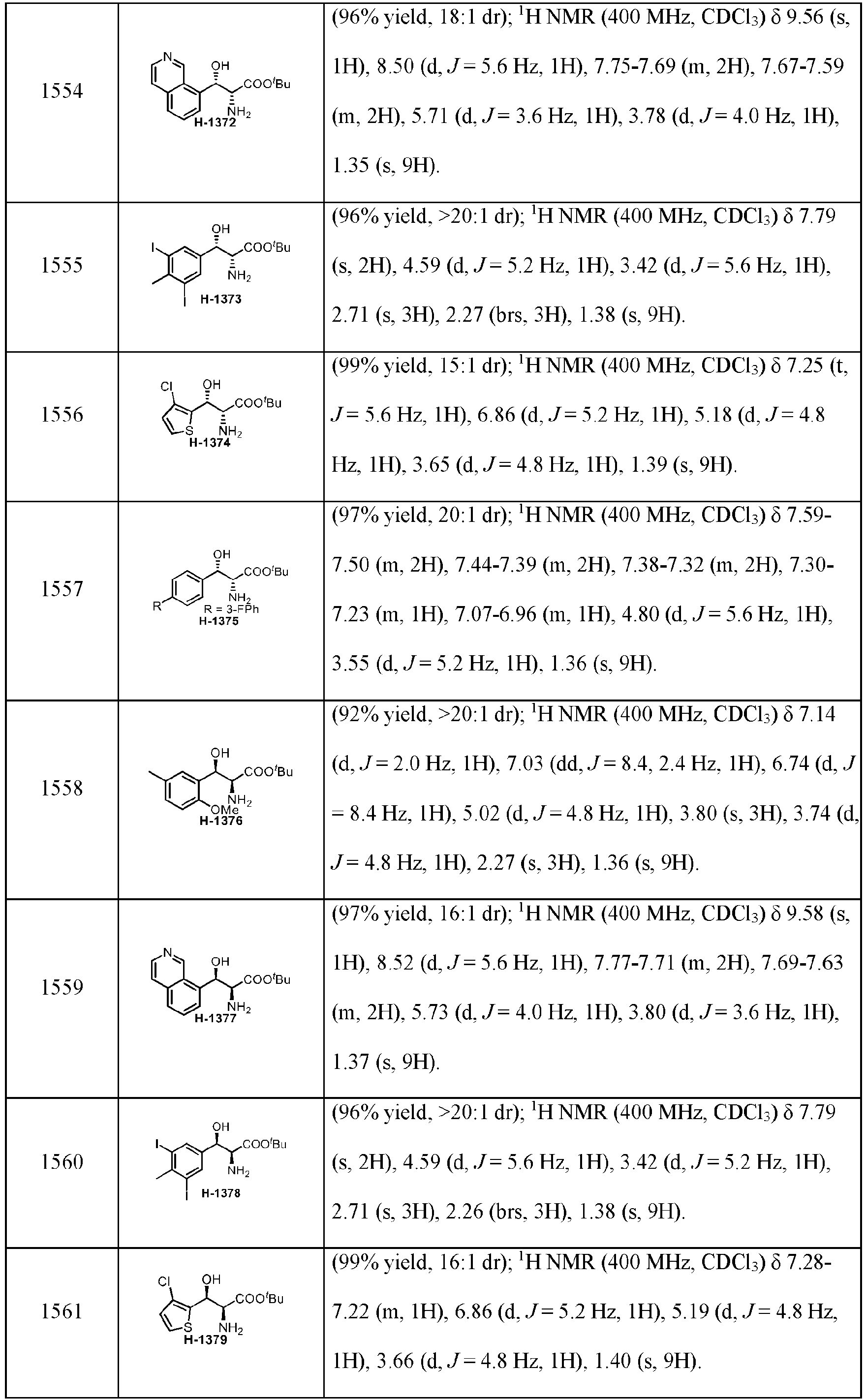

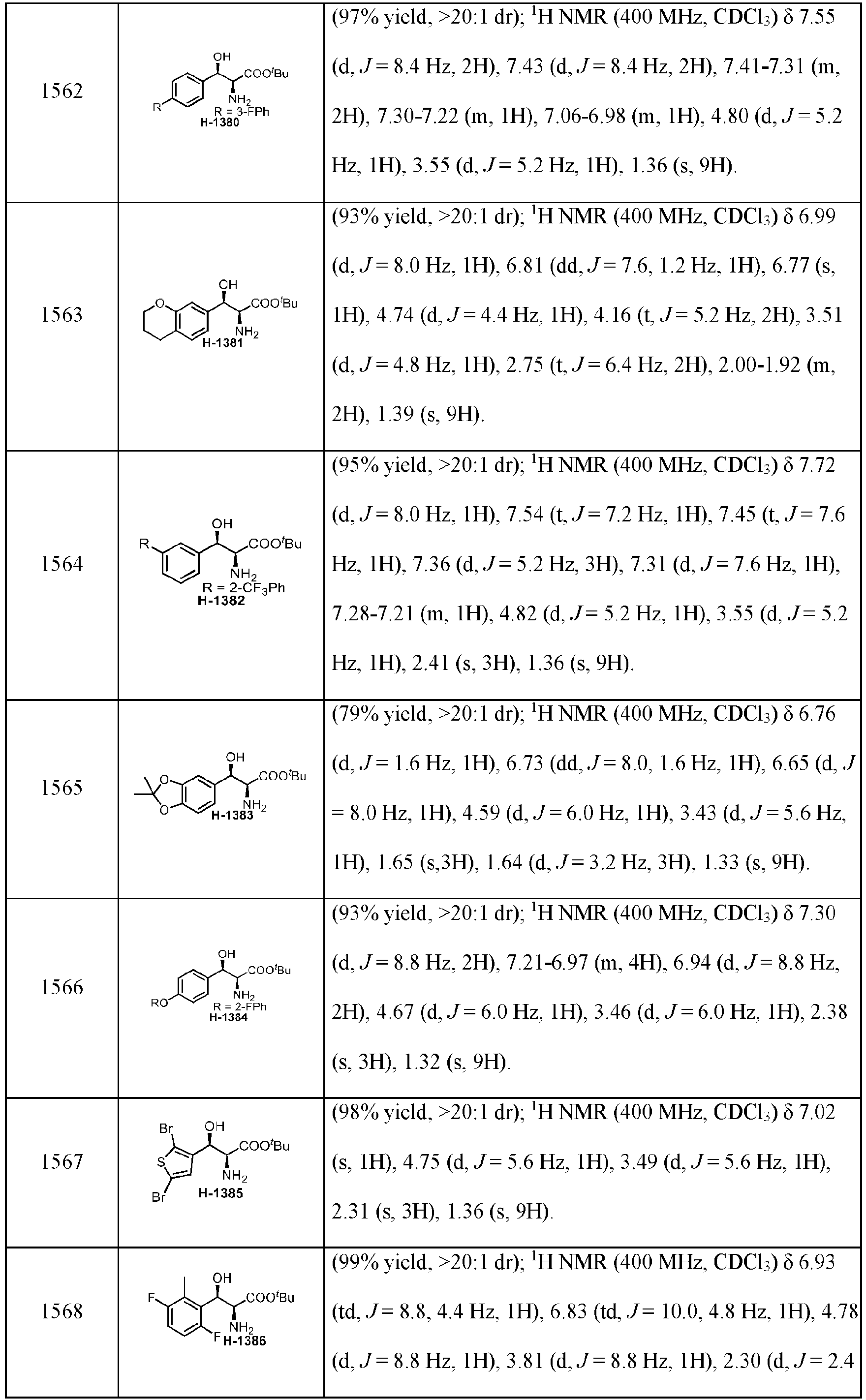

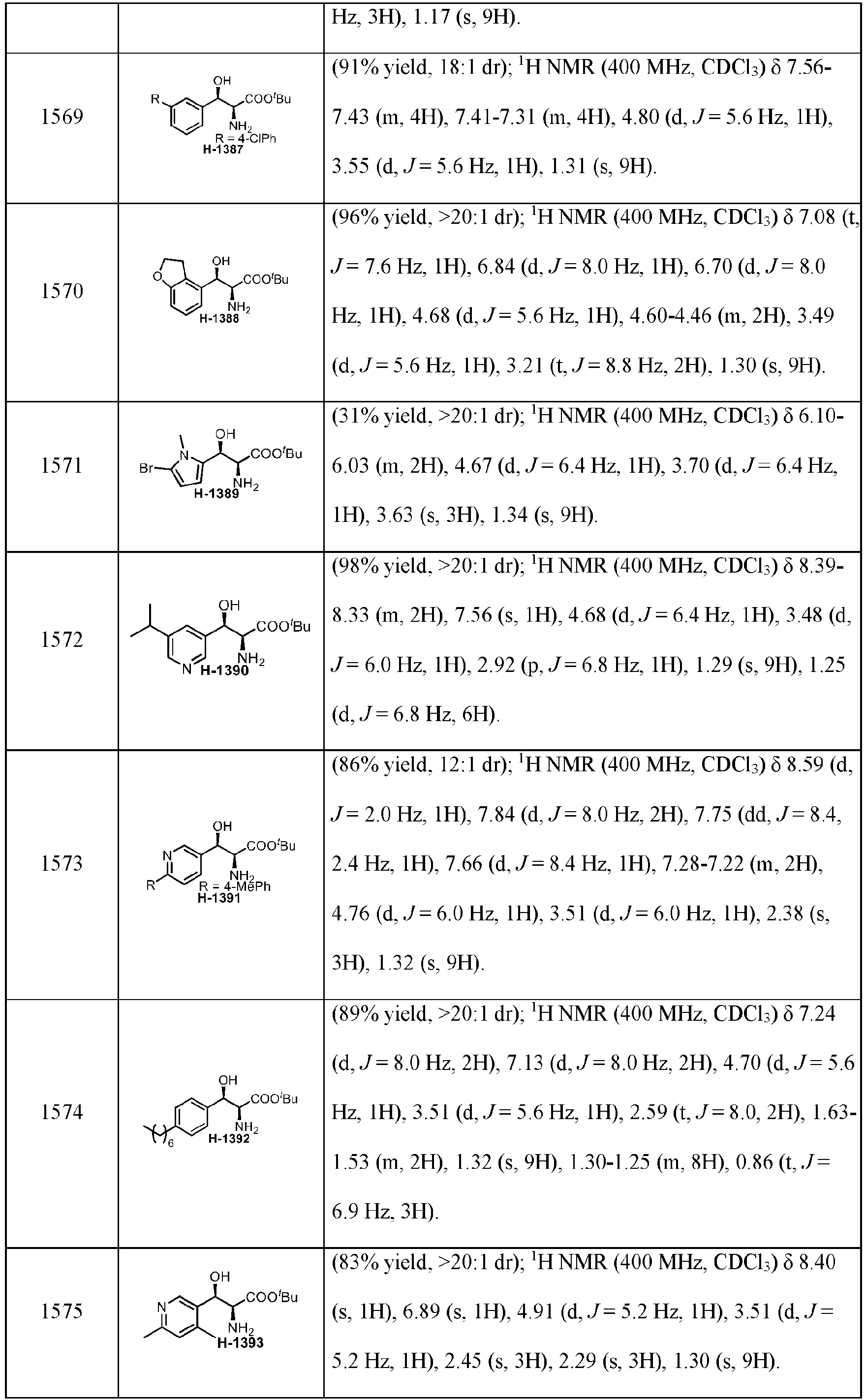

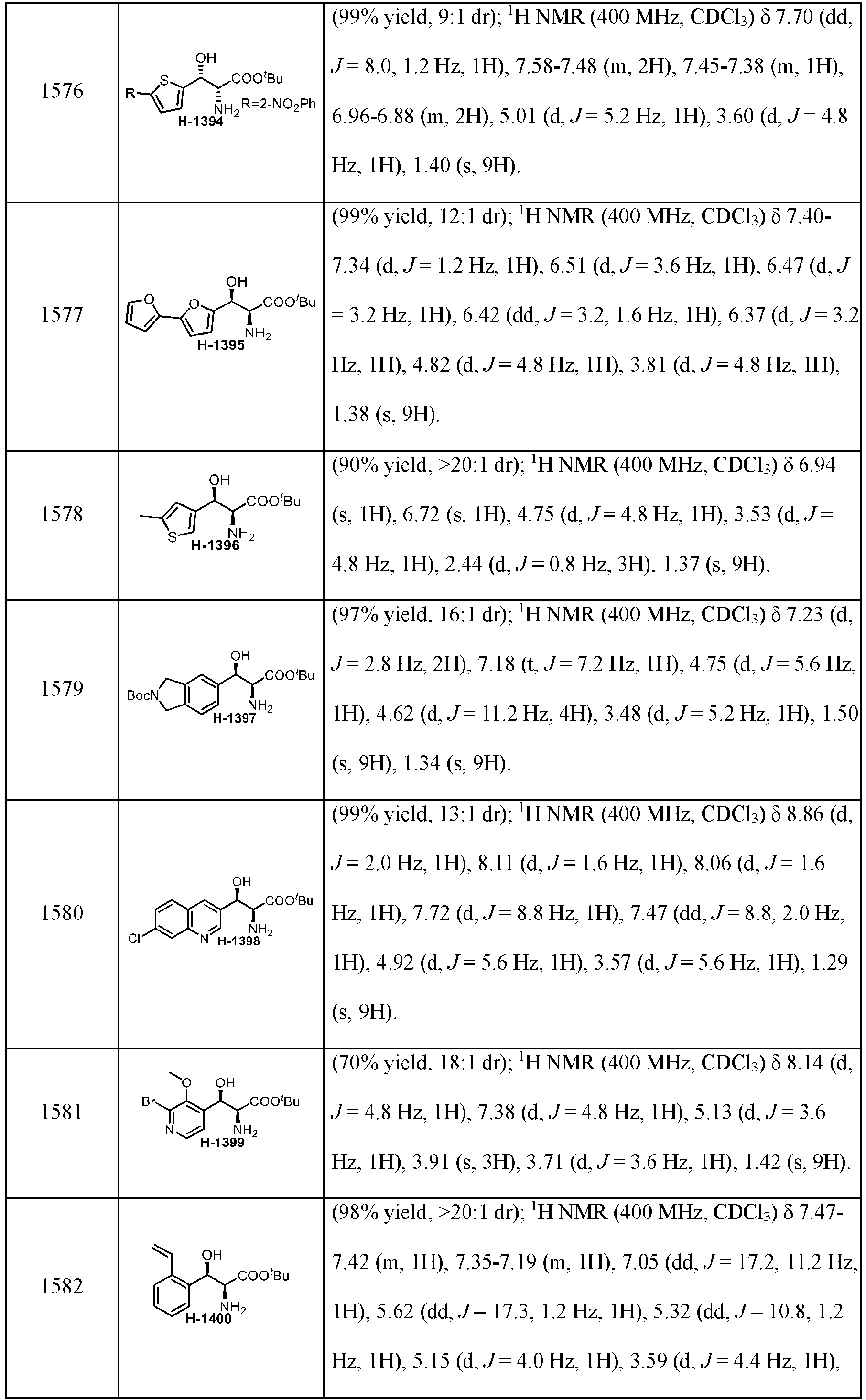

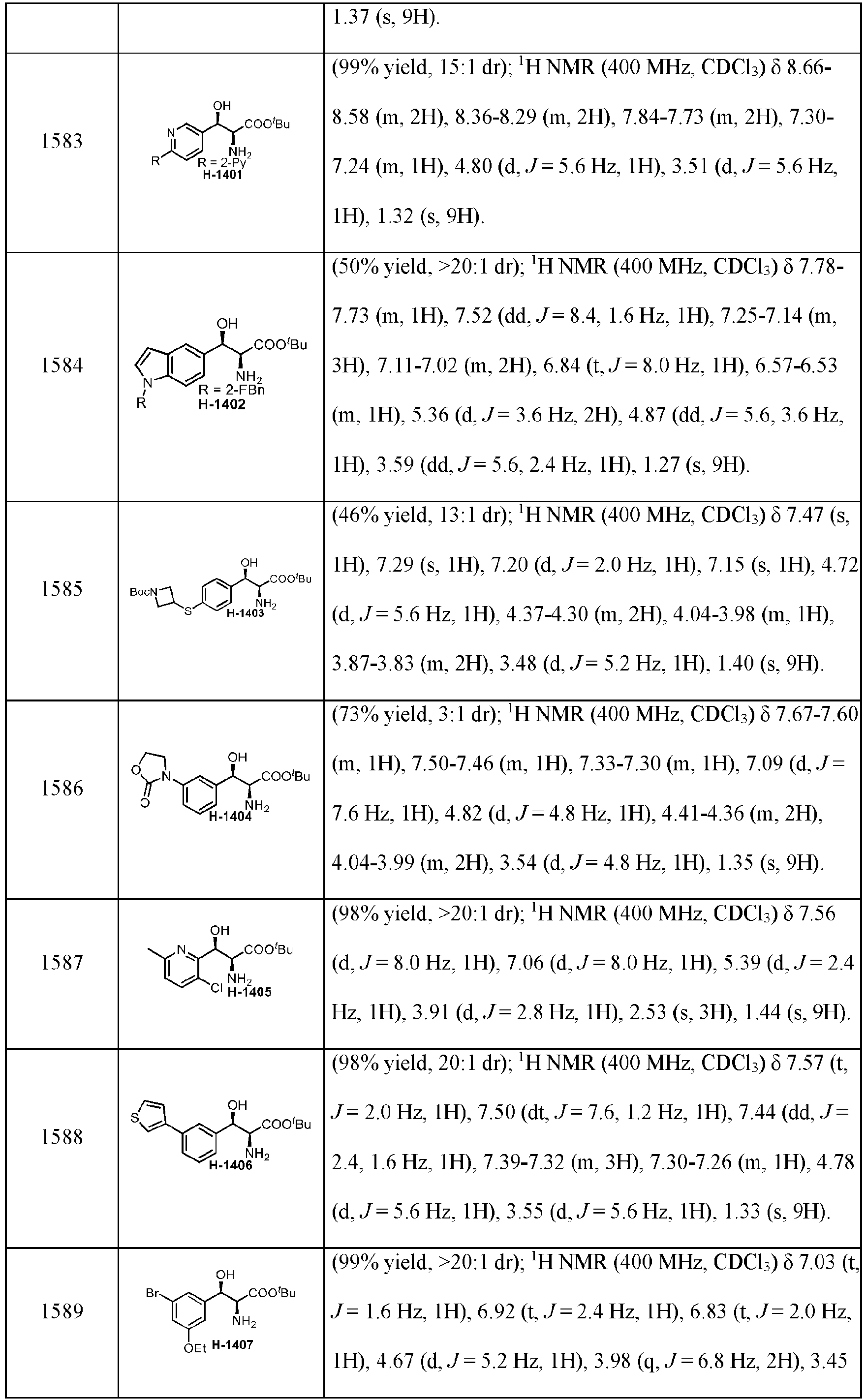

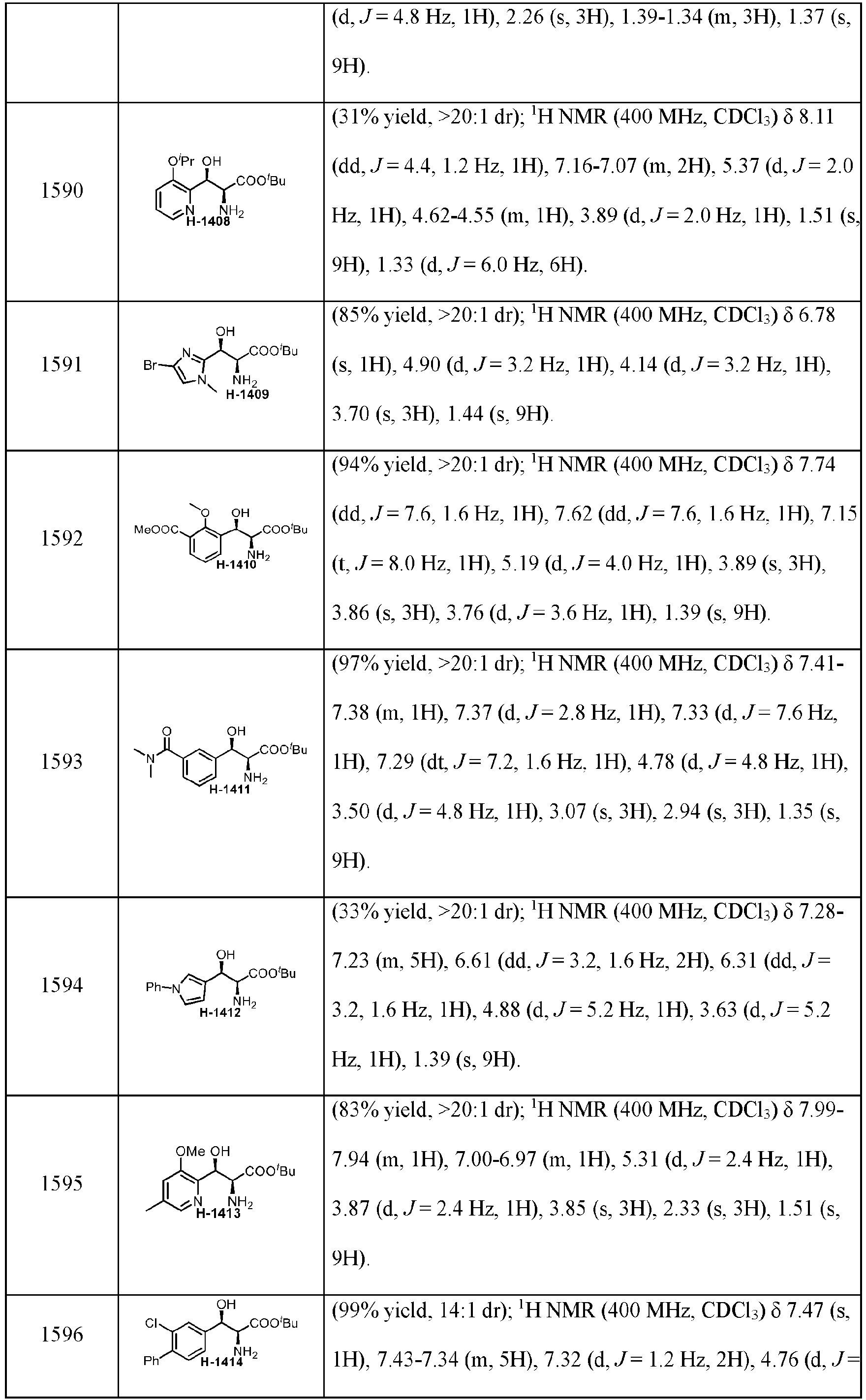

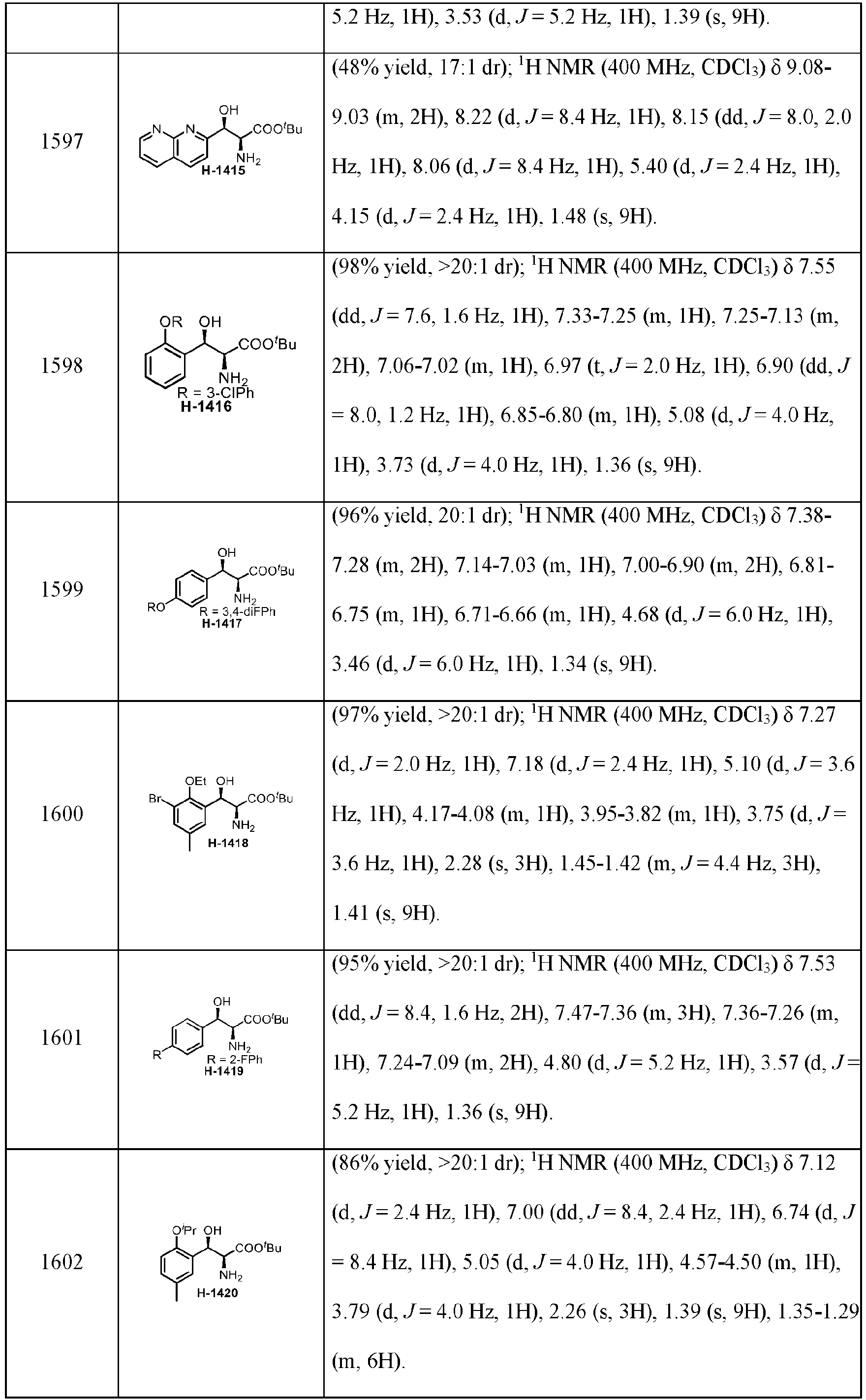

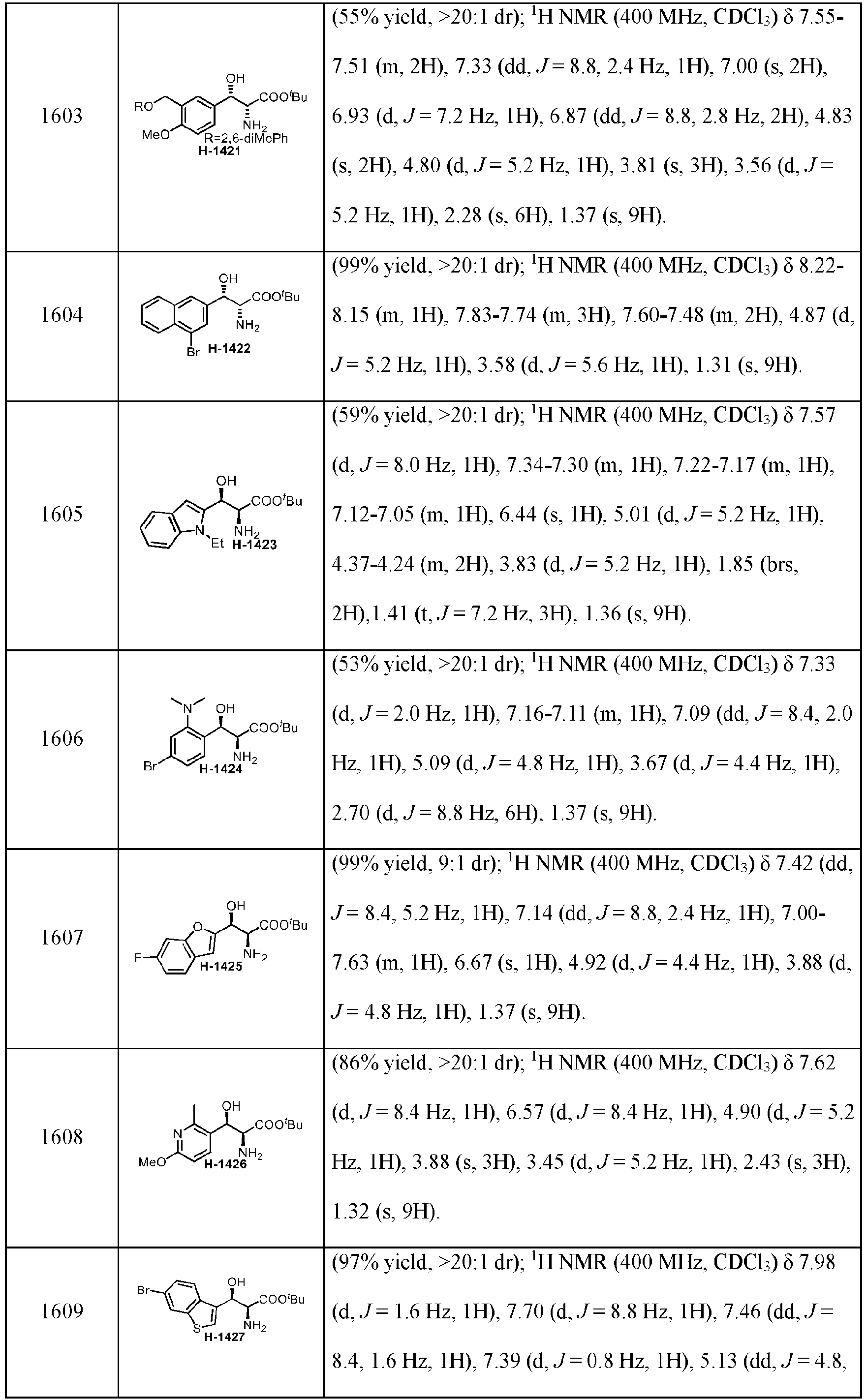

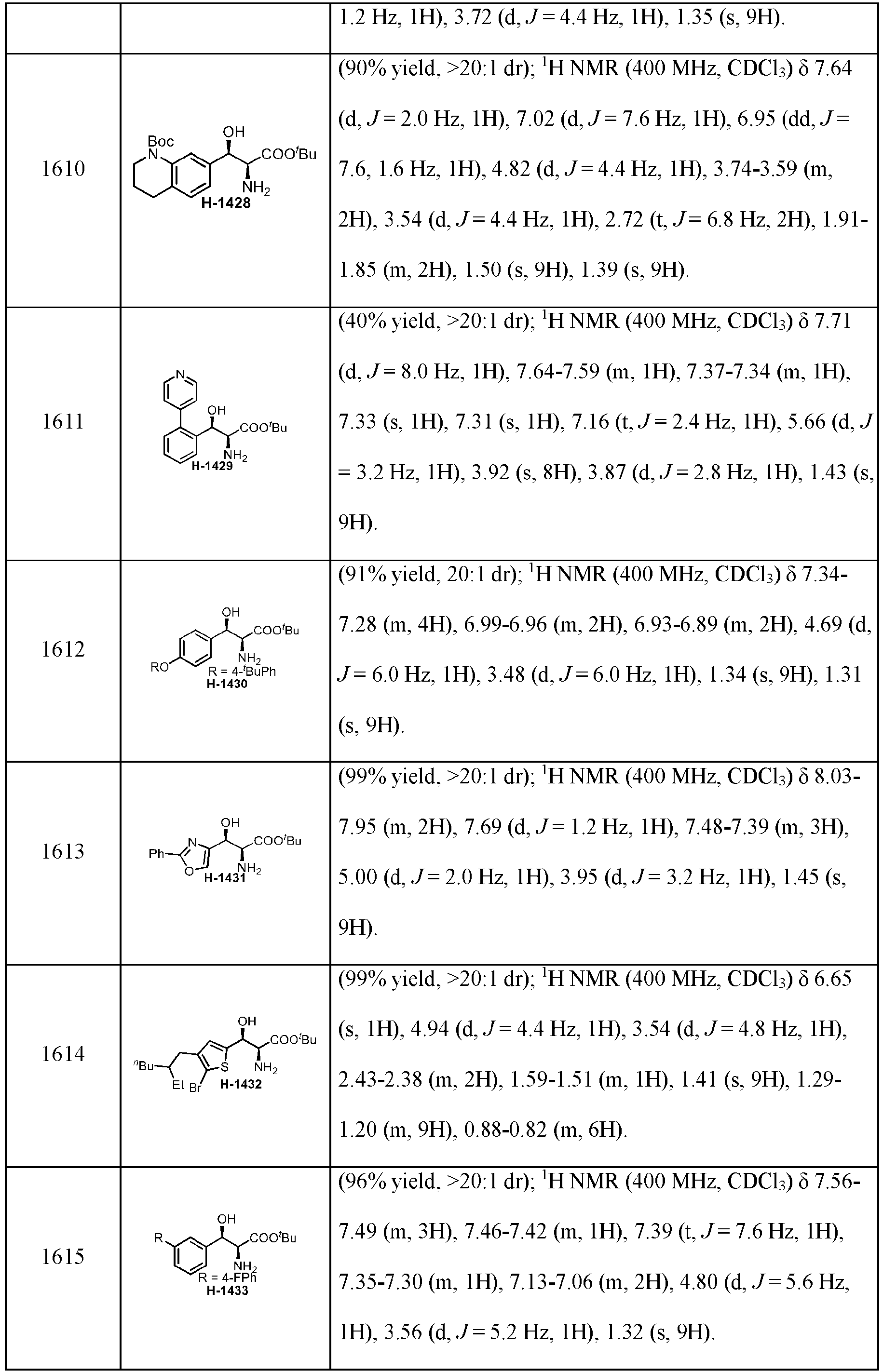

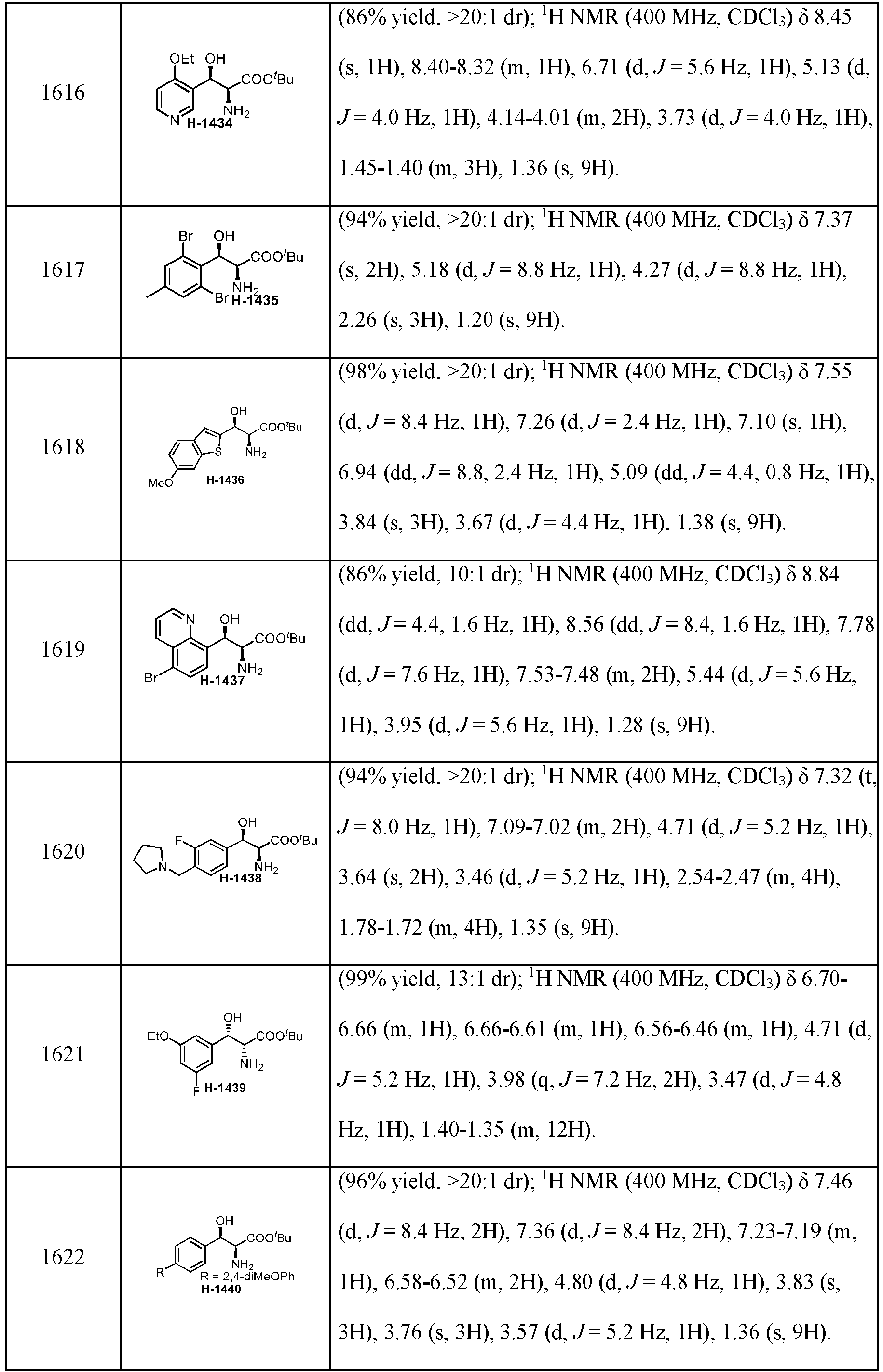

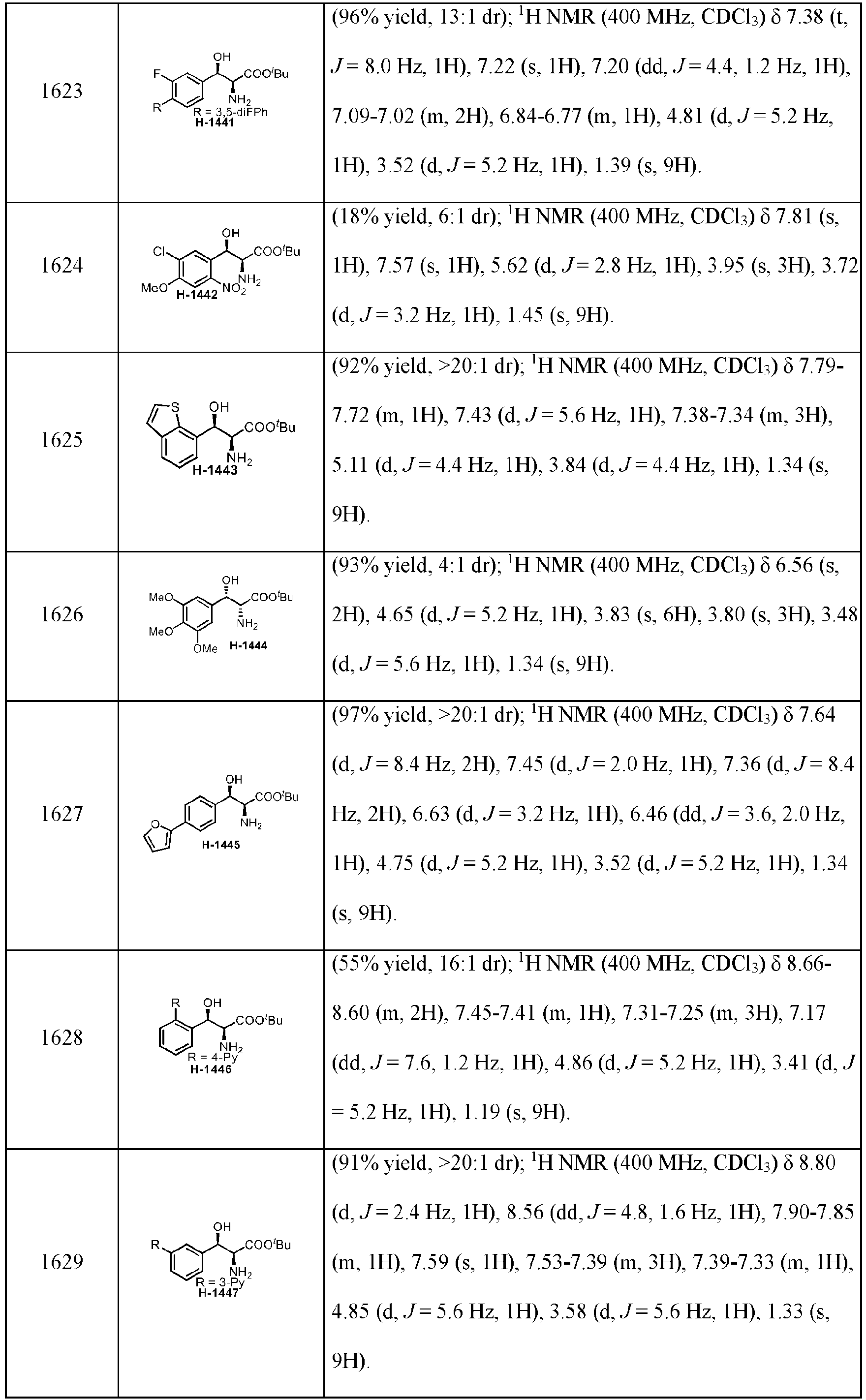

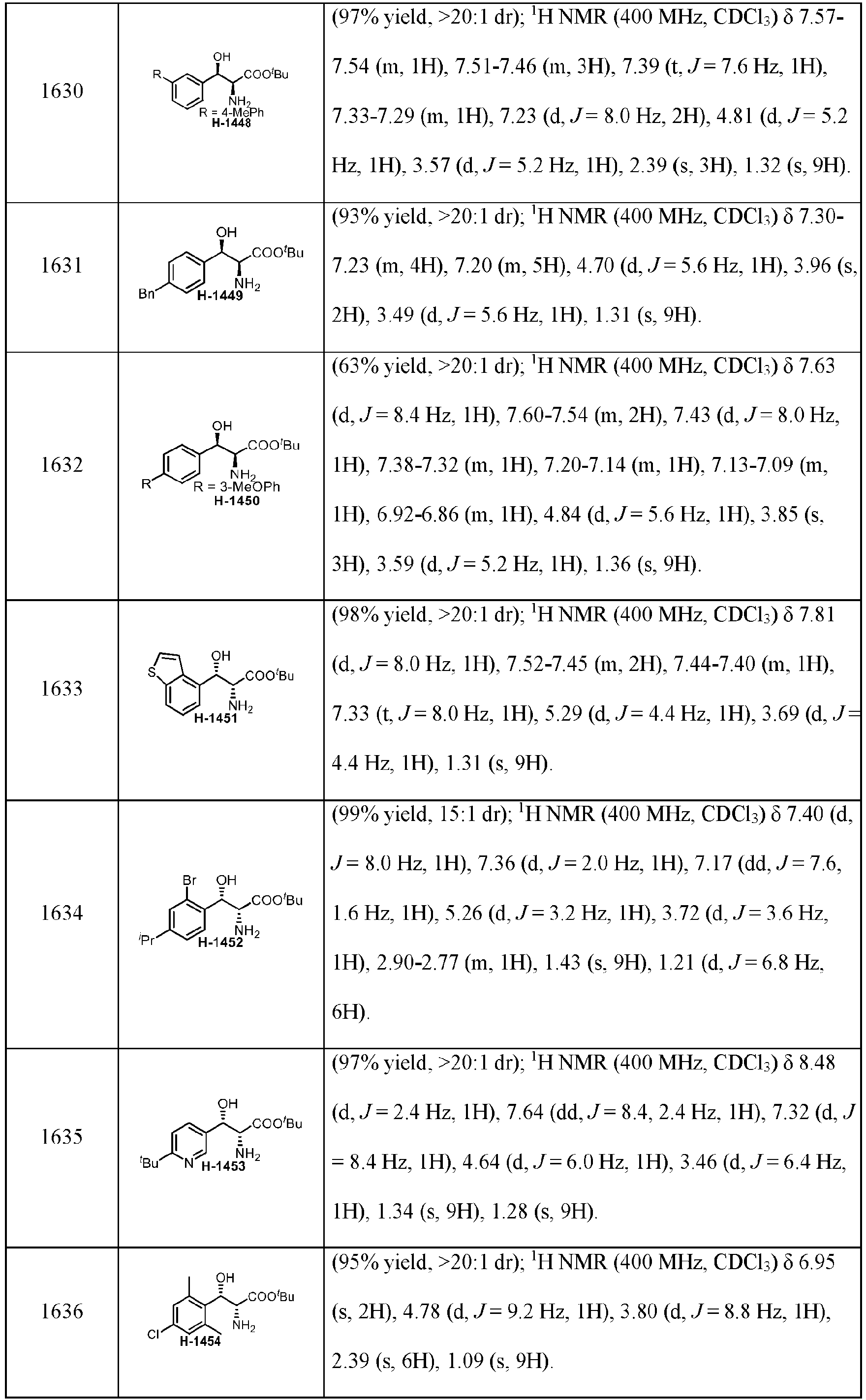

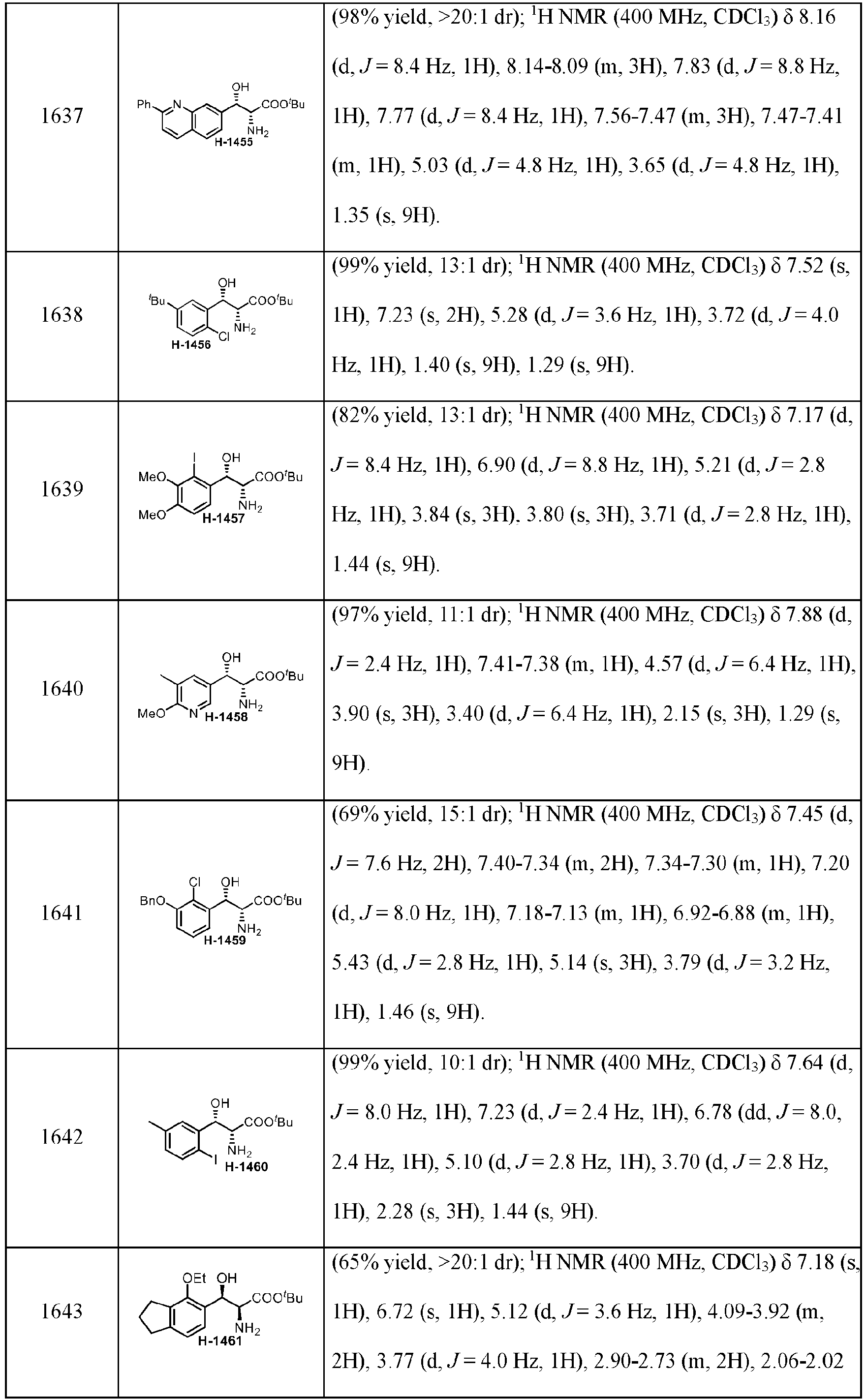

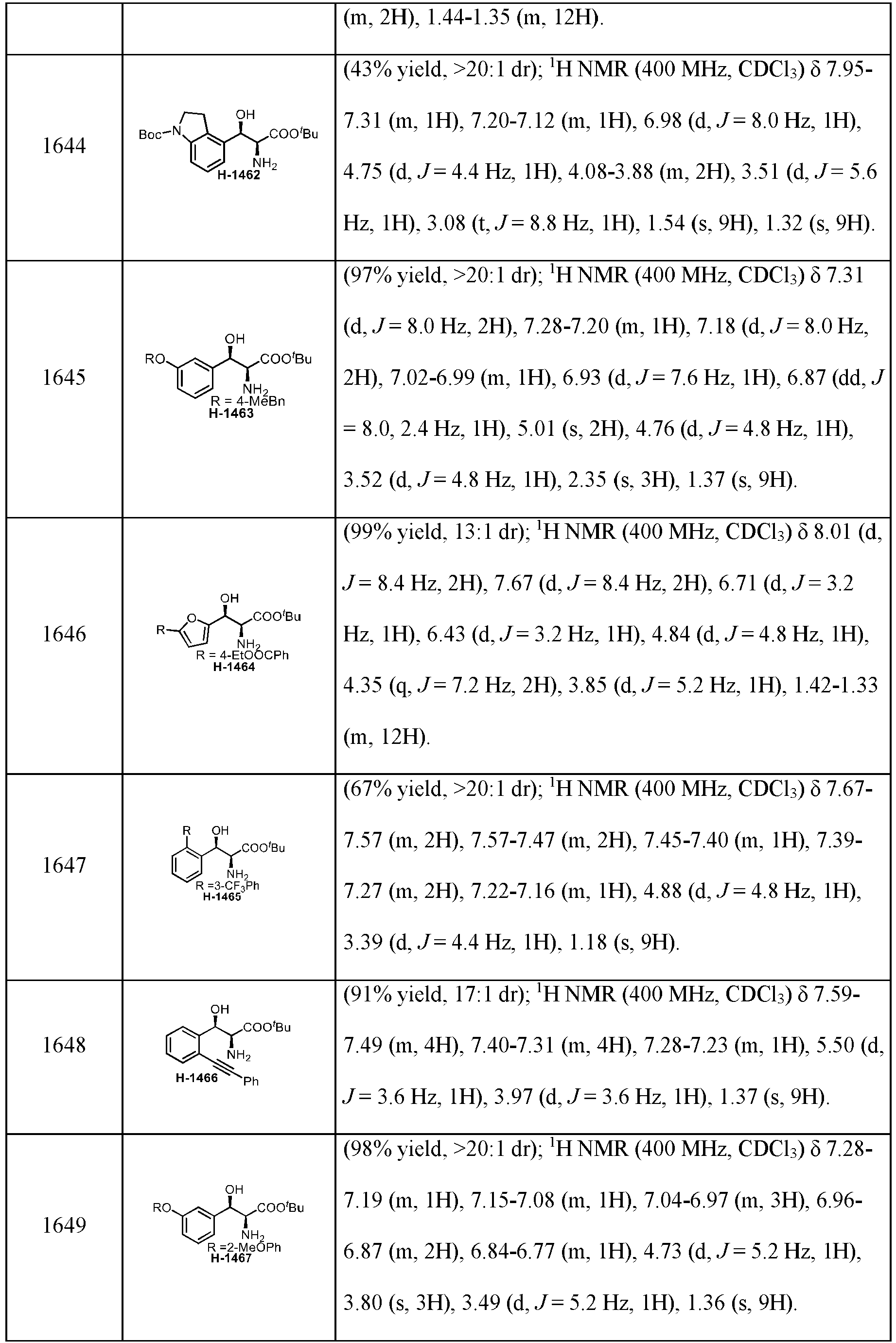

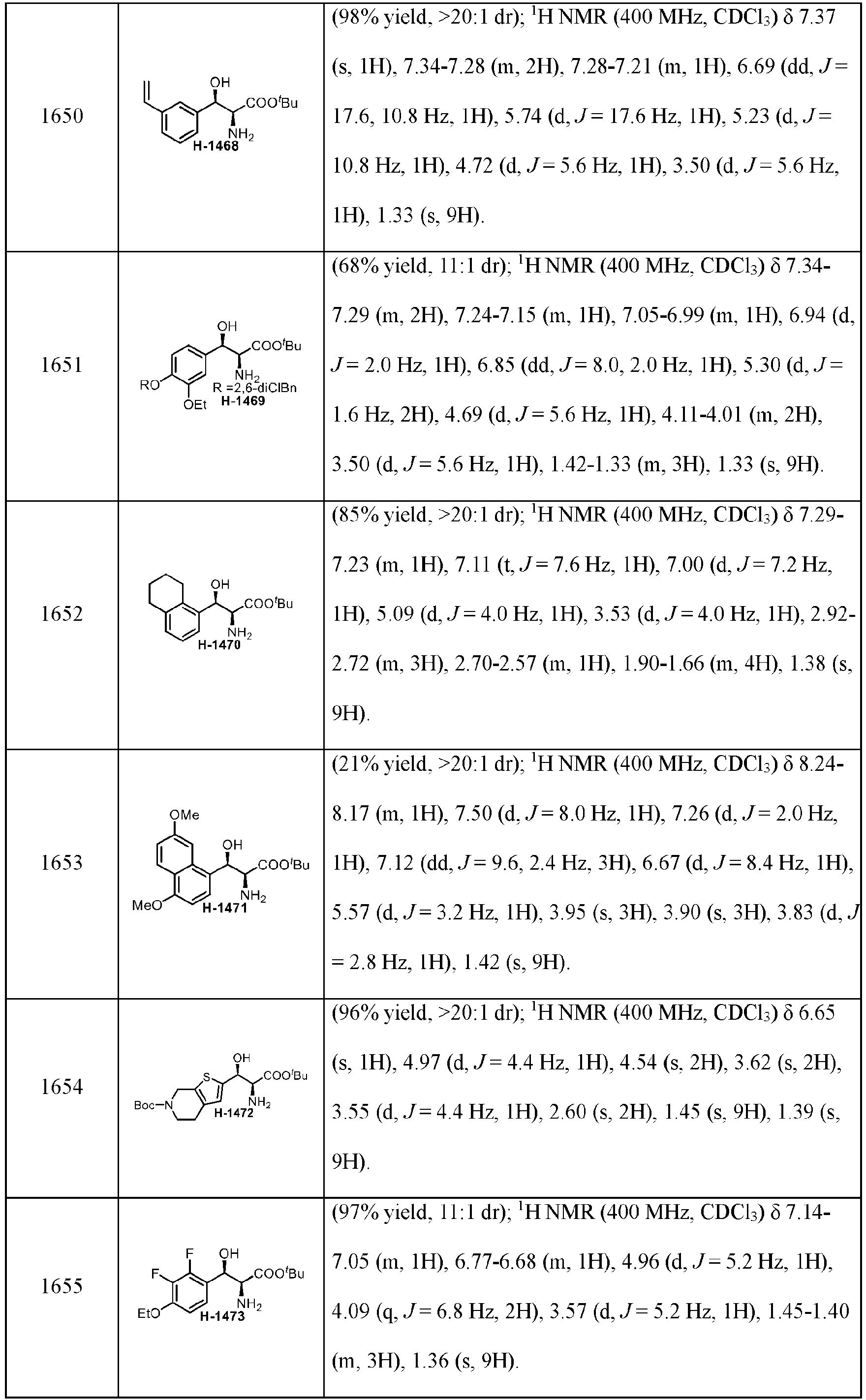

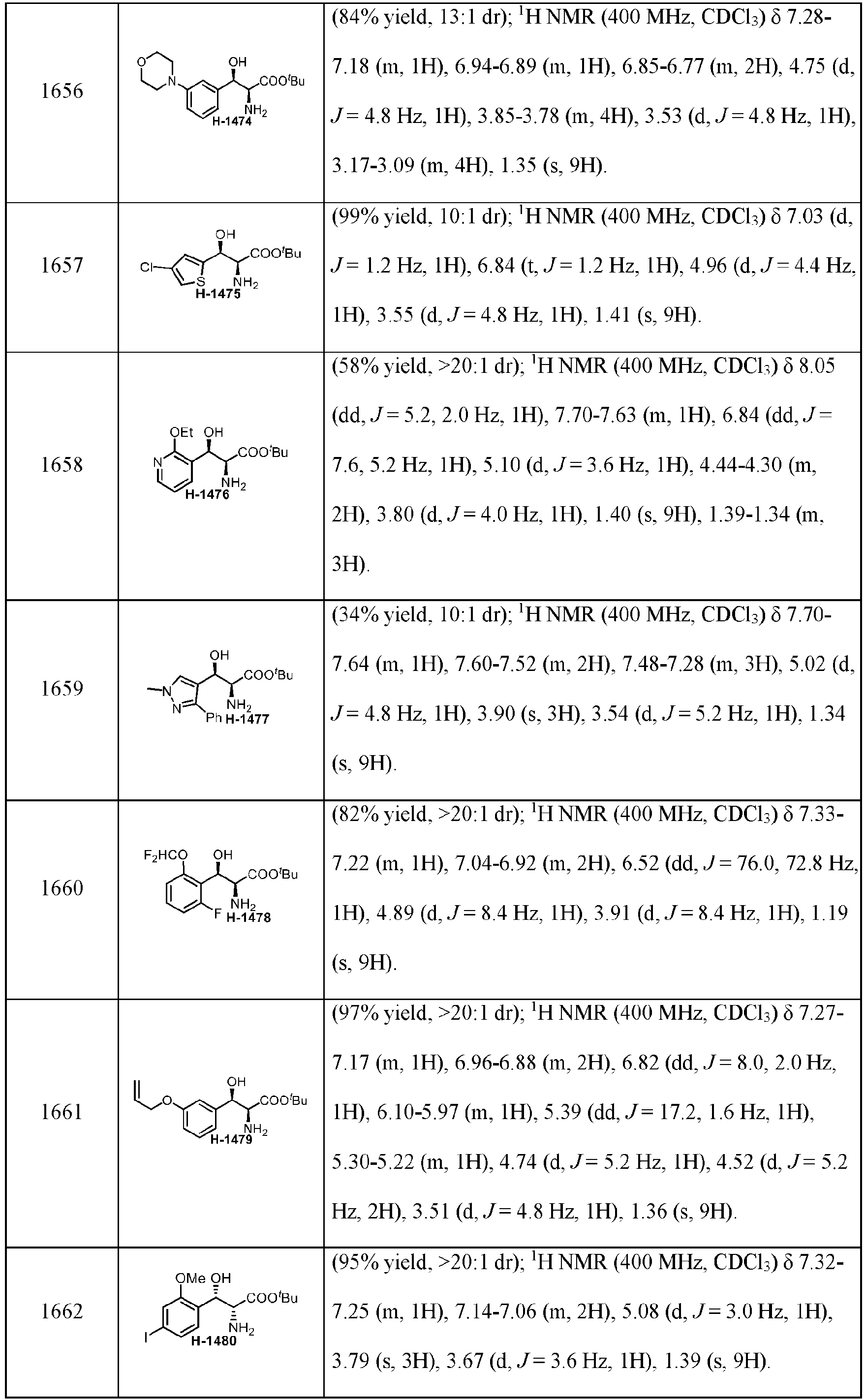

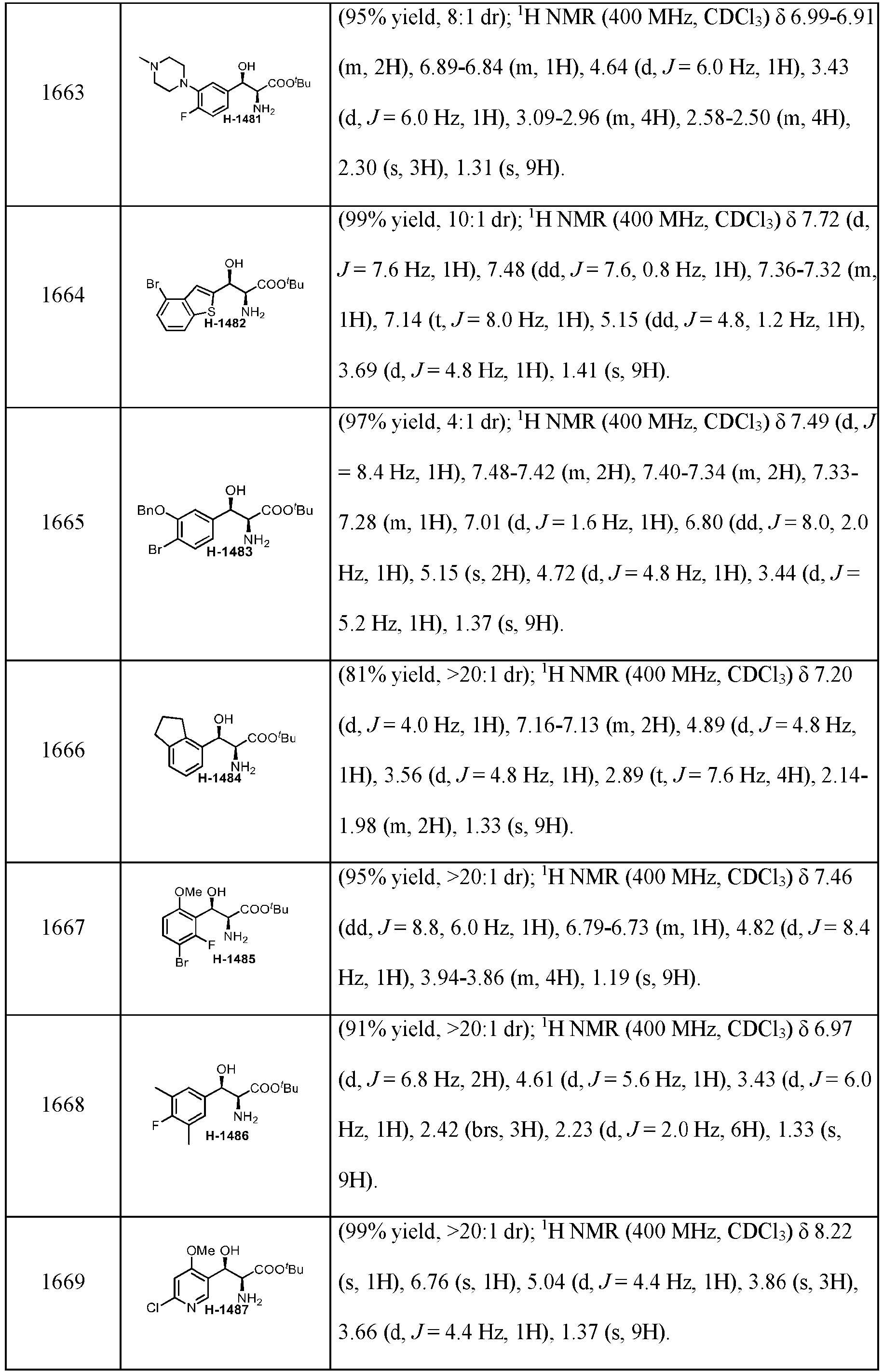

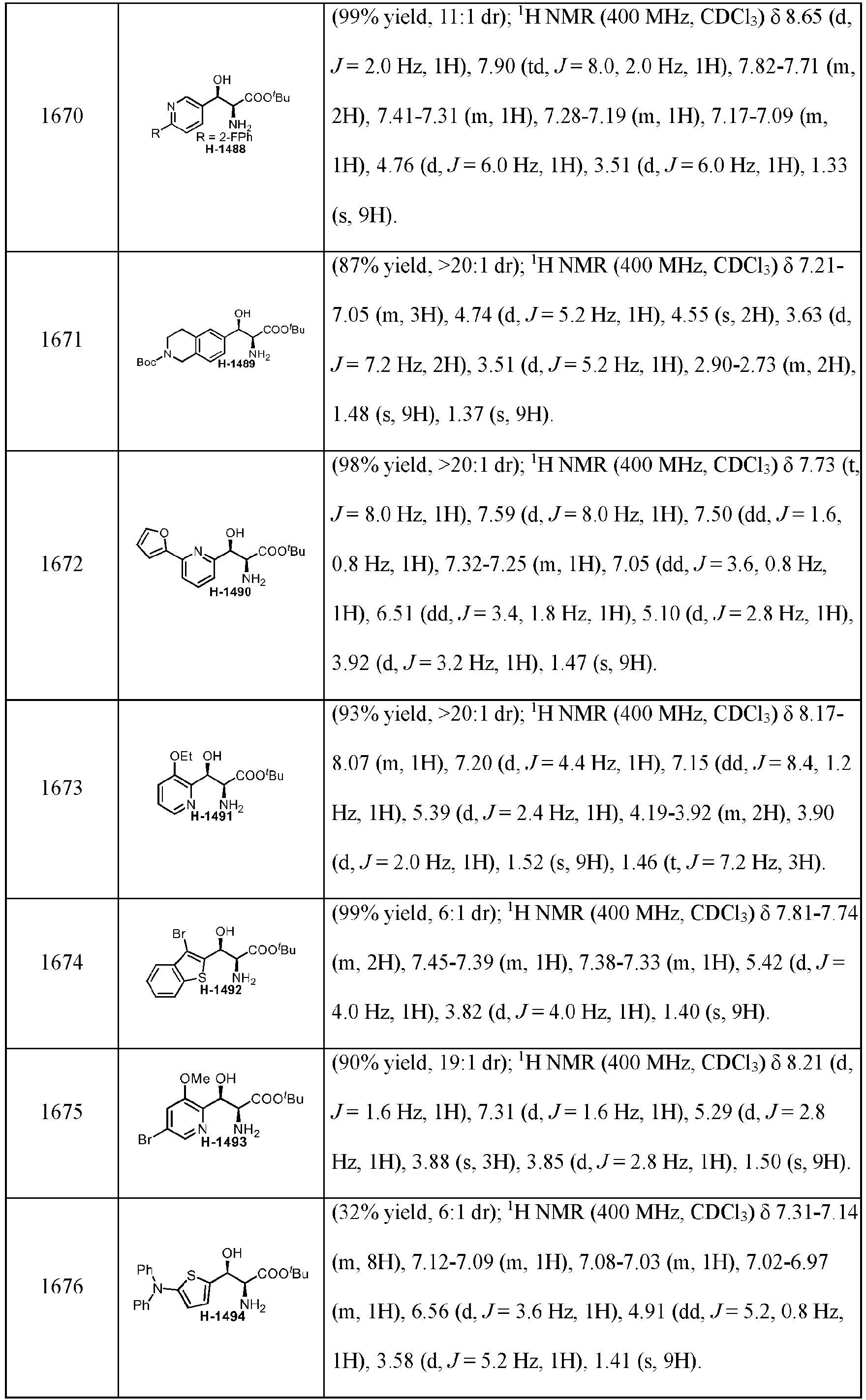

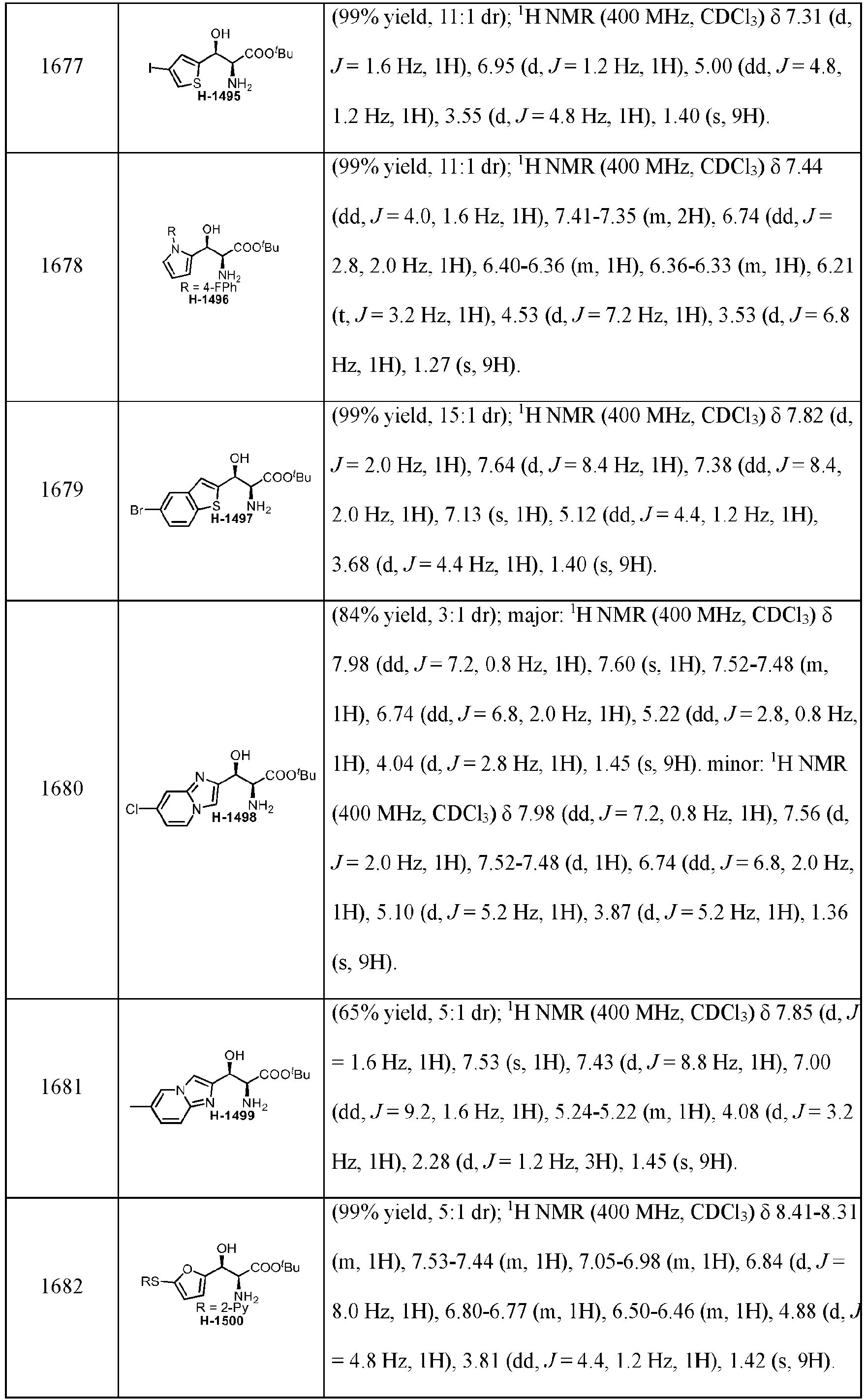

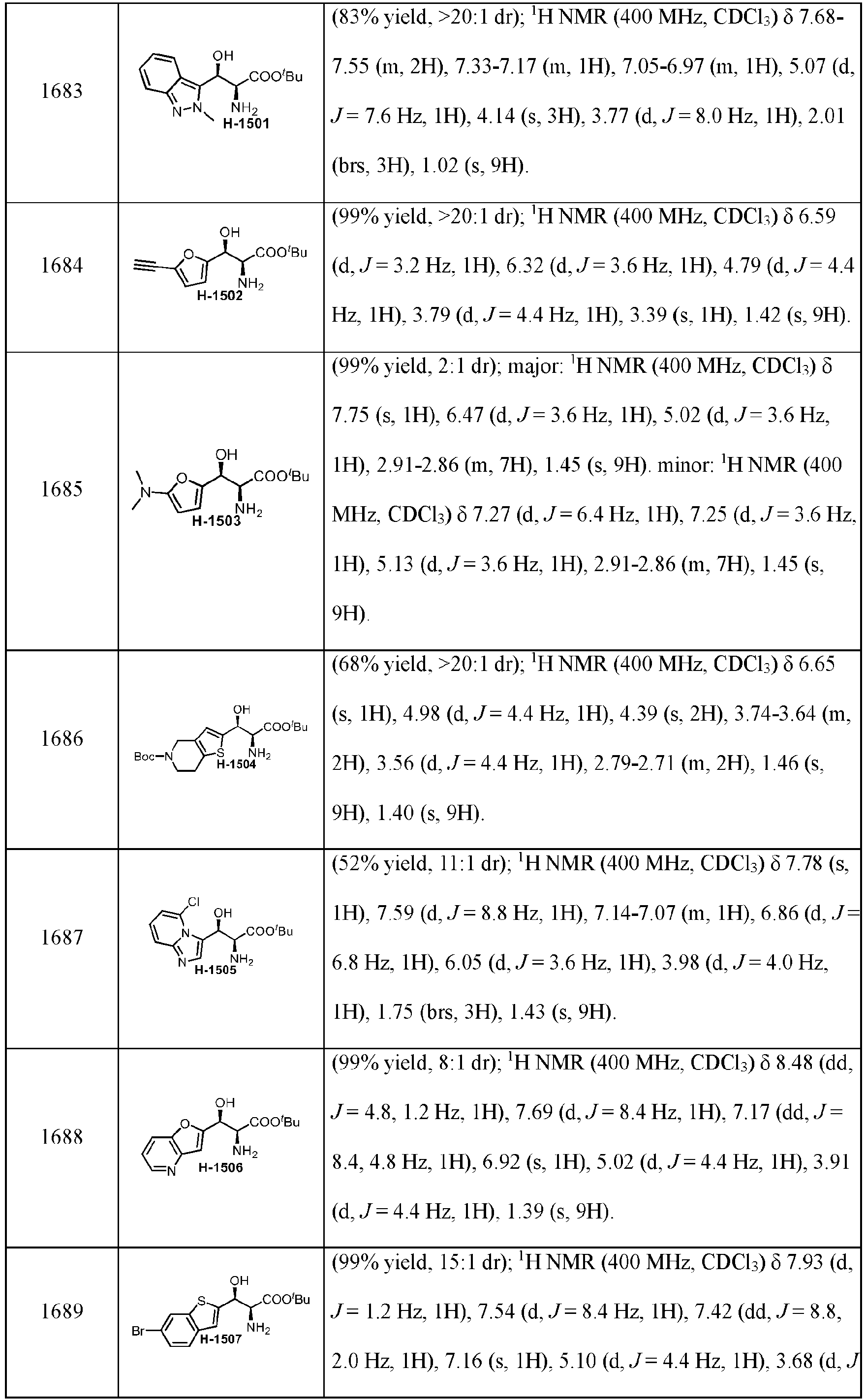

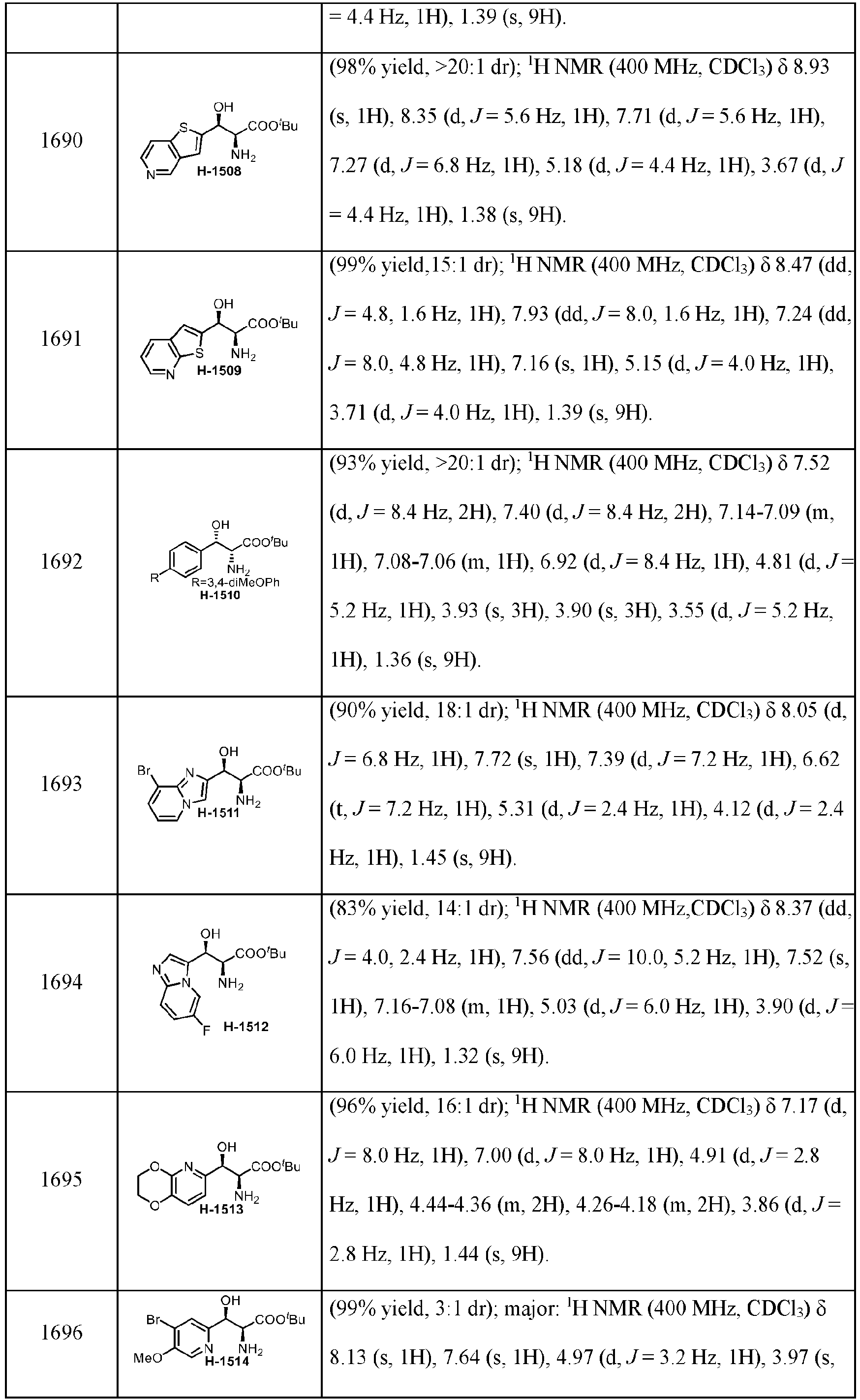

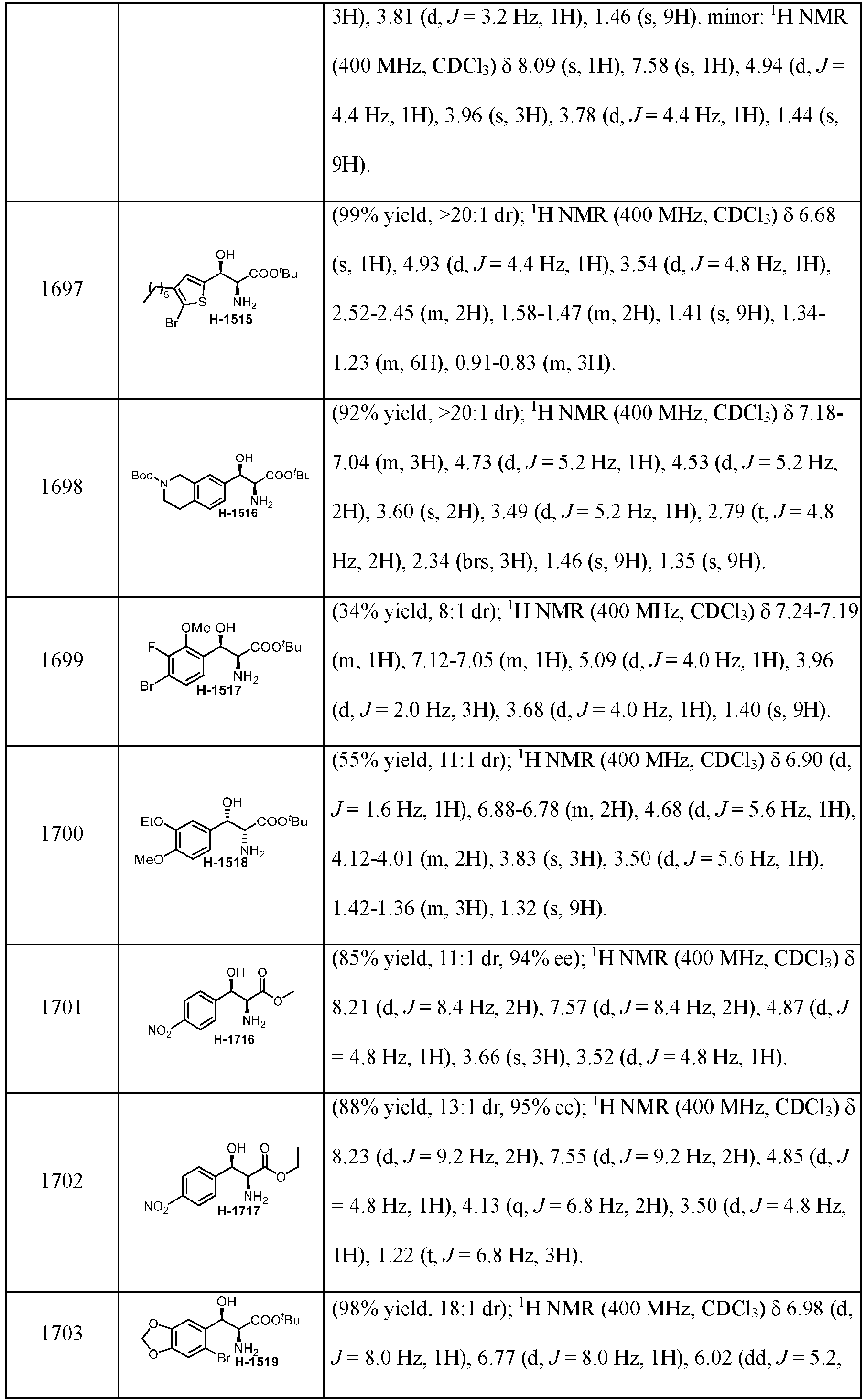

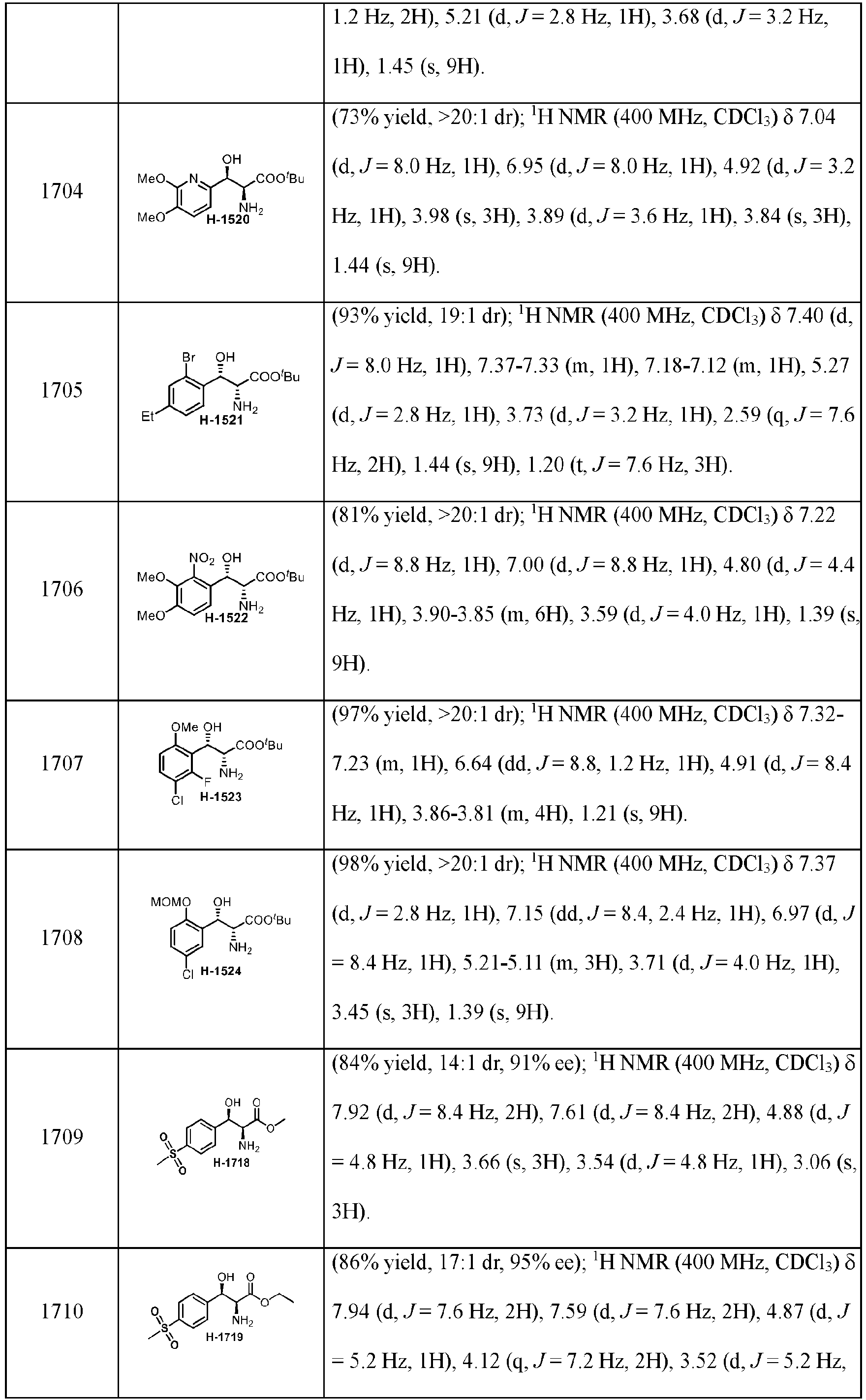

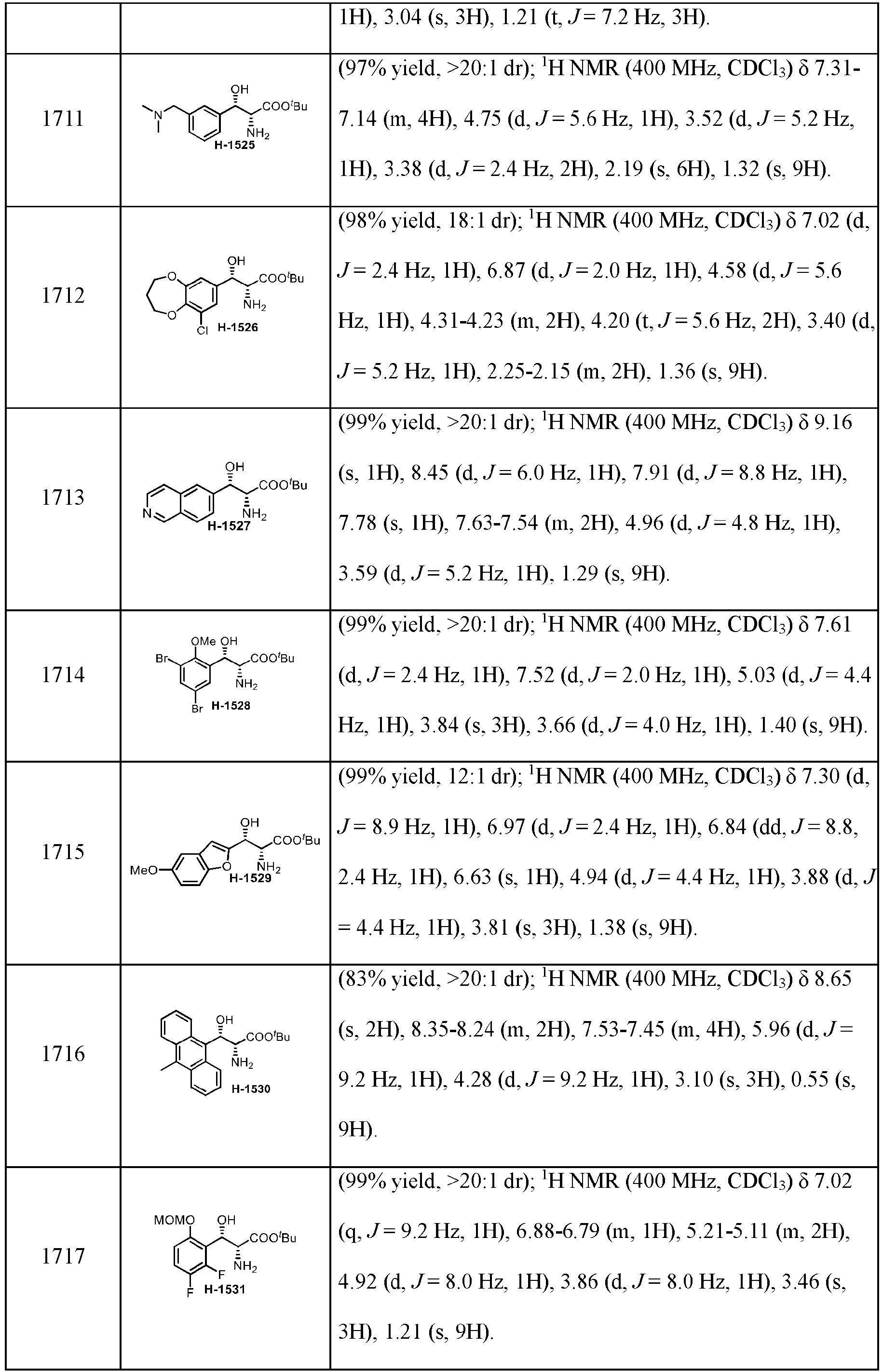

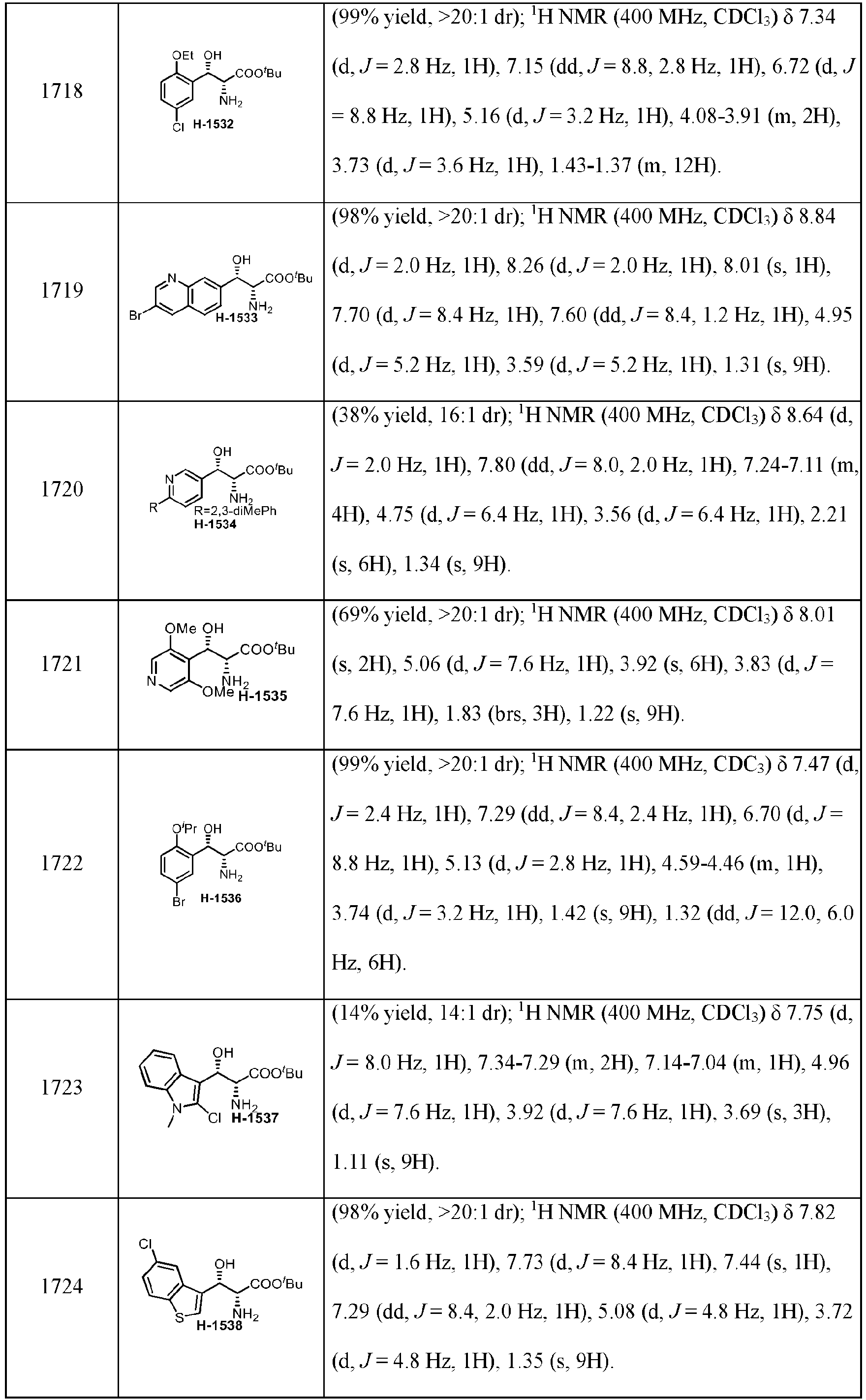

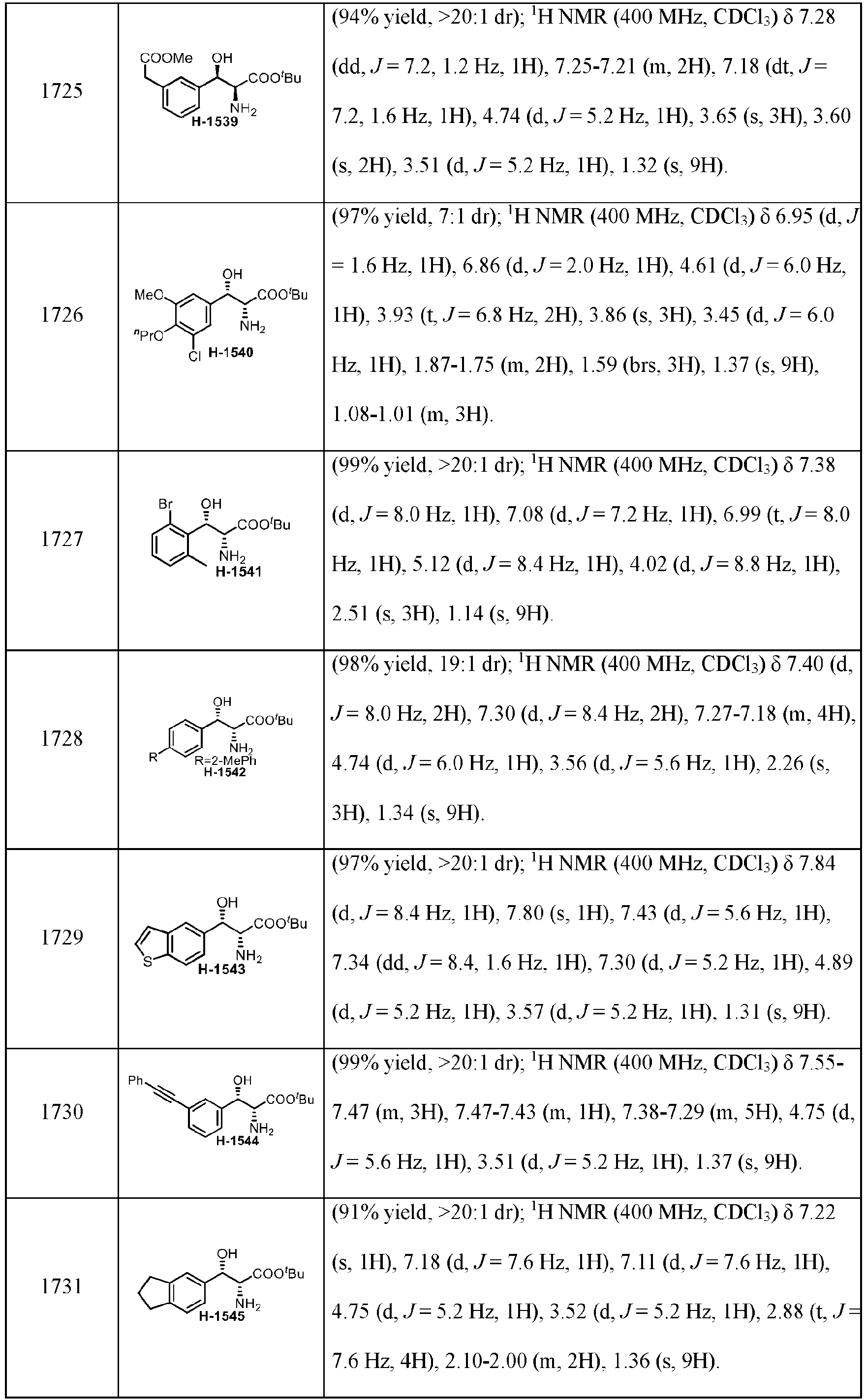

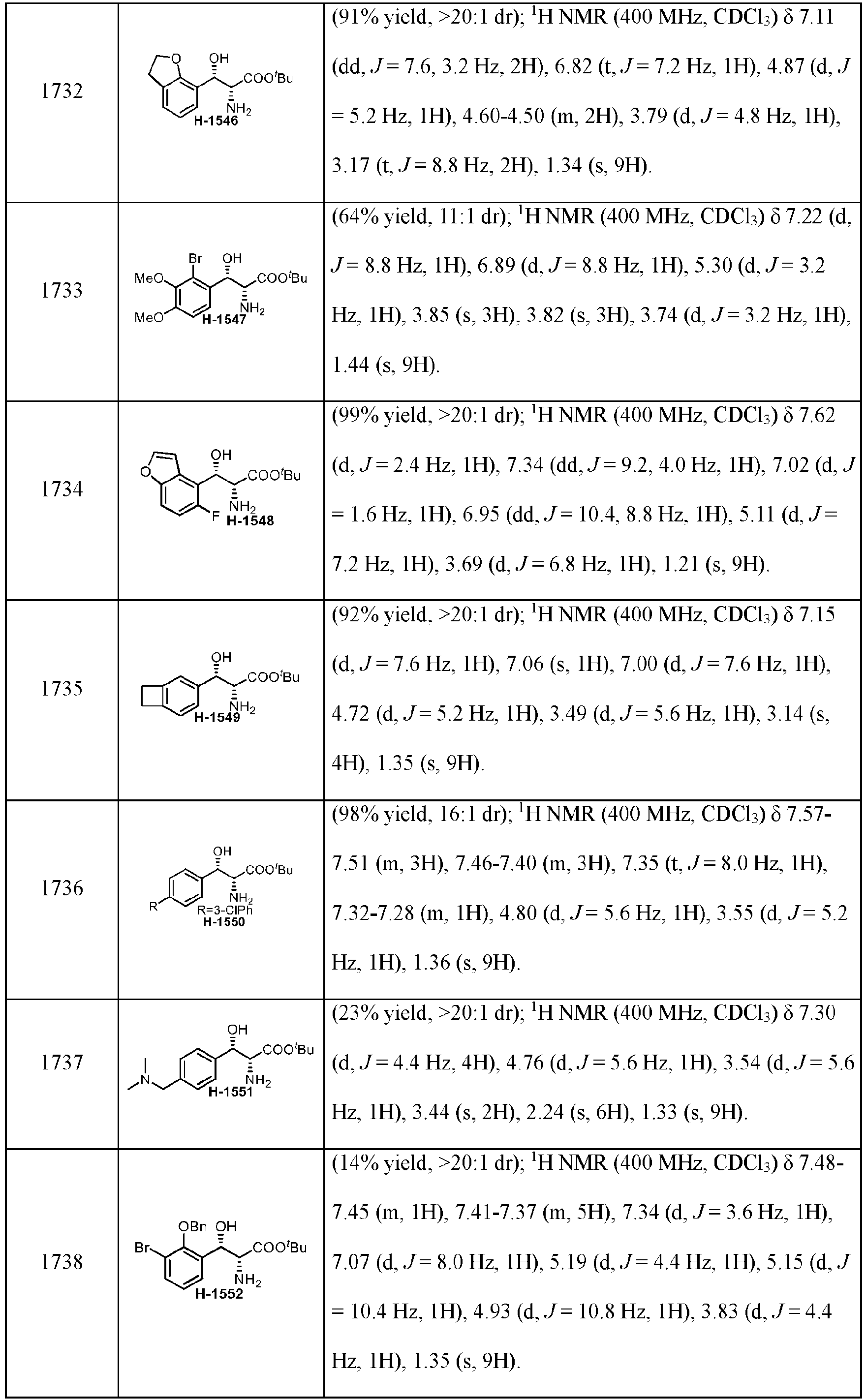

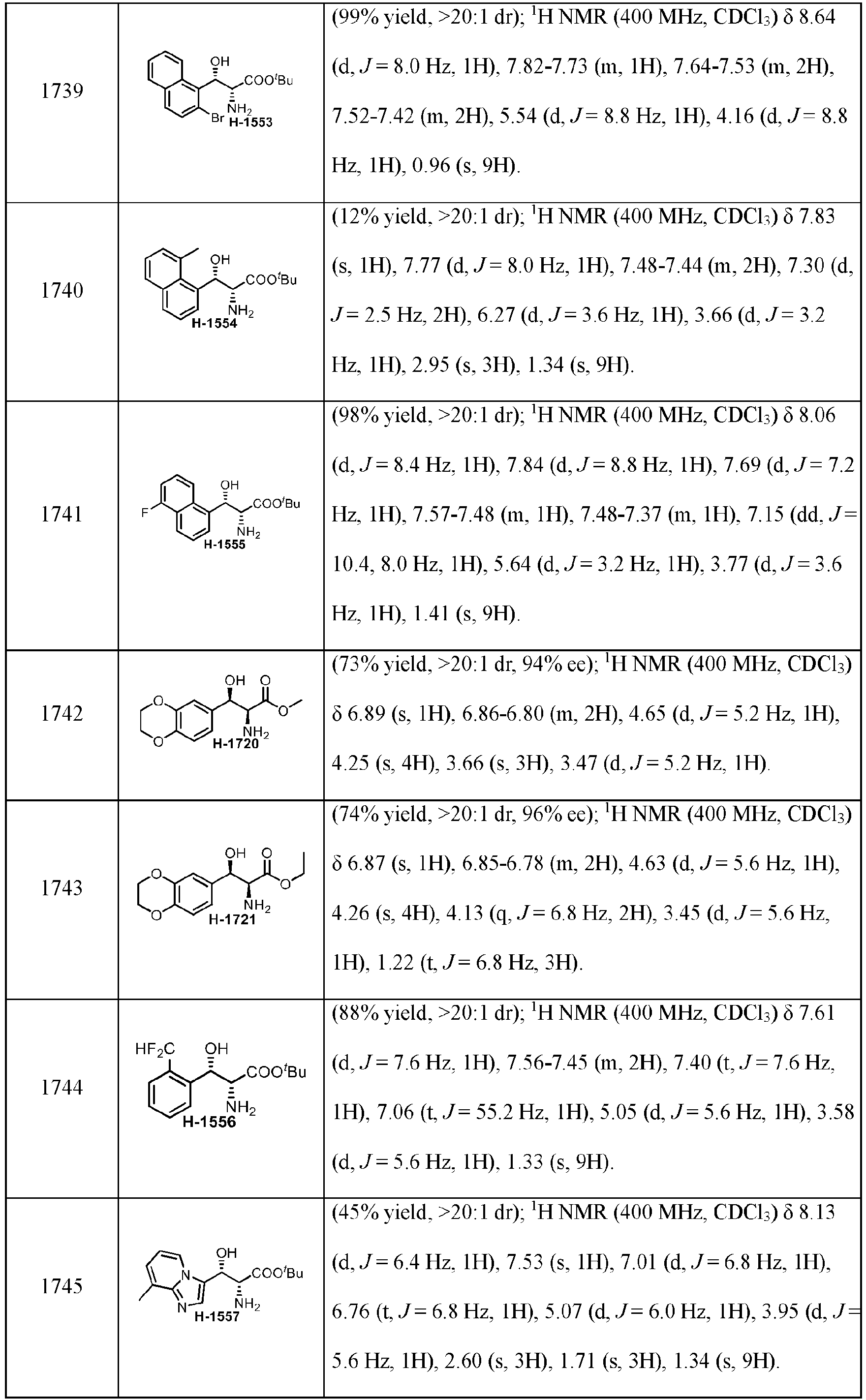

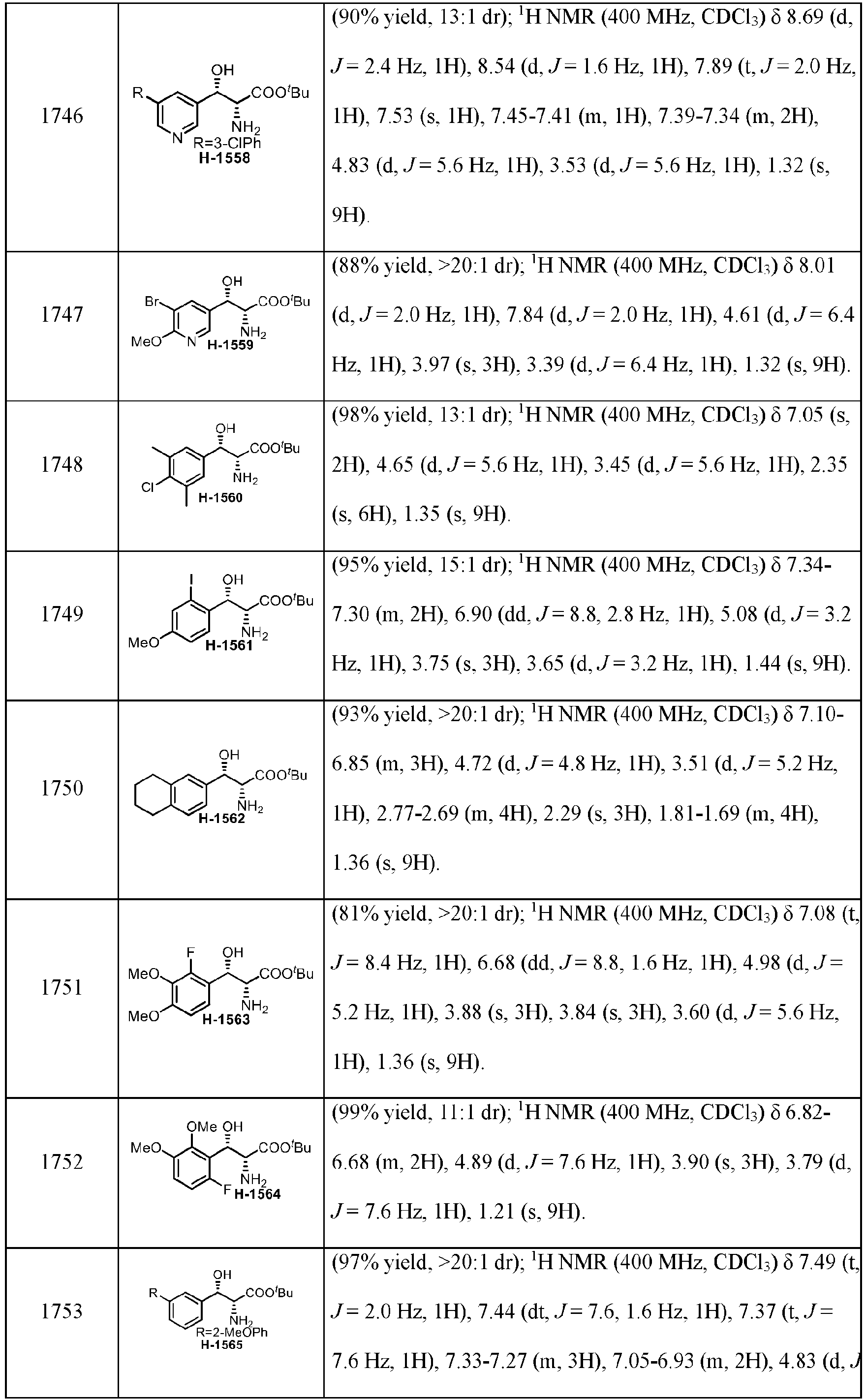

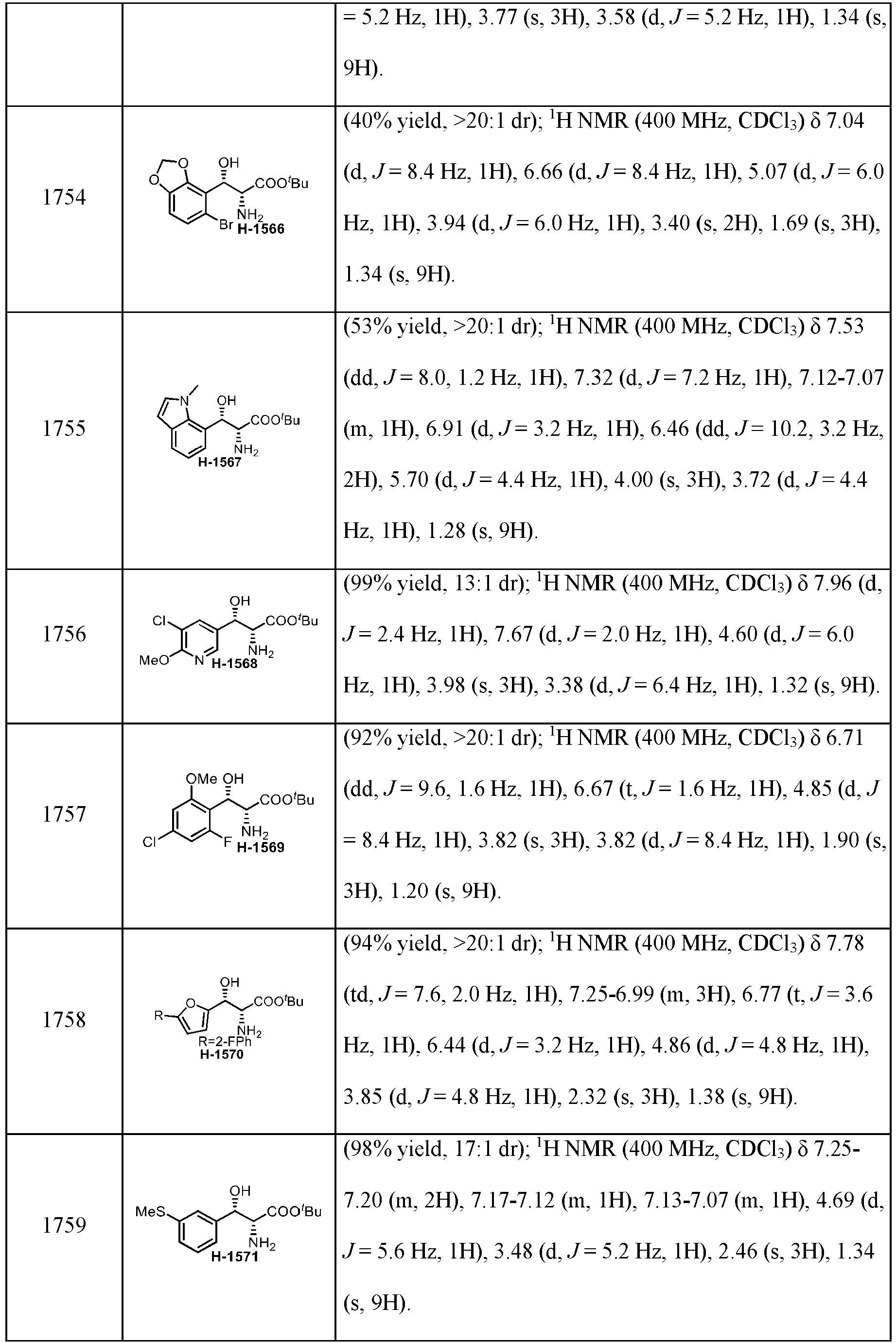

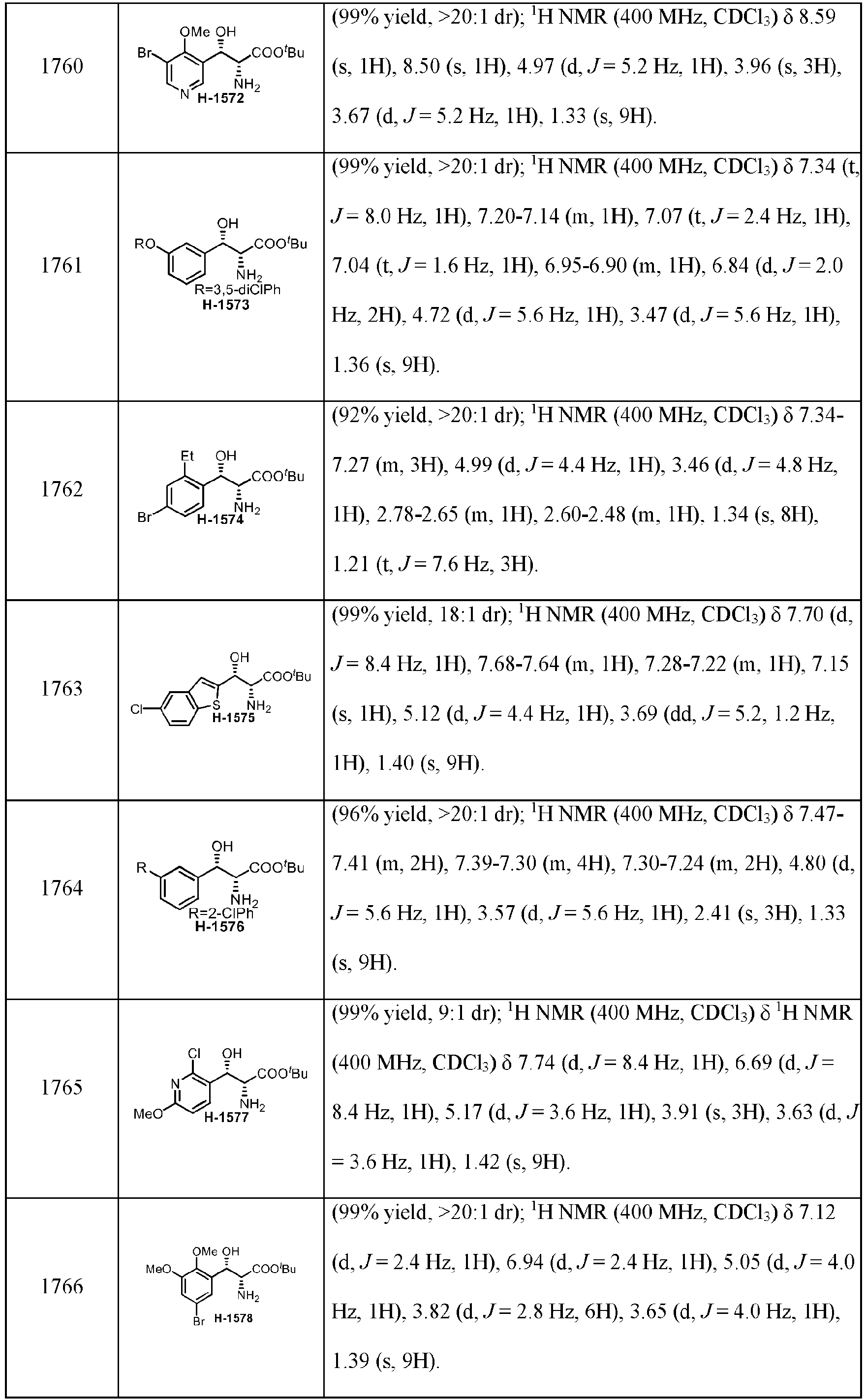

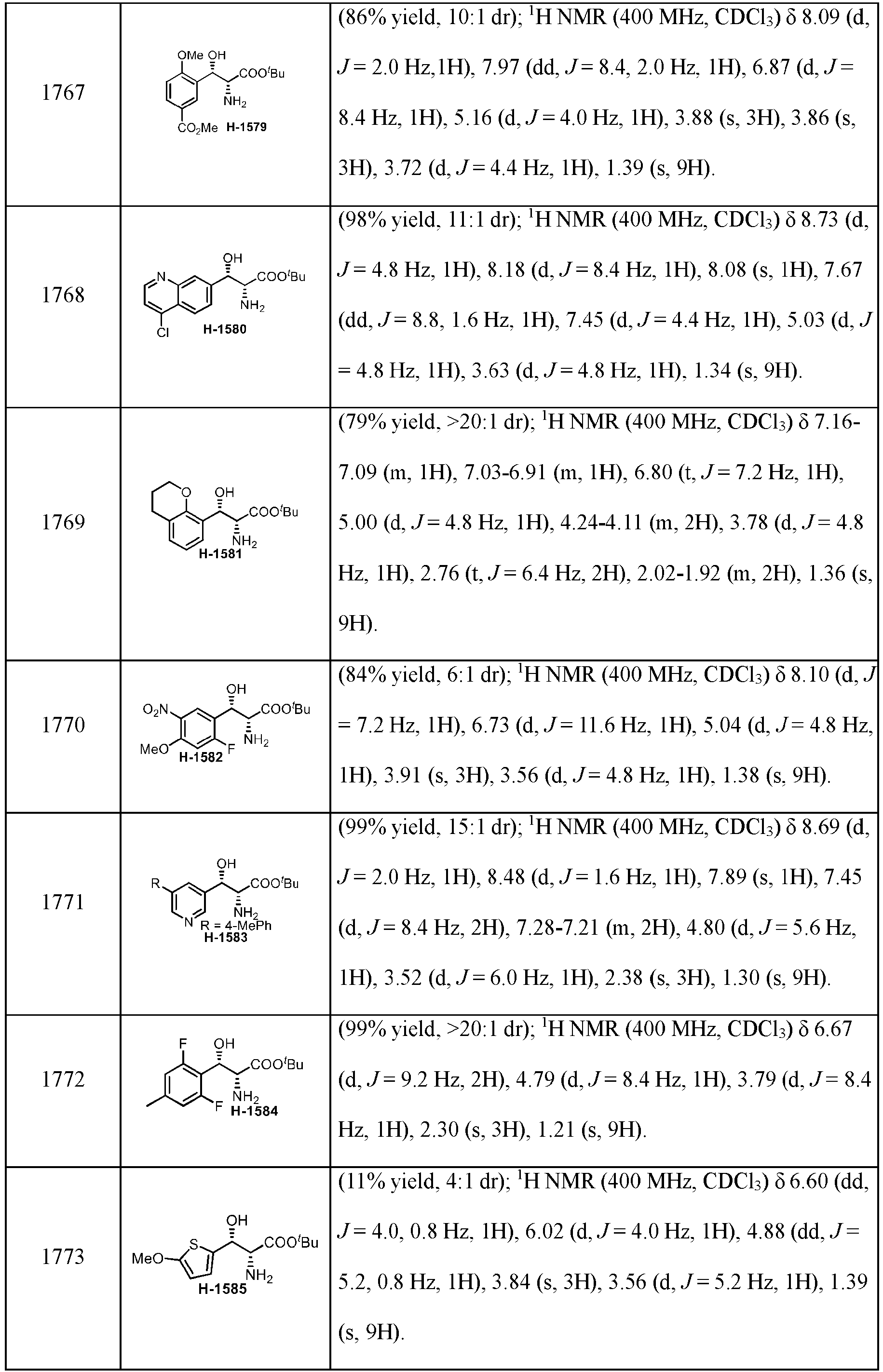

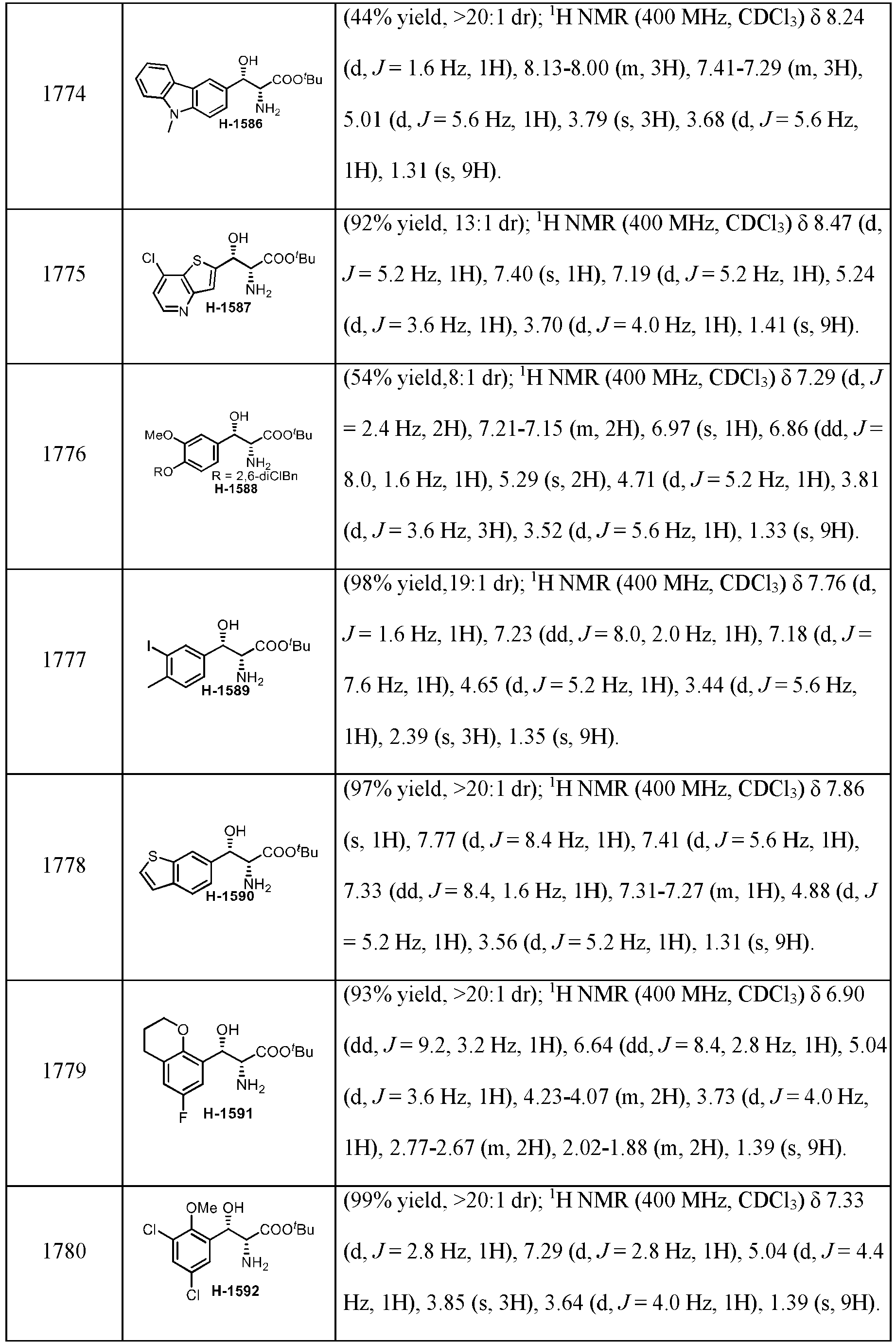

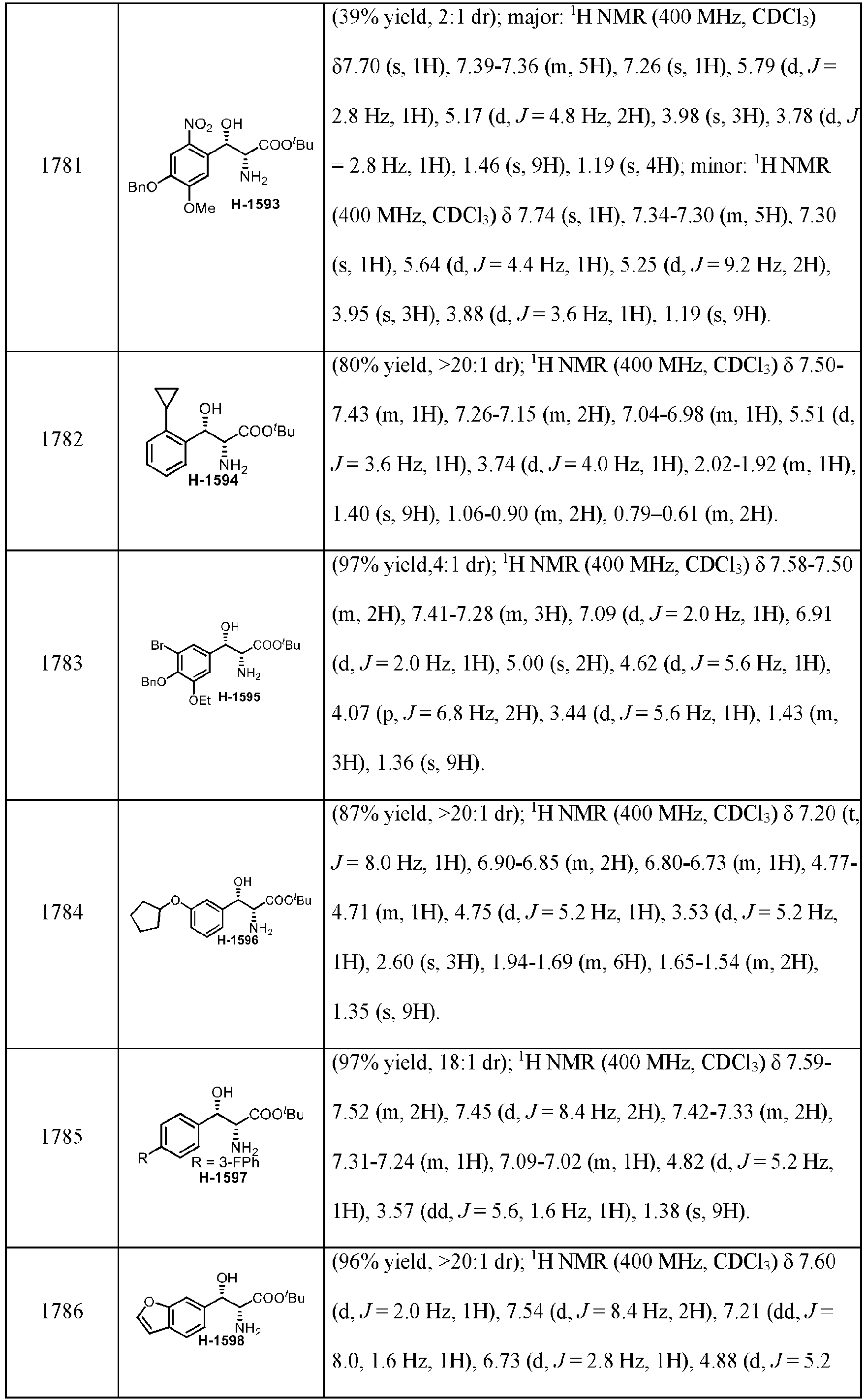

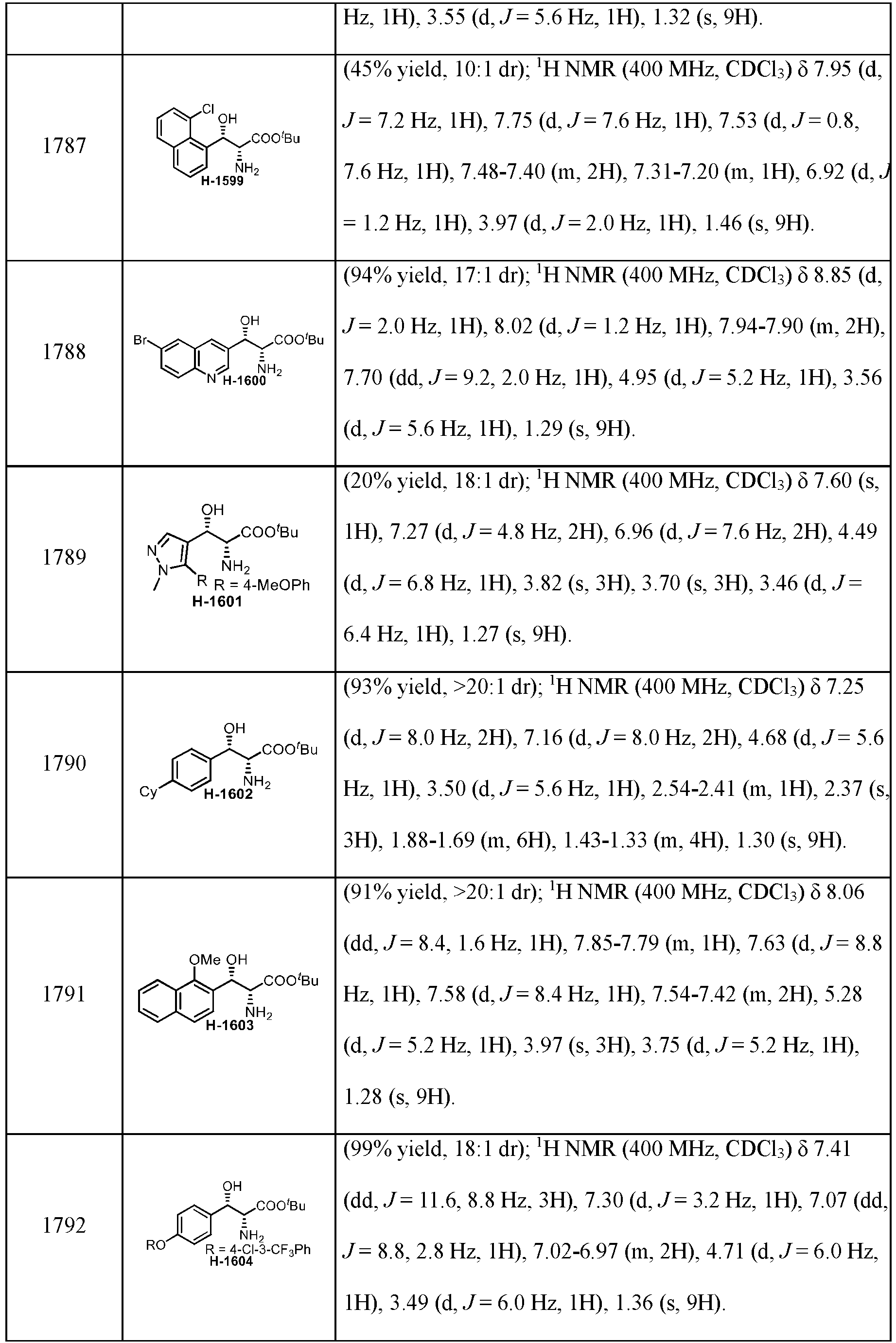

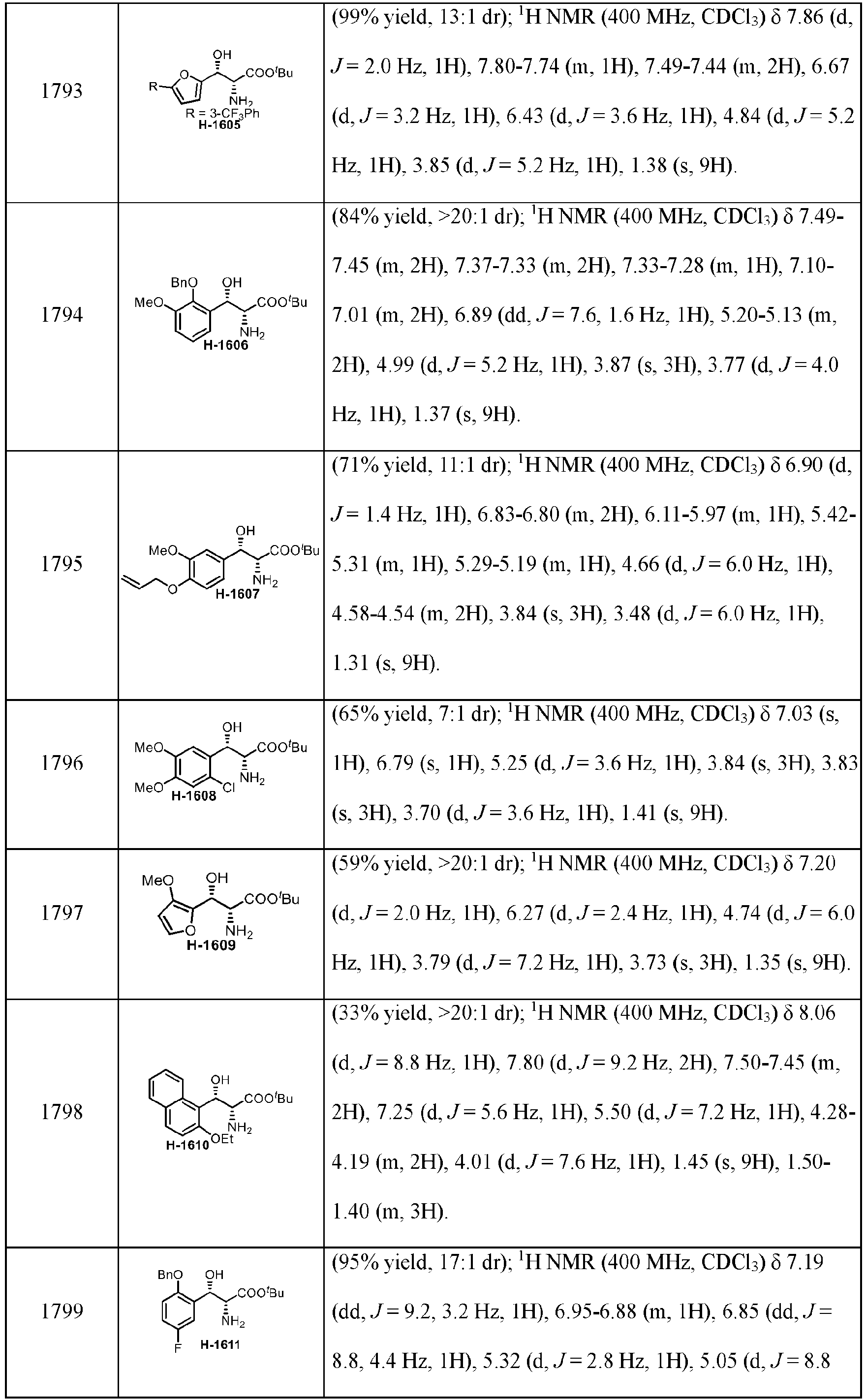

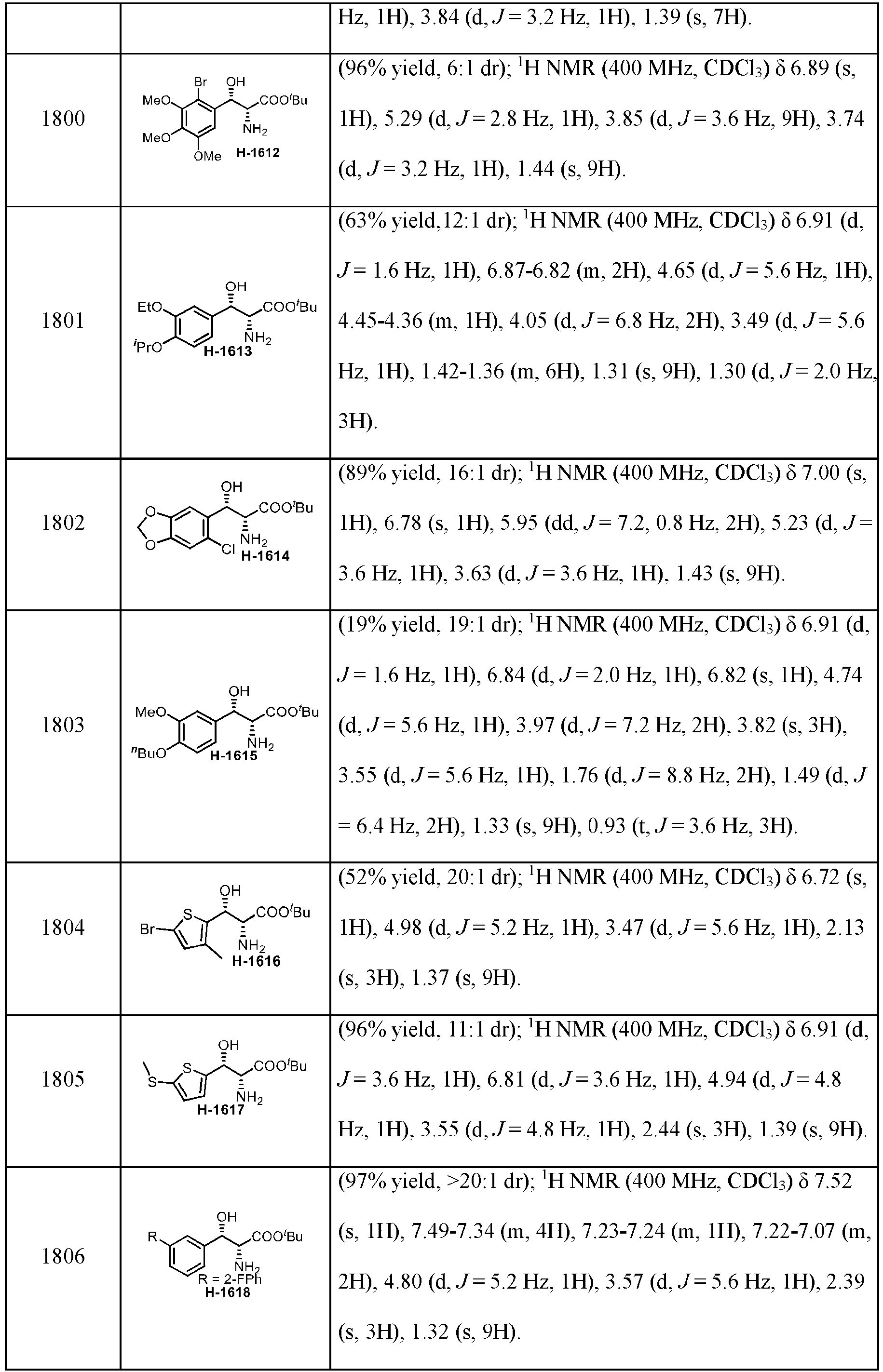

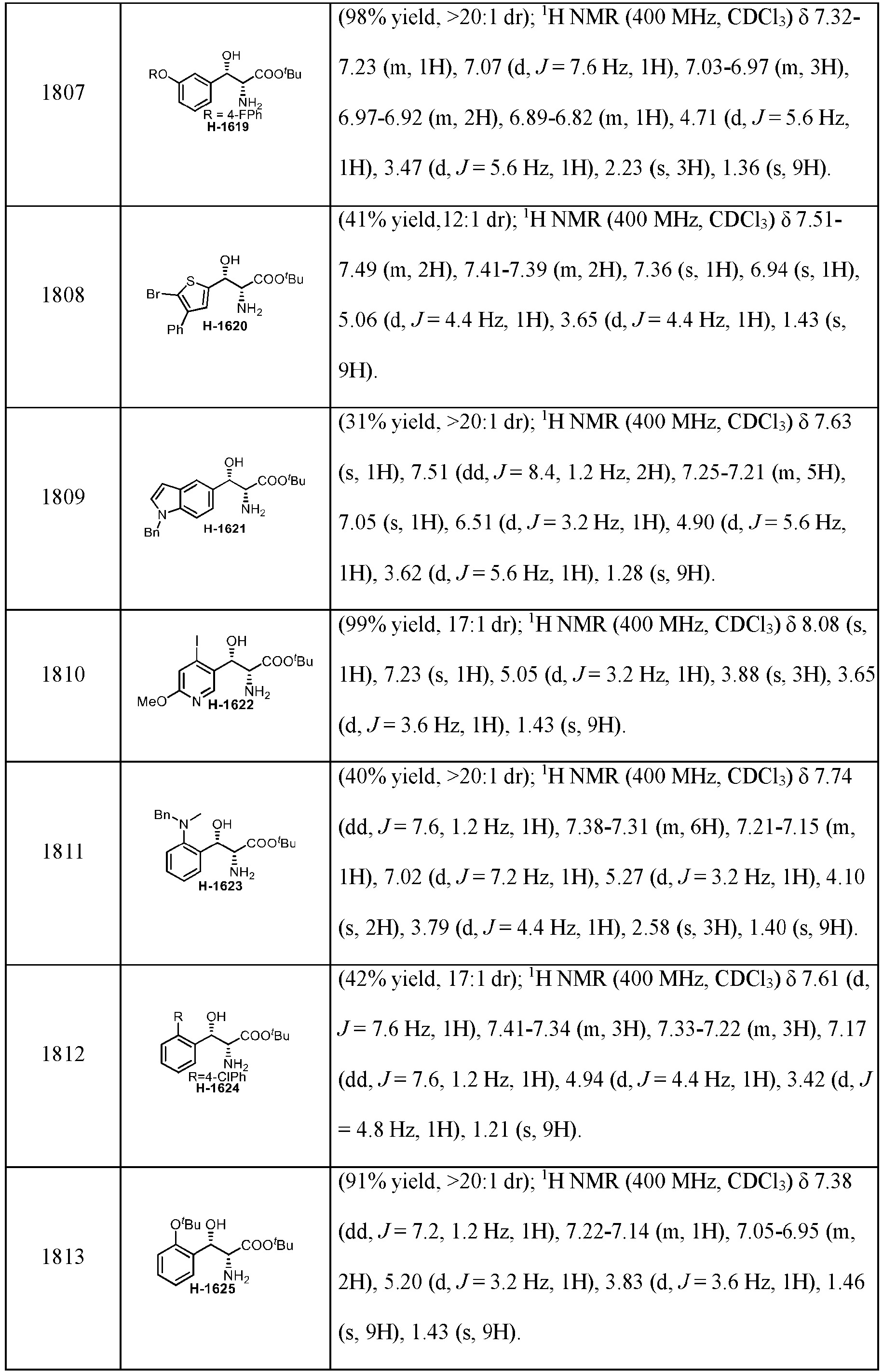

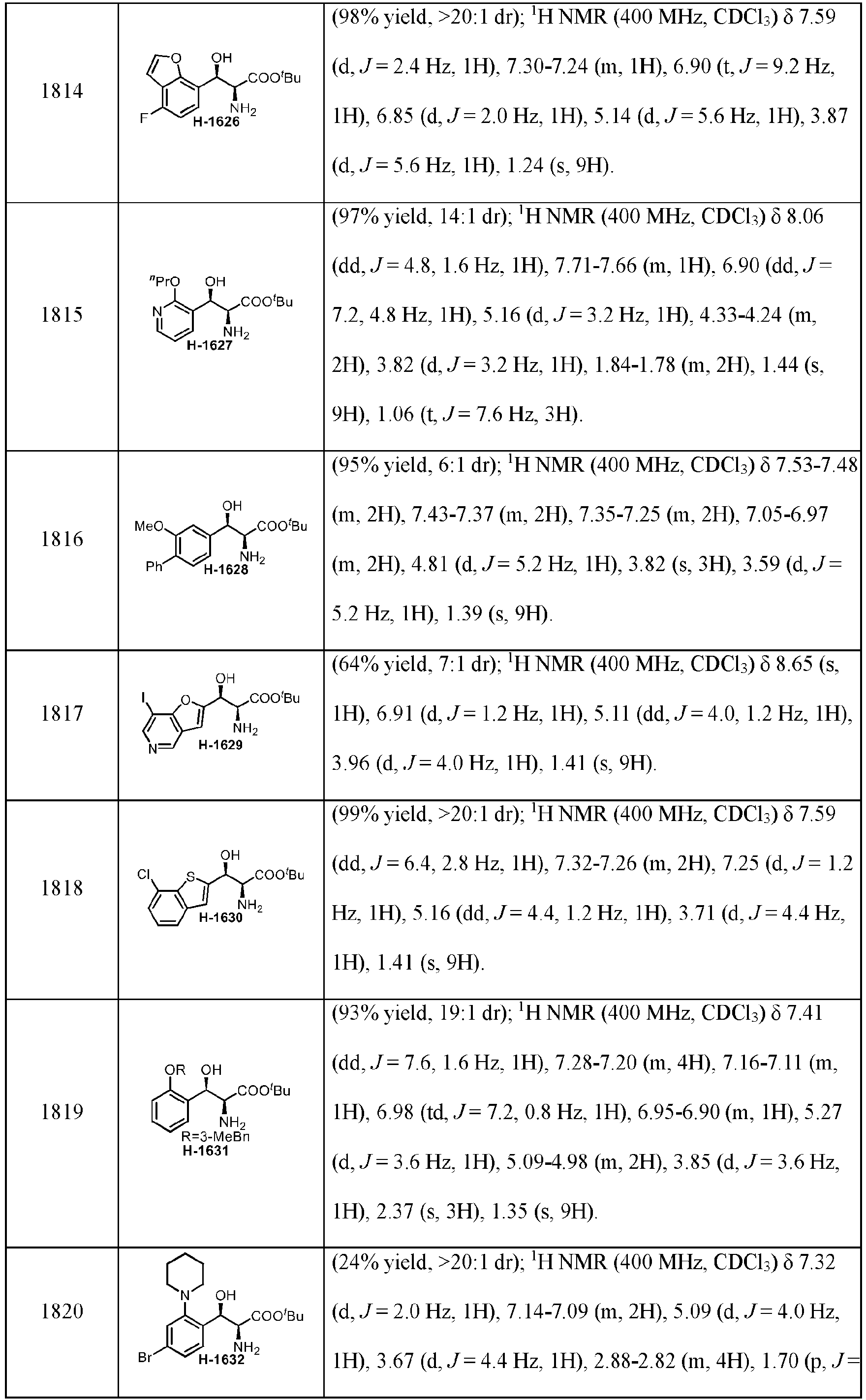

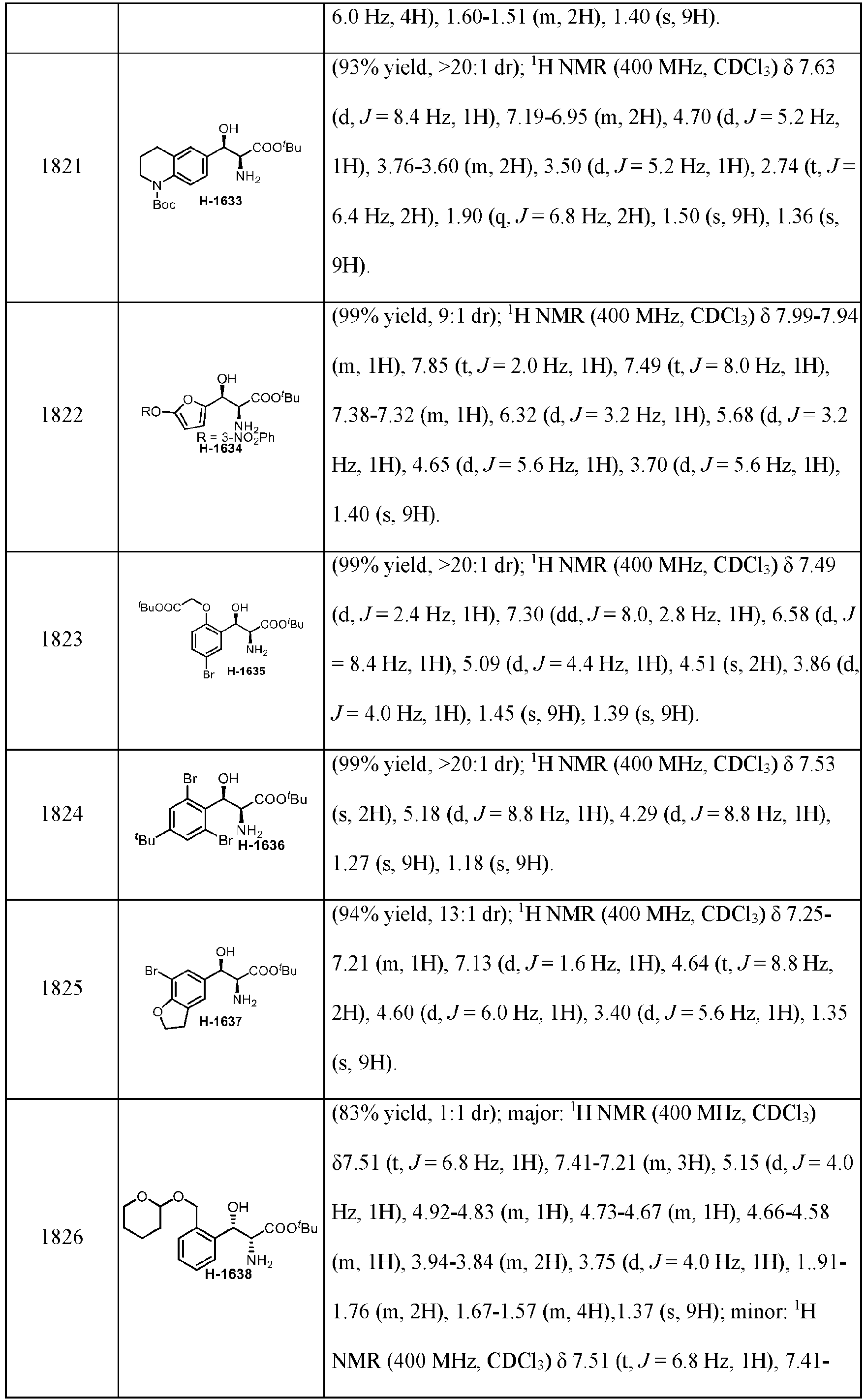

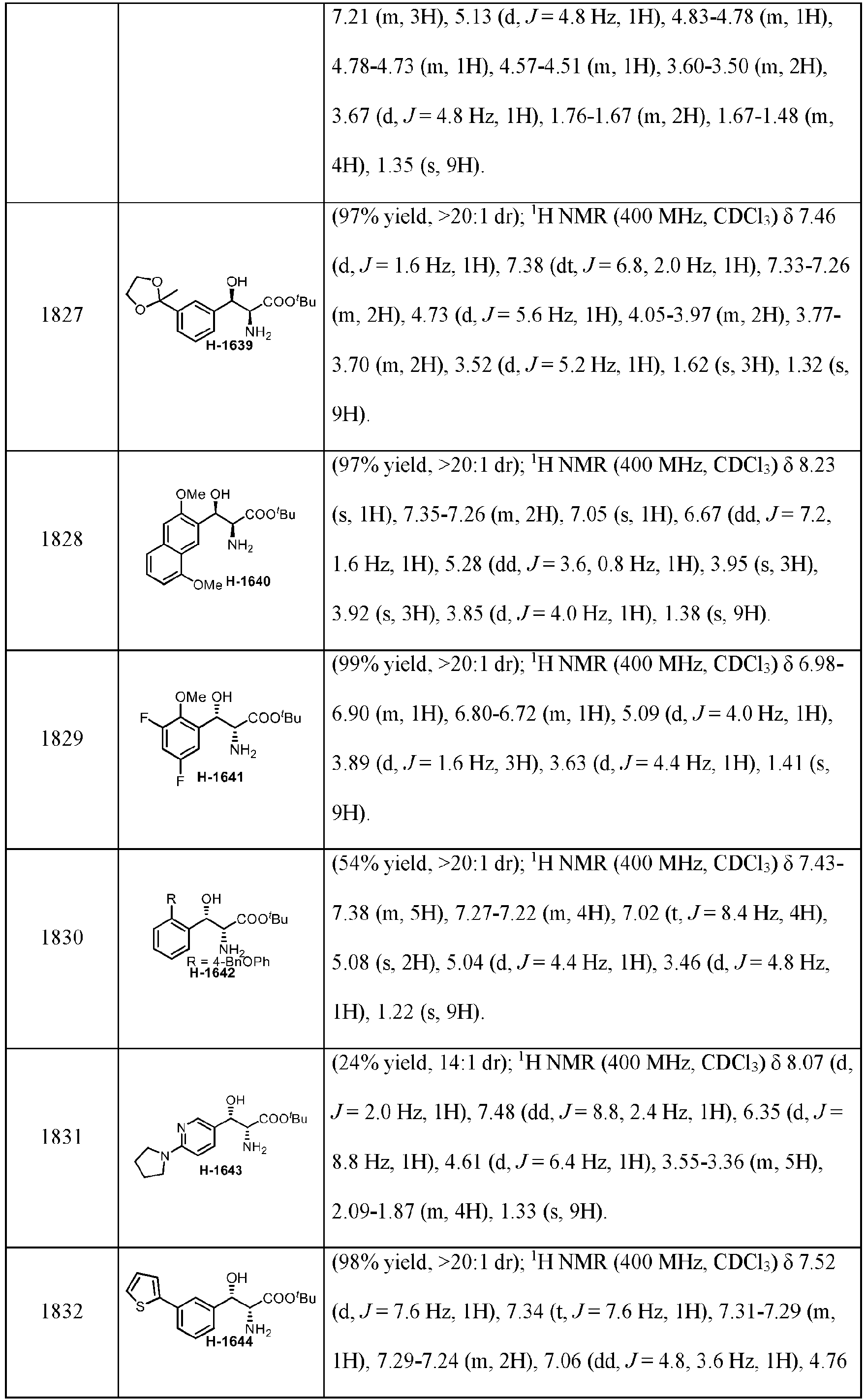

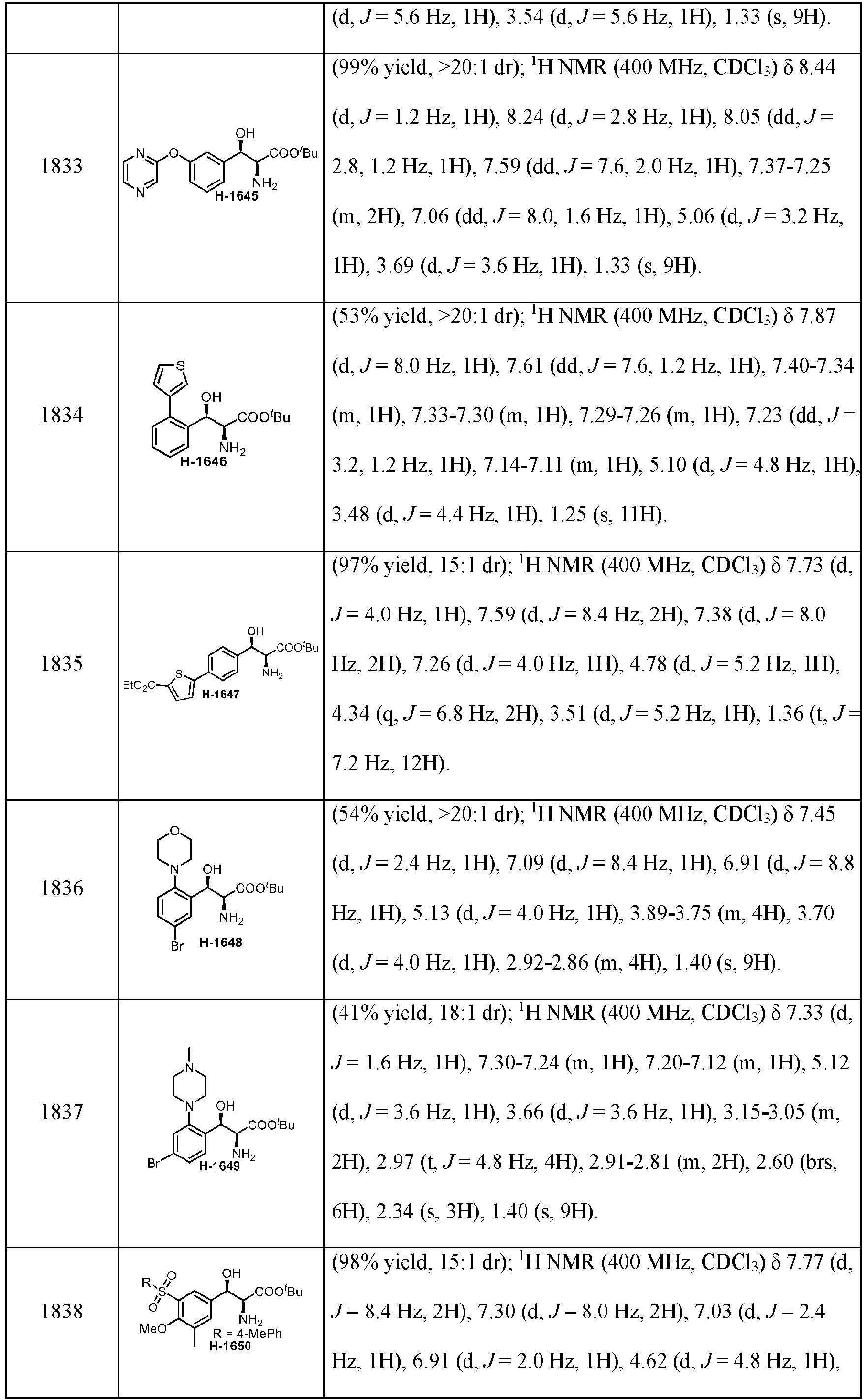

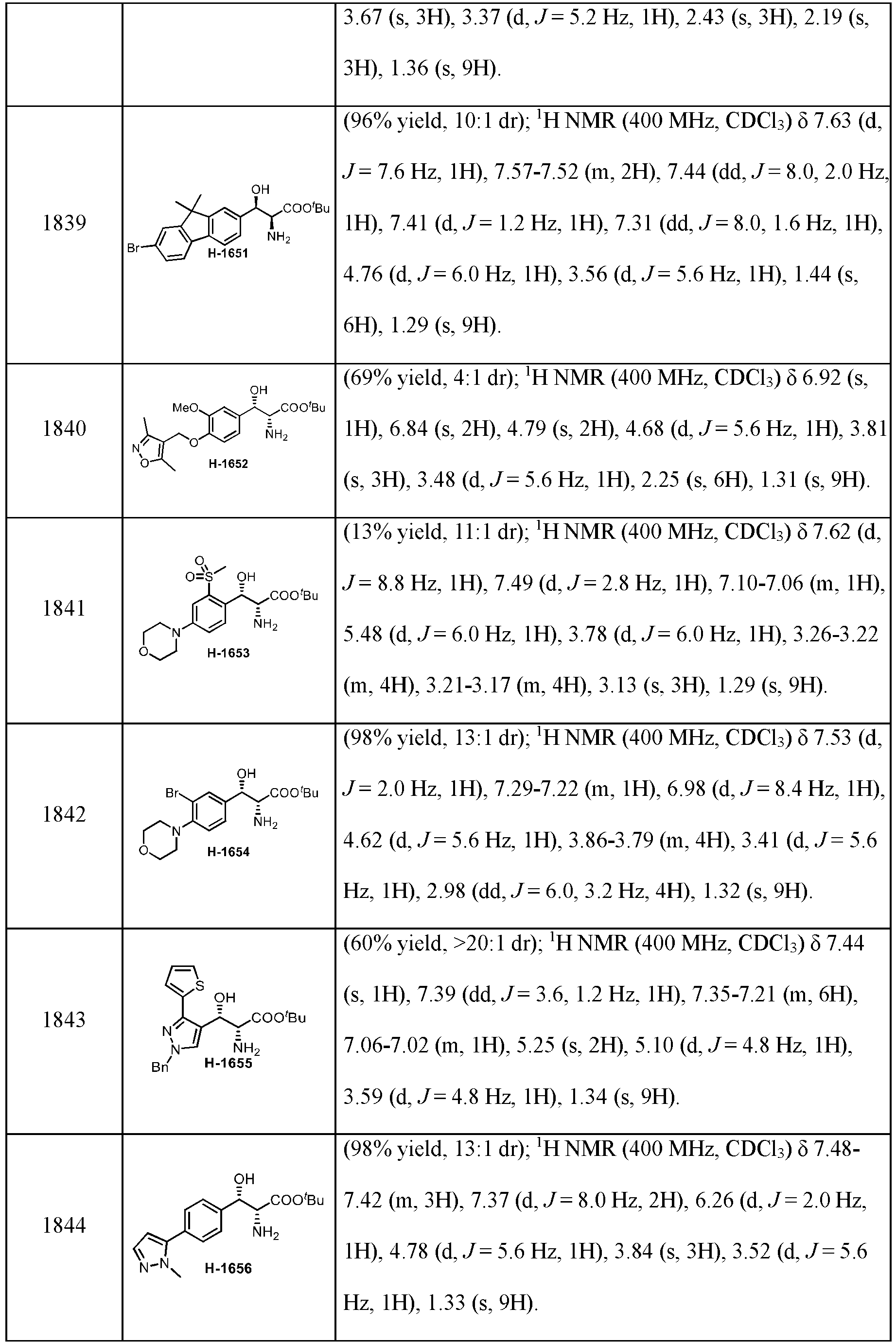

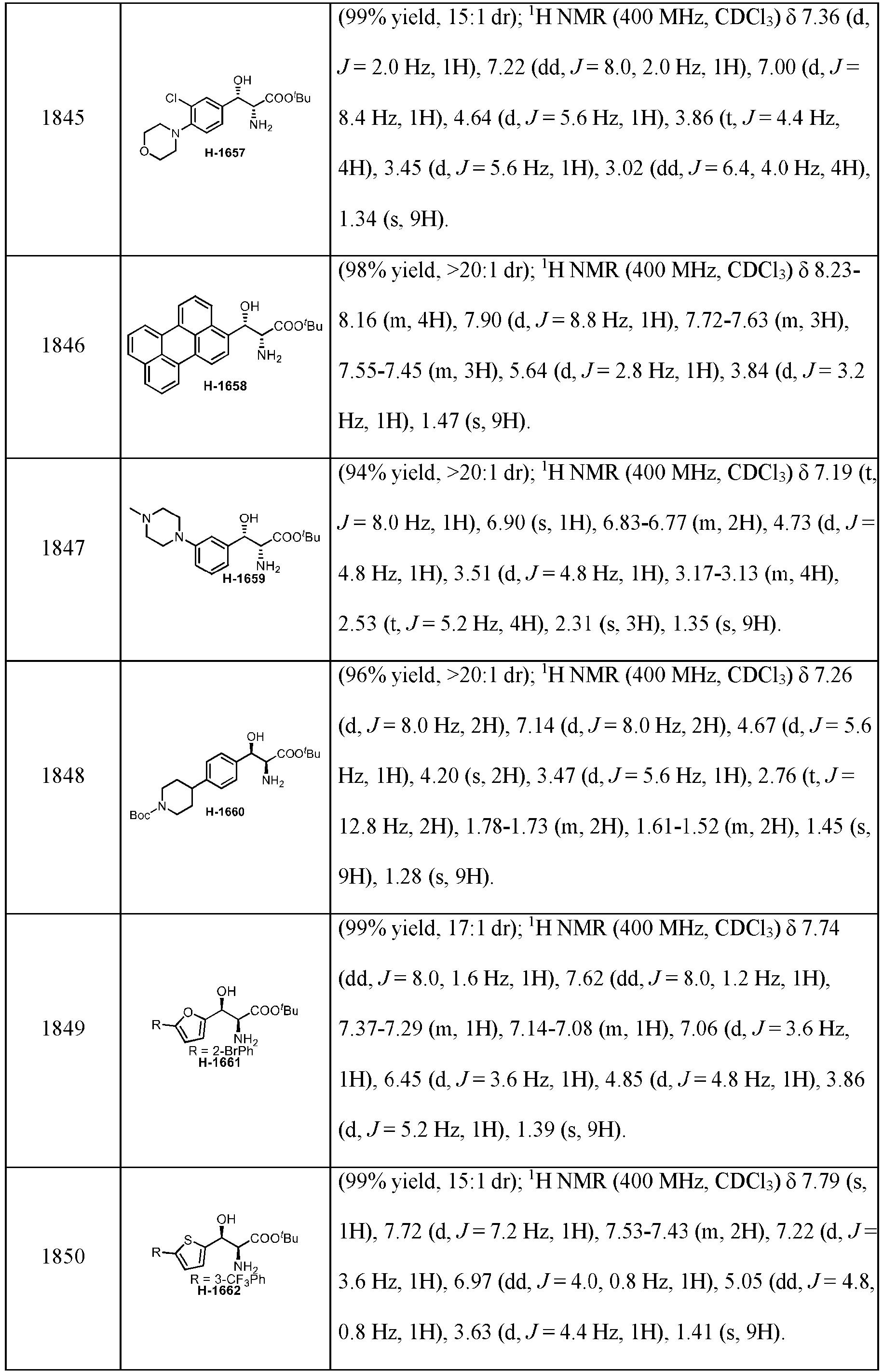

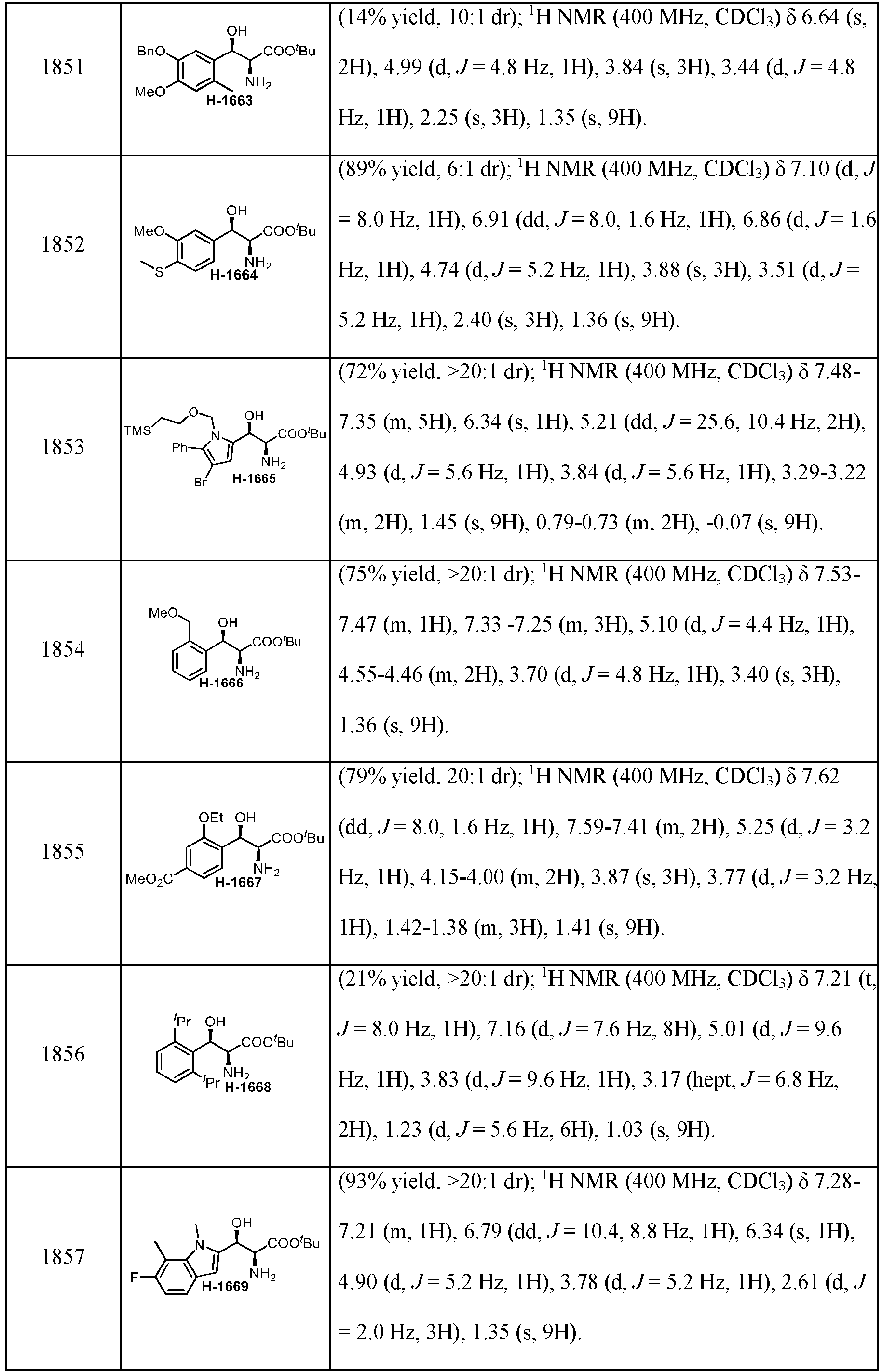

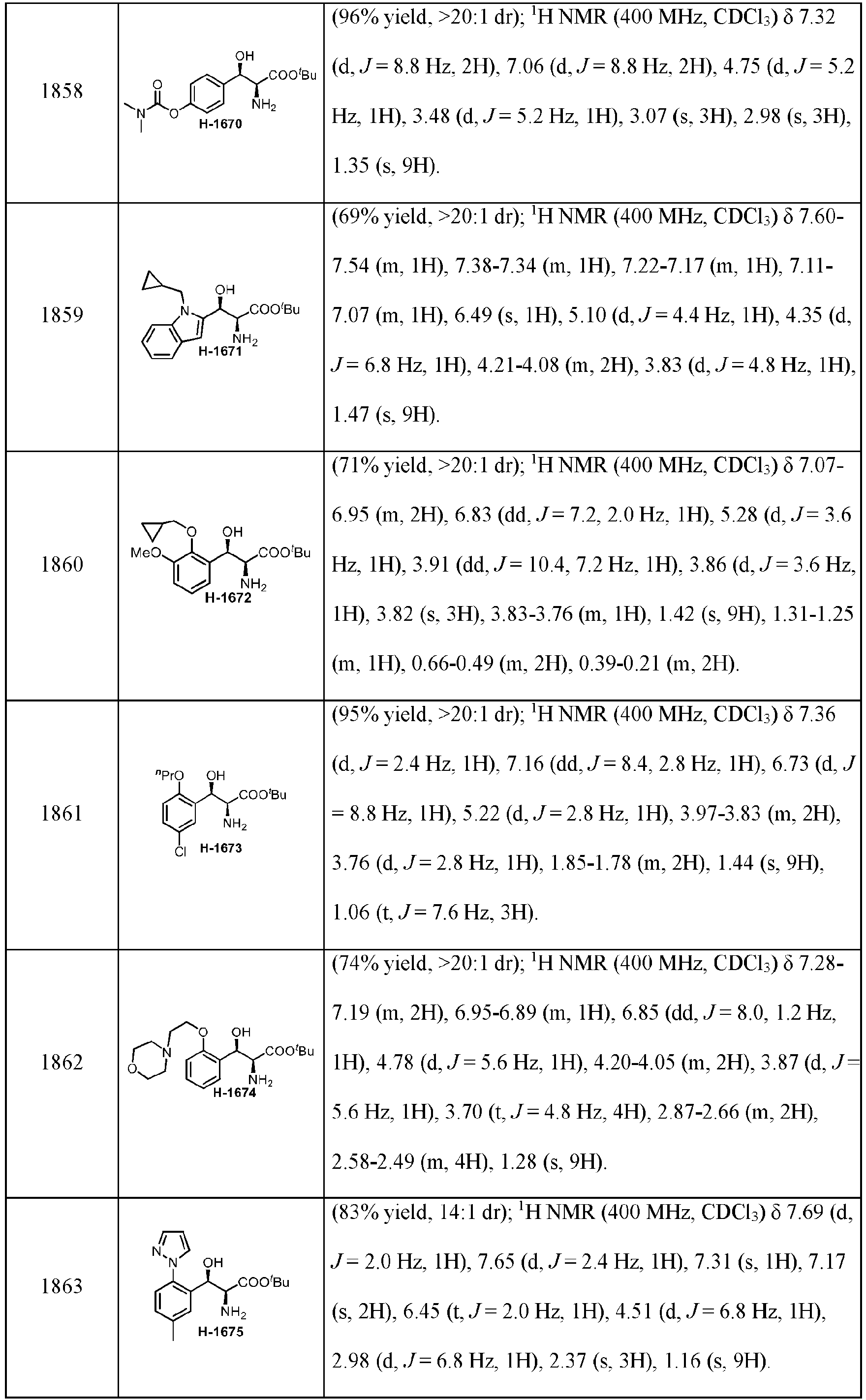

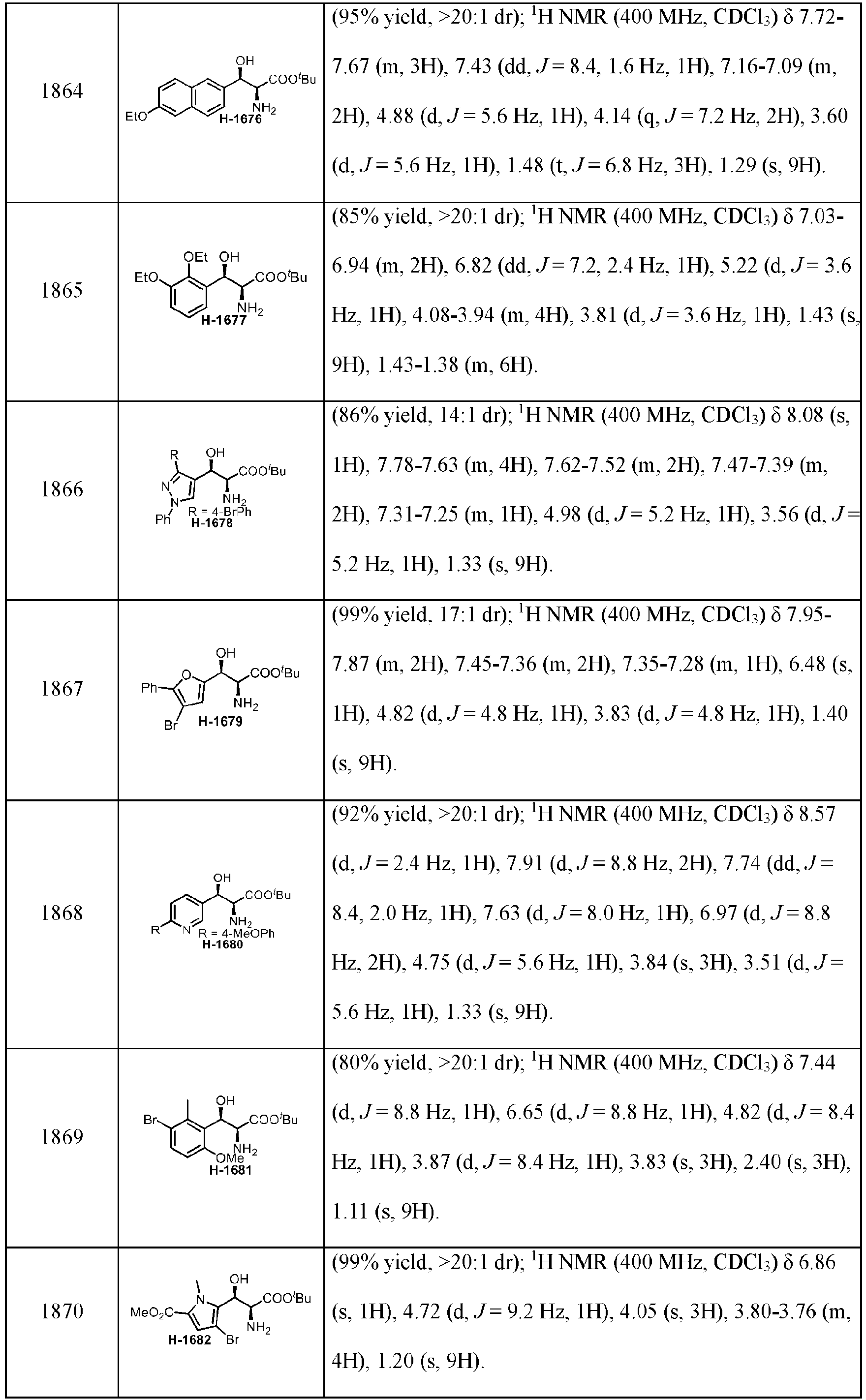

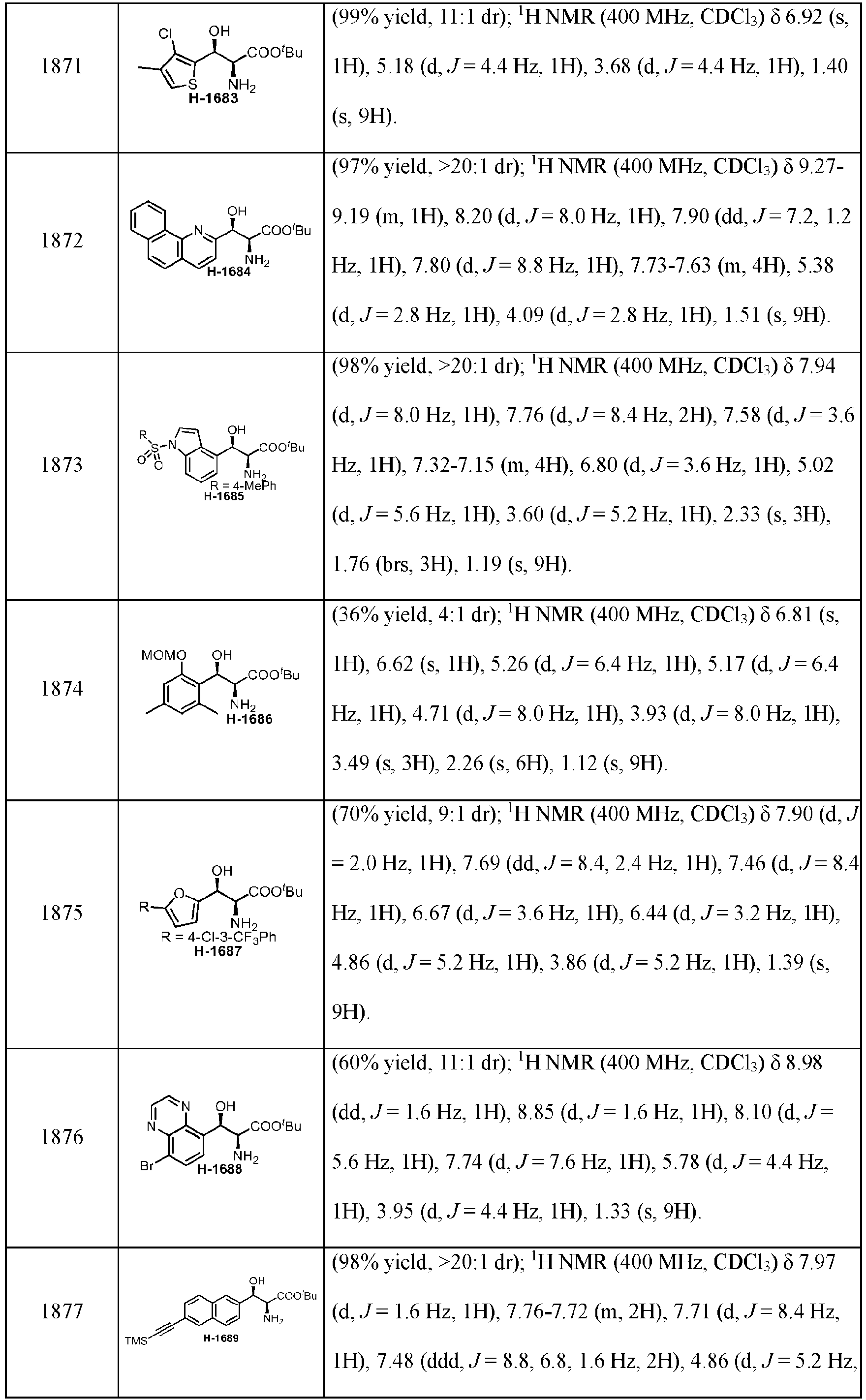

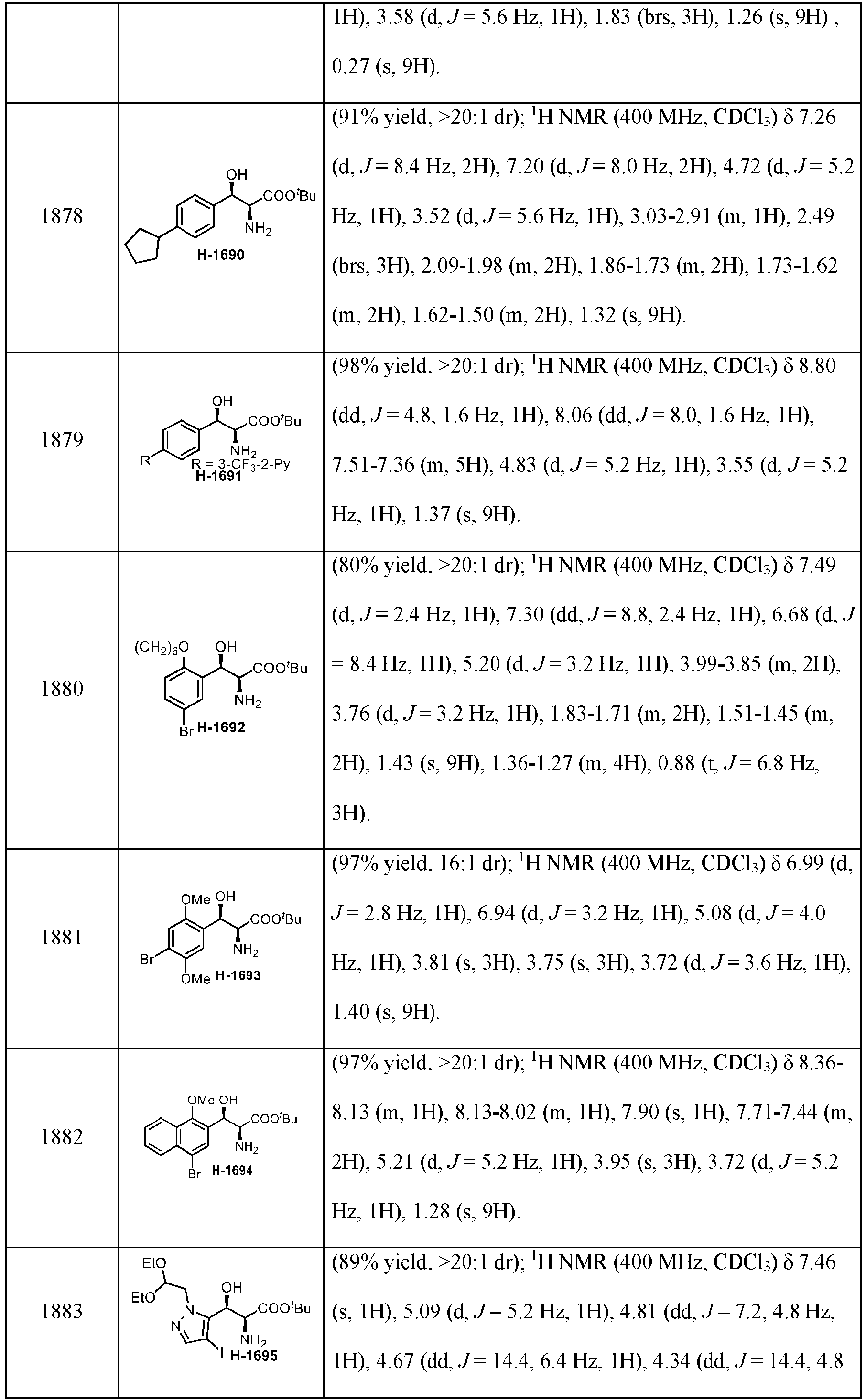

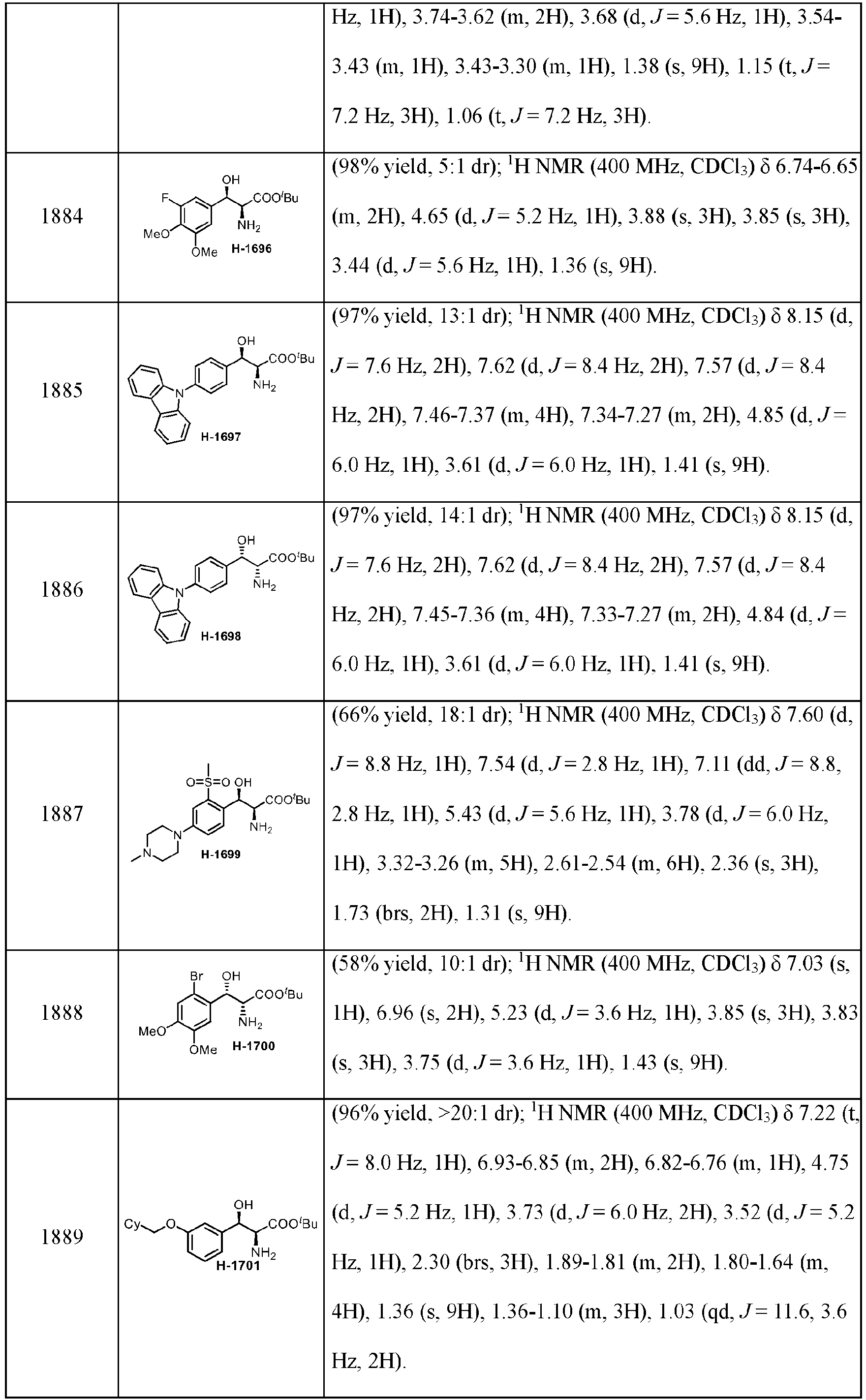

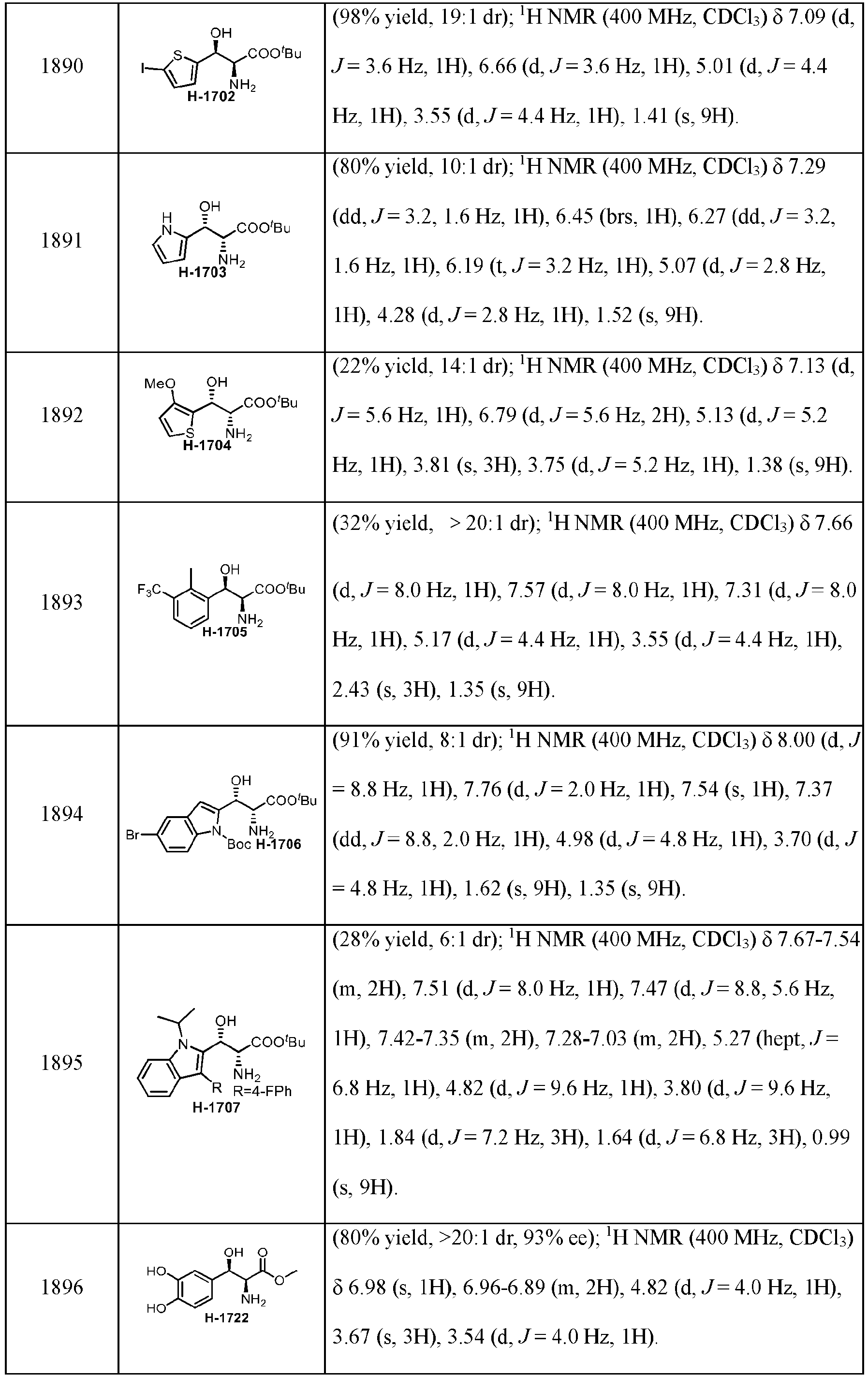

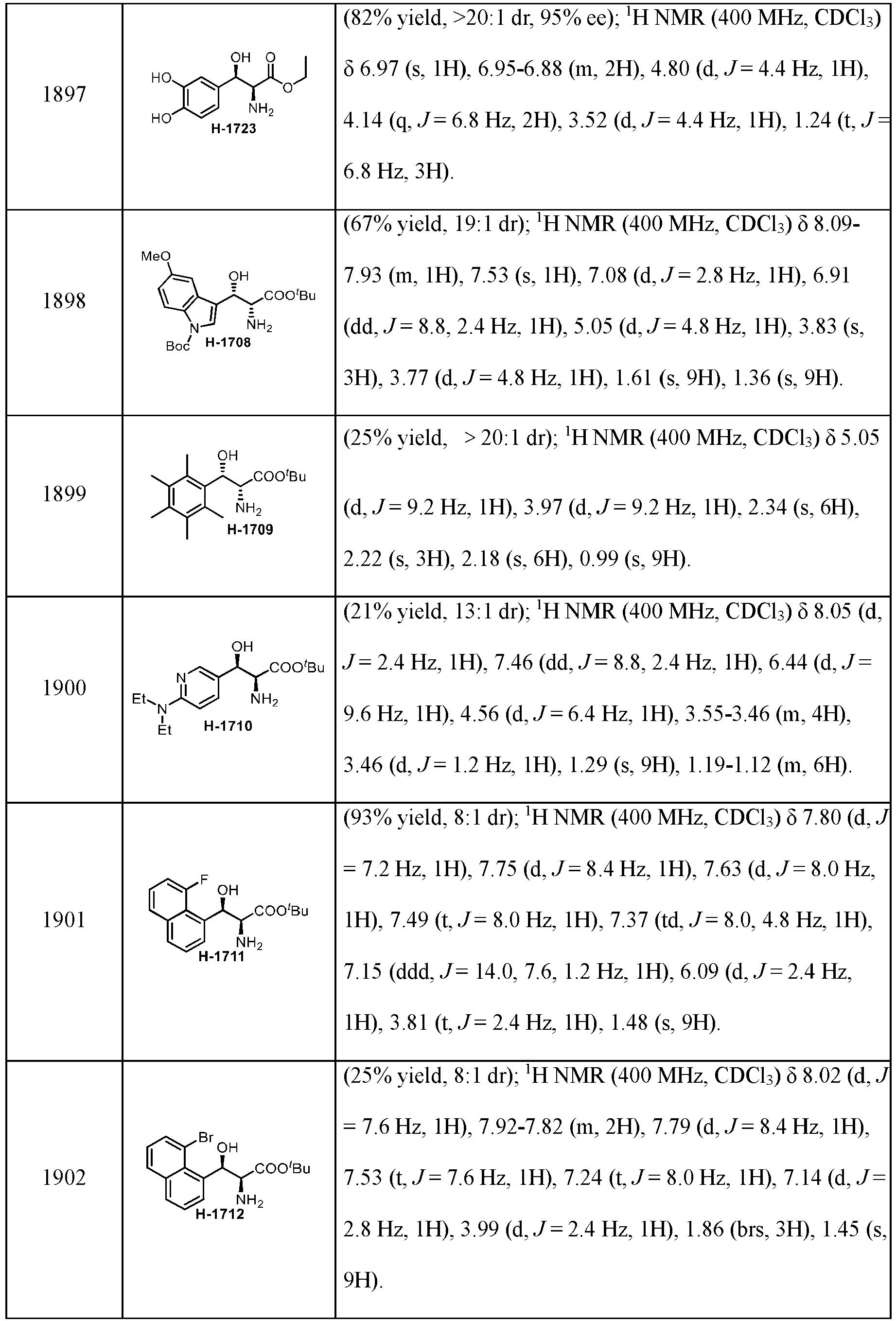

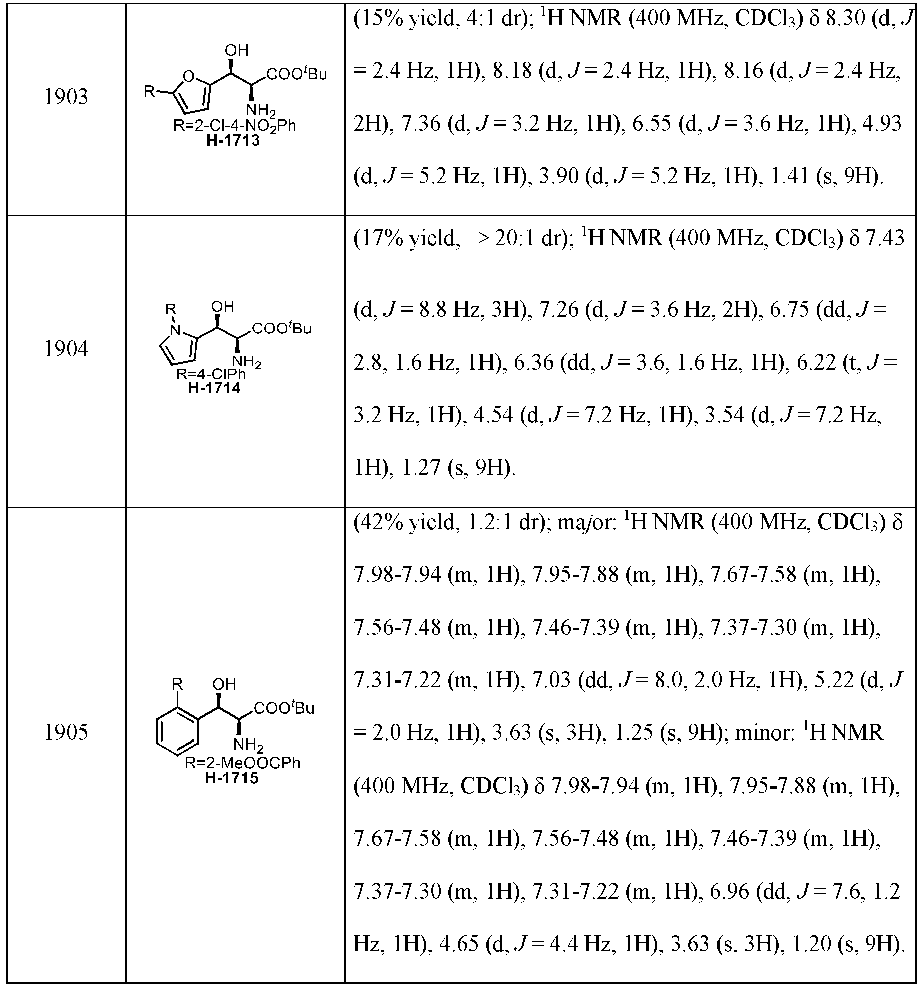

The foregoing description of the embodiments is intended to facilitate the understanding and use of the invention by those of ordinary skill in the art. Obviously, those skilled in the art can readily make various modifications to these embodiments and apply the general principles illustrated herein to other embodiments without making creative effort. Therefore, the present invention is not limited to the above embodiments, and without departing from the scope of the present invention, any improvements and modifications made by those skilled in the art based on the disclosure of the present invention should fall within the protection scope of the present invention.

## Claims

1. A chiral pyridinium salt carbonyl catalyst, wherein the pyridinium salt carbonyl catalyst 1 has a general structural formula of: 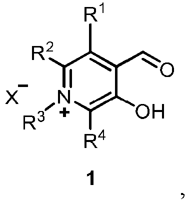 wherein R¹ has a structural formula of: 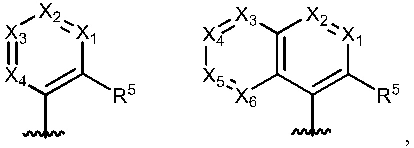
X₁ comprises N or C-R⁶, X₂ comprises N or C-R⁷, X₃ comprises N or C-R⁸, X₄ comprises N or C-R⁹, X₅ comprises N or C-R¹⁰, and X₆ comprises N or C-R¹¹;
R², R⁴, R⁶, R⁷, R⁸, R⁹, R¹⁰ and R¹¹ each comprise hydrogen, C₁₋₂₄ alkyl, alkyloxy, 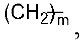 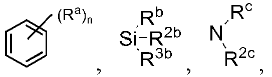 CO₂R^{d}, halogen, nitro, cyano or trifluoromethyl;
R^{a} comprises hydrogen, C₁₋₁₀ alkyl, aryl, halogen, nitro, cyano or trifluoromethyl, R^{b}, R^{2b}, R^{3b}, R^{c}, R^{2c} and R^{d} each comprise hydrogen, C₁₋₁₀ alkyl or aryl, m is 1, 2 or 3, and n is 0, 1, 2, 3, 4 or 5;
R³ comprises substituted or unsubstituted C₁₋₂₄ alkyl, the substituent comprises halogen, C₁₋₁₀ alkyl, C₃₋₁₀ cycloalkyl or aryl, C₁₋₈ carbonyl, C₁₋₈ sulfonyl or phosphoryl, or C₁₋₁₀ alkoxy or amino, and the carbonyl comprises aldehyde group, ketone carbonyl, ester carbonyl, carboxyl or amido;
R⁵ has a structural formula of: 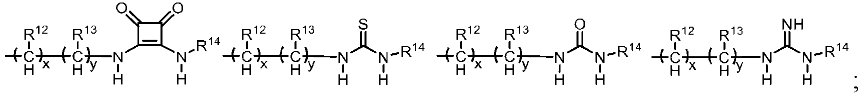
R¹² and R¹³ each comprise hydrogen, C₁₋₂₄ alkyl or 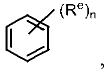 R¹⁴ comprises hydrogen, C₁₋₂₄ alkyl, 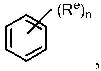 arylmethyl, diarylmethyl, triarylmethyl, halogen or trifluoromethyl;
R^{e} comprises hydrogen, C₁₋₁₀ alkyl, alkyloxy, aryl, halogen, nitro, cyano or trifluoromethyl, and x and y are 0, 1, 2 or 3;
the alkyl comprises methyl, ethyl, n-propyl, isopropyl, n-butyl, tert-butyl, isobutyl, cyclopentyl, n-pentyl, n-hexyl, cyclohexyl, n-heptyl, cycloheptyl, n-octyl, cyclooctyl, n-nonyl, cyclononyl, n-decyl or cyclodecyl, and the aryl comprises benzene, 2-biphenyl, 3-biphenyl, 4-biphenyl, 2,6-dibiphenyl, 3,5-dibiphenyl, 1-naphthyl, 2-naphthyl, 3,5-di-tert-butylbenzene, 4-tert-butylbenzene, 3,5-difluorobenzene, 4-fluorobenzene, 3,5-dichlorobenzene, 4-chlorobenzene, 3,5-bis(trifluoromethyl)benzene, 4-(trifluoromethyl)benzene, 3,5-dimethylbenzene, 4-methylbenzene or 4-methoxybenzene;
X⁻ is an anion, which comprises a fluoride ion, a chloride ion, a bromide ion, an iodide ion, a trifluoromethanesulfonate ion, a hexafluorophosphate ion, a tetrafluoroborate ion, a sulfate ion, a sulfite ion, a hydroxide ion, a nitrate ion, a phosphate ion, a carbonate ion, a silicate ion, a bicarbonate ion, a hydrogen phosphate ion, a dihydrogen phosphate ion, an iodate ion, a cyanide ion, or a thiocyanide ion.

2. The chiral pyridinium salt carbonyl catalyst according to claim 1, wherein an axially chiral configuration is formed between R¹ and a pyridine ring, and the axially chiral configuration is an R or S configuration.

3. The chiral pyridinium salt carbonyl catalyst according to claim 2, wherein the catalyst has a structure of formula Iₐ, formula I_{d}, formula IIₐ or formula II_{b} the formula Iₐ and the formula I_{b} are enantiomers of each other, and the formula II_{b} and the formula II_{b} are enantiomers of each other:

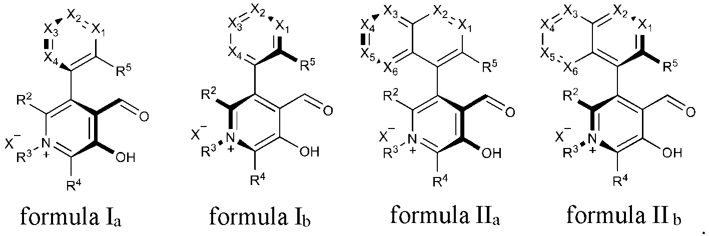

4. A preparation method for the chiral pyridinium salt carbonyl catalyst according to any one of claims 1 to 3, wherein in the preparation method, by taking a split single-configuration compound 5, i.e., a chiral amine compound, as a starting material, the chiral amine compound 5 is condensed with dimethyl squarate to produce a compound 6, the compound 6 is then condensed with primary amine to obtain an amide intermediate, the amide intermediate subsequently reacts with halogenated hydrocarbon to obtain a quaternary ammonium salt intermediate, which is finally hydrolyzed to obtain a final pyridinium salt carbonyl catalyst **1a,** with a reaction flow as follows:

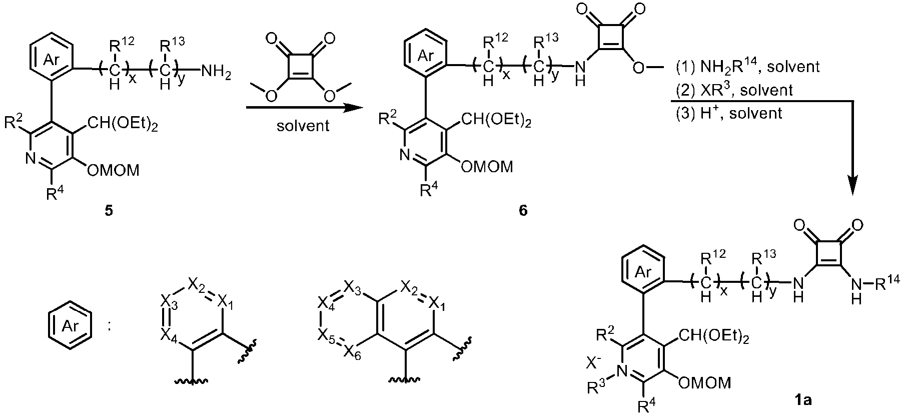

5. The preparation method for the chiral pyridinium salt carbonyl catalyst according to claim 4, wherein a molar ratio of the compound 5 to the dimethyl squarate is 1:(1 to 10), with a reaction temperature of -20°C to 120°C and a reaction time of 1 h to 72 h;
a molar ratio of the compound **6** to the primary amine to the halogenated hydrocarbon is 1:(1 to 50):(1 to 50), with a reaction temperature of 0°C to 100°C and a reaction time of 1 h to 48 h.

6. A preparation method for the chiral pyridinium salt carbonyl catalyst according to any one of claims 1 to 3, wherein in the preparation method, by taking a split single-configuration compound **5,** i.e., a chiral amine compound, as a starting material, the chiral amine compound **5** is condensed with unilateral squaramide to obtain an amide intermediate, and the amide intermediate subsequently reacts with halogenated hydrocarbon to obtain a quaternary ammonium salt intermediate, which is finally hydrolyzed to obtain a final pyridinium salt carbonyl catalyst **1a**, with a reaction flow as follows:

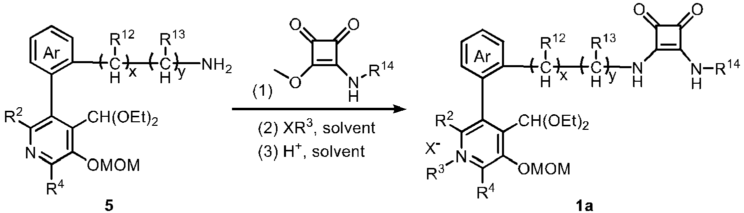

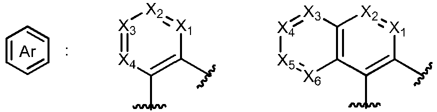

7. The preparation method for the chiral pyridinium salt carbonyl catalyst according to claim 6, wherein a molar ratio of the compound **5** to the unilateral squaramide to the halogenated hydrocarbon is 1:(1 to 50):(1 to 50), with a reaction temperature of -20°C to 120°C and a reaction time of 1 h to 72 h.

8. A preparation method for the chiral pyridinium salt carbonyl catalyst according to any one of claims 1 to 3, wherein in the preparation method, by taking a split single-configuration compound **5,** i.e., a chiral amine compound, as a starting material, the chiral amine compound **5** is condensed with thioisocyanate or isocyanate to produce a compound **7** or **8,** and the compound **7** or **8** subsequently reacts with halogenated hydrocarbon to obtain a quaternary ammonium salt intermediate, which is finally hydrolyzed to obtain a final pyridinium salt carbonyl catalyst **1b** or **1c**, with a reaction flow as follows:

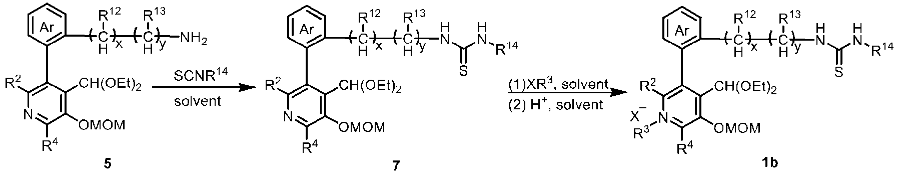

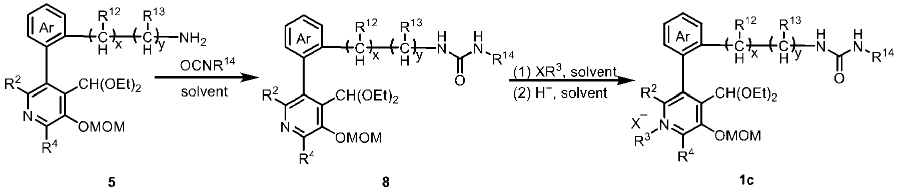

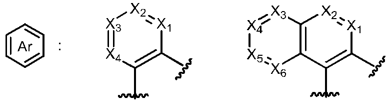

9. The preparation method for the chiral pyridinium salt carbonyl catalyst according to claim 8, wherein a molar ratio of the compound **5** to the thioisocyanate or isocyanate is 1:(1 to 10), with a reaction temperature of -20°C to 120°C and a reaction time of 1 h to 72 h;
a molar ratio of the compound **7** or **8** to the halogenated hydrocarbon is 1:(1 to 50), with a reaction temperature of 0°C to 100°C, and a reaction time of 1 h to 48 h.

10. A preparation method for the chiral pyridinium salt carbonyl catalyst according to any one of claims 1 to 3, wherein in the preparation method, by taking a split single-configuration compound **5,** i.e., a chiral amine compound, as a starting material, the chiral amine compound **5** is condensed with thioisocyanate to produce a compound **9,** the compound **9** is coupled with primary amine in the presence of carbodiimide hydrochloride to produce a compound **10,** the compound **10** is stripped of an ethyl carbonate protecting group in the presence of trimethyl bromosilane to produce a compound **11,** and the compound **11** subsequently reacts with halogenated hydrocarbon to obtain a quaternary ammonium salt intermediate, which is finally hydrolyzed to obtain a final pyridinium salt carbonyl catalyst **1d**, with a reaction flow as follows:

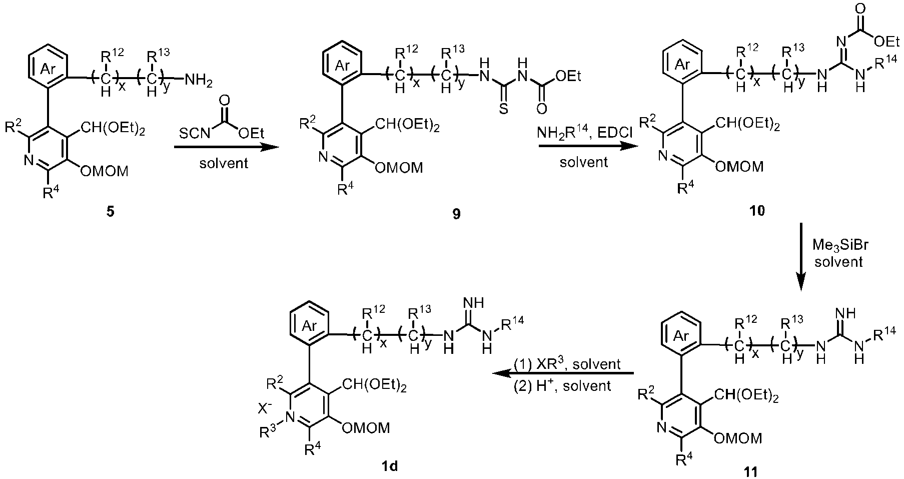

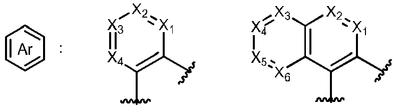

11. The preparation method for the chiral pyridinium salt carbonyl catalyst according to claim 10, wherein a molar ratio of the compound 5 to the thioisocyanate is 1:(1 to 10), with a reaction temperature of -20°C to 120°C and a reaction time of 1 h to 72 h;
a molar ratio of the compound **9** to the carbodiimide hydrochloride to the primary amine is 1:(1 to 10):(1 to 10), with a reaction temperature of 0°C to 100°C and a reaction time of 1 h to 72 h;
a molar ratio of the compound 10 to the trimethyl bromosilane is 1:(1 to 10), with a reaction temperature of 0°C to 100°C, and a reaction time of 1 h to 72 h;
a molar ratio of the compound **11** to the halogenated hydrocarbon is 1:(1 to 50), with a reaction temperature of 0°C to 100°C, and a reaction time of 1 h to 48 h.

12. The preparation method for the chiral pyridinium salt carbonyl catalyst according to claim 4, 6, 8 or 10, wherein a solvent used in the preparation method is selected from one or more of water, benzene, toluene, xylene, trimethylbenzene, acetonitrile, ethyl ether, tetrahydrofuran, ethylene glycol dimethyl ether, chloroform, dichloromethane, methanol, ethanol, isopropanol, tert-butanol, 1,4-dioxane, *N,N*-dimethylformamide, *N,N-*dimethylacetamide, dimethyl sulfoxide, and *N*-methylpyrrolidone;
an acid used is selected from one or more of sulfuric acid, hydrochloric acid, hydrochloric acid in dichloromethane, hydrochloric acid in methanol, hydrochloric acid in tetrahydrofuran, hydrochloric acid in dioxane, phosphoric acid, hydrobromic acid, hydroiodic acid, acetic acid, trifluoroacetic acid, trichloroacetic acid, benzenesulphonic acid, p-toluenesulfonic acid, methanesulfonic acid and trifluoromethanesulfonic acid.

13. A preparation method for the chiral pyridinium salt carbonyl catalyst according to any one of claims 1 to 3, wherein in the preparation method, a silver salt is added to a chiral pyridinium salt carbonyl catalyst with an iodide ion for replacement to obtain a chiral pyridinium salt carbonyl catalyst with a corresponding anion of a silver salt;
the silver salt comprises silver fluoride, silver chloride, silver bromide, silver trifluoromethanesulfonate, silver hexafluorophosphate, silver tetrafluoroborate, silver sulfate, silver sulfite, silver hydroxide, silver nitrate, silver phosphate, silver carbonate, silver silicate, silver bicarbonate, silver hydrogen phosphate, silver dihydrogen phosphate, silver iodate, silver cyanide or silver thiocyanate.

14. Application of the chiral pyridinium salt carbonyl catalyst according to any one of claims 1 to 3, wherein the pyridinium salt carbonyl catalyst 1 is applied to an asymmetric biomimetic aldol reaction of an amino acid derivative and a carbonyl compound to synthesize a chiral β-hydroxy-α-amino acid compound, and the reaction comprises the steps of:
carrying out an aldol reaction on an amino acid derivative **2** and a carbonyl compound **3** under catalysis of the pyridinium salt carbonyl catalyst **1** to produce a chiral β-hydroxy-α-amino acid compound **4,** wherein different configurations such as *syn-***4A***, anti*-**4A** and *syn-***4B***, anti*-**4B**, are comprised, and an absolute configuration of the pyridinium salt carbonyl catalyst **1** determines an absolute configuration of the chiral β-hydroxy-α-amino acid compound 4, with reaction equations as follows: 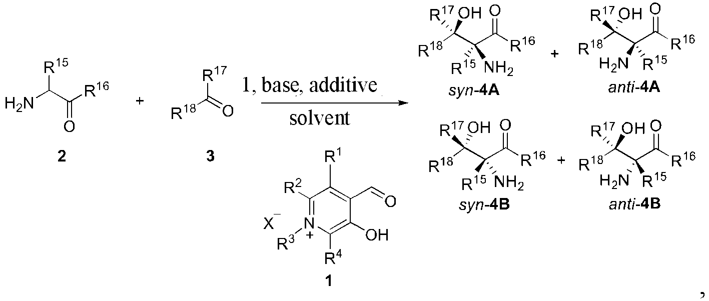
wherein R¹⁵ comprises hydrogen, substituted or unsubstituted C₁₋₂₄ alkyl, substituted or unsubstituted C₃-C₁₈ aryl, substituted or unsubstituted C₂₋₁₈ alkenyl, substituted or unsubstituted C₂₋₂₄ alkynyl, or C₁₋₁₈ carbonyl, the substituent comprises halogen, cyano, hydroxyl, nitro, C₁₋₁₈ alkyl, C₃₋₁₈ aryl, C₁₋₁₈ carbonyl, C₁₋₁₈ sulfonyl or phosphoryl, C₁₋₁₈ alkoxy, C₃₋₁₈ aryloxy or C₁₋₁₈ amino, the carbonyl comprises aldehyde group, ketone carbonyl, ester carbonyl, carboxyl or amido;
R¹⁶ is OR¹⁹ or NR²⁰R²¹, wherein R¹⁹ comprises hydrogen, substituted or unsubstituted C₁₋₂₄ alkyl, substituted or unsubstituted C₃₋₁₈ aryl, or C₁₋₁₈ carbonyl, R²⁰ and R²¹ each comprise hydrogen, substituted or unsubstituted C₁₋₂₄ alkyl, substituted or unsubstituted C₃₋₁₈ aryl, or C₁₋₁₈ carbonyl, or R²⁰ and R²¹ are linked together to form a ring B or NR²⁰R²¹ comprises amino acid amino, amino acid ester amino, amino acid amide amino or polypeptide amino, the substituent comprises halogen, cyano, hydroxyl, nitro, C₁₋₁₈ alkyl, C₃₋₁₈ aryl, C₁₋₁₈ carbonyl, C₁₋₁₈ sulfonyl or phosphoryl, C₁₋₁₈ alkoxy, C₃₋₁₈ aryloxy or C₁₋₁₈ amino, and the carbonyl comprises aldehyde group, ketone carbonyl, ester carbonyl, carboxyl or amido, the ring B is a C₁₋₁₈ ring with 1-4 heteroatoms, which comprise O, N or S atoms;
R¹⁷ and R¹⁸ each comprise hydrogen, substituted or unsubstituted C₁₋₂₄ alkyl, substituted or unsubstituted C₃₋₁₈ aryl, substituted or unsubstituted C₂₋₁₈ alkenyl, substituted or unsubstituted C₂₋₂₄ alkynyl, the substituent comprises halogen, cyano, hydroxyl, nitro, trifluoromethyl, C₁₋₁₈ alkyl, C₃₋₁₈ aryl, C₁₋₁₈ carbonyl, C₁₋₁₈ sulfonyl or phosphoryl, C₁₋₁₈
alkoxy, C₃₋₁₈ aryloxy or C₁₋₁₈ amino, 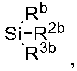
and the carbonyl comprises aldehyde group, ketone carbonyl, ester carbonyl, carboxyl or amido;
R^{b}, R^{2b} and R^{3b} each comprise hydrogen, C₁₋₁₀ alkyl or aryl, m is 1, 2 or 3, and n is 0, 1, 2, 3, 4 or 5.

15. The application of the chiral pyridinium salt carbonyl catalyst according to claim 14, wherein a molar ratio of the amino acid derivative **2** to the carbonyl compound **3** is (0.5 to 5):1, and the molar ratio of the pyridinium salt carbonyl catalyst **1** to the carbonyl compound **3** is (0.00001 to 0.5):1, with a reaction temperature of -60°C to 100°C and a reaction time of 1 h to 72 h;
a solvent used in the reaction is selected from one or more of water, methanol, ethanol, isopropanol, n-propanol, n-butanol, tert-butanol, 1,4-dioxane, trifluoroethanol, benzene, toluene, xylene, trimethylbenzene, acetonitrile, ethyl ether, tetrahydrofuran, ethylene glycol dimethyl ether, chloroform, dichloromethane, *N,N-*dimethylformamide, *N,N-*dimethylacetamide, dimethyl sulfoxide, and *N*-methylpyrrolidone;
an base used is selected from one or more lithium hydroxide, sodium hydroxide, potassium hydroxide, sodium carbonate, potassium carbonate, cesium carbonate, sodium bicarbonate, potassium bicarbonate, sodium phosphate, potassium phosphate, disodium hydrogen phosphate, dipotassium hydrogen phosphate, sodium hydride, potassium hydride, calcium hydride, trimethylamine, triethylamine, diisopropylamine, diisopropylethylamine, tetramethylethylenediamine, *N,N*-diethylmethylamine, *N,N-*dimethylethylamine, *N,N-*dimethylpropylamine, *N,N*-dimethylbutylamine, *N,N*-dimethylaniline, *N,N-*diethylaniline, 1,4-diazabicyclooctane, 1,8-diazabicyclo[5.4.0]undec-7-ene, 1,5-diazabicyclo[4.3.0]-5-nonene, n-butyl lithium, 1,4-dimethylpiperazine, 1-methylpiperidine, 1-methylpyrrole, n-butylamine, tert-butylamine, diethylamine, ethylenediamine, quinoline and pyridine;
an additive used is selected from one or more of ammonium phosphate, ammonium dihydrogen phosphate, diammonium hydrogen phosphate, sodium phosphate, sodium dihydrogen phosphate, disodium hydrogen phosphate, potassium phosphate, potassium dihydrogen phosphate, dipotassium hydrogen phosphate, lithium phosphate, lithium dihydrogen phosphate, dilithium hydrogen phosphate, sodium carbonate, sodium bicarbonate, potassium carbonate, potassium bicarbonate, sodium acetate, potassium acetate, potassium fluoride and sodium fluoride.
